# EUROPEAN PATENT APPLICATION

(11) **EP 3 895 729 A1**
(43) Date of publication of application: **20.10.2021**
(21) Application number: 21167076.5
(22) Date of filing: 06.04.2021
(51) Int. Cl.: A61K 39/12, A61P 31/14, A61K 39/00

(54) **CPG-ADJUVANTED SARS-COV-2 VIRUS VACCINE**

(30) Priority: 01.03.2020 US 202062983737 P; 06.04.2020 EP 20168324; 15.10.2020 EP 20202124; 04.12.2020 EP 20211936; 01.02.2021 EP 21154645; 01.03.2021 WO PCT/US2021/020313; 05.03.2021 EP 21160933
(71) Applicant: Valneva Austria GmbH, 1030 Vienna (AT); Dynavax Technologies Corporation, Emeryville, CA 94608 (US)
(72) Inventor: MEINKE, Andreas, 3031 Pressbaum (AT); REINISCH, Christoph, 2500 Siegenfeld (AT); SCHLEGL, Robert, 2500 Siegenfeld (AT); TAUCHER, Christian, 1210 Vienna (AT); NOVACK, David, Emeryville, CA 94608 (US); JANSSEN, Robert S., Emeryville, CA 94608 (US); CAMPBELL, John D., Emeryville, CA 94608 (US); MÖHLEN, Michael, 1050 Vienna (AT)
(74) Representative: Script IP Limited

(57) **Abstract**

Described herein are CpG-adjuvanted SARS-CoV-2 vaccines and compositions and methods of producing and administering said vaccines to subjects in need thereof.

SEQUENCE LISTING

<110>  Valneva Austria GmbH
       Dynavax Technologies Corporation

<120>  CpG-Adjuvanted SARS-CoV-2 virus vaccine

<130>  P6141WO00

<150> US 62983737
<151> 2020-03-01

<150> EP 20168324.0
<151> 2020-04-06

<150> EP 20202124.2
<151> 2020-10-15

<150> EP 20211936.8
<151> 2020-12-04

<150> EP 21154645.2
<151> 2021-02-01

<150> EP 21160933.4
<151> 2021-03-05

<150> US 21/20313
<151> 2021-03-01

<160>  32

<170>  PatentIn version 3.5

<210>  1
<211>  29903
<212>  DNA
<213>  SARS-CoV2

<400>  1
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc     180

ttctgcaggc tgcttacggt ttcgtccgtg ttgcagccga tcatcagcac atctaggttt     240

cgtccgggtg tgaccgaaag gtaagatgga gagccttgtc cctggtttca acgagaaaac     300

acacgtccaa ctcagtttgc ctgttttaca ggttcgcgac gtgctcgtac gtggctttgg     360

agactccgtg gaggaggtct tatcagaggc acgtcaacat cttaaagatg gcacttgtgg     420

cttagtagaa gttgaaaaag gcgttttgcc tcaacttgaa cagccctatg tgttcatcaa     480

acgttcggat gctcgaactg cacctcatgg tcatgttatg gttgagctgg tagcagaact     540

cgaaggcatt cagtacggtc gtagtggtga gacacttggt gtccttgtcc ctcatgtggg     600

cgaaatacca gtggcttacc gcaaggttct tcttcgtaag aacggtaata aaggagctgg     660

tggccatagt tacggcgccg atctaaagtc atttgactta ggcgacgagc ttggcactga     720

tccttatgaa gattttcaag aaaactggaa cactaaacat agcagtggtg ttacccgtga 780

actcatgcgt gagcttaacg gaggggcata cactcgctat gtcgataaca acttctgtgg 840

ccctgatggc taccctcttg agtgcattaa agaccttcta gcacgtgctg gtaaagcttc 900

atgcactttg tccgaacaac tggactttat tgacactaag aggggtgtat actgctgccg 960

tgaacatgag catgaaattg cttggtacac ggaacgttct gaaaagagct atgaattgca 1020

gacacctttt gaaattaaat tggcaaagaa atttgacacc ttcaatgggg aatgtccaaa 1080

ttttgtattt cccttaaatt ccataatcaa gactattcaa ccaagggttg aaaagaaaaa 1140

gcttgatggc tttatgggta gaattcgatc tgtctatcca gttgcgtcac caaatgaatg 1200

caaccaaatg tgcctttcaa ctctcatgaa gtgtgatcat tgtggtgaaa cttcatggca 1260

gacgggcgat tttgttaaag ccacttgcga attttgtggc actgagaatt tgactaaaga 1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 3000

actgggcatt gatttagatg agtggagtat ggctacatac tacttatttg atgagtctgg 3000

tgagtttaaa ttggcttcac atatgtattg ttctttctac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

taaggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttgttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacctcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgtct ggttttaagc taaaagactg tgttatgtat gcatcagctg tagtgttact 11340

aatccttatg acagcaagaa ctgtgtatga tgatggtgct aggagagtgt ggacacttat 11400

gaatgtcttg acactcgttt ataaagttta ttatggtaat gctttagatc aagccatttc 11460

catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccaccta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140

gtttaagaat attgatggtt attttaaaat atattctaag cacacgccta ttaatttagt 22200

gcgtgatctc cctcagggtt tttcggcttt agaaccattg gtagatttgc caataggtat 22260

taacatcact aggtttcaaa ctttacttgc tttacataga agttatttga ctcctggtga 22320

ttcttcttca ggttggacag ctggtgctgc agcttattat gtgggttatc ttcaacctag 22380

gacttttcta ttaaaatata atgaaaatgg aaccattaca gatgctgtag actgtgcact 22440

tgaccctctc tcagaaacaa agtgtacgtt gaaatccttc actgtagaaa aaggaatcta 22500

tcaaacttct aactttagag tccaaccaac agaatctatt gttagatttc ctaatattac 22560

aaacttgtgc ccttttggtg aagtttttaa cgccaccaga tttgcatctg tttatgcttg 22620

gaacaggaag agaatcagca actgtgttgc tgattattct gtcctatata attccgcatc 22680

attttccact tttaagtgtt atggagtgtc tcctactaaa ttaaatgatc tctgctttac 22740

taatgtctat gcagattcat ttgtaattag aggtgatgaa gtcagacaaa tcgctccagg 22800

gcaaactgga aagattgctg attataatta taaattacca gatgatttta caggctgcgt 22860

tatagcttgg aattctaaca atcttgattc taaggttggt ggtaattata attacctgta 22920

tagattgttt aggaagtcta atctcaaacc ttttgagaga gatatttcaa ctgaaatcta 22980

tcaggccggt agcacacctt gtaatggtgt tgaaggtttt aattgttact ttcctttaca 23040

atcatatggt ttccaaccca ctaatggtgt tggttaccaa ccatacagag tagtagtact 23100

ttcttttgaa cttctacatg caccagcaac tgtttgtgga cctaaaaagt ctactaattt 23160

ggttaaaaac aaatgtgtca atttcaactt caatggttta acaggcacag gtgttcttac 23220

tgagtctaac aaaaagtttc tgcctttcca acaatttggc agagacattg ctgacactac 23280

tgatgctgtc cgtgatccac agacacttga gattcttgac attacaccat gttcttttgg 23340

tggtgtcagt gttataacac caggaacaaa tacttctaac caggttgctg ttctttatca 23400

ggatgttaac tgcacagaag tccctgttgc tattcatgca gatcaactta ctcctacttg 23460

gcgtgtttat tctacaggtt ctaatgtttt tcaaacacgt gcaggctgtt taataggggc 23520

tgaacatgtc aacaactcat atgagtgtga catacccatt ggtgcaggta tatgcgctag 23580

ttatcagact cagactaatt ctcctcggcg ggcacgtagt gtagctagtc aatccatcat 23640

tgcctacact atgtcacttg gtgcagaaaa ttcagttgct tactctaata actctattgc 23700

catacccaca aattttacta ttagtgttac cacagaaatt ctaccagtgt ctatgaccaa 23760

gacatcagta gattgtacaa tgtacatttg tggtgattca actgaatgca gcaatctttt 23820

gttgcaatat ggcagttttt gtacacaatt aaaccgtgct ttaactggaa tagctgttga 23880

acaagacaaa aacacccaag aagtttttgc acaagtcaaa caaatttaca aaacaccacc 23940

aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag cttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acggttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagac aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaacgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta ggggaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagaatgac aaaaaaaaaa 29880

aaaaaaaaaa aaaaaaaaaa aaa 29903

<210> 2
<211> 7096
<212> PRT
<213> SARS-CoV2

<400> 2

<210> 3
<211> 1273
<212> PRT
<213> SARS-CoV2

<400> 3

<210>  4
<211>  22
<212>  DNA
<213>  Artificial Sequence

<220>
<223>  Artificial sequence

<400>  4
tgactgtgaa cgttcgagat ga                                              22

<210>  5
<211>  11
<212>  PRT
<213>  Artificial Sequence

<220>
<223>  Artificial sequence

<400>  5

Lys Leu Lys Leu Leu Leu Leu Leu Lys Leu Lys

1                5                   10

<210>  6
<211>  26
<212>  DNA
<213>  Artificial Sequence

<220>
<223>  Artificial sequence

<220>
<221>  misc_feature
<222>  (1)..(26)
<223>  n=deoxyinosine

<400>  6
ncncncncnc ncncncncnc ncncnc                                          26

<210>  7
<211>  20
<212>  DNA
<213>  Artificial Sequence

<220>
<223>  Artificial sequence

<400>  7
tccatgacgt tcctgacgtt                                                 20

<210>  8
<211>  24
<212>  DNA
<213>  Artificial Sequence

<220>
<223>  Artificial sequence

<400>  8
tcgtcgtttt gtcgttttgt cgtt                                            24

<210>  9
<211>  29867
<212>  DNA
<213>  SARS-CoV2

<220>
<221>  misc_feature
<222>  (11083)..(11083)
<223>  n is a, c, g, or t

<400>  9
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc     180

ttctgcaggc tgcttacggt ttcgtccgtg ttgcagccga tcatcagcac atctaggttt 240

cgtccgggtg tgaccgaaag gtaagatgga gagccttgtc cctggtttca acgagaaaac 300

acacgtccaa ctcagtttgc ctgttttaca ggttcgcgac gtgctcgtac gtggctttgg 360

agactccgtg gaggaggtct tatcagaggc acgtcaacat cttaaagatg gcacttgtgg 420

cttagtagaa gttgaaaaag gcgttttgcc tcaacttgaa cagccctatg tgttcatcaa 480

acgttcggat gctcgaactg cacctcatgg tcatgttatg gttgagctgg tagcagaact 540

cgaaggcatt cagtacggtc gtagtggtga gacacttggt gtccttgtcc ctcatgtggg 600

cgaaatacca gtggcttacc gcaaggttct tcttcgtaag aacggtaata aaggagctgg 660

tggccatagt tacggcgccg atctaaagtc atttgactta ggcgacgagc ttggcactga 720

tccttatgaa gattttcaag aaaactggaa cactaaacat agcagtggtg ttacccgtga 780

actcatgcgt gagcttaacg gaggggcata cactcgctat gtcgataaca acttctgtgg 840

ccctgatggc taccctcttg agtgcattaa agaccttcta gcacgtgctg gtaaagcttc 900

atgcactttg tccgaacaac tggactttat tgacactaag aggggtgtat actgctgccg 960

tgaacatgag catgaaattg cttggtacac ggaacgttct gaaaagagct atgaattgca 1020

gacacctttt gaaattaaat tggcaaagaa atttgacacc ttcaatgggg aatgtccaaa 1080

ttttgtattt cccttaaatt ccataatcaa gactattcaa ccaagggttg aaaagaaaaa 1140

gcttgatggc tttatgggta gaattcgatc tgtctatcca gttgcgtcac caaatgaatg 1200

caaccaaatg tgcctttcaa ctctcatgaa gtgtgatcat tgtggtgaaa cttcatggca 1260

gacgggcgat tttgttaaag ccacttgcga attttgtggc actgagaatt tgactaaaga 1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcta aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 2940

actgggcatt gatttagatg agtggagtat ggctacatac tacttatttg atgagtctgg 3000

tgagtttaaa ttggcttcac atatgtattg ttctttctac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720
tttctattgg ttctttagta attacctaaa gagacgtgta gtctttaatg gtgtttcctt 9780
tagtactttt gaagaagctg cgctgtgcac ctttttgtta aataaagaaa tgtatctaaa 9840
gttgcgtagt gatgtgctat tacctcttac gcaatataat agatacttag ctctttataa 9900
taagtacaag tattttagtg gagcaatgga tacaactagc tacagagaag ctgcttgttg 9960
tcatctcgca aaggctctca atgacttcag taactcaggt tctgatgttc tttaccaacc 10020
accacaaacc tctatcacct cagctgtttt gcagagtggt tttagaaaaa tggcattccc 10080
atctggtaaa gttgagggtt gtatggtaca agtaacttgt ggtacaacta cacttaacgg 10140
tctttggctt gatgacgtag tttactgtcc aagacatgtg atctgcacct ctgaagacat 10200
gcttaaccct aattatgaag atttactcat tcgtaagtct aatcataatt tcttggtaca 10260
ggctggtaat gttcaactca gggttattgg acattctatg caaaattgtg tacttaagct 10320
taaggttgat acagccaatc ctaagacacc taagtataag tttgttcgca ttcaaccagg 10380
acagactttt tcagtgttag cttgttacaa tggttcacca tctggtgttt accaatgtgc 10440
tatgaggccc aatttcacta ttaagggttc attccttaat ggttcatgtg gtagtgttgg 10500
ttttaacata gattatgact gtgtctcttt ttgttacatg caccatatgg aattaccaac 10560
tggagttcat gctggcacag acttagaagg taacttttat ggaccttttg ttgacaggca 10620
aacagcacaa gcagctggta cggacacaac tattacagtt aatgttttag cttggttgta 10680
cgctgctgtt ataaatggag acaggtggtt tctcaatcga tttaccacaa ctcttaatga 10740
ctttaacctt gtggctatga agtacaatta tgaacctcta acacaagacc atgttgacat 10800
actaggacct ctttctgctc aaactggaat tgccgtttta gatatgtgtg cttcattaaa 10860
agaattactg caaaatggta tgaatggacg taccatattg ggtagtgctt tattagaaga 10920
tgaatttaca ccttttgatg ttgttagaca atgctcaggt gttactttcc aaagtgcagt 10980
gaaaagaaca atcaagggta cacaccactg gttgttactc acaattttga cttcactttt 11040
agttttagtc cagagtactc aatggtcttt gttctttttt ttntatgaaa atgccttttt 11100
accttttgct atgggtatta ttgctatgtc tgcttttgca atgatgtttg tcaaacataa 11160
gcatgcattt ctctgtttgt ttttgttacc ttctcttgcc actgtagctt attttaatat 11220
ggtctatatg cctgctagtt gggtgatgcg tattatgaca tggttggata tggttgatac 11280
tagtttgtct ggttttaagc taaaagactg tgttatgtat gcatcagctg tagtgttact 11340
aatccttatg acagcaagaa ctgtgtatga tgatggtgct aggagagtgt ggacacttat 11400
gaatgtcttg acactcgttt ataaagttta ttatggtaat gctttagatc aagccatttc 11460
catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccaccta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140

gtttaagaat attgatggtt attttaaaat atattctaag cacacgccta ttaatttagt 22200

gcgtgatctc cctcagggtt tttcggcttt agaaccattg gtagatttgc caataggtat 22260

taacatcact aggtttcaaa ctttacttgc tttacataga agttatttga ctcctggtga 22320

ttcttcttca ggttggacag ctggtgctgc agcttattat gtgggttatc ttcaacctag 22380

gacttttcta ttaaaatata atgaaaatgg aaccattaca gatgctgtag actgtgcact 22440

tgaccctctc tcagaaacaa agtgtacgtt gaaatccttc actgtagaaa aaggaatcta 22500

tcaaacttct aactttagag tccaaccaac agaatctatt gttagatttc ctaatattac 22560

aaacttgtgc ccttttggtg aagtttttaa cgccaccaga tttgcatctg tttatgcttg 22620

gaacaggaag agaatcagca actgtgttgc tgattattct gtcctatata attccgcatc 22680

attttccact tttaagtgtt atggagtgtc tcctactaaa ttaaatgatc tctgctttac 22740

taatgtctat gcagattcat ttgtaattag aggtgatgaa gtcagacaaa tcgctccagg 22800

gcaaactgga aagattgctg attataatta taaattacca gatgatttta caggctgcgt 22860

tatagcttgg aattctaaca atcttgattc taaggttggt ggtaattata attacctgta 22920

tagattgttt aggaagtcta atctcaaacc ttttgagaga gatatttcaa ctgaaatcta 22980

tcaggccggt agcacacctt gtaatggtgt tgaaggtttt aattgttact ttcctttaca 23040

atcatatggt ttccaaccca ctaatggtgt tggttaccaa ccatacagag tagtagtact 23100

ttcttttgaa cttctacatg caccagcaac tgtttgtgga cctaaaaagt ctactaattt 23160

ggttaaaaac aaatgtgtca atttcaactt caatggttta acaggcacag gtgttcttac 23220

tgagtctaac aaaaagtttc tgcctttcca acaatttggc agagacattg ctgacactac 23280

tgatgctgtc cgtgatccac agacacttga gattcttgac attacaccat gttcttttgg 23340

tggtgtcagt gttataacac caggaacaaa tacttctaac caggttgctg ttctttatca 23400

ggatgttaac tgcacagaag tccctgttgc tattcatgca gatcaactta ctcctacttg 23460

gcgtgtttat tctacaggtt ctaatgtttt tcaaacacgt gcaggctgtt taataggggc 23520

tgaacatgtc aacaactcat atgagtgtga catacccatt ggtgcaggta tatgcgctag 23580

ttatcagact cagactaatt ctcctcggcg ggcacgtagt gtagctagtc aatccatcat 23640

tgcctacact atgtcacttg gtgcagaaaa ttcagttgct tactctaata actctattgc 23700

catacccaca aattttacta ttagtgttac cacagaaatt ctaccagtgt ctatgaccaa 23760

gacatcagta gattgtacaa tgtacatttg tggtgattca actgaatgca gcaatctttt 23820

gttgcaatat ggcagttttt gtacacaatt aaaccgtgct ttaactggaa tagctgttga 23880

acaagacaaa aacacccaag aagtttttgc acaagtcaaa caaatttaca aaacaccacc 23940

aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag cttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acgtttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagac aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaacgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta ggggaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagaat 29867

<210> 10
<211> 7062
<212> PRT
<213> SARS-CoV2

<220>
<221> misc_feature
<222> (3606)..(3606)
<223> Xaa can be any naturally occurring amino acid

<400> 10

Met Glu Ser Leu Val Pro Gly Phe Asn Glu Lys Thr His Val Gln Leu
1               5                   10                  15

Ser Leu Pro Val Leu Gln Val Arg Asp Val Leu Val Arg Gly Phe Gly
            20                  25                  30

Asp Ser Val Glu Glu Val Leu Ser Glu Ala Arg Gln His Leu Lys Asp
        35                  40                  45

Gly Thr Cys Gly Leu Val Glu Val Glu Lys Gly Val Leu Pro Gln Leu
    50                  55                  60

Glu Gln Pro Tyr Val Phe Ile Lys Arg Ser Asp Ala Arg Thr Ala Pro
65                  70                  75                  80

His Gly His Val Met Val Glu Leu Val Ala Glu Leu Glu Gly Ile Gln
                85                  90                  95

Tyr Gly Arg Ser Gly Glu Thr Leu Gly Val Leu Val Pro His Val Gly
            100                 105                 110

Glu Ile Pro Val Ala Tyr Arg Lys Val Leu Leu Arg Lys Asn Gly Asn
        115                 120                 125

Lys Gly Ala Gly Gly His Ser Tyr Gly Ala Asp Leu Lys Ser Phe Asp
    130                 135                 140

Leu Gly Asp Glu Leu Gly Thr Asp Pro Tyr Glu Asp Phe Gln Glu Asn
145                 150                 155                 160

Trp Asn Thr Lys His Ser Ser Gly Val Thr Arg Glu Leu Met Arg Glu
                165                 170                 175

Leu Asn Gly Gly Ala Tyr Thr Arg Tyr Val Asp Asn Asn Phe Cys Gly
            180                 185                 190

Pro Asp Gly Tyr Pro Leu Glu Cys Ile Lys Asp Leu Leu Ala Arg Ala
        195                 200                 205

Gly Lys Ala Ser Cys Thr Leu Ser Glu Gln Leu Asp Phe Ile Asp Thr
    210                 215                 220

Lys Arg Gly Val Tyr Cys Cys Arg Glu His Glu His Glu Ile Ala Trp
225                 230                 235                 240

Tyr Thr Glu Arg Ser Glu Lys Ser Tyr Glu Leu Gln Thr Pro Phe Glu
                245                 250                 255

Ile Lys Leu Ala Lys Lys Phe Asp Thr Phe Asn Gly Glu Cys Pro Asn
            260                 265                 270

Phe Val Phe Pro Leu Asn Ser Ile Ile Lys Thr Ile Gln Pro Arg Val
        275                 280                 285

Glu Lys Lys Lys Leu Asp Gly Phe Met Gly Arg Ile Arg Ser Val Tyr
    290                 295                 300

Pro Val Ala Ser Pro Asn Glu Cys Asn Gln Met Cys Leu Ser Thr Leu
305                 310                 315                 320

Met Lys Cys Asp His Cys Gly Glu Thr Ser Trp Gln Thr Gly Asp Phe
                325                 330                 335

Val Lys Ala Thr Cys Glu Phe Cys Gly Thr Glu Asn Leu Thr Lys Glu
            340                 345                 350

Gly Ala Thr Thr Cys Gly Tyr Leu Pro Gln Asn Ala Val Val Lys Ile
        355                 360                 365

Tyr Cys Pro Ala Cys His Asn Ser Glu Val Gly Pro Glu His Ser Leu
    370                 375                 380

Ala Glu Tyr His Asn Glu Ser Gly Leu Lys Thr Ile Leu Arg Lys Gly
385                 390                 395                 400

Gly Arg Thr Ile Ala Phe Gly Gly Cys Val Phe Ser Tyr Val Gly Cys
                405                 410                 415

His Asn Lys Cys Ala Tyr Trp Val Pro Arg Ala Ser Ala Asn Ile Gly
            420                 425                 430

Cys Asn His Thr Gly Val Val Gly Glu Gly Ser Glu Gly Leu Asn Asp
        435                 440                 445

Asn Leu Leu Glu Ile Leu Gln Lys Glu Lys Val Asn Ile Asn Ile Val
    450                 455                 460

Gly Asp Phe Lys Leu Asn Glu Glu Ile Ala Ile Ile Leu Ala Ser Phe
465                 470                 475                 480

Ser Ala Ser Thr Ser Ala Phe Val Glu Thr Val Lys Gly Leu Asp Tyr
                485                 490                 495

Lys Ala Phe Lys Gln Ile Val Glu Ser Cys Gly Asn Phe Lys Val Thr
            500                 505                 510

Lys Gly Lys Ala Lys Lys Gly Ala Trp Asn Ile Gly Glu Gln Lys Ser
        515                 520                 525

Ile Leu Ser Pro Leu Tyr Ala Phe Ala Ser Glu Ala Ala Arg Val Val
    530                 535                 540

Arg Ser Ile Phe Ser Arg Thr Leu Glu Thr Ala Gln Asn Ser Val Arg
545                 550                 555                 560

Val Leu Gln Lys Ala Ala Ile Thr Ile Leu Asp Gly Ile Ser Gln Tyr
                565                 570                 575

Ser Leu Arg Leu Ile Asp Ala Met Met Phe Thr Ser Asp Leu Ala Thr
            580                 585                 590

Asn Asn Leu Val Val Met Ala Tyr Ile Thr Gly Gly Val Val Gln Leu
        595                 600                 605

Thr Ser Gln Trp Leu Thr Asn Ile Phe Gly Thr Val Tyr Glu Lys Leu
    610                 615                 620

Lys Pro Val Leu Asp Trp Leu Glu Glu Lys Phe Lys Glu Gly Val Glu
625                 630                 635                 640

Phe Leu Arg Asp Gly Trp Glu Ile Val Lys Phe Ile Ser Thr Cys Ala
                645                 650                 655

Cys Glu Ile Val Gly Gly Gln Ile Val Thr Cys Ala Lys Glu Ile Lys
            660                 665                 670

Glu Ser Val Gln Thr Phe Phe Lys Leu Val Asn Lys Phe Leu Ala Leu
        675                 680                 685

Cys Ala Asp Ser Ile Ile Ile Gly Gly Ala Lys Leu Lys Ala Leu Asn
    690                 695                 700

Leu Gly Glu Thr Phe Val Thr His Ser Lys Gly Leu Tyr Arg Lys Cys
705                 710                 715                 720

Val Lys Ser Arg Glu Glu Thr Gly Leu Leu Met Pro Leu Lys Ala Pro
                725                 730                 735

Lys Glu Ile Ile Phe Leu Glu Gly Glu Thr Leu Pro Thr Glu Val Leu
            740                 745                 750

Thr Glu Glu Val Val Leu Lys Thr Gly Asp Leu Gln Pro Leu Glu Gln
        755                 760                 765

Pro Thr Ser Glu Ala Val Glu Ala Pro Leu Val Gly Thr Pro Val Cys

770                 775                 780

Ile Asn Gly Leu Met Leu Leu Glu Ile Lys Asp Thr Glu Lys Tyr Cys
785                 790                 795                 800

Ala Leu Ala Pro Asn Met Met Val Thr Asn Asn Thr Phe Thr Leu Lys
                805                 810                 815

Gly Gly Ala Pro Thr Lys Val Thr Phe Gly Asp Asp Thr Val Ile Glu
            820                 825                 830

Val Gln Gly Tyr Lys Ser Val Asn Ile Thr Phe Glu Leu Asp Glu Arg
        835                 840                 845

Ile Asp Lys Val Leu Asn Glu Lys Cys Ser Ala Tyr Thr Val Glu Leu
    850                 855                 860

Gly Thr Glu Val Asn Glu Phe Ala Cys Val Val Ala Asp Ala Val Ile
865                 870                 875                 880

Lys Thr Leu Gln Pro Val Ser Glu Leu Leu Thr Pro Leu Gly Ile Asp
                885                 890                 895

Leu Asp Glu Trp Ser Met Ala Thr Tyr Tyr Leu Phe Asp Glu Ser Gly
            900                 905                 910

Glu Phe Lys Leu Ala Ser His Met Tyr Cys Ser Phe Tyr Pro Pro Asp
        915                 920                 925

Glu Asp Glu Glu Glu Gly Asp Cys Glu Glu Glu Glu Phe Glu Pro Ser
    930                 935                 940

Thr Gln Tyr Glu Tyr Gly Thr Glu Asp Asp Tyr Gln Gly Lys Pro Leu
945                 950                 955                 960

Glu Phe Gly Ala Thr Ser Ala Ala Leu Gln Pro Glu Glu Glu Gln Glu
                965                 970                 975

Glu Asp Trp Leu Asp Asp Asp Ser Gln Gln Thr Val Gly Gln Gln Asp
            980                 985                 990

Gly Ser Glu Asp Asn Gln Thr Thr  Thr Ile Gln Thr Ile  Val Glu Val
        995                 1000                1005

Gln Pro  Gln Leu Glu Met Glu  Leu Thr Pro Val Val  Gln Thr Ile
    1010                1015                1020

Glu Val Asn Ser Phe Ser Gly Tyr Leu Lys Leu Thr Asp Asn Val
    1025                1030                1035

Tyr Ile Lys Asn Ala Asp Ile Val Glu Glu Ala Lys Lys Val Lys
    1040                1045                1050

Pro Thr Val Val Val Asn Ala Ala Asn Val Tyr Leu Lys His Gly
    1055                1060                1065

Gly Gly Val Ala Gly Ala Leu Asn Lys Ala Thr Asn Asn Ala Met
    1070                1075                1080

Gln Val Glu Ser Asp Asp Tyr Ile Ala Thr Asn Gly Pro Leu Lys
    1085                1090                1095

Val Gly Gly Ser Cys Val Leu Ser Gly His Asn Leu Ala Lys His
    1100                1105                1110

Cys Leu His Val Val Gly Pro Asn Val Asn Lys Gly Glu Asp Ile
    1115                1120                1125

Gln Leu Leu Lys Ser Ala Tyr Glu Asn Phe Asn Gln His Glu Val
    1130                1135                1140

Leu Leu Ala Pro Leu Leu Ser Ala Gly Ile Phe Gly Ala Asp Pro
    1145                1150                1155

Ile His Ser Leu Arg Val Cys Val Asp Thr Val Arg Thr Asn Val
    1160                1165                1170

Tyr Leu Ala Val Phe Asp Lys Asn Leu Tyr Asp Lys Leu Val Ser
    1175                1180                1185

Ser Phe Leu Glu Met Lys Ser Glu Lys Gln Val Glu Gln Lys Ile
    1190                1195                1200

Ala Glu Ile Pro Lys Glu Glu Val Lys Pro Phe Ile Thr Glu Ser
    1205                1210                1215

Lys Pro Ser Val Glu Gln Arg Lys Gln Asp Asp Lys Lys Ile Lys
    1220                1225                1230

Ala Cys Val Glu Glu Val Thr Thr Thr Leu Glu Glu Thr Lys Phe
    1235                1240                1245

Leu Thr Glu Asn Leu Leu Leu Tyr Ile Asp Ile Asn Gly Asn Leu
    1250                1255                1260

His Pro Asp Ser Ala Thr Leu Val Ser Asp Ile Asp Ile Thr Phe
    1265                1270                1275

Leu Lys Lys Asp Ala Pro Tyr Ile Val Gly Asp Val Val Gln Glu
    1280                1285                1290

Gly Val Leu Thr Ala Val Val Ile Pro Thr Lys Lys Ala Gly Gly
    1295                1300                1305

Thr Thr Glu Met Leu Ala Lys Ala Leu Arg Lys Val Pro Thr Asp
    1310                1315                1320

Asn Tyr Ile Thr Thr Tyr Pro Gly Gln Gly Leu Asn Gly Tyr Thr
    1325                1330                1335

Val Glu Glu Ala Lys Thr Val Leu Lys Lys Cys Lys Ser Ala Phe
    1340                1345                1350

Tyr Ile Leu Pro Ser Ile Ile Ser Asn Glu Lys Gln Glu Ile Leu
    1355                1360                1365

Gly Thr Val Ser Trp Asn Leu Arg Glu Met Leu Ala His Ala Glu
    1370                1375                1380

Glu Thr Arg Lys Leu Met Pro Val Cys Val Glu Thr Lys Ala Ile
    1385                1390                1395

Val Ser Thr Ile Gln Arg Lys Tyr Lys Gly Ile Lys Ile Gln Glu
    1400                1405                1410

Gly Val Val Asp Tyr Gly Ala Arg Phe Tyr Phe Tyr Thr Ser Lys
    1415                1420                1425

Thr Thr Val Ala Ser Leu Ile Asn Thr Leu Asn Asp Leu Asn Glu
    1430                1435                1440

Thr Leu Val Thr Met Pro Leu Gly Tyr Val Thr His Gly Leu Asn
    1445                1450                1455

Leu Glu Glu Ala Ala Arg Tyr Met Arg Ser Leu Lys Val Pro Ala
    1460                1465                1470

Thr Val Ser Val Ser Ser Pro Asp Ala Val Thr Ala Tyr Asn Gly
    1475                1480                1485

Tyr Leu Thr Ser Ser Ser Lys Thr Pro Glu Glu His Phe Ile Glu
    1490                1495                1500

Thr Ile Ser Leu Ala Gly Ser Tyr Lys Asp Trp Ser Tyr Ser Gly
    1505                1510                1515

Gln Ser Thr Gln Leu Gly Ile Glu Phe Leu Lys Arg Gly Asp Lys
    1520                1525                1530

Ser Val Tyr Tyr Thr Ser Asn Pro Thr Thr Phe His Leu Asp Gly
    1535                1540                1545

Glu Val Ile Thr Phe Asp Asn Leu Lys Thr Leu Leu Ser Leu Arg
    1550                1555                1560

Glu Val Arg Thr Ile Lys Val Phe Thr Thr Val Asp Asn Ile Asn
    1565                1570                1575

Leu His Thr Gln Val Val Asp Met Ser Met Thr Tyr Gly Gln Gln
    1580                1585                1590

Phe Gly Pro Thr Tyr Leu Asp Gly Ala Asp Val Thr Lys Ile Lys
    1595                1600                1605

Pro His Asn Ser His Glu Gly Lys Thr Phe Tyr Val Leu Pro Asn
    1610                1615                1620

Asp Asp Thr Leu Arg Val Glu Ala Phe Glu Tyr Tyr His Thr Thr
    1625                1630                1635

Asp Pro Ser Phe Leu Gly Arg Tyr Met Ser Ala Leu Asn His Thr
    1640                1645                1650

Lys Lys Trp Lys Tyr Pro Gln Val Asn Gly Leu Thr Ser Ile Lys
    1655                1660                1665

Trp Ala Asp Asn Asn Cys Tyr Leu Ala Thr Ala Leu Leu Thr Leu
    1670                1675                1680

Gln Gln Ile Glu Leu Lys Phe Asn Pro Pro Ala Leu Gln Asp Ala
    1685                1690                1695

Tyr Tyr Arg Ala Arg Ala Gly Glu Ala Ala Asn Phe Cys Ala Leu
    1700                1705                1710

Ile Leu Ala Tyr Cys Asn Lys Thr Val Gly Glu Leu Gly Asp Val
    1715                1720                1725

Arg Glu Thr Met Ser Tyr Leu Phe Gln His Ala Asn Leu Asp Ser

1730                1735                1740

Cys Lys Arg Val Leu Asn Val Val Cys Lys Thr Cys Gly Gln Gln
    1745                1750                1755

Gln Thr Thr Leu Lys Gly Val Glu Ala Val Met Tyr Met Gly Thr
    1760                1765                1770

Leu Ser Tyr Glu Gln Phe Lys Lys Gly Val Gln Ile Pro Cys Thr
    1775                1780                1785

Cys Gly Lys Gln Ala Thr Lys Tyr Leu Val Gln Gln Glu Ser Pro
    1790                1795                1800

Phe Val Met Met Ser Ala Pro Pro Ala Gln Tyr Glu Leu Lys His
    1805                1810                1815

Gly Thr Phe Thr Cys Ala Ser Glu Tyr Thr Gly Asn Tyr Gln Cys
    1820                1825                1830

Gly His Tyr Lys His Ile Thr Ser Lys Glu Thr Leu Tyr Cys Ile
    1835                1840                1845

Asp Gly Ala Leu Leu Thr Lys Ser Ser Glu Tyr Lys Gly Pro Ile
    1850                1855                1860

Thr Asp Val Phe Tyr Lys Glu Asn Ser Tyr Thr Thr Thr Ile Lys
    1865                1870                1875

Pro Val Thr Tyr Lys Leu Asp Gly Val Val Cys Thr Glu Ile Asp
    1880                1885                1890

Pro Lys Leu Asp Asn Tyr Tyr Lys Lys Asp Asn Ser Tyr Phe Thr
    1895                1900                1905

Glu Gln Pro Ile Asp Leu Val Pro Asn Gln Pro Tyr Pro Asn Ala
    1910                1915                1920

Ser Phe Asp Asn Phe Lys Phe Val Cys Asp Asn Ile Lys Phe Ala
    1925                1930                1935

Asp Asp Leu Asn Gln Leu Thr Gly Tyr Lys Lys Pro Ala Ser Arg
    1940                1945                1950

Glu Leu Lys Val Thr Phe Phe Pro Asp Leu Asn Gly Asp Val Val
    1955                1960                1965

Ala Ile Asp Tyr Lys His Tyr Thr Pro Ser Phe Lys Lys Gly Ala
    1970                1975                1980

Lys Leu Leu His Lys Pro Ile Val Trp His Val Asn Asn Ala Thr
    1985                1990                1995

Asn Lys Ala Thr Tyr Lys Pro Asn Thr Trp Cys Ile Arg Cys Leu
    2000                2005                2010

Trp Ser Thr Lys Pro Val Glu Thr Ser Asn Ser Phe Asp Val Leu
    2015                2020                2025

Lys Ser Glu Asp Ala Gln Gly Met Asp Asn Leu Ala Cys Glu Asp
    2030                2035                2040

Leu Lys Pro Val Ser Glu Glu Val Val Glu Asn Pro Thr Ile Gln
    2045                2050                2055

Lys Asp Val Leu Glu Cys Asn Val Lys Thr Thr Glu Val Val Gly
    2060                2065                2070

Asp Ile Ile Leu Lys Pro Ala Asn Asn Ser Leu Lys Ile Thr Glu
    2075                2080                2085

Glu Val Gly His Thr Asp Leu Met Ala Ala Tyr Val Asp Asn Ser
    2090                2095                2100

Ser Leu Thr Ile Lys Lys Pro Asn Glu Leu Ser Arg Val Leu Gly
    2105                2110                2115

Leu Lys Thr Leu Ala Thr His Gly Leu Ala Ala Val Asn Ser Val
    2120                2125                2130

Pro Trp Asp Thr Ile Ala Asn Tyr Ala Lys Pro Phe Leu Asn Lys
    2135                2140                2145

Val Val Ser Thr Thr Thr Asn Ile Val Thr Arg Cys Leu Asn Arg
    2150                2155                2160

Val Cys Thr Asn Tyr Met Pro Tyr Phe Phe Thr Leu Leu Leu Gln
    2165                2170                2175

Leu Cys Thr Phe Thr Arg Ser Thr Asn Ser Arg Ile Lys Ala Ser
    2180                2185                2190

Met Pro Thr Thr Ile Ala Lys Asn Thr Val Lys Ser Val Gly Lys
    2195                2200                2205

Phe Cys Leu Glu Ala Ser Phe Asn Tyr Leu Lys Ser Pro Asn Phe
    2210                2215                2220

Ser Lys Leu Ile Asn Ile Ile Ile Trp Phe Leu Leu Leu Ser Val
    2225                2230                2235

Cys Leu Gly Ser Leu Ile Tyr Ser Thr Ala Ala Leu Gly Val Leu
    2240                2245                2250

Met Ser Asn Leu Gly Met Pro Ser Tyr Cys Thr Gly Tyr Arg Glu
    2255                2260                2265

Gly Tyr Leu Asn Ser Thr Asn Val Thr Ile Ala Thr Tyr Cys Thr
    2270                2275                2280

Gly Ser Ile Pro Cys Ser Val Cys Leu Ser Gly Leu Asp Ser Leu
    2285                2290                2295

Asp Thr Tyr Pro Ser Leu Glu Thr Ile Gln Ile Thr Ile Ser Ser
    2300                2305                2310

Phe Lys Trp Asp Leu Thr Ala Phe Gly Leu Val Ala Glu Trp Phe
    2315                2320                2325

Leu Ala Tyr Ile Leu Phe Thr Arg Phe Phe Tyr Val Leu Gly Leu
    2330                2335                2340

Ala Ala Ile Met Gln Leu Phe Phe Ser Tyr Phe Ala Val His Phe
    2345                2350                2355

Ile Ser Asn Ser Trp Leu Met Trp Leu Ile Ile Asn Leu Val Gln
    2360                2365                2370

Met Ala Pro Ile Ser Ala Met Val Arg Met Tyr Ile Phe Phe Ala
    2375                2380                2385

Ser Phe Tyr Tyr Val Trp Lys Ser Tyr Val His Val Val Asp Gly
    2390                2395                2400

Cys Asn Ser Ser Thr Cys Met Met Cys Tyr Lys Arg Asn Arg Ala
    2405                2410                2415

Thr Arg Val Glu Cys Thr Thr Ile Val Asn Gly Val Arg Arg Ser
    2420                2425                2430

Phe Tyr Val Tyr Ala Asn Gly Gly Lys Gly Phe Cys Lys Leu His
    2435                2440                2445

Asn Trp Asn Cys Val Asn Cys Asp Thr Phe Cys Ala Gly Ser Thr
    2450                2455                2460

Phe Ile Ser Asp Glu Val Ala Arg Asp Leu Ser Leu Gln Phe Lys
    2465                2470                2475

Arg Pro Ile Asn Pro Thr Asp Gln Ser Ser Tyr Ile Val Asp Ser
    2480                2485                2490

Val Thr Val Lys Asn Gly Ser Ile His Leu Tyr Phe Asp Lys Ala
    2495                2500                2505

Gly Gln Lys Thr Tyr Glu Arg His Ser Leu Ser His Phe Val Asn
    2510                2515                2520

Leu Asp Asn Leu Arg Ala Asn Asn Thr Lys Gly Ser Leu Pro Ile
    2525                2530                2535

Asn Val Ile Val Phe Asp Gly Lys Ser Lys Cys Glu Glu Ser Ser
    2540                2545                2550

Ala Lys Ser Ala Ser Val Tyr Tyr Ser Gln Leu Met Cys Gln Pro
    2555                2560                2565

Ile Leu Leu Leu Asp Gln Ala Leu Val Ser Asp Val Gly Asp Ser
    2570                2575                2580

Ala Glu Val Ala Val Lys Met Phe Asp Ala Tyr Val Asn Thr Phe
    2585                2590                2595

Ser Ser Thr Phe Asn Val Pro Met Glu Lys Leu Lys Thr Leu Val
    2600                2605                2610

Ala Thr Ala Glu Ala Glu Leu Ala Lys Asn Val Ser Leu Asp Asn
    2615                2620                2625

Val Leu Ser Thr Phe Ile Ser Ala Ala Arg Gln Gly Phe Val Asp
    2630                2635                2640

Ser Asp Val Glu Thr Lys Asp Val Val Glu Cys Leu Lys Leu Ser
    2645                2650                2655

His Gln Ser Asp Ile Glu Val Thr Gly Asp Ser Cys Asn Asn Tyr
    2660                2665                2670

Met Leu Thr Tyr Asn Lys Val Glu Asn Met Thr Pro Arg Asp Leu

2675                2680                2685

Gly Ala Cys Ile Asp Cys Ser Ala Arg His Ile Asn Ala Gln Val
    2690                2695                2700

Ala Lys Ser His Asn Ile Ala Leu Ile Trp Asn Val Lys Asp Phe
    2705                2710                2715

Met Ser Leu Ser Glu Gln Leu Arg Lys Gln Ile Arg Ser Ala Ala
    2720                2725                2730

Lys Lys Asn Asn Leu Pro Phe Lys Leu Thr Cys Ala Thr Thr Arg
    2735                2740                2745

Gln Val Val Asn Val Val Thr Thr Lys Ile Ala Leu Lys Gly Gly
    2750                2755                2760

Lys Ile Val Asn Asn Trp Leu Lys Gln Leu Ile Lys Val Thr Leu
    2765                2770                2775

Val Phe Leu Phe Val Ala Ala Ile Phe Tyr Leu Ile Thr Pro Val
    2780                2785                2790

His Val Met Ser Lys His Thr Asp Phe Ser Ser Glu Ile Ile Gly
    2795                2800                2805

Tyr Lys Ala Ile Asp Gly Gly Val Thr Arg Asp Ile Ala Ser Thr
    2810                2815                2820

Asp Thr Cys Phe Ala Asn Lys His Ala Asp Phe Asp Thr Trp Phe
    2825                2830                2835

Ser Gln Arg Gly Gly Ser Tyr Thr Asn Asp Lys Ala Cys Pro Leu
    2840                2845                2850

Ile Ala Ala Val Ile Thr Arg Glu Val Gly Phe Val Val Pro Gly
    2855                2860                2865

Leu Pro Gly Thr Ile Leu Arg Thr Thr Asn Gly Asp Phe Leu His
    2870                2875                2880

Phe Leu Pro Arg Val Phe Ser Ala Val Gly Asn Ile Cys Tyr Thr
    2885                2890                2895

Pro Ser Lys Leu Ile Glu Tyr Thr Asp Phe Ala Thr Ser Ala Cys
    2900                2905                2910

Val Leu Ala Ala Glu Cys Thr Ile Phe Lys Asp Ala Ser Gly Lys
    2915                2920                2925

Pro Val Pro Tyr Cys Tyr Asp Thr Asn Val Leu Glu Gly Ser Val
    2930                2935                2940

Ala Tyr Glu Ser Leu Arg Pro Asp Thr Arg Tyr Val Leu Met Asp
    2945                2950                2955

Gly Ser Ile Ile Gln Phe Pro Asn Thr Tyr Leu Glu Gly Ser Val
    2960                2965                2970

Arg Val Val Thr Thr Phe Asp Ser Glu Tyr Cys Arg His Gly Thr
    2975                2980                2985

Cys Glu Arg Ser Glu Ala Gly Val Cys Val Ser Thr Ser Gly Arg
    2990                2995                3000

Trp Val Leu Asn Asn Asp Tyr Tyr Arg Ser Leu Pro Gly Val Phe
    3005                3010                3015

Cys Gly Val Asp Ala Val Asn Leu Leu Thr Asn Met Phe Thr Pro
    3020                3025                3030

Leu Ile Gln Pro Ile Gly Ala Leu Asp Ile Ser Ala Ser Ile Val
    3035                3040                3045

Ala Gly Gly Ile Val Ala Ile Val Val Thr Cys Leu Ala Tyr Tyr
    3050                3055                3060

Phe Met Arg Phe Arg Arg Ala Phe Gly Glu Tyr Ser His Val Val
    3065                3070                3075

Ala Phe Asn Thr Leu Leu Phe Leu Met Ser Phe Thr Val Leu Cys
    3080                3085                3090

Leu Thr Pro Val Tyr Ser Phe Leu Pro Gly Val Tyr Ser Val Ile
    3095                3100                3105

Tyr Leu Tyr Leu Thr Phe Tyr Leu Thr Asn Asp Val Ser Phe Leu
    3110                3115                3120

Ala His Ile Gln Trp Met Val Met Phe Thr Pro Leu Val Pro Phe
    3125                3130                3135

Trp Ile Thr Ile Ala Tyr Ile Ile Cys Ile Ser Thr Lys His Phe
    3140                3145                3150

Tyr Trp Phe Phe Ser Asn Tyr Leu Lys Arg Arg Val Val Phe Asn
    3155                3160                3165

Gly Val Ser Phe Ser Thr Phe Glu Glu Ala Ala Leu Cys Thr Phe
    3170                3175                3180

Leu Leu Asn Lys Glu Met Tyr Leu Lys Leu Arg Ser Asp Val Leu
    3185                3190                3195

Leu Pro Leu Thr Gln Tyr Asn Arg Tyr Leu Ala Leu Tyr Asn Lys
    3200                3205                3210

Tyr Lys Tyr Phe Ser Gly Ala Met Asp Thr Thr Ser Tyr Arg Glu
    3215                3220                3225

Ala Ala Cys Cys His Leu Ala Lys Ala Leu Asn Asp Phe Ser Asn
    3230                3235                3240

Ser Gly Ser Asp Val Leu Tyr Gln Pro Pro Gln Thr Ser Ile Thr
    3245                3250                3255

Ser Ala Val Leu Gln Ser Gly Phe Arg Lys Met Ala Phe Pro Ser
    3260                3265                3270

Gly Lys Val Glu Gly Cys Met Val Gln Val Thr Cys Gly Thr Thr
    3275                3280                3285

Thr Leu Asn Gly Leu Trp Leu Asp Asp Val Val Tyr Cys Pro Arg
    3290                3295                3300

His Val Ile Cys Thr Ser Glu Asp Met Leu Asn Pro Asn Tyr Glu
    3305                3310                3315

Asp Leu Leu Ile Arg Lys Ser Asn His Asn Phe Leu Val Gln Ala
    3320                3325                3330

Gly Asn Val Gln Leu Arg Val Ile Gly His Ser Met Gln Asn Cys
    3335                3340                3345

Val Leu Lys Leu Lys Val Asp Thr Ala Asn Pro Lys Thr Pro Lys
    3350                3355                3360

Tyr Lys Phe Val Arg Ile Gln Pro Gly Gln Thr Phe Ser Val Leu
    3365                3370                3375

Ala Cys Tyr Asn Gly Ser Pro Ser Gly Val Tyr Gln Cys Ala Met
    3380                3385                3390

Arg Pro Asn Phe Thr Ile Lys Gly Ser Phe Leu Asn Gly Ser Cys
    3395                3400                3405

Gly Ser Val Gly Phe Asn Ile Asp Tyr Asp Cys Val Ser Phe Cys
    3410                3415                3420

Tyr Met His His Met Glu Leu Pro Thr Gly Val His Ala Gly Thr
    3425                3430                3435

Asp Leu Glu Gly Asn Phe Tyr Gly Pro Phe Val Asp Arg Gln Thr
    3440                3445                3450

Ala Gln Ala Ala Gly Thr Asp Thr Thr Ile Thr Val Asn Val Leu
    3455                3460                3465

Ala Trp Leu Tyr Ala Ala Val Ile Asn Gly Asp Arg Trp Phe Leu
    3470                3475                3480

Asn Arg Phe Thr Thr Thr Leu Asn Asp Phe Asn Leu Val Ala Met
    3485                3490                3495

Lys Tyr Asn Tyr Glu Pro Leu Thr Gln Asp His Val Asp Ile Leu
    3500                3505                3510

Gly Pro Leu Ser Ala Gln Thr Gly Ile Ala Val Leu Asp Met Cys
    3515                3520                3525

Ala Ser Leu Lys Glu Leu Leu Gln Asn Gly Met Asn Gly Arg Thr
    3530                3535                3540

Ile Leu Gly Ser Ala Leu Leu Glu Asp Glu Phe Thr Pro Phe Asp
    3545                3550                3555

Val Val Arg Gln Cys Ser Gly Val Thr Phe Gln Ser Ala Val Lys
    3560                3565                3570

Arg Thr Ile Lys Gly Thr His His Trp Leu Leu Leu Thr Ile Leu
    3575                3580                3585

Thr Ser Leu Leu Val Leu Val Gln Ser Thr Gln Trp Ser Leu Phe
    3590                3595                3600

Phe Phe Xaa Tyr Glu Asn Ala Phe Leu Pro Phe Ala Met Gly Ile
    3605                3610                3615

Ile Ala Met Ser Ala Phe Ala Met Met Phe Val Lys His Lys His

3620                3625                3630

Ala Phe Leu Cys Leu Phe Leu Leu Pro Ser Leu Ala Thr Val Ala
    3635                3640                3645

Tyr Phe Asn Met Val Tyr Met Pro Ala Ser Trp Val Met Arg Ile
    3650                3655                3660

Met Thr Trp Leu Asp Met Val Asp Thr Ser Leu Ser Gly Phe Lys
    3665                3670                3675

Leu Lys Asp Cys Val Met Tyr Ala Ser Ala Val Val Leu Leu Ile
    3680                3685                3690

Leu Met Thr Ala Arg Thr Val Tyr Asp Asp Gly Ala Arg Arg Val
    3695                3700                3705

Trp Thr Leu Met Asn Val Leu Thr Leu Val Tyr Lys Val Tyr Tyr
    3710                3715                3720

Gly Asn Ala Leu Asp Gln Ala Ile Ser Met Trp Ala Leu Ile Ile
    3725                3730                3735

Ser Val Thr Ser Asn Tyr Ser Gly Val Val Thr Thr Val Met Phe
    3740                3745                3750

Leu Ala Arg Gly Ile Val Phe Met Cys Val Glu Tyr Cys Pro Ile
    3755                3760                3765

Phe Phe Ile Thr Gly Asn Thr Leu Gln Cys Ile Met Leu Val Tyr
    3770                3775                3780

Cys Phe Leu Gly Tyr Phe Cys Thr Cys Tyr Phe Gly Leu Phe Cys
    3785                3790                3795

Leu Leu Asn Arg Tyr Phe Arg Leu Thr Leu Gly Val Tyr Asp Tyr
    3800                3805                3810

Leu Val Ser Thr Gln Glu Phe Arg Tyr Met Asn Ser Gln Gly Leu
    3815                3820                3825

Leu Pro Pro Lys Asn Ser Ile Asp Ala Phe Lys Leu Asn Ile Lys
    3830                3835                3840

Leu Leu Gly Val Gly Gly Lys Pro Cys Ile Lys Val Ala Thr Val
    3845                3850                3855

Gln Ser Lys Met Ser Asp Val Lys Cys Thr Ser Val Val Leu Leu
    3860                3865                3870

Ser Val Leu Gln Gln Leu Arg Val Glu Ser Ser Ser Lys Leu Trp
    3875                3880                3885

Ala Gln Cys Val Gln Leu His Asn Asp Ile Leu Leu Ala Lys Asp
    3890                3895                3900

Thr Thr Glu Ala Phe Glu Lys Met Val Ser Leu Leu Ser Val Leu
    3905                3910                3915

Leu Ser Met Gln Gly Ala Val Asp Ile Asn Lys Leu Cys Glu Glu
    3920                3925                3930

Met Leu Asp Asn Arg Ala Thr Leu Gln Ala Ile Ala Ser Glu Phe
    3935                3940                3945

Ser Ser Leu Pro Ser Tyr Ala Ala Phe Ala Thr Ala Gln Glu Ala
    3950                3955                3960

Tyr Glu Gln Ala Val Ala Asn Gly Asp Ser Glu Val Val Leu Lys
    3965                3970                3975

Lys Leu Lys Lys Ser Leu Asn Val Ala Lys Ser Glu Phe Asp Arg
    3980                3985                3990

Asp Ala Ala Met Gln Arg Lys Leu Glu Lys Met Ala Asp Gln Ala
    3995                4000                4005

Met Thr Gln Met Tyr Lys Gln Ala Arg Ser Glu Asp Lys Arg Ala
    4010                4015                4020

Lys Val Thr Ser Ala Met Gln Thr Met Leu Phe Thr Met Leu Arg
    4025                4030                4035

Lys Leu Asp Asn Asp Ala Leu Asn Asn Ile Ile Asn Asn Ala Arg
    4040                4045                4050

Asp Gly Cys Val Pro Leu Asn Ile Ile Pro Leu Thr Thr Ala Ala
    4055                4060                4065

Lys Leu Met Val Val Ile Pro Asp Tyr Asn Thr Tyr Lys Asn Thr
    4070                4075                4080

Cys Asp Gly Thr Thr Phe Thr Tyr Ala Ser Ala Leu Trp Glu Ile
    4085                4090                4095

Gln Gln Val Val Asp Ala Asp Ser Lys Ile Val Gln Leu Ser Glu
    4100                4105                4110

Ile Ser Met Asp Asn Ser Pro Asn Leu Ala Trp Pro Leu Ile Val
    4115                4120                4125

Thr Ala Leu Arg Ala Asn Ser Ala Val Lys Leu Gln Asn Asn Glu
    4130                4135                4140

Leu Ser Pro Val Ala Leu Arg Gln Met Ser Cys Ala Ala Gly Thr
    4145                4150                4155

Thr Gln Thr Ala Cys Thr Asp Asp Asn Ala Leu Ala Tyr Tyr Asn
    4160                4165                4170

Thr Thr Lys Gly Gly Arg Phe Val Leu Ala Leu Leu Ser Asp Leu
    4175                4180                4185

Gln Asp Leu Lys Trp Ala Arg Phe Pro Lys Ser Asp Gly Thr Gly
    4190                4195                4200

Thr Ile Tyr Thr Glu Leu Glu Pro Pro Cys Arg Phe Val Thr Asp
    4205                4210                4215

Thr Pro Lys Gly Pro Lys Val Lys Tyr Leu Tyr Phe Ile Lys Gly
    4220                4225                4230

Leu Asn Asn Leu Asn Arg Gly Met Val Leu Gly Ser Leu Ala Ala
    4235                4240                4245

Thr Val Arg Leu Gln Ala Gly Asn Ala Thr Glu Val Pro Ala Asn
    4250                4255                4260

Ser Thr Val Leu Ser Phe Cys Ala Phe Ala Val Asp Ala Ala Lys
    4265                4270                4275

Ala Tyr Lys Asp Tyr Leu Ala Ser Gly Gly Gln Pro Ile Thr Asn
    4280                4285                4290

Cys Val Lys Met Leu Cys Thr His Thr Gly Thr Gly Gln Ala Ile
    4295                4300                4305

Thr Val Thr Pro Glu Ala Asn Met Asp Gln Glu Ser Phe Gly Gly
    4310                4315                4320

Ala Ser Cys Cys Leu Tyr Cys Arg Cys His Ile Asp His Pro Asn
    4325                4330                4335

Pro Lys Gly Phe Cys Asp Leu Lys Gly Lys Tyr Val Gln Ile Pro
    4340                4345                4350

Thr Thr Cys Ala Asn Asp Pro Val Gly Phe Thr Leu Lys Asn Thr
    4355                4360                4365

Val Cys Thr Val Cys Gly Met Trp Lys Gly Tyr Gly Cys Ser Cys
    4370                4375                4380

Asp Gln Leu Arg Glu Pro Met Leu Gln Ser Ala Asp Ala Gln Ser
    4385                4390                4395

Phe Leu Asn Arg Val Cys Gly Val Ser Ala Ala Arg Leu Thr Pro
    4400                4405                4410

Cys Gly Thr Gly Thr Ser Thr Asp Val Val Tyr Arg Ala Phe Asp
    4415                4420                4425

Ile Tyr Asn Asp Lys Val Ala Gly Phe Ala Lys Phe Leu Lys Thr
    4430                4435                4440

Asn Cys Cys Arg Phe Gln Glu Lys Asp Glu Asp Asp Asn Leu Ile
    4445                4450                4455

Asp Ser Tyr Phe Val Val Lys Arg His Thr Phe Ser Asn Tyr Gln
    4460                4465                4470

His Glu Glu Thr Ile Tyr Asn Leu Leu Lys Asp Cys Pro Ala Val
    4475                4480                4485

Ala Lys His Asp Phe Phe Lys Phe Arg Ile Asp Gly Asp Met Val
    4490                4495                4500

Pro His Ile Ser Arg Gln Arg Leu Thr Lys Tyr Thr Met Ala Asp
    4505                4510                4515

Leu Val Tyr Ala Leu Arg His Phe Asp Glu Gly Asn Cys Asp Thr
    4520                4525                4530

Leu Lys Glu Ile Leu Val Thr Tyr Asn Cys Cys Asp Asp Tyr
    4535                4540                4545

Phe Asn Lys Lys Asp Trp Tyr Asp Phe Val Glu Asn Pro Asp Ile
    4550                4555                4560

Leu Arg Val Tyr Ala Asn Leu Gly Glu Arg Val Arg Gln Ala Leu

4565                    4570                    4575

Leu Lys  Thr Val Gln Phe Cys  Asp Ala Met Arg Asn  Ala Gly Ile
    4580                    4585                    4590

Val Gly  Val Leu Thr Leu Asp  Asn Gln Asp Leu Asn  Gly Asn Trp
    4595                    4600                    4605

Tyr Asp  Phe Gly Asp Phe Ile  Gln Thr Thr Pro Gly  Ser Gly Val
    4610                    4615                    4620

Pro Val  Val Asp Ser Tyr Tyr  Ser Leu Leu Met Pro  Ile Leu Thr
    4625                    4630                    4635

Leu Thr  Arg Ala Leu Thr Ala  Glu Ser His Val Asp  Thr Asp Leu
    4640                    4645                    4650

Thr Lys  Pro Tyr Ile Lys Trp  Asp Leu Leu Lys Tyr  Asp Phe Thr
    4655                    4660                    4665

Glu Glu  Arg Leu Lys Leu Phe  Asp Arg Tyr Phe Lys  Tyr Trp Asp
    4670                    4675                    4680

Gln Thr  Tyr His Pro Asn Cys  Val Asn Cys Leu Asp  Asp Arg Cys
    4685                    4690                    4695

Ile Leu  His Cys Ala Asn Phe  Asn Val Leu Phe Ser  Thr Val Phe
    4700                    4705                    4710

Pro Pro  Thr Ser Phe Gly Pro  Leu Val Arg Lys Ile  Phe Val Asp
    4715                    4720                    4725

Gly Val  Pro Phe Val Val Ser  Thr Gly Tyr His Phe  Arg Glu Leu
    4730                    4735                    4740

Gly Val  Val His Asn Gln Asp  Val Asn Leu His Ser  Ser Arg Leu
    4745                    4750                    4755

Ser Phe  Lys Glu Leu Leu Val  Tyr Ala Ala Asp Pro  Ala Met His
    4760                    4765                    4770

Ala Ala  Ser Gly Asn Leu Leu  Leu Asp Lys Arg Thr  Thr Cys Phe
    4775                    4780                    4785

Ser Val  Ala Ala Leu Thr Asn  Asn Val Ala Phe Gln  Thr Val Lys
    4790                    4795                    4800

Pro Gly Asn Phe Asn Lys Asp Phe Tyr Asp Phe Ala Val Ser Lys
    4805                4810                4815

Gly Phe Phe Lys Glu Gly Ser Ser Val Glu Leu Lys His Phe Phe
    4820                4825                4830

Phe Ala Gln Asp Gly Asn Ala Ala Ile Ser Asp Tyr Asp Tyr Tyr
    4835                4840                4845

Arg Tyr Asn Leu Pro Thr Met Cys Asp Ile Arg Gln Leu Leu Phe
    4850                4855                4860

Val Val Glu Val Val Asp Lys Tyr Phe Asp Cys Tyr Asp Gly Gly
    4865                4870                4875

Cys Ile Asn Ala Asn Gln Val Ile Val Asn Asn Leu Asp Lys Ser
    4880                4885                4890

Ala Gly Phe Pro Phe Asn Lys Trp Gly Lys Ala Arg Leu Tyr Tyr
    4895                4900                4905

Asp Ser Met Ser Tyr Glu Asp Gln Asp Ala Leu Phe Ala Tyr Thr
    4910                4915                4920

Lys Arg Asn Val Ile Pro Thr Ile Thr Gln Met Asn Leu Lys Tyr
    4925                4930                4935

Ala Ile Ser Ala Lys Asn Arg Ala Arg Thr Val Ala Gly Val Ser
    4940                4945                4950

Ile Cys Ser Thr Met Thr Asn Arg Gln Phe His Gln Lys Leu Leu
    4955                4960                4965

Lys Ser Ile Ala Ala Thr Arg Gly Ala Thr Val Val Ile Gly Thr
    4970                4975                4980

Ser Lys Phe Tyr Gly Gly Trp His Asn Met Leu Lys Thr Val Tyr
    4985                4990                4995

Ser Asp Val Glu Asn Pro His Leu Met Gly Trp Asp Tyr Pro Lys
    5000                5005                5010

Cys Asp Arg Ala Met Pro Asn Met Leu Arg Ile Met Ala Ser Leu
    5015                5020                5025

Val Leu Ala Arg Lys His Thr Thr Cys Cys Ser Leu Ser His Arg
    5030                5035                5040

Phe Tyr Arg Leu Ala Asn Glu Cys Ala Gln Val Leu Ser Glu Met
    5045                5050                5055

Val Met Cys Gly Gly Ser Leu Tyr Val Lys Pro Gly Gly Thr Ser
    5060                5065                5070

Ser Gly Asp Ala Thr Thr Ala Tyr Ala Asn Ser Val Phe Asn Ile
    5075                5080                5085

Cys Gln Ala Val Thr Ala Asn Val Asn Ala Leu Leu Ser Thr Asp
    5090                5095                5100

Gly Asn Lys Ile Ala Asp Lys Tyr Val Arg Asn Leu Gln His Arg
    5105                5110                5115

Leu Tyr Glu Cys Leu Tyr Arg Asn Arg Asp Val Asp Thr Asp Phe
    5120                5125                5130

Val Asn Glu Phe Tyr Ala Tyr Leu Arg Lys His Phe Ser Met Met
    5135                5140                5145

Ile Leu Ser Asp Asp Ala Val Val Cys Phe Asn Ser Thr Tyr Ala
    5150                5155                5160

Ser Gln Gly Leu Val Ala Ser Ile Lys Asn Phe Lys Ser Val Leu
    5165                5170                5175

Tyr Tyr Gln Asn Asn Val Phe Met Ser Glu Ala Lys Cys Trp Thr
    5180                5185                5190

Glu Thr Asp Leu Thr Lys Gly Pro His Glu Phe Cys Ser Gln His
    5195                5200                5205

Thr Met Leu Val Lys Gln Gly Asp Asp Tyr Val Tyr Leu Pro Tyr
    5210                5215                5220

Pro Asp Pro Ser Arg Ile Leu Gly Ala Gly Cys Phe Val Asp Asp
    5225                5230                5235

Ile Val Lys Thr Asp Gly Thr Leu Met Ile Glu Arg Phe Val Ser
    5240                5245                5250

Leu Ala Ile Asp Ala Tyr Pro Leu Thr Lys His Pro Asn Gln Glu
    5255                5260                5265

Tyr Ala Asp Val Phe His Leu Tyr Leu Gln Tyr Ile Arg Lys Leu
    5270                5275                5280

His Asp Glu Leu Thr Gly His Met Leu Asp Met Tyr Ser Val Met
    5285                5290                5295

Leu Thr Asn Asp Asn Thr Ser Arg Tyr Trp Glu Pro Glu Phe Tyr
    5300                5305                5310

Glu Ala Met Tyr Thr Pro His Thr Val Leu Gln Ala Val Gly Ala
    5315                5320                5325

Cys Val Leu Cys Asn Ser Gln Thr Ser Leu Arg Cys Gly Ala Cys
    5330                5335                5340

Ile Arg Arg Pro Phe Leu Cys Cys Lys Cys Cys Tyr Asp His Val
    5345                5350                5355

Ile Ser Thr Ser His Lys Leu Val Leu Ser Val Asn Pro Tyr Val
    5360                5365                5370

Cys Asn Ala Pro Gly Cys Asp Val Thr Asp Val Thr Gln Leu Tyr
    5375                5380                5385

Leu Gly Gly Met Ser Tyr Tyr Cys Lys Ser His Lys Pro Pro Ile
    5390                5395                5400

Ser Phe Pro Leu Cys Ala Asn Gly Gln Val Phe Gly Leu Tyr Lys
    5405                5410                5415

Asn Thr Cys Val Gly Ser Asp Asn Val Thr Asp Phe Asn Ala Ile
    5420                5425                5430

Ala Thr Cys Asp Trp Thr Asn Ala Gly Asp Tyr Ile Leu Ala Asn
    5435                5440                5445

Thr Cys Thr Glu Arg Leu Lys Leu Phe Ala Ala Glu Thr Leu Lys
    5450                5455                5460

Ala Thr Glu Glu Thr Phe Lys Leu Ser Tyr Gly Ile Ala Thr Val
    5465                5470                5475

Arg Glu Val Leu Ser Asp Arg Glu Leu His Leu Ser Trp Glu Val
    5480                5485                5490

Gly Lys Pro Arg Pro Pro Leu Asn Arg Asn Tyr Val Phe Thr Gly
    5495                5500                5505

Tyr Arg Val Thr Lys Asn Ser Lys Val Gln Ile Gly Glu Tyr Thr

5510                5515                5520

Phe Glu Lys Gly Asp Tyr Gly Asp Ala Val Val Tyr Arg Gly Thr
    5525                5530                5535

Thr Thr Tyr Lys Leu Asn Val Gly Asp Tyr Phe Val Leu Thr Ser
    5540                5545                5550

His Thr Val Met Pro Leu Ser Ala Pro Thr Leu Val Pro Gln Glu
    5555                5560                5565

His Tyr Val Arg Ile Thr Gly Leu Tyr Pro Thr Leu Asn Ile Ser
    5570                5575                5580

Asp Glu Phe Ser Ser Asn Val Ala Asn Tyr Gln Lys Val Gly Met
    5585                5590                5595

Gln Lys Tyr Ser Thr Leu Gln Gly Pro Pro Gly Thr Gly Lys Ser
    5600                5605                5610

His Phe Ala Ile Gly Leu Ala Leu Tyr Tyr Pro Ser Ala Arg Ile
    5615                5620                5625

Val Tyr Thr Ala Cys Ser His Ala Ala Val Asp Ala Leu Cys Glu
    5630                5635                5640

Lys Ala Leu Lys Tyr Leu Pro Ile Asp Lys Cys Ser Arg Ile Ile
    5645                5650                5655

Pro Ala Arg Ala Arg Val Glu Cys Phe Asp Lys Phe Lys Val Asn
    5660                5665                5670

Ser Thr Leu Glu Gln Tyr Val Phe Cys Thr Val Asn Ala Leu Pro
    5675                5680                5685

Glu Thr Thr Ala Asp Ile Val Val Phe Asp Glu Ile Ser Met Ala
    5690                5695                5700

Thr Asn Tyr Asp Leu Ser Val Val Asn Ala Arg Leu Arg Ala Lys
    5705                5710                5715

His Tyr Val Tyr Ile Gly Asp Pro Ala Gln Leu Pro Ala Pro Arg
    5720                5725                5730

Thr Leu Leu Thr Lys Gly Thr Leu Glu Pro Glu Tyr Phe Asn Ser
    5735                5740                5745

Val Cys Arg Leu Met Lys Thr Ile Gly Pro Asp Met Phe Leu Gly
    5750                5755                5760

Thr Cys Arg Arg Cys Pro Ala Glu Ile Val Asp Thr Val Ser Ala
    5765                5770                5775

Leu Val Tyr Asp Asn Lys Leu Lys Ala His Lys Asp Lys Ser Ala
    5780                5785                5790

Gln Cys Phe Lys Met Phe Tyr Lys Gly Val Ile Thr His Asp Val
    5795                5800                5805

Ser Ser Ala Ile Asn Arg Pro Gln Ile Gly Val Val Arg Glu Phe
    5810                5815                5820

Leu Thr Arg Asn Pro Ala Trp Arg Lys Ala Val Phe Ile Ser Pro
    5825                5830                5835

Tyr Asn Ser Gln Asn Ala Val Ala Ser Lys Ile Leu Gly Leu Pro
    5840                5845                5850

Thr Gln Thr Val Asp Ser Ser Gln Gly Ser Glu Tyr Asp Tyr Val
    5855                5860                5865

Ile Phe Thr Gln Thr Thr Glu Thr Ala His Ser Cys Asn Val Asn
    5870                5875                5880

Arg Phe Asn Val Ala Ile Thr Arg Ala Lys Val Gly Ile Leu Cys
    5885                5890                5895

Ile Met Ser Asp Arg Asp Leu Tyr Asp Lys Leu Gln Phe Thr Ser
    5900                5905                5910

Leu Glu Ile Pro Arg Arg Asn Val Ala Thr Leu Gln Ala Glu Asn
    5915                5920                5925

Val Thr Gly Leu Phe Lys Asp Cys Ser Lys Val Ile Thr Gly Leu
    5930                5935                5940

His Pro Thr Gln Ala Pro Thr His Leu Ser Val Asp Thr Lys Phe
    5945                5950                5955

Lys Thr Glu Gly Leu Cys Val Asp Ile Pro Gly Ile Pro Lys Asp
    5960                5965                5970

Met Thr Tyr Arg Arg Leu Ile Ser Met Met Gly Phe Lys Met Asn
    5975                5980                5985

Tyr Gln Val Asn Gly Tyr Pro Asn Met Phe Ile Thr Arg Glu Glu
        5990                5995                6000

Ala Ile Arg His Val Arg Ala Trp Ile Gly Phe Asp Val Glu Gly
        6005                6010                6015

Cys His Ala Thr Arg Glu Ala Val Gly Thr Asn Leu Pro Leu Gln
        6020                6025                6030

Leu Gly Phe Ser Thr Gly Val Asn Leu Val Ala Val Pro Thr Gly
        6035                6040                6045

Tyr Val Asp Thr Pro Asn Asn Thr Asp Phe Ser Arg Val Ser Ala
        6050                6055                6060

Lys Pro Pro Pro Gly Asp Gln Phe Lys His Leu Ile Pro Leu Met
        6065                6070                6075

Tyr Lys Gly Leu Pro Trp Asn Val Val Arg Ile Lys Ile Val Gln
        6080                6085                6090

Met Leu Ser Asp Thr Leu Lys Asn Leu Ser Asp Arg Val Val Phe
        6095                6100                6105

Val Leu Trp Ala His Gly Phe Glu Leu Thr Ser Met Lys Tyr Phe
        6110                6115                6120

Val Lys Ile Gly Pro Glu Arg Thr Cys Cys Leu Cys Asp Arg Arg
        6125                6130                6135

Ala Thr Cys Phe Ser Thr Ala Ser Asp Thr Tyr Ala Cys Trp His
        6140                6145                6150

His Ser Ile Gly Phe Asp Tyr Val Tyr Asn Pro Phe Met Ile Asp
        6155                6160                6165

Val Gln Gln Trp Gly Phe Thr Gly Asn Leu Gln Ser Asn His Asp
        6170                6175                6180

Leu Tyr Cys Gln Val His Gly Asn Ala His Val Ala Ser Cys Asp
        6185                6190                6195

Ala Ile Met Thr Arg Cys Leu Ala Val His Glu Cys Phe Val Lys
        6200                6205                6210

Arg Val Asp Trp Thr Ile Glu Tyr Pro Ile Ile Gly Asp Glu Leu
        6215                6220                6225

Lys Ile Asn Ala Ala Cys Arg Lys Val Gln His Met Val Val Lys
        6230                6235                6240

Ala Ala Leu Leu Ala Asp Lys Phe Pro Val Leu His Asp Ile Gly
        6245                6250                6255

Asn Pro Lys Ala Ile Lys Cys Val Pro Gln Ala Asp Val Glu Trp
        6260                6265                6270

Lys Phe Tyr Asp Ala Gln Pro Cys Ser Asp Lys Ala Tyr Lys Ile
        6275                6280                6285

Glu Glu Leu Phe Tyr Ser Tyr Ala Thr His Ser Asp Lys Phe Thr
        6290                6295                6300

Asp Gly Val Cys Leu Phe Trp Asn Cys Asn Val Asp Arg Tyr Pro
        6305                6310                6315

Ala Asn Ser Ile Val Cys Arg Phe Asp Thr Arg Val Leu Ser Asn
        6320                6325                6330

Leu Asn Leu Pro Gly Cys Asp Gly Gly Ser Leu Tyr Val Asn Lys
        6335                6340                6345

His Ala Phe His Thr Pro Ala Phe Asp Lys Ser Ala Phe Val Asn
        6350                6355                6360

Leu Lys Gln Leu Pro Phe Phe Tyr Tyr Ser Asp Ser Pro Cys Glu
        6365                6370                6375

Ser His Gly Lys Gln Val Val Ser Asp Ile Asp Tyr Val Pro Leu
        6380                6385                6390

Lys Ser Ala Thr Cys Ile Thr Arg Cys Asn Leu Gly Gly Ala Val
        6395                6400                6405

Cys Arg His His Ala Asn Glu Tyr Arg Leu Tyr Leu Asp Ala Tyr
        6410                6415                6420

Asn Met Met Ile Ser Ala Gly Phe Ser Leu Trp Val Tyr Lys Gln
        6425                6430                6435

Phe Asp Thr Tyr Asn Leu Trp Asn Thr Phe Thr Arg Leu Gln Ser
        6440                6445                6450

Leu Glu Asn Val Ala Phe Asn Val Val Asn Lys Gly His Phe Asp

6455                6460                6465

Gly Gln Gln Gly Glu Val Pro Val Ser Ile Ile Asn Asn Thr Val
    6470                6475                6480

Tyr Thr Lys Val Asp Gly Val Asp Val Glu Leu Phe Glu Asn Lys
    6485                6490                6495

Thr Thr Leu Pro Val Asn Val Ala Phe Glu Leu Trp Ala Lys Arg
    6500                6505                6510

Asn Ile Lys Pro Val Pro Glu Val Lys Ile Leu Asn Asn Leu Gly
    6515                6520                6525

Val Asp Ile Ala Ala Asn Thr Val Ile Trp Asp Tyr Lys Arg Asp
    6530                6535                6540

Ala Pro Ala His Ile Ser Thr Ile Gly Val Cys Ser Met Thr Asp
    6545                6550                6555

Ile Ala Lys Lys Pro Thr Glu Thr Ile Cys Ala Pro Leu Thr Val
    6560                6565                6570

Phe Phe Asp Gly Arg Val Asp Gly Gln Val Asp Leu Phe Arg Asn
    6575                6580                6585

Ala Arg Asn Gly Val Leu Ile Thr Glu Gly Ser Val Lys Gly Leu
    6590                6595                6600

Gln Pro Ser Val Gly Pro Lys Gln Ala Ser Leu Asn Gly Val Thr
    6605                6610                6615

Leu Ile Gly Glu Ala Val Lys Thr Gln Phe Asn Tyr Tyr Lys Lys
    6620                6625                6630

Val Asp Gly Val Val Gln Gln Leu Pro Glu Thr Tyr Phe Thr Gln
    6635                6640                6645

Ser Arg Asn Leu Gln Glu Phe Lys Pro Arg Ser Gln Met Glu Ile
    6650                6655                6660

Asp Phe Leu Glu Leu Ala Met Asp Glu Phe Ile Glu Arg Tyr Lys
    6665                6670                6675

Leu Glu Gly Tyr Ala Phe Glu His Ile Val Tyr Gly Asp Phe Ser
    6680                6685                6690

His Ser Gln Leu Gly Gly Leu His Leu Leu Ile Gly Leu Ala Lys
        6695                6700                6705

Arg Phe Lys Glu Ser Pro Phe Glu Leu Glu Asp Phe Ile Pro Met
        6710                6715                6720

Asp Ser Thr Val Lys Asn Tyr Phe Ile Thr Asp Ala Gln Thr Gly
        6725                6730                6735

Ser Ser Lys Cys Val Cys Ser Val Ile Asp Leu Leu Leu Asp Asp
        6740                6745                6750

Phe Val Glu Ile Ile Lys Ser Gln Asp Leu Ser Val Val Ser Lys
        6755                6760                6765

Val Val Lys Val Thr Ile Asp Tyr Thr Glu Ile Ser Phe Met Leu
        6770                6775                6780

Trp Cys Lys Asp Gly His Val Glu Thr Phe Tyr Pro Lys Leu Gln
        6785                6790                6795

Ser Ser Gln Ala Trp Gln Pro Gly Val Ala Met Pro Asn Leu Tyr
        6800                6805                6810

Lys Met Gln Arg Met Leu Leu Glu Lys Cys Asp Leu Gln Asn Tyr
        6815                6820                6825

Gly Asp Ser Ala Thr Leu Pro Lys Gly Ile Met Met Asn Val Ala
        6830                6835                6840

Lys Tyr Thr Gln Leu Cys Gln Tyr Leu Asn Thr Leu Thr Leu Ala
        6845                6850                6855

Val Pro Tyr Asn Met Arg Val Ile His Phe Gly Ala Gly Ser Asp
        6860                6865                6870

Lys Gly Val Ala Pro Gly Thr Ala Val Leu Arg Gln Trp Leu Pro
        6875                6880                6885

Thr Gly Thr Leu Leu Val Asp Ser Asp Leu Asn Asp Phe Val Ser
        6890                6895                6900

Asp Ala Asp Ser Thr Leu Ile Gly Asp Cys Ala Thr Val His Thr
        6905                6910                6915

Ala Asn Lys Trp Asp Leu Ile Ile Ser Asp Met Tyr Asp Pro Lys
        6920                6925                6930

Thr Lys Asn Val Thr Lys Glu Asn Asp Ser Lys Glu Gly Phe Phe
        6935                6940                6945

Thr Tyr Ile Cys Gly Phe Ile Gln Gln Lys Leu Ala Leu Gly Gly
        6950                6955                6960

Ser Val Ala Ile Lys Ile Thr Glu His Ser Trp Asn Ala Asp Leu
        6965                6970                6975

Tyr Lys Leu Met Gly His Phe Ala Trp Trp Thr Ala Phe Val Thr
        6980                6985                6990

Asn Val Asn Ala Ser Ser Ser Glu Ala Phe Leu Ile Gly Cys Asn
        6995                7000                7005

Tyr Leu Gly Lys Pro Arg Glu Gln Ile Asp Gly Tyr Val Met His
        7010                7015                7020

Ala Asn Tyr Ile Phe Trp Arg Asn Thr Asn Pro Ile Gln Leu Ser
        7025                7030                7035

Ser Tyr Ser Leu Phe Asp Met Ser Lys Phe Pro Leu Lys Leu Arg
        7040                7045                7050

Gly Thr Ala Val Met Ser Leu Lys Glu
        7055                7060

<210> 11
<211> 1273
<212> PRT
<213> SARS-CoV2

<400> 11

<210>  12
<211>  29874
<212>  DNA
<213>  SARS-CoV2

<400>  12
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc 180

ttctgcaggc tgcttacggt ttcgtccgtg ttgcagccga tcatcagcac atctaggttt 240

cgtccgggtg tgaccgaaag gtaagatgga gagccttgtc cctggtttca acgagaaaac 300

acacgtccaa ctcagtttgc ctgttttaca ggttcgcgac gtgctcgtac gtggctttgg 360

agactccgtg gaggaggtct tatcagaggc acgtcaacat cttaaagatg gcacttgtgg 420

cttagtagaa gttgaaaaag gcgttttgcc tcaacttgaa cagccctatg tgttcatcaa 480

acgttcggat gctcgaactg cacctcatgg tcatgttatg gttgagctgg tagcagaact 540

cgaaggcatt cagtacggtc gtagtggtga gacacttggt gtccttgtcc ctcatgtggg 600

cgaaatacca gtggcttacc gcaaggttct tcttcgtaag aacggtaata aaggagctgg 660

tggccatagt tacggcgccg atctaaagtc atttgactta ggcgacgagc ttggcactga 720

tccttatgaa gattttcaag aaaactggaa cactaaacat agcagtggtg ttacccgtga 780

actcatgcgt gagcttaacg gaggggcata cactcgctat gtcgataaca acttctgtgg 840

ccctgatggc taccctcttg agtgcattaa agaccttcta gcacgtgctg gtaaagcttc 900

atgcactttg tccgaacaac tggactttat tgacactaag aggggtgtat actgctgccg 960

tgaacatgag catgaaattg cttggtacac ggaacgttct gaaaagagct atgaattgca 1020

gacacctttt gaaattaaat tggcaaagaa atttgacacc ttcaatgggg aatgtccaaa 1080

ttttgtattt cccttaaatt ccataatcaa gactattcaa ccaagggttg aaaagaaaaa 1140

gcttgatggc tttatgggta gaattcgatc tgtctatcca gttgcgtcac caaatgaatg 1200

caaccaaatg tgcctttcaa ctctcatgaa gtgtgatcat tgtggtgaaa cttcatggca 1260

gacgggcgat tttgttaaag ccacttgcga attttgtggc actgagaatt tgactaaaga 1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 2940

actgggcatt gatttagatg agtggagtat ggctacatac tacttatttg atgagtctgg 3000

tgagtttaaa ttggcttcac atatgtattg ttctttctac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

taaggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttgttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacctcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgtct ggttttaagc taaaagactg tgttatgtat gcatcagctg tagtgttact 11340

aatccttatg acagcaagaa ctgtgtatga tgatggtgct aggagagtgt ggacacttat 11400

gaatgtcttg acactcgttt ataaagttta ttatggtaat gctttagatc aagccatttc 11460

catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccaccta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140

gtttaagaat attgatggtt attttaaaat atattctaag cacacgccta ttaatttagt 22200

gcgtgatctc cctcagggtt tttcggcttt agaaccattg gtagatttgc caataggtat 22260

taacatcact aggtttcaaa ctttacttgc tttacataga agttatttga ctcctggtga 22320

ttcttcttca ggttggacag ctggtgctgc agcttattat gtgggttatc ttcaacctag 22380

gacttttcta ttaaaatata atgaaaatgg aaccattaca gatgctgtag actgtgcact 22440

tgaccctctc tcagaaacaa agtgtacgtt gaaatccttc actgtagaaa aaggaatcta 22500

tcaaacttct aactttagag tccaaccaac agaatctatt gttagatttc ctaatattac 22560

aaacttgtgc ccttttggtg aagtttttaa cgccaccaga tttgcatctg tttatgcttg 22620

gaacaggaag agaatcagca actgtgttgc tgattattct ttcctatata attccgcatc 22680

attttccact tttaagtgtt atggagtgtc tcctactaaa ttaaatgatc tctgctttac 22740

taatgtctat gcagattcat ttgtaattag aggtgatgaa gtcagacaaa tcgctccagg 22800

gcaaactgga aagattgctg attataatta taaattacca gatgatttta caggctgcgt 22860

tatagcttgg aattctaaca atcttgattc taaggttggt ggtaattata attacctgta 22920

tagattgttt aggaagtcta atctcaaacc ttttgagaga gatatttcaa ctgaaatcta 22980

tcaggccggt agcacacctt gtaatggtgt tgaaggtttt aattgttact ttcctttaca 23040

atcatatggt ttccaaccca ctaatggtgt tggttaccaa ccatacagag tagtagtact 23100

ttcttttgaa cttctacatg caccagcaac tgtttgtgga cctaaaaagt ctactaattt 23160

ggttaaaaac aaatgtgtca atttcaactt caatggttta acaggcacag gtgttcttac 23220

tgagtctaac aaaaagtttc tgcctttcca acaatttggc agagacattg ctgacactac 23280

tgatgctgtc cgtgatccac agacacttga gattcttgac attacaccat gttcttttgg 23340

tggtgtcagt gttataacac caggaacaaa tacttctaac caggttgctg ttctttatca 23400

ggatgttaac tgcacagaag tccctgttgc tattcatgca gatcaactta ctcctacttg 23460

gcgtgtttat tctacaggtt ctaatgtttt tcaaacacgt gcaggctgtt taataggggc 23520

tgaacatgtc aacaactcat atgagtgtga catacccatt ggtgcaggta tatgcgctag 23580

ttatcagact cagactaatt ctcctcggcg ggcacgtagt gtagctagtc aatccatcat 23640

tgcctacact atgtcacttg gtgcagaaaa ttcagttgct tactctaata actctattgc 23700

catacccaca aattttacta ttagtgttac cacagaaatt ctaccagtgt ctatgaccaa 23760

gacatcagta gattgtacaa tgtacatttg tggtgattca actgaatgca gcaatctttt 23820

gttgcaatat ggcagttttt gtacacaatt aaaccgtgct ttaactggaa tagctgttga 23880

acaagacaaa aacacccaag aagtttttgc acaagtcaaa caaatttaca aaacaccacc 23940

aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag cttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acgtttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagac aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaacgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta ggggaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagaatgac aaaa 29874

<210> 13
<211> 4405
<212> PRT
<213> SARS-CoV2

<400> 13

<210> 14
<211> 1273
<212> PRT
<213> SARS-CoV2

<400> 14

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Phe Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly

                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Thr Lys Met Ser  Glu Cys Val Leu Gly  Gln Ser Lys
    1025                1030                1035

Arg Val  Asp Phe Cys Gly Lys  Gly Tyr His Leu Met  Ser Phe Pro
    1040                1045                1050

Gln Ser  Ala Pro His Gly Val  Val Phe Leu His Val  Thr Tyr Val
    1055                1060                1065

Pro Ala  Gln Glu Lys Asn Phe  Thr Thr Ala Pro Ala  Ile Cys His
    1070                1075                1080

Asp Gly  Lys Ala His Phe Pro  Arg Glu Gly Val Phe  Val Ser Asn
    1085                1090                1095

Gly Thr  His Trp Phe Val Thr  Gln Arg Asn Phe Tyr  Glu Pro Gln
    1100                1105                1110

Ile Ile  Thr Thr Asp Asn Thr  Phe Val Ser Gly Asn  Cys Asp Val
    1115                1120                1125

Val Ile  Gly Ile Val Asn Asn  Thr Val Tyr Asp Pro  Leu Gln Pro
    1130                1135                1140

Glu Leu  Asp Ser Phe Lys Glu  Glu Leu Asp Lys Tyr  Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210> 15
<211> 29885
<212> DNA
<213> SARS-CoV2

<220>
<221> misc_feature
<222> (1)..(30)
<223> n is a, c, g, or t

<220>
<221> misc_feature
<222> (29865)..(29865)
<223> n is a, c, g, or t

<220>
<221> misc_feature
<222> (29872)..(29885)
<223> n is a, c, g, or t

<400> 15
nnnnnnnnnn nnnnnnnnnn nnnnnnnnnn accaaccaac tttcgatctc ttgtagatct 60

gttctctaaa cgaactttaa aatctgtgtg gctgtcactc ggctgcatgc ttagtgcact 120

cacgcagtat aattaataac taattactgt cgttgacagg acacgagtaa ctcgtctatc 180

ttctgcaggc tgcttacggt ttcgtccgtg ttgcagccga tcatcagcac atctaggttt 240

tgtccgggtg tgaccgaaag gtaagatgga gagccttgtc cctggtttca acgagaaaac 300

acacgtccaa ctcagtttgc ctgttttaca ggttcgcgac gtgctcgtac gtggctttgg 360

agactccgtg gaggaggtct tatcagaggc acgtcaacat cttaaagatg gcacttgtgg 420

cttagtagaa gttgaaaaag gcgttttgcc tcaacttgaa cagccctatg tgttcatcaa 480

acgttcggat gctcgaactg cacctcatgg tcatgttatg gttgagctgg tagcagaact 540

cgaaggcatt cagtacggtc gtagtggtga gacacttggt gtccttgtcc ctcatgtggg 600

cgaaatacca gtggcttacc gcaaggttct tcttcgtaag aacggtaata aaggagctgg 660

tggccatagt tacggcgccg atctaaagtc atttgactta ggcgacgagc ttggcactga 720

tccttatgaa gattttcaag aaaactggaa cactaaacat agcagtggtg ttacccgtga 780

actcatgcgt gagcttaacg gaggggcata cactcgctat gtcgataaca acttctgtgg 840

ccctgatggc taccctcttg agtgcattaa agaccttcta gcacgtgctg gtaaagcttc 900

atgcactttg tccgaacaac tggactttat tgacactaag aggggtgtat actgctgccg 960

tgaacatgag catgaaattg cttggtacac ggaacgttct gaaaagagct atgaattgca 1020

gacacctttt gaaattaaat tggcaaagaa atttgacacc ttcaatgggg aatgtccaaa 1080

ttttgtattt cccttaaatt ccataatcaa gactattcaa ccaagggttg aaaagaaaaa 1140

gcttgatggc tttatgggta gaattcgatc tgtctatcca gttgcgtcac caaatgaatg 1200

caaccaaatg tgcctttcaa ctctcatgaa gtgtgatcat tgtggtgaaa cttcatggca 1260

gacgggcgat tttgttaaag ccacttgcga attttgtggc actgagaatt tgactaaaga 1320

aggtgccact acttgtggtt acttacccca aaatgctgtt gttaaaattt attgtccagc 1380

atgtcacaat tcagaagtag gacctgagca tagtcttgcc gaataccata atgaatctgg 1440

cttgaaaacc attcttcgta agggtggtcg cactattgcc tttggaggct gtgtgttctc 1500

ttatgttggt tgccataaca agtgtgccta ttgggttcca cgtgctagcg ctaacatagg 1560

ttgtaaccat acaggtgttg ttggagaagg ttccgaaggt cttaatgaca accttcttga 1620

aatactccaa aaagagaaag tcaacatcaa tattgttggt gactttaaac ttaatgaaga 1680

gatcgccatt attttggcat ctttttctgc ttccacaagt gcttttgtgg aaactgtgaa 1740

aggtttggat tataaagcat tcaaacaaat tgttgaatcc tgtggtaatt ttaaagttac 1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa caaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 2940

actgggcatt gatttagatg agtggagtat ggctacatac tacttatttg atgagtctgg 3000

tgagtttaaa ttggcttcac atatgtattg ttctttttac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gctagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaag tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttataaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatctac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct attttctatt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttacttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

taaggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttgttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacctcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgtct ggttttaagc taaaagactg tgttatgtat gcatcagctg tagtgttact 11340

aatccttatg acagcaagaa ctgtgtatga tgatggtgct aggagagtgt ggacacttat 11400

gaatgtcttg acactcgttt ataaagttta ttatggtaat gctttagatc aagccatttc 11460

catgtgggct cttataatct ctgttacttc taactactca ggtgtagtta caactgtcat 11520

gtttttggcc agaggtattg tttttatgtg tgttgagtat tgccctattt tcttcataac 11580

tggtaataca cttcagtgta taatgctagt ttattgtttc ttaggctatt tttgtacttg 11640

ttactttggc ctcttttgtt tactcaaccg ctactttaga ctgactcttg gtgtttatga 11700

ttacttagtt tctacacagg agtttagata tatgaattca cagggactac tcccacccaa 11760

gaatagcata gatgccttca aactcaacat taaattgttg ggtgttggtg gcaaaccttg 11820

tatcaaagta gccactgtac agtctaaaat gtcagatgta aagtgcacat cagtagtctt 11880

actctcagtt ttgcaacaac tcagagtaga atcatcatct aaattgtggg ctcaatgtgt 11940

ccagttacac aatgacattc tcttagctaa agatactact gaagcctttg aaaaaatggt 12000

ttcactactt tctgttttgc tttccatgca gggtgctgta gacataaaca agctttgtga 12060

agaaatgctg gacaacaggg caaccttaca agctatagcc tcagagttta gttcccttcc 12120

atcatatgca gcttttgcta ctgctcaaga agcttatgag caggctgttg ctaatggtga 12180

ttctgaagtt gttcttaaaa agttgaagaa gtctttgaat gtggctaaat ctgaatttga 12240

ccgtgatgca gccatgcaac gtaagttgga aaagatggct gatcaagcta tgacccaaat 12300

gtataaacag gctagatctg aggacaagag ggcaaaagtt actagtgcta tgcagacaat 12360

gcttttcact atgcttagaa agttggataa tgatgcactc aacaacatta tcaacaatgc 12420

aagagatggt tgtgttccct tgaacataat acctcttaca acagcagcca aactaatggt 12480

tgtcatacca gactataaca catataaaaa tacgtgtgat ggtacaacat ttacttatgc 12540

atcagcattg tgggaaatcc aacaggttgt agatgcagat agtaaaattg ttcaacttag 12600

tgaaattagt atggacaatt cacctaattt agcatggcct cttattgtaa cagctttaag 12660

ggccaattct gctgtcaaat tacagaataa tgagcttagt cctgttgcac tacgacagat 12720

gtcttgtgct gccggtacta cacaaactgc ttgcactgat gacaatgcgt tagcttacta 12780

caacacaaca aagggaggta ggtttgtact tgcactgtta tccgatttac aggatttgaa 12840

atgggctaga ttccctaaga gtgatggaac tggtactatc tatacagaac tggaaccacc 12900

ttgtaggttt gttacagaca cacctaaagg tcctaaagtg aagtatttat actttattaa 12960

aggattaaac aacctaaata gaggtatggt acttggtagt ttagctgcca cagtacgtct 13020

acaagctggt aatgcaacag aagtgcctgc caattcaact gtattatctt tctgtgcttt 13080

tgctgtagat gctgctaaag cttacaaaga ttatctagct agtgggggac aaccaatcac 13140

taattgtgtt aagatgttgt gtacacacac tggtactggt caggcaataa cagttacacc 13200

ggaagccaat atggatcaag aatcctttgg tggtgcatcg tgttgtctgt actgccgttg 13260

ccacatagat catccaaatc ctaaaggatt ttgtgactta aaaggtaagt atgtacaaat 13320

acctacaact tgtgctaatg accctgtggg ttttacactt aaaaacacag tctgtaccgt 13380

ctgcggtatg tggaaaggtt atggctgtag ttgtgatcaa ctccgcgaac ccatgcttca 13440

gtcagctgat gcacaatcgt ttttaaacgg gtttgcggtg taagtgcagc ccgtcttaca 13500

ccgtgcggca caggcactag tactgatgtc gtatacaggg cttttgacat ctacaatgat 13560

aaagtagctg gttttgctaa attcctaaaa actaattgtt gtcgcttcca agaaaaggac 13620

gaagatgaca atttaattga ttcttacttt gtagttaaga gacacacttt ctctaactac 13680

caacatgaag aaacaattta taatttactt aaggattgtc cagctgttgc taaacatgac 13740

ttctttaagt ttagaataga cggtgacatg gtaccacata tatcacgtca acgtcttact 13800

aaatacacaa tggcagacct cgtctatgct ttaaggcatt ttgatgaagg taattgtgac 13860

acattaaaag aaatacttgt cacatacaat tgttgtgatg atgattattt caataaaaag 13920

gactggtatg attttgtaga aaacccagat atattacgcg tatacgccaa cttaggtgaa 13980

cgtgtacgcc aagctttgtt aaaaacagta caattctgtg atgccatgcg aaatgctggt 14040

attgttggtg tactgacatt agataatcaa gatctcaatg gtaactggta tgatttcggt 14100

gatttcatac aaaccacgcc aggtagtgga gttcctgttg tagattctta ttattcattg 14160

ttaatgccta tattaacctt gaccagggct ttaactgcag agtcacatgt tgacactgac 14220

ttaacaaagc cttacattaa gtgggatttg ttaaaatatg acttcacgga agagaggtta 14280

aaactctttg accgttattt taaatattgg gatcagacat accacccaaa ttgtgttaac 14340

tgtttggatg acagatgcat tctgcattgt gcaaacttta atgttttatt ctctacagtg 14400

ttcccactta caagttttgg accactagtg agaaaaatat ttgttgatgg tgttccattt 14460

gtagtttcaa ctggatacca cttcagagag ctaggtgttg tacataatca ggatgtaaac 14520

ttacatagct ctagacttag ttttaaggaa ttacttgtgt atgctgctga ccctgctatg 14580

cacgctgctt ctggtaatct attactagat aaacgcacta cgtgcttttc agtagctgca 14640

cttactaaca atgttgcttt tcaaactgtc aaacccggta attttaacaa agacttctat 14700

gactttgctg tgtctaaggg tttctttaag gaaggaagtt ctgttgaatt aaaacacttc 14760

ttctttgctc aggatggtaa tgctgctatc agcgattatg actactatcg ttataatcta 14820

ccaacaatgt gtgatatcag acaactacta tttgtagttg aagttgttga taagtacttt 14880

gattgttacg atggtggctg tattaatgct aaccaagtca tcgtcaacaa cctagacaaa 14940

tcagctggtt ttccatttaa taaatggggt aaggctagac tttattatga ttcaatgagt 15000

tatgaggatc aagatgcact tttcgcatat acaaaacgta atgtcatccc tactataact 15060

caaatgaatc ttaagtatgc cattagtgca aagaatagag ctcgcaccgt agctggtgtc 15120

tctatctgta gtactatgac caatagacag tttcatcaaa aattattgaa atcaatagcc 15180

gccactagag gagctactgt agtaattgga acaagcaaat tctatggtgg ttggcacaac 15240

atgttaaaaa ctgtttatag tgatgtagaa aaccctcacc ttatgggttg ggattatcct 15300

aaatgtgata gagccatgcc taacatgctt agaattatgg cctcacttgt tcttgctcgc 15360

aaacatacaa cgtgttgtag cttgtcacac cgtttctata gattagctaa tgagtgtgct 15420

caagtattga gtgaaatggt catgtgtggc ggttcactat atgttaaacc aggtggaacc 15480

tcatcaggag atgccacaac tgcttatgct aatagtgttt ttaacatttg tcaagctgtc 15540

acggccaatg ttaatgcact tttatctact gatggtaaca aaattgccga taagtatgtc 15600

cgcaatttac aacacagact ttatgagtgt ctctatagaa atagagatgt tgacacagac 15660

tttgtgaatg agttttacgc atatttgcgt aaacatttct caatgatgat actctctgac 15720

gatgctgttg tgtgtttcaa tagcacttat gcatctcaag gtctagtggc tagcataaag 15780

aactttaagt cagttcttta ttatcaaaac aatgttttta tgtctgaagc aaaatgttgg 15840

actgagactg accttactaa aggacctcat gaattttgct ctcaacatac aatgctagtt 15900

aaacagggtg atgattatgt gtaccttcct tacccagatc catcaagaat cctaggggcc 15960

ggctgttttg tagatgatat cgtaaaaaca gatggtacac ttatgattga acggttcgtg 16020

tctttagcta tagatgctta cccacttact aaacatccta atcaggagta tgctgatgtc 16080

tttcatttgt acttacaata cataagaaag ctacatgatg agttaacagg acacatgtta 16140

gacatgtatt ctgttatgct tactaatgat aacacttcaa ggtattggga acctgagttt 16200

tatgaggcta tgtacacacc gcatacagtc ttacaggctg ttggggcttg tgttctttgc 16260

aattcacaga cttcattaag atgtggtgct tgcatacgta gaccattctt atgttgtaaa 16320

tgctgttacg accatgtcat atcaacatca cataaattag tcttgtctgt taatccgtat 16380

gtttgcaatg ctccaggttg tgatgtcaca gatgtgactc aactttactt aggaggtatg 16440

agctattatt gtaaatcaca taaaccaccc attagttttc cattgtgtgc taatggacaa 16500

gtttttggtt tatataaaaa tacatgtgtt ggtagcgata atgttactga ctttaatgca 16560

attgcaacat gtgactggac aaatgctggt gattacattt tagctaacac ctgtactgaa 16620

agactcaagc tttttgcagc agaaacgctc aaagctactg aggagacatt taaactgtct 16680

tatggtattg ctactgtacg tgaagtgctg tctgacagag aattacatct ttcatgggaa 16740

gttggtaaac ctagaccacc acttaaccga aattatgtct ttactggtta tcgtgtaact 16800

aaaaacagta aagtacaaat aggagagtac acctttgaaa aaggtgacta tggtgatgct 16860

gttgtttacc gaggtacaac aacttacaaa ttaaatgttg gtgattattt tgtgctgaca 16920

tcacatacag taatgccatt aagtgcacct acactagtgc cacaagagca ctatgttaga 16980

attactggct tatacccaac actcaatatc tcagatgagt tttctagcaa tgttgcaaat 17040

tatcaaaagg ttggtatgca aaagtattct acactccagg gaccacctgg tactggtaag 17100

agtcattttg ctattggcct agctctctac tacccttctg ctcgcatagt gtatacagct 17160

tgctctcatg ccgctgttga tgcactatgt gagaaggcat taaaatattt gcctatagat 17220

aaatgtagta gaattatacc tgcacgtgct cgtgtagagt gttttgataa attcaaagtg 17280

aattcaacat tagaacagta tgtcttttgt actgtaaatg cattgcctga gacgacagca 17340

gatatagttg tctttgatga aatttcaatg gccacaaatt atgatttgag tgttgtcaat 17400

gccagattac gtgctaagca ctatgtgtac attggcgacc ctgctcaatt acctgcacca 17460

cgcacattgc taactaaggg cacactagaa ccagaatatt tcaattcagt gtgtagactt 17520

atgaaaacta taggtccaga catgttcctc ggaacttgtc ggcgttgtcc tgctgaaatt 17580

gttgacactg tgagtgcttt ggtttatgat aataagctta aagcacataa agacaaatca 17640

gctcaatgct ttaaaatgtt ttataagggt gttatcacgc atgatgtttc atctgcaatt 17700

aacaggccac aaataggcgt ggtaagagaa ttccttacac gtaaccctgc ttggagaaaa 17760

gctgtcttta tttcacctta taattcacag aatgctgtag cctcaaagat tttgggacta 17820

ccaactcaaa ctgttgattc atcacagggc tcagaatatg actatgtcat attcactcaa 17880

accactgaaa cagctcactc ttgtaatgta aacagattta atgttgctat taccagagca 17940

aaagtaggca tactttgcat aatgtctgat agagaccttt atgacaagtt gcaatttaca 18000

agtcttgaaa ttccacgtag gaatgtggca actttacaag ctgaaaatgt aacaggactc 18060

tttaaagatt gtagtaaggt aatcactggg ttacatccta cacaggcacc tacacacctc 18120

agtgttgaca ctaaattcaa aactgaaggt ttatgtgttg acatacctgg catacctaag 18180

gacatgacct atagaagact catctctatg atgggtttta aaatgaatta tcaagttaat 18240

ggttacccta acatgtttat cacccgcgaa gaagctataa gacatgtacg tgcatggatt 18300

ggcttcgatg tcgaggggtg tcatgctact agagaagctg ttggtaccaa tttaccttta 18360

cagctaggtt tttctacagg tgttaaccta gttgctgtac ctacaggtta tgttgataca 18420

cctaataata cagatttttc cagagttagt gctaaaccac cgcctggaga tcaatttaaa 18480

cacctcatac cacttatgta caaaggactt ccttggaatg tagtgcgtat aaagattgta 18540

caaatgttaa gtgacacact taaaaatctc tctgacagag tcgtatttgt cttatgggca 18600

catggctttg agttgacatc tatgaagtat tttgtgaaaa taggacctga gcgcacctgt 18660

tgtctatgtg atagacgtgc cacatgcttt tccactgctt cagacactta tgcctgttgg 18720

catcattcta ttggatttga ttacgtctat aatccgttta tgattgatgt tcaacaatgg 18780

ggttttacag gtaacctaca aagcaaccat gatctgtatt gtcaagtcca tggtaatgca 18840

catgtagcta gttgtgatgc aatcatgact aggtgtctag ctgtccacga gtgctttgtt 18900

aagcgtgttg actggactat tgaatatcct ataattggtg atgaactgaa gattaatgcg 18960

gcttgtagaa aggttcaaca catggttgtt aaagctgcat tattagcaga caaattccca 19020

gttcttcacg acattggtaa ccctaaagct attaagtgtg tacctcaagc tgatgtagaa 19080

tggaagttct atgatgcaca gccttgtagt gacaaagctt ataaaataga agaattattc 19140

tattcttatg ccacacattc tgacaaattc acagatggtg tatgcctatt ttggaattgc 19200

aatgtcgata gatatcctgc taattccatt gtttgtagat ttgacactag agtgctatct 19260

aaccttaact tgcctggttg tgatggtggc agtttgtatg taaataaaca tgcattccac 19320

acaccagctt ttgataaaag tgcttttgtt aatttaaaac aattaccatt tttctattac 19380

tctgacagtc catgtgagtc tcatggaaaa caagtagtgt cagatataga ttatgtacca 19440

ctaaagtctg ctacgtgtat aacacgttgc aatttaggtg gtgctgtctg tagacatcat 19500

gctaatgagt acagattgta tctcgatgct tataacatga tgatctcagc tggctttagc 19560

ttgtgggttt acaaacaatt tgatacttat aacctctgga acacttttac aagacttcag 19620

agtttagaaa atgtggcttt taatgttgta aataagggac actttgatgg acaacagggt 19680

gaagtaccag tttctatcat taataacact gtttacacaa aagttgatgg tgttgatgta 19740

gaattgtttg aaaataaaac aacattacct gttaatgtag catttgagct ttgggctaag 19800

cgcaacatta aaccagtacc agaggtgaaa atactcaata atttgggtgt ggacattgct 19860

gctaatactg tgatctggga ctacaaaaga gatgctccag cacatatatc tactattggt 19920

gtttgttcta tgactgacat agccaagaaa ccaactgaaa cgatttgtgc accactcact 19980

gtcttttttg atggtagagt tgatggtcaa gtagacttat ttagaaatgc ccgtaatggt 20040

gttcttatta cagaaggtag tgttaaaggt ttacaaccat ctgtaggtcc caaacaagct 20100

agtcttaatg gagtcacatt aattggagaa gccgtaaaaa cacagttcaa ttattataag 20160

aaagttgatg gtgttgtcca acaattacct gaaacttact ttactcagag tagaaattta 20220

caagaattta aacccaggag tcaaatggaa attgatttct tagaattagc tatggatgaa 20280

ttcattgaac ggtataaatt agaaggctat gccttcgaac atatcgttta tggagatttt 20340

agtcatagtc agttaggtgg tttacatcta ctgattggac tagctaaacg ttttaaggaa 20400

tcaccttttg aattagaaga ttttattcct atggacagta cagttaaaaa ctatttcata 20460

acagatgcgc aaacaggttc atctaagtgt gtgtgttctg ttattgattt attacttgat 20520

gattttgttg aaataataaa atcccaagat ttatctgtag tttctaaggt tgtcaaagtg 20580

actattgact atacagaaat ttcatttatg ctttggtgta aagatggcca tgtagaaaca 20640

ttttacccaa aattacaatc tagtcaagcg tggcaaccgg gtgttgctat gcctaatctt 20700

tacaaaatgc aaagaatgct attagaaaag tgtgaccttc aaaattatgg tgatagtgca 20760

acattaccta aaggcataat gatgaatgtc gcaaaatata ctcaactgtg tcaatattta 20820

aacacattaa cattagctgt accctataat atgagagtta tacattttgg tgctggttct 20880

gataaaggag ttgcaccagg tacagctgtt ttaagacagt ggttgcctac gggtacgctg 20940

cttgtcgatt cagatcttaa tgactttgtc tctgatgcag attcaacttt gattggtgat 21000

tgtgcaactg tacatacagc taataaatgg gatctcatta ttagtgatat gtacgaccct 21060

aagactaaaa atgttacaaa agaaaatgac tctaaagagg gttttttcac ttacatttgt 21120

gggtttatac aacaaaagct agctcttgga ggttccgtgg ctataaagat aacagaacat 21180

tcttggaatg ctgatcttta taagctcatg ggacacttcg catggtggac agcctttgtt 21240

actaatgtga atgcgtcatc atctgaagca tttttaattg gatgtaatta tcttggcaaa 21300

ccacgcgaac aaatagatgg ttatgtcatg catgcaaatt acatattttg gaggaataca 21360

aatccaattc agttgtcttc ctattcttta tttgacatga gtaaatttcc ccttaaatta 21420

aggggtactg ctgttatgtc tttaaaagaa ggtcaaatca atgatatgat tttatctctt 21480

cttagtaaag gtagacttat aattagagaa aacaacagag ttgttatttc tagtgatgtt 21540

cttgttaaca actaaacgaa caatgtttgt ttttcttgtt ttattgccac tagtctctag 21600

tcagtgtgtt aatcttacaa ccagaactca attaccccct gcatacacta attctttcac 21660

acgtggtgtt tattaccctg acaaagtttt cagatcctca gttttacatt caactcagga 21720

cttgttctta cctttctttt ccaatgttac ttggttccat gctatacatg tctctgggac 21780

caatggtact aagaggtttg ataaccctgt cctaccattt aatgatggtg tttattttgc 21840

ttccactgag aagtctaaca taataagagg ctggattttt ggtactactt tagattcgaa 21900

gacccagtcc ctacttattg ttaataacgc tactaatgtt gttattaaag tctgtgaatt 21960

tcaattttgt aatgatccat ttttgggtgt ttattaccac aaaaacaaca aaagttggat 22020

ggaaagtgag ttcagagttt attctagtgc gaataattgc acttttgaat atgtctctca 22080

gccttttctt atggaccttg aaggaaaaca gggtaatttc aaaaatctta gggaatttgt 22140

gtttaagaat attgatggtt attttaaaat atattctaag cacacgccta ttaatttagt 22200

gcgtgatctc cctcagggtt tttcggcttt agaaccattg gtagatttgc caataggtat 22260

taacatcact aggtttcaaa ctttacttgc tttacataga agttatttga ctcctggtga 22320

ttcttcttca ggttggacag ctggtgctgc agcttattat gtgggttatc ttcaacctag 22380

gacttttcta ttaaaatata atgaaaatgg aaccattaca gatgctgtag actgtgcact 22440

tgaccctctc tcagaaacaa agtgtacgtt gaaatccttc actgtagaaa aaggaatcta 22500

tcaaacttct aactttagag kccaaccaac agaatctatt gttagatttc ctaatattac 22560

aaacttgtgc ccttttggtg aagtttttaa cgccaccaga tttgcatctg tttatgcttg 22620

gaacaggaag agaatcagca actgtgttgc tgattattct gtcctatata attccgcatc 22680

attttccact tttaagtgtt atggagtgtc tcctactaaa ttaaatgatc tctgctttac 22740

taatgtctat gcagattcat ttgtaattag aggtgatgaa gtcagacaaa tcgctccagg 22800

gcaaactgga aagattgctg attataatta taaattacca gatgatttta caggctgcgt 22860

tatagcttgg aattctaaca atcttgattc taaggttggt ggtaattata attacctgta 22920

tagattgttt aggaagtcta atctcaaacc ttttgagaga gatatttcaa ctgaaatcta 22980

tcaggccggt agcacacctt gtaatggtgt tgaaggtttt aattgttact ttcctttaca 23040

atcatatggt ttccaaccca ctaatggtgt tggttaccaa ccatacagag tagtagtact 23100

ttcttttgaa cttctacatg caccagcaac tgtttgtgga cctaaaaagt ctactaattt 23160

ggttaaaaac aaatgtgtca atttcaactt caatggttta acaggcacag gtgttcttac 23220

tgagtctaac aaaaagtttc tgcctttcca acaatttggc agagacattg ctgacactac 23280

tgatgctgtc cgtgatccac agacacttga gattcttgac attacaccat gttcttttgg 23340

tggtgtcagt gttataacac caggaacaaa tacttctaac caggttgctg ttctttatca 23400

gggtgttaac tgcacagaag tccctgttgc tattcatgca gatcaactta ctcctacttg 23460

gcgtgtttat tctacaggtt ctaatgtttt tcaaacacgt gcaggctgtt taataggggc 23520

tgaacatgtc aacaactcat atgagtgtga catacccatt ggtgcaggta tatgcgctag 23580

ttatcagact cagactaatt ctcctcggcg ggcacgtagt gtagctagtc aatccatcat 23640

tgcctacact atgtcacttg gtgcagaaaa ttcagttgct tactctrata actctattgc 23700

catacccaca aattttacta ttagtgttac cacagaaatt ctaccagtgt ctatgaccaa 23760

gacatcagta gattgtacaa tgtacatttg tggtgattca actgaatgca gcaatctttt 23820

gttgcaatat ggcagttttt gtacacaatt aaaccgtgct ttaactggaa tagctgttga 23880

acaagacaaa aacacccaag aagtttttgc acaagtcaaa caaatttaca aaacaccacc 23940

aattaaagat tttggtggtt ttaatttttc acaaatatta ccagatccat caaaaccaag 24000

caagaggtca tttattgaag atctactttt caacaaagtg acacttgcag atgctggctt 24060

catcaaacaa tatggtgatt gccttggtga tattgctgct agagacctca tttgtgcaca 24120

aaagtttaac ggccttactg ttttgccacc tttgctcaca gatgaaatga ttgctcaata 24180

cacttctgca ctgttagcgg gtacaatcac ttctggttgg acctttggtg caggtgctgc 24240

attacaaata ccatttgcta tgcaaatggc ttataggttt aatggtattg gagttacaca 24300

gaatgttctc tatgagaacc aaaaattgat tgccaaccaa tttaatagtg ctattggcaa 24360

aattcaagac tcactttctt ccacagcaag tgcacttgga aaacttcaag atgtggtcaa 24420

ccaaaatgca caagctttaa acacgcttgt taaacaactt agctccaatt ttggtgcaat 24480

ttcaagtgtt ttaaatgata tcctttcacg tcttgacaaa gttgaggctg aagtgcaaat 24540

tgataggttg atcacaggca gacttcaaag tttgcagaca tatgtgactc aacaattaat 24600

tagagctgca gaaatcagag tttctgctaa tcttgctgct actaaaatgt cagagtgtgt 24660

acttggacaa tcaaaaagag ttgatttttg tggaaagggc tatcatctta tgtccttccc 24720

tcagtcagca cctcatggtg tagtcttctt gcatgtgact tatgtccctg cacaagaaaa 24780

gaacttcaca actgctcctg ccatttgtca tgatggaaaa gcacactttc ctcgtgaagg 24840

tgtctttgtt tcaaatggca cacactggtt tgtaacacaa aggaattttt atgaaccaca 24900

aatcattact acagacaaca catttgtgtc tggtaactgt gatgttgtaa taggaattgt 24960

caacaacaca gtttatgatc ctttgcaacc tgaattagac tcattcaagg aggagttaga 25020

taaatatttt aagaatcata catcaccaga tgttgattta ggtgacatct ctggcattaa 25080

tgcttcagtt gtaaacattc aaaaagaaat tgaccgcctc aatgaggttg ccaagaattt 25140

aaatgaatct ctcatcgatc tccaagaact tggaaagtat gagcagtata taaaatggcc 25200

atggtacatt tggctaggtt ttatagctgg cttgattgcc atagtaatgg tgacaattat 25260

gctttgctgt atgaccagtt gctgtagttg tctcaagggc tgttgttctt gtggatcctg 25320

ctgcaaattt gatgaagacg actctgagcc agtgctcaaa ggagtcaaat tacattacac 25380

ataaacgaac ttatggattt gtttatgaga atcttcacaa ttggaactgt aactttgaag 25440

caaggtgaaa tcaaggatgc tactccttca gattttgttc gcgctactgc aacgataccg 25500

atacaagcct cactcccttt cggatggctt attgttggcg ttgcacttct tgctgttttt 25560

cagagcgctt ccaaaatcat aaccctcaaa aagagatggc aactagcact ctccaagggt 25620

gttcactttg tttgcaactt gctgttgttg tttgtaacag tttactcaca ccttttgctc 25680

gttgctgctg gccttgaagc cccttttctc tatctttatg ctttagtcta cttcttgcag 25740

agtataaact ttgtaagaat aataatgagg ctttggcttt gctggaaatg ccgttccaaa 25800

aacccattac tttatgatgc caactatttt ctttgctggc atactaattg ttacgactat 25860

tgtatacctt acaatagtgt aacttcttca attgtcatta cttcaggtga tggcacaaca 25920

agtcctattt ctgaacatga ctaccagatt ggtggttata ctgaaaaatg ggaatctgga 25980

gtaaaagact gtgttgtatt acacagttac ttcacttcag actattacca gctgtactca 26040

actcaattga gtacagacac tggtgttgaa catgttacct tcttcatcta caataaaatt 26100

gttgatgagc ctgaagaaca tgtccaaatt cacacaatcg acggttcatc cggagttgtt 26160

aatccagtaa tggaaccaat ttatgatgaa ccgacgacga ctactagcgt gcctttgtaa 26220

gcacaagctg atgagtacga acttatgtac tcattcgttt cggaagagmc aggtacgtta 26280

atagttaata gcgtacttct ttttcttgct ttcgtggtat tcttgctagt tacactagcc 26340

atccttactg cgcttcgatt gtgtgcgtac tgctgcaata ttgttaacgt gagtcttgta 26400

aaaccttctt tttacgttta ctctcgtgtt aaaaatctga attcttctag agttcctgat 26460

cttctggtct aaacgaacta aatattatat tagtttttct gtttggaact ttaattttag 26520

ccatggcaga ttccaacggt actattaccg ttgaagagct taaaaagctc cttgaacaat 26580

ggaacctagt aataggtttc ctattcctta catggatttg tcttctacaa tttgcctatg 26640

ccaacaggaa taggtttttg tatataatta agttaatttt cctctggctg ttatggccag 26700

taactttagc ttgttttgtg cttgctgctg tttacagaat aaattggatc accggtggaa 26760

ttgctatcgc aatggcttgt cttgtaggct tgatgtggct cagctacttc attgcttctt 26820

tcagactgtt tgcgcgtacg cgttccatgt ggtcattcaa tccagaaact aacattcttc 26880

tcaacgtgcc actccatggc actattctga ccagaccgct tctagaaagt gaactcgtaa 26940

tcggagctgt gatccttcgt ggacatcttc gtattgctgg acaccatcta ggacgctgtg 27000

acatcaagga cctgcctaaa gaaatcactg ttgctacatc acgaatgctt tcttattaca 27060

aattgggagc ttcgcagcgt gtagcaggtg actcaggttt tgctgcatac agtcgctaca 27120

ggattggcaa ctataaatta aacacagacc attccagtag cagtgacaat attgctttgc 27180

ttgtacagta agtgacaaca gatgtttcat ctcgttgact ttcaggttac tatagcagag 27240

atattactaa ttattatgag gacttttaaa gtttccattt ggaatcttga ttacatcata 27300

aacctcataa ttaaaaattt atctaagtca ctaactgaga ataaatattc tcaattagat 27360

gaagagcaac caatggagat tgattaaacg aacatgaaaa ttattctttt cttggcactg 27420

ataacactcg ctacttgtga gctttatcac taccaagagt gtgttagagg tacaacagta 27480

cttttaaaag aaccttgctc ttctggaaca tacgagggca attcaccatt tcatcctcta 27540

gctgataaca aatttgcact gacttgcttt agcactcaat ttgcttttgc ttgtcctgac 27600

ggcgtaaaac acgtctatca gttacgtgcc agatcagttt cacctaaact gttcatcaga 27660

caagaggaag ttcaagaact ttactctcca atttttctta ttgttgcggc aatagtgttt 27720

ataacacttt gcttcacact caaaagaaag acagaatgat tgaactttca ttaattgact 27780

tctatttgtg ctttttagcc tttctgctat tccttgtttt aattatgctt attatctttt 27840

ggttctcact tgaactgcaa gatcataatg aaacttgtca cgcctaaacg aacatgaaat 27900

ttcttgtttt cttaggaatc atcacaactg tagctgcatt tcaccaagaa tgtagtttac 27960

agtcatgtac tcaacatcaa ccatatgtag ttgatgaccc gtgtcctatt cacttctatt 28020

ctaaatggta tattagagta ggagctagaa aatcagcacc tttaattgaa ttgtgcgtgg 28080

atgaggctgg ttctaaatca cccattcagt acatcgatat cggtaattat acagtttcct 28140

gtttaccttt tacaattaat tgccaggaac ctaaattggg tagtcttgta gtgcgttgtt 28200

cgttctatga agacttttta gagtatcatg acgttcgtgt tgttttagat ttcatctaaa 28260

cgaacaaact aaaatgtctg ataatggacc ccaaaatcag cgaaatgcac cccgcattac 28320

gtttggtgga ccctcagatt caactggcag taaccagaat ggagaacgca gtggggcgcg 28380

atcaaaacaa cgtcggcccc aaggtttacc caataatact gcgtcttggt tcaccgctct 28440

cactcaacat ggcaaggaag accttaaatt ccctcgagga caaggcgttc caattaacac 28500

caatagcagt ccagatgacc aaattggcta ctaccgaaga gctaccagac gaattcgtgg 28560

tggtgacggt aaaatgaaag atctcagtcc aagatggtat ttctactacc taggaactgg 28620

gccagaagct ggacttccct atggtgctaa caaagacggc atcatatggg ttgcaactga 28680

gggagccttg aatacaccaa aagatcacat tggcacccgc aatcctgcta acaatgctgc 28740

aatcgtgcta caacttcctc aaggaacaac attgccaaaa ggcttctacg cagaagggag 28800

cagaggcggc agtcaagcct cttctcgttc ctcatcacgt agtcgcaaca gttcaagaaa 28860

ttcaactcca ggcagcagta aacgaacttc tcctgctaga atggctggca atggcggtga 28920

tgctgctctt gctttgctgc tgcttgacag attgaaccag cttgagagca aaatgtctgg 28980

taaaggccaa caacaacaag gccaaactgt cactaagaaa tctgctgctg aggcttctaa 29040

gaagcctcgg caaaaacgta ctgccactaa agcatacaat gtaacacaag ctttcggcag 29100

acgtggtcca gaacaaaccc aaggaaattt tggggaccag gaactaatca gacaaggaac 29160

tgattacaaa cattggccgc aaattgcaca atttgccccc agcgcttcag cgttcttcgg 29220

aatgtcgcgc attggcatgg aagtcacacc ttcgggaacg tggttgacct acacaggtgc 29280

catcaaattg gatgacaaag atccaaattt caaagatcaa gtcattttgc tgaataagca 29340

tattgacgca tacaaaacat tcccaccaac agagcctaaa aaggacaaaa agaagaaggc 29400

tgatgaaact caagccttac cgcagagaca gaagaaacag caaactgtga ctcttcttcc 29460

tgctgcagat ttggatgatt tctccaaaca attgcaacaa tccatgagca gtgctgactc 29520

aactcaggcc taaactcatg cagaccacac aaggcagatg ggctatataa acgttttcgc 29580

ttttccgttt acgatatata gtctactctt gtgcagaatg aattctcgta actacatagc 29640

acaagtagat gtagttaact ttaatctcac atagcaatct ttaatcagtg tgtaacatta 29700

gggaggactt gaaagagcca ccacattttc accgaggcca cgcggagtac gatcgagtgt 29760

acagtgaaca atgctaggga gagctgccta tatggaagag ccctaatgtg taaaattaat 29820

tttagtagtg ctatccccat gtgattttaa tagcttctta ggagnatgac annnnnnnnn 29880

nnnnn 29885

<210> 16
<211> 4405
<212> PRT
<213> SARS-CoV2

<400> 16

<210> 17
<211> 1273
<212> PRT
<213> SARS-CoV2

<220>
<221> misc_feature
<222> (320)..(320)
<223> Xaa can be any naturally occurring amino acid

<220>
<221> misc_feature
<222> (709)..(709)
<223> Xaa can be any naturally occurring amino acid

<400> 17

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Xaa
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Gly Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Xaa Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly

865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Val  Ser Ala Asn
    1010                1015                1020

Leu Ala  Ala Thr Lys Met Ser  Glu Cys Val Leu Gly  Gln Ser Lys
    1025                1030                1035

Arg Val  Asp Phe Cys Gly Lys  Gly Tyr His Leu Met  Ser Phe Pro
    1040                1045                1050

Gln Ser  Ala Pro His Gly Val  Val Phe Leu His Val  Thr Tyr Val
    1055                1060                1065

Pro Ala  Gln Glu Lys Asn Phe  Thr Thr Ala Pro Ala  Ile Cys His
    1070                1075                1080

Asp Gly  Lys Ala His Phe Pro  Arg Glu Gly Val Phe  Val Ser Asn
    1085                1090                1095

Gly Thr  His Trp Phe Val Thr  Gln Arg Asn Phe Tyr  Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210> 18
<211> 29897
<212> DNA
<213> SARS-CoV2

<220>
<221> misc_feature
<222> (11288)..(11293)
<223> n is a, c, g, or t

<220>
<221> misc_feature
<222> (22278)..(22283)
<223> n is a, c, g, or t

<220>
<221> misc_feature
<222> (29836)..(29864)

<223>  n is a, c, g, or t

<400>  18
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcttctatc     180

ttctgcaggc tgcttacggt ttcgtccgtg ttgcagccga tcatcagcac atctaggttt     240

tgtccgggtg tgaccgaaag gtaagatgga gagccttgtc cctggtttca acgagaaaac     300

acacgtccaa ctcagtttgc ctgttttaca ggttcgcgac gtgctcgtac gtggctttgg     360

agactccgtg gaggaggtct tatcagaggc acgtcaacat cttaaagatg gcacttgtgg     420

cttagtagaa gttgaaaaag gcgttttgcc tcaacttgaa cagccctatg tgttcatcaa     480

acgttcggat gctcgaactg cacctcatgg tcatgttatg gttgagctgg tagcagaact     540

cgaaggcatt cagtacggtc gtagtggtga gacacttggt gtccttgtcc ctcatgtggg     600

cgaaatacca gtggcttacc gcaaggttct tcttcgtaag aacggtaata aaggagctgg     660

tggccatagt tacggcgccg atctaaagtc atttgactta ggcgacgagc ttggcactga     720

tccttatgaa gattttcaag aaaactggaa cactaaacat agcagtggtg ttacccgtga     780

actcatgcgt gagcttaacg gaggggcata cactcgctat gtcgataaca acttctgtgg     840

ccctgatggc taccctcttg agtgcattaa agaccttcta gcacgtgctg gtaaagcttc     900

atgcactttg tccgaacaac tggactttat tgacactaag aggggtgtat actgctgccg     960

tgaacatgag catgaaattg cttggtacac ggaacgttct gaaaagagct atgaattgca    1020

gacacctttt gaaattaaat tggcaaagaa atttgacatc ttcaatgggg aatgtccaaa    1080

ttttgtattt cccttaaatt ccataatcaa gactattcaa ccaagggttg aaaagaaaaa    1140

gcttgatggc tttatgggta gaattcgatc tgtctatcca gttgcgtcac caaatgaatg    1200

caaccaaatg tgcctttcaa ctctcatgaa gtgtgatcat tgtggtgaaa cttcatggca    1260

gacgggcgat tttgttaaag ccacttgcga attttgtggc actgagaatt tgactaaaga    1320

aggtgccact acttgtggtt acttacccca aaatgctgtt gttaaaattt attgtccagc    1380

atgtcacaat tcagaagtag gacctgagca tagtcttgcc gaataccata atgaatctgg    1440

cttgaaaacc attcttcgta agggtggtcg cactattgcc tttggaggct gtgtgttctc    1500

ttatgttggt tgccataaca agtgtgccta ttgggttcca cgtgctagcg ctaacatagg    1560

ttgtaaccat acaggtgttg ttggagaagg ttccgaaggt cttaatgaca accttcttga    1620

aatactccaa aaagagaaag tcaacatcaa tattgttggt gactttaaac ttaatgaaga    1680

gatcgccatt attttggcat ctttttctgc ttccacaagt gcttttgtgg aaactgtgaa    1740

aggtttggat tataaagcat tcaaacaaat tgttgaatcc tgtggtaatt ttaaagttac    1800

aaaaggaaaa gctaaaaaag gtgcctggaa tattggtgaa cagaaatcaa tactgagtcc 1860

tctttatgca tttgcatcag aggctgctcg tgttgtacga tcaattttct cccgcactct 1920

tgaaactgct caaaattctg tgcgtgtttt acagaaggcc gctataacaa tactagatgg 1980

aatttcacag tattcactga gactcattga tgctatgatg ttcacatctg atttggctac 2040

taacaatcta gttgtaatgg cctacattac aggtggtgtt gttcagttga cttcgcagtg 2100

gctaactaac atctttggca ctgtttatga aaaactcaaa cccgtccttg attggcttga 2160

agagaagttt aaggaaggtg tagagtttct tagagacggt tgggaaattg ttaaatttat 2220

ctcaacctgt gcttgtgaaa ttgtcggtgg acaaattgtc acctgtgcaa aggaaattaa 2280

ggagagtgtt cagacattct ttaagcttgt aaataaattt ttggctttgt gtgctgactc 2340

tatcattatt ggtggagcta aacttaaagc cttgaattta ggtgaaacat ttgtcacgca 2400

ctcaaaggga ttgtacagaa agtgtgttaa atccagagaa gaaactggcc tactcatgcc 2460

tctaaaagcc ccaaaagaaa ttatcttctt agagggagaa acacttccca cagaagtgtt 2520

aacagaggaa gttgtcttga aaactggtga tttacaacca ttagaacaac ctactagtga 2580

agctgttgaa gctccattgg ttggtacacc agtttgtatt aacgggctta tgttgctcga 2640

aatcaaagac acagaaaagt actgtgccct tgcacctaat atgatggtaa ctaacaatac 2700

cttcacactc aaaggcggtg caccaacaaa ggttactttt ggtgatgaca ctgtgataga 2760

agtgcaaggt tacaagagtg tgaatatcac ttttgaactt gatgaaagga ttgataaagt 2820

acttaatgag aagtgctctg cctatacagt tgaactcggt acagaagtaa atgagttcgc 2880

ctgtgttgtg gcagatgctg tcataaaaac tttgcaacca gtatctgaat tacttacacc 2940

actgggcatt gatttagatg agtggagtat ggctacatac tacttatttg atgagtctgg 3000

tgagtttaaa ttggcttcac atatgtattg ttctttttac cctccagatg aggatgaaga 3060

agaaggtgat tgtgaagaag aagagtttga gccatcaact caatatgagt atggtactga 3120

agatgattac caaggtaaac ctttggaatt tggtgccact tctgctgctc ttcaacctga 3180

agaagagcaa gaagaagatt ggttagatga tgatagtcaa caaactgttg gtcaacaaga 3240

cggcagtgag gacaatcaga caactactat tcaaacaatt gttgaggttc aacctcaatt 3300

agagatggaa cttacaccag ttgttcagac tattgaagtg aatagtttta gtggttattt 3360

aaaacttact gacaatgtat acattaaaaa tgcagacatt gtggaagaag ctaaaaaggt 3420

aaaaccaaca gtggttgtta atgcagccaa tgtttacctt aaacatggag gaggtgttgc 3480

aggagcctta aataaggcta ctaacaatgc catgcaagtt gaatctgatg attacatagc 3540

tactaatgga ccacttaaag tgggtggtag ttgtgtttta agcggacaca atcttgctaa 3600

acactgtctt catgttgtcg gcccaaatgt taacaaaggt gaagacattc aacttcttaa 3660

gagtgcttat gaaaatttta atcagcacga agttctactt gcaccattat tatcagctgg 3720

tatttttggt gctgacccta tacattcttt aagagtttgt gtagatactg ttcgcacaaa 3780

tgtctactta gctgtctttg ataaaaatct ctatgacaaa cttgtttcaa gctttttgga 3840

aatgaagagt gaaaagcaag ttgaacaaaa gatcgctgag attcctaaag aggaagttaa 3900

gccatttata actgaaagta aaccttcagt tgaacagaga aaacaagatg ataagaaaat 3960

caaagcttgt gttgaagaag ttacaacaac tctggaagaa actaagttcc tcacagaaaa 4020

cttgttactt tatattgaca ttaatggcaa tcttcatcca gattctgcca ctcttgttag 4080

tgacattgac atcactttct taaagaaaga tgctccatat atagtgggtg atgttgttca 4140

agagggtgtt ttaactgctg tggttatacc tactaaaaag gctggtggca ctactgaaat 4200

gttagcgaaa gctttgagaa aagtgccaac agacaattat ataaccactt acccgggtca 4260

gggtttaaat ggttacactg tagaggaggc aaagacagtg cttaaaaagt gtaaaagtgc 4320

cttttacatt ctaccatcta ttatctctaa tgagaagcaa gaaattcttg gaactgtttc 4380

ttggaatttg cgagaaatgc ttgcacatgc agaagaaaca cgcaaattaa tgcctgtctg 4440

tgtggaaact aaagccatag tttcaactat acagcgtaaa tataagggta ttaaaataca 4500

agagggtgtg gttgattatg gtgctagatt ttacttttac accagtaaaa caactgtagc 4560

gtcacttatc aacacactta acgatctaaa tgaaactctt gttacaatgc cacttggcta 4620

tgtaacacat ggcttaaatt tggaagaagc tgctcggtat atgagatctc tcaaagtgcc 4680

agctacagtt tctgtttctt cacctgatgc tgttacagcg tataatggtt atcttacttc 4740

ttcttctaaa acacctgaag aacattttat tgaaaccatc tcacttgctg gttcctataa 4800

agattggtcc tattctggac aatctacaca actaggtata gaatttctta agagaggtga 4860

taaaagtgta tattacacta gtaatcctac cacattccac ctagatggtg aagttatcac 4920

ctttgacaat cttaagacac ttctttcttt gagagaagtg aggactatta aggtgtttac 4980

aacagtagac aacattaacc tccacacgca agttgtggac atgtcaatga catatggaca 5040

acagtttggt ccaacttatt tggatggagc tgatgttact aaaataaaac ctcataattc 5100

acatgaaggt aaaacatttt atgttttacc taatgatgac actctacgtg ttgaggcttt 5160

tgagtactac cacacaactg atcctagttt tctgggtagg tacatgtcag cattaaatca 5220

cactaaaaat tggaaatacc cacaagttaa tggtttaact tctattaaat gggcagataa 5280

caactgttat cttgccactg cattgttaac actccaacaa atagagttga agtttaatcc 5340

acctgctcta caagatgctt attacagagc aagggctggt gaagctgcta acttttgtgc 5400

acttatctta gcctactgta ataagacagt aggtgagtta ggtgatgtta gagaaacaat 5460

gagttacttg tttcaacatg ccaatttaga ttcttgcaaa agagtcttga acgtggtgtg 5520

taaaacttgt ggacaacagc agacaaccct taagggtgta gaagctgtta tgtacatggg 5580

cacactttct tatgaacaat ttaagaaagg tgttcagata ccttgtacgt gtggtaaaca 5640

agctacaaaa tatctagtac aacaggagtc accttttgtt atgatgtcag caccacctgc 5700

tcagtatgaa cttaagcatg gtacatttac ttgtgctagt gagtacactg gtaattacca 5760

gtgtggtcac tataaacata taacttctaa agaaactttg tattgcatag acggtgcttt 5820

acttacaaag tcctcagaat acaaaggtcc tattacggat gttttctaca aagaaaacag 5880

ttacacaaca accataaaac cagttactta taaattggat ggtgttgttt gtacagaaat 5940

tgaccctaag ttggacaatt attataagaa agacaattct tatttcacag agcaaccaat 6000

tgatcttgta ccaaaccaac catatccaaa cgcaagcttc gataatttta agtttgtatg 6060

tgataatatc aaatttgctg atgatttaaa ccagttaact ggttacaaga aacctgcttc 6120

aagagagctt aaagttacat ttttccctga cttaaatggt gatgtggtgg ctattgatta 6180

taaacactac acaccctctt ttaagaaagg agctaaattg ttacataaac ctattgtttg 6240

gcatgttaac aatgcaacta ataaagccac gtataaacca aatacctggt gtatacgttg 6300

tctttggagc acaaaaccag ttgaaacatc aaattcgttt gatgtactga agtcagagga 6360

cgcgcaggga atggataatc ttgcctgcga agatctaaaa ccagtctctg aagaagtagt 6420

ggaaaatcct accatacaga aagacgttct tgagtgtaat gtgaaaacta ccgaagttgt 6480

aggagacatt atacttaaac cagcaaataa tagtttaaaa attacagaag aggttggcca 6540

cacagatcta atggctgctt atgtagacaa ttctagtctt actattaaga aacctaatga 6600

attatctaga gtattaggtt tgaaaaccct tgctactcat ggtttagctg ctgttaatag 6660

tgtcccttgg gatactatag ctaattatgc taagcctttt cttaacaaag ttgttagtac 6720

aactactaac atagttacac ggtgtttaaa ccgtgtttgt actaattata tgccttattt 6780

ctttacttta ttgctacaat tgtgtacttt tactagaagt acaaattcta gaattaaagc 6840

atctatgccg actactatag caaagaatac tgttaagagt gtcggtaaat tttgtctaga 6900

ggcttcattt aattatttga agtcacctaa tttttctaaa ctgataaata ttataatttg 6960

gtttttacta ttaagtgttt gcctaggttc tttaatmtac tcaaccgctg ctttaggtgt 7020

tttaatgtct aatttaggca tgccttctta ctgtactggt tacagagaag gctatttgaa 7080

ctctactaat gtcactattg caacctactg tactggttct ataccttgta gtgtttgtct 7140

tagtggttta gattctttag acacctatcc ttctttagaa actatacaaa ttaccatttc 7200

atcttttaaa tgggatttaa ctgcttttgg cttagttgca gagtggtttt tggcatatat 7260

tcttttcact aggtttttct atgtacttgg attggctgca atcatgcaat tgtttttcag 7320

ctattttgca gtacatttta ttagtaattc ttggcttatg tggttaataa ttaatcttgt 7380

acaaatggcc ccgatttcag ctatggttag aatgtacatc ttctttgcat cattttatta 7440

tgtatggaaa agttatgtgc atgttgtaga cggttgtaat tcatcaactt gtatgatgtg 7500

ttacaaacgt aatagagcaa caagagtcga atgtacaact attgttaatg gtgttagaag 7560

gtccttttat gtctatgcta atggaggtaa aggcttttgc aaactacaca attggaattg 7620

tgttaattgt gatacattct gtgctggtag tacatttatt agtgatgaag ttgcgagaga 7680

cttgtcacta cagtttaaaa gaccaataaa tcctactgac cagtcttctt acatcgttga 7740

tagtgttaca gtgaagaatg gttccatcca tctttacttt gataaagctg gtcaaaagac 7800

ttatgaaaga cattctctct ctcattttgt taacttagac aacctgagag ctaataacac 7860

taaaggttca ttgcctatta atgttatagt ttttgatggt aaatcaaaat gtgaagaatc 7920

atctgcaaaa tcagcgtctg tttactacag tcagcttatg tgtcaaccta tactgttact 7980

agatcaggca ttagtgtctg atgttggtga tagtgcggaa gttgcagtta aaatgtttga 8040

tgcttacgtt aatacgtttt catcaacttt taacgtacca atggaaaaac tcaaaacact 8100

agttgcaact gcagaagctg aacttgcaaa gaatgtgtcc ttagacaatg tcttatctac 8160

ttttatttca gcagctcggc aagggtttgt tgattcagat gtagaaacta aagatgttgt 8220

tgaatgtctt aaattgtcac atcaatctga catagaagtt actggcgata gttgtaataa 8280

ctatatgctc acctataaca aagttgaaaa catgacaccc cgtgaccttg gtgcttgtat 8340

tgactgtagt gcgcgtcata ttaatgcgca ggtagcaaaa agtcacaaca ttgctttgat 8400

atggaacgtt aaagatttca tgtcattgtc tgaacaacta cgaaaacaaa tacgtagtgc 8460

tgctaaaaag aataacttac cttttaagtt gacatgtgca actactagac aagttgttaa 8520

tgttgtaaca acaaagatag cacttaaggg tggtaaaatt gttaataatt ggttgaagca 8580

gttaattaaa gttacacttg tgttcctttt tgttgctgct atttctattt taataacacc 8640

tgttcatgtc atgtctaaac atactgactt ttcaagtgaa atcataggat acaaggctat 8700

tgatggtggt gtcactcgtg acatagcatc tacagatact tgttttgcta acaaacatgc 8760

tgattttgac acatggttta gccagcgtgg tggtagttat actaatgaca aagcttgccc 8820

attgattgct gcagtcataa caagagaagt gggttttgtc gtgcctggtt tgcctggcac 8880

gatattacgc acaactaatg gtgacttttt gcatttctta cctagagttt ttagtgcagt 8940

tggtaacatc tgttacacac catcaaaact tatagagtac actgactttg caacatcagc 9000

ttgtgttttg gctgctgaat gtacaatttt taaagatgct tctggtaagc cagtaccata 9060

ttgttatgat accaatgtac tagaaggttc tgttgcttat gaaagtttac gccctgacac 9120

acgttatgtg ctcatggatg gctctattat tcaatttcct aacacctacc ttgaaggttc 9180

tgttagagtg gtaacaactt ttgattctga gtactgtagg cacggcactt gtgaaagatc 9240

agaagctggt gtttgtgtat ctactagtgg tagatgggta cttaacaatg attattacag 9300

atctttacca ggagttttct gtggtgtaga tgctgtaaat ttatttacta atatgtttac 9360

accactaatt caacctattg gtgctttgga catatcagca tctatagtag ctggtggtat 9420

tgtagctatc gtagtaacat gccttgccta ctattttatg aggtttagaa gagcttttgg 9480

tgaatacagt catgtagttg cctttaatac tttactattc cttatgtcat tcactgtact 9540

ctgtttaaca ccagtttact cattcttacc tggtgtttat tctgttattt acttgtactt 9600

gacattttat cttactaatg atgtttcttt tttagcacat attcagtgga tggttatgtt 9660

cacaccttta gtacctttct ggataacaat tgcttatatc atttgtattt ccacaaagca 9720

tttctattgg ttctttagta attacctaaa gagacgtgta gtctttaatg gtgtttcctt 9780

tagtactttt gaagaagctg cgctgtgcac ctttttgtta aataaagaaa tgtatctaaa 9840

gttgcgtagt gatgtgctat tacctcttac gcaatataat agatacttag ctctttataa 9900

taagtacaag tattttagtg gagcaatgga tacaactagc tacagagaag ctgcttgttg 9960

tcatctcgca aaggctctca atgacttcag taactcaggt tctgatgttc tttaccaacc 10020

accacaaacc tctatcacct cagctgtttt gcagagtggt tttagaaaaa tggcattccc 10080

atctggtaaa gttgagggtt gtatggtaca agtaacttgt ggtacaacta cacttaacgg 10140

tctttggctt gatgacgtag tttactgtcc aagacatgtg atctgcacct ctgaagacat 10200

gcttaaccct aattatgaag atttactcat tcgtaagtct aatcataatt tcttggtaca 10260

ggctggtaat gttcaactca gggttattgg acattctatg caaaattgtg tacttaagct 10320

tagggttgat acagccaatc ctaagacacc taagtataag tttgttcgca ttcaaccagg 10380

acagactttt tcagtgttag cttgttacaa tggttcacca tctggtgttt accaatgtgc 10440

tatgaggccc aatttcacta ttaagggttc attccttaat ggttcatgtg gtagtgttgg 10500

ttttaacata gattatgact gtgtctcttt ttkttacatg caccatatgg aattaccaac 10560

tggagttcat gctggcacag acttagaagg taacttttat ggaccttttg ttgacaggca 10620

aacagcacaa gcagctggta cggacacaac tattacagtt aatgttttag cttggttgta 10680

cgctgctgtt ataaatggag acaggtggtt tctcaatcga tttaccacaa ctcttaatga 10740

ctttaacctt gtggctatga agtacaatta tgaacytcta acacaagacc atgttgacat 10800

actaggacct ctttctgctc aaactggaat tgccgtttta gatatgtgtg cttcattaaa 10860

agaattactg caaaatggta tgaatggacg taccatattg ggtagtgctt tattagaaga 10920

tgaatttaca ccttttgatg ttgttagaca atgctcaggt gttactttcc aaagtgcagt 10980

gaaaagaaca atcaagggta cacaccactg gttgttactc acaattttga cttcactttt 11040

agttttagtc cagagtactc aatggtcttt gttctttttt ttgtatgaaa atgccttttt 11100

accttttgct atgggtatta ttgctatgtc tgcttttgca atgatgtttg tcaaacataa 11160

gcatgcattt ctctgtttgt ttttgttacc ttctcttgcc actgtagctt attttaatat 11220

ggtctatatg cctgctagtt gggtgatgcg tattatgaca tggttggata tggttgatac 11280

tagtttgnnn nnnaagctaa aagactgtgt tatgtatgca tcagctgtag tgttactaat 11340

ccttatgaca gcaagaactg tgtatgatga tggtgctagg agagtgtgga cacttatgaa 11400

tgtcttgaca ctcgtttata aagtttatta tggtaatgct ttagatcaag ccatttccat 11460

gtgggctctt ataatctctg ttacttctaa ctactcaggt gtagttacaa ctgtcatgtt 11520

tttggccaga ggtattgttt ttatgtgtgt tgagtattgc cctattttct tcataactgg 11580

taatacactt cagtgtataa tgctagttta ttgtttctta ggctattttt gtacttgtta 11640

ctttggcctc ttttgtttac tcaaccgcta ctttagactg actcttggtg tttatgatta 11700

cttagtttct acacaggagt ttagatatat gaattcacag ggactaytcc cacccaagaa 11760

tagcatagat gccttcaaac tcaacattaa attgttgggt gttggtggca aaccttgtat 11820

caaagtagcc actgtacagt ctaaaatgtc agatgtaaag tgcacatcag tagtcttact 11880

ctcagttttg caacaactca gagtagaatc atcatctaaa ttgtgggctc aatgtgtcca 11940

gttacacaat gacattctct tagctaaaga tactactgaa gcctttgaaa aaatggtttc 12000

actactttct gttttgcttt ccatgcaggg tgctgtagac ataaacaagc tttgtgaaga 12060

aatgctggac aacagggcaa ccttacaagc tatagcctca gagtttagtt cccttccatc 12120

atatgcagct tttgctactg ctcaagaagc ttatgagcag gctgttgcta atggtgattc 12180

tgaagttgtt cttaaaaagt tgaagaagtc tttgaatgtg gctaaatctg aatttgaccg 12240

tgatgcagcc atgcaacgta agttggaaaa gatggctgat caagctatga cccaaatgta 12300

taaacaggct agatctgagg acaagagggc aaaagttact agtgctatgc agacaatgct 12360

tttcactatg cttagaaagt tggataatga tgcactcaac aacattatca acaatgcaag 12420

agatggttgt gttcccttga acataatacc tcttacaaca gcagccaaac taatggttgt 12480

cataccagac tataacacat ataaaaatac gtgtgatggt acaacattta cttatgcatc 12540

agcattgtgg gaaatccaac aggttgtaga tgcagatagt aaaattgttc aacttagtga 12600

aattagtatg gacaattcac ctaatttagc atggcctctt attgtaacag ctttaagggc 12660

caattctgct gtcaaattac agaataatga gcttagtcct gttgcactac gacagatgtc 12720

ttgtgctgcc ggtactacac aaactgcttg cactgatgac aatgcgttag cttactacaa 12780

cacaacaaag ggaggtaggt ttgtacttgc actgttatcc gatttacagg atttgaaatg 12840

ggctagattc cctaagagtg atggaactgg tactatctat acagaactgg aaccaccttg 12900

taggtttgtt acagacacac ctaaaggtcc taaagtgaag tatttatact ttattaaagg 12960

attaaacaac ctaaatagag gtatggtact tggtagttta gctgccacag tacgtctaca 13020

agctggtaat gcaacagaag tgcctgccaa ttcaactgta ttatctttct gtgcttttgc 13080

tgtagatgct gctaaagctt acaaagatta tctagctagt gggggacaac caatcactaa 13140

ttgtgttaag atgttgtgta cacacactgg tactggtcag gcaataacag ttacaccgga 13200

agccaatatg gatcaagaat cctttggtgg tgcatcgtgt tgtctgtact gccgttgcca 13260

catagatcat ccaaatccta aaggattttg tgacttaaaa ggtaagtatg tacaaatacc 13320

tacaacttgt gctaatgacc ctgtgggttt tacacttaaa aacacagtct gtaccgtctg 13380

cggtatgtgg aaaggttatg gctgtagttg tgatcaactc cgcgaaccca tgcttcagtc 13440

agctgatgca caatcgtttt taaacgggtt tgcggtgtaa gtgcagcccg tcttacaccg 13500

tgcggcacag gcactagtac tgatgtcgta tacagggctt ttgacatcta caatgataaa 13560

gtagctggtt ttgctaaatt cctaaaaact aattgttgtc gcttccaaga aaaggacgaa 13620

gatgacaatt taattgattc ttactttgta gttaagagac acactttctc taactaccaa 13680

catgaagaaa caatttataa tttacttaag gattgtccag ctgttgctaa acatgacttc 13740

tttaagttta gaatagacgg tgacatggta ccacatatat cacgtcaacg tcttactaaa 13800

tacacaatgg cagacctcgt ctatgcttta aggcattttg atgaaggtaa ttgtgacaca 13860

ttaaaagaaa tacttgtcac atacaattgt tgtgatgatg attatttcaa taaaaaggac 13920

tggtatgatt ttgtagaaaa cccagatata ttacgcgtat acgccaactt aggtgaacgt 13980

gtacgccaag ctttgttaaa aacagtacaa ttctgtgatg ccatgcgaaa tgctggtatt 14040

gttggtgtac tgacattaga taatcaagat ctcaatggta actggtatga tttcggtgat 14100

ttcatacaaa ccacgccagg tagtggagtt cctgttgtag attcttatta ttcattgtta 14160

atgcctatat taaccttgac cagggcttta actgcagagt cacatgttga cactgactta 14220

acaaagcctt acattaagtg ggatttgtta aaatatgact tcacggaaga gaggttaaaa 14280

ctctttgacc gttattttaa atattgggat cagacatacc acccaaattg tgttaactgt 14340

ttggatgaca gatgcattct gcattgtgca aactttaatg ttttattctc tacagtgttc 14400

ccacttacaa gttttggacc actagtgaga aaaatatttg ttgatggtgt tccatttgta 14460

gtttcaactg gataccactt cagagagcta ggtgttgtac ataatcagga tgtaaactta 14520

catagctcta gacttagttt taaggaatta cttgtgtatg ctgctgaccc tgctatgcac 14580

gctgcttctg gtaatctatt actagataaa cgcactacgt gcttttcagt agctgcactt 14640

actaacaatg ttgcttttca aactgtcaaa cccggtaatt ttaacaaaga cttctatgac 14700

tttgctgtgt ctaagggttt ctttaaggaa ggaagttctg ttgaattaaa acacttcttc 14760

tttgctcagg atggtaatgc tgctatcagc gattatgact actatcgtta taatctacca 14820

acaatgtgtg atatcagaca actactattt gtagttgaag ttgttgataa gtactttgat 14880

tgttacgatg gtggctgtat taatgctaac caagtcatcg tcaacaacct agacaaatca 14940

gctggttttc catttaataa atggggtaag gctagacttt attatgattc aatgagttat 15000

gaggatcaag atgcactttt cgcatataca aaacgtaatg tcatccctac tataactcaa 15060

atgaatctta agtatgccat tagtgcaaag aatagagctc gcaccgtagc tggtgtctct 15120

atctgtagta ctatgaccaa tagacagttt catcaaaaat tattgaaatc aatagccgcc 15180

actagaggag ctactgtagt aattggaaca agcaaattct atggtggttg gcacaacatg 15240

ttaaaaactg tttatagtga tgtagaaaac cctcacctta tgggttggga ttatcctaaa 15300

tgtgatagag ccatgcctaa catgcttaga attatggcct cacttgttct tgctcgcaaa 15360

catacaacgt gttgtagctt gtcacaccgt ttctatagat tagctaatga gtgtgctcaa 15420

gtattgagtg aaatggtcat gtgtggcggt tcactatatg ttaaaccagg tggaacctca 15480

tcaggagatg ccacaactgc ttatgctaat agtgttttta acatttgtca agctgtcacg 15540

gccaatgtta atgcactttt atctactgat ggtaacaaaa ttgccgataa gtatgtccgc 15600

aatttacaac acagacttta tgagtgtctc tatagaaata gagatgttga cacagacttt 15660

gtgaatgagt tttacgcata tttgcgtaaa catttctcaa tgatgatact ctctgacgat 15720

gctgttgtgt gtttcaatag cacttatgca tctcaaggtc tagtggctag cataaagaac 15780

tttaagtcag ttctttatta tcaaaacaat gtttttatgt ctgaagcaaa atgttggact 15840

gagactgacc ttactaaagg acctcatgaa ttttgctctc aacatacaat gctagttaaa 15900

cagggtgatg attatgtgta ccttccttac ccagatccat caagaatcct aggggccggc 15960

tgttttgtag atgatatcgt aaaaacagat ggtacactta tgattgaacg gttcgtgtct 16020

ttagctatag atgcttaccc acttactaaa catcctaatc aggagtatgc tgatgtcttt 16080

catttgtact tacaatacat aagaaagcta catgatgagt taacaggaca catgttagac 16140

atgtattctg ttatgcttac taatgataac acttcaaggt attgggaacc tgagttttat 16200

gaggctatgt acacaccgca tacagtctta caggctgttg gggcttgtgt tctttgcaat 16260

tcacagactt cattaagatg tggtgcttgc atacgtagac cattcttatg ttgtaaatgc 16320

tgttacgacc atgtcatatc aacatcacat aaattagtct tgtctgttaa tccgtatgtt 16380

tgcaatgctt caggttgtga tgtcacagat gtgactcaac tttacttagg aggtatgagc 16440

tattattgta aatcacataa accacccatt agttttccat tgtgtgctaa tggacaagtt 16500

tttggtttat ataaaaatac atgtgttggt agcgataatg ttactgactt taatgcaatt 16560

gcaacatgtg actggacaaa tgctggtgat tacattttag ctaacacctg tactgaaaga 16620

ctcaagcttt ttgcagcaga aacgctcaaa gctactgagg agacatttaa actgtcttat 16680

ggtattgcta ctgtacgtga agtgctgtct gacagagaat tacatctttc atgggaagtt 16740

ggtaaaccta gaccaccact taaccgaaat tatgtcttta ctggttatcg tgtaactaaa 16800

aacagtaaag tacaaatagg agagtacacc tttgaaaaag gtgactatgg tgatgctgtt 16860

gtttaccgag gtacaacaac ttacaaatta aatgttggtg attattttgt gctgacatca 16920

catacagtaa tgccattaag tgcacctaca ctagtgccac aagagcacta tgttagaatt 16980

actggcttat acccaacact caatatctca gatgagtttt ctagcaatgt tgcaaattat 17040

caaaaggttg gtatgcaaaa gtattctaca ctccagggac cacctggtac tggtaagagt 17100

cattttgcta ttggcctagc tctctactac ccttctgctc gcatagtgta tacagcttgc 17160

tctcatgccg ctgttgatgc actatgtgag aaggcattaa aatatttgcc tatagataaa 17220

tgtagtagaa ttatacctgc acgtgctcgt gtagagtgtt ttgataaatt caaagtgaat 17280

tcaacattag aacagtatgt cttttgtact gtaaatgcat tgcctgagac gacagcagat 17340

atagttgtct ttgatgaaat ttcaatggcc acaaattatg atttgagtgt tgtcaatgcc 17400

agattacgtg ctaagcacta tgtgtacatt ggcgaccctg ctcaattacc tgcaccacgc 17460

acattgctaa ctaagggcac actagaacca gaatatttca attcagtgtg tagacttatg 17520

aaaactatag gtccagacat gttcctcgga acttgtcggc gttgtcctgc tgaaattgtt 17580

gacactgtga gtgctttggt ttatgataat aagcttaaag cacataaaga caaatcagct 17640

caatgcttta aaatgtttta taagggtgtt atcacgcatg atgtttcatc tgcaattaac 17700

aggccacaaa taggcgtggt aagagaattc cttacacgta accctgcttg gagaaaagct 17760

gtctttattt caccttataa ttcacagaat gctgtagcct caaagatttt gggactacca 17820

actcaaactg ttgattcatc acaggctcag aatatgacta atgtcatatt cactcaaacc 17880

actgaaacag ctcactcttg taatgtaaac agatttaatg ttgctattac cagagcaaaa 17940

gtaggcatac tttgcataat gtctgataga gacctttatg acaagttgca atttacaagt 18000

cttgaaattc cacgtaggaa tgtggcaact ttacaagctg aaaatgtaac aggactcttt 18060

aaagattgta gtaaggtaat cactgggtta catcctacac aggcacctac acacctcagt 18120

gttgacacta aattcaaaac tgaaggttta tgtgttgaca tacctggcat acctaaggac 18180

atgacctata gaagactcat ctctatgatg ggttttaaaa tgaattatca agttaatggt 18240

taccctaaca tgtttatcac ccgcgaagaa gctataagac atgtacgtgc atggattggc 18300

ttcgatgtcg aggggtgtca tgctactaga gaagctgttg gtaccaattt acctttacag 18360

ctaggttttt ctacaggtgt taacctagtt gctgtaccta caggttatgt tgatacacct 18420

aataatacag atttttccag agttagtgct aaaccaccgc ctggagatca atttaaacac 18480

ctcataccac ttatgtacaa aggacttcct tggaatgtag tgcgtataaa gattgtacaa 18540

atgttaagtg acacacttaa aaatctctct gacagagtcg tatttgtctt atgggcacat 18600

ggctttgagt tgacatctat gaagtatttt gtgaaaatag gacctgagcg cacctgttgt 18660

ctatgtgata gacgtgccac atgcttttcc actgcttcag acacttatgc ctgttggcat 18720

cattctattg gatttgatta cgtctataat ccgtttatga ttgatgttca acaatggggt 18780

tttacaggta acctacaaag caaccatgat ctgtattgtc aagtccatgg taatgcacat 18840

gtagctagtt gtgatgcaat catgactagg tgtctagctg tccacgagtg ctttgttaag 18900

cgtgttgact ggactattga atatcctata attggtgatg aactgaagat taatgcggct 18960

tgtagaaagg ttcaacacat ggttgttaaa gctgcattat tagcagacaa attcccagtt 19020

cttcacgaca ttggtaaccc taaagctatt aagtgtgtac ctcaagctga tgtagaatgg 19080

aagttctatg atgcacagcc ttgtagtgac aaagcttata aaatagaaga attattctat 19140

tcttatgcca cacattctga caaattcaca gatggtgtat gcctattttg gaattgcaat 19200

gtcgatagat atcctgctaa ttccattgtt tgtagatttg acactagagt gctatctaac 19260

cttaacttgc ctggttgtga tggtggcagt ttgtatgtaa ataaacatgc attccacaca 19320

ccagcttttg ataaaagtgc ttttgttaat ttaaaacaat taccattttt ctattactct 19380

gacagtccat gtgagtctca tggaaaacaa gtagtgtcag atatagatta tgtaccacta 19440

aagtctgcta cgtgtataac acgttgcaat ttaggtggtg ctgtctgtag acatcatgct 19500

aatgagtaca gattgtatct cgatgcttat aacatgatga tctcagctgg ctttagcttg 19560

tgggtttaca aacaatttga tacttataac ctctggaaca cttttacaag acttcagagt 19620

ttagaaaatg tggcttttaa tgttgtaaat aagggacact ttgatggaca acagggtgaa 19680

gtaccagttt ctatcattaa taacactgtt tacacaaaag ttgatggtgt tgatgtagaa 19740

ttgtttgaaa ataaaacaac attacctgtt aatgtagcat ttgagctttg ggctaagcgc 19800

aacattaaac cagtaccaga ggtgaaaata ctcaataatt tgggtgtgga cattgctgct 19860

aatactgtga tctgggacta caaaagagat gctccagcac atatatctac tattggtgtt 19920

tgttctatga ctgacatagc caagaaacca actgaaacga tttgtgcacc actcactgtc 19980

ttttttgatg gtagagttga tggtcaagta gacttattta gaaatgcccg taatggtgtt 20040

cttattacag aaggtagtgt taaaggttta caaccatctg taggtcccaa acaagctagt 20100

cttaatggag tcacattaat tggagaagcc gtaaaaacac agttcaatta ttataagaaa 20160

gttgatggtg ttgtccaaca attacctgaa acttacttta ctcagagtag aaatttacaa 20220

gaatttaaac ccaggagtca aatggaaatt gatttcttag aattagctat ggatgaattc 20280

attgaacggt ataaattaga aggctatgcc ttcgaacata tcgtttatgg agattttagt 20340

catagtcagt taggtggttt acatctactg attggactag ctaaacgttt taaggaatca 20400

ccttttgaat tagaagattt tattcctatg gacagtacag ttaaaaacta tttcataaca 20460

gatgcgcaaa caggttcatc taagtgtgtg tgttctgtta ttgatttatt acttgatgat 20520

tttgttgaaa taataaaatc ccaagattta tctgtagttt ctaaggttgt caaagtgact 20580

attgactata cagaaatttc atttatgctt tggtgtaaag atggccatgt agaaacattt 20640

tacccaaaat tacaatctag tcaagcgtgg caaccgggtg ttgctatgcc taatctttac 20700

aaaatgcaaa gaatgctatt agaaaagtgt gaccttcaaa attatggtga tagtgcaaca 20760

ttacctaaag gcataatgat gaatgtcgca aaatatactc aactgtgtca atatttaaac 20820

acattaacat tagctgtacc ctataatatg agagttatac attttggtgc tggttctgat 20880

aaaggagttg caccaggtac agctgtttta agacagtggt tgcctacggg tacgctgctt 20940

gtcgattcag atcttaatga ctttgtctct gatgcagatt caactttgat tggtgattgt 21000

gcaactgtac atacagctaa taaatgggat ctcattatta gtgatatgta cgaccctaag 21060

actaaaaatg ttacaaaaga aaatgactct aaagagggtt ttttcactta catttgtggg 21120

tttatacaac aaaagctagc tcttggaggt tccgtggcta taaagataac agaacattct 21180

tggaatgctg atctttataa gctcatggga cacttcgcat ggtggacagc ctttgttact 21240

aatgtgaatg cgtcatcatc tgaagcattt ttaattggat gtaattatct tggcaaacca 21300

cgcgaacaaa tagatggtta tgtcatgcat gcaaattaca tattttggag gaatacaaat 21360

ccaattcagt tgtcttccta ttctttattt gacatgagta aatttcccct taaattaagg 21420

ggtactgctg ttatgtcttt aaaagaaggt caaatcaatg atatgatttt atctcttctt 21480

agtaaaggta gacttataat tagagaaaac aacagagttg ttatttctag tgatgttctt 21540

gttaacaact aaacgaacaa tgtttgtttt tcttgtttta ttgccactag tctctagtca 21600

gtgtgttaat cttacaacca gaactcaatt accccctgca tacactaatt ctttcacacg 21660

tggtgtttat taccctgaca aagttttcag atcctcagtt ttacattcaa ctcaggactt 21720

gttcttacct ttcttttcca atgttacttg gttccatgct atacatgtct ctgggaccaa 21780

tggtactaag aggtttgcta accctgtcct accatttaat gatggtgttt attttgcttc 21840

cactgagaag tctaacataa taagaggctg gatttttggt actactttag attcgaagac 21900

ccagtcccta cttattgtta ataacgctac taatgttgtt attaaagtct gtgaatttca 21960

attttgtaat gatccatttt tgggtgttta ttaccacaaa aacaacaaaa gttggatgga 22020

aagtgagttc agagtttatt ctagtgcgaa taattgcact tttgaatatg tctctcagcc 22080

ttttcttatg gaccttgaag gaaaacaggg taatttcaaa aatcttaggg aatttgtgtt 22140

taagaatatt gatggttatt ttaaaatata ttctaagcac acgcctatta atttagtgcg 22200

tggtctccct cagggttttt cggctttaga accattggta gatttgccaa taggtattaa 22260

catcactagg tttcaaannn nnnctttaca tagaagttat ttgactcctg gtgattcttc 22320

ttcaggttgg acagctggtg ctgcagctta ttatgtgggt tatcttcaac ctaggacttt 22380

tctattaaaa tataatgaaa atggaaccat tacagatgct gtagactgtg cacttgaccc 22440

tctctcagaa acaaagtgta cgttgaaatc cttcactgta gaaaaaggaa tctatcaaac 22500

ttctaacttt agagtccaac caacagaatc tattgttaga tttcctaata ttacaaactt 22560

gtgccctttt ggtgaagttt ttaacgccac cagatttgca tctgtttatg cttggaacag 22620

gaagagaatc agcaactgtg ttgctgatta ttctgtccta tataattccg catcattttc 22680

cacttttaag tgttatggag tgtctcctac taaattaaat gatctctgct ttactaatgt 22740

ctatgcagat tcatttgtaa ttagaggtga tgaagtcaga caaatcgctc cagggcaaac 22800

tggaaatatt gctgattata attataaatt accagatgat tttacaggct gcgttatagc 22860

ttggaattct aacaatcttg attctaaggt tggtggtaat tataattacc tgtatagatt 22920

gtttaggaag tctaatctca aaccttttga gagagatatt tcaactgaaa tctatcaggc 22980

cggtagcaca ccttgtaatg gtgttaaagg ttttaattgt tactttcctt tacaatcata 23040

tggtttccaa cccacttatg gtgttggtta ccaaccatac agagtagtag tactttcttt 23100

tgaacttcta catgcaccag caactgtttg tggacctaaa aagtctacta atttggttaa 23160

aaacaaatgt gtcaatttca acttcaatgg tttaacaggc acaggtgttc ttactgagtc 23220

taacaaaaag tttctgcctt tccaacaatt tggcagagac attgctgaca ctactgatgc 23280

tgtccgtgat ccacagacac ttgagattct tgacattaca ccatgttctt ttggtggtgt 23340

cagtgttata acaccaggaa caaatacttc taaccaggtt gctgttcttt atcagggtgt 23400

taactgcaca gaagtccctg ttgctattca tgcagatcaa cttactccta cttggcgtgt 23460

ttattctaca ggttctaatg tttttcaaac acgtgcaggc tgtttaatag gggctgaaca 23520

tgtcaacaac tcatatgagt gtgacatacc cattggtgca ggtatatgcg ctagttatca 23580

gactcagact aattctcctc ggcgggcacg tagtgtagct agtcaatcca tcattgccta 23640

cactatgtca cttggtgtag aaaattcagt tgcttactct aataactcta ttgccatacc 23700

cacaaatttt actattagtg ttaccacaga aattctacca gtgtctatga ccaagacatc 23760

agtagattgt acaatgtaca tttgtggtga ttcaactgaa tgcagcaatc ttttgttgca 23820

atatggcagt ttttgtacac aattaaaccg tgctttaact ggaatagctg ttgaacaaga 23880

caaaaacacc caagaagttt ttgcacaagt caaacaaatt tacaaaacac caccaattaa 23940

agattttggt ggttttaatt tttcacaaat attaccagat ccatcaaaac caagcaagag 24000

gtcatttatt gaagatctac ttttcaacaa agtgacactt gcagatgctg gcttcatcaa 24060

acaatatggt gattgccttg gtgatattgc tgctagagac ctcatttgtg cacaaaagtt 24120

taacggcctt actgttttgc cacctttgct cacagatgaa atgattgctc aatacacttc 24180

tgcactgtta gcgggtacaa tcacttctgg ttggaccttt ggtgcaggtg ctgcattaca 24240

aataccattt gctatgcaaa tggcttatag gtttaatggt attggagtta cacagaatgt 24300

tctctatgag aaccaaaaat tgattgccaa ccaatttaat agtgctattg gcaaaattca 24360

agactcactt tcttccacag caagtgcact tggaaaactt caagatgtgg tcaaccaaaa 24420

tgcacaagct ttaaacacgc ttgttaaaca acttagctcc aattttggtg caatttcaag 24480

tgttttaaat gatatccttt cacgtcttga caaagttgag gctgaagtgc aaattgatag 24540

gttgatcaca ggcagacttc aaagtttgca gacatatgtg actcaacaat taattagagc 24600

tgcagaaatc agagcttctg ctaatcttgc tgctactaaa atgtcagagt gtgtacttgg 24660

acaatcaaaa agagttgatt tttgtggaaa gggctatcat cttatgtcct tccctcagtc 24720

agcacctcat ggtgtagtct tcttgcatgt gacttatgtc cctgcacaag aaaagaactt 24780

cacaactgct cctgccattt gtcatgatgg aaaagcacac tttcctcgtg aaggtgtctt 24840

tgtttcaaat ggcacacact ggtttgtaac acaaaggaat ttttatgaac cacaaatcat 24900

tactacagac aacacatttg tgtctggtaa ctgtgatgtt gtaataggaa ttgtcaacaa 24960

cacagtttat gatcctttgc aacctgaatt agactcattc aaggaggagt tagataaata 25020

ttttaagaat catacatcac cagatgttga tttaggtgac atctctggca ttaatgcttc 25080

agttgtaaac attcaaaaag aaattgaccg cctcaatgag gttgccaaga atttaaatga 25140

atctctcatc gatctccaag aacttggaaa gtatgagcag tatataaaat ggccatggta 25200

catttggcta ggttttatag ctggcttgat tgccatagta atggtgacaa ttatgctttg 25260

ctgtatgacc agttgctgta gttgtctcaa gggctgttgt tcttgtggat cctgctgcaa 25320

atttgatgaa gacgactctg agccagtgct caaaggagtc aaattacatt acacataaac 25380

gaacttatgg atttgtttat gagaatcttc acaattggaa ctgtaacttt gaagcaaggt 25440

gaaatcaagg atgctactcc ttcagatttt gttcgcgcta ctgcaacgat accgatacaa 25500

gcctcactcc ctttcggatg gcttattgtt ggcgttgcac ttcttgctgt ttttcatagc 25560

gcttccaaaa tcataaccct caaaaagaga tggcaactag cactctccaa gggtgttcac 25620

tttgtttgca acttgctgtt gttgtttgta acagtttact cacacctttt gctcgttgct 25680

gctggccttg aagccccttt tctctatctt tatgctttag tctacttctt gcagagtata 25740

aactttgtaa gaataataat gaggctttgg ctttgctgga aatgccgttc caaaaaccca 25800

ttactttatg atgccaacta ttttctttgc tggcatacta attgttacga ctattgtata 25860

ccttacaata gtgtaacttc ttcaattgtc attactttag gtgatggcac aacaagtcct 25920

atttctgaac atgactacca gattggtggt tatactgaaa aatgggaatc tggagtaaaa 25980

gactgtgttg tattacacag ttacttcact tcagactatt accagctgta ctcaactcaa 26040

ttgagtacag acactggtgt tgaacatgtt accttcttca tctacaataa aattgttgat 26100

gagcctgaag aacatgtcca aattcacaca atcgacggtt catccggagt tgttaatcca 26160

gtaatggaac caatttatga tgaaccgacg acgactacta gcgtgccttt gtaagcacaa 26220

gctgatgagt acgaacttat gtactcattc gtttcggaag agacaggtac gttaatagtt 26280

aatagcgtac ttctttttct tgctttcgtg gtattcttgc tagttacact agccatcctt 26340

actgcgcttc gattgtgtgc gtactgctgc aatattgtta acgtgagtct tgtaaaacct 26400

tctttttacg tttactctcg tgttaaaaat ctgaattctt ctagagttct tgatcttctg 26460

gtctaaacga actaaatatt atattagttt ttctgtttgg aactttaatt ttagccatgg 26520

cagattccaa cggtactatt accgttgaag agcttaaaaa gctccttgaa caatggaacc 26580

tagtaatagg tttcctattc cttacatgga tttgtcttct acaatttgcc tatgccaaca 26640

ggaataggtt tttgtatata attaagttaa ttttcctctg gctgttatgg ccagtaactt 26700

tagcttgttt tgtgcttgct gctgtttaca gaataaattg gatcaccggt ggaattgcta 26760

tcgcaatggc ttgtcttgta ggcttgatgt ggctcagcta cttcattgct tctttcagac 26820

tgtttgcgcg tacgcgttcc atgtggtcat tcaatccaga aactaacatt cttctcaacg 26880

tgccactcca tggcactatt ctgaccagac cgcttctaga aagtgaactc gtaatcggag 26940

ctgtgatcct tcgtggacat cttcgtattg ctggacacca tctaggacgc tgtgacatca 27000

aggacctgcc taaagaaatc actgttgcta catcacgaac gctttcttat tacaaattgg 27060

gagcttcgca gcgtgtagca ggtgactcag gttttgctgc atacagtcgc tacaggattg 27120

gcaactataa attaaacaca gaccattcca gtagcagtga caatattgct ttgcttgtac 27180

agtaagcgac aacagatgtt tcatctcgtt gactttcagg ttactatagc agagatatta 27240

ctaattatta tgaggacttt taaagtttcc atttggaatc ttgattacat cataaacctc 27300

ataattaaaa atttatctaa gtcactaact gagaataaat attctcaatt agatgaagag 27360

caaccaatgg agattgatta aacgaacatg aaaattattc ttttcttggc actgataaca 27420

ctcgctactt gtgagcttta tcactaccaa gagtgtgtta gaggtacaac agtactttta 27480

aaagaacctt gctcttctgg aacatacgag ggcaattcac catttcatcc tctagctgat 27540

aacaaatttg cactgacttg ctttagcact caatttgctt ttgcttgtcc tgacggcgta 27600

aaacacgtct atcagttacg tgccagatca gtttcaccta aactgttcat cagacaagag 27660

gaagttcaag aactttactc tccaattttt cttattgttg cggcaatagt gtttataaca 27720

ctttgcttca cactcaaaag aaagacagaa tgattgaact ttcattaatt gacttctatt 27780

tgtgcttttt agcctttctg ctattccttg ttttaattat gcttattatc ttttggttct 27840

cacttgaact gcaagatcat aatgaaactt gtcacgccta aacgaacatg aaatttcttg 27900

ttttcttagg aatcatcaca actgtagctg catttcacca agaatgtagt ttacagtcat 27960

gtactcaaca tcaaccatat gtagttgatg acccgtgtcc tattcacttc tattctaaat 28020

ggtatattag agtaggagct agaaaatcag cacctttaat tgaattgtgc gtggatgagg 28080

ctggttctaa atcacccatt cagtacatcg atatcggtaa ttatacagtt tcctgtttac 28140

cttttacaat taattgccag gaacctaaat tgggtagtct tgtagtgcgt tgttcgttct 28200

atgaagactt tttagagtat catgacgttc gtgttgtttt agattttatc taaacgaaca 28260

aactaaaatg tctgataatg gaccccaaaa tcagcgaaat gcaccccgca ttacgtttgg 28320

tggaccctca gattcaactg gcagtaacca gaatggagaa cgcagtgggg cgcgatcaaa 28380

acaacgtcgg ccccaaggtt tacccaataa tactgcgtct tggttcaccg ctctcactca 28440

acatggcaag gaagacctta aattccctcg aggacaaggc gttccaatta acaccaatag 28500

cagtccagat gaccaaattg gctactaccg aagagctacc agacgaattc gtggtggtga 28560

cggtaaaatg aaagatctca gtccaagatg gtatttctac tacctaggaa ctgggccaga 28620

agctggactt ccctatggtg ctaacaaaga cggcatcata tgggttgcaa ctgagggagc 28680

cttgaataca ccaaaagatc acattggcac ccgcaatcct gctaacaatg ctgcaatcgt 28740

gctacaactt cctcaaggaa caacattgcc aaaaggcttc tacgcagaag ggagcagagg 28800

cggcagtcaa gcctcttctc gttcctcatc acgtagtcgc aacagttcaa gaaattcaac 28860

tccaggcagc agtaggggaa tttctcctgc tagaatggct ggcaatggcg gtgatgctgc 28920

tcttgctttg ctgctgcttg acagattgaa ccagcttgag agcaaaatgt ctggtaaagg 28980

ccaacaacaa caaggccaaa ctgtcactaa gaaatctgct gctgaggctt ctaagaagcc 29040

tcggcaaaaa cgtactgcca ctaaagcata caatgtaaca caagctttcg gcagacgtgg 29100

tccagaacaa acccaaggaa attttgggga ccaggaacta atcagacaag gaactgatta 29160

caaacattgg ccgcaaattg cacaatttgc ccccagcgct tcagcgttct tcggaatgtc 29220

gcgcattggc atggaagtca caccttcggg aacgtggttg acctacacag gtgccatcaa 29280

attggatgac aaagatccaa atttcaaaga tcaagtcatt ttgctgaata agcatattga 29340

cgcatacaaa acattcccac caacagagcc taaaaaggac aaaaagaaga aggctgatga 29400

aactcaagcc ttaccgcaga gacagaagaa acagcaaact gtgactcttc ttcctgctgc 29460

agatttggat gatttctcca aacaattgca acaatccatg agcagtgctg actcaactca 29520

ggcctaaact catgcagacc acacaaggca gatgggctat ataaacgttt tcgcttttcc 29580

gtttacgata tatagtctac tcttgtgcag aatgaattct cgtaactaca tagcacaagt 29640

agatgtagtt aactttaatc tcacatagca atctttaatc agtgtgtaac attagggagg 29700

acttgaaaga gccaccacat tttcaccgag gccacgcgga gtacgatcga gtgtacagtg 29760

aacaatgcta gggagagctg cctatatgga agagccctaa tgtgtaaaat taattttagt 29820

agtgctatcc ccatgnnnnn nnnnnnnnnn nnnnnnnnnn nnnnaaaaaa aaaaaaaaaa 29880

aaaaaaaaaa aaaaaaa 29897

<210> 19
<211> 1272
<212> PRT
<213> SARS-CoV2

<220>
<221>  misc_feature
<222>  (240)..(242)
<223>  Xaa can be any naturally occurring amino acid

<400>  19

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Ala
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Gly Leu Pro Gln Gly Phe Ser Ala Leu Glu
    210                 215                 220

Pro Leu Val Asp Leu Pro Ile Gly Ile Asn Ile Thr Arg Phe Gln Xaa
225                 230                 235                 240

Xaa Xaa Leu His Arg Ser Tyr Leu Thr Pro Gly Asp Ser Ser Ser Gly
                245                 250                 255

Trp Thr Ala Gly Ala Ala Ala Tyr Tyr Val Gly Tyr Leu Gln Pro Arg
            260                 265                 270

Thr Phe Leu Leu Lys Tyr Asn Glu Asn Gly Thr Ile Thr Asp Ala Val
        275                 280                 285

Asp Cys Ala Leu Asp Pro Leu Ser Glu Thr Lys Cys Thr Leu Lys Ser
    290                 295                 300

Phe Thr Val Glu Lys Gly Ile Tyr Gln Thr Ser Asn Phe Arg Val Gln
305                 310                 315                 320

Pro Thr Glu Ser Ile Val Arg Phe Pro Asn Ile Thr Asn Leu Cys Pro
                325                 330                 335

Phe Gly Glu Val Phe Asn Ala Thr Arg Phe Ala Ser Val Tyr Ala Trp
            340                 345                 350

Asn Arg Lys Arg Ile Ser Asn Cys Val Ala Asp Tyr Ser Val Leu Tyr
        355                 360                 365

Asn Ser Ala Ser Phe Ser Thr Phe Lys Cys Tyr Gly Val Ser Pro Thr
    370                 375                 380

Lys Leu Asn Asp Leu Cys Phe Thr Asn Val Tyr Ala Asp Ser Phe Val
385                 390                 395                 400

Ile Arg Gly Asp Glu Val Arg Gln Ile Ala Pro Gly Gln Thr Gly Asn
                405                 410                 415

Ile Ala Asp Tyr Asn Tyr Lys Leu Pro Asp Asp Phe Thr Gly Cys Val
            420                 425                 430

Ile Ala Trp Asn Ser Asn Asn Leu Asp Ser Lys Val Gly Gly Asn Tyr
        435                 440                 445

Asn Tyr Leu Tyr Arg Leu Phe Arg Lys Ser Asn Leu Lys Pro Phe Glu
    450                 455                 460

Arg Asp Ile Ser Thr Glu Ile Tyr Gln Ala Gly Ser Thr Pro Cys Asn
465                 470                 475                 480

Gly Val Lys Gly Phe Asn Cys Tyr Phe Pro Leu Gln Ser Tyr Gly Phe
                485                 490                 495

Gln Pro Thr Tyr Gly Val Gly Tyr Gln Pro Tyr Arg Val Val Val Leu
            500                 505                 510

Ser Phe Glu Leu Leu His Ala Pro Ala Thr Val Cys Gly Pro Lys Lys
        515                 520                 525

Ser Thr Asn Leu Val Lys Asn Lys Cys Val Asn Phe Asn Phe Asn Gly
    530                 535                 540

Leu Thr Gly Thr Gly Val Leu Thr Glu Ser Asn Lys Lys Phe Leu Pro
545                 550                 555                 560

Phe Gln Gln Phe Gly Arg Asp Ile Ala Asp Thr Thr Asp Ala Val Arg
                565                 570                 575

Asp Pro Gln Thr Leu Glu Ile Leu Asp Ile Thr Pro Cys Ser Phe Gly
            580                 585                 590

Gly Val Ser Val Ile Thr Pro Gly Thr Asn Thr Ser Asn Gln Val Ala
        595                 600                 605

Val Leu Tyr Gln Gly Val Asn Cys Thr Glu Val Pro Val Ala Ile His
    610                 615                 620

Ala Asp Gln Leu Thr Pro Thr Trp Arg Val Tyr Ser Thr Gly Ser Asn
625                 630                 635                 640

Val Phe Gln Thr Arg Ala Gly Cys Leu Ile Gly Ala Glu His Val Asn
                645                 650                 655

Asn Ser Tyr Glu Cys Asp Ile Pro Ile Gly Ala Gly Ile Cys Ala Ser
            660                 665                 670

Tyr Gln Thr Gln Thr Asn Ser Pro Arg Arg Ala Arg Ser Val Ala Ser
        675                 680                 685

Gln Ser Ile Ile Ala Tyr Thr Met Ser Leu Gly Val Glu Asn Ser Val
    690                 695                 700

Ala Tyr Ser Asn Asn Ser Ile Ala Ile Pro Thr Asn Phe Thr Ile Ser
705                 710                 715                 720

Val Thr Thr Glu Ile Leu Pro Val Ser Met Thr Lys Thr Ser Val Asp
                725                 730                 735

Cys Thr Met Tyr Ile Cys Gly Asp Ser Thr Glu Cys Ser Asn Leu Leu
                740                 745                 750

Leu Gln Tyr Gly Ser Phe Cys Thr Gln Leu Asn Arg Ala Leu Thr Gly
            755                 760                 765

Ile Ala Val Glu Gln Asp Lys Asn Thr Gln Glu Val Phe Ala Gln Val
    770                 775                 780

Lys Gln Ile Tyr Lys Thr Pro Pro Ile Lys Asp Phe Gly Gly Phe Asn
785                 790                 795                 800

Phe Ser Gln Ile Leu Pro Asp Pro Ser Lys Pro Ser Lys Arg Ser Phe
                805                 810                 815

Ile Glu Asp Leu Leu Phe Asn Lys Val Thr Leu Ala Asp Ala Gly Phe
                820                 825                 830

Ile Lys Gln Tyr Gly Asp Cys Leu Gly Asp Ile Ala Ala Arg Asp Leu
            835                 840                 845

Ile Cys Ala Gln Lys Phe Asn Gly Leu Thr Val Leu Pro Pro Leu Leu
    850                 855                 860

Thr Asp Glu Met Ile Ala Gln Tyr Thr Ser Ala Leu Leu Ala Gly Thr
865                 870                 875                 880

Ile Thr Ser Gly Trp Thr Phe Gly Ala Gly Ala Ala Leu Gln Ile Pro
                885                 890                 895

Phe Ala Met Gln Met Ala Tyr Arg Phe Asn Gly Ile Gly Val Thr Gln
                900                 905                 910

Asn Val Leu Tyr Glu Asn Gln Lys Leu Ile Ala Asn Gln Phe Asn Ser
            915                 920                 925

Ala Ile Gly Lys Ile Gln Asp Ser Leu Ser Ser Thr Ala Ser Ala Leu
    930                 935                 940

Gly Lys Leu Gln Asp Val Val Asn Gln Asn Ala Gln Ala Leu Asn Thr
945                 950                 955                 960

Leu Val Lys Gln Leu Ser Ser Asn Phe Gly Ala Ile Ser Ser Val Leu
                965                 970                 975

Asn Asp Ile Leu Ser Arg Leu Asp Lys Val Glu Ala Glu Val Gln Ile

            980                 985                 990

Asp Arg Leu Ile Thr Gly Arg Leu Gln Ser Leu Gln Thr Tyr Val Thr
        995                 1000                1005

Gln Gln Leu Ile Arg Ala Ala Glu Ile Arg Ala Ser Ala Asn Leu
    1010                1015                1020

Ala Ala Thr Lys Met Ser Glu Cys Val Leu Gly Gln Ser Lys Arg
    1025                1030                1035

Val Asp Phe Cys Gly Lys Gly Tyr His Leu Met Ser Phe Pro Gln
    1040                1045                1050

Ser Ala Pro His Gly Val Val Phe Leu His Val Thr Tyr Val Pro
    1055                1060                1065

Ala Gln Glu Lys Asn Phe Thr Thr Ala Pro Ala Ile Cys His Asp
    1070                1075                1080

Gly Lys Ala His Phe Pro Arg Glu Gly Val Phe Val Ser Asn Gly
    1085                1090                1095

Thr His Trp Phe Val Thr Gln Arg Asn Phe Tyr Glu Pro Gln Ile
    1100                1105                1110

Ile Thr Thr Asp Asn Thr Phe Val Ser Gly Asn Cys Asp Val Val
    1115                1120                1125

Ile Gly Ile Val Asn Asn Thr Val Tyr Asp Pro Leu Gln Pro Glu
    1130                1135                1140

Leu Asp Ser Phe Lys Glu Glu Leu Asp Lys Tyr Phe Lys Asn His
    1145                1150                1155

Thr Ser Pro Asp Val Asp Leu Gly Asp Ile Ser Gly Ile Asn Ala
    1160                1165                1170

Ser Val Val Asn Ile Gln Lys Glu Ile Asp Arg Leu Asn Glu Val
    1175                1180                1185

Ala Lys Asn Leu Asn Glu Ser Leu Ile Asp Leu Gln Glu Leu Gly
    1190                1195                1200

Lys Tyr Glu Gln Tyr Ile Lys Trp Pro Trp Tyr Ile Trp Leu Gly
    1205                1210                1215

Phe Ile Ala Gly Leu Ile Ala Ile Val Met Val Thr Ile Met Leu
    1220                1225                1230

Cys Cys Met Thr Ser Cys Cys Ser Cys Leu Lys Gly Cys Cys Ser
    1235                1240                1245

Cys Gly Ser Cys Cys Lys Phe Asp Glu Asp Asp Ser Glu Pro Val
    1250                1255                1260

Leu Lys Gly Val Lys Leu His Tyr Thr
    1265                1270

<210> 20
<211> 29819
<212> DNA
<213> SARS-CoV2

<400> 20
caactttcga tctcttgtag atctgttctc taaacgaact ttaaaatctg tgtggctgtc 60

actcggctgc atgcttagtg cactcacgca gtataattaa taactaatta ctgtcgttga 120

caggacacga gtaactcgtc tatcttctgc aggctgctta cggtttcgtc cgtgttgcag 180

ccgatcatca gcacatctag gttttgtccg ggtgtgaccg aaaggtaaga tggagagcct 240

tgtccctggt ttcaacgaga aaacacacgt ccaactcagt ttgcctgttt tacaggttcg 300

cgacgtgctc gtacgtggct ttggagactc cgtggaggag gtcttatcag aggcacgtca 360

acatcttaaa gatggcactt gtggcttagt agaagttgaa aaaggcgttt tgcctcaact 420

tgaacagccc tatgtgttca tcaaacgttc ggatgctcga actgcacctc atggtcatgt 480

tatggttgag ctggtagcag aactcgaagg cattcagtac ggtcgtagtg gtgagacact 540

tggtgtcctt gtccctcatg tgggcgaaat accagtggct taccgcaagg ttcttcttcg 600

taagaacggt aataaaggag ctggtggcca tagttacggc gccgatctaa agtcatttga 660

cttaggcgac gagcttggca ctgatcctta tgaagacttt caagaaaact ggaacactaa 720

acatagcagt ggtgttaccc gtgaactcat gcgtgagctt aacggagggg catacactcg 780

ctatgtcgat aacaacttct gtggccctga tggctaccct cttgagtgca ttaaagacct 840

tctagcacgt gctggtaaag cttcatgcac tttgtccgaa caactggact ttattgacac 900

taagaggggt gtatactgct gccgtgaaca tgagcatgaa attgcttggt acacggaacg 960

ttctgaaaag agctatgaat tgcagacacc ttttgaaatt aaattggcaa agaaatttga 1020

caccttcaat ggggaatgtc caaattttgt atttccctta aattccataa tcaagactat 1080

tcaaccaagg gttgaaaaga aaaagcttga tggctttatg ggtagaattc gatctgtcta 1140

tccagttgcg tcaccaaatg aatgcaacca aatgtgcctt tcaactctca tgaagtgtga 1200

tcattgtggt gaaacttcat ggcagacggg cgattttgtt aaagccactt gcgaattttg 1260

tggcactgag aatttgacta aagaaggtgc cactacttgt ggttacttac cccaaaatgc 1320

tgttgttaaa atttattgtc cagcatgtca caattcagaa gtaggacctg agcatagtct 1380

tgccgaatac cataatgaat ctggcttgaa aaccattctt cgtaagggtg gtcgcactat 1440

tgcctttgga ggctgtgtgt tctcttatgt tggttgccat aacaagtgtg cctattgggt 1500

tccacgtgct agcgctaaca taggttgtaa ccatacaggt gttgttggag aaggttccga 1560

aggtcttaat gacaaccttc ttgaaatact ccaaaaagag aaagtcaaca tcaatattgt 1620

tggtgacttt aaacttaatg aagagatcgc cattattttg gcatcttttt ctgcttccac 1680

aagtgctttt gtggaaactg tgaaaggttt ggattataaa gcattcaaac aaattgttga 1740

atcctgtggt aattttaaag ttacaaaagg aaaagctaaa aaaggtgcct ggaatattgg 1800

tgaacagaaa tcaatactga gtcctcttta tgcatttgca tcagaggctg ctcgtgttgt 1860

acgatcaatt ttctcccgca ctcttgaaac tgctcaaaat tctgtgcgtg ttttacagaa 1920

ggccgctata acaatactag atggaatttc acagtattca ctgagactca ttgatgctat 1980

gatgttcaca tctgatttgg ctactaacaa tctagttgta atggcctaca ttacaggtgg 2040

tgttgttcag ttgacttcgc agtggctaac taacatcttt ggcactgttt atgaaaaact 2100

caaacccgtc cttgattggc ttgaagagaa gtttaaggaa ggtgtagagt ttcttagaga 2160

cggttgggaa attgttaaat ttatctcaac ctgtgcttgt gaaattgtcg gtggacaaat 2220

tgtcacctgt gcaaaggaaa ttaaggagag tgttcagaca ttctttaagc ttgtaaataa 2280

atttttggct ttgtgtgctg actctatcat tattggtgga gctaaactta aagccttgaa 2340

tttaggtgaa acatttgtca cgcactcaaa gggattgtac agaaagtgtg ttaaatccag 2400

agaagaaact ggcctactca tgcctctaaa agccccaaaa gaaattatct tcttagaggg 2460

agaaacactt cccacagaag tgttaacaga ggaagttgtc ttgaaaactg gtgatttaca 2520

accattagaa caacctacta gtgaagctgt tgaagctcca ttggttggta caccagtttg 2580

tattaacggg cttatgttgc tcgaaatcaa agacacagaa aagtactgtg cccttgcacc 2640

taatatgatg gtaacaaaca ataccttcac actcaaaggc ggtgcaccaa caaaggttac 2700

ttttggtgat gatactgtga tagaagtgca aggttacaag agtgtgaata tcacttttga 2760

acttgatgaa aggattgata aagtacttaa tgagaagtgc tctgcctata cagttgaact 2820

cggtacagaa gtaaatgagt tcgcctgtgt tgtggcagat gctgtcataa aaactttgca 2880

accagtatct gaattactta caccactggg cattgattta gatgagtgga gtatggctac 2940

atactactta tttgatgagt ctggtgagtt taaattggct tcacatatgt attgttcttt 3000

ttaccctcca gatgaggatg aagaagaagg tgattgtgaa gaagaagagt ttgagccatc 3060

aactcaatat gagtatggta ctgaagatga ttaccaaggt aaacctttgg aatttggtgc 3120

cacttctgct gctcttcaac ctgaagaaga gcaagaagaa gattggttag atgatgatag 3180

tcaacaaact gttggtcaac aagacggcag tgaggacaat cagacaacta ctattcaaac 3240

aattgttgag gttcaacctc aattagagat ggaacttaca ccagttgttc agactattga 3300

agtgaatagt tttagtggtt atttaaaact tactgacaat gtatacatta aaaatgcaga 3360

cattgtggaa gaagctaaaa aggtaaaacc aacagtggtt gttaatgcag ccaatgttta 3420

ccttaaacat ggaggaggtg ttgcaggagc cttaaataag gctactaaca atgccatgca 3480

agttgaatct gatgattaca tagctactaa tggaccactt aaagtgggtg gtagttgtgt 3540

tttaagcgga cacaatcttg ctaaacactg tcttcatgtt gtcggcccaa atgttaacaa 3600

aggtgaagac attcaacttc ttaagagtgc ttatgaaaat tttaatcagc acgaagttct 3660

acttgcacca ttattatcag ctggtatttt tggtgctgac cctatacatt ctttaagagt 3720

ttgtgtagat actgttcgca caaatgtcta cttagctgtc tttgataaaa atctctatga 3780

caaacttgtt ttaagctttt tggaaatgaa gagtgaaaag caagttgaac aaaagatcgc 3840

tgagattcct aaagaggaag ttaagccatt tataactgaa agtaaacctt cagttgaaca 3900

gagaaaacaa gatgataaga aaatcaaagc ttgtgttgaa gaagttacaa caactctgga 3960

agaaactaag ttcctcacag aaaacttgtt actttatatt gacattaatg gcaatcttca 4020

tccagattct gccactcttg ttagtgacat tgacatcact ttcttaaaga aagatgctcc 4080

atatatagtg ggtgatgttg ttcaagaggg tgttttaact gctgtggtta tacctactaa 4140

aaaggctggt ggcactactg aaatgctagc gaaagctttg agaaaagtgc caacagacaa 4200

ttatataacc acttacccgg gtcagggttt aaatggttac actgtagagg aggcaaagac 4260

agtgcttaaa aagtgtaaaa gtgcctttta cattctacca tctattatct ctaatgagaa 4320

gcaagaaatt cttggaactg tttcttggaa tttgcgagaa atgcttgcac atgcagaaga 4380

aacacgcaaa ttaatgcctg tctgtgtgga aactaaagcc atagtttcaa ctatacagcg 4440

taaatataag ggtattaaaa tacaagaggg tgtggttgat tatggtgcta gattttactt 4500

ttacaccagt aaaacaactg tagcgtcact tatcaacaca cttaacgatc taaatgaaac 4560

tcttgttaca atgccacttg gctatgtaac acatggctta aatttggaag aagctgctcg 4620

gtatatgaga tctctcaaag tgccagctac agtttctgtt tcttcacctg atgctgttac 4680

agcgtataat ggttatctta cttcttcttc taaaacacct gaagaacatt ttattgaaac 4740

catctcactt gctggttcct ataaagattg gtcctattct ggacaatcta cacaactagg 4800

tatagaattt cttaagagag gtgataaaag tgtatattac actagtaatc ctaccacatt 4860

ccacctagat ggtgaagtta tcacctttga caatcttaag acacttcttt ctttgagaga 4920

agtgaggact attaaggtgt ttacaacagt agacaacatt aacctccaca cgcaagttgt 4980

ggacatgtca atgacatatg gacaacagtt tggtccaact tatttggatg gagctgatgt 5040

tactaaaata aaacctcata attcacatga aggtaaaaca ttttatgttt tacctaatga 5100

tgacactcta cgtgttgagg cttttgagta ctaccacaca actgatccta gttttctggg 5160

taggtacatg tcagcattaa atcacactaa aaagtggaaa tacccacaag ttaatggttt 5220

aacttctatt aaatgggcag ataacaactg ttatcttgcc actgcattgt taacactcca 5280

acaaatagag ttgaagttta atccacctgc tctacaagat gcttattaca gagcaagggc 5340

tggtgaagct gctaactttt gtgcacttat cttagcctac tgtaataaga cagtaggtga 5400

gttaggtgat gttagagaaa caatgagtta cttgtttcaa catgccaatt tagattcttg 5460

caaaagagtc ttgaacgtgg tgtgtaaaac ttgtggacaa cagcagacaa cccttaaggg 5520

tgtagaagct gttatgtaca tgggcacact ttcttatgaa caatttaaga aaggtgttca 5580

gataccttgt acgtgtggta aacaagctac acaatatcta gtacaacagg agtcaccttt 5640

tgttatgatg tcagcaccac ctgctcagta tgaacttaag catggtacat ttacttgtgc 5700

tagtgagtac actggtaatt accagtgtgg tcactataaa catataactt ctaaagaaac 5760

tttgtattgc atagacggtg ctttacttac aaagtcctca gaatacaaag gtcctattac 5820

ggatgttttc tacaaagaaa acagttacac aacaaccata aaaccagtta cttataaatt 5880

ggatggtgtt gtttgtacag aaattgaccc taagttggac aattattata agaaagacaa 5940

ttcttatttc acagagcaac caattgatct tgtaccaaac caaccatatc caaacgcaag 6000

cttcgataat tttaagtttg tatgtgataa tatcaaattt gctgatgatt taaaccagtt 6060

aactggttat aagaaacctg cttcaagaga gcttaaagtt acatttttcc ctgacttaaa 6120

tggtgatgtg gtggctattg attataaaca ctacacaccc tcttttaaga aaggagctaa 6180

attgttacat aaacctattg tttggcatgt taacaatgca actaataaag ccacgtataa 6240

accaaatacc tggtgtatac gttgtctttg gagcacaaaa ccggttgaaa catcaaattc 6300

gtttgatgta ctgaagtcag aggacgcgca gggaatggat aatcttgcct gcgaagatct 6360

aaaaccagtc tctgaagaag tagtggaaaa tcctaccata cagaaagacg ttcttgagtg 6420

taatgtgaaa actaccgaag ttgtaggaga cattatactt aaaccagcaa ataatagttt 6480

aaaaattaca gaagaggttg gccacacaga tctaatggct gcttatgtag acaattctag 6540

tcttactatt aagaaaccta atgaattatc tagagtgtta ggtttgaaaa cccttgctac 6600

tcatggttta gctgctgtta atagtgtccc ttgggatact atagctaatt atgctaagcc 6660

ttttcttaac aaagttgtta gtacaactac taacatagtt acacggtgtt taaaccgtgt 6720

ttgtactaat tatatgcctt atttctttac tttattgcta caattgtgta cttttactag 6780

aagtacaaat tctagaatta aagcatctat gccgactact atagcaaaga atactgttaa 6840

gagtgtcggt aaattttgtc tagaggcttc atttaattat ttgaagtcac ctaatttttc 6900

taaactgata aatattataa tttggttttt actattaagt gtttgcctag gttctttaat 6960

ctactcaacc gctgctttag gtgttttaat gtctaattta ggcatgcctt cttactgtac 7020

tggttacaga gaaggctatt tgaactctac taatgtcact attgcaacct actgtactgg 7080

ttctatacct tgtagtgttt gtcttagtgg tttagattct ttagacacct atccttcttt 7140

agaaactata caaattacca tttcatcttt taaatgggat ttaactgctt ttggcttagt 7200

tgcagagtgg tttttggcat atattctttt cactaggttt ttctatgtac ttggattggc 7260

tgcaatcatg caattgtttt tcagctattt tgcagtacat tttattagta attcttggct 7320

tatgtggtta ataattaatc ttgtacaaat ggccccgatt tcagctatgg ttagaatgta 7380

catcttcttt gcatcatttt attatgtatg gaaaagttat gtgcatgttg tagacggttg 7440

taattcatca acttgtatga tgtgttacaa acgtaataga gcaacaagag tcgaatgtac 7500

aactattgtt aatggtgtta gaaggtcctt ttatgtctat gctaatggag gtaaaggctt 7560

ttgcaaacta cacaattgga attgtgttaa ttgtgataca ttctgtgctg gtagtacatt 7620

tattagtgat gaagttgcga gagacttgtc actacagttt aaaagaccaa taaatcctac 7680

tgaccagtct tcttacatcg ttgatagtgt tacagtgaag aatggttcca tccatcttta 7740

ctttgataaa gctggtcaaa agacttatga aagacattct ctctctcatt ttgttaactt 7800

agacaacctg agagctaata acactaaagg ttcattgcct attaatgtta tagtttttga 7860

tggtaaatca aaatgtgaag aatcatctgc aaaatcagcg tctgtttact acagtcagct 7920

tatgtgtcaa cctatactgt tactagatca ggcattagtg tctgatgttg gtgatagtgc 7980

ggaagttgca gttaaaatgt ttgatgctta cgttaatacg ttttcatcaa cttttaacgt 8040

accaatggaa aaactcaaaa cactagttgc aactgcagaa gctgaacttg caaagaatgt 8100

gtccttagac aatgtcttat ctacttttat ttcagcagct cggcaagggt ttgttgattc 8160

agatgtagaa actaaagatg ttgttgaatg tcttaaattg tcacatcaat ctgacataga 8220

agttactggc gatagttgta ataactatat gctcacctat aacaaagttg aaaacatgac 8280

accccgtgac cttggtgctt gtattgactg tagtgcgcgt catattaatg cgcaggtagc 8340

aaaaagtcac aacattgctt tgatatggaa cgttaaagat ttcatgtcat tgtctgaaca 8400

actacgaaaa caaatacgta gtgctgctaa aaagaataac ttacctttta agttgacatg 8460

tgcaactact agacaagttg ttaatgttgt aacaacaaag atagcactta agggtggtaa 8520

aattgttaat aattggttga agcagttaat taaagttaca cttgtgttcc tttttgttgc 8580

tgctattttc tatttaataa cacctgttca tgtcatgtct aaacatactg acttttcaag 8640

tgaaatcata ggatacaagg ctattgatgg tggtgtcact cgtgacatag catctacaga 8700

tacttgtttt gctaacaaac atgctgattt tgacacatgg tttagccagc gtggtggtag 8760

ttatactaat gacaaagctt gcccattgat tgctgcagtc ataacaagag aagtgggttt 8820

tgtcgtgcct ggtttgcctg gcacgatatt acgcacaact aatggtgact ttttgcattt 8880

cttacctaga gtttttagtg cagttggtaa catctgttac acaccatcaa aacttataga 8940

gtacactgac tttgcaacat cagcttgtgt tttggctgct gaatgtacaa tttttaaaga 9000

tgcttctggt aagccagtac catattgtta tgataccaat gtactagaag gttctgttgc 9060

ttatgaaaat ttacgccctg acacacgtta tgtgctcatg gatggctcta ttattcaatt 9120

tcctaacacc taccttgaag gttctgttag agtggtaaca acttttgatt ctgagtactg 9180

taggcacggc acttgtgaaa gatcagaagc tggtgtttgt gtatctacta gtggtagatg 9240

ggtacttaac aatgattatt acagatcttt accaggagtt ttctgtggtg tagatgctgt 9300

aaatttactt actaatatgt ttacaccact aattcaacct attggtgctt tggacatatc 9360

agcatctata gtagctggtg gtattgtagc tatcgtagta acatgccttg cctactattt 9420

tatgaggttt agaagagctt ttggtgaata cagtcatgta gttgccttta atactttact 9480

attccttatg tcattcactg tactctgttt aacaccagtt tactcattct tacctggtgt 9540

ttattctgtt atttacttgt acttgacatt ttatcttact aatgatgttt cttttttagc 9600

acatattcag tggatggtta tgttcacacc tttagtacct ttctggataa caattgctta 9660

tatcatttgt atttccacaa agcatttcta ttggttcttt agtaattacc taaagagacg 9720

tgtagtcttt aatggtgttt cctttagtac ttttgaagaa gctgcgctgt gcaccttttt 9780

gttaaataaa gaaatgtatc taaagttgcg tagtgatgtg ctattacctc ttacgcaata 9840

taatagatac ttagctcttt ataataagta caagtatttt agtggagcaa tggatacaac 9900

tagctacaga gaagctgctt gttgtcatct cgcaaaggct ctcaatgact tcagtaactc 9960

aggttctgat gttctttacc aaccaccaca aacctctatc acctcagctg ttttgcagag 10020

tggttttaga aaaatggcat tcccatctgg taaagttgag ggttgtatgg tacaagtaac 10080

ttgtggtaca actacactta acggtctttg gcttgatgac gtagtttact gtccaagaca 10140

tgtgatctgc acctctgaag acatgcttaa ccctaattat gaagatttac tcattcgtaa 10200

gtctaatcat aatttcttgg tacaggctgg taatgttcaa ctcagggtta ttggacattc 10260

tatgcaaaat tgtgtactta agcttaaggt tgatacagcc aatcctaaga cacctaagta 10320

taagtttgtt cgcattcaac caggacagac tttttcagtg ttagcttgtt acaatggttc 10380

accatctggt gtttaccaat gtgctatgag gcccaatttc actattaagg gttcattcct 10440

taatggttca tgtggtagtg ttggttttaa catagattat gactgtgtct ctttttgtta 10500

catgcaccat atggaattac caactggagt tcatgctggc acagacttag aaggtaactt 10560

ttatggacct tttgttgaca ggcaaacagc acaagcagct ggtacggaca caactattac 10620

agttaatgtt ttagcttggt tgtacgctgc tgttataaat ggagacaggt ggtttctcaa 10680

tcgatttacc acaactctta atgactttaa ccttgtggct atgaagtaca attatgaacc 10740

tctaacacaa gaccatgttg acatactagg acctctttct gctcaaactg gaattgtcgt 10800

tttagatatg tgtgcttcat taaaagaatt actgcaaaat ggtatgaatg gacgtaccat 10860

attgggtagt gctttattag aagatgaatt tacacctttt gatgttgtta gacaatgctc 10920

aggtgttact ttccaaagtg cagtgaaaag aacaatcaag ggtacacacc actggttgtt 10980

actcacaatt ttgacttcac ttttagtttt agtccagagt actcaatggt ctttgttctt 11040

ttttttgtat gaaaatgcct ttttaccttt tgctatgggt attattgcta tgtctgcttt 11100

tgcaatgatg tttgtcaaac ataagcatgc atttctctgt ttgtttttgt taccttctct 11160

tgccactgta gcttatttta atatggtcta tatgcctgct agttgggtga tgcgtattat 11220

gacatggttg gatatggttg atactagttt gaagctaaaa gactgtgtta tgtatgcatc 11280

agctgtagtg ttactaatcc ttatgacagc aagaactgtg tatgatgatg gtgctaggag 11340

agtgtggaca cttatgaatg tcttgacact cgtttataaa gtttattatg gtaatgcttt 11400

agatcaagcc atttccatgt gggctcttat aatctctgtt acttctaact actcaggtgt 11460

agttacaact gtcatgtttt tggccagagg tattgttttt atgtgtgttg agtattgccc 11520

tattttcttc ataactggta atacacttca gtgtataatg ctagtttatt gtttcttagg 11580

ctatttttgt acttgttact ttggcctctt ttgtttactc aaccgctact ttagactgac 11640

tcttggtgtt tatgattact tagtttctac acaggagttt agatatatga attcacaggg 11700

actactccca cccaagaata gcatagatgc cttcaaactc aacattaaat tgttgggtgt 11760

tggtggcaaa ccttgtatca aagtagccac tgtacagtct aaaatgtcag atgtaaagtg 11820

cacatcagta gtcttactct cagttttgca acaactcaga gtagaatcat catctaaatt 11880

gtgggctcaa tgtgtccagt tacacaatga cattctctta gctaaagata ctactgaagc 11940

ctttgaaaaa atggtttcac tactttctgt tttgctttcc atgcagggtg ctgtagacat 12000

aaacaagctt tgtgaagaaa tgctggacaa cagggcaacc ttacaagcta tagcctcaga 12060

gtttagttcc cttccatcat atgcagcttt tgctactgct caagaagctt atgagcaggc 12120

tgttgctaat ggtgattctg aagttgttct taaaaagttg aagaagtctt tgaatgtggc 12180

taaatctgaa tttgaccgtg atgcagccat gcaacgtaag ttggaaaaga tggctgatca 12240

agctatgacc caaatgtata aacaggctag atctgaggac aagagggcaa aagttactag 12300

tgctatgcag acaatgcttt tcactatgct tagaaagttg gataatgatg cactcaacaa 12360

cattatcaac aatgcaagag atggttgtgt tcccttgaac ataatacctc ttacaacagc 12420

agccaaacta atggttgtca taccagacta taacacatat aaaaatacgt gtgatggtac 12480

aacatttact tatgcatcag cattgtggga aatccaacag gttgtagatg cagatagtaa 12540

aattgttcaa cttagtgaaa ttagtatgga caattcacct aatttagcat ggcctcttat 12600

tgtaacagct ttaagggcca attctgctgt caaattacag aataatgagc ttagtcctgt 12660

tgcactacga cagatgtctt gtgctgccgg tactacacaa actgcttgca ctgatgacaa 12720

tgcgttagct tattacaaca caacaaaggg aggtaggttt gtacttgcac tgttatccga 12780

tttacaggat ttgaaatggg ctagattccc taagagtgat ggaactggta ctatctatac 12840

agaactggaa ccaccttgta ggtttgttac agacacacct aaaggtccta aagtgaagta 12900

tttatacttt attaaaggat taaacaacct aaatagaggt atggtacttg gtagtttagc 12960

tgccacagta cgtctacaag ctggtaatgc aacagaagtg cctgccaatt caactgtatt 13020

atctttctgt gcttttgctg tagatgctgc taaagcttac aaagattatc tagctagtgg 13080

gggacaacca atcactaatt gtgttaagat gttgtgtaca cacactggta ctggtcaggc 13140

aataacagtt acaccggaag ccaatatgga tcaagaatcc tttggtggtg catcgtgttg 13200

tctgtactgc cgttgccaca tagatcatcc aaatcctaaa ggattttgtg acttaaaagg 13260

taagtatgta caaataccta caacttgtgc taatgaccct gtgggtttta cacttaaaaa 13320

cacagtctgt accgtctgcg gtatgtggaa aggttatggc tgtagttgtg atcaactccg 13380

cgaacccatg cttcagtcag ctgatgcaca atcgttttta aacgggtttg cggtgtaagt 13440

gcagcccgtc ttacaccgtg cggcacaggc actagtactg atgtcgtata cagggctttt 13500

gacatctaca atgataaagt agctggtttt gctaaattcc taaaaactaa ttgttgtcgc 13560

ttccaagaaa aggacgaaga tgacaattta attgattctt actttgtagt taagagacac 13620

actttctcta actaccaaca tgaagaaaca atttataatt tacttaagga ttgtccagct 13680

gttgctaaac atgacttctt taagtttaga atagacggtg acatggtacc acatatatca 13740

cgtcaacgtc ttactaaata cacaatggca gacctcgtct atgctttaag gcattttgat 13800

gaaggtaatt gtgatacatt aaaagaaata cttgtcacat acaattgttg tgatgatgat 13860

tatttcaata aaaaggactg gtatgatttt gtagaaaacc cagatatatt acgcgtatac 13920

gccaacttag gtgaacgtgt acgccaagct ttgttaaaaa cagtacaatt ctgtgatgcc 13980

atgcgaaatg ctggtattgt tggtgtactg acattagata atcaagatct caatggtaac 14040

tggtatgatt tcggtgattt catacaaacc acgccaggta gtggagttcc tgttgtagat 14100

tcttattatt cattgttaat gcctatatta accttgacca gggctttaac tgcagagtca 14160

catgttgaca ctgacttaac aaagccttac attaagtggg atttgttaaa atatgacttc 14220

acggaagaga ggttaaaact ctttgaccgt tattttaaat attgggatca gacataccac 14280

ccaaattgtg ttaactgttt ggatgacaga tgcattctgc attgtgcaaa ctttaatgtt 14340

ttattctcta cagtgttccc acttacaagt tttggaccac tagtgagaaa aatatttgtt 14400

gatggtgttc catttgtagt ttcaactgga taccacttca gagagctagg tgttgtacat 14460

aatcaggatg taaacttaca tagctctaga cttagtttta aggaattact tgtgtatgct 14520

gctgaccctg ctatgcacgc tgcttctggt aatctattac tagataaacg cactacgtgc 14580

ttttcagtag ctgcacttac taacaatgtt gcttttcaaa ctgtcaaacc cggtaatttt 14640

aacaaagact tctatgactt tgctgtgtct aagggtttct ttaaggaagg aagttctgtt 14700

gaattaaaac acttcttctt tgctcaggat ggtaatgctg ctatcagcga ttatgactac 14760

tatcgttata atctaccaac aatgtgtgat atcagacaac tactatttgt agttgaagtt 14820

gttgataagt actttgattg ttacgatggt ggctgtatta atgctaacca agtcatcgtc 14880

aacaacctag acaaatcagc tggttttcca tttaataaat ggggtaaggc tagactttat 14940

tatgattcaa tgagttatga ggatcaagat gcacttttcg catatacaaa acgtaatgtc 15000

atccctacta taactcaaat gaatcttaag tatgccatta gtgcaaagaa tagagctcgc 15060

accgtagctg gtgtctctat ctgtagtact atgaccaata gacagtttca tcaaaaatta 15120

ttgaaatcaa tagccgccac tagaggagct actgtagtaa ttggaacaag caaattctat 15180

ggtggttggc acaacatgtt aaaaactgtt tatagtgatg tagaaaaccc tcaccttatg 15240

ggttgggatt atcctaaatg tgatagagcc atgcctaaca tgcttagaat tatggcctca 15300

cttgttcttg ctcgcaaaca tacaacgtgt tgtagcttgt cacaccgttt ctatagatta 15360

gctaatgagt gtgctcaagt attgagtgaa atggtcatgt gtggcggttc actatatgtt 15420

aaaccaggtg gaacctcatc aggagatgcc acaactgctt atgctaatag tgtttttaac 15480

atttgtcaag ctgtcacggc caatgttaat gcacttttat ctactgatgg taacaaaatt 15540

gccgataagt atgtccgcaa tttacaacac agactttatg agtgtctcta tagaaataga 15600

gatgttgaca cagactttgt gaatgagttt tacgcatatt tgcgtaaaca tttctcaatg 15660

atgatactct ctgacgatgc tgttgtgtgt ttcaatagca cttatgcatc tcaaggtcta 15720

gtggctagca taaagaactt taagtcagtt ctttattatc aaaacaatgt ttttatgtct 15780

gaagcaaaat gttggactga gactgacctt actaaaggac ctcatgaatt ttgctctcaa 15840

catacaatgc tagttaaaca gggtgatgat tatgtgtacc ttccttaccc agatccatca 15900

agaatcctag gggccggctg ttttgtagat gatatcgtaa aaacagatgg tacacttatg 15960

attgaacggt tcgtgtcttt agctatagat gcttacccac ttactaaaca tcctaatcag 16020

gagtatgctg atgtctttca tttgtactta caatacataa gaaagctaca tgatgagtta 16080

acaggacaca tgttagacat gtattctgtt atgcttacta atgataacac ttcaaggtat 16140

tgggaacctg agttttatga ggctatgtac acaccgcata cagtcttaca ggctgttggg 16200

gcttgtgttc tttgcaattc acagacttca ttaagatgtg gtgcttgcat acgtagacca 16260

ttcttatgtt gtaaatgctg ttacgaccat gtcatatcaa catcacataa attagtcttg 16320

tctgttaatc cgtatgtttg caatgctcca ggttgtgatg tcacagatgt gactcaactt 16380

tacttaggag gtatgagcta ttattgtaaa tcacataaac cacccattag ttttccattg 16440

tgtgctaatg gacaagtttt tggtttatat aaaaatacat gtgttggtag cgataatgtt 16500

actgacttta atgcaattgc aacatgtgac tggacaaatg ctggtgatta cattttagct 16560

aacacctgta ctgaaagact caagcttttt gcagcagaaa cgctcaaagc tactgaggag 16620

acatttaaac tgtcttatgg tattgctact gtacgtgaag tgctgtctga cagagaatta 16680

catctttcat gggaagttgg taaacctaga ccaccactta accgaaatta tgtctttact 16740

ggttatcgtg taactaaaaa cagtaaagta caaataggag agtacacctt tgaaaaaggt 16800

gactatggtg atgctgttgt ttaccgaggt acaacaactt acaaattaaa tgttggtgat 16860

tattttgtgc tgacatcaca tacagtaatg ccattaagtg cacctacact agtgccacaa 16920

gagcactatg ttagaattac tggcttatac ccaacactca atatctcaga tgagttttct 16980

agcaatgttg caaattatca aaaggttggt atgcaaaagt attctacact ccagggacca 17040

cctggtactg gtaagagtca ttttgctatt ggcctagctc tctactaccc ttctgctcgc 17100

atagtgtata cagcttgctc tcatgccgct gttgatgcac tatgtgagaa ggcattaaaa 17160

tatttgccta tagataaatg tagtagaatt atacctgcac gtgctcgtgt agattgtttt 17220

gataaattca aagtgaattc aacattagaa cagtatgtct tttgtactgt aaatgcattg 17280

cctgagacga cagcagatat agttgtcttt gatgaaattt caatggccac aaattatgat 17340

ttgagtgttg tcaatgccag attacgtgct aagcactatg tgtacattgg cgaccctgct 17400

caattacctg caccacgcac attgctaact aagggcacac tagaaccaga atatttcaat 17460

tcagtgtgta gacttatgaa aactataggt ccagacatgt tcctcggaac ttgtcggcgt 17520

tgtcctgctg aaattgttga cactgtgagt gctttggttt atgataataa gcttaaagca 17580

cataaagaca aatcagctca atgctttaaa atgttttata agggtgttat cacgcatgat 17640

gtttcatctg caattaacag gccacaaata ggcgtggtaa gagaattcct tacacgtaac 17700

cctgcttgga gaaaagctgt ctttatttca ccttataatt cacagaatgc tgtagcctca 17760

aagattttgg gactaccaac tcaaactgtt gattcatcac agggctcaga atatgactat 17820

gtcatattca ctcaaaccac tgaaacagct cactcttgta atgtaaacag atttaatgtt 17880

gctattacca gagcaaaagt aggcatactt tgcataatgt ctgatagaga cctttatgac 17940

aagttgcaat ttacaagtct tgaaattcca cgtaggaatg tggcaacttt acaagctgaa 18000

aatgtaacag gactctttaa agattgtagt aaggtaatca ctgggttaca tcctacacag 18060

gcacctacac acctcagtgt tgacactaaa ttcaaaactg aaggtttatg tgttgacata 18120

cctggcatac ctaaggacat gacctataga agactcatct ctatgatggg ttttaaaatg 18180

aattatcaag ttaatggtta ccctaacatg tttatcaccc gcgaagaagc tataagacat 18240

gtacgtgcat ggattggctt cgatgtcgag gggtgtcatg ctactagaga agctgttggt 18300

accaatttac ctttacagct aggtttttct acaggtgtta acctagttgc tgtacctaca 18360

ggttatgttg atacacctaa taatacagat ttttccagag ttagtgctaa accaccgcct 18420

ggagatcaat ttaaacacct cataccactt atgtacaaag gacttccttg gaatgtagtg 18480

cgtataaaga ttgtacaaat gttaagtgac acacttaaaa atctctctga cagagtcgta 18540

tttgtcttat gggcacatgg ctttgagttg acatctatga agtattttgt gaaaatagga 18600

cctgagcgca cctgttgtct atgtgataga cgtgccacat gcttttccac tgcttcagac 18660

acttatgcct gttggcatca ttctattgga tttgattacg tctataatcc gtttatgatt 18720

gatgttcaac aatggggttt tacaggtaac ctacaaagca accatgatct gtattgtcaa 18780

gtccatggta atgcacatgt agctagttgt gatgcaatca tgactaggtg tctagctgtc 18840

cacgagtgct ttgttaagcg tgttgactgg actattgaat atcctataat tggtgatgaa 18900

ctgaagatta atgcggcttg tagaaaggtt caacacatgg ttgttaaagc tgcattatta 18960

gcagacaaat tcccagttct tcacgacatt ggtaacccta aagctattaa gtgtgtacct 19020

caagctgatg tagaatggaa gttctatgat gcacagcctt gtagtgacaa agcttataaa 19080

atagaagaat tattctattc ttatgccaca cattctgaca aattcacaga tggtgtatgc 19140

ctattttgga attgcaatgt cgatagatat cctgctaatt ccattgtttg tagatttgac 19200

actagagtgc tatctaacct taacttgcct ggttgtgatg gtggcagttt gtatgtaaat 19260

aaacatgcat tccacacacc agcttttgat aaaagtgctt ttgttaattt aaaacaatta 19320

ccatttttct attactctga cagtccatgt gagtctcatg gaaaacaagt agtgtcagat 19380

atagattatg taccactaaa gtctgctacg tgtataacac gttgcaattt aggtggtgct 19440

gtctgtagac atcatgctaa tgagtacaga ttgtatctcg atgcttataa catgatgatc 19500

tcagctggct ttagcttgtg ggtttacaaa caatttgata cttataacct ctggaacact 19560

tttacaagac ttcagagttt agaaaatgtg gcttttaatg ttgtaaataa gggacacttt 19620

gatggacaac agggtgaagt accagtttct atcattaata acactgttta cacaaaagtt 19680

gatggtgttg atgtagaatt gtttgaaaat aaaacaacat tacctgttaa tgtagcattt 19740

gagctttggg ctaagcgcaa cattaaacca gtaccagagg tgaaaatact caataatttg 19800

ggtgtggaca ttgctgctaa tactgtgatc tgggactaca aaagagatgc tccagcacat 19860

atatctacta ttggtgtttg ttctatgact gacatagcca agaaaccaac tgaaacgatt 19920

tgtgcaccac tcactgtctt ttttgatggt agagttgatg gtcaagtaga cttatttaga 19980

aatgcccgta atggtgttct tattacagaa ggtagtgtta aaggtttaca accatctgta 20040

ggtcccaaac aagctagtct taatggagtc acattaattg gagaagccgt aaaaacacag 20100

ttcaattatt ataagaaagt tgatggtgtt gtccaacaat tacctgaaac ttactttact 20160

cagagtagaa atttacaaga atttaaaccc aggagtcaaa tggaaattga tttcttagaa 20220

ttagctatgg atgaattcat tgaacggtat aaattagaag gctatgcctt cgaacatatc 20280

gtttatggag attttagtca tagtcagtta ggtggtttac atctactgat tggactagct 20340

aaacgtttta aggaatcacc ttttgaatta gaagatttta ttcctatgga cagtacagtt 20400

aaaaactatt tcataacaga tgcgcaaaca ggttcatcta agtgtgtgtg ttctgttatt 20460

gatttattac ttgatgattt tgttgaaata ataaaatccc aagatttatc tgtagtttct 20520

aaggttgtca aagtgactat tgactataca gaaatttcat ttatgctttg gtgtaaagat 20580

ggccatgtag aaacatttta cccaaaatta caatctagtc aagcgtggca accgggtgtt 20640

gctatgccta atctttacaa aatgcaaaga atgctattag aaaagtgtga ccttcaaaat 20700

tatggtgata gtgcaacatt acctaaaggc ataatgatga atgtcgcaaa atatactcaa 20760

ctgtgtcaat atttaaacac attaacatta gctgtaccct ataatatgag agttatacat 20820

tttggtgctg gttctgataa aggagttgca ccaggtacag ctgttttaag acagtggttg 20880

cctacgggta cgctgcttgt cgattcagat cttaatgact ttgtctctga tgcagattca 20940

actttgattg gtgattgtgc aactgtacat acagctaata aatgggatct cattattagt 21000

gatatgtacg accctaagac taaaaatgtt acaaaagaaa atgactctaa agagggtttt 21060

ttcacttaca tttgtgggtt tatacaacaa aagctagctc ttggaggttc cgtggctata 21120

aagataacag aacattcttg gaatgctgat ctttataagc tcatgggaca cttcgcatgg 21180

tggacagcct ttgttactaa tgtgaatgcg tcatcatctg aagcattttt aattggatgt 21240

aattatcttg gcaaaccacg cgaacaaata gatggttatg tcatgcatgc aaattacata 21300

ttttggagga atacaaatcc aattcagttg tcttcctatt ctttatttga catgagtaaa 21360

tttcccctta aattaagggg tactgctgtt atgtctttaa aagaaggtca aatcaatgat 21420

atgattttat ctcttcttag taaaggtaga cttataatta gagaaaacaa cagagttgtt 21480

atttctagtg atgttcttgt taacaactaa acgaacaatg tttgtttttc ttgttttatt 21540

gccactagtc tctagtcagt gtgttaattt tacaaacaga actcaattac cctctgcata 21600

cactaattct ttcacacgtg gtgtttatta ccctgacaaa gttttcagat cctcagtttt 21660

acattcaact caggacttgt tcttaccttt cttttccaat gttacttggt tccatgctat 21720

acatgtctct gggaccaatg gtactaagag gtttgataac cctgtcctac catttaatga 21780

tggtgtttat tttgcttcca ctgagaagtc taacataata agaggctgga tttttggtac 21840

tactttagat tcgaagaccc agtccctact tattgttaat aacgctacta atgttgttat 21900

taaagtctgt gaatttcaat tttgtaatta tccatttttg ggtgtttatt accacaaaaa 21960

caacaaaagt tggatggaaa gtgagttcag agtttattct agtgcgaata attgcacttt 22020

tgaatatgtc tctcagcctt ttcttatgga ccttgaagga aaacagggta atttcaaaaa 22080

tcttagtgaa tttgtgttta agaatattga tggttatttt aaaatatatt ctaagcacac 22140

gcctattaat ttagtgcgtg atctccctca gggtttttcg gctttagaac cattggtaga 22200

tttgccaata ggtattaaca tcactaggtt tcaaacttta cttgctttac atagaagtta 22260

tttgactcct ggtgattctt cttcaggttg gacagctggt gctgcagctt attatgtggg 22320

ttatcttcaa cctaggactt ttctattaaa atataatgaa aatggaacca ttacagatgc 22380

tgtagactgt gcacttgacc ctctctcaga aacaaagtgt acgttgaaat ccttcactgt 22440

agaaaaagga atctatcaaa cttctaactt tagagtccaa ccaacagaat ctattgttag 22500

atttcctaat attacaaact tgtgcccttt tggtgaagtt tttaacgcca ccagatttgc 22560

atctgtttat gcttggaaca ggaagagaat cagcaactgt gttgctgatt attctgtcct 22620

atataattcc gcatcatttt ccacttttaa gtgttatgga gtgtctccta ctaaattaaa 22680

tgatctctgc tttactaatg tctatgcaga ttcatttgta attagaggtg atgaagtcag 22740

acaaatcgct ccagggcaaa ctggaacgat tgctgattat aattataaat taccagatga 22800

ttttacaggc tgcgttatag cttggaattc taacaatctt gattctaagg ttggtggtaa 22860

ttataattac ctgtatagat tgtttaggaa gtctaatctc aaaccttttg agagagatat 22920

ttcaactgaa atctatcagg ccggtagcac accttgtaat ggtgttaaag gttttaattg 22980

ttactttcct ttacaatcat atggtttcca acccacttat ggtgttggtt accaaccata 23040

cagagtagta gtactttctt ttgaacttct acatgcacca gcaactgttt gtggacctaa 23100

aaagtctact aatttggtta aaaacaaatg tgtcaatttc aacttcaatg gtttaacagg 23160

cacaggtgtt cttactgagt ctaacaaaaa gtttctgcct ttccaacaat ttggcagaga 23220

cattgctgac actactgatg ctgtccgtga tccacagaca cttgagattc ttgacattac 23280

accatgttct tttggtggtg tcagtgttat aacaccagga acaaatactt ctaatcaggt 23340

tgctgttctt tatcagggtg ttaactgcac agaagtccct gttgctattc atgcagatca 23400

acttactcct acttggcgtg tttattctac aggttctaat gtttttcaaa cacgtgcagg 23460

ctgtttaata ggggctgaat atgtcaacaa ctcatatgag tgtgacatac ccattggtgc 23520

aggtatatgc gctagttatc agactcagac taattctcct cggcgggcac gtagtgtagc 23580

tagtcaatcc atcattgcct acactatgtc acttggtgca gaaaattcag ttgcttactc 23640

taataactct attgccatac ccacaaattt tactattagt gttaccacag aaattctacc 23700

agtgtctatg accaagacat cagtagattg tacaatgtac atttgtggtg attcaactga 23760

atgcagcaat cttttgttgc aatatggcag tttttgtaca caattaaacc gtgctttaac 23820

tggaatagct gttgaacaag acaaaaacac ccaagaagtt tttgcacaag tcaaacaaat 23880

ttacaaaaca ccaccaatta aagattttgg tggttttaat ttttcacaaa tattaccaga 23940

tccatcaaaa ccaagcaaga ggtcatttat tgaagatcta cttttcaaca aagtgacact 24000

tgcagatgct ggcttcatca aacaatatgg tgattgcctt ggtgatattg ctgctagaga 24060

cctcatttgt gcacaaaagt ttaacggcct tactgttttg ccacctttgc tcacagatga 24120

aatgattgct caatacactt ctgcactgtt agcgggtaca atcacttctg gttggacctt 24180

tggtgcaggt gctgcattac aaataccatt tgctatgcaa atggcttata ggtttaatgg 24240

tattggagtt acacagaatg ttctctatga gaaccaaaaa ttgattgcca accaatttaa 24300

tagtgctatt ggcaaaattc aagactcact ttcttccaca gcaagtgcac ttggaaaact 24360

tcaagatgtg gtcaaccaaa atgcacaagc tttaaacacg cttgttaaac aacttagctc 24420

caattttggt gcaatttcaa gtgttttaaa tgatatcctt tcacgtcttg acaaagttga 24480

ggctgaagtg caaattgata ggttgatcac aggcagactt caaagtttgc agacatatgt 24540

gactcaacaa ttaattagag ctgcagaaat cagagcttct gctaatcttg ctgctattaa 24600

aatgtcagag tgtgtacttg gacaatcaaa aagagttgat ttttgtggaa agggctatca 24660

tcttatgtcc ttccctcagt cagcacctca tggtgtagtc ttcttgcatg tgacttatgt 24720

ccctgcacaa gaaaagaact tcacaactgc tcctgccatt tgtcatgatg gaaaagcaca 24780

ctttcctcgt gaaggtgtct ttgtttcaaa tggcacacac tggtttgtaa cacaaaggaa 24840

tttttatgaa ccacaaatca ttactacaga caacacattt gtgtctggta actgtgatgt 24900

tgtaatagga attgtcaaca acacagttta tgatcctttg caacctgaat tagactcatt 24960

caaggaggag ttagataaat attttaagaa tcatacatca ccagatgttg atttaggtga 25020

catctctggc attaatgctt catttgtaaa cattcaaaaa gaaattgacc gcctcaatga 25080

ggttgccaag aatttaaatg aatctctcat cgatctccaa gaacttggaa agtatgagca 25140

gtatataaaa tggccatggt acatttggct aggttttata gctggcttga ttgccatagt 25200

aatggtgaca attatgcttt gctgtatgac cagttgctgt agttgtctca agggctgttg 25260

ttcttgtgga tcctgctgca aatttgatga agacgactct gagccagtgc tcaaaggagt 25320

caaattacat tacacataaa cgaacttatg gatttgttta tgagaatctt cacaattgga 25380

actgtaactt tgaagcaagg tgaaatcaag gatgctactc cttcagattt tgttcgcgct 25440

actgcaacga taccgataca agcctcactc cctttcggat ggcttattgt tggcgttgca 25500

cttcttgctg tttttcagag cgcttccaaa atcataaccc tcaaaaagag atggcaacta 25560

gcactctcca agggtgttca ctttgtttgc aacttgctgt tgttgtttgt aacagtttac 25620

tcacaccttt tgctcgttgc tgctggcctt gaagcccctt ttctctatct ttatgcttta 25680

gtctacttct tgcagagtat aaactttgta agaataataa tgaggctttg gctttgctgg 25740

aaatgccgtt ccaaaaaccc attactttat gatgccaact attttctttg ctggcatact 25800

aattgttacg actattgtat accttacaat agtgtaactt cttcaattgt cattacttca 25860

ggtgatggca caacaagtcc tatttctgaa catgactacc agattggtgg ttatactgaa 25920

aaatgggaat ctggagtaaa agactgtgtt gtattacaca gttacttcac ttcagactat 25980

taccagctgt actcaactca attgagtaca gacactggtg ttgaacatgt taccttcttc 26040

atctacaata aaattgttga tgagcctgaa gaacatgtcc aaattcacac aatcgacggt 26100

tcacccggag ttgttaatcc agtaatggaa ccaatttatg atgaaccgac gacgactact 26160

agcgtgcctt tgtaagcaca agctgatgag tacgaactta tgtactcatt cgtttcggaa 26220

gagacaggta cgttaatagt taatagcgta cttctttttc ttgctttcgt ggtattcttg 26280

ctagttacac tagccatcct tactgcgctt cgattgtgtg cgtactgctg caatattgtt 26340

aacgtgagtc ttgtaaaacc ttctttttac gtttactctc gtgttaaaaa tctgaattct 26400

tctagagttc ctgatcttct ggtctaaacg aactaaatat tatattagtt tttctgtttg 26460

gaactttaat tttagccatg gcagattcca acggtactat taccgttgaa gagcttaaaa 26520

agctccttga acaatggaac ctagtaatag gtttcctatt ccttacatgg atttgtcttc 26580

tacaatttgc ctatgccaac aggaataggt ttttgtatat aattaagtta attttcctct 26640

ggctgttatg gccagtaact ttagcttgtt ttgtgcttgc tgctgtttac agaataaatt 26700

ggatcaccgg tggaattgct atcgcaatgg cttgtcttgt aggcttgatg tggctcagct 26760

acttcattgc ttctttcaga ctgtttgcgc gtacgcgttc catgtggtca ttcaatccag 26820

aaactaacat tcttctcaac gtgccactcc atggcactat tctgaccaga ccgcttctag 26880

aaagtgaact cgtaatcgga gctgtgatcc ttcgtggaca tcttcgtatt gctggacacc 26940

atctaggacg ctgtgacatc aaggacctgc ctaaagaaat cactgttgct acatcacgaa 27000

cgctttctta ttacaaattg ggagcttcgc agcgtgtagc aggtgactca ggttttgctg 27060

catacagtcg ctacaggatt ggcaactata aattaaacac agaccattcc agtagcagtg 27120

acaatattgc tttgcttgta cagtaagtga caacagatgt ttcatctcgt tgactttcag 27180

gttactatag cagagatatt actaattatt atgaggactt ttaaagtttc catttggaat 27240

cttgattaca tcataaacct cataattaaa aatttatcta agtcactaac tgagaataaa 27300

tattctcaat tagatgaaga gcaaccaatg gagattgatt aaacgaacat gaaaattatt 27360

cttttcttgg cactgataac actcgctact tgtgagcttt atcactacca agagtgtgtt 27420

agaggtacaa cagtactttt aaaagaacct tgctcttctg gaacatacga gggcaattca 27480

ccatttcatc ctctagctga taacaaattt gcactgactt gctttagcac tcaatttgct 27540

tttgcttgtc ctgacggcgt aaaacacgtc tatcagttac gtgccagatc agtttcacct 27600

aaactgttca tcagacaaga ggaagttcaa gaactttact ctccaatttt tcttattgtt 27660

gcggcaatag tgtttataac actttgcttc acactcaaaa gaaagacaga atgattgaac 27720

tttcattaat tgacttctat ttgtgctttt tagcctttct gctattcctt gttttaatta 27780

tgcttattat cttttggttc tcacttgaac tgcaagatca taatgaaact tgtcacgcct 27840

aaacgaacat gaaatttctt gttttcttag gaatcatcac aactgtagct gcatttcacc 27900

aagaatgtag tttacagtca tgtactcaac atcaaccata tgtagttgat gacccgtgtc 27960

ctattcactt ctattctaaa tggtatatta gagtaggagc tagaaaatca gcacctttaa 28020

ttgaattgtg cgtggatgag gctggttcta aatcacccat tcagtacatc gatatcggta 28080

attatacagt ttcctgttta ccttttacaa ttaattgcca gaaacctaaa ttgggtagtc 28140

ttgtagtgcg ttgttcgttc tatgaagact ttttagagta tcatgacgtt cgtgttgttt 28200

tagatttcat ctaaacgaac aaacaaacta aaatgtctga taatggaccc caaaatcagc 28260

gaaatgcacc ccgcattacg tttggtggac cctcagattc aactggcagt aaccagaatg 28320

gagaacgcag tggggcgcga tcaaaacaac gtcggcccca aggtttaccc aataatactg 28380

cgtcttggtt caccgctctc actcaacatg gcaaggaaga ccttaaattc cctcgaggac 28440

aaggcgttcc aattaacacc aatagcagtc gagatgacca aattggctac taccgaagag 28500

ctaccagacg aattcgtggt ggtgacggta aaatgaaaga tctcagtcca agatggtatt 28560

tctactacct aggaactggg ccagaagctg gacttcccta tggtgctaac aaagacggca 28620

tcatatgggt tgcaactgag ggagccttga atacaccaaa agatcacatt ggcacccgca 28680

atcctgctaa caatgctgca atcgtgctac aacttcctca aggaacaaca ttgccaaaag 28740

gcttctacgc agaagggagc agaggcggca gtcaagcctc ttctcgttcc tcatcacgta 28800

gtcgcaacag ttcaagaaat tcaactccag gcagctctaa acgaacttct cctgctagaa 28860

tggctggcaa tggcggtgat gctgctcttg ctttgctgct gcttgacaga ttgaaccagc 28920

ttgagagcaa aatgtctggt aaaggccaac aacaacaagg ccaaactgtc actaagaaat 28980

ctgctgctga ggcttctaag aagcctcggc aaaaacgtac tgccactaaa gcatacaatg 29040

taacacaagc tttcggcaga cgtggtccag aacaaaccca aggaaatttt ggggaccagg 29100

aactaatcag acaaggaact gattacaaac attggccgca aattgcacaa tttgccccca 29160

gcgcttcagc gttcttcgga atgtcgcgca ttggcatgga agtcacacct tcgggaacgt 29220

ggttgaccta cacaggtgcc atcaaattgg atgacaaaga tccaaatttc aaagatcaag 29280

tcattttgct gaataagcat attgacgcat acaaaacatt cccaccaaca gagcctaaaa 29340

aggacaaaaa gaagaaggct gatgaaactc aagccttacc gcagagacag aagaaacagc 29400

aaactgtgac tcttcttcct gctgcagatt tggatgattt ctccaaacaa ttgcaacaat 29460

ccatgagcag tgctgactca actcaggcct aaactcatgc agaccacaca aggcagatgg 29520

gctatataaa cgttttcgct tttccgttta cgatatatag tctactcttg tgcagaatga 29580

attctcgtaa ctacatagca caagtagatg tagttaactt taatctcaca tagcaatctt 29640

taatcagtgt gtaacattag ggaggacttg aaagagccac cacattttca ccgaggccac 29700

gcggagtacg atcgagtgta cagtgaacaa tgctagggag agctgcctat atggaagagc 29760

cctaatgtgt aaaattaatt ttagtagtgc taaccccatg tgattttaat agcttctta 29819

<210> 21
<211> 1273
<212> PRT
<213> SARS-CoV2

<400> 21

Met Phe Val Phe Leu Val Leu Leu Pro Leu Val Ser Ser Gln Cys Val
1               5                   10                  15

Asn Phe Thr Asn Arg Thr Gln Leu Pro Ser Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Tyr Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Ser Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Thr Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Lys Gly Phe Asn Cys Tyr Phe Pro Leu Gln Ser Tyr Gly
                485                 490                 495

Phe Gln Pro Thr Tyr Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Gly Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu Tyr Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser

690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                 1005

Thr Gln  Gln Leu Ile Arg Ala  Ala Glu Ile Arg Ala  Ser Ala Asn
    1010                 1015                 1020

Leu Ala  Ala Ile Lys Met Ser  Glu Cys Val Leu Gly  Gln Ser Lys
    1025                 1030                 1035

Arg Val  Asp Phe Cys Gly Lys  Gly Tyr His Leu Met  Ser Phe Pro
    1040                 1045                 1050

Gln Ser  Ala Pro His Gly Val  Val Phe Leu His Val  Thr Tyr Val
    1055                 1060                 1065

Pro Ala  Gln Glu Lys Asn Phe  Thr Thr Ala Pro Ala  Ile Cys His
    1070                 1075                 1080

Asp Gly  Lys Ala His Phe Pro  Arg Glu Gly Val Phe  Val Ser Asn
    1085                 1090                 1095

Gly Thr  His Trp Phe Val Thr  Gln Arg Asn Phe Tyr  Glu Pro Gln
    1100                 1105                 1110

Ile Ile  Thr Thr Asp Asn Thr  Phe Val Ser Gly Asn  Cys Asp Val
    1115                 1120                 1125

Val Ile  Gly Ile Val Asn Asn  Thr Val Tyr Asp Pro  Leu Gln Pro
    1130                 1135                 1140

Glu Leu  Asp Ser Phe Lys Glu  Glu Leu Asp Lys Tyr  Phe Lys Asn
    1145                 1150                 1155

His Thr  Ser Pro Asp Val Asp  Leu Gly Asp Ile Ser  Gly Ile Asn
    1160                 1165                 1170

Ala Ser  Phe Val Asn Ile Gln  Lys Glu Ile Asp Arg  Leu Asn Glu
    1175                 1180                 1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210>  22
<211>  29883
<212>  DNA
<213>  SARS-CoV2

<220>
<221>  misc_feature
<222>  (29849)..(29883)
<223>  n is a, c, g, or t

<400>  22
   attaaaggtt tataccttcc caggtaacaa accaaccaac tttcgatctc ttgtagatct      60
   gttctctaaa cgaactttaa aatctgtgtg gctgtcactc ggctgcatgc ttagtgcact     120
   cacgcagtat aattaataac taattactgt cgttgacagg acacgagtaa ctcgtctatc     180
   ttctgcaggc tgcttacggt ttcgtccgtg ttgcagccga tcatcagcac atctaggttt     240
   tgtccgggtg tgaccgaaag gtaagatgga gagccttgtc cctggtttca acgagaaaac     300
   acacgtccaa ctcagtttgc ctgttttaca ggttcgcgac gtgctcgtac gtggctttgg     360
   agactccgtg gaggaggtct tatcagaggc acgtcaacat cttaaagatg gcacttgtgg     420
   cttagtagaa gttgaaaaag gcgttttgcc tcaacttgaa cagccctatg tgttcatcaa     480
   acgttcggat gctcgaactg cacctcatgg tcatgttatg gttgagctgg tagcagaact     540
   cgaaggcatt cagtacggtc gtagtggtga gacacttggt gtccttgtcc ctcatgtggg     600
   cgaaatacca gtggcttacc gcaaggttct tcttcgtaag aacggtaata aaggagctgg     660
   tggccatagt tacggcgccg atctaaagtc atttgactta ggcgacgagc ttggcactga     720
   tccttatgaa gattttcaag aaaactggaa cactaaacat agcagtggtg ttacccgtga     780
   actcatgcgt gagcttaacg gaggggcata cactcgctat gtcgataaca acttctgtgg 840
   ccctgatggc taccctcttg agtgcattaa agaccttcta gcacgtgctg gtaaagcttc 900
   atgcactttg tctgaacaac tggactttat tgacactaag aggggtgtat actgctgccg 960
   tgaacatgag catgaaattg cttggtacac ggaacgttct gaaaagagct atgaattgca 1020
   gacacctttt gaaattaaat tggcaaagaa atttgacacc ttcaatgggg aatgtccaaa 1080
   ttttgtattt cccttaaatt ccataatcaa gactattcaa ccaagggttg aaaagaaaaa 1140
   gcttgatggc tttatgggta gaattcgatc tgtctatcca gttgcgtcac caaatgaatg 1200
   caaccaaatg tgcctttcaa ctctcatgaa gtgtgatcat tgtggtgaaa cttcatggca 1260
   gacgggcgat tttgttaaag ccacttgcga attttgtggc actgagaatt tgactaaaga 1320
   aggtgccact acttgtggtt acttacccca aaatgctgtt gttaaaattt attgtccagc 1380
   atgtcacaat tcagaagtag gacctgagca tagtcttgcc gaataccata atgaatctgg 1440
   cttgaaaacc attcttcgta agggtggtcg cactattgcc tttggaggct gtgtgttctc 1500
   ttatgttggt tgccataaca agtgtgccta ttgggttcca cgtgctagcg ctaacatagg 1560
   ttgtaaccat acaggtgttg ttggagaagg ttccgaaggt cttaatgaca accttcttga 1620
   aatactccaa aaagagaaag tcaacatcaa tattgttggt gactttaaac ttaatgaaga 1680
   gatcgccatt attttggcat ctttttctgc ttccacaagt gcttttgtgg aaactgtgaa 1740
   aggtttggat tataaagcat tcaaacaaat tgttgaatcc tgtggtaatt ttaaagttac 1800
   aaaaggaaaa gctaaaaaag gtgcctggaa tattggtgaa cagaaatcaa tactgagtcc 1860
   tctttatgca tttgcatcag aggctgctcg tgttgtacga tcaattttct cccgcactct 1920
   tgaaactgct caaaattctg tgcgtgtttt acagaaggcc gctataacaa tactagatgg 1980
   aatttcacag tattcactga gactcattga tgctatgatg ttcacatctg atttggctac 2040
   taacaatcta gttgtaatgg cctacattac aggtggtgtt gttcagttga cttcgcagtg 2100
   gctaactaac atctttggca ctgtttatga aaaactcaaa cccgtccttg attggcttga 2160
   agagaagttt aaggaaggtg tagagtttct tagagacggt tgggaaattg ttaaatttat 2220
   ctcaacctgt gcttgtgaaa ttgtcggtgg acaaattgtc acctgtgcaa aggaaattaa 2280
   ggagagtgtt cagacattct ttaagcttgt aaataaattt ttggctttgt gtgctgactc 2340
   tatcattatt ggtggagcta aacttaaagc cttgaattta ggtgaaacat ttgtcacgca 2400
   ctcaaaggga ttgtacagaa agtgtgttaa atccagagaa gaaactggcc tactcatgcc 2460
   tctaaaagcc ccaaaagaaa ttatcttctt agagggagaa acacttccca cagaagtgtt 2520
   aacagaggaa gttgtcttga aaactggtga tttacaacca ttagaacaac ctactagtga 2580
   agctgttgaa gctccattgg ttggtacacc agtttgtatt aacgggctta tgttgctcga 2640
   aatcaaagac acagaaaagt actgtgccct tgcacctaat atgatggtaa caaacaatac 2700
   cttcacactc aaaggcggtg caccaacaaa ggttactttt ggtgatgaca ctgtgataga 2760
   agtgcaaggt tacaagagtg tgaatatcac ttttgaactt gatgaaagga ttgataaagt 2820
   acttaatgag aagtgctctg cctatacagt tgaactcggt acagaagtaa atgagttcgc 2880
   ctgtgttgtg gcagatgctg tcataaaaac tttgcaacca gtatctgaat tacttacacc 2940
   actgggcatt gatttagatg agtggagtat ggctacatac tacttatttg atgagtctgg 3000
   tgagtttaaa ttggcttcac atatgtattg ttctttttac cctccagatg aggatgaaga 3060
   agaaggtgat tgtgaagaag aagagtttga gccatcaact caatatgagt atggtactga 3120
   agatgattac caaggtaaac ctttggaatt tggtgccact tctgctgctc ttcaacctga 3180
   agaagagcaa gaagaagatt ggttagatga tgatagtcaa caaactgttg gtcaacaaga 3240
   cggcagtgag gacaatcaga caactattat tcaaacaatt gttgaggttc aacctcaatt 3300
   agagatggaa cttacaccag ttgttcagac tattgaagtg aatagtttta gtggttattt 3360
   aaaacttact gacaatgtat acattaaaaa tgcagacatt gtggaagaag ctaaaaaggt 3420
   aaaaccaaca gtggttgtta atgcagccaa tgtttacctt aaacatggag gaggtgttgc 3480
   aggagcctta aataaggcta ctaacaatgc catgcaagtt gaatctgatg attacatagc 3540
   tactaatgga ccacttaaag tgggtggtag ttgtgtttta agcggacaca atcttgctaa 3600
   acactgtctt catgttgtcg gcccaaatgt taacaaaggt gaagacattc aacttcttaa 3660
   gagtgcttat gaaaatttta atcagcacga agttctactt gcaccattat tatcagctgg 3720
   tatttttggt gctgacccta tacattcttt aagagtttgt gtagatactg ttcgcacaaa 3780
   tgtctactta gctgtctttg ataaaaatct ctatgacaaa cttgtttcaa gctttttgga 3840
   aatgaagagt gaaaagcaag ttgaacaaaa gatcgctgag attcctaaag aggaagttaa 3900
   gccatttata actgaaagta aaccttcagt tgaacagaga aaacaagatg ataagaaaat 3960
   caaagcttgt gttgaagaag ttacaacaac tctggaagaa actaagttcc tcacagaaaa 4020
   cttgttactt tatattgaca ttaatggcaa tcttcatcca gattctgcca ctcttgttag 4080
   tgacattgac atcactttct taaagaaaga tgctccatat atagtgggtg atgttgttca 4140
   agagggtgtt ttaactgctg tggttatacc tactaaaaag gctggtggca ctactgaaat 4200
   gctagcgaaa gctttgagaa aagtgccaac agacaattat ataaccactt acccgggtca 4260
   gggtttaaat ggttacactg tagaggaggc aaagacagtg cttaaaaagt gtaaaagtgc 4320
   cttttacatt ctaccatcta ttatctctaa tgagaagcaa gaaattcttg gaactgtttc 4380
   ttggaatttg cgagaaatgc ttgcacatgc agaagaaaca cgcaaattaa tgcctgtctg 4440
   tgtggaaact aaagccatag tttcaactat acagcgtaaa tataagggta ttaaaataca 4500
   agagggtgtg gttgattatg gtgctagatt ttacttttac accagtaaaa caactgtagc 4560
   gtcacttatc aacacactta acgatctaaa tgaaactctt gttacaatgc cacttggcta 4620
   tgtaacacat ggcttaaatt tggaagaagc tgctcggtat atgagatctc tcaaagtgcc 4680
   agctacagtt tctgtttctt cacctgatgc tgttacagcg tataatggtt atcttacttc 4740
   ttcttctaaa acacctgaag aacattttat tgaaaccatc tcacttgctg gttcctataa 4800
   agattggtcc tattctggac aatctacaca actaggtata gaatttctta agagaggtga 4860
   taaaagtgta tattacacta gtaatcctac cacattccac ctagatggtg aagttatcac 4920
   ctttgacaat cttaagacac ttctttcttt gagagaagtg aggactatta aggtgtttac 4980
   aacagtagac aacattaatc tccacacgca agttgtggac atgtcaatga catatggaca 5040
   acagtttggt ccaacttatt tggatggagc tgatgttact aaaataaaac ctcataattc 5100
   acatgaaggt aaaacatttt atgttttacc taatgatgac actctacgtg ttgaggcttt 5160
   tgagtactac cacacaactg atcctagttt tctgggtagg tacatgtcag cattaaatca 5220
   cactaaaaag tggaaatacc cacaagttaa tggtttaact tctattaaat gggcagataa 5280
   caactgttat cttgccactg cattgttaac actccaacaa atagagttga agtttaatcc 5340
   acctgctcta caagatgctt attacagagc aagggctggt gaagctgcta acttttgtgc 5400
   acttatctta gcctactgta ataagacagt aggtgagtta ggtgatgtta gagaaacaat 5460
   gagttacttg tttcaacatg ccaatttaga ttcttgcaaa agagtcttga acgtggtgtg 5520
   taaaacttgt ggacaacagc agacaaccct taagggtgta gaagctgtta tgtacatggg 5580
   cacactttct tatgaacaat ttaagaaagg tgttcagata ccttgtacgt gtggtaaaca 5640
   agctacaaaa tatctagtac aacaggagtc accttttgtt atgatgtcag caccacctgc 5700
   tcagtatgaa cttaagcatg gtacatttac ttgtgctagt gagtacactg gtaattacca 5760
   gtgtggtcac tataaacata taacttctaa agaaactttg tattgcatag acggtgcttt 5820
   acttacaaag tcctcagaat acaaaggtcc tattacggat gttttctaca aagaaaacag 5880
   ttacacaaca accataaaac cagttactta taaattggat ggtgttgttt gtacagaaat 5940
   tgaccctaag ttggacaatt attataagaa agacaattct tattttacag agcaaccaat 6000
   tgatcttgta ccaaaccaac catatccaaa cgcaagcttc gataatttta agtttgtatg 6060
   tgataatatc aaatttgctg atgatttaaa ccagttaact ggttataaga aacctgcttc 6120
   aagagagctt aaagttacat ttttccctga cttaaatggt gatgtggtgg ctattgatta 6180
   taaacactac acaccctctt ttaagaaagg agctaaattg ttacataaac ctattgtttg 6240
   gcatgttaac aatgcaacta ataaagccac gtataaacca aatacctggt gtatacgttg 6300
   tctttggagc acaaaaccag ttgaaacatc aaattcgttt gatgtactga agtcagagga 6360
   cgcgcaggga atggataatc ttgcctgcga agatctaaaa ccagtctctg aagaagtagt 6420
   ggaaaatcct accatacaga aagacgttct tgagtgtaat gtgaaaacta ccgaagttgt 6480
   aggagacatt atacttaaac cagcaaataa tagtttaaaa attacagaag aggttggcca 6540
   cacagatcta atggctgctt atgtagacaa ttctagtctt actattaaga aacctaatga 6600
   attatctaga gtattaggtt tgaaaaccct tgttactcat ggtttagctg ctgttaatag 6660
   tgtcccttgg gatactatag ctaattatgc taagcctttt cttaacaaag ttgttagtac 6720
   aactactaac atagttacac ggtgtttaaa ccgtgtttgt actaattata tgccttattt 6780
   ctttacttta ttgctacaat tgtgtacttt tactagaagt acaaattcta gaattaaagc 6840
   atctatgccg actactatag caaagaatac tgttaagagt gtcggtaaat tttgtctaga 6900
   ggcttcattt aattatttga agtcacctaa tttttctaaa ctgataaata ttacaatttg 6960
   gtttttacta ttaagtgttt gcctaggttc tttaatctac tcaaccgctg ctttaggtgt 7020
   tttaatgtct aatttaggca tgccttctta ctgtactggt tacagagaag gctatttgaa 7080
   ctctactaat gtcactattg caacctactg tactggttct ataccttgta gtgtttgtct 7140
   tagtggttta gattctttag acacctatcc ttctttagaa actatacaaa ttaccatttc 7200
   atcttttaaa tgggatttaa ctgcttttgg cttagttgca gagtggtttt tggcatatat 7260
   tcttttcact aggtttttct atgtacttgg attggctgca atcatgcaat tgtttttcag 7320
   ctattttgca gtacatttta ttagtaattc ttggcttatg tggttaataa ttaatcttgt 7380
   acaaatggcc ccgatttcag ctatggttag aatgtacatc ttctttgcat cattttatta 7440
   tgtatggaaa agttatgtgc atgttgtaga cggttgtaat tcatcaactt gtatgatgtg 7500
   ttacaaacgt aatagagcaa caagagtcga atgtacaact attgttaatg gtgttagaag 7560
   gtccttttat gtctatgcta atggaggtaa aggcttttgc aaactacaca attggaattg 7620
   tgttaattgt gatacattct gtgctggtag tacatttatt agtgatgaag ttgcgagaga 7680
   cttgtcacta cagtttaaaa gaccaataaa tcctactgac cagtcttctt acatcgttga 7740
   tagtgttaca gtgaagaatg gttccatcca tctttacttt gataaagctg gtcaaaagac 7800
   ttatgaaaga cattctctct ctcattttgt taacttagac aacctgagag ctaataacac 7860
   taaaggttca ttgcctatta atgttatagt ttttgatggt aaatcaaaat gtgaagaatc 7920
   atctgcaaaa tcagcgtctg tttactacag tcagcttatg tgtcaaccta tactgttact 7980
   agatcaggca ttagtgtctg atgttggtga tagtgcggaa gttgcagtta aaatgtttga 8040
   tgcttacgtt aatacgtttt catcaacttt taacgtacca atggaaaaac tcaaaacact 8100
   agttgcaact gcagaagctg aacttgcaaa gaatgtgtcc ttagacaatg tcttatctac 8160
   ttttatttca gcagctcggc aagggtttgt tgattcagat gtagaaacta aagatgttgt 8220
   tgaatgtctt aaattgtcac atcaatctga catagaagtt actggcgata gttgtaataa 8280
   ctatatgctc acctataaca aagttgaaaa catgacaccc cgtgaccttg gtgcttgtat 8340
   tgactgtagt gcgcgtcata ttaatgcgca ggtagcaaaa agtcacaaca ttgctttgat 8400
   atggaacgtt aaagatttca tgtcattgtc tgaacaacta cgaaaacaaa tacgtagtgc 8460
   tgctaaaaag aataacttac cttttaagtt gacatgtgca actactagac aagttgttaa 8520
   tgttgtaaca acaaagatag cacttaaggg tggtaaaatt gttaataatt ggttgaagca 8580
   gttaattaaa gttacacttg tgttcctttt tgttgctgct attttctatt taataacacc 8640
   tgttcatgtc atgtctaaac atactgactt ttcaagtgaa atcataggat acaaggctat 8700
   tgatggtggt gtcactcgtg acatagcatc tacagatact tgttttgcta acaaacatgc 8760
   tgattttgac acatggttta gccagcgtgg tggtagttat actaatgaca aagcttgccc 8820
   attgattgct gcagtcataa caagagaagt gggttttgtc gtgcctggtt tgcctggcac 8880
   gatattacgc acaactaatg gtgacttttt gcatttctta cctagagttt ttagtgcagt 8940
   tggtaacatc tgttacacac catcaaaact tatagagtac actgactttg caacatcagc 9000
   ttgtgttttg gctgctgaat gtacaatttt taaagatgct tctggtaagc cagtaccata 9060
   ttgttatgat accaatgtac tagaaggttc tgttgcttat gaaagtttac gccctgacac 9120
   acgttatgtg ctcatggatg gctctattat tcaatttcct aacacctacc ttgaaggttc 9180
   tgttagagtg gtaacaactt ttgattctga gtactgtagg cacggcactt gtgaaagatc 9240
   agaagctggt gtttgtgtat ctactagtgg tagatgggta cttaacaatg attattacag 9300
   atctttacca ggagttttct gtggtgtaga tgctgtaaat ttacttacta atatgtttac 9360
   accactaatt caacctattg gtgctttgga catatcagca tctatagtag ctggtggtat 9420
   tgtagctatc gtagtaacat gccttgccta ctattttatg aggtttagaa gagcttttgg 9480
   tgaatacagt catgtagttg cctttaatac tttactattc cttatgtcat tcactgtact 9540
   ctgtttaaca ccagtttact cattcttacc tggtgtttat tctgttattt acttgtactt 9600
   gacattttat cttactaatg atgtttcttt tttagcacat attcagtgga tggttatgtt 9660
   cacaccttta gtacctttct ggataacaat tgcttatatc atttgtattt ccacaaagca 9720
   tttctattgg ttctttagta attacctaaa gagacgtgta gtctttaatg gtgtttcctt 9780
   tagtactttt gaagaagctg cgctgtgcac ctttttgtta aataaagaaa tgtatctaaa 9840
   gttgcgtagt gatgtgctat tacctcttac gcaatataat agatacttag ctctttataa 9900
   taagtacaag tattttagtg gagcaatgga tacaactagc tacagagaag ctgcttgttg 9960
   tcatctcgca aaggctctca atgacttcag taactcaggt tctgatgttc tttaccaacc 10020
   accacaaacc tctatcacct cagctgtttt gcagagtggt tttagaaaaa tggcattccc 10080
   atctggtaaa gttgagggtt gtatggtaca agtaacttgt ggtacaacta cacttaacgg 10140
   tctttggctt gatgacgtag tttactgtcc aagacatgtg atctgcacct ctgaagacat 10200
   gcttaaccct aattatgaag atttactcat tcgtaagtct aatcataatt tcttggtaca 10260
   ggctggtaat gttcaactca gggttattgg acattctatg caaaattgtg tacttaagct 10320
   taaggttgat acagccaatc ctaagacacc taagtataag tttgttcgca ttcaaccagg 10380
   acagactttt tcagtgttag cttgttacaa tggttcacca tctggtgttt accaatgtgc 10440
   tatgaggccc aatttcacta ttaagggttc attccttaat ggttcatgtg gtagtgttgg 10500
   ttttaacata gattatgact gtgtctcttt ttgttacatg caccatatgg aattaccaac 10560
   tggagttcat gctggcacag acttagaagg taacttttat ggaccttttg ttgacaggca 10620
   aacagcacaa gcagctggta cggacacaac tattacagtt aatgttttag cttggttgta 10680
   cgctgctgtt ataaatggag acaggtggtt tctcaatcga tttaccacaa ctcttaatga 10740
   ctttaacctt gtggctatga agtacaatta tgaacctcta acacaagacc atgttgacat 10800
   actaggacct ctttctgctc aaactggaat tgccgtttta gatatgtgtg cttcattaaa 10860
   agaattactg caaaatggta tgaatggacg taccatattg ggtagtgctt tattagaaga 10920
   tgaatttaca ccttttgatg ttgttagaca atgctcaggt gttactttcc aaagtgcagt 10980
   gaaaagaaca atcaagggta cacaccactg gttgttactc acaattttga cttcactttt 11040
   agttttagtc cagagtactc aatggtcttt gttctttttt ttgtatgaaa atgccttttt 11100
   accttttgct atgggtatta ttgctatgtc tgcttttgca atgatgtttg tcaaacataa 11160
   gcatgcattt ctctgtttgt ttttgttacc ttctcttgcc actgtagctt attttaatat 11220
   ggtctatatg cctgctagtt gggtgatgcg tattatgaca tggttggata tggttgatac 11280
   tagtttgaag ctaaaagact gtgttatgta tgcatcagct gtagtgttac taatccttat 11340
   gacagcaaga actgtgtatg atgatggtgc taggagagtg tggacactta tgaatgtctt 11400
   gacactcgtt tataaagttt attatggtaa tgctttagat caagccattt ccatgtgggc 11460
   tcttataatc tctgttactt ctaactactc aggtgtagtt acaactgtca tgtttttggc 11520
   cagaggtatt gtttttatgt gtgttgagta ttgccctatt ttcttcataa ctggtaatac 11580
   acttcagtgt ataatgctag tttattgttt cttaggctat ttttgtactt gttactttgg 11640
   cctcttttgt ttactcaacc gctactttag actgactctt ggtgtttatg attacttagt 11700
   ttctacacag gagtttagat atatgaattc acagggacta ctcccaccca agaatagcat 11760
   agatgccttc aaactcaaca ttaaattgtt gggtgttggt ggcaaacctt gtatcaaagt 11820
   agccactgta cagtctaaaa tgtcagatgt aaagtgcaca tcartagtct tactctcagt 11880
   tttgcaacaa ctcagagtag aatcatcatc taaattgtgg gctcaatgtg tccagttaca 11940
   caatgacatt ctcttagcta aagatactac tgaagccttt gaaaaaatgg tttcactact 12000
   ttctgttttg ctttccatgc agggtgctgt agacataaac aagctttgtg aagaaatgct 12060
   ggacaacagg gcaaccttac aagctatagc ctcagagttt agttcccttc catcatatgc 12120
   agcttttgct actgctcaag aagcttatga gcaggctgtt gctaatggtg attctgaagt 12180
   tgttcttaaa aagttgaaga agtctttgaa tgtggctaaa tctgaatttg accgtgatgc 12240
   agccatgcaa cgtaagttgg aaaagatggc tgatcaagct atgacccaaa tgtataaaca 12300
   ggctagatct gaggacaaga gggcaaaagt tactagtgct atgcagacaa tgcttttcac 12360
   tatgcttaga aagttggata atgatgcact caacaacatt atcaacaatg caagagatgg 12420
   ttgtgttccc ttgaacataa tacctcttac aacagcagcc aaactaatgg ttgtcatacc 12480
   agactataac acatataaaa atacgtgtga tggtacaaca tttacttatg catcagcatt 12540
   gtgggaaatc caacaggttg tagatgcaga tagtaaaatt gttcaactta gtgaaattag 12600
   tatggacaat tcacctaatt tagcatggcc tcttattgta acagctttaa gggccaattc 12660
   tgctgtcaaa ttacagaata atgagcttag tcctgttgca ctacgacaga tgtcttgtgc 12720
   tgccggtact acacaaactg cttgcactga tgacaatgcg ttagcttact acaacacaac 12780
   aaagggaggt aggtttgtac ttgcactgtt atccgattta caggatttga aatgggctag 12840
   attccctaag agtgatggaa ctggtactat ctatacagaa ctggaaccac cttgtaggtt 12900
   tgttacagac acacctaaag gtcctaaagt gaagtattta tactttatta aaggattaaa 12960
   caacctaaat agaggtatgg tacttggtag tttagctgcc acagtacgtc tacaagctgg 13020
   taatgcaaca gaagtgcctg ccaattcaac tgtattatct ttctgtgctt ttgctgtaga 13080
   tgctgctaaa gcttacaaag attatctagc tagtggggga caaccaatca ctaattgtgt 13140
   taagatgttg tgtacacaca ctggtactgg tcaggcaata acagttacac cggaagccaa 13200
   tatggatcaa gaatcctttg gtggtgcatc gtgttgtctg tactgccgtt gccacataga 13260
   tcatccaaat cctaaaggat tttgtgactt aaaaggtaag tatgtacaaa tacctacaac 13320
   ttgtgctaat gaccctgtgg gttttacact taaaaacaca gtctgtaccg tctgcggtat 13380
   gtggaaaggt tatggctgta gttgtgatca actccgcgaa cccatgcttc agtcagctga 13440
   tgcacaatcg tttttaaacg ggtttgcggt gtaagtgcag cccgtcttac accgtgcggc 13500
   acaggcacta gtactgatgt cgtatacagg gcttttgaca tctacaatga taaagtagct 13560
   ggttttgcta aattcctaaa aactaattgt tgtcgcttcc aagaaaagga cgaagatgac 13620
   aatttaattg attcttactt tgtagttaag agacacactt tctctaacta ccaacatgaa 13680
   gaaacaattt ataatttact taaggattgt ccagctgttg ctaaacatga cttctttaag 13740
   tttagaatag acggtgacat ggtaccacat atatcacgtc aacgtcttac taaatacaca 13800
   atggcagacc tcgtctatgc tttaaggcat tttgatgaag gtaattgtga cacattaaaa 13860
   gaaatacttg tcacatacaa ttgttgtgat gatgattatt tcaataaaaa ggactggtat 13920
   gattttgtag aaaacccaga tatattacgc gtatacgcca acttaggtga acgtgtacgc 13980
   caagctttgt taaaaacagt acaattctgt gatgccatgc gaaatgctgg tattgttggt 14040
   gtactgacat tagataatca agatctcaat ggtaactggt atgatttcgg tgatttcata 14100
   caaaccacgc caggtagtgg agttcctgtt gtagattctt attattcatt gttaatgcct 14160
   atattaacct tgaccagggc tttaactgca gagtcacatg ttgacactga cttaacaaag 14220
   ccttacatta agtgggattt gttaaaatat gacttcacgg aagagaggtt aaaactcttt 14280
   gaccgttatt ttaaatattg ggatcagaca taccacccaa attgtgttaa ctgtttggat 14340
   gacagatgca ttctgcattg tgcaaacttt aatgttttat tctctacagt gttcccactt 14400
   acaagttttg gaccactagt gagaaaaata tttgttgatg gtgttccatt tgtagtttca 14460
   actggatacc acttcagaga gctaggtgtt gtacataatc aggatgtaaa cttacatagc 14520
   tctagactta gttttaagga attacttgtg tatgctgctg accctgctat gcacgctgct 14580
   tctggtaatc tattactaga taaacgcact acgtgctttt cagtagctgc acttactaac 14640
   aatgttgctt ttcaaactgt caaacctggt aattttaaca aagacttcta tgactttgct 14700
   gtgtctaagg gtttctttaa ggaaggaagt tctgttgaat taaaacactt cttctttgct 14760
   caggatggta atgctgctat cagcgattat gactactatc gttataatct accaacaatg 14820
   tgtgatatca gacaactact atttgtagtt gaagttgttg ataagtactt tgattgttac 14880
   gatggtggct gtattaatgc taaccaagtc atcgtcaaca acctagacaa atcagctggt 14940
   tttccattta ataaatgggg taaggctaga ctttattatg attcaatgag ttatgaggat 15000
   caagatgcac ttttcgcata tacaaaacgt aatgtcatcc ctactataac tcaaatgaat 15060
   cttaagtatg ccattagtgc aaagaataga gctcgcaccg tagctggtgt ctctatctgt 15120
   agtactatga ccaatagaca gtttcatcaa aaattattga aatcaatagc cgccactaga 15180
   ggagctactg tagtaattgg aacaagcaaa ttctatggtg gttggcacaa catgttaaaa 15240
   actgtttata gtgatgtaga aaaccctcat cttatgggtt gggattatcc taaatgtgat 15300
   agagccatgc ctaacatgct tagaattatg gcctcacttg ttcttgctcg caaacataca 15360
   acgtgttgta gcttgtcaca ccgtttctat agattagcta atgagtgtgc tcaagtattg 15420
   agtgaaatgg tcatgtgtgg cggttcacta tatgttaaac caggtggaac ctcatcagga 15480
   gatgccacaa ctgcttatgc taatagtgtt tttaacattt gtcaagctgt cacggccaat 15540
   gttaatgcac ttttatctac tgatggtaac aaaattgccg ataagtatgt ccgcaattta 15600
   caacacagac tttatgagtg tctctataga aatagagatg ttgacacaga ctttgtgaat 15660
   gagttttacg catatttgcg taaacatttc tcaatgatga tactctctga cgatgctgtt 15720
   gtgtgtttca atagcactta tgcatctcaa ggtctagtgg ctagcataaa gaactttaag 15780
   tcagttcttt attatcaaaa caatgttttt atgtctgaag caaaatgttg gactgagact 15840
   gaccttacta aaggacctca tgaattttgc tctcaacata caatgctagt taaacagggt 15900
   gatgattatg tgtaccttcc ttacccagat ccatcaagaa tcctaggggc cggctgtttt 15960
   gtagatgata tcgtaaaaac agatggtaca cttatgattg aacggttcgt gtctttagct 16020
   atagatgctt acccacttac taaacatcct aatcaggagt atgctgatgt ctttcatttg 16080
   tacttacaat acataagaaa gctacatgat gagttaacag gacacatgtt agacatgtat 16140
   tctgttatgc ttactaatga taacacctca aggtattggg aacctgagtt ttatgaggct 16200
   atgtacacac cgcatacagt cttacaggct gttggggctt gtgttctttg caattcacag 16260
   acttcattaa gatgtggtgc ttgcatacgt agaccattct tatgttgtaa atgctgttac 16320
   gaccatgtca tatcaacatc acataaatta gtcttgtctg ttaatccgta tgtttgcaat 16380
   gctccaggtt gtgatgtcac agatgtgact caactttact taggaggtat gagctattat 16440
   tgtaaatcac ataaaccacc cattagtttt ccattgtgtg ctaatggaca agtttttggt 16500
   ttatataaaa atacatgtgt tggtagcgat aatgttactg actttaatgc aattgcaaca 16560
   tgtgactgga caaatgctgg tgattacatt ttagctaaca cctgtactga aagactcaag 16620
   ctttttgcag cagaaacgct caaagctact gaggagacat ttaaactgtc ttatggtatt 16680
   gctactgtac gtgaagtgct gtctgacaga gaattacatc tttcatggga agttggtaaa 16740
   cctagaccac cacttaaccg aaattatgtc tttactggtt atcgtgtaac taaaaacagt 16800
   aaagtacaaa taggagagta cacctttgaa aaaggtgact atggtgatgc tgttgtttac 16860
   cgaggtacaa caacttacaa attaaatgtt ggtgattatt ttgtgctgac atcacataca 16920
   gtaatgccat taagtgcacc tacactagtg ccacaagagc actatgttag aattactggc 16980
   ttatacccaa cactcaatat ctcagatgag ttttctagca atgttgcaaa ttatcaaaag 17040
   gttggtatgc aaaagtattc tacactccag ggaccacctg gtactggtaa gagtcatttt 17100
   gctattggcc tagctctcta ctacccttct gctcgcatag tgtatacagc ttgctctcat 17160
   gccgctgttg atgcactatg tgagaaggca ttaaaatatt tgcctataga taaatgtagt 17220
   agaattatac ctgcacgtgc tcgtgtagag tgttttgata aattcaaagt gaattcaaca 17280
   ttagaacagt atgtcttttg tactgtaaat gcattgcctg agacgacagc agatatagtt 17340
   gtctttgatg aaatttcaat ggccacaaat tatgatttga gtgttgtcaa tgccagatta 17400
   cgtgctaagc actatgtgta cattggcgac cctgctcaat tacctgcacc acgcacattg 17460
   ctaactaagg gcacactaga accagaatat ttcaattcag tgtgtagact tatgaaaact 17520
   ataggtccag acatgttcct cggaacttgt cggcgttgtc ctgctgaaat tgttgacact 17580
   gtgagtgctt tggtttatga taataggctt aaagcacata aagacaaatc agctcaatgc 17640
   tttaaaatgt tttataaggg tgttatcacg catgatgttt catctgcaat taacaggcca 17700
   caaataggcg tggtaagaga attccttaca cgtaaccctg cttggagaaa agctgtcttt 17760
   atttcacctt ataattcaca gaatgctgta gcctcaaaga ttttgggact accaactcaa 17820
   actgttgatt catcacaggg ctcagaatat gactatgtca tattcactca aaccactgaa 17880
   acagctcact cttgtaatgt aaacagattt aatgttgcta ttaccagagc aaaagtaggc 17940
   atactttgca taatgtctga tagagacctt tatgacaagt tgcaatttac aagtcttgaa 18000
   attccacgta ggaatgtggc aactttacaa gctgaaaatg taacaggact ctttaaagat 18060
   tgtagtaagg taatcactgg gttacatcct acacaggcac ctacacacct cagtgttgac 18120
   actaaattca aaactgaagg tttatgtgtt gacatacctg gcatacctaa ggacatgacc 18180
   tatagaagac tcatctctat gatgggtttt aaaatgaatt atcaagttaa tggttaccct 18240
   aacatgttta tcacccgcga agaagctata agacatgtac gtgcatggat tggcttcgat 18300
   gtcgaggggt gtcatgctac tagagaagct gttggtacca atttaccttt acagctaggt 18360
   ttttctacag gtgttaacct agttgctgta cctacaggtt atgttgatac acctaataat 18420
   acagattttt ccagagttag tgctaaacca ccgcctggag atcaatttaa acacctcata 18480
   ccacttatgt acaaaggact tccttggaat gtagtgcgta taaagattgt acaaatgtta 18540
   agtgacacac ttaaaaatct ctctgacaga gtcgtatttg tcttatgggc acatggcttt 18600
   gagttgacat ctatgaagta ttttgtgaaa ataggacctg agcgcacctg ttgtctatgt 18660
   gatagacgtg ccacatgctt ttccactgct tcagacactt atgcctgttg gcatcattct 18720
   attggatttg attacgtcta taatccgttt atgattgatg ttcaacaatg gggttttaca 18780
   ggtaacctac aaagcaacca tgatctgtat tgtcaagtcc atggtaatgc acatgtagct 18840
   agttgtgatg caatcatgac taggtgtcta gctgtccacg agtgctttgt taagcgtgtt 18900
   gactggacta ttgaatatcc tataattggt gatgaactga agattaatgc ggcttgtaga 18960
   aaggttcaac acatggttgt taaagctgca ttattagcag acaaattccc agttcttcac 19020
   gacattggta accctaaagc tattaagtgt gtacctcaag ctgatgtagg atggaagttc 19080
   tatgatgcac agccttgtag tgacaaagct tataaaatag aagaattatt ctattcttat 19140
   gccacacatt ctgacaaatt cacagatggt gtatgcctat tttggaattg caatgtcgat 19200
   agatatcctg ctaattccat tgtttgtaga tttgacacta gagtgctatc taaccttaac 19260
   ttgcctggtt gtgatggtgg cagtttgtat gtaaataaac atgcattcca cacaccagct 19320
   tttgataaaa gtgcttttgt taatttaaaa caattaccat ttttctatta ctctgacagt 19380
   ccatgtgagt ctcatggaaa acaagtagtg tcagatatag attatgtacc actaaagtct 19440
   gctacgtgta taacacgttg caatttaggt ggtgctgtct gtagacatca tgctaatgag 19500
   tacagattgt atctcgatgc ttataacatg atgatctcag ctggctttag cttgtgggtt 19560
   tacaaacaat ttgatactta taacctctgg aacactttta caagacttca gagtttagaa 19620
   aatgtggctt ttaatgttgt aaataaggga cactttgatg gacaacaggg tgaagtacca 19680
   gtttctatca ttaataacac tgtttacaca aaagttgatg gtgttgatgt agaattgttt 19740
   gaaaataaaa caacattacc tgttaatgta gcatttgagc tttgggctaa gcgcaacatt 19800
   aaaccagtac cagaggtgaa aatactcaat aatttgggtg tggatattgc tgctaatact 19860
   gtgatctggg actacaaaag agatgctcca gcacatatat ctactattgg tgtttgttct 19920
   atgactgaca tagccaagaa accaactgaa acgatttgtg caccactcac tgtctttttt 19980
   gatggtagag ttgatggtca agtagactta tttagaaatg cccgtaatgg tgttcttatt 20040
   acagaaggta gtgttaaagg tttacaacca tctgtaggtc ccaaacaagc tagtcttaat 20100
   ggagtcacat taattggaga agccgtaaaa acacagttca attattataa gaaagttgat 20160
   ggtgttgtcc aacaattacc tgaaacttac tttactcaga gtagaaattt acaagaattt 20220
   aaacccagga gtcaaatgga aattgatttc ttagaattag ctatggatga attcattgaa 20280
   cggtataaat tagaaggcta tgccttcgaa catatcgttt atggagattt tagtcatagt 20340
   cagttaggtg gtttacatct actgattgga ctagctaaac gttttaagga atcacctttt 20400
   gaattagaag attttattcc tatggacagt acagttaaaa actatttcat aacagatgcg 20460
   caaacaggtt catctaagtg tgtgtgttct gttattgatt tattacttga tgattttgtt 20520
   gaaataataa aatcccaaga tttatctgta gtttctaagg ttgtcaaagt gactattgac 20580
   tatacagaaa tttcatttat gctttgtgta aagatggcca tgtagaaaca ttttacccaa 20640
   aattacaatc tagtcaagcg tggcaaccgg gtgttgctat gcctaatctt tacaaaatgc 20700
   aaagaatgct attagaaaag tgtgaccttc aaaattatgg tgatagtgca acattaccta 20760
   aaggcataat gatgaatgtc gcaaaatata ctcaactgtg tcaatattta aacacattaa 20820
   cattagctgt accctataat atgagagtta tacattttgg tgctggttct gataaaggag 20880
   ttgcaccagg tacagctgtt ttaagacagt ggttgcctac gggtacgctg cttgtcgatt 20940
   cagatcttaa tgactttgtc tctgatgcag attcaacttt gattggtgat tgtgcaactg 21000
   tacatacagc taataaatgg gatctcatta ttagtgatat gtacgaccct aagactaaaa 21060
   atgttacaaa agaaaatgac tctaaagagg gttttttcac ttacatttgt gggtttatac 21120
   aacaaaagct agctcttgga ggttccgtgg ctataaagat aacagaacat tcttggaatg 21180
   ctgatcttta taagctcatg ggacacttcg catggtggac agcctttgtt actaatgtga 21240
   atgcgtcatc atctgaagca tttttaattg gatgtaatta tcttggcaaa ccacgcgaac 21300
   aaatagatgg ttatgtcatg catgcaaatt acatattttg gaggaataca aatccaattc 21360
   agttgtcttc ctattcttta tttgacatga gtaaatttcc ccttaaatta aggggtactg 21420
   ctgttatgtc tttaaaagaa ggtcaaatca atgatatgat tttatctctt cttagtaaag 21480
   gtagacttat aattagagaa aacaacagag ttgttatttc tagtgatgtt cttgttaaca 21540
   actaaacgaa caatgtttgt ttttcttgtt ttattgccac tagtctctag tcagtgtgtt 21600
   aatcttacaa ccagaactca attaccccct gcatacacta attctttcac acgtggtgtt 21660
   tattaccctg acaaagtttt cagatcctca gttttacatt caactcagga cttgttctta 21720
   cctttctttt ccaatgttac ttggttccat gctatctctg ggaccaatgg tactaagagg 21780
   tttgataacc ctgtcctacc atttaatgat ggtgtttatt ttgcttccac tgagaagtct 21840
   aacataataa gaggctggat ttttggtact actttagatt cgaagaccca gtccctactt 21900
   attgttaata acgctactaa tgttgttatt aaagtctgtg aatttcaatt ttgtaatgat 21960
   ccatttttgg gtgtttacca caaaaacaac aaaagttgga tggaaagtga gttcagagtt 22020
   tattctagtg cgaataattg cacttttgaa tatgtctctc agccttttct tatggacctt 22080
   gaaggaaaac agggtaattt caaaaatctt agggaatttg tgtttaagaa tattgatggt 22140
   tattttaaaa tatattctaa gcacacgcct attaatttag tgcgtgatct ccctcagggt 22200
   ttttcggctt tagaaccatt ggtagatttg ccaataggta ttaacatcac taggtttcaa 22260
   actttacttg ctttacatag aagttatttg actcctggtg attcttcttc aggttggaca 22320
   gctggtgctg cagcttatta tgtgggttat cttcaaccta ggacttttct attaaaatat 22380
   aatgaaaatg gaaccattac agatgctgta gactgtgcac ttgaccctct ctcagaaaca 22440
   aagtgtacgt tgaaatcctt cactgtagaa aaaggaatct atcaaacttc taactttaga 22500
   gtccaaccaa cagaatctat tgttagattt cctaatatta caaacttgtg cccttttggt 22560
   gaagttttta acgccaccag atttgcatct gtttatgctt ggaacaggaa gagaatcagc 22620
   aactgtgttg ctgattattc tgtcctatat aattccgcat cattttccac ttttaagtgt 22680
   tatggagtgt ctcctactaa attaaatgat ctctgcttta ctaatgtcta tgcagattca 22740
   tttgtaatta gaggtgatga agtcagacaa atcgctccag ggcaaactgg aaagattgct 22800
   gattataatt ataaattacc agatgatttt acaggctgcg ttatagcttg gaattctaac 22860
   aatcttgatt ctaaggttgg tggtaattat aattacctgt atagattgtt taggaagtct 22920
   aatctcaaac cttttgagag agatatttca actgaaatct atcaggccgg tagcacacct 22980
   tgtaatggtg ttgaaggttt taattgttac tttcctttac aatcatatgg tttccaaccc 23040
   acttatggtg ttggttacca accatacaga gtagtagtac tttcttttga acttctacat 23100
   gcaccagcaa ctgtttgtgg acctaaaaag tctactaatt tggttaaaaa caaatgtgtc 23160
   aatttcaact tcaatggttt aacaggcaca ggtgttctta ctgagtctaa caaaaagttt 23220
   ctgcctttcc aacaatttgg cagagacatt gatgacacta ctgatgctgt ccgtgatcca 23280
   cagacacttg agattcttga cattacacca tgttcttttg gtggtgtcag tgttataaca 23340
   ccaggaacaa atacttctaa ccaggttgct gttctttatc agggtgttaa ctgcacagaa 23400
   gtccctgttg ctattcatgc agatcaactt actcctactt ggcgtgttta ttctacaggt 23460
   tctaatgttt ttcaaacacg tgcaggctgt ttaatagggg ctgaacatgt caacaactca 23520
   tatgagtgtg acatacccat tggtgcaggt atatgcgcta gttatcagac tcagactaat 23580
   tctcatcggc gggcacgtag tgtagctagt caatccatca ttgcctacac tatgtcactt 23640
   ggtgcagaaa attcagttgc ttactctaat aactctattg ccatacccat aaattttact 23700
   attagtgtta ccacagaaat tctaccagtg tctatgacca agacatcagt agattgtaca 23760
   atgtacattt gtggtgattc aactgaatgc agcaatcttt tgttgcaata tggcagtttt 23820
   tgtacacaat taaaccgtgc tttaactgga atagctgttg aacaagacaa aaacacccaa 23880
   gaagtttttg cacaagtcaa acaaatttac aaaacaccac caattaaaga ttttggtggt 23940
   tttaattttt cacaaatatt accagatcca tcaaaaccaa gcaagaggtc atttattgaa 24000
   gatctacttt tcaacaaagt gacacttgca gatgctggct tcatcaaaca atatggtgat 24060
   tgccttggtg atattgctgc tagagacctc atttgtgcac aaaagtttaa cggccttact 24120
   gttttgccac ctttgctcac agatgaaatg attgctcaat acacttctgc actgttagcg 24180
   ggtacaatca cttctggttg gacctttggt gcaggtgctg cattacaaat accatttgct 24240
   atgcaaatgg cttataggtt taatggtatt ggagttacac agaatgttct ctatgagaac 24300
   caaaaattga ttgccaacca atttaatagt gctattggca aaattcaaga ctcactttct 24360
   tccacagcaa gtgcacttgg aaaacttcaa gatgtggtca accaaaatgc acaagcttta 24420
   aacacgcttg ttaaacaact tagctccaat tttggtgcaa tttcaagtgt tttaaatgat 24480
   atccttgcac gtcttgacaa agttgaggct gaagtgcaaa ttgataggtt gatcacaggc 24540
   agacttcaaa gtttgcagac atatgtgact caacaattaa ttagagctgc agaaatcaga 24600
   gcttctgcta atcttgctgc tactaaaatg tcagagtgtg tacttggaca atcaaaaaga 24660
   gttgattttt gtggaaaggg ctatcatctt atgtccttcc ctcagtcagc acctcatggt 24720
   gtagtcttct tgcatgtgac ttatgtccct gcacaagaaa agaacttcac aactgctcct 24780
   gccatttgtc atgatggaaa agcacacttt cctcgtgaag gtgtctttgt ttcaaatggc 24840
   acacactggt ttgtaacaca aaggaatttt tatgaaccac aaatcattac tacacacaac 24900
   acatttgtgt ctggtaactg tgatgttgta ataggaattg tcaacaacac agtttatgat 24960
   cctttgcaac ctgaattaga ctcattcaag gaggagttag ataaatattt taagaatcat 25020
   acatcaccag atgttgattt aggtgacatc tctggcatta atgcttcagt tgtaaacatt 25080
   caaaaagaaa ttgaccgcct caatgaggtt gccaagaatt taaatgaatc tctcatcgat 25140
   ctccaagaac ttggaaagta tgagcagtat ataaaatggc catggtacat ttggctaggt 25200
   tttatagctg gcttgattgc catagtaatg gtgacaatta tgctttgctg tatgaccagt 25260
   tgctgtagtt gtctcaaggg ctgttgttct tgtggatcct gctgcaaatt tgatgaagac 25320
   gactctgagc cagtgctcaa aggagtcaaa ttacattaca cataaacgaa cttatggatt 25380
   tgtttatgag aatcttcaca attggaactg taactttgaa gcaaggtgaa atcaaggatg 25440
   ctactccttc agattttgtt cgcgctactg caacgatacc gatacaagcc tcactccctt 25500
   tcggatggct tattgttggc gttgcacttc ttgctgtttt tcagagcgct tccaaaatca 25560
   taaccctcaa aaagagatgg caactagcac tctccaaggg tgttcacttt gtttgcaact 25620
   tgctgttgtt gtttgtaaca gtttactcac accttttgct cgttgctgct ggccttgaag 25680
   ccccttttct ctatctttat gctttagtct acttcttgca gagtataaac tttgtaagaa 25740
   taataatgag gctttggctt tgctggaaat gccgttccaa aaacccatta ctttatgatg 25800
   ccaactattt tctttgctgg catactaatt gttacgacta ttgtatacct tacaatagtg 25860
   taacttcttc aattgtcatt acttcaggtg atggcacaac aagtcctatt tctgaacatg 25920
   actaccagat tggtggttat actgaaaaat gggaatctgg agtaaaagac tgtgttgtat 25980
   tacacagtta cttcacttca gactattacc agctgtactc aactcaattg agtacagaca 26040
   ctggtgttga acatgttacc ttcttcatct acaataaaat tgttgatgag cctgaagaac 26100
   atgtccaaat tcacacaatc gacggttcat ccggagttgt taatccagta atggaaccaa 26160
   tttatgatga accgacgacg actactagcg tgcctttgta agcacaagct gatgagtacg 26220
   aacttatgta ctcattcgtt tcggaagaga caggtacgtt aatagttaat agcgtacttc 26280
   tttttcttgc tttcgtggta ttcttgctag ttacactagc catccttact gcgcttcgat 26340
   tgtgtgcgta ctgctgcaat attgttaacg tgagtcttgt aaaaccttct ttttacgttt 26400
   actctcgtgt taaaaatctg aattcttcta gagttcctga tcttctggtc taaacgaact 26460
   aaatattata ttagtttttc tgtttggaac tttaatttta gccatggcag attccaacgg 26520
   tactattacc gttgaagagc ttaaaaagct ccttgaacaa tggaacctag taataggttt 26580
   cctattcctt acatggattt gtcttctaca atttgcctat gccaacagga ataggttttt 26640
   gtatataatt aagttaattt tcctctggct gttatggcca gtaactttag cttgttttgt 26700
   gcttgctgct gtttacagaa taaattggat caccggtgga attgctatcg caatggcttg 26760
   tcttgtaggc ttgatgtggc tcagctactt cattgcttct ttcagactgt ttgcgcgtac 26820
   gcgttccatg tggtcattca atccagaaac taacattctt ctcaacgtgc cactccatgg 26880
   cactattctg accagaccgc ttctagaaag tgaactcgta atcggagctg tgatccttcg 26940
   tggacatctt cgtattgctg gacaccatct aggacgctgt gacatcaagg acctgcctaa 27000
   agaaatcact gttgctacat cacgaacgct ttcttattac aaattgggag cttcgcagcg 27060
   tgtagcaggt gactcaggtt ttgctgcata cagtcgctac aggattggca actataaatt 27120
   aaacacagac cattccagta gcagtgacaa tattgctttg cttgtacagt aagtgacaac 27180
   agatgtttca tctcgttgac tttcaggtta ctatagcaga gatattacta attattatga 27240
   ggacttttaa agtttccatt tggaatcttg attacatcat aaacctcata attaaaaatt 27300
   tatctaagtc actaactgag aataaatatt ctcaattaga tgaagagcaa ccaatggaga 27360
   ttgattaaac gaacatgaaa attattcttt tcttggcact gataacactc gctacttgtg 27420
   agctttatca ctaccaagag tgtgttagag gtacaacagt acttttaaaa gaaccttgct 27480
   cttctggaac atacgagggc aattcaccat ttcatcctct agctgataac aaatttgcac 27540
   tgacttgctt tagcactcaa tttgcttttg cttgtcctga cggcgtaaaa cacgtctatc 27600
   agttacgtgc cagatcagtt tcacctaaac tgttcatcag acaagaggaa gttcaagaac 27660
   tttactctcc aatttttctt attgttgcgg caatagtgtt tataacactt tgcttcacac 27720
   tcaaaagaaa gacagaatga ttgaactttc attaattgac ttctatttgt gctttttagc 27780
   ctttctgcta ttccttgttt taattatgct tattatcttt tggttctcac ttgaactgca 27840
   agatcataat gaaacttgtc acgcctaaac gaacatgaaa tttcttgttt tcttaggaat 27900
   catcacaact gtagctgcat ttcaccaaga atgtagttta cagtcatgta cttaacatca 27960
   accatatgta gttgatgacc cgtgtcctat tcacttctat tctaaatggt atattagagt 28020
   aggagctata aaatcagcac ctttaattga attgtgcgtg gatgaggctg gttctaaatc 28080
   acccattcag tgcatcgata tcggtaatta tacagtttcc tgtttacctt ttacaattaa 28140
   ttgccaggaa cctaaattgg gtagtcttgt agtgcgttgt tcgttctatg aagacttttt 28200
   agagtatcat gacgttcgtg ttgttttaga tttcatctaa acgaacaaac taaatgtctc 28260
   taaatggacc ccaaaatcag cgaaatgcac cccgcattac gtttggtgga ccctcagatt 28320
   caactggcag taaccagaat ggagaacgca gtggggcgcg atcaaaacaa cgtcggcccc 28380
   aaggtttacc caataatact gcgtcttggt tcaccgctct cactcaacat ggcaaggaag 28440
   accttaaatt ccctcgagga caaggcgttc caattaacac caatagcagt ccagatgacc 28500
   aaattggcta ctaccgaaga gctaccagac gaattcgtgg tggtgacggt aaaatgaaag 28560
   atctcagtcc aagatggtat ttctactacc taggaactgg gccagaagct ggacttccct 28620
   atggtgctaa caaagacggc atcatatggg ttgcaactga gggagccttg aatacaccaa 28680
   aagatcacat tggcacccgc aatcctgcta acaatgctgc aatcgtgcta caacttcctc 28740
   aaggaacaac attgccaaaa ggcttctacg cagaagggag cagaggcggc agtcaagcct 28800
   cttctcgttc ctcatcacgt agtcgcaaca gttcaagaaa ttcaactcca ggcagcagta 28860
   aacgaacttc tcctgctaga atggctggca atggcggtga tgctgctctt gctttgctgc 28920
   tgcttgacag attgaaccag cttgagagca aaatgtttgg taaaggccaa caacaacaag 28980
   gccaaactgt cactaagaaa tctgctgctg aggcttctaa gaagcctcgg caaaaacgta 29040
   ctgccactaa agcatacaat gtaacacaag ctttcggcag acgtggtcca gaacaaaccc 29100
   aaggaaattt tggggaccag gaactaatca gacaaggaac tgattacaaa cattggccgc 29160
   aaattgcaca atttgccccc agcgcttcag cgttcttcgg aatgtcgcgc attggcatgg 29220
   aagtcacacc ttcgggaacg tggttgacct acacaggtgc catcaaattg gatgacaaag 29280
   atccaaattt caaagatcaa gtcattttgc tgaataagca tattgacgca tacaaaacat 29340
   tcccaccaac agagcctaaa aaggacaaaa agaagaaggc tgatgaaact caagccttac 29400
   cgcagagaca gaagaaacag caaactgtga ctcttcttcc tgctgcagat ttggatgatt 29460
   tctccaaaca attgcaacaa tccatgagca gtgctgactc aactcaggcc taaactcatg 29520
   cagaccacac aaggcagatg ggctatataa acgttttcgc ttttccgttt acgatatata 29580
   gtctactctt gtgcagaatg aattctcgta actacatagc acaagtagat gtagttaact 29640
   ttaatctcac atagcaatct ttaatcagtg tgtaacatta gggaggactt gaaagagcca 29700
   ccacattttc accgaggcca cgcggagtac gatcgagtgt acagtgaaca atgctaggga 29760
   gagctgccta tatggaagag ccctaatgtg taaaattaat tttagtagtg ctatccccat 29820
   gtgattttaa tagcttctta ggagaatgnn nnnnnnnnnn nnnnnnnnnn nnnnnnnnnn 29880
   nnn 29883

<210> 23
<211> 1270
<212> PRT
<213> SARS-CoV2

<400> 23

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile Ser Gly Thr Asn Gly Thr Lys Arg Phe Asp Asn Pro
65                  70                  75                  80

Val Leu Pro Phe Asn Asp Gly Val Tyr Phe Ala Ser Thr Glu Lys Ser
                85                  90                  95

Asn Ile Ile Arg Gly Trp Ile Phe Gly Thr Thr Leu Asp Ser Lys Thr
            100                 105                 110

Gln Ser Leu Leu Ile Val Asn Asn Ala Thr Asn Val Val Ile Lys Val
        115                 120                 125

Cys Glu Phe Gln Phe Cys Asn Asp Pro Phe Leu Gly Val Tyr His Lys
    130                 135                 140

Asn Asn Lys Ser Trp Met Glu Ser Glu Phe Arg Val Tyr Ser Ser Ala
145                 150                 155                 160

Asn Asn Cys Thr Phe Glu Tyr Val Ser Gln Pro Phe Leu Met Asp Leu
                165                 170                 175

Glu Gly Lys Gln Gly Asn Phe Lys Asn Leu Arg Glu Phe Val Phe Lys
            180                 185                 190

Asn Ile Asp Gly Tyr Phe Lys Ile Tyr Ser Lys His Thr Pro Ile Asn
        195                 200                 205

Leu Val Arg Asp Leu Pro Gln Gly Phe Ser Ala Leu Glu Pro Leu Val
    210                 215                 220

Asp Leu Pro Ile Gly Ile Asn Ile Thr Arg Phe Gln Thr Leu Leu Ala
225                 230                 235                 240

Leu His Arg Ser Tyr Leu Thr Pro Gly Asp Ser Ser Ser Gly Trp Thr
                245                 250                 255

Ala Gly Ala Ala Ala Tyr Tyr Val Gly Tyr Leu Gln Pro Arg Thr Phe
            260                 265                 270

Leu Leu Lys Tyr Asn Glu Asn Gly Thr Ile Thr Asp Ala Val Asp Cys
        275                 280                 285

Ala Leu Asp Pro Leu Ser Glu Thr Lys Cys Thr Leu Lys Ser Phe Thr
    290                 295                 300

Val Glu Lys Gly Ile Tyr Gln Thr Ser Asn Phe Arg Val Gln Pro Thr
305                 310                 315                 320

Glu Ser Ile Val Arg Phe Pro Asn Ile Thr Asn Leu Cys Pro Phe Gly
                325                 330                 335

Glu Val Phe Asn Ala Thr Arg Phe Ala Ser Val Tyr Ala Trp Asn Arg
            340                 345                 350

Lys Arg Ile Ser Asn Cys Val Ala Asp Tyr Ser Val Leu Tyr Asn Ser
        355                 360                 365

Ala Ser Phe Ser Thr Phe Lys Cys Tyr Gly Val Ser Pro Thr Lys Leu
    370                 375                 380

Asn Asp Leu Cys Phe Thr Asn Val Tyr Ala Asp Ser Phe Val Ile Arg
385                 390                 395                 400

Gly Asp Glu Val Arg Gln Ile Ala Pro Gly Gln Thr Gly Lys Ile Ala
                405                 410                 415

Asp Tyr Asn Tyr Lys Leu Pro Asp Asp Phe Thr Gly Cys Val Ile Ala
            420                 425                 430

Trp Asn Ser Asn Asn Leu Asp Ser Lys Val Gly Gly Asn Tyr Asn Tyr
        435                 440                 445

Leu Tyr Arg Leu Phe Arg Lys Ser Asn Leu Lys Pro Phe Glu Arg Asp
    450                 455                 460

Ile Ser Thr Glu Ile Tyr Gln Ala Gly Ser Thr Pro Cys Asn Gly Val
465                 470                 475                 480

Glu Gly Phe Asn Cys Tyr Phe Pro Leu Gln Ser Tyr Gly Phe Gln Pro
                485                 490                 495

Thr Tyr Gly Val Gly Tyr Gln Pro Tyr Arg Val Val Val Leu Ser Phe
            500                 505                 510

Glu Leu Leu His Ala Pro Ala Thr Val Cys Gly Pro Lys Lys Ser Thr
        515                 520                 525

Asn Leu Val Lys Asn Lys Cys Val Asn Phe Asn Phe Asn Gly Leu Thr
    530                 535                 540

Gly Thr Gly Val Leu Thr Glu Ser Asn Lys Lys Phe Leu Pro Phe Gln
545                 550                 555                 560

Gln Phe Gly Arg Asp Ile Asp Asp Thr Thr Asp Ala Val Arg Asp Pro
                565                 570                 575

Gln Thr Leu Glu Ile Leu Asp Ile Thr Pro Cys Ser Phe Gly Gly Val
            580                 585                 590

Ser Val Ile Thr Pro Gly Thr Asn Thr Ser Asn Gln Val Ala Val Leu
        595                 600                 605

Tyr Gln Gly Val Asn Cys Thr Glu Val Pro Val Ala Ile His Ala Asp
    610                 615                 620

Gln Leu Thr Pro Thr Trp Arg Val Tyr Ser Thr Gly Ser Asn Val Phe
625                 630                 635                 640

Gln Thr Arg Ala Gly Cys Leu Ile Gly Ala Glu His Val Asn Asn Ser
                645                 650                 655

Tyr Glu Cys Asp Ile Pro Ile Gly Ala Gly Ile Cys Ala Ser Tyr Gln
            660                 665                 670

Thr Gln Thr Asn Ser His Arg Arg Ala Arg Ser Val Ala Ser Gln Ser
        675                 680                 685

Ile Ile Ala Tyr Thr Met Ser Leu Gly Ala Glu Asn Ser Val Ala Tyr
    690                 695                 700

Ser Asn Asn Ser Ile Ala Ile Pro Ile Asn Phe Thr Ile Ser Val Thr
705                 710                 715                 720

Thr Glu Ile Leu Pro Val Ser Met Thr Lys Thr Ser Val Asp Cys Thr
                725                 730                 735

Met Tyr Ile Cys Gly Asp Ser Thr Glu Cys Ser Asn Leu Leu Leu Gln
            740                 745                 750

Tyr Gly Ser Phe Cys Thr Gln Leu Asn Arg Ala Leu Thr Gly Ile Ala
        755                 760                 765

Val Glu Gln Asp Lys Asn Thr Gln Glu Val Phe Ala Gln Val Lys Gln
    770                 775                 780

Ile Tyr Lys Thr Pro Pro Ile Lys Asp Phe Gly Gly Phe Asn Phe Ser
785                 790                 795                 800

Gln Ile Leu Pro Asp Pro Ser Lys Pro Ser Lys Arg Ser Phe Ile Glu
                805                 810                 815

Asp Leu Leu Phe Asn Lys Val Thr Leu Ala Asp Ala Gly Phe Ile Lys
            820                 825                 830

Gln Tyr Gly Asp Cys Leu Gly Asp Ile Ala Ala Arg Asp Leu Ile Cys
        835                 840                 845

Ala Gln Lys Phe Asn Gly Leu Thr Val Leu Pro Pro Leu Leu Thr Asp
    850                 855                 860

Glu Met Ile Ala Gln Tyr Thr Ser Ala Leu Leu Ala Gly Thr Ile Thr

865                 870                 875                 880

Ser Gly Trp Thr Phe Gly Ala Gly Ala Ala Leu Gln Ile Pro Phe Ala
                885                 890                 895

Met Gln Met Ala Tyr Arg Phe Asn Gly Ile Gly Val Thr Gln Asn Val
            900                 905                 910

Leu Tyr Glu Asn Gln Lys Leu Ile Ala Asn Gln Phe Asn Ser Ala Ile
        915                 920                 925

Gly Lys Ile Gln Asp Ser Leu Ser Ser Thr Ala Ser Ala Leu Gly Lys
    930                 935                 940

Leu Gln Asp Val Val Asn Gln Asn Ala Gln Ala Leu Asn Thr Leu Val
945                 950                 955                 960

Lys Gln Leu Ser Ser Asn Phe Gly Ala Ile Ser Ser Val Leu Asn Asp
                965                 970                 975

Ile Leu Ala Arg Leu Asp Lys Val Glu Ala Glu Val Gln Ile Asp Arg
            980                 985                 990

Leu Ile Thr Gly Arg Leu Gln Ser  Leu Gln Thr Tyr Val  Thr Gln Gln
        995                 1000                1005

Leu Ile  Arg Ala Ala Glu Ile  Arg Ala Ser Ala Asn  Leu Ala Ala
    1010                1015                1020

Thr Lys  Met Ser Glu Cys Val  Leu Gly Gln Ser Lys  Arg Val Asp
    1025                1030                1035

Phe Cys  Gly Lys Gly Tyr His  Leu Met Ser Phe Pro  Gln Ser Ala
    1040                1045                1050

Pro His  Gly Val Val Phe Leu  His Val Thr Tyr Val  Pro Ala Gln
    1055                1060                1065

Glu Lys  Asn Phe Thr Thr Ala  Pro Ala Ile Cys His  Asp Gly Lys
    1070                1075                1080

Ala His  Phe Pro Arg Glu Gly  Val Phe Val Ser Asn  Gly Thr His
    1085                1090                1095

Trp Phe  Val Thr Gln Arg Asn  Phe Tyr Glu Pro Gln  Ile Ile Thr
    1100                1105                1110

Thr His Asn Thr Phe Val Ser Gly Asn Cys Asp Val Val Ile Gly
    1115                1120                1125

Ile Val Asn Asn Thr Val Tyr Asp Pro Leu Gln Pro Glu Leu Asp
    1130                1135                1140

Ser Phe Lys Glu Glu Leu Asp Lys Tyr Phe Lys Asn His Thr Ser
    1145                1150                1155

Pro Asp Val Asp Leu Gly Asp Ile Ser Gly Ile Asn Ala Ser Val
    1160                1165                1170

Val Asn Ile Gln Lys Glu Ile Asp Arg Leu Asn Glu Val Ala Lys
    1175                1180                1185

Asn Leu Asn Glu Ser Leu Ile Asp Leu Gln Glu Leu Gly Lys Tyr
    1190                1195                1200

Glu Gln Tyr Ile Lys Trp Pro Trp Tyr Ile Trp Leu Gly Phe Ile
    1205                1210                1215

Ala Gly Leu Ile Ala Ile Val Met Val Thr Ile Met Leu Cys Cys
    1220                1225                1230

Met Thr Ser Cys Cys Ser Cys Leu Lys Gly Cys Cys Ser Cys Gly
    1235                1240                1245

Ser Cys Cys Lys Phe Asp Glu Asp Asp Ser Glu Pro Val Leu Lys
    1250                1255                1260

Gly Val Lys Leu His Tyr Thr
    1265                1270

<210> 24
<211> 29832
<212> DNA
<213> SARS-CoV2

<400> 24
aaggtttata ccttcccagg taacaaacca accaactttc gatctcttgt agatctgttc 60

tctaaacgaa ctttaaaatc tgtgtggctg tcactcggct gcatgcttag tgcactcacg 120

cagtataatt aataactaat tactgtcgtt gacaggacac gagtaactcg tctatcttct 180

gcaggctgct tacggtttcg tccgtgttgc agccgatcat cagcacatct aggttttgtc 240

cgggtgtgac cgaaaggtaa gatggagagc cttgtccctg gtttcaacga gaaaacacac 300

gtccaactca gtttgcctgt tttacaggtt cgcgacgtgc tcgtacgtgg ctttggagac 360

tccgtggagg aggtcttatc agaggcacgt caacatctta aagatggcac ttgtggctta 420

gtagaagttg aaaaaggcgt tttgcctcaa cttgaacagc cctatgtgtt catcaaacgt 480

tcggatgctc gaactgcacc tcatggtcat gttatggttg agctggtagc agaactcgaa 540

ggcattcagt acggtcgtag tggtgagaca cttggtgtcc ttgtccctca tgtgggcgaa 600

ataccagtgg cttaccgcaa ggttcttctt cgtaagaacg gtaataaagg agctggtggc 660

catagttacg gcgccgatct aaagtcattt gacttaggcg acgagcttgg cactgatcct 720

tatgaagatt ttcaagaaaa ctggaacact aaacatagca gtggtgttac ccgtgaactc 780

atgcgtgagc ttaacggagg ggcatacact cgctatgtcg ataacaactt ctgtggccct 840

gatggctacc ctcttgagtg cattaaagac cttctagcac gtgctggtaa agcttcatgc 900

actttgtccg aacaactgga ctttattgac actaagaggg gtgtatactg ctgccgtgaa 960

catgagcatg aaattgcttg gtacacggaa cgttctgaaa agagctatga attgcagaca 1020

ccttttgaaa ttaaattggc aaagaaattt gacatcttca atggggaatg tccaaatttt 1080

gtatttccct taaattccat aatcaagact attcaaccaa gggttgaaaa gaaaaagctt 1140

gatggcttta tgggtagaat tcgatctgtc tatccagttg cgtcaccaaa tgaatgcaac 1200

caaatgtgcc tttcaactct catgaagtgt gatcattgtg gtgaaacttc atggcagacg 1260

ggcgattttg ttaaagccac ttgcgaattt tgtggcactg agaatttgac taaagaaggt 1320

gccactactt gtggttactt accccaaaat gctgttgtta aaatttattg tccagcatgt 1380

cacaattcag aagtaggacc tgagcatagt cttgccgaat accataatga atctggcttg 1440

aaaaccattc ttcgtaaggg tggtcgcact attgcctttg gaggctgtgt gttctcttat 1500

gttggttgcc ataacaagtg tgcctattgg gttccacgtg ctagcgctaa cataggttgt 1560

aaccatacag gtgttgttgg agaaggttcc gaaggtctta atgacaacct tcttgaaata 1620

ctccaaaaag agaaagtcaa catcaatatt gttggtgact ttaaacttaa tgaagagatc 1680

gccattattt tggcatcttt ttctgcttcc acaagtgctt ttgtggaaac tgtgaaaggt 1740

ttggattata aagcattcaa acaaattgtt gaatcctgtg gtaattttaa agttacaaaa 1800

ggaaaagcta aaaaaggtgc ctggaatatt ggtgaacaga aatcaatact gagtcctctt 1860

tatgcatttg catcagaggc tgctcgtgtt gtacgatcaa ttttctcccg cactcttgaa 1920

actgctcaaa attctgtgcg tgttttacag aaggccgcta taacaatact agatggaatt 1980

tcacagtatt cactgagact cattgatgct atgatgttca catctgattt ggctactaac 2040

aatctagttg taatggccta cattacaggt ggtgttgttc agttgacttc gcagtggcta 2100

actaacatct ttggcactgt ttatgaaaaa ctcaaacccg tccttgattg gcttgaagag 2160

aagtttaagg aaggtgtaga gtttcttaga gacggttggg aaattgttaa atttatctca 2220

acctgtgctt gtgaaattgt cggtggacaa attgtcacct gtgcaaagga aattaaggag 2280

agtgttcaga cattctttaa gcttgtaaat aaatttttgg ctttgtgtgc tgactctatc 2340

attattggtg gagctaaact taaagccttg aatttaggtg aaacatttgt cacgcactca 2400

aagggattgt acagaaagtg tgttaaatcc agagaagaaa ctggcctact catgcctcta 2460

aaagccccaa aagaaattat cttcttagag ggagaaacac ttcccacaga agtgttaaca 2520

gaggaagttg tcttgaaaac tggtgattta caaccattag aacaacctac tagtgaagct 2580

gttgaagctc cattggttgg tacaccagtt tgtattaacg ggcttatgtt gctcgaaatc 2640

aaagacacag aaaagtactg tgcccttgca cctaatatga tggtaacaaa caataccttc 2700

acactcaaag gcggtgcacc aacaaaggtt acttttggtg atgacactgt gatagaagtg 2760

caaggttaca agagtgtgaa tatcactttt gaacttgatg aaaggattga taaagtactt 2820

aatgagaagt gctctgccta tacagttgaa ctcggtacag aagtaaatga gttcgcctgt 2880

gttgtggcag atgctgtcat aaaaactttg caaccagtat ctgaattact tacaccactg 2940

ggcattgatt tagatgagtg gagtatggct acatactact tatttgatga gtctggtgag 3000

tttaaattgg cttcacatat gtattgttct ttttaccctc cagatgagga tgaagaagaa 3060

ggtgattgtg aagaagaaga gtttgagcca tcaactcaat atgagtatgg tactgaagat 3120

gattaccaag gtaaaccttt ggaatttggt gccacttctg ctgctcttca acctgaagaa 3180

gagcaagaag aagattggtt agatgatgat agtcaacaaa ctgttggtca acaagacggc 3240

agtgaggaca atcagacaac tactattcaa acaattgttg aggttcaacc tcaattagag 3300

atggaactta caccagttgt tcagactatt gaagtgaata gttttagtgg ttatttaaaa 3360

cttactgaca atgtatacat taaaaatgca gacattgtgg aagaagctaa aaaggtaaaa 3420

ccaacagtgg ttgttaatgc agccaatgtt taccttaaac atggaggagg tgttgcagga 3480

gccttaaata aggctactaa caatgccatg caagttgaat ctgatgatta catagctact 3540

aatggaccac ttaaagtggg tggtagttgt gttttaagcg gacacaatct tgctaaacac 3600

tgtcttcatg ttgtcggccc aaatgttaac aaaggtgaag acattcaact tcttaagagt 3660

gcttatgaaa attttaatca gcacgaagtt ctacttgcac cattattatc agctggtatt 3720

tttggtgctg accctataca ttctttaaga gtttgtgtag atactgttcg cacaaatgtc 3780

tacttagctg tctttgataa aaatctctat gacaaacttg tttcaagctt tttggaaatg 3840

aagagtgaaa agcaagttga acaaaagatc gctgagattc ctaaagagga agttaagcca 3900

tttataactg aaagtaaacc ttcagttgaa cagagaaaac aagatgataa gaaaatcaaa 3960

gcttgtgttg aagaagttac aacaactctg gaagaaacta agttcctcac agaaaacttg 4020

ttactttata ttgacattaa tggcaatctt catccagatt ctgccactct tgttagtgac 4080

attgacatca ctttcttaaa gaaagatgct ccatatatag tgggtgatgt tgttcaagag 4140

ggtgttttaa ctgctgtggt tatacctact aaaaaggctg gtggcactac tgaaatgcta 4200

gcgaaagctt tgagaaaagt gccaacagac aattatataa ccacttaccc gggtcagggt 4260

ttaaatggtt acactgtaga ggaggcaaag acagtgctta aaaagtgtaa aagtgccttt 4320

tacattctac catctattat ctctaatgag aagcaagaaa ttcttggaac tgtttcttgg 4380

aatttgcgag aaatgcttgc acatgcagaa gaaacacgca aattaatgcc tgtctgtgtg 4440

gaaactaaag ccatagtttc aactatacag cgtaaatata agggtattaa aatacaagag 4500

ggtgtggttg attatggtgc tagattttac ttttacacca gtaaaacaac tgtagcgtca 4560

cttatcaaca cacttaacga tctaaatgaa actcttgtta caatgccact tggctatgta 4620

acacatggct taaatttgga agaagctgct cggtatatga gatctctcaa agtgccagct 4680

acagtttctg tttcttcacc tgatgctgtt acagcgtata atggttatct tacttcttct 4740

tctaaaacac ctgaagaaca ttttattgaa accatctcac ttgctggttc ctataaagat 4800

tggtcctatt ctggacaatc tacacaacta ggtatagaat ttcttaagag aggtgataaa 4860

agtgtatatt acactagtaa tcctaccaca ttccacctag atggtgaagt tatcaccttt 4920

gacaatctta agacacttct ttctttgaga gaagtgagga ctattaaggt gtttacaaca 4980

gtagacaaca ttaacctcca cacgcaagtt gtggacatgt caatgacata tggacaacag 5040

tttggtccaa cttatttgga tggagctgat gttactaaaa taaaacctca taattcacat 5100

gaaggtaaaa cattttatgt tttacctaat gatgacactc tacgtgttga ggcttttgag 5160

tactaccaca caactgatcc tagttttctg ggtaggtaca tgtcagcatt aaatcacact 5220

aaaaagtgga aatacccaca agttaatggt ttaacttcta ttaaatgggc agataacaac 5280

tgttatcttg ccactgcatt gttaacactc caacaaatag agttgaagtt taatccacct 5340

gctctacaag atgcttatta cagagcaagg gctggtgaag ctgctaactt ttgtgcactt 5400

atcttagcct actgtaataa gacagtaggt gagttaggtg atgttagaga aacaatgagt 5460

tacttgtttc aacatgccaa tttagattct tgcaaaagag tcttgaacgt ggtgtgtaaa 5520

acttgtggac aacagcagac aacccttaag ggtgtagaag ctgttatgta catgggcaca 5580

ctttcttatg aacaatttaa gaaaggtgtt cagatacctt gtacgtgtgg taaacaagct 5640

acaaaatatc tagtacaaca ggagtcacct tttgttatga tgtcagcacc acctgctcag 5700

tatgaactta agcatggtac atttacttgt gctagtgagt acactggtaa ttaccagtgt 5760

ggtcactata aacatataac ttctaaagaa actttgtatt gcatagacgg tgctttactt 5820

acaaagtcct cagaatacaa aggtcctatt acggatgttt tctacaaaga aaacagttac 5880

acaacaacca taaaaccagt tacttataaa ttggatggtg ttgtttgtac agaaattgac 5940

cctaagttgg acaattatta taagaaagac aattcttatt tcacagagca accaattgat 6000

cttgtaccaa accaaccata tccaaacgca agcttcgata attttaagtt tgtatgtgat 6060

aatatcaaat ttgctgatga tttaaaccag ttaactggtt ataagaaacc tgcttcaaga 6120

gagcttaaag ttacattttt ccctgactta aatggtgatg tggtggctat tgattataaa 6180

cactacacac cctcttttaa gaaaggagct aaattgttac ataaacctat tgtttggcat 6240

gttaacaatg caactaataa agccacgtat aaaccaaata cctggtgtat acgttgtctt 6300

tggagcacaa aaccagttga aacatcaaat tcgtttgatg tactgaagtc agaggacgcg 6360

cagggaatgg ataatcttgt ctgcgaagat ctaaaaccag tctctgaaga agtagtggaa 6420

aatcctacca tacagaaaga cgttcttgag tgtaatgtga aaactaccga agttgtagga 6480

gacattatac ttaaaccagc aaataatagt ttaaaaatta cagaagaggt tggccacaca 6540

gatctaatgg ctgcttatgt agacaattct agtcttacta ttaagaaacc taatgaatta 6600

tctagagtat taggtttgaa aacccttgct actcatggtt tagctgctgt taatagtgtc 6660

ccttgggata ctatagctaa ttatgctaag ccttttctta acaaagttgt tagtacaact 6720

actaacatag ttacacggtg tttaaaccgt gtttgtacta attatatgcc ttatttcttt 6780

actttattgc tacaattgtg tacttttact agaagtacaa attctagaat taaagcatct 6840

atgccgacta ctatagcaaa gaatactgtt aagagtgtcg gtaaattttg tctagaggct 6900

tcatttaatt atttgaagtc acctaatttt tctaaactga taaatattat aatttggttt 6960

ttactattaa gtgtttgcct aggttcttta atctactcaa ccgctgcttt aggtgtttta 7020

atgtctaatt taggcatgcc ttcttactgt actggttaca gagaaggcta tttgaactct 7080

actaatgtca ctattgcaac ctactgtact ggttctatac cttgtagtgt ttgtcttagt 7140

ggtttagatt ctttagacac ctatccttct ttagaaacta tacaaattac catttcatct 7200

tttaaatggg atttaactgc ttttggctta gttgcagagt ggtttttggc atatattctt 7260

ttcactaggt ttttctatgt acttggattg gctgcaatca tgcaattgtt tttcagctat 7320

tttgcagtac attttattag taattcttgg cttatgtggt taataattaa tcttgtacaa 7380

atggccccga tttcagctat ggttagaatg tacatcttct ttgcatcatt ttattatgta 7440

tggaaaagtt atgtgcatgt tgtagacggt tgtaattcat caacttgtat gatgtgttac 7500

aaacgtaata gagcaacaag agtcgaatgt acaactattg ttaatggtgt tagaaggtcc 7560

ttttatgtct atgctaatgg aggtaaaggc ttttgcaaac tacacaattg gaattgtgtt 7620

aattgtgata cattctgtgc tggtagtaca tttattagtg atgaagttgc gagagacttg 7680

tcactacagt ttaaaagacc aataaatcct actgaccagt cttcttacat cgttgatagt 7740

gttacagtga agaatggttc catccatctt tactttgata aagctggtca aaagacttat 7800

gaaagacatt ctctctctca ttttgttaac ttagacaacc tgagagctaa taacactaaa 7860

ggttcattgc ctattaatgt tatagttttt gatggtaaat caaaatgtga agaatcatct 7920

gcaaaatcag cgtctgttta ctacagtcag cttatgtgtc aacctatact gttactagat 7980

caggcattag tgtctgatgt tggtgatagt gcggaagttg cagttaaaat gtttgatgct 8040

tacgttaata cgttttcatc aacttttaac gtaccaatgg aaaaactcaa aacactagtt 8100

gcaactgcag aagctgaact tgcaaagaat gtgtccttag acaatgtctt atctactttt 8160

atttcagcag ctcggcaagg gtttgttgat tcagatgtag aaactaaaga tgttgttgaa 8220

tgtcttaaat tgtcacatca atctgacata gaagttactg gcgatagttg taataactat 8280

atgctcacct ataacaaagt tgaaaacatg acaccccgtg accttggtgc ttgtattgac 8340

tgtagtgcgc gtcatattaa tgcgcaggta gcaaaaagtc acaacattgc tttgatatgg 8400

aacgttaaag atttcatgtc attgtctgaa caactacgaa aacaaatacg tagtgctgct 8460

aaaaagaata acttaccttt taagttgaca tgtgcaacta ctagacaagt tgttaatgtt 8520

gtaacaacaa agatagcact taagggtggt aaaattgtta ataattggtt gaagcagtta 8580

attaaagtta cacttgtgtt cctttttgtt gctgctattt tctatttaat aacacctgtt 8640

catgtcatgt ctaaacatac tgacttttca agtgaaatca taggatacaa ggctattgat 8700

ggtggtgtca ctcgtgacat agcatctaca gatacttgtt ttgctaacaa acatgctgat 8760

tttgacacat ggtttagcca gcgtggtggt agttatacta atgacaaagc ttgcccattg 8820

attgctgcag tcataacaag agaagtgggt tttgtcgtgc ctggtttgcc tggcacgata 8880

ttacgcacaa ctaatggtga ctttttgcat ttcttaccta gagtttttag tgcagttggt 8940

aacatctgtt acacaccatc aaaacttata gagtacactg actttgcaac atcagcttgt 9000

gttttggctg ctgaatgtac aatttttaaa gatgcttctg gtaagccagt accatattgt 9060

tatgatacca atgtactaga aggttctgtt gcttatgaaa gtttacgccc tgacacacgt 9120

tatgtgctca tggatggctc tattattcaa tttcctaaca cctaccttga aggttctgtt 9180

agagtggtaa caacttttga ttctgagtac tgtaggcacg gcacttgtga aagatcagaa 9240

gctggtgttt gtgtatctac tagtggtaga tgggtactta acaatgatta ttacagatct 9300

ttaccaggag ttttctgtgg tgtagatgct gtaaatttac ttactaatat gtttacacca 9360

ctaattcaac ctattggtgc tttggacata tcagcatcta tagtagctgg tggtattgta 9420

gctatcgtag taacatgcct tgcctactat tttatgaggt ttagaagagc ttttggtgaa 9480

tacagtcatg tagttgcctt taatacttta ctattcctta tgtcattcac tgtactctgt 9540

ttaacaccag tttactcatt cttacctggt gtttattctg ttatttactt gtacttgaca 9600

ttttatctta ctaatgatgt ttctttttta gcacatattc agtggatggt tatgttcaca 9660

cctttagtac ctttctggat aacaattgct tatatcattt gtatttccac aaagcatttc 9720

tattggttct ttactaatta cctaaagaga cgtgtagtct ttaatggtgt ttcctttagt 9780

acttttgaag aagctgcgct gtgcaccttt ttgttaaata aagaaatgta tctaaagttg 9840

cgtagtgatg tgctattacc tcttacgcaa tataatagat acttagctct ttataataag 9900

tacaagtatt ttagtggagc aatggataca actagctaca gagaagctgc ttgttgtcat 9960

ctcgcaaagg ctctcaatga cttcagtaac tcaggttctg atgttcttta ccaaccacca 10020

caaacctcta tcacctcagc tgttttgcag agtggtttta gaaaaatggc attcccatct 10080

ggtaaagttg agggttgtat ggtacaagta acttgtggta caactacact taacggtctt 10140

tggcttgatg acgtagttta ctgtccaaga catgtgatct gcacctctga agacatgctt 10200

aaccctaatt atgaagattt actcattcgt aagtctaatc ataatttctt ggtacaggct 10260

ggtaatgttc aactcagggt tattggacat tctatgcaaa attgtgtact taagcttaag 10320

gttgatacag ccaatcctaa gacacctaag tataagtttg ttcgcattca accaggacag 10380

actttttcag tgttagcttg ttacaatggt tcaccatctg gtgtttacca atgtgctatg 10440

aggcccaatt tcactattaa gggttcattc cttaatggtt catgtggtag tgttggtttt 10500

aacatagatt atgactgtgt ctctttttgt tacatgcacc atatggaatt accaactgga 10560

gttcatgctg gcacagactt agaaggtaac ttttatggac cttttgttga caggcaaaca 10620

gcacaagcag ctggtacgga cacaactatt acagttaatg ttttagcttg gttgtacgct 10680

gctgttataa atggagacag gtggtttctc aatcgattta ccacaactct taatgacttt 10740

aaccttgtgg ctatgaagta caattatgaa cctctaacac aagaccatgt tgacatacta 10800

ggacctcttt ctgctcaaac tggaattgcc gttttagata tgtgtgcttc attaaaagaa 10860

ttactgcaaa atggtatgaa tggacgtacc atattgggta gtgctttatt agaagatgaa 10920

tttacacctt ttgatgttgt tagacaatgc tcaggtgtta ctttccaaag tgcagtgaaa 10980

agaacaatca agggtacaca ccactggttg ttactcacaa ttttgacttc acttttagtt 11040

ttagtccaga gtactcaatg gtctttgttc ttttttttgt atgaaaatgc ctttttacct 11100

tttgctatgg gtattattgc tatgtctgct tttgcaatga tgtttgtcaa acataagcat 11160

gcatttctct gtttgttttt gttaccttct cttgccactg tagcttattt taatatggtc 11220

tatatgcctg ctagttgggt gatgcgtatt atgacatggt tggatatggt tgatactagt 11280

ttgtctggtt ttaagctaaa agactgtgtt atgtatgcat cagctgtagt gttactaatc 11340

cttatgacag caagaactgt gtatgatgat ggtgctagga gagtgtggac acttatgaat 11400

gtcttgacac tcgtttataa agtttattat ggtaatgctt tagatcaagc catttccatg 11460

tgggctctta taatctctgt tacttctaac tactcaggtg tagttacaac tgtcatgttt 11520

ttggccaaag gtattgtttt tatgtgtgtt gagtattgcc ctattttctt cataactggt 11580

aatacacttc agtgtataat gctagtttat tgtttcttag gctatttttg tacttgttac 11640

tttggcctct tttgtttact caaccgctac tttagactga ctcttggtgt ttatgattac 11700

ttagtttcta cacaggagtt tagatatatg aattcacagg gactactccc acccaagaat 11760

agcatagatg ccttcaaact caacattaaa ttgttgggtg ttggtggcaa accttgtatc 11820

aaagtagcca ctgtacagtc taaaatgtca gatgtaaagt gcacatcagt agtcttactc 11880

tcagttttgc aacaactcag agtagaatca tcatctaaat tgtgggctca atgtgtccag 11940

ttacacaatg acattctctt agctaaagat actactgaag cctttgaaaa aatggtttca 12000

ctactttctg ttttgctttc catgcagggt gctgtagaca taaacaagct ttgtgaagaa 12060

atgctggaca acagggcaac cttacaagct atagcctcag agtttagttc ccttccatca 12120

tatgcagctt ttgctactgc tcaagaagct tatgagcagg ctgttgctaa tggtgattct 12180

gaagttgttc ttaaaaagtt gaagaagtct ttgaatgtgg ctaaatctga atttgaccgt 12240

gatgcagcca tgcaacgtaa gttggaaaag atggctgatc aagctatgac ccaaatgtat 12300

aaacaggcta gatctgagga caagagggca aaagttacta gtgctatgca gacaatgctt 12360

ttcactatgc ttagaaagtt ggataatgat gcactcaaca acattatcaa caatgcaaga 12420

gatggttgtg ttcccttgaa cataatacct cttacaacag cagccaaatt aatggttgtc 12480

ataccagact ataacacata taaaaatacg tgtgatggta caacatttac ttatgcatca 12540

gcattgtggg aaatccaaca ggttgtagat gcagatagta aaattgttca acttagtgaa 12600

attagtatgg acaattcacc taatttagca tggcctctta ttgtaacagc tttaagggcc 12660

aattctgctg tcaaattaca gaataatgag cttagtcctg ttgcactacg acagatgtct 12720

tgtgctgccg gtactacaca aactgcttgc actgatgaca atgcgttagc ttactacaac 12780

acaacaaagg gaggtaggtt tgtacttgca ctgttatccg atttacagga tttgaaatgg 12840

gctagattcc ctaagagtga tggaactggt actatctata cagaactgga accaccttgt 12900

aggtttgtta cagacacacc taaaggtcct aaagtgaagt atttatactt tattaaagga 12960

ttaaacaacc taaatagagg tatggtactt ggtagtttag ctgccacagt acgtctacaa 13020

gctggtaatg caacagaagt gcctgccaat tcaactgtat tatctttctg tgcttttgct 13080

gtagatgctg ctaaagctta caaagattat ctagctagtg ggggacaacc aatcactaat 13140

tgtgttaaga tgttgtgtac acacactggt actggtcagg caataacagt tacaccggaa 13200

gccaatatgg atcaagaatc ctttggtggt gcatcgtgtt gtctgtactg ccgttgccac 13260

atagatcatc caaatcctaa aggattttgt gacttaaaag gtaagtatgt acaaatacct 13320

acaacttgtg ctaatgaccc tgtgggtttt acacttaaaa acacagtctg taccgtctgc 13380

ggtatgtgga aaggttatgg ctgtagttgt gatcaactcc gcgaacccat gcttcagtca 13440

gctgatgcac aatcgttttt aaacgggttt gcggtgtaag tgcagcccgt cttacaccgt 13500

gcggcacagg cactagtact gatgtcgtat acagggcttt tgacatctac aatgataaag 13560

tagctggttt tgctaaattc ctaaaaacta attgttgtcg cttccaagaa aaggacgaag 13620

atgacaattt aattgattct tactttgtag ttaagagaca cactttctct aactaccaac 13680

atgaagaaac aatttataat ttacttaaag attgtccagc tgttgctaaa catgacttct 13740

ttaagtttag aatagacggt gacatggtac cacatatatc acgtcaacgt cttactaaat 13800

acacaatggc agacctcgtc tatgctttaa ggcattttga tgaaggtaat tgtgacacat 13860

taaaagaaat acttgtcaca tacaattgtt gtgatgatga ttatttcaat aaaaaggact 13920

ggtatgattt tgtagaaaac ccagatatat tacgcgtata cgccaactta ggtgaacgtg 13980

tacgccaagc tttgttaaaa acagtacaat tctgtgatgc catgcgaaat gctggtattg 14040

ttggtgtact gacattagat aatcaagatc tcaatggtaa ctggtatgat ttcggtgatt 14100

tcatacaaac cacgccaggt agtggagttc ctgttgtaga ttcttattat tcattgttaa 14160

tgcctatatt aaccttgacc agggctttaa ctgcagagtc acatgttgac actgacttaa 14220

caaagcctta cattaagtgg gatttgttaa aatatgactt cacggaagag aggttaaaac 14280

tctttgaccg ttattttaaa tattgggatc agacatacca cccaaattgt gttaactgtt 14340

tggatgacag atgcattctg cattgtgcaa actttaatgt tttattctct acagtgttcc 14400

cacttacaag ttttggacca ctagtgagaa aaatatttgt tgatggtgtt ccatttgtag 14460

tttcaactgg ataccacttc agagagctag gtgttgtaca taatcaggat gtaaacttac 14520

atagctctag acttagtttt aaggaattac ttgtgtatgc tgctgaccct gctatgcacg 14580

ctgcttctgg taatctatta ctagataaac gcactacgtg cttttcagta gctgcactta 14640

ctaacaatgt tgcttttcaa actgtcaaac ccggtaattt taacaaagac ttctatgact 14700

ttgctgtgtc taagggtttc tttaaggaag gaagttctgt tgaattaaaa cacttcttct 14760

ttgctcagga tggtaatgct gctatcagcg attatgacta ctatcgttat aatctaccaa 14820

caatgtgtga tatcagacaa ctactatttg tagttgaagt tgttgataag tactttgatt 14880

gttacgatgg tggctgtatt aatgctaacc aagtcatcgt caacaaccta gacaaatcag 14940

ctggttttcc atttaataaa tggggtaagg ctagacttta ttatgattca atgagttatg 15000

aggatcaaga tgcacttttc gcatatacaa aacgtaatgt catccctact ataactcaaa 15060

tgaatcttaa gtatgccatt agtgcaaaga atagagctcg caccgtagct ggtgtctcta 15120

tctgtagtac tatgaccaat agacagtttc atcaaaaatt attgaaatca atagccgcca 15180

ctagaggagc tactgtagta attggaacaa gcaaattcta tggtggttgg cacaacatgt 15240

taaaaactgt ttatagtgat gtagaaaacc ctcaccttat gggttgggat tatcctaaat 15300

gtgatagagc catgcctaac atgcttagaa ttatggcctc acttgttctt gctcgcaaac 15360

atacaacgtg ttgtagcttg tcacaccgtt tctatagatt agctaatgag tgtgctcaag 15420

tattgagtga aatggtcatg tgtggcggtt cactatatgt taaaccaggt ggaacctcat 15480

caggagatgc cacaactgct tatgctaata gtgtttttaa catttgtcaa gctgtcacgg 15540

ccaatgttaa tgcactttta tctactgatg gtaacaaaat tgccgataag tatgtccgca 15600

atttacaaca cagactttat gagtgtctct atagaaatag agatgttgac acagactttg 15660

tgaatgagtt ttacgcatat ttgcgtaaac atttctcaat gatgatactc tctgacgatg 15720

ctgttgtgtg tttcaatagc acttatgcat ctcaaggtct agtggctagc ataaagaact 15780

ttaagtcagt tctttattat caaaacaatg tttttatgtc tgaagcaaaa tgttggactg 15840

agactgacct tactaaagga cctcatgaat tttgctctca acatacaatg ctagttaaac 15900

agggtgatga ttatgtgtac cttccttacc cagatccatc aagaatccta ggggccggct 15960

gttttgtaga tgatatcgta aaaacagatg gtacacttat gattgaacgg ttcgtgtctt 16020

tagctataga tgcttaccca cttactaaac atcctaatca ggagtatgct gatgtctttc 16080

atttgtactt acaatacata agaaagctac atgatgagtt aacaggacac atgttagaca 16140

tgtattctgt tatgcttact aatgataaca cttcaaggta ttgggaacct gagttttatg 16200

aggctatgta cacaccgcat acagtcttac aggctgttgg ggcttgtgtt ctttgcaatt 16260

cacagacttc attaagatgt ggtgcttgca tacgtagacc attcttatgt tgtaaatgct 16320

gttacgacca tgtcatatca acatcacata aattagtctt gtctgttaat ccgtatgttt 16380

gcaatgctct aggttgtgat gtcacagatg tgactcaact ttacttagga ggtatgagct 16440

attattgtaa atcacataaa ccacccatta gttttccatt gtgtgctaat ggacaagttt 16500

ttggtttata taaaaataca tgtgttggta gcgataatgt tactgacttt aatgcaattg 16560

caacatgtga ctggacaaat gctggtgatt acattttagc taacacctgt actgaaagac 16620

tcaagctttt tgcagcagaa acgctcaaag ctactgagga gacatttaaa ctgtcttatg 16680

gtattgctac tgtacgtgaa gtgctgtctg acagagaatt acatctttca tgggaagttg 16740

gtaaacctag accaccactt aaccgaaatt atgtctttac tggttatcgt gtaactaaaa 16800

acagtaaagt acaaatagga gagtacacct ttgaaaaagg tgactatggt gatgctgttg 16860

tttaccgagg tacaacaact tacaaattaa atgttggtga ttattttgtg ctgacatcac 16920

atacagtaat gccattaagt gcacctacac tagtgccaca agagcactat gttagaatta 16980

ctggcttata cccaacactc aatatctcat atgagttttc tagcaatgtt gcaaattatc 17040

aaaaggttgg tatgcaaaag tattctacac tccagggacc acctggtact ggtaagagtc 17100

attttgctat tggcctagct ctctactacc cttctgctcg catagtgtat acagcttgct 17160

ctcatgccgc tgttgatgca ctatgtgaga aggcattaaa atatttgcct atagataaat 17220

gtagtagaat tatacctgca cgtgctcgtg tagagtgttt tgataaattc aaagtgaatt 17280

caacattaga acagtatgtc ttttgtactg taaatgcatt gcctgagacg acagcagata 17340

tagttgtctt tgatgaaatt tcaatggcca caaattatga tttgagtgtt gtcaatgcca 17400

gattacgtgc taagcactat gtgtacattg gcgaccctgc tcaattacct gcaccacgca 17460

cattgctaac taagggcaca ctagaaccag aatatttcaa ttcagtgtgt agacttatga 17520

aaactatagg tccagacatg ttcctcggaa cttgtcggcg ttgtcctgct gaaattgttg 17580

acactgtgag tgctttggtt tatgataata agcttaaagc acataaagac aaatcagctc 17640

aatgctttaa aatgttttat aagggtgtta tcacgcatga tgtttcatct gcaattaaca 17700

ggccacaaat aggcgtggta agagaattcc ttacacgtaa ccctgcttgg agaaaagctg 17760

tctttatttc accttataat tcacagaatg ctgtagcctc aaagattttg ggactaccaa 17820

ctcaaactgt tgattcatca cagggctcag aatatgacta tgtcatattc actcaaacca 17880

ctgaaacagc tcactcttgt aatgtaaaca gatttaatgt tgctattacc agagcaaaag 17940

taggcatact ttgcataatg tctgatagag acctttatga caagttgcaa tttacaagtc 18000

ttgaaattcc acgtaggaat gtggcaactt tacaagctga aaatgtaaca ggactcttta 18060

aagattgtag taaggtaatc actgggttac atcctacaca ggcacctaca cacctcagtg 18120

ttgacactaa attcaaaact gaaggtttat gtgttgacat acctggcata cctaaggaca 18180

tgacctatag aagactcatc tctatgatgg gttttaaaat gaattatcaa gttaatggtt 18240

accctaacat gtttatcacc cgcgaagaag ctataagaca tgtacgtgca tggattggct 18300

tcgatgtcga ggggtgtcat gctactagag aagctgttgg taccaattta cctttacagc 18360

taggtttttc tacaggtgtt aacctagttg ctgtacctac aggttatgtt gatacaccta 18420

ataatacaga tttttccaga gttagtgcta aaccaccgcc tggagatcaa tttaaacacc 18480

tcataccact tatgtacaaa ggacttcctt ggaatgtagt gcgtataaag attgtacaaa 18540

tgttaagtga cacacttaaa aatctctctg acagagtcgt atttgtctta tgggcacatg 18600

gctttgagtt gacatctatg aagtattttg tgaaaatagg acctgagcgc acctgttgtc 18660

tatgtgatag acgtgccaca tgcttttcca ctgcttcaga cacttatgcc tgttggcatc 18720

attctattgg atttgattac gtctataatc cgtttatgat tgatgttcaa caatggggtt 18780

ttacaggtaa cctacaaagc aaccatgatc tgtattgtca agtccatggt aatgcacatg 18840

tagctagttg tgatgcaatc atgactaggt gtctagctgt ccacgagtgc tttgttaagc 18900

gtgttgactg gactattgaa tatcctataa ttggtgatga actgaagatt aatgcggctt 18960

gtagaaaggt tcaacacatg gttgttaaag ctgcattatt agcagacaaa ttcccagttc 19020

ttcacgacat tggtaaccct aaagctatta agtgtgtacc tcaagctgat gtagaatgga 19080

agttctatga tgcacagcct tgtagtgaca aagcttataa aatagaagaa ttattctatt 19140

cttatgccac acattctgac aaattcacag atggtgtatg cctattttgg aattgcaatg 19200

tcgatagata tcctgctaat tccattgttt gtagatttga cactagagtg ctatctaacc 19260

ttaacttgcc tggttgtgat ggtggcagtt tgtatgtaaa taaacatgca ttccacacac 19320

cagcttttga taaaagtgct tttgttaatt taaaacaatt accatttttc tattactctg 19380

acagtccatg tgagtctcat ggaaaacaag tagtgtcaga tatagattat gtaccactaa 19440

agtctgctac gtgtataaca cgttgcaatt taggtggtgc tgtctgtaga catcatgcta 19500

atgagtacag attgtatctc gatgcttata acatgatgat ctcagctggc tttagcttgt 19560

gggtttacaa acaatttgat acttataacc tctggaacac ttttacaaga cttcagagtt 19620

tagaaaatgt ggcttttaat gttgtaaata agggacactt tgatggacaa cagggtgaag 19680

taccagtttc tatcattaat aacactgttt acacaaaagt tgatggtgtt gatgtagaat 19740

tgtttgaaaa taaaacaaca ttacctgtta atgtagcatt tgagctttgg gctaagcgca 19800

acattaaacc agtaccagag gtgaaaatac tcaataattt gggtgtggac attgctgcta 19860

atactgtgat ctgggactac aaaagagatg ctccagcaca tatatctact attggtgttt 19920

gttctatgac tgacatagcc aagaaaccaa ctgaaacgat ttgtgcacca ctcactgtct 19980

tttttgatgg tagagttgat ggtcaagtag acttatttag aaatgcccgt aatggtgttc 20040

ttattacaga aggtagtgtt aaaggtttac aaccatctgt aggtcccaaa caagctagtc 20100

ttaatggagt cacattaatt ggagaagccg taaaaacaca gttcaattat tataagaaag 20160

ttgatggtgt tgtccaacaa ttacctgaaa cttactttac tcagagtaga aatttacaag 20220

aatttaaacc caggagtcaa atggaaattg atttcttaga attagctatg gatgaattca 20280

ttgaacggta taaattagaa ggctatgcct tcgaacatat cgtttatgga gattttagtc 20340

atagtcagtt aggtggttta catctactga ttggactagc taaacgtttt aaggaatcac 20400

cttttgaatt agaagatttt attcctatgg acagtacagt taaaaactat ttcataacag 20460

atgcgcaaac aggttcatct aagtgtgtgt gttctgttat tgatttatta cttgatgatt 20520

ttgttgaaat aataaaatcc caagatttat ctgtagtttc taaggttgtc aaagtgacta 20580

ttgactatac agaaatttca tttatgcttt ggtgtaaaga tggccatgta gaaacatttt 20640

acccaaaatt acaatctagt caagcgtggc aaccgggtgt tgctatgcct aatctttaca 20700

aaatgcaaag aatgctatta gaaaagtgtg accttcaaaa ttatggtgat agtgcaacat 20760

tacctaaagg cataatgatg aatgtcgcaa aatatactca actgtgtcaa tatttaaaca 20820

cattaacatt agctgtaccc tataatatga gagttataca ttttggtgct ggttctgata 20880

aaggagttgc accaggtaca gctgttttaa gacagtggtt gcctacgggt acgctgcttg 20940

tcgattcaga tcttaatgac tttgtctctg atgcagattc aactttgatt ggtgattgtg 21000

caactgtaca tacagctaat aaatgggatc tcattattag tgatatgtac gaccctaaga 21060

ctaaaaatgt tacaaaagaa aatgactcta aagagggttt tttcacttac atttgtgggt 21120

ttatacaaca aaagctagct cttggaggtt ccgtggctat aaagataaca gaacattctt 21180

ggaatgctga tctttataag ctcatgggac acttcgcatg gtggacagcc tttgttacta 21240

atgtgaatgc gtcatcatct gaagcatttt taattggatg taattatctt ggcaaaccac 21300

gcgaacaaat agatggttat gtcatgcatg caaattacat attttggagg aatacaaatc 21360

caattcagtt gtcttcctat tctttatttg acatgagtaa atttcccctt aaattaaggg 21420

gtactgctgt tatgtcttta aaagaaggtc aaatcaatga tatgatttta tctcttctta 21480

gtaaaggtag acttataatt agagaaaaca acagagttgt tatttctagt gatgttcttg 21540

ttaacaacta aacgaacaat gtttgttttt cttgttttat tgccactagt ctctattcag 21600

tgtgttaatc ttacaaccag aactcaatta ccccctgcat acactaattc tttcacacgt 21660

ggtgtttatt accctgacaa agttttcaga tcctcagttt tacattcaac tcaggacttg 21720

ttcttacctt tcttttccaa tgttacttgg ttccatgcta tacatgtctc tgggaccaat 21780

ggtactaaga ggtttgataa ccctgtccta ccatttaatg atggtgttta ttttgcttcc 21840

actgagaagt ctaacataat aagaggctgg atttttggta ctactttaga ttcgaagacc 21900

cagtccctac ttattgttaa taacgctact aatgttgtta ttaaagtctg tgaatttcaa 21960

ttttgtaatg atccattttt gggtgtttat taccacaaaa acaacaaaag ttgtatggaa 22020

agtgagttca gagtttattc tagtgcgaat aattgcactt ttgaatatgt ctctcagcct 22080

tttcttatgg accttgaagg aaaacagggt aatttcaaaa atcttaggga atttgtgttt 22140

aagaatattg atggttattt taaaatatat tctaagcaca cgcctattaa tttagtgcgt 22200

gatctccctc agggtttttc ggctttagaa ccattggtag atttgccaat aggtattaac 22260

atcactaggt ttcaaacttt acttgcttta catagaagtt atttgactcc tggtgattct 22320

tcttcaggtt ggacagctgg tgctgcagct tattatgtgg gttatcttca acctaggact 22380

tttctattaa aatataatga aaatggaacc attacagatg ctgtagactg tgcacttgac 22440

cctctctcag aaacaaagtg tacgttgaaa tccttcactg tagaaaaagg aatctatcaa 22500

acttctaact ttagagtcca accaacagaa tctattgtta gatttcctaa tattacaaac 22560

ttgtgccctt ttggtgaagt ttttaacgcc accagatttg catctgttta tgcttggaac 22620

aggaagagaa tcagcaactg tgttgctgat tattctgtcc tatataattc cgcatcattt 22680

tccactttta agtgttatgg agtgtctcct actaaattaa atgatctctg ctttactaat 22740

gtctatgcag attcatttgt aattagaggt gatgaagtca gacaaatcgc tccagggcaa 22800

actggaaaga ttgctgatta taattataaa ttaccagatg attttacagg ctgcgttata 22860

gcttggaatt ctaacaatct tgattctaag gttggtggta attataatta ccggtataga 22920

ttgtttagga agtctaatct caaacctttt gagagagata tttcaactga aatctatcag 22980

gccggtagca caccttgtaa tggtgttgaa ggttttaatt gttactttcc tttacaatca 23040

tatggtttcc aacccactaa tggtgttggt taccaaccat acagagtagt agtactttct 23100

tttgaacttc tacatgcacc agcaactgtt tgtggaccta aaaagtctac taatttggtt 23160

aaaaacaaat gtgtcaattt caacttcaat ggtttaacag gcacaggtgt tcttactgag 23220

tctaacaaaa agtttctgcc tttccaacaa tttggcagag acattgctga cactactgat 23280

gctgtccgtg atccacagac acttgagatt cttgacatta caccatgttc ttttggtggt 23340

gtcagtgtta taacaccagg aacaaatact tctaaccagg ttgctgttct ttatcagggt 23400

gttaactgca cagaagtccc tgttgctatt catgcagatc aacttactcc tacttggcgt 23460

gtttattcta caggttctaa tgtttttcaa acacgtgcag gctgtttaat aggggctgaa 23520

catgtcaaca actcatatga gtgtgacata cccattggtg caggtatatg cgctagttat 23580

cagactcaga ctaattctcc tcggcgggca cgtagtgtag ctagtcaatc catcattgcc 23640

tacactatgt cacttggtgc agaaaattca gttgcttact ctaataactc tattgccata 23700

cccacaaatt ttactattag tgttaccaca gaaattctac cagtgtctat gaccaagaca 23760

tcagtagatt gtacaatgta catttgtggt gattcaactg aatgcagcaa tcttttgttg 23820

caatatggca gtttttgtac acaattaaac cgtgctttaa ctggaatagc tgttgaacaa 23880

gacaaaaaca cccaagaagt ttttgcacaa gtcaaacaaa tttacaaaac accaccaatt 23940

aaagattttg gtggttttaa tttttcacaa atattaccag atccatcaaa accaagcaag 24000

aggtcattta ttgaagatct acttttcaac aaagtgacac ttgcagatgc tggcttcatc 24060

aaacaatatg gtgattgcct tggtgatatt gctgctagag acctcatttg tgcacaaaag 24120

tttaacggcc ttactgtttt gccacctttg ctcacagatg aaatgattgc tcaatacact 24180

tctgcactgt tagcgggtac aatcacttct ggttggacct ttggtgcagg tgctgcatta 24240

caaataccat ttgctatgca aatggcttat aggtttaatg gtattggagt tacacagaat 24300

gttctctatg agaaccaaaa attgattgcc aaccaattta atagtgctat tggcaaaatt 24360

caagactcac tttcttccac agcaagtgca cttggaaaac ttcaagatgt ggtcaaccaa 24420

aatgcacaag ctttaaacac gcttgttaaa caacttagct ccaattttgg tgcaatttca 24480

agtgttttaa atgatatcct ttcacgtctt gacaaagttg aggctgaagt gcaaattgat 24540

aggttgatca caggcagact tcaaagtttg cagacatatg tgactcaaca attaattaga 24600

gctgcagaaa tcagagcttc tgctaatctt gctgctacta aaatgtcaga gtgtgtactt 24660

ggacaatcaa aaagagttga tttttgtgga aagggctatc atcttatgtc cttccctcag 24720

tcagcacctc atggtgtagt cttcttgcat gtgacttatg tccctgcaca agaaaagaac 24780

ttcacaactg ctcctgccat ttgtcatgat ggaaaagcac actttcctcg tgaaggtgtc 24840

tttgtttcaa atggcacaca ctggtttgta acacaaagga atttttatga accacaaatc 24900

attactacag acaacacatt tgtgtctggt aactgtgatg ttgtaatagg aattgtcaac 24960

aacacagttt atgatccttt gcaacctgaa ttagactcat tcaaggagga gttagataaa 25020
tattttaaga atcatacatc accagatgtt gatttaggtg acatctctgg cattaatgct 25080
tcagttgtaa acattcaaaa agaaattgac cgcctcaatg aggttgccaa gaatttaaat 25140
gaatctctca tcgatctcca agaacttgga aagtatgagc agtatataaa atggccatgg 25200
tacatttggc taggttttat agctggcttg attgccatag taatggtgac aattatgctt 25260
tgctgtatga ccagttgctg tagttgtctc aagggctgtt gttcttgtgg atcctgctgc 25320
aaatttgatg aagacgactc tgagccagtg ctcaaaggag tcaaattaca ttacacataa 25380
acgaacttat ggatttgttt atgagaatct tcacaattgg aactgtaact ttgaagcaag 25440
gtgaaatcaa ggatgctact ccttcagatt ttgttcgcgc tactgcaacg ataccgatac 25500
aagcctcact ccctttcgga tggcttattg ttggcgttgc acttcttgct gtttttcata 25560
gcgcttccaa aatcataacc ctcaaaaaga gatggcaact agcactctcc aagggtgttc 25620
actttgtttg caacttgctg ttgttgtttg taacagttta ctcacacctt ttgctcgttg 25680
ctgctggcct tgaagcccct tttctctatc tttatgcttt agtctacttc ttgcagagta 25740
taaactttgt aagaataata atgaggcttt ggctttgctg gaaatgccgt tccaaaaacc 25800
cattacttta tgatgccaac tattttcttt gctggcatac taattgttac gactattgta 25860
taccttacaa tagtgtaact tcttcaattg tcattacttc aggtgatggc acaacaagtc 25920
ctatttctga acatgactac cagattggtg gttatactga aaaatgggaa tctggagtaa 25980
aagactgtgt tgtattacac agttacttca cttcagacta ttaccagctg tactcaactc 26040
aattgagtac agacactggt gttgaacatg ttaccttctt catctacaat aaaattgttg 26100
atgagcctga agaacatgtc caaattcaca caatcgacgg ttcatccgga gttgttaatc 26160
cagtaatgga accaatttat gatgaaccga cgacgactac tagcgtgcct ttgtaagcac 26220
aagctgatga gtacgaactt atgtactcat tcgtttcgga agagacaggt acgttaatag 26280
ttaatagcgt acttcttttt cttgctttcg tggtattctt gctagttaca ctagccatcc 26340
ttactgcgct tcgattgtgt gcgtactgct gcaatattgt taacgtgagt cttgtaaaac 26400
cttcttttta cgtttactct cgtgttaaaa atctgaattc ttctagagtt cctgatcttc 26460
tggtctaaac gaactaaata ttatattagt ttttctgttt ggaactttaa ttttagccat 26520
ggtagattcc aacggtacta ttaccgttga agagcttaaa aagctccttg aacaatggaa 26580
cctagtaata ggtttcctat tccttacatg gatttgtctt ctacaatttg cctatgccaa 26640
caggaatagg tttttgtata taattaagtt aatttttctc tggctgttat ggccagtaac 26700
tttagcttgt tttgtgcttg ctgctgttta cagaataaat tggatcaccg gtggaattgc 26760
tatcgcaatg gcttgtcttg taggcttgat gtggctcagc tacttcattg cttctttcag 26820
actgtttgcg cgtacgcgtt ccatgtggtc attcaatcca gaaactaaca ttcttctcaa 26880

cgtgccactc catggcacta ttctgaccag accgcttcta gaaagtgaac tcgtaatcgg 26940

agctgtgatc cttcgtggac atcttcgtat tgctggacac catctaggac gctgtgacat 27000

caaggacctg cctaaagaaa tcactgttgc tacatcacga acgctttctt attacaaatt 27060

gggagcttcg cagcgtgtag caggtgactc aggttttgct gcatacagtc gctacaggat 27120

tggcaactat aaattaaaca cagaccattc cagtagcagt gacaatattg ctttgcttgt 27180

acagtaagtg acaacagatg tttcatctcg ttgactttca ggttactata gcagagatat 27240

tactaattat tatgaggact tttaaagttt ccatttggaa tcttgattac atcataaacc 27300

tcataattaa aaatttatct aagtcactaa ctgagaataa atattctcaa ttagatgaag 27360

agcaaccaat ggagattgat taaacgaaca tgaaaattat tcttttcttg gcactgataa 27420

cactcgctac ttgtgagctt tatcactacc aagagtgtgt tagaggtaca acagtacttt 27480

taaaagaacc ttgctcttct ggaacatacg agggcaattc accatttcat cctctagctg 27540

ataacaaatt tgcactgact tgctttagca ctcaatttgc ttttgcttgt cctgacggcg 27600

taaaacacgt ctatcagtta cgtgccagat cagtttcacc taaactgttc atcagacaag 27660

aggaagttca agaactttac tctccaattt ttcttattgt tgcggcaata gtgtttataa 27720

cactttgctt cacactcaaa agaaagacag aatgattgaa ctttcattaa ttgacttcta 27780

tttgtgcttt ttagcctttc tgctattcct tgttttaatt atgcttatta tcttttggtt 27840

ctcacttgaa ctgcaagatc ataatgaaac ttgtcacgcc taaacgaaca tgaaatttct 27900

tgttttctta ggaatcatca caactgtagc tgcatttcac caagaatgta gtttacagtc 27960

atgtactcaa catcaaccat atgtagttga tgacccgtgt cctattcact tctattctaa 28020

atggtatatt agagtaggag ctagaaaatc agcaccttta attgaattgt gcgtggatga 28080

ggctggttct aaatcaccca ttcagtacat cgatatcggt aattatacag tttcctgttt 28140

accttttaca attaattgcc aggaacctaa attgggtagt cttgtagtgc gttgttcgtt 28200

ctatgaagac tttttagagt atcatgacgt tcgtgttgtt ttagatttca tctaaacgaa 28260

caaactataa tgtctgataa tggaccccaa aatcagcgaa atgcaccccg cattacgttt 28320

ggtggaccct cagattcaac tggcagtaac cagaatggag aacgcagtgg ggcgcgatca 28380

aaacaacgtc ggccccaagg tttacccaat aatactgcgt cttggttcac cgctctcact 28440

caacatggca aggaagacct taaattccct cgaggacaag gcgttccaat taacaccaat 28500

agcagtccag atgaccaaat tggctactac cgaagagcta ccagacgaat tcgtggtggt 28560

gacggtaaaa tgaaagatct cagtccaaga tggtatttct actacctagg aactgggcca 28620

gaagctggac ttccctatgg tgctaacaaa gacggcatca tatgggttgc aactgaggga 28680

gccttgaata caccaaaaga tcacattggc acccgcaatc ctgctaacaa tgctgcaatc 28740

gtgctacaac ttcctcaagg aacaacattg ccaaaaggct tctacgcaga agggagcaga 28800

ggcggcagtc aagcctcttc tcgttcctca tcacgtagtc gcaacagttc aagaaattca 28860

actccaggca gcagtagggg aatttctcct gctagaatgg ctggcaatgg cggtgatgct 28920

gctcttgctt tgctgctgct tgacagattg aaccagcttg agagcaaaat gtctggtaaa 28980

ggccaacaac aacaaggcca aactgtcact aagaaatctg ctgctgaggc ttctaagaag 29040

cctcggcaaa aacgtactgc cactaaagca tacaatgtaa cacaagcttt cggcagacgt 29100

ggtccagaac aaacccaagg aaattttggg gaccaggaac taatcagaca aggaactgat 29160

tacaaacatt ggccgcaaat tgcacaattt gcccccagcg cttcagcgtt cttcggaatg 29220

tcgcgcattg gcatggaagt cacaccttcg ggaacgtggt tgacctacac aggtgccatc 29280

aaattggatg acaaagatcc aaatttcaaa gatcaagtca ttttgctgaa taagcatatt 29340

gacgcataca aaacatttcc accaacagag cctaaaaagg acaaaaagaa gaaggctgat 29400

gaaactcaag ccttaccgca gagacagaag aaacagcaaa ctgtgactct tcttcctgct 29460

gcagatttgg atgatttctc caaacaattg caacaatcca tgagcagtgc tgactcaact 29520

caggcctaaa ctcatgcaga ccacacaagg cagatgggct atataaacgt tttcgctttt 29580

ccgtttacga tatatagtct actcttgtgc agaatgaatt ctcgtaacta catagcacaa 29640

gtagatgtag ttaactttaa tctcacatag caatctttaa tcagtgtgta acattaggga 29700

ggacttgaaa gagccaccac attttcaccg aggccacgcg gagtacgatc gagtgtacag 29760

tgaacaatgc tagggagagc tgcctatatg gaagagccct aatgtgtaaa attaatttta 29820

gtagtgctat cc 29832

<210> 25
<211> 1273
<212> PRT
<213> SARS-CoV2

<400> 25

Met Phe Val Phe Leu Val Leu Leu Pro Leu Val Ser Ile Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp

65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Cys Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Arg Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Gly Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg Leu Gln Ser Leu Gln Thr Tyr Val
        995                 1000                1005

Thr Gln Gln Leu Ile Arg Ala Ala Glu Ile Arg Ala Ser Ala Asn
    1010                1015                1020

Leu Ala Ala Thr Lys Met Ser Glu Cys Val Leu Gly Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His

1070                    1075                    1080

Asp Gly Lys Ala His Phe Pro Arg Glu Gly Val Phe Val Ser Asn
    1085                    1090                    1095

Gly Thr His Trp Phe Val Thr Gln Arg Asn Phe Tyr Glu Pro Gln
    1100                    1105                    1110

Ile Ile Thr Thr Asp Asn Thr Phe Val Ser Gly Asn Cys Asp Val
    1115                    1120                    1125

Val Ile Gly Ile Val Asn Asn Thr Val Tyr Asp Pro Leu Gln Pro
    1130                    1135                    1140

Glu Leu Asp Ser Phe Lys Glu Glu Leu Asp Lys Tyr Phe Lys Asn
    1145                    1150                    1155

His Thr Ser Pro Asp Val Asp Leu Gly Asp Ile Ser Gly Ile Asn
    1160                    1165                    1170

Ala Ser Val Val Asn Ile Gln Lys Glu Ile Asp Arg Leu Asn Glu
    1175                    1180                    1185

Val Ala Lys Asn Leu Asn Glu Ser Leu Ile Asp Leu Gln Glu Leu
    1190                    1195                    1200

Gly Lys Tyr Glu Gln Tyr Ile Lys Trp Pro Trp Tyr Ile Trp Leu
    1205                    1210                    1215

Gly Phe Ile Ala Gly Leu Ile Ala Ile Val Met Val Thr Ile Met
    1220                    1225                    1230

Leu Cys Cys Met Thr Ser Cys Cys Ser Cys Leu Lys Gly Cys Cys
    1235                    1240                    1245

Ser Cys Gly Ser Cys Cys Lys Phe Asp Glu Asp Asp Ser Glu Pro
    1250                    1255                    1260

Val Leu Lys Gly Val Lys Leu His Tyr Thr
    1265                    1270

<210>  26
<211>  29816
<212>  DNA
<213>  SARS-CoV2

<400>  26
actttcgatc tcttgtagat ctgttctcta aacgaacttt aaaatctgtg tggctgtcac        60

tcggctgcat gcttagtgca ctcacgcagt ataattaata actaattact gtcgttgaca 120

ggacacgagt aactcgtcta tcttctgcag gctgcttacg gtttcgtccg tgttgcagcc 180

gatcatcagc acatctaggt tttgtccggg tgtgaccgaa aggtaagatg gagagccttg 240

tccctggttt caacgagaaa acacacgtcc aactcagttt gcctgtttta caggttcgcg 300

acgtgctcgt acgtggcttt ggagactccg tggaggaggt cttatcagag gcacgtcaac 360

atcttaaaga tggcacttgt ggcttagtag aagttgaaaa aggcgttttg cctcaacttg 420

aacagcccta tgtgttcatc aaacgttcgg atgctcgaac tgcacctcat ggtcatgtta 480

tggttgagct ggtagcagaa ctcgaaggca ttcagtacgg tcgtagtggt gagacacttg 540

gtgtccttgt ccctcatgtg ggcgaaatac cagtggctta ccgcaaggtt cttcttcgta 600

agaacggtaa taaaggagct ggtggccata gttacggcgc cgatctaaag tcatttgact 660

taggcgacga gcttggcact gatccttatg aagattttca agaaaactgg aacactaaac 720

atagcagtgg tgttacccgt gaactcatgc gtgagcttaa cggaggggca tacactcgct 780

atgtcgataa caacttctgt ggccctgatg gctaccctct tgagtgcatt aaagaccttc 840

tagcacgtgc tggtaaagct tcatgcactt tgtccgaaca actggacttt attgacacta 900

agaggggtgt atactgctgc cgtgaacatg agcatgaaat tgcttggtac acggaacgtt 960

ctgaaaagag ctatgaattg cagacacctt ttgaaattaa attggcaaag aaatttgaca 1020

tcttcaatgg ggaatgtcca aattttgtat ttcccttaaa ttccataatc aagactattc 1080

aaccaagggt tgaaaagaaa aagcttgatg gctttatggg tagaattcga tctgtctatc 1140

cagttgcgtc accaaatgaa tgcaaccaaa tgtgcctttc aactctcatg aagtgtgatc 1200

attgtggtga aacttcatgg cagacgggcg attttgttaa agccacttgc gaattttgtg 1260

gcactgagaa tttgactaaa gaaggtgcca ctacttgtgg ttacttaccc caaaatgctg 1320

ttgttaaaat ttattgtcca gcatgtcaca attcagaagt aggacctgag catagtcttg 1380

ccgaatacca taatgaatct ggcttgaaaa ccattcttcg taagggtggt cgcactattg 1440

cctttggagg ctgtgtgttc tcttatgttg gttgccataa caagtgtgcc tattgggttc 1500

cacgtgctag cgctaacata ggttgtaacc atacaggtgt tgttggagaa ggttccgaag 1560

gtcttaatga caaccttctt gaaatactcc aaaaagagaa agtcaacatc aatattgttg 1620

gtgactttaa acttaatgaa gagatcgcca ttattttggc atctttttct gcttccacaa 1680

gtgcttttgt ggaaactgtg aaaggtttgg attataaagc attcaaacaa attgttgaat 1740

cctgtggtaa ttttaaagtt acaaaaggaa aagctaaaaa aggtgcctgg aatattggtg 1800

aacagaaatc aatactgagt cctctttatg catttgcatc agaggctgct cgtgttgtac 1860

gatcaatttt ctcccgcact cttgaaactg ctcaaaattc tgtgcgtgtt ttacagaagg 1920

ccgctataac aatactagat ggaatttcac agtattcact gagactcatt gatgctatga 1980

tgttcacatc tgatttggct actaacaatc tagttgtaat ggcctacatt acaggtggtg 2040

ttgttcagtt gacttcgcag tggctaacta acatctttgg cactgtttat gaaaaactca 2100

aacccgtcct tgattggctt gaagagaagt ttaaggaagg tgtagagttt cttagagacg 2160

gttgggaaat tgttaaattt atctcaacct gtgcttgtga aattgtcggt ggacaaattg 2220

tcacctgtgc aaaggaaatt aaggagagtg ttcagacatt ctttaagctt gtaaataaat 2280

ttttggcttt gtgtgctgac tctatcatta ttggtggagc taaacttaaa gccttgaatt 2340

taggtgaaac atttgttacg cactcaaagg gattgtacag aaagtgtgtt aaatccagag 2400

aagaaactgg cctactcatg cctctaaaag ccccaaaaga aattatcttc ttagagggag 2460

aaacacttcc cacagaagtg ttaacagagg aagttgtctt gaaaactggt gatttacaac 2520

cattagaaca acctactagt gaagctgttg aagctccact ggttggtaca ccagtttgta 2580

ttaacgggct tatgttgctc gaaatcaaag acacagaaaa gtactgtgcc cttgcaccta 2640

atatgatggt aacaaacaat accttcacac tcaaaggcgg tgcaccaaca aaggttactt 2700

ttggtgatga cactgtgata gaagtgcaag gttacaagag tgtgaatatc acttttgaac 2760

ttgatgaaag gattgataaa gtacttaatg agaagtgctc tgcctataca gttgaactcg 2820

gtacagaagt aaatgagttc gcctgtgttg tggcagatgc tgtcataaaa actttgcaac 2880

cagtatctga attacttaca ccactgggca ttgatttaga tgagtggagt atggctacat 2940

actacttatt tgatgagtct ggtgagttta aattggcttc acatatgtat tgttcttttt 3000

accctccaga tgaggatgaa gaagaaggtg attgtgaaga agaagagttt gagccatcaa 3060

ctcaatatga gtatggtact gaagatgatt accaaggtaa acctttggaa tttggtgcca 3120

cttctgctgc tcttcaacct gaagaagagc aagaagaaga ttggttagat gatgatagtc 3180

aacaaactgt tggtcaacaa gacggcagtg aggacaatca gacaactact attcaaacaa 3240

ttgttgaggt tcaacctcaa ttagagatgg aacttacacc agttgttcag actattgaag 3300

tgaatagttt tagtggttat ttaaaactta ctgacaatgt atacattaaa aatgcagaca 3360

ttgtggaaga agctaaaaag gtaaaaccaa cagtggttgt taatgcagcc aatgtttacc 3420

ttaaacatgg aggaggtgtt gcaggagcct taaataaggc tactaacaat gccatgcaag 3480

ttgaatctga tgattacata gctactaatg gaccacttaa agtgggtggt agttgtgttt 3540

taagcggaca caatcttgct aaacactgtc ttcatgttgt cggcccaaat gttaacaaag 3600

gtgaagacat tcaacttctt aagagtgctt atgaaaattt taatcagcac gaagttctac 3660

ttgcaccatt attatcagct ggtatttttg gtgctgaccc tatacattct ttaagagttt 3720

gtgtagatac tgttcgcaca aatgtctact tagctgtctt tgataaaaat ctctatgaca 3780

aacttgtttc aagctttttg gaaatgaaga gtgaaaagca agttgaacaa aagatcgctg 3840

agattcctaa agaggaagtt aagccattta taactgaaag taaaccttca gttgaacaga 3900

gaaaacaaga tgataagaaa atcaaagctt gtgttgaaga agttacaaca actctggaag 3960

aaactaagtt cctcacagaa aacttgttac tttatattga cattaatggc aatcttcatc 4020

cagattctgc cactcttgtt agtgacattg acatcacttt cttaaagaaa gatgctccat 4080

atatagtggg tgatgttgtt caagagggtg ttttaactgc tgtggttata cctactaaaa 4140

aggctggtgg cactactgaa atgctagcga aagctttgag aaaagtgcca acagacaatt 4200

atataaccac ttacccgggt cagggtttaa atggttacac tgtagaggag gcaaagacag 4260

tgcttaaaaa gtgtaaaagt gccttttaca ttctaccatc tattatctct aatgagaagc 4320

aagaaattct tggaactgtt tcttggaatt tgcgagaaat gcttgcacat gcagaagaaa 4380

cacgcaaatt aatgcctgtc tgtgtggaaa ctaaagccat agtttcaact atacagcgta 4440

aatataaggg tattaaaata caagagggtg tggttgatta tggtgctaga ttttactttt 4500

acaccagtaa aacaactgta gcgtcactta tcaacacact taacgatcta aatgaaactc 4560

ttgttacaat gccacttggc tatgtaacac atggcttaaa tttggaagaa gctgctcggt 4620

atatgagatc tctcaaagtg ccagctacag tttctgtttc ttcacctgat gctgttacag 4680

cgtataatgg ttatcttact tcttcttcta aaacacctga agaacatttt attgaaacca 4740

tctcacttgc tggttcctat aaagattggt cctattctgg acaatctaca caactaggta 4800

tagaatttct taagagaggt gataaaagtg tatattacac tagtaatcct accacattcc 4860

acctagatgg tgaagttatc acctttgaca atcttaagac acttctttct ttgagagaag 4920

tgaggactat taaggtgttt acaacagtag acaacattaa cctccacacg caagttgtgg 4980

acatgtcaat gacatatgga caacagtttg gtccaactta tttggatgga gctgatgtta 5040

ctaaaataaa acctcataat tcacatgaag gtaaaacatt ttatgtttta cctaatgatg 5100

acactctacg tgttgaggct tttgagtact accacacaac tgatcctagt tttctgggta 5160

ggtacatgtc agcattaaat cacactaaaa agtggaaata cccacaagtt aatggtttaa 5220

cttctattaa atgggcagat aacaactgtt atcttgccac tgcattgtta acactccaac 5280

aaatagagtt gaagtttaat ccacctgctc tacaagatgc ttattacaga gcaagggctg 5340

gtgaagctgc taacttttgt gcacttatct tagcctactg taataagaca gtaggtgagt 5400

taggtgatgt tagagaaaca atgagttact tgtttcaaca tgccaattta gattcttgca 5460

aaagagtctt gaacgtggtg tgtaaaactt gtggacaaca gcagacaacc cttaagggtg 5520

tagaagctgt tatgtacatg ggcacacttt cttatgaaca atttaagaaa ggtgttcaga 5580

taccttgtac gtgtggtaaa caagctacaa aatatctagt acaacaggag tcaccttttg 5640

ttatgatgtc agcaccacct gctcagtatg aacttaagca tggtacattt acttgtgcta 5700

gtgagtacac tggtaattac cagtgtggtc actataaaca tataacttct aaagaaactt 5760

tgtattgcat agacggtgct ttacttacaa agtcctcaga atacaaaggt cctattacgg 5820

atgttttcta caaagaaaac agttacacaa caaccataaa accagttact tataaattgg 5880

atggtgttgt ttgtacagaa attgacccta agttggacaa ttattataag aaagacaatt 5940

cttatttcac agagcaacca attgatcttg taccaaacca accatatcca aacgcaagct 6000

tcgataattt taagtttgta tgtgataata tcaaatttgc tgatgattta aaccagttaa 6060

ctggttataa gaaacctgct tcaagagagc ttaaagttac atttttccct gacttaaatg 6120

gtgatgtggt ggctattgat tataaacact acacaccctc ttttaagaaa ggagctaaat 6180

tgttacataa acctattgtt tggcatgtta acaatgcaac taataaagcc acgtataaac 6240

caaatacctg gtgtatacgt tgtctttgga gcacaaaacc agttgaaaca tcaaattcgt 6300

ttgatgtact gaagtcagag gacgcgcagg gaatggataa tcttgcctgc gaagatctaa 6360

aaccagtctc tgaagaagta gtggaaaatc ctaccataca gaaagacgtt cttgagtgta 6420

atgtgaaaac taccgaagtt gtaggagaca ttatacttaa accagcaaat aatagtttaa 6480

aaattacaga agaggttggc cacacagatc taatggctgc ttatgtagac aattctagtc 6540

ttactattaa gaaacctaat gaattatcta gagtattagg tttgaaaacc cttgctactc 6600

atggtttagc tgctgttaat agtgtccctt gggatactat agctaattat gctaagcctt 6660

ttcttaacaa agttgttagt acaactacta acatagttac acggtgttta aaccgtgttt 6720

gtactaatta tatgccttat ttctttactt tattgctaca attgtgtact tttactagaa 6780

gtacaaattc tagaattaaa gcatctatgc cgactactat agcaaagaat actgttaaga 6840

gtgtcggtaa attttgtcta gaggcttcat ttaattattt gaagtcacct aatttttcta 6900

aactgataaa tattataatt tggtttttac tattaagtgt ttgcctaggt tctttaatct 6960

actcaaccgc tgctttaggt gttttaatgt ctaatttagg catgccttct tactgtactg 7020

gttacagaga aggctatttg aactctacta atgtcactat tgcaacctac tgtactggtt 7080

ctataccttg tagtgtttgt cttagtggtt tagattcttt agacacctat ccttctttag 7140

aaactataca aattaccatt tcatctttta aatgggattt aactgctttt ggcttagttg 7200

cagagtggtt tttggcatat attcttttca ctaggttttt ctatgtactt ggattggctg 7260

caatcatgca attgtttttc agctattttg cagtacattt tattagtaat tcttggctta 7320

tgtggttaat aattaatctt gtacaaatgg ccccgatttc agctatggtt agaatgtaca 7380

tcttctttgc atcattttat tatgtatgga aaagttatgt gcatgttgta gacggttgta 7440

attcatcaac ttgtatgatg tgttacaaac gtaatagagc aacaagagtc gaatgtacaa 7500

ctattgttaa tggtgttaga aggtcctttt atgtctatgc taatggaggt aaaggctttt 7560

gcaaactaca caattggaat tgtgttaatt gtgatacatt ctgtgctggt agtacattta 7620

ttagtgatga agttgcgaga gacttgtcac tacagtttaa aagaccaata aatcctactg 7680

accagtcttc ttacatcgtt gatagtgtta cagtgaagaa tggttccatc catctttact 7740

ttgataaagc tggtcaaaag acttatgaaa gacattctct ctctcatttt gttaacttag 7800

acaacctgag agctaataac actaaaggtt cattgcctat taatgttata gtttttgatg 7860

gtaaatcaaa atgtgaagaa tcatctgcaa aatcagcgtc tgtttactac agtcagctta 7920

tgtgtcaacc tatactgtta ctagatcagg cattagtgtc tgatgttggt gatagtgcgg 7980

aagttgcagt taaaatgttt gatgcttacg ttaatacgtt ttcatcaact tttaacgtac 8040

caatggaaaa actcaaaaca ctagttgcaa ctgcagaagc tgaacttgca aagaatgtgt 8100

ccttagacaa tgtcttatct acttttattt cagcagctcg gcaagggttt gttgattcag 8160

atgtagaaac taaagatgtt gttgaatgtc ttaaattgtc acatcaatct gacatagaag 8220

ttactggcga tagttgtaat aactatatgc tcacctataa caaagttgaa aacatgacac 8280

cccgtgacct tggtgcttgt attgactgta gtgcgcgtca tattaatgcg caggtagcaa 8340

aaagtcacaa cattgctttg atatggaacg ttaaagattt catgtcattg tctgaacaac 8400

tacgaaaaca aatacgtagt gctgctaaaa agaataactt accttttaag ttgacatgtg 8460

caactactag acaagttgtt aatgttgtaa caacaaagat agcacttaag ggtggtaaaa 8520

ttgttaataa ttggttgaag cagttaatta aagttacact tgtgttcctt tttgttgctg 8580

ctattttcta tttaataaca cctgttcatg tcatgtctaa acatactgac ttttcaagtg 8640

aaatcatagg atacaaggct attgatggtg gtgtcactcg tgacatagca tctacagata 8700

cttgttttgc taacaaacat gctgattttg acacatggtt tagccagcgt ggtggtagtt 8760

atactaatga caaagcttgc ccattgattg ctgcagtcat aacaagagaa gtgggttttg 8820

tcgtgcctgg tttgcctggc acgatattac gcacaactaa tggtgacttt ttgcatttct 8880

tacctagagt ttttagtgca gttggtaata tctgttacac accatcaaaa cttatagagt 8940

acactgactt tgcaacatca gcttgtgttt tggctgctga atgtacaatt tttaaagatg 9000

cttctggtaa gccagtacca tattgttatg ataccaatgt actagaaggt tctgttgctt 9060

atgaaagttt acgccctgac acacgttatg tgctcatgga tggctctatt attcaatttc 9120

ctaacaccta ccttgaaggt tctgttagag tggtaacaac ttttgattct gagtactgta 9180

ggcacggcac ttgtgaaaga tcagaagctg gtgtttgtgt atctactagt ggtagatggg 9240

tacttaacaa tgattattac agatctttac caggagtttt ctgtggtgta gatgctgtaa 9300

atttacttac taatatgttt acaccactaa ttcaacctat tggtgctttg gacatatcag 9360

catctatagt agctggtggt attgtagcta tcgtagtaac atgccttgcc tactatttta 9420

tgaggtttag aagagctttt ggtgaataca gtcatgtagt tgcctttaat actttactat 9480

tccttatgtc attcactgta ctctgtttaa caccagttta ctcattctta cctggtgttt 9540

attctgttat ttacttgtac ttgacatttt atcttactaa tgatgtttct tttttagcac 9600

atattcagtg gatggttatg ttcacacctt tagtaccttt ctggataaca attgcttata 9660

tcatttgtat ttccacaaag catttctatt ggttctttag taattaccta aagagacgtg 9720

tagtctttaa tggtgtttcc tttagtactt ttgaagaagc tgcgctgtgc acctttttgt 9780

taaataaaga aatgtatcta aagttgcgta gtgatgtgct attacctctt acgcaatata 9840

atagatactt agctctttat aataagtaca agtattttag tggagcaatg gatacaacta 9900

gctacagaga agctgcttgt tgtcatctcg caaaggctct caatgacttc agtaactcag 9960

gttctgatgt tctttaccaa ccaccacaaa cctctatcac ctcagctgtt ttgcagagtg 10020

gttttagaaa aatggcattc ccatctggta aagttgaggg ttgtatggta caagtaactt 10080

gtggtacaac tacacttaac ggtctttggc ttgatgacgt agtttactgt ccaagacatg 10140

tgatctgcac ctctgaagac atgcttaacc ctaattatga agatttactc attcgtaagt 10200

ctaatcataa tttcttggta caggctggta atgttcaact cagggttatt ggacattcta 10260

tgcaaaattg tgtacttaag cttaaggttg atacagccaa tcctaagaca cctaagtata 10320

agtttgttcg cattcaacca ggacagactt tttcagtgtt agcttgttac aatggttcac 10380

catctggtgt ttaccaatgt gctatgaggc ccaatttcac tattaagggt tcattcctta 10440

atggttcatg tggtagtgtt ggttttaaca tagattatga ctgtgtctct ttttgttaca 10500

tgcaccatat ggaattacca actggagttc atgctggcac agacttagaa ggtaactttt 10560

atggaccttt tgttgacagg caaacagcac aagcagctgg tacggacaca actattacag 10620

ttaatgtttt agcttggttg tacgctgctg ttataaatgg agacaggtgg tttctcaatc 10680

gatttaccac aactcttaat gactttaacc ttgtggctat gaagtacaat tatgaacctc 10740

taacacaaga ccatgttgac atactaggac ctctttctgc tcaaactgga attgccgttt 10800

tagatatgtg tgcttcatta aaagaattac tgcaaaatgg tatgaatgga cgtaccatat 10860

tgggtagtgc tttattagaa gatgaattta caccttttga tgttgttaga caatgctcag 10920

gtgttacttt ccaaagtgca gtgaaaagaa caatcaaggg tacacaccac tggttgttac 10980

tcacaatttt gacttcactt ttagttttag tccagagtac tcaatggtct ttgttctttt 11040

ttttgtatga aaatgccttt ttaccttttg ctatgggtat tattgctatg tctgcttttg 11100

caatgatgtt tgtcaaacat aagcatgcat ttctctgttt gtttttgtta ccttctcttg 11160

ccactgtagc ttattttaat atggtctata tgcctgctag ttgggtgatg cgtattatga 11220

catggttgga tatggttgat actagtttgt ctggttttaa gctaaaagac tgtgttatgt 11280

atgcatcagc tgtagtgtta ctaatcctta tgacagcaag aactgtgtat gatgatggtg 11340

ctaggagagt gtggacactt atgaatgtct tgacactcgt ttataaagtt tattatggta 11400

atgctttaga tcaagccatt tccatgtggg ctcttataat ctctgttact tctaactact 11460

caggtgtagt tacaactgtc atgtttttgg ccagaggtat tgtttttatg tgtgttgagt 11520

attgccctat tttcttcata actggtaata cacttcagtg tataatgcta gtttattgtt 11580

tcttaggcta tttttgtact tgttactttg gcctcttttg tttactcaac cgctacttta 11640

gactgactct tggtgtttat gattacttag tttctacaca ggagtttaga tatatgaatt 11700

cacagggact actcccaccc aagaatagca tagatgcctt caaactcaac attaaattgt 11760

tgggtgttgg tggcaaacct tgtatcaaag tagccactgt acagtctaaa atgtcagatg 11820

taaagtgcac atcagtagtc ttactctcag ttttgcaaca actcagagta gaatcatcat 11880

ctaaattgtg ggctcaatgt gtccagttac acaatgacat tctcttagct aaagatacta 11940

ctgaagcctt tgaaaaaatg gtttcactac tttctgtttt gctttccatg cagggtgctg 12000

tagacataaa caagctttgt gaagaaatgc tggacaacag ggcaacctta caagctatag 12060

cttcagagtt tagttccctt ccatcatatg cagcttttgc tactgctcaa gaagcttatg 12120

agcaggctgt tgctaatggt gattctgaag ttgttcttaa aaagttgaag aagtctttga 12180

atgtggctaa atctgaattt gaccgtgatg cagccatgca acgtaagttg gaaaagatgg 12240

ctgatcaagc tatgacccaa atgtataaac aggctagatc tgaggacaag agggcaaaag 12300

ttactagtgc tatgcagaca atgcttttca ctatgcttag aaagttggat aatgatgcac 12360

tcaacaacat tatcaacaat gcaagagatg gttgtgttcc cttgaacata atacctctta 12420

caacagcagc caaactaatg gttgtcatac cagactataa cacatataaa aatacgtgtg 12480

atggtacaac atttacttat gcatcagcat tgtgggaaat ccaacaggtt gtagatgcag 12540

atagtaaaat tgttcaactt agtgaaatta gtatggacaa ttcacctaat ttagcatggc 12600

ctcttattgt aacagcttta agggccaatt ctgctgtcaa attacagaat aatgagctta 12660

gtcctgttgc actacgacag atgtcttgtg ctgccggtac tacacaaact gcttgcactg 12720

atgacaatgc gttagcttac tacaacacaa caaagggagg taggtttgta cttgcactgt 12780

tatccgattt acaggatttg aaatgggcta gattccctaa gagtgatgga actggtactg 12840

tctatacaga actggaacca ccttgtaggt ttgttacaga cacacctaaa ggtcctaaag 12900

tgaagtattt atactttatt aaaggattaa acaacctaaa tagaggtatg gtacttggta 12960

gtttagctgc cacagtacgt ctacaagctg gtaatgcaac agaagtgcct gccaattcaa 13020

ctgtattatc tttctgtgct tttgctgtag atgctgctaa agcttacaaa gattatctag 13080

ctagtggggg acaaccaatc actaattgtg ttaagatgtt gtgtacacac actggtactg 13140

gtcaggcaat aacagttaca ccggaagcca atatggatca agaatccttt ggtggtgcat 13200

cgtgttgtct gtactgccgt tgccacatag atcatccaaa tcctaaagga ttttgtgact 13260

taaaaggtaa gtatgtacaa atacctacaa cttgtgctaa tgaccctgtg ggttttacac 13320

ttaaaaacac agtctgtacc gtctgcggta tgtggaaagg ttatggctgt agttgtgatc 13380

aactccgcga acccatgctt cagtcagctg atgcacaatc gtttttaaac gggtttgcgg 13440

tgtaagtgca gcccgtctta caccgtgcgg cacaggcact agtactgatg tcgtatacag 13500

ggcttttgac atctacaatg ataaagtagc tggttttgct aaattcctaa aaactaattg 13560

ttgtcgcttc caagaaaagg acgaagatga caatttaatt gattcttact ttgtagttaa 13620

gagacacact ttctctaact accaacatga agaaacaatt tataatttac ttaaggattg 13680

tccagctgtt gctaaacatg acttctttaa gtttagaata gacggtgaca tggtaccaca 13740

tatatcacgt caacgtctta ctaaatacac aatggcagac ctcgtctatg ctttaaggca 13800

ttttgatgaa ggtaattgtg acacattaaa agaaatactt gtcacataca attgttgtga 13860

tgatgattat ttcaataaaa aggactggta tgattttgta gaaaacccag atatattacg 13920

cgtatacgcc aacttaggtg aacgtgtacg ccaagctttg ttaaaaacag tacaattctg 13980

tgatgccatg cgaaatgctg gtattgttgg tgtactgaca ttagataatc aagatctcaa 14040

tggtaactgg tatgatttcg gtgatttcat acaaaccacg ccaggtagtg gagttcctgt 14100

tgtagattct tattattcat tgttaatgcc tatattaacc ttgaccaggg ctttaactgc 14160

agagtcacat gttgacactg acttaacaaa gccttacatt aagtgggatt tgttaaaata 14220

tgacttcacg gaagagaggt taaaactctt tgaccgttat tttaaatatt gggatcagac 14280

ataccaccca aattgtgtta actgtttgga tgacagatgc attctgcatt gtgcaaactt 14340

taatgtttta ttctctacag tgttcccact tacaagtttt ggaccactag tgagaaaaat 14400

atttgttgat ggtgttccat ttgtagtttc aactggatac cacttcagag agctaggtgt 14460

tgtacataat caggatgtaa acttacatag ctctagactt agttttaagg aattacttgt 14520

gtatgctgct gaccctgcta tgcacgctgc ttctggtaat ctattactag ataaacgcac 14580

tacgtgcttt tcagtagctg cacttactaa caatgttgct tttcaaactg tcaaacccgg 14640

taattttaac aaagacttct atgactttgc tgtgtctaag ggtttcttta aggaaggaag 14700

ttctgttgaa ttaaaacact tcttctttgc tcaggatggt aatgctgcta tcagcgatta 14760

tgactactat cgttataatc taccaacaat gtgtgatatc agacaactac tatttgtagt 14820

tgaagttgtt gataagtact ttgattgtta cgatggtggc tgtattaatg ctaaccaagt 14880

catcgtcaac aacctagaca aatcagctgg ttttccattt aataaatggg gtaaggctag 14940

actttattat gattcaatga gttatgagga tcaagatgca cttttcgcat atacaaaacg 15000

taatgtcatc cctactataa ctcaaatgaa tcttaagtat gccattagtg caaagaatag 15060

agctcgcacc gtagctggtg tctctatctg tagtactatg accaatagac agtttcatca 15120

aaaattattg aaatcaatag ccgccactag aggagctact gtagtaattg gaacaagcaa 15180

attctatggt ggttggcaca acatgttaaa aactgtttat agtgatgtag aaaaccctca 15240

ccttatgggt tgggattatc ctaaatgtga tagagccatg cctaacatgc ttagaattat 15300

ggcctcactt gttcttgctc gcaaacatac aacgtgttgt agcttgtcac accgtttcta 15360

tagattagct aatgagtgtg ctcaagtatt gagtgaaatg gtcatgtgtg gcggttcact 15420

atatgttaaa ccaggtggaa cctcatcagg agatgccaca actgcttatg ctaatagtgt 15480

ttttaacatt tgtcaagctg tcacggccaa tgttaatgca cttttatcta ctgatggtaa 15540

caaaattgcc gataagtatg tccgcaattt acaacacaga ctttatgagt gtctctatag 15600

aaatagagat gttgacacag actttgtgaa tgagttttac gcatatttgc gtaaacattt 15660

ctcaatgatg atactctctg acgatgctgt tgtgtgtttc aatagcactt atgcatctca 15720

aggtctagtg gctagcataa agaactttaa gtcagttctt tattatcaaa acaatgtttt 15780

tatgtctgaa gcaaaatgtt ggactgagac tgaccttact aaaggacctc atgaattttg 15840

ctctcaacat acaatgctag ttaaacaggg tgatgattat gtgtaccttc cttacccaga 15900

tccatcaaga atcctagggg ccggctgttt tgtagatgat atcgtaaaaa cagatggtac 15960

acttatgatt gaacggttcg tgtctttagc tatagatgct tacccactta ctaaacatcc 16020

taatcaggag tatgctgatg tctttcattt gtacttacaa tacataagaa agctacatga 16080

tgagttaaca ggacacatgt tagacatgta ttctgttatg cttactaatg ataacacttc 16140

aaggtattgg gaacctgagt tttatgaggc tatgtacaca ccgcatacag tcttacaggc 16200

tgttggggct tgtgttcttt gcaattcaca gacttcatta agatgtggtg cttgcatacg 16260

tagaccattc ttatgttgta aatgctgtta cgaccatgtc atatcaacat cacataaatt 16320

agtcttgtct gttaatccgt atgtttgcaa tgctccaggt tgtgatgtca cagatgtgac 16380

tcaactttac ttaggaggta tgagctatta ttgtaaatca cataaaccac ccattagttt 16440

tccattgtgt gctaatggac aagtttttgg tttatataaa aatacatgtg ttggtagcga 16500

taatgttact gactttaatg caattgcaac atgtgactgg acaaatgctg gtgattacat 16560

tttagctaac acctgtactg aaagactcaa gctttttgca gcagaaacgc tcaaagctac 16620

tgaggagaca tttaaactgt cttatggtat tgctactgta cgtgaagtgc tgtctgacag 16680

agaattacat ctttcatggg aagttggtaa acctagacca ccacttaacc gaaattatgt 16740

ctttactggt tatcgtgtaa ctaaaaacag taaagtacaa ataggagagt acacctttga 16800

aaaaggtgac tatggtgatg ctgttgttta ccgaggtaca acaacttaca aattaaatgt 16860

tggtgattat tttgtgctga catcacatac agtaatgcca ttaagtgcac ctacactagt 16920

gccacaagag cactatgtta gaattactgg cttataccca acactcaata tctcatatga 16980

gttttctagc aatgttgcaa attatcaaaa ggttggtatg caaaagtatt ctacactcca 17040

gggaccacct ggtactggta agagtcattt tgctattggc ctagctctct actacccttc 17100

tgctcgcata gtgtatacag cttgctctca tgccgctgtt gatgcactat gtgagaaggc 17160

attaaaatat ttgcctatag ataaatgtag tagaattata cctgcacgtg ctcgtgtaga 17220

gtgttttgat aaattcaaag tgaattcaac attagaacag tatgtctttt gtactgtaaa 17280

tgcattgcct gagacgacag cagatatagt tgtctttgat gaaatttcaa tggccacaaa 17340

ttatgatttg agtgttgtca atgccagatt acgtgctaag cactatgtgt acattggcga 17400

ccctgctcaa ttacctgcac cacgcacatt gctaactaag ggcacactag aaccagaata 17460

tttcaattca gtgtgtagac ttatgaaaac tataggtcca gacatgttcc tcggaacttg 17520

tcggcgttgt cctgctgaaa ttgttgacac tgtgagtgct ttggtttatg ataataagct 17580

taaagcacat aaagacaaat cagctcaatg ctttaaaatg ttttataagg gtgttatcac 17640

gcatgatgtt tcatctgcaa ttaacaggcc acaaataggc gtggtaagag aattccttac 17700

acgtaaccct gcttggagaa aagctgtctt tatttcacct tataattcac agaatgctgt 17760

agcctcaaag attttgggac taccaactca aactgttgat tcatcacagg gctcagaata 17820

tgactatgtc atattcactc aaaccactga aacagctcac tcttgtaatg taaacagatt 17880

taatgttgct attaccagag caaaagtagg catactttgc ataatgtctg atagagacct 17940

ttatgacaag ttgcaattta caagtcttga aattccacgt aggaatgtgg caactttaca 18000

agctgaaaat gtaacaggac tctttaaaga ttgtagtaag gtaatcactg ggttacatcc 18060

tacacaggca cctacacacc tcagtgttga cactaaattc aaaactgaag gtttatgtgt 18120

tgacatacct ggcataccta aggacatgac ctatagaaga ctcatctcta tgatgggttt 18180

taaaatgaat tatcaagtta atggttaccc taacatgttt atcacccgcg aagaagctat 18240

aagacatgta cgtgcatgga ttggcttcga tgtcgagggg tgtcatgcta ctagagaagc 18300

tgttggtacc aatttacctt tacagctagg tttttctaca ggtgttaacc tagttgctgt 18360

acctacaggt tatgttgata cacctaataa tacagatttt tccagagtta gtgctaaacc 18420

accgcctgga gatcaattta aacacctcat accacttatg tacaaaggac ttccttggaa 18480

tgtagtgcgt ataaagattg tacaaatgtt aagtgacaca cttaaaaatc tctctgacag 18540

agtcgtattt gtcttatggg cacatggctt tgagttgaca tctatgaagt attttgtgaa 18600

aataggacct gagcgcacct gttgtctatg tgatagacgt gccacatgct tttccactgc 18660

ttcagacact tatgcctgtt ggcatcattc tattggattt gattacgtct ataatccgtt 18720

tatgattgat gttcaacaat ggggttttac aggtaaccta caaagcaacc atgatctgta 18780

ttgtcaagtc catggtaatg cacatgtagc tagttgtgat gcaatcatga ctaggtgtct 18840

agctgtccac gagtgctttg ttaagcgtgt tgactggact attgaatatc ctataattgg 18900

tgatgaactg aagattaatg cggcttgtag aaaggttcaa cacatggttg ttaaagctgc 18960

attattagca gacaaattcc cagttcttca cgacattggt aaccctaaag ctattaagtg 19020

tgtacctcaa gctgatgtag aatggaagtt ctatgatgca cagccttgta gtgacaaagc 19080

ttataaaata gaagaattat tctattctta tgccacacat tctgacaaat tcacagatgg 19140

tgtatgccta ttttggaatt gcaatgtcga tagatatcct gctaattcca ttgtttgtag 19200

atttgacact agagtgctat ctaaccttaa cttgcctggt tgtgatggtg gcagtttgta 19260

tgtaaataaa catgcattcc acacaccagc ttttgataaa agtgcttttg ttaatttaaa 19320

acaattacca tttttctatt actctgacag tccatgtgag tctcatggaa aacaagtagt 19380

gtcagatata gattatgtac cactaaagtc tgctacgtgt ataacacgtt gcaatttagg 19440

tggtgctgtc tgtagacatc atgctaatga gtacagattg tatctcgatg cttataacat 19500

gatgatctca gctggcttta gcttgtgggt ttacaaacaa tttgatactt ataacctctg 19560

gaacactttt acaagacttc agagtttaga aaatgtggct tttaatgttg taaataaggg 19620

acactttgat ggacaacagg gtgaagtacc agtttctatc attaataaca ctgtttacac 19680

aaaagttgat ggtgttgatg tagaattgtt tgaaaataaa acaacattac ctgttaatgt 19740

agcatttgag ctttgggcta agcgcaacat taaaccagta ccagaggtga aaatactcaa 19800

taatttgggt gtggacattg ctgctaatac tgtgatctgg gactacaaaa gagatgctcc 19860

agcacatata tctactattg gtgtttgttc tatgactgac atagccaaga aaccaactga 19920

aacgatttgt gcaccactca ctgtcttttt tgatggtaga gttgatggtc aagtagactt 19980

atttagaaat gcccgtaatg gtgttcttat tacagaaggt agtgttaaag gtttacaacc 20040

atctgtaggt cccaaacaag ctagtcttaa tggagtcaca ttaattggag aagccgtaaa 20100

aacacagttc aattattata agaaagttga tggtgttgtc caacaattac ctgaaactta 20160

ctttactcag agtagaaatt tacaagaatt taaacccagg agtcaaatgg aaattgattt 20220

cttagaatta gctatggatg aattcattga acggtataaa ttagaaggct atgccttcga 20280

acatatcgtt tatggagatt ttagtcatag tcagttaggt ggtttacatc tactgattgg 20340

actagctaaa cgttttaagg aatcaccttt tgaattagaa gattttattc ctatggacag 20400

tacagttaaa aactatttca taacagatgc gcaaacaggt tcatctaagt gtgtgtgttc 20460

tgttattgat ttattacttg atgattttgt tgaaataata aaatcccaag atttatctgt 20520

agtttctaag gttgtcaaag tgactattga ctatacagaa atttcattta tgctttggtg 20580

taaagatggc catgtagaaa cattttaccc aaaattacaa tctagtcaag cgtggcaacc 20640

gggtgttgct atgcctaatc tttacaaaat gcaaagaatg ctattagaaa agtgtgacct 20700

tcaaaattat ggtgatagtg caacattacc taaaggcata atgatgaatg tcgcaaaata 20760

tactcaactg tgtcaatatt taaacacatt aacattagct gtaccctata atatgagagt 20820

tatacatttt ggtgctggtt ctgataaagg agttgcacca ggtacagctg ttttaagaca 20880

gtggttgcct acgggtacgc tgcttgtcga ttcagatctt aatgactttg tctctgatgc 20940

agattcaact ttgattggtg attgtgcaac tgtacataca gctaataaat gggatctcat 21000

tattagtgat atgtacgacc ctaagactaa aaatgttaca aaagaaaatg actctaaaga 21060

gggttttttc acttacattt gtgggtttat acaacaaaag ctagctcttg gaggttccgt 21120

ggctataaag ataacagaac attcttggaa tgctgatctt tataagctca tgggacactt 21180

cgcatggtgg acagcctttg ttactaatgt gaatgcgtca tcatctgaag catttttaat 21240

tggatgtaat tatcttggca aaccacgcga acaaatagat ggttatgtca tgcatgcaaa 21300

ttacatattt tggaggaata caaatccaat tcagttgtct tcctattctt tatttgacat 21360

gagtaaattt ccccttaaat taaggggtac tgctgttatg tctttaaaag aaggtcaaat 21420

caatgatatg attttatctc ttcttagtaa aggtagactt ataattagag aaaacaacag 21480

agttgttatt tctagtgatg ttcttgttaa caactaaacg aacaatgttt gtttttcttg 21540

ttttattgcc actagtctct attcagtgtg ttaatcttac aaccagaact caattacccc 21600

ctgcatacac taattctttc acacgtggtg tttattaccc tgacaaagtt ttcagatcct 21660

cagttttaca ttcaactcag gacttgttct tacctttctt ttccaatgtt acttggttcc 21720

atgctataca tgtctctggg accaatggta ctaagaggtt tgataaccct gtcctaccat 21780

ttaatgatgg tgtttatttt gcttccactg agaagtctaa cataataaga ggctggattt 21840

ttggtactac tttagattcg aagacccagt ccctacttat tgttaataac gctactaatg 21900

ttgttattaa agtctgtgaa tttcaatttt gtaatgatcc atttttgggt gtttattacc 21960

acaaaaacaa caaaagttgt atggaaagtg agttcagagt ttattctagt gcgaataatt 22020

gcacttttga atatgtctct cagccttttc ttatggacct tgaaggaaaa cagggtaatt 22080

tcaaaaatct tagggaattt gtgtttaaga atattgatgg ttattttaaa atatattcta 22140

agcacacgcc tattaattta gtgcgtgatc tccctcaggg tttttcggct ttagaaccat 22200

tggtagattt gccaataggt attaacatca ctaggtttca aactttactt gctttacata 22260

gaagttattt gactcctggt gattcttctt caggttggac agctggtgct gcagcttatt 22320

atgtgggtta tcttcaacct aggacttttc tattaaaata taatgaaaat ggaaccatta 22380

cagatgctgt agactgtgca cttgaccctc tctcagaaac aaagtgtacg ttgaaatcct 22440

tcactgtaga aaaaggaatc tatcaaactt ctaactttag agtccaacca acagaatcta 22500

ttgttagatt tcctaatatt acaaacttgt gccctttggg tgaagttttt aacgccacca 22560

gatttgcatc tgtttatgct tggaacagga agagaatcag caactgtgtt gctgattatt 22620

ctgtcctata taattccgca tcattttcca cttttaagtg ttatggagtg tctcctacta 22680

aattaaatga tctctgcttt actaatgtct atgcagattc atttgtaatt agaggtgatg 22740

aagtcagaca aatcgctcca gggcaaactg gaaagattgc tgattataat tataaattac 22800

cagatgattt tacaggctgc gttatagctt ggaattctaa caatcttgat tctaaggttg 22860

gtggtaatta taattaccgg tatagattgt ttaggaagtc taatctcaaa ccttttgaga 22920

gagatatttc aactgaaatc tatcaggccg gtagcacacc ttgtaatggt gttgaaggtt 22980

ttaattgtta ctttccttta caatcatatg gtttccaacc cactaatggt gttggttacc 23040

aaccatacag agtagtagta ctttcttttg aacttctaca tgcaccagca actgtttgtg 23100

gacctaaaaa gtctactaat ttggttaaaa acaaatgtgt caatttcaac ttcaatggtt 23160

taacaggcac aggtgttctt actgagtcta acaaaaagtt tctgcctttc caacaatttg 23220

gcagagacat tgctgacact actgatgctg tccgtgatcc acagacactt gagattcttg 23280

acattacacc atgttctttt ggtggtgtca gtgttataac accaggaaca aatacttcta 23340

accaggttgc tgttctttat caggtgttaa actgcacaga agtccctgtt gctattcatg 23400

cagatcaact tactcctact tggcgtgttt attctacagg ttctaatgtt tttcaaacac 23460

gtgcaggctg tttaataggg gctgaacatg tcaacaactc atatgagtgt gacataccca 23520

ttggtgcagg tatatgcgct agttatcaga ctcagactaa ttctcctcgg cgggcacgta 23580

gtgtagctag tcaatccatc attgcctaca ctatgtcact tggtgcagaa aattcagttg 23640

cttactctaa taactctatt gccataccca caaattttac tattagtgtt accacagaaa 23700

ttctaccagt gtctatgacc aagacatcag tagattgtac aatgtacatt tgtggtgatt 23760

caactgaatg cagcaatctt ttgttgcaat atggcagttt ttgtacacaa ttaaaccgtg 23820

ctttaactgg aatagctgtt gaacaagaca aaaacaccca agaagttttt gcacaagtca 23880

aacaaattta caaaacacca ccaattaaag attttggtgg ttttaatttt tcacaaatat 23940

taccagatcc atcaaaacca agcaagaggt catttattga agatctactt ttcaacaaag 24000

tgacacttgc agatgctggc ttcatcaaac aatatggtga ttgccttggt gatattgctg 24060

ctagagacct catttgtgca caaaagttta acggccttac tgttttgcca cctttgctca 24120

cagatgaaat gattgctcaa tacacttctg cactgttagc gggtacaatc acttctggtt 24180

ggacctttgg tgcaggtgct gcattacaaa taccatttgc tatgcaaatg gcttataggt 24240

ttaatggtat tggagttaca cagaatgttc tctatgagaa ccaaaaattg attgccaacc 24300

aatttaatag cgctattggc aaaattcaag actcactttc ttccacagca agtgcacttg 24360

gaaaacttca agatgtggtc aaccaaaatg cacaagcttt aaacacgctt gttaaacaac 24420

ttagctccaa ttttggtgca atttcaagtg ttttaaatga tatcctttca cgtcttgaca 24480

aagttgaggc tgaagtgcaa attgataggt tgatcacagg cagacttcaa agtttgcaga 24540

catatgtgac tcaacaatta attagagctg cagaaatcag agcttctgct aatcttgctg 24600

ctactaaaat gtcagagtgt gtacttggac aatcaaaaag agttgatttt tgtggaaagg 24660

gctatcatct tatgtccttc cctcagtcag cacctcatgg tgtagtcttc ttgcatgtga 24720

cttatgtccc tgcacaagaa aagaacttca caactgctcc tgccatttgt catgatggaa 24780

aagcacactt tcctcgtgaa ggtgtctttg tttcaaatgg cacacactgg tttgtaacac 24840

aaaggaattt ttatgaacca caaatcatta ctacagacaa cacatttgtg tctggtaact 24900

gtgatgttgt aataggaatt gtcaacaaca cagtttatga tcctttgcaa cctgaattag 24960

actcattcaa ggaggagtta gataaatatt ttaagaatca tacatcacca gatgttgatt 25020

taggtgacat ctctggcatt aatgcttcag ttgtaaacat tcaaaaagaa attgaccgcc 25080

tcaatgaggt tgccaagaat ttaaatgaat ctctcatcga tctccaagaa cttggaaagt 25140

atgagcagta tataaaatgg ccatggtaca tttggctagg ttttatagct ggcttgattg 25200

ccatagtaat ggtgacaatt atgctttgct gtatgaccag ttgctgtagt tgtctcaagg 25260

gctgttgttc ttgtggatcc tgctgcaaat ttgatgaaga cgactctgag ccagtgctca 25320

aaggagtcaa attacattac acataaacga acttatggat ttgtttatga gaatcttcac 25380

aattggaact gtaactttga agcaaggtga aatcaaggat gctactcctt cagattttgt 25440

tcgcgctact gcaacgatac cgatacaagc ctcactccct ttcggatggc ttattgttgg 25500

cgttgcactt cttgctgttt ttcatagcgc ttccaaaatc ataaccctca aaaagagatg 25560

gcaactagca ctctccaagg gtgttcactt tgtttgcaac ttgctgttgt tgtttgtaac 25620

agtttactca caccttttgc tcgttgctgt tggccttgaa gccccttttc tctatcttta 25680

tgctttagtc tacttcttgc agagtataaa ctttgtaaga ataataatga ggctttggct 25740

ttgctggaaa tgccgttcca aaaacccatt actttatgat gccaactatt ttctttgctg 25800

gcatactaat tgttacgact attgtatacc ttacaatagt gtaacttctt caattgtcat 25860

tacttcaggt gatggcacaa caagtcctat ttctgaacat gactaccaga ttggtggtta 25920

tactgaaaaa tgggaatctg gagtaaaaga ctgtgttgta ttacacagtt acttcacttc 25980

agactattac cagctgtact caactcaatt gagtacagac actggtgttg aacatgttac 26040

cttcttcatc tacaataaaa ttgttgatga gcctgaagaa catgtccaaa ttcacacaat 26100

cgacggttca tccggagttg ttaatccagt aatggaacca atttatgatg aaccgacgac 26160

gactactagc gtgcctttgt aagcacaagc tgatgagtac gaacttatgt actcattcgt 26220

ttcggaagag acaggtacgt taatagttaa tagcgtactt ctttttcttg ctttcgtggt 26280

attcttgcta gttacactag ccatccttac tgcgcttcga ttgtgtgcgt actgctgcaa 26340

tattgttaac gtgagtcttg taaaaccttc tttttacgtt tactctcgtg ttaaaaatct 26400

gaattcttct agagttcctg atcttctggt ctaaacgaac taaatattat attagttttt 26460

ctgtttggaa ctttaatttt agccatggca gattccaacg gtactattac cgttgaagag 26520

cttaaaaagc tccttgaaca atggaaccta gtaataggtt tcctattcct tacatggatt 26580

tgtcttctac aatttgccta tgccaacagg aataggtttt tgtatataat taagttaatt 26640

tttctctggc tgttatggcc agtaacttta gcttgttttg tgcttgctgc tgtttacaga 26700

ataaattgga tcaccggtgg aattgctatc gcaatggctt gtcttgtagg cttgatgtgg 26760

ctcagctact tcattgcttc tttcagactg tttgcgcgta cgcgttccat gtggtcattc 26820

aatccagaaa ctaacattct tctcaacgtg ccactccatg gcactattct gaccagaccg 26880

cttctagaaa gtgaactcgt aatcggagct gtgatccttc gtggacatct tcgtattgct 26940

ggacaccatc taggacgctg tgacatcaag gacctgccta aagaaatcac tgttgctaca 27000

tcacgaacgc tttcttatta caaattggga gcttcgcagc gtgtagcagg tgactcaggt 27060

tttgctgcat acagtcgcta caggattggc aactataaat taaacacaga ccattccagt 27120

agcagtgaca atattgcttt gcttgtacag taagtgacaa cagatgtttc atctcgttga 27180

ctttcaggtt actatagcag agatattact aattattatg aggactttta aagtttccat 27240

ttggaatctt gattacatca taaacctcat aattaaaaat ttatctaagt cactaactga 27300

gaataaatat tctcaattag atgaagagca accaatggag attgattaaa cgaacatgaa 27360

aattattctt ttcttggcac tgataacact cgctacttgt gagctttatc actaccaaga 27420

gtgtgttaga ggtacaacag tacttttaaa agaaccttgc tcttctggaa catacgaggg 27480

caattcacca tttcatcctc tagctgataa caaatttgca ctgacttgct ttagcactca 27540

atttgctttt gcttgtcctg acggcgtaaa acacgtctat cagttacgtg ccagatcagt 27600

ttcacctaaa ctgttcatca gacaagagga agttcaagaa ctttactctc caatttttct 27660

tattgttgcg gcaatagtgt ttataacact ttgcttcaca ctcaaaagaa agacagaatg 27720

attgaacttt cattaattga cttctatttg tgctttttag cctttctgct attccttgtt 27780

ttaattatgc ttattatctt ttggttctca cttgaactgc aagatcataa tgaaacttgt 27840

cacgcctaaa ctaacatgaa atttcttgtt ttcttaggaa tcatcacaac tgtagctgca 27900

tttcaccaag aatgtagttt acagtcatgt actcaacatc aaccatatgt agttgatgac 27960

ccgtgtccta ttcacttcta ttctaaatgg tatattagag taggagctag aaaatcagca 28020

cctttaattg aattgtgcgt ggatgaggct ggttctaaat cacccattca gtacatcgat 28080

atcggtaatt atacagtttc ctgtttacct tttacaatta attgccagga acctaaattg 28140

ggtagtcttg tagtgcgttg ttcgttctat gaagactttt tagagtatca tgacgttcgt 28200

gttgttttag atttcatcta aacgaacaaa ctataatgtc tgataatgga ccccaaaatc 28260

agcgaaatgc accccgcatt acgtttggtg gaccctcaga ttcaactggc agtaaccaga 28320

atggagaacg cagtggggcg cgatcaaaac aacgtcggcc ccaaggttta cccaataata 28380

ctgcgtcttg gttcaccgct ctcactcaac atggcaagga agaccttaaa ttccctcgag 28440

gacaaggcgt tccaattaac accaatagca gtccagatga ccaaattggc tactaccgaa 28500

gagctaccag acgaattcgt ggtggtgacg gtaaaatgaa agatctcagt ccaagatggt 28560

atttctacta cctaggaact gggccagaag ctggacttcc ctatggtgct aacaaagacg 28620

gcatcatatg ggttgcaact gagggagcct tgaatacacc aaaagatcac attggcaccc 28680

gcaatcctgc taacaatgct gcaatcgtgc tacaacttcc tcaaggaaca acattgccaa 28740

aaggcttcta cgcagaaggg agcagaggcg gcagtcaagc ctcttctcgt tcctcatcac 28800

gtagtcgcaa cagttcaaga aattcaactc caggcagcag taggggaatt tctcctgcta 28860

gaatggctgg caatggcggt gatgctgctc ttgctttgct gctgcttgac agattgaacc 28920

agcttgagag caaaatgtct ggtaaaggcc aacaacaaca aggccaaact gtcactaaga 28980

aatctgctgc tgaggcttct aagaagcctc ggcaaaaacg tactgccact aaagcataca 29040

atgtaacaca agctttcggc agacgtggtc cagaacaaac ccaaggaaat tttggggacc 29100

aggaactaat cagacaagga actgattaca aacattggcc gcaaattgca caatttgccc 29160

ccagcgcttc agcgttcttc ggaatgtcgc gcattggcat ggaagtcaca ccttcgggaa 29220

cgtggttgac ctacacaggt gccatcaaat tggatgacaa agatccaaat ttcaaagatc 29280

aagtcatttt gctgaataag catattgacg catacaaaac atttccacca acagagccta 29340

aaaaggacaa aaagaagaag gctgatgaaa ctcaagcctt accgcagaga cagaagaaac 29400

agcaaactgt gactcttctt cctgctgcag atttggatga tttctccaaa caattgcaac 29460

aatccatgag cagtgctgac tcaactcagg cctaaactca tgcagaccac acaaggcaga 29520

tgggctatat aaacgttttc gcttttccgt ttacgatata tagtctactc ttgtgcagaa 29580

tgaattctcg taactacata gcacaagtag atgtagttaa ctttaatctc acatagcaat 29640

ctttaatcag tgtgtaacat tagggaggac ttgaaagagc caccacattt tcaccgaggc 29700

cacgcggagt acgatcgagt gtacagtgaa caatgctagg gagagctgcc tatatggaag 29760

agccctaatg tgtaaaatta attttagtag tgctatcccc atgtgatttt aatagc 29816

<210> 27
<211> 1273
<212> PRT
<213> SARS-CoV2

<400> 27

Met Phe Val Phe Leu Val Leu Leu Pro Leu Val Ser Ile Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Cys Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Arg Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Gly Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe

785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Thr Lys Met Ser  Glu Cys Val Leu Gly  Gln Ser Lys
    1025                1030                1035

Arg Val Asp Phe Cys Gly Lys Gly Tyr His Leu Met Ser Phe Pro
    1040                1045                1050

Gln Ser Ala Pro His Gly Val Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Ala His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Asp Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Ile Asp Arg Leu Asn Glu
    1175                1180                1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210>  28
<211>  419
<212>  PRT
<213>  SARS-CoV2

<400>  28

Met Ser Asp Asn Gly Pro Gln Asn Gln Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Ala Arg Ser Lys Gln Arg Arg Pro Gln Gly Leu Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Pro
65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Ser Arg Asn
            180                 185                 190

Ser Ser Arg Asn Ser Thr Pro Gly Ser Ser Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Gln Leu Glu Ser Lys Met Ser Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Leu Ile Arg Gln Gly Thr Asp Tyr Lys His Trp Pro Gln Ile
    290                 295                 300

Ala Gln Phe Ala Pro Ser Ala Ser Ala Phe Phe Gly Met Ser Arg Ile
305                 310                 315                 320

Gly Met Glu Val Thr Pro Ser Gly Thr Trp Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro
        355                 360                 365

Lys Lys Asp Lys Lys Lys Lys Ala Asp Glu Thr Gln Ala Leu Pro Gln
    370                 375                 380

Arg Gln Lys Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  29
<211>  222
<212>  PRT
<213>  SARS-CoV2

<400>  29

Met Ala Asp Ser Asn Gly Thr Ile Thr Val Glu Glu Leu Lys Lys Leu
1               5                   10                  15

Leu Glu Gln Trp Asn Leu Val Ile Gly Phe Leu Phe Leu Thr Trp Ile
            20                  25                  30

Cys Leu Leu Gln Phe Ala Tyr Ala Asn Arg Asn Arg Phe Leu Tyr Ile
        35                  40                  45

Ile Lys Leu Ile Phe Leu Trp Leu Leu Trp Pro Val Thr Leu Ala Cys
    50                  55                  60

Phe Val Leu Ala Ala Val Tyr Arg Ile Asn Trp Ile Thr Gly Gly Ile
65                  70                  75                  80

Ala Ile Ala Met Ala Cys Leu Val Gly Leu Met Trp Leu Ser Tyr Phe
                85                  90                  95

Ile Ala Ser Phe Arg Leu Phe Ala Arg Thr Arg Ser Met Trp Ser Phe
            100                 105                 110

Asn Pro Glu Thr Asn Ile Leu Leu Asn Val Pro Leu His Gly Thr Ile
        115                 120                 125

Leu Thr Arg Pro Leu Leu Glu Ser Glu Leu Val Ile Gly Ala Val Ile
    130                 135                 140

Leu Arg Gly His Leu Arg Ile Ala Gly His His Leu Gly Arg Cys Asp
145                 150                 155                 160

Ile Lys Asp Leu Pro Lys Glu Ile Thr Val Ala Thr Ser Arg Thr Leu
                165                 170                 175

Ser Tyr Tyr Lys Leu Gly Ala Ser Gln Arg Val Ala Gly Asp Ser Gly
            180                 185                 190

Phe Ala Ala Tyr Ser Arg Tyr Arg Ile Gly Asn Tyr Lys Leu Asn Thr
        195                 200                 205

Asp His Ser Ser Ser Ser Asp Asn Ile Ala Leu Leu Val Gln
    210                 215                 220

<210>  30
<211>  10
<212>  DNA
<213>  Artificial Sequence

<220>
<223>  artificial sequence

<400>  30
aacgttcgag                                                            10

<210>  31
<211>  11
<212>  DNA
<213>  Artificial Sequence

<220>
<223>  artificial sequence

<220>
<221>  misc_feature
<222>  (1)..(11)
<223>  n=2'-deoxy-7-deazaguanosine

<400>  31
tcnaacnttc n                                                            11

<210>  32
<211>  23
<212>  DNA
<213>  Artificial Sequence

<220>
<223>  artificial sequence

<220>
<221>  misc_feature
<222>  (1)..(11)
<223>  n=2'-deoxy-7-deazaguanosine

<220>
<221>  misc_feature
<222>  (12)..(12)
<223>  x=glycerol

<220>
<221>  misc_feature
<222>  (13)..(23)
<223>  n=2'-deoxy-7-deazaguanosine

<400>  32
   tcnaacnttc nnncttncaa nct                                              23

## Description

### FIELD OF THE INVENTION

The disclosure relates to CpG-adjuvanted SARS-CoV-2 vaccines and compositions and methods for producing said vaccines and administering the vaccines to subjects for the generation of an anti-SARS-CoV-2 immune response.

### BACKGROUND OF THE INVENTION

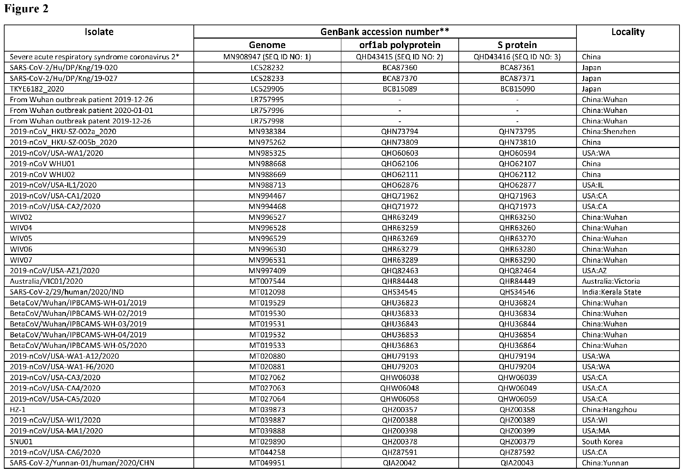

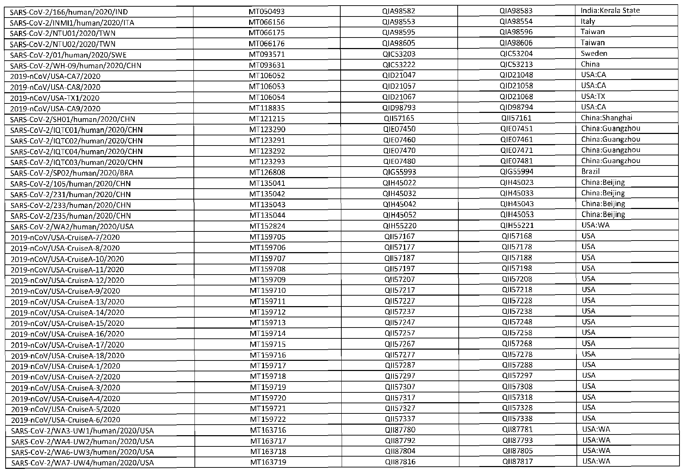

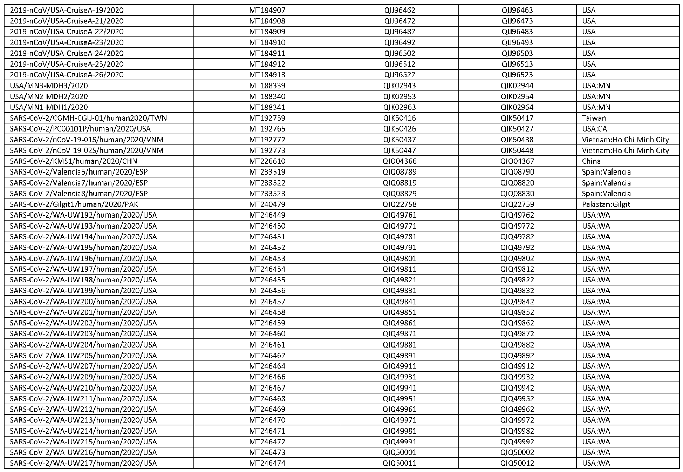

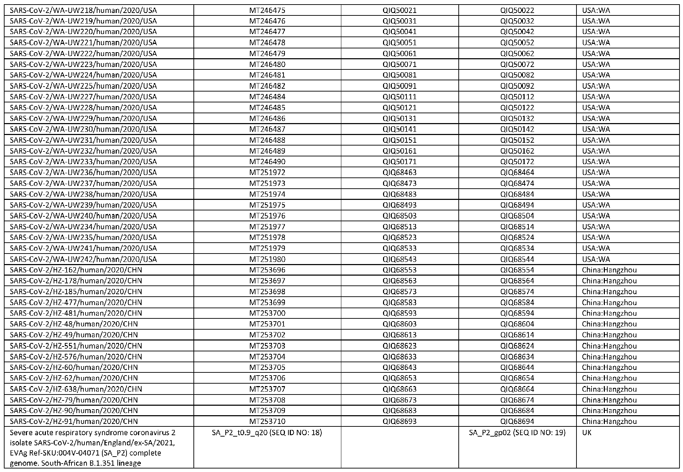

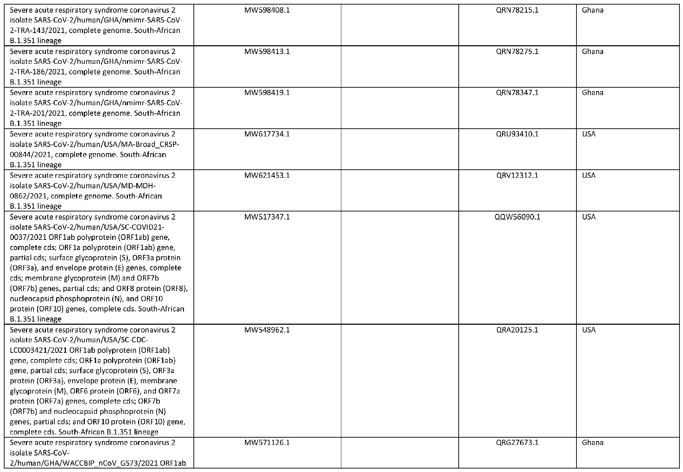

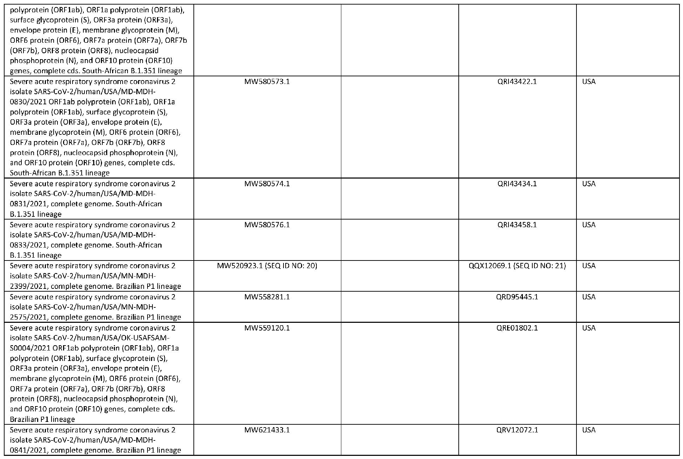

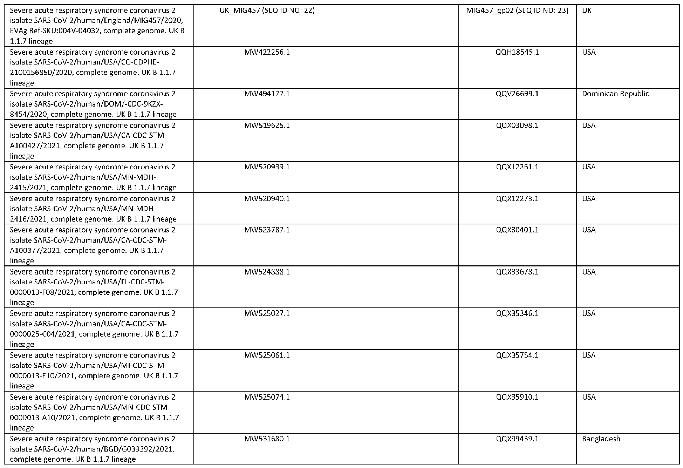

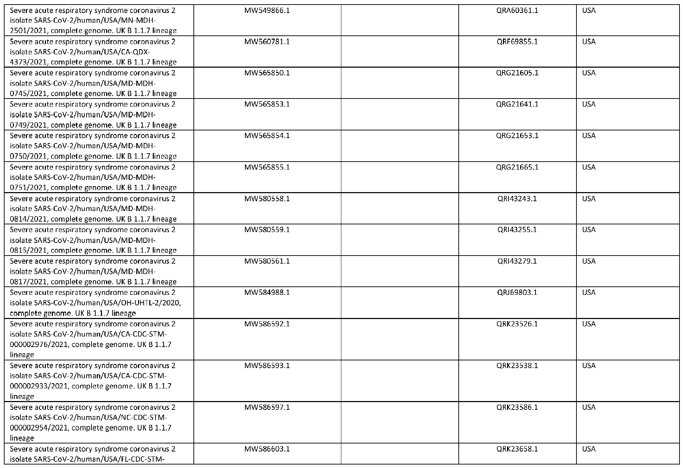

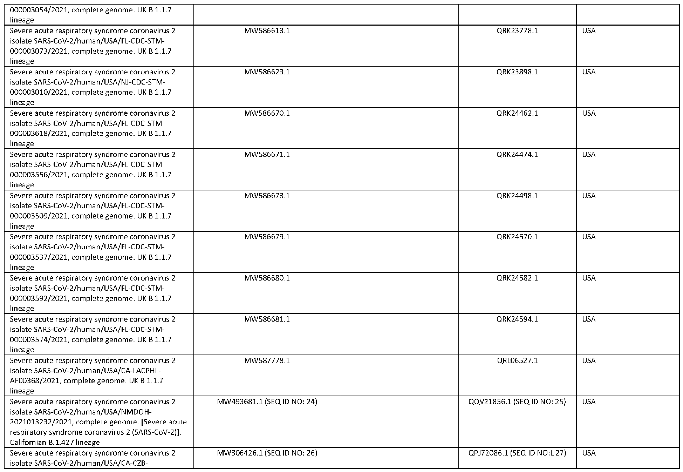

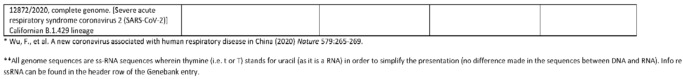

SARS-CoV-2 (hereinafter the "virus") was detected for the first time in China around November 2019. Since then, the virus has caused a global pandemic. The natural reservoir are bats and the virus belongs to the Coronaviridae family, genus Betacoronavirus (betaCoV). The virus has a ssRNA genome composed of 29,903 bp (Wuhan-Hu-1: Genbank Reference sequence: NC_045512.2), which encode a 9,860 amino acid polyprotein, comprising 25 non-structural proteins and 4 structural proteins: spike (S), envelope (E), membrane (M) and nucleocapsid (N) proteins. The virus particle has a variable diameter of between 60 and 140 nm. It is enveloped and sensitive to UV, heat, and lipid solvents. It has 89% nucleotide sequence identity with bat SARS-like-CoVZXC21 and 82% nucleotide sequence identity with human SARS-CoV (Chan et al. 2020). Evidence suggests that this virus spreads when an infected person coughs small droplets - packed with the virus - into the air. These can be breathed in, or cause an infection if one touches a surface they have landed on, then the eyes, nose or mouth. In addition, other vectors may exist, and the virus may be transmitted by blood transfusion, transplacentally, and through sexual transmission. Though infection with SARS-CoV-2 may result in only mild symptoms, such as, typically, a fever and a cough, or even be asymptomatic; in the other extreme, it can be fatal. The key symptoms are usually high temperature, cough and breathing difficulties. There is currently no specific treatment or vaccine for the virus, and the only preventative methods involve social distancing. SARS-CoV-2 presents a substantial public health threat. The Imperial College COVID-19 (disease caused by SARS-CoV-2) Response Team published in March 16, 2020, a report evaluating all possible methods available to stop or delay the spread of the virus, which could ultimately lead to the break-down of the healthcare system and hundreds of thousands of deaths in the UK alone. The report stated that only population-wide social distancing has a chance to reduce effects to manageable levels and these measures need to be followed until a vaccine is available. This recommendation would mean for most of the population quarantine for at least 18 months. The report concluded that a mass-producible vaccine is the only option to stop this pandemic, other than a willingness to sacrifice the elderly population. In view of the dramatic situation, there is an absolute urgent need for an effective vaccine against SARS-CoV-2 as fast as possible. Furthermore, various escape mutants have emerged (e.g. UK_B.1.1.7; South African_B.1.351; Californian_B.1.427/B.1.429 and Brazilian_P.1 variants, see also figure 2) which further worsen the situation and thus addressing this unfortunate development needs to be addressed as well.

### SUMMARY OF THE INVENTION

Accordingly, the present invention provides an inactivated whole virus SARS-CoV-2 vaccine. Multiple SARS-CoV-2 vaccines are in development, including vectored vaccines, whole-virion inactivated vaccines, and recombinant protein vaccines. Although mRNA or DNA vaccines or vectored vaccine candidates elicit T cell responses without adjuvants, adjuvants may be important for subunit and inactivated vaccines to increase their immunogenicity. Furthermore, a major challenge during rapid development is to avoid safety issues both by thoughtful vaccine design and by thorough evaluation in a timely manner. With regard to a SARS-CoV-2 vaccine, safety concerns have been raised in relation to potential immune-mediated disease enhancement. There is evidence for disease enhancement in vaccinated animals after challenge with live virus in multiple studies with SARS-CoV-1 vaccine candidates, including an alum-adjuvanted whole virus inactivated vaccine candidate. In mice, immunopathology induced by SARS-CoV-1 was considered a consequence of a dominant Th2 type response to the vaccine antigens (Tseng C-T et al. 2012 Immunization with SARS Coronavirus Vaccines Leads to Pulmonary Immunopathology on Challenge with the SARS Virus. PlosOne 7(4):e35421). This was not observed after including other adjuvants (e.g. CpG) in the vaccine or other vaccine formulations known to drive immune responses towards Th1. Insofar as an inactivated vaccine approach has been contemplated, the use of typical inactivating agents (e.g. formaldehyde) under standard conditions may have drawbacks, such as, particularly, destruction of native epitopes, which hinder development of an effective vaccine candidate. The present invention aims to address these problems and thus to produce a safe and effective whole-virus inactivated SARS-CoV-2 vaccine that overcomes the drawbacks of the prior art.

Thus in one aspect, the present invention provides a SARS-CoV-2 vaccine comprising an inactivated SARS-CoV-2 particle in combination with cytidine-phospho-guanosine (CpG) and alum adjuvantation. As mentioned above, the selection of an appropriate adjuvant or adjuvants for the SARS-CoV-2 vaccine may be of critical importance. Even though use of alum may not necessarily lead to Th2 skewing in humans, the addition of CpG is believed to mitigate possible vaccine-related disease enhancement safety concerns. The addition of CpG may further allow for significant reduction of the antigen dose needed to achieve seroconversion in a subject (i.e. "dose sparing"), another important consideration in light of the urgent global need for a SARS-CoV-2 vaccine. Lastly, addition of adjuvants can help generating robust immune responses in subjects particularly susceptible or vulnerable to SARS-CoV-2 morbidity or mortality, i.e. immunocompromised, pregnant or elderly subjects. Such vaccine compositions are described in more detail below.

In a preferred aspect, the surface of the inactivated SARS-CoV-2 particle in the vaccine presents a native conformation such that the vaccine is capable of generating neutralizing antibodies against native SARS-CoV-2 particles in a human subject. In particular, the present invention aims to provide optimally inactivated SARS-CoV-2 particles, which are incapable of replication and infection of human cells, but which retain immunogenic epitopes of viral surface proteins and are thus suitable for generating protective immunity in vaccinated subjects. By optimizing the inactivation process (e.g. using beta-propiolactone) and other steps in the production of the vaccine, including the selection of an appropriate adjuvant, a novel vaccine composition can be obtained that preserves a native surface conformation of SARS-CoV-2 particles and which reduces the risk of vaccine induced immunopathology or enhancement of disease. Thus in one aspect, the SARS-CoV-2 vaccine composition comprises a beta-propiolactone-inactivated SARS-CoV-2 particle, wherein the vaccine is capable of generating neutralizing antibodies against native SARS-CoV-2 particles in a human subject, preferably wherein a native surface conformation of the SARS-CoV-2 particle is preserved in the vaccine.

In a further particular aspect, the inventions aims to provide an optimal combination of optimally inactivated (e.g. beta-propiolactone-inactivated) SARS-CoV-2 particles, which are incapable of replication and infection of human cells, but which retain immunogenic epitopes of viral surface proteins and are thus suitable for generating protective immunity in vaccinated subjects. By an optimal combination of inactivated SARS-CoV-2 particles also in combination with alum and CpG adjuvantation, an improved vaccine composition can be obtained that is capable of generating neutralizing antibodies against native SARS-CoV-2 particles and/or other immunological responses in a human subject that are able to protect partly or fully more than 50%, preferably more than 60%, more than 70%, more than 80%, more than 90% of said vaccinated human subjects.

Each of the limitations of the invention can encompass various embodiments of the invention. It is therefore anticipated that each of the limitations of the invention involving any one element or combinations of elements can be included in each aspect of the invention. This invention is not limited in its application to the details of construction and the arrangement of components set forth in the following description or illustrated in the drawings. The invention is capable of other embodiments and of being practiced or of being carried out in various ways.

### BRIEF DESCRIPTION OF THE DRAWINGS

The accompanying drawings are not intended to be drawn to scale. The figures are illustrative only and are not required for enablement of the disclosure. For purposes of clarity, not every component may be labeled in every drawing. In the drawings:

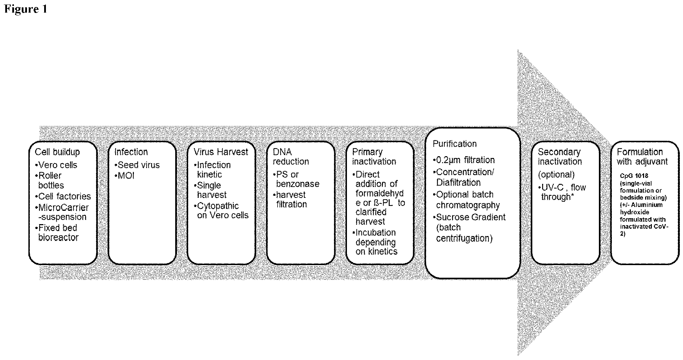

**Figure 1****.** The process for production of the inactivated SARS-CoV-2 vaccine of the current invention. Steps include cell buildup of Vero host cells, infection of Vero cells with SARS-CoV-2, virus harvest, DNA reduction, primary inactivation, purification, optional secondary inactivation and formulation with adjuvant. The virus may be formulated with or without aluminium hydroxide. The CpG adjuvant may be included in the same vial with the virus (and optional alum) or mixed with the virus before administration.
**Figure 2****.** During the course of the SARS-CoV-2 pandemic, SARS-CoV-2 genomic sequences from isolates from around the world have been reported including the recent new variants or lineages such as the UK B 1.1.7, Brazilian PI, Californian B.1.427/B.1.429 and South African B.1.351 lineages. The accession numbers and origins of complete SARS-CoV-2 genomic sequences are provided in tabular form, along with accession numbers for the corresponding orflab polyprotein and S protein, when available (- or no entry = not available).

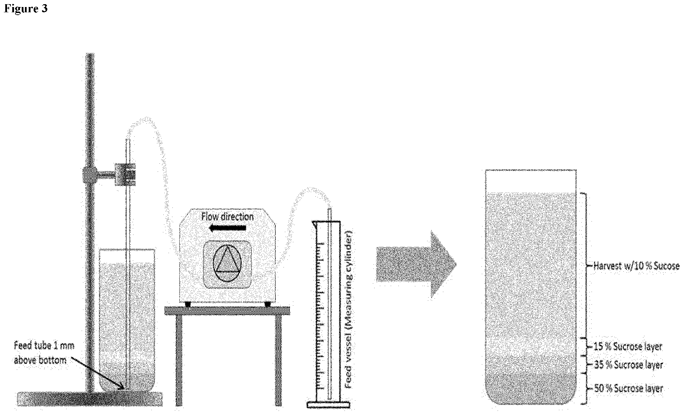

**Figure 3****.** A preferred set-up for the sucrose gradient centrifugation used as a polishing step for the SARS-CoV-2 vaccine of the invention.

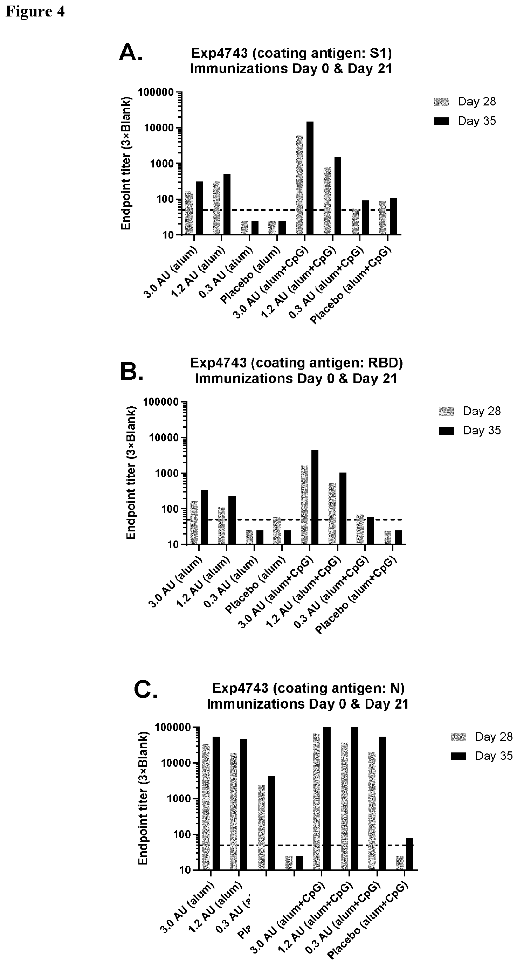

**Figure 4****.** Total IgG in response to SARS-CoV-2 vaccine. Coating antigens: S1 (A), receptor binding domain of spike protein (B) and nucleoprotein (C). Endpoint titer: absorbance of 3-fold the blank used as cut-off (dashed line).

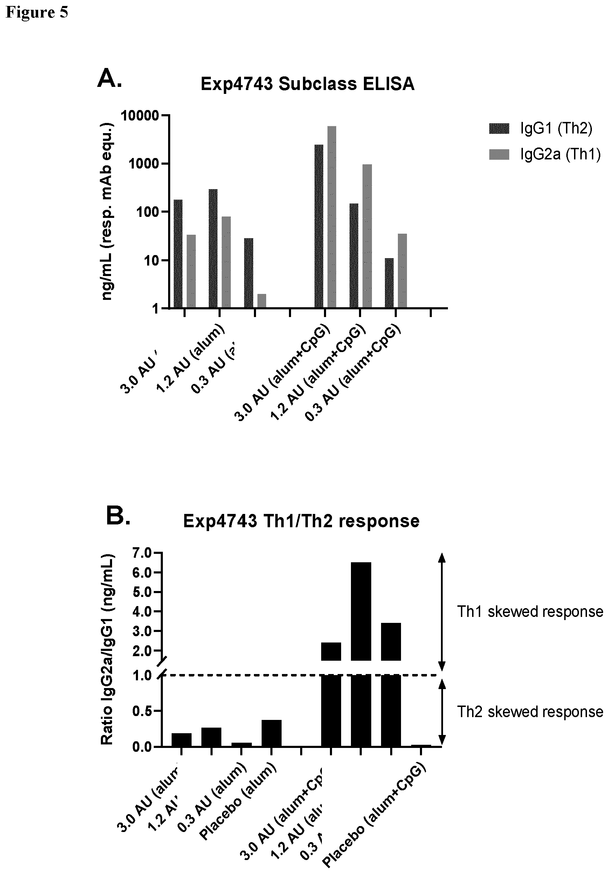

**Figure 5****.** IgG1 and IgG2a titers in response to SARS-CoV-2 vaccine adjuvanted with alum alone and alum/CpG 1018. Antibody titers specific to S1 protein were determined by ELISA. The concentrations were determined by comparison with a mAb subclass standard curve.

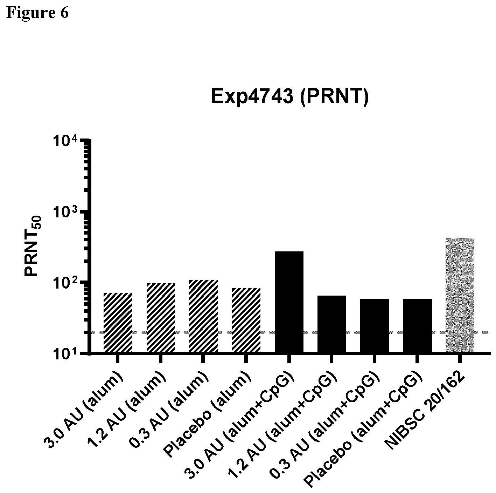

**Figure 6****.** Neutralizing titers in response to SARS-CoV-2 vaccine. The tested mouse sera were collected at d35. Neutralizing response in the presence of alum/CpG 1018 observed to be in the range of plasma from convalescent donors positive for SARS-CoV-2 (NIBSC 20/162; pooled sera from three donors).

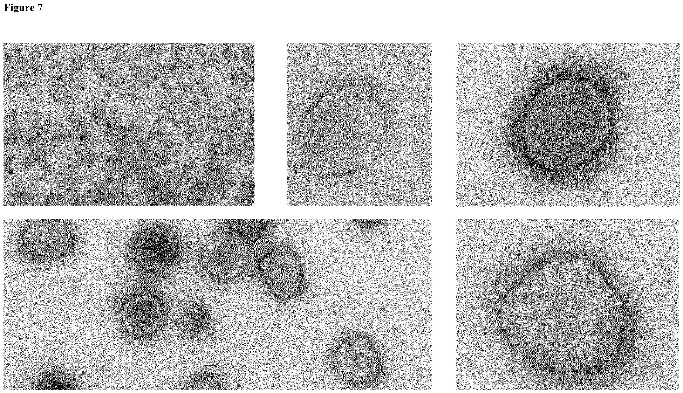

**Figure 7****.** Production process delivers high density and intact spike proteins. Shown are electron micrographs of the SARS-CoV-2 inactivated drug substance produced according to Example 1. About 1-1.5 10⁷ viral particles per AU.

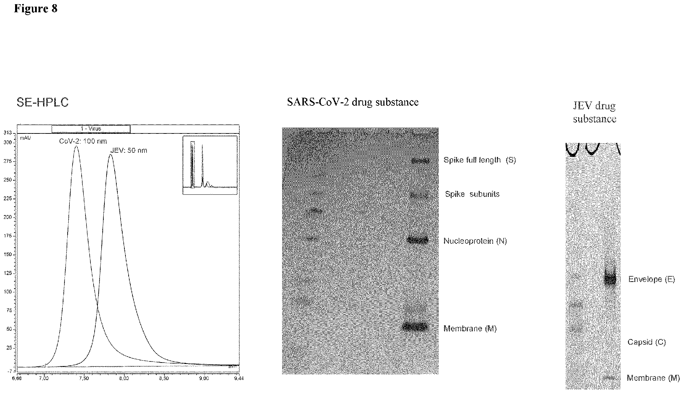

**Figure 8****.** Comparison of Size-Exclusion-Chromatography and SDS-PAGE profiles of SARS-CoV-2 and JEV drug substance. High purity (>95%) according to SDS-PAGE (silver stain, reduced) and monomer virus (>95%) according to SE-HPLC. Difference in retention time due to different virus particle size (JEV (IXIARO) about 50nm, SARS-CoV2 about 100nm).

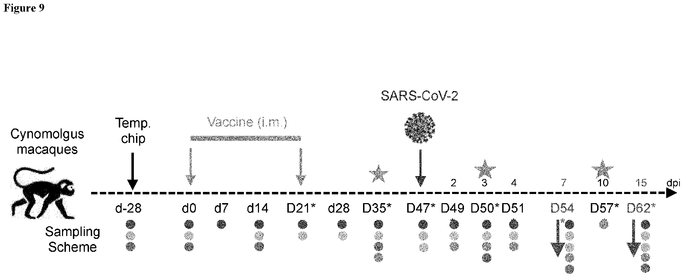

**Figure 9****.** Study design for NHP challenge study. Three groups of 8 animals each; Two dose groups for SARS-CoV-2 vaccine (10 AU & 40 AU, formulated with 0.5 mg/dose Al³⁺ and 1 mg CpG 1018 per dose added directly before administration) and a placebo group (DPBS). The SARS-CoV-2 challenge strain is BetaCoV/France/IDF/0372/2020 (Maisonmasse et al., Hydroxychloroquine use against SARS-CoV-2 infection in non-human primates, 2020, Nature 585:584-587). Methods and timing of testing: Hematology on d-28, d0, d7, d14, d21, d28, d35, d47, d49, d50, d51, d54, d62. Ab response (ELISA, IFA) on d-28, d0, d14, d21, d28, d35, d47, d54, d62. T cell response (ICS, ELISPOT) on d-28, d0, d14, d35, d54, d62. Cytokine response (LUMINEX) on d47, d49, d50, d51, d54, d62. SWABS (viral load (qRT-PCR-genomic + subgenomic): nasal & tracheal swabs on d35, d49, d50, d51, d54, d57, d62; rectal swabs at baseline and on d2, d7, d15. BAL viral load (qRT-PCR-genomic + subgenomic): d50. Euthanasia: lung harvest, viral load (qRT-PCR - genomic + subgenomic): d54, d62. CT scans: d35, d50, d57.

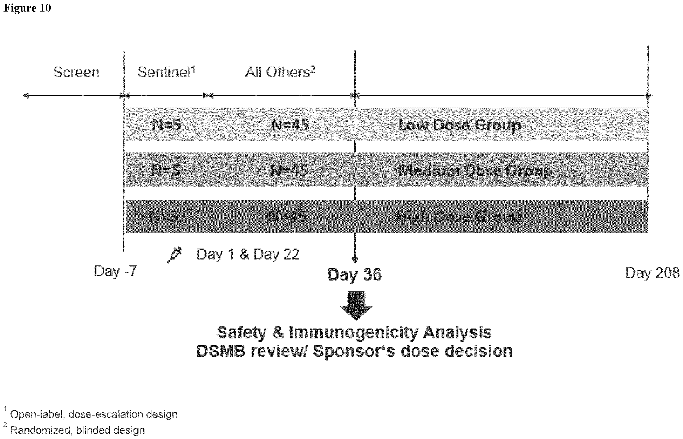

**Figure 10****.** Phase 1/2 clinical study: a blinded, randomized, dose-escalation study. Study Population: 150 healthy volunteers (50 subjects per dose group) aged 18 to 55 years. Dosage: two vaccinations (D1, D22; also referred to as D0, D21), low (∼3 AU), medium (∼10 AU) and high (∼40 AU) dose. Immunization route: i.m.

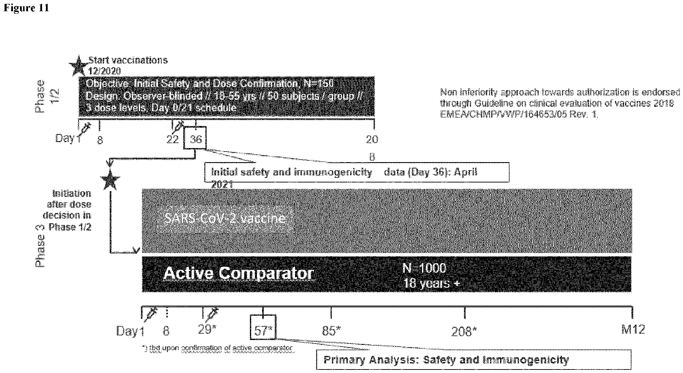

**Figure 11****.** Study design for a non-inferiority pivotal phase 3 immunogenicity trial for initial licensure. Vaccine efficacy determined by demonstrating non-inferior neutralizing antibody titers between SARS-CoV-2 vaccine of the invention and a licensed COVID-19 vaccine for which efficacy has been established.

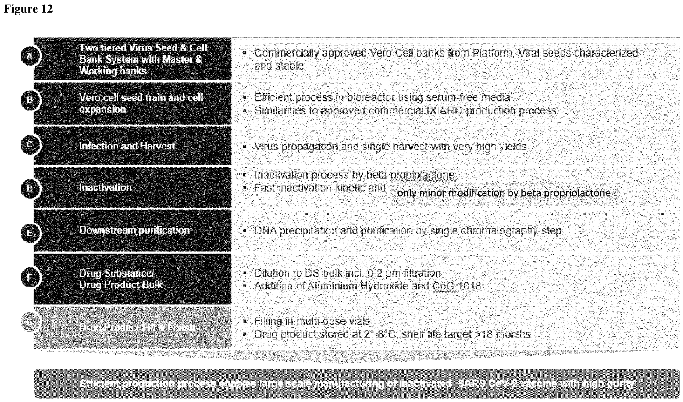

**Figure 12****.** Outline of the final SARS-CoV-2 manufacturing process-the fully industrialized production process.

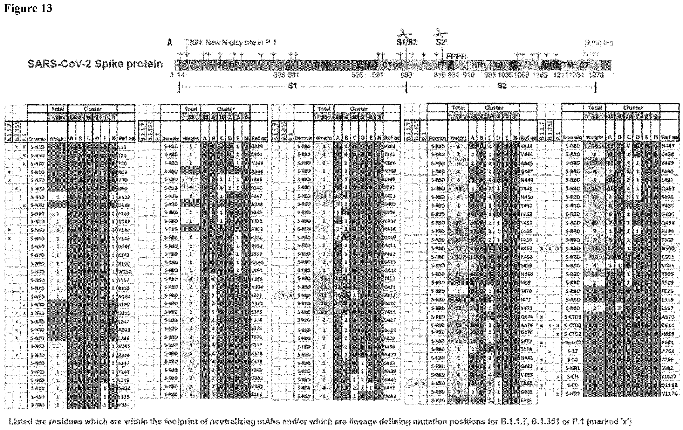

**Figure 13****:** Counts of residues within the footprints of 33 neutralizing mAbs, or respectively clusters 13, 4, 10, 2, 1, 3. Listed are residues within the footprint of neutralizing mAbs and/or which are lineage defining mutation positions for B.1.1.7, B.1.351 or P.1 (marked "x"). E.g. K417 and E484 mutations which are amino acid positions in the S-protein are only to be found in the South African and Brazilian lineages.

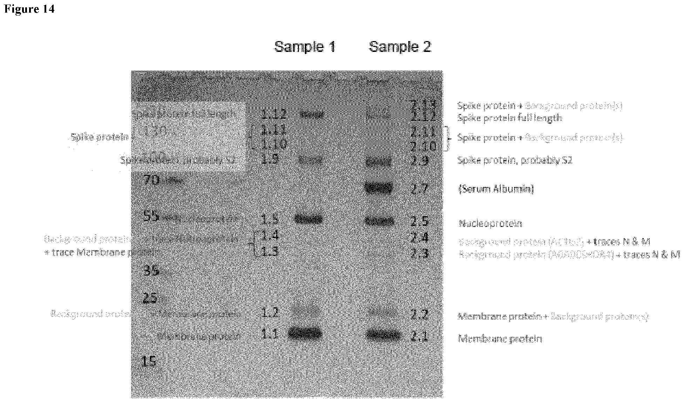

**Figure 14****:** SDS-PAGE, silver stain, of two samples of SARS-CoV-2 candidates according to example 1 (iCELLIS 500 bioreactor, protamine sulfate precipitated, BPL inactivated). The bands could be clearly attributed to the three main viral proteins (Spike-protein, Membrane-protein, Nucleoprotein) as well as to background proteins from the host system.

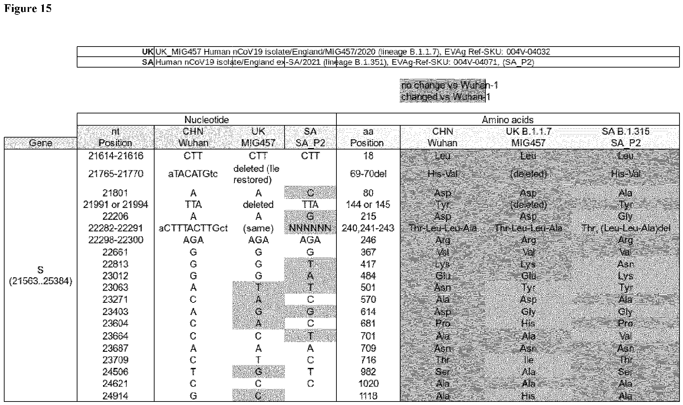

**Figure 15****:** SARS-CoV-2 Mutations within the Spike Protein of strain UK MIG457 (B.1.1.7 lineage) and strain SA_P2 (B.1.351 lineage) from PHE.

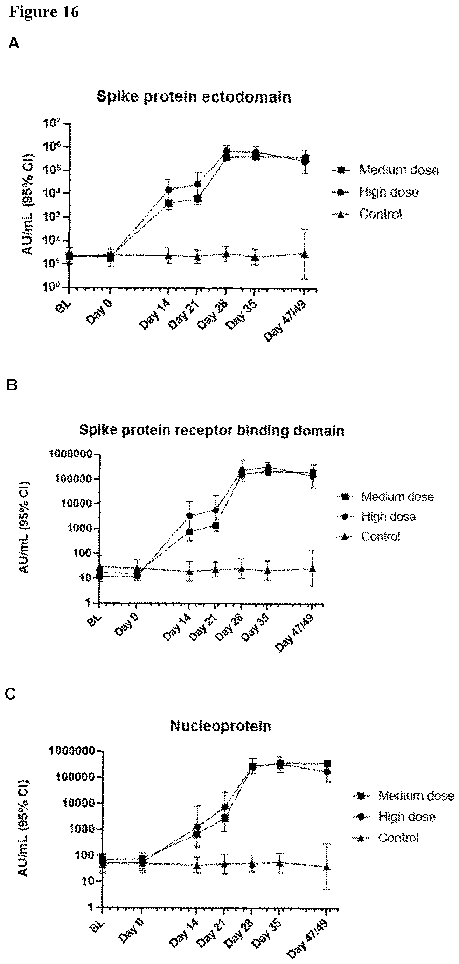

**Figure 16****:** Immunogenicity of the vaccine candidate in cynomolgus macaques. A) Plates were coated with ectodomain of spike protein with a T4 trimerization domain. B) Plates were coated with receptor binding domain (RBD) of spike glycoprotein. C) Plates were coated with nucleoprotein.

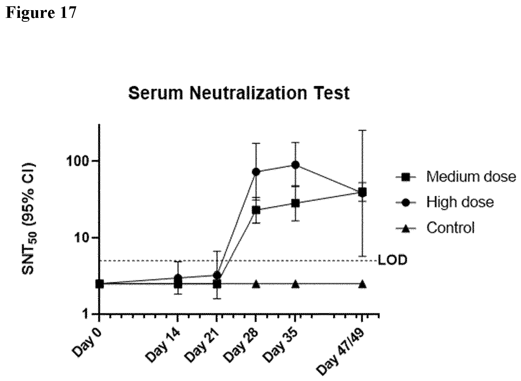

**Figure 17****:** Neutralizing titers determined by SNT. The dashed line represents the limit of detection (LOD, SNT₅₀ = 5). Samples with a SNT₅₀ titer below 5 were imputed to 2.5

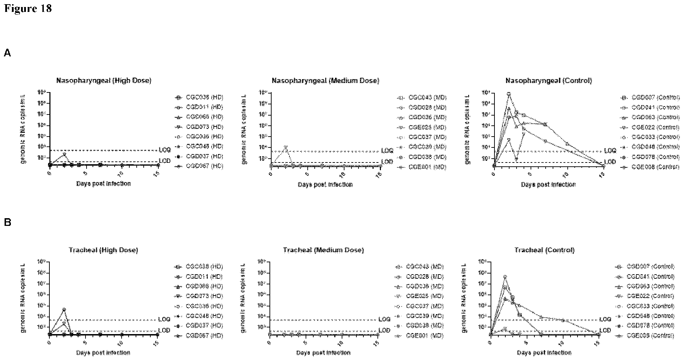

**Figure 18****:** Genomic copies of SARS-CoV-2 RNA determined by RT-qPCR. A) Nasopharyngeal swabs. B) Tracheal swabs. The dashed lines represent the limit of detection (LOD = 476 RNA copies/mL) and limit of quantification (LOQ = 4760 RNA copies/mL). Samples with RNA copies/mL below 476 were imputed to 238.

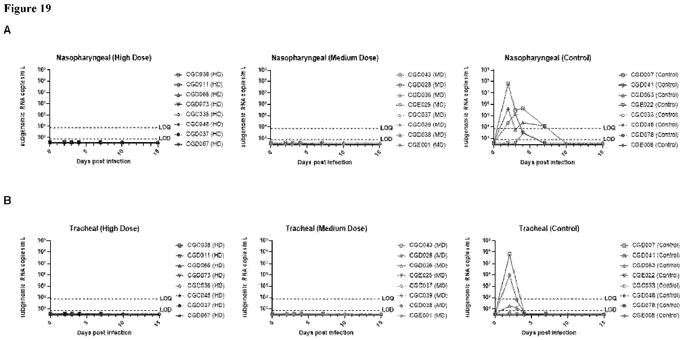

**Figure 19****:** Subgenomic copies of SARS-CoV-2 RNA determined by RT-qPCR. A) Nasopharyngeal swabs. B) Tracheal swabs. The dashed lines represent the limit of detection (LOD = 749 RNA copies/mL) and limit of quantification (LOQ = 7490 RNA copies/mL). Samples with RNA copies/mL below 749 were imputed to 375.

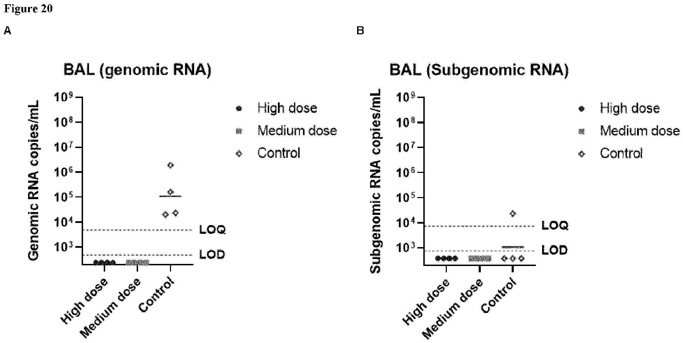

**Figure 20****:** Analyses of bronchoalveolar lavage by RT-qPCR. A) Genomic RNA, samples with RNA copies/mL below 476 (LOD) were imputed to 238. B) Subgenomic RNA, samples with RNA copies/mL below 749 (LOD) were imputed to 375. The dashed lines represent the limit of detection (LOD) and limit of quantification (LOQ) in the respective assays.

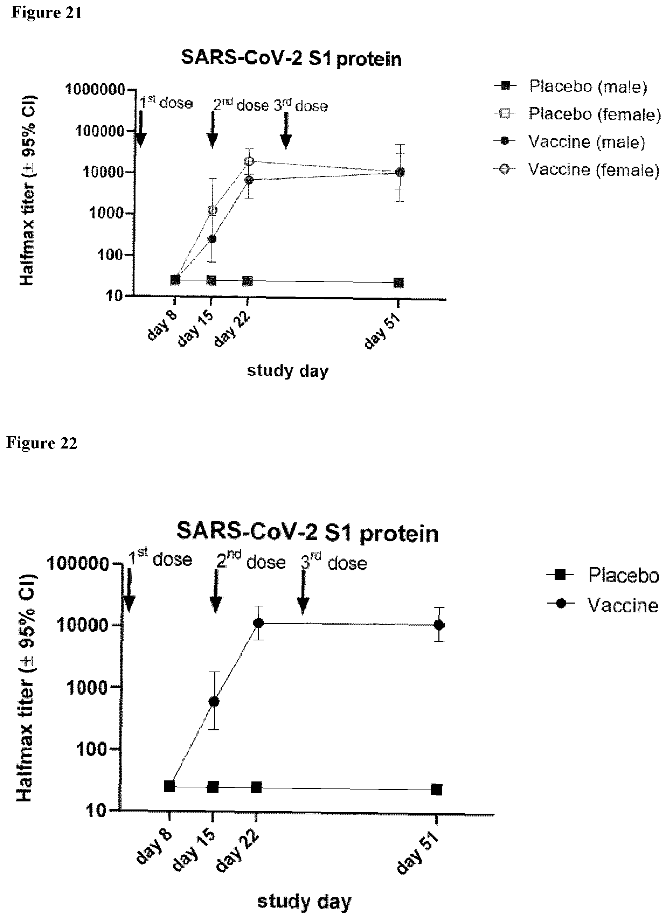

**Figure 21****:** Graphical presentation of GMTs for male and female rats for each treatment group. Placebo is square symbols (male: filled symbols, female: unfilled symbols) and inactivated SARS-CoV-2 vaccine is circular symbols (male: filled symbols, female: unfilled symbols) over the course of the study. Error bars indicate 95% confidence intervals (CI). GMT <50 were imputed to 25.
**Figure 22****:** Graphical presentation of GMTs for each treatment group, data for male and female rats combined. Placebo is square symbols and inactivated SARS-CoV-2 vaccine is circular sym. Error bars indicate 95% confidence intervals (CI). GMT <50 were imputed to 25.

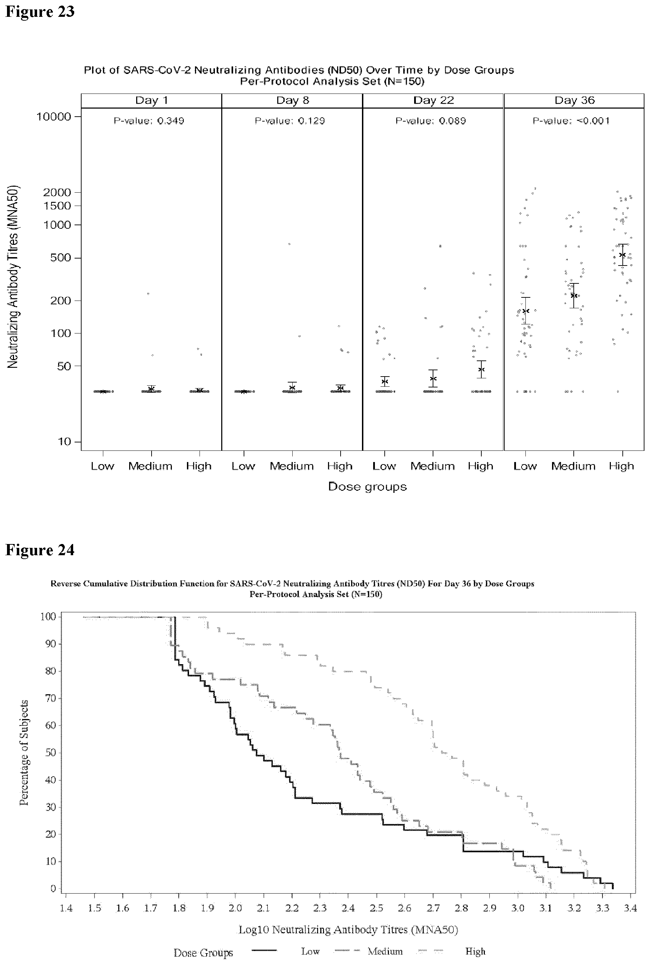

**Figure 23****:** Plot of SARS-CoV-2 neutralizing antibodies (MNA50) over time by dose groups per-protocol analysis set (N=150). Day 1: low (N=51), medium (N=49), and high (N=50). Day 8: low (N=51), medium (N=49), and high (N=50). Day 22: low (N=50), medium (N=48), and high (N=48). Day 36: low (N=51), medium (N=48), and high (N=50). Graph shows GMT and 95% CI. Scatter dots are the actual distribution of neutralizing antibody titres.
**Figure 24****:** Reverse cumulative distribution function for SARS-CoV-2 neutralizing antibody titres (ND50) for day 36 by dose groups per protocol analysis set (N=150). Low dose (N=51), medium dose (N=48) and high dose (N=50).

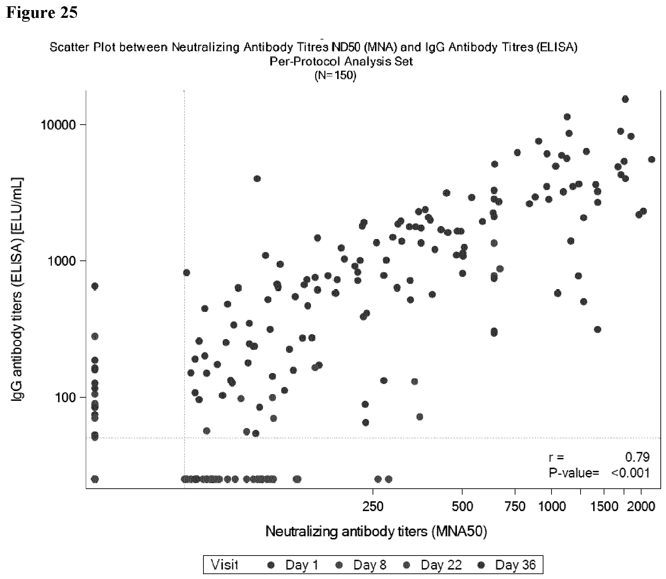

**Figure 25****:** Neutralization titer correlates with S-protein specific IgG. Scatter plot between neutralizing antibody titres ND50 (MNA) and IgG antibody titres (ELISA) per-protocol analysis set (N=150). Scatter plot shows correlation between results of ELISA (ELU/mL) and MNA (ND50). Pearson correlation coefficient (r) between ELISA (ELU/mL) and MNA(ND50) and P-value for testing the significance of correlation coefficient is also presented in the plot. Red dotted lines present the limit of detection for ELISA (50.3 ELU/mL) and MNA (ND50=58).

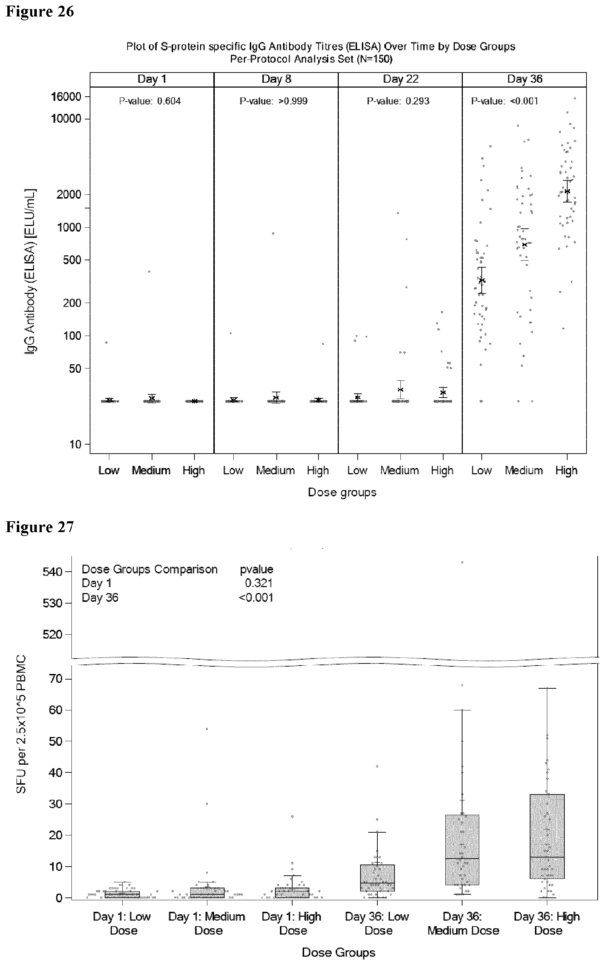

**Figure 26****:** Plot of S-protein specific IgG antibody titers (ELISA) overtime by dose groups per-protocol analysis set (N=150). Day 1: low (N=51), medium (N=49), and high (N=50). Day 8: low (N=51), medium (N=49), and high (N=50). Day 22: low (N=51), medium (N=49), and high (N=50). Day 36: low (N=51), medium (N=49), and high (N=50). Graph shows GMT and 95% CI. Scatter dots are the actual distribution of IgG antibody titres.
**Figure 27****:** Plot of IFN gamma spot forming units per 2x10^5 PBMC by dose groups and assessment days for panel 14 spike protein, full sequence (N=150). Dose groups comparison (pvalue): day 1 (p = 0.321) and day 36 (p<0.001). Day 1: low (N=46), medium (N=43), and high (N=44). Day 36: low (N=44), medium (N=44), and high (N=45). The boxplots show the median, lower quartile and upper quartile. The horizontal line within each bar is the median and the plus sign (+) represents the mean value for each group. Scatter dots are the actual distribution of SFU per 2.5x10^5 PBMC within each group.

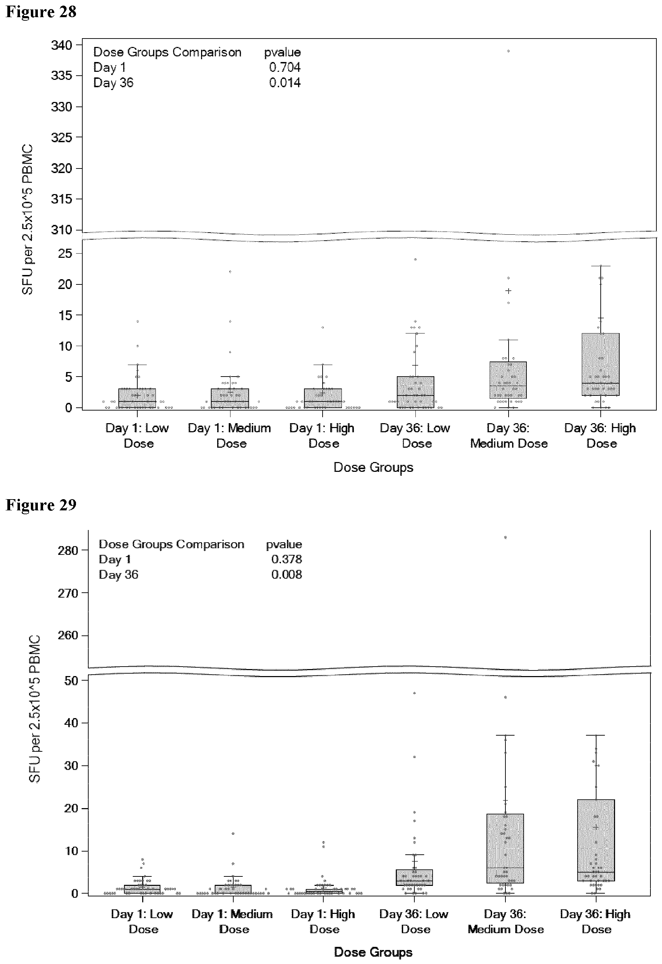

**Figure 28****:** Plot of IFN gamma spot forming units per 2x10^5 PBMC by dose groups and assessment days for membrane protein (N=150). Dose groups comparison (pvalue): day 1 (p=0.704), day 36 (p-0.014). Day 1: low (N=46), medium (N=43), and high (N=44). Day 36: low (N=44), medium (N=44), and high (N=45). The boxplots show the median, lower quartile and upper quartile; the horizontal line within each bar is the median and the plus (+) sign represents the mean value for each group. Scatter dots are the actual distribution of SFU per 2.5x10^5 PBMC within each group.
**Figure 29****:** Plot of IFN gamma spot forming units per 2x10^5 PBMC by dose groups and assessment days for nucleocapsid (N=150). Dose groups comparison (pvalue): day 1 (p=0.378), day 36 (p=0.008). Day 1: low (N=46), medium (N=43), and high (N=44). Day 36: low (N=44), medium (N=44), and high (N=45). The boxplots show the median, lower quartile and upper quartile. The horizontal line within each bar is the median and the plus (+) sign represents the mean value for each group. Scatter dots are the actual distribution of SFU per 2.5x10^5 PBMC within each group.

### DETAILED DESCRIPTION OF THE INVENTION

Embodiments of the present invention are directed to a SARS-CoV-2 vaccine or immunogenic composition comprising inactivated SARS-CoV-2 particles in combination with a CpG adjuvant. Typically, the inactivated SARS-CoV-2 particles are whole, inactivated particles, i.e. the inactivated virus particles are derived from whole native SARS-CoV-2 particles that have been inactivated. As used herein, "SARS-CoV-2" refers to the SARS-CoV-2 virus and "SARS-CoV-2 particles" typically refers to whole SARS-CoV-2 viral particles, i.e. virions.

In one embodiment, the inactivated SARS-CoV-2 particles are combined with the Th1 response-directing adjuvant CpG, preferably CpG 1018. As used herein, "CpG" refers to a cytosine-phospho-guanosine (CpG) motif-containing oligodeoxynucleotide (or CpG-ODN), e.g. which is capable of acting as a toll-like receptor 9 (TLR9) agonist. The CpG motif refers to an unmethylated cytidine-phospho-guanosine dinucleotide sequence, e.g. which is capable of binding to TLR9. Th1 response-directing adjuvants such as CpG promote the induction of a predominantly T helper type 1 (i.e. Th1) immune response in an immunized subject (rather than a Th2 type response), i.e. a "Th1-biased response". The Th1- or Th2-directing properties of commonly used vaccines are known in the art. It has surprisingly been found that using an adjuvant that promotes a Th1 response, e.g., CpG 1018, can improve immunogenicity of the vaccine and thus antiviral responses, as well as reducing the risk of disadvantageous effects such as immunopathology (which may result from a predominantly Th2 type response possibly due to hypersensitivity against viral components). In one embodiment, the SARS-CoV-2 vaccine of the current invention also comprises the Th2-stimulating adjuvant alum, which has a known tendency for Th2 skewing in humans. Neutralizing antibodies, the production of which is critical for anti-viral immunity, are strongly stimulated by Th2-stimulating adjuvants, such as e.g. alum. Also important for anti-viral immunity are cellular immune responses, which are only weakly stimulated by alum. Delivered together, alum and Th1-inducing adjuvants, such as CpG, can provide a potent anti-viral response. In short, the use of CpG and alum together in a vaccine formulation can provide a more balanced immune response to antigens, including both humoral and cellular components, and may have less deleterious effects than alum alone with a predominantly Th2 response.

In one embodiment, the CpG adjuvant comprised in the vaccine of the invention is a class A, class B or class C CpG (see **Table A-1**), preferably a class B CpG. Class B CpG molecules include CpG 1018, CpG 1826 and CpG 7909 (SEQ ID Nos: 4, 7 and 8, respectively; **Table A-2).** Most preferred is CpG 1018.

**Table A-1. Comparative features of CpG classes A, B and C (Campbell JD, 2017, in Christopher B. Fox (ed.), Vaccine Adjuvants: Methods and Protocols, Methods in Molecular Biology, vol. 1494, DOI 10.1007/978-1-4939-6445-1_2).**

| **Class** | **Structural characteristics** | **Immunological characteristics** |
|---|---|---|
| CpG-A | Phosphodiester CpG motif(s) | Strong pDC IFN-α induction |
| | Phosphorothioate poly-G at 5' and 3' | Moderate pDC maturation |
| | Forms aggregates | Weak B cell activation |
| CpG-B | Phosphorothioate backbone | Strong B cell activation |
| | T-rich with CpG motifs | Strong pDC maturation |
| | Monomeric | Weak pDC IFN-α induction |
| CpG-C | Phosphorothioate backbone | Good pDC IFN-α induction |
| | 5 '-TCG, CpG motif in central palindrome | Good pDC maturation |
| | Forms duplexes | Good B cell activation |

| | | |
|---|---|---|
| *IFN,* interferon; *pDC,* plasmacytoid dendritic cell | | |

**Table A-2. Class B CpGs**

| | Sequence | SEQ ID NO: |
|---|---|---|
| CpG 1018 | TGACTGTGAACGTTCGAGATGA | 4 |
| CpG 1826 | TCCATGACGTTCCTGACGTT | 7 |
| CpG 7909 | TCGTCGTTTTGTCGTTTTGTCGTT | 8 |

CpG 1018 may be adsorbed onto alum and thus used as a combination adjuvant that induces both Th1 and Th2 responses (as described in e.g. Tian et al. Oncotarget, 2017, Vol. 8, (No. 28), pp: 45951-45964). Thus, in one embodiment, the CpG-adjuvanted vaccine composition of the invention further comprises aluminium (Al³⁺), preferably in the form of an aluminium salt, e.g. aluminium oxide, aluminium hydroxide or aluminium phosphate, preferably aluminium hydroxide. A preferred aluminium salt is the aluminium hydroxide with reduced Cu content, e.g. lower than 1.25 ppb based on the weight of the vaccine composition, an adjuvant described in detail in WO2013/083726 or Schlegl et al., Vaccine 33 (2015) 5989-5996. In a preferred embodiment, the vaccine composition comprises both CpG and Al³⁺, i.e., the adjuvant is a combination or mixture of CpG and Al³⁺, preferably CpG 1018 (SEQ ID NO: 4) and Al³⁺ provided in the form of aluminium hydroxide (Al(OH)₃). The presence of Al³⁺ may reduce the required dose of CpG, i.e., have a "dose-sparing" effect. In one embodiment, the SARS-CoV-2 vaccine is formulated with Al³⁺, and combined with a separate CpG-containing solution directly before vaccination of a subject; i.e. "bed-side mixing". In a preferred embodiment, the two adjuvants are both comprised in the formulation of the SARS-CoV-2 vaccine of the invention; i.e. "single-vial formulation".

In one embodiment, the Al³⁺:CpG weight/weight (w/w) ratio, preferably the Al³⁺ provided in the form of Al(OH)₃:CpG 1018 (w/w) ratio, in the vaccine composition is about 1:10, about 1:5, about 1:4, about 1:3, about 1:2, about 1:1, about 2:1, about 3:1, about 4:1, about 5:1, about 10:1, preferably between about 1:3 and 3:1, more preferably between about 1:2 and 1:1, most preferably about 1:2, even more preferably 1:2 in humans. In this regard, the effects of CpG in mice and humans are known to be very different, in part due to different TLR9 receptors which respond differently to CpG-DNA sequences as well as unique cellular distribution patterns of TLR9 expression in mice and humans (Chuang TS et al. 2002 Toll-like receptor 9 mediates CpG-DNA signaling. J Leukocyte Biol. (71)538-544). Therefore, the alum:CpG ratio giving an optimal response to any given antigen is likely to differ, perhaps substantially, in humans and mice. As referred to herein, the weight of the alum component refers to the weight of the Al³⁺ in the solution, regardless of what type of aluminium salt is used. For example, 0.5 mg of Al³⁺ corresponds to 1.5 mg alum. In one embodiment, the amount alum (Al³⁺) present in the SARS-CoV-2 vaccine composition is between about 0.1 and 2 mg/mL, between about 0.2 and 1.5 mg/mL, between about 0.5 and 1.3 mg/mL, especially between about 0.8 to 1.2 mg/mL, most preferably about 1 mg/mL, i.e., 0.5 mg/dose. In a preferred embodiment, the relatively high amount of alum (compared with currently licensed alum-adjuvanted vaccines) is used to ensure complete binding of antigen, as well as binding of at least a portion of the total CpG in the formulation. In this regard, the ratio of alum: CpG affects the amount of "free" desorbed CpG, i.e., the CpG which is not bound to alum and/or antigen in the vaccine composition. In a preferred embodiment, the amount of free (unbound) CpG in the vaccine composition is greater than 10%, greater than 20%, greater than 30%, greater than 40%, greater than 50%, greater than 60%, greater than 70%, greater than 80%, greater than 90%, greater than 95%, preferably about 70% to 95%, most preferably about 80% to 90%, e.g. by weight (based on the total weight of CpG in the vaccine composition). In particular, the alum:CpG ratio should facilitate a majority of the CpG content in "free" (desorbed) CpG; i.e., the CpG is not bound to components of the vaccine such that it remains in a depot. In a preferred embodiment, the amount of free CpG versus bound CpG is greater than 50%, greater than 60%, greater than 70%, greater than 80%, greater than 85%, greater than 90%, preferably greater than between 70 to 90%, especially between about 80 and 90%, e.g. by weight (i.e. the amount of free CpG by weight based on the total weight of CpG in the vaccine composition). In one embodiment, the amount of CpG in the SARS-CoV-2 vaccine composition of the current invention is between about 0.25 and 6 mg/mL, between about 0.5 and 3 mg/mL, between about 1 and 3 mg/mL, especially between about 1.5 to 2.5 mg/mL, most preferably about 2 mg/mL, i.e., 1 mg/dose. In an especially preferred embodiment, the the SARS-CoV-2 vaccine composition of the current invention is adjuvanted with 1 mg/mL Al³⁺ and 2 mg/mL CpG 1018; i.e., 0.5 mg Al³⁺ and 1 mg/mL CpG 1018 per dose.

In addition to the ratio of alum to CpG, different buffer systems may affect the adsorption of CpG to Al³⁺ and/or antigen in the vaccine composition. For example, the use of a Tris buffer system in the vaccine composition resulted in reduced free CpG (i.e. reduced desorption), whereas a phosphate buffer system allowed better desorption of CpG. In a particular example, a phosphate-buffered formulation containing 1 mg/mL Al³⁺ (aluminium hydroxide) and 2 mg/mL CpG 1018 (1:2 w/w ratio) had only about 10-20% bound CpG, i.e. about 80-90% free CpG. When formulated in phosphate buffer, about 0.3 mg of CpG is adsorbed per mg of Al³⁺ (data not shown).

Typically, the adjuvant is combined with the inactivated SARS-CoV-2 particles during manufacture of the vaccine product, i.e. the manufactured vaccine product comprises the adjuvant and is sold/distributed in this form. In alternative embodiments the adjuvant may be combined with the inactivated SARS-CoV-2 particles at the point of use, e.g. immediately before clinical administration of the vaccine (sometimes referred to as "bedside mixing" of the components of the vaccine). Thus the present invention comprises both vaccine products comprising inactivated SARS-CoV-2 particles and an adjuvant as described herein, as well as kits comprising the individual components thereof (e.g. suitable for bedside mixing), and the combined use of the individual components of the vaccine in preventing or treating SARS-CoV-2 infection.

In some embodiments of the present invention, the SARS-CoV-2 particles are inactivated without substantially modifying their surface structure. In other words, a native surface conformation of the SARS-CoV-2 particles is retained in the inactivated virus particles. It has surprisingly been found that by optimizing an inactivation process, e.g. using beta-propiolactone, infectivity of native SARS-CoV-2 particles can be substantially abrogated, i.e., completely abolished, without adversely affecting their antigenicity and/or immunogenicity. Thus, the present invention provides in one aspect an inactivated virus vaccine (e.g. a beta-propiolactone-inactivated virus vaccine) that generates neutralizing antibodies and/or protective immunity against SARS-CoV-2 infection.

In one embodiment, the SARS-CoV-2 particles are inactivated by a method that preferentially targets viral RNA. By this it is meant that e.g. the inactivation step modifies viral RNA more than viral proteins. Thus, the inactivated SARS-CoV-2 particles may comprise replication-deficient viral RNA, i.e. the viral RNA is modified in the inactivation step such that the inactivated particles are incapable of replicating. By utilizing an inactivation method that preferentially targets viral RNA, the present invention advantageously allows the preservation of immunogenic epitopes in viral surface proteins.

Preferably, the inactivation method spares viral (surface) proteins relative to viral RNA, e.g. the viral surface proteins (e.g. the spike (S) protein) may comprise fewer or more infrequent modifications resulting from the inactivation step compared to viral RNA. For instance, a lower proportion of amino acid residues in the viral surface proteins (e.g. S protein) may be modified by the inactivation step compared to the proportion of modified nucleotide residues in the viral RNA. In some embodiments, the proportion of modified amino acid residues in the viral surface proteins (e.g. S protein) may be at least 5%, 10%, 20%, 30%, 50%, 70% or 90% lower than the proportion of modified nucleotide residues in the viral RNA. By "modifications" or "modified residues" it is meant to refer to non-native residues that are not present in the native SARS-CoV-2 particles, e.g. chemical (covalent) modifications of such residues resulting from the inactivation step.

In one embodiment, the viral RNA is inactivated by alkylation and/or acylation, i.e. the modifications in the SARS-CoV-2 inactivated particles comprise alkylated and/or acylated nucleotide residues. In some embodiments, the modifications are preferentially targeted to purine (especially guanine) residues, e.g. the SARS-CoV-2 inactivated particles comprise one or more modified (e.g. alkylated or acylated) guanine residues. In some cases, the inactivation step may lead to cross-linking of viral RNA with viral proteins, e.g. via guanine residues in the viral RNA. The inactivation step may also introduce nicks or strand breaks into viral RNA, e.g. resulting in fragmentation of the viral genome.

Suitable alkylating and/or acylating agents are known in the art. In one embodiment, the inactivating agent comprises beta-propiolactone, i.e. the vaccine comprises beta-propiolactone-inactivated virus particles. In any case, in a particular embodiment, beta-propiolactone (herein referred to also as "BPL") treatment is particularly preferred according to the present invention, because it results in SARS-CoV-2 particles, that are substantially inactive, but which retain high antigenicity and immunogenicity against neutralizing epitopes present in native SARS-CoV-2. In particular, it has been surprisingly found that beta-propiolactone can be used to inactivate SARS-CoV-2 particles with a minimum number of protein modifications. For instance, as demonstrated in Examples 7 and 10 below, inactivation of SARS-CoV-2 particles using beta-propiolactone results in a much lower number of modifications of viral proteins compared to inactivation of influenza particles by beta-propiolactone. Thus in beta-propiolactone-inactivated SARS-CoV-2 particles, a native surface conformation of the viral particles can be preserved.

In a preferred embodiment of the invention, the viral RNA is inactivated in an optimized manner, i.e. such it is just sufficiently inactivated not to be infectious anymore but not "over"-inactivated so that numerous modification at different amino acids in particular at the S-protein occur. In a further even more preferred embodiment, the BPL inactivation not only sufficiently inactivates (but not over-inactivates) the SARS-CoV-2 virus but also just sufficiently inactivates viruses that might be co-enriched and co-cultured in the manufacturing process (see e.g. experimental part). A particular hard virus to inactivate that can co-culture and be co-enriched is PPV (porcine parvovirus) - see experimental part. The concentration of beta-propiolactone in the inactivation step may be optimized to ensure complete inhibition of viral replication whilst preserving the conformation of surface proteins in the virus. For instance, the concentration of beta-propiolactone in the inactivation step may be e.g. 0.01 to 1% by weight, preferably 0.01 to 0.1% by weight, more preferably about 0.03% by weight. A preferred amount of BPL was found to be 500ppm where the SARS-CoV-2 virus but also other concerning viruses/impurities are inactivated whilst preserving (i.e. not modifying) most of the amino acids of the S-protein (i.e. only a few amino acids were shown to be modified at low probability).

In some embodiments, the native SARS-CoV-2 particles may be contacted with beta-propiolactone for at least 5 hours, at least 10 hours, at least 24 hours or at least 4 days, e.g. 5 to 24 hours or longer such as 48 hours. The inactivation step may be performed at about 0°C to about 25°C, preferably about 4°C or about 22°C, or e.g. 18 to 24°C. In one embodiment the inactivation step (e.g. with beta-propiolactone) is performed at 2°C to 8°C for 24 hours. The inactivation step may optionally and preferably be followed by a hydrolyzation step of the inactivating agent, as is known in the art (which may be performed e.g. at about 37°C+/- 2°C for a total time of 2.5 hours +/- 0.5 hours for beta-propiolactone). Typically, longer incubation times and/or higher temperatures in the inactivation step may enhance viral inactivation, but may also lead to an increased risk of undesirable surface modifications of the viral particles, leading to reduced immunogenicity. Therefore, the inactivation step may be performed for e.g. the shortest time necessary in order to produce a fully inactivated virus particle. After completion of the hydrolysis, the inactivated viral solution was in one embodiment immediately cooled down to 5±3°C and stored there until inactivation was confirmed by large volume plaque assay and serial passaging assay.

Beta-propiolactone inactivation of SARS-CoV-2 particles may preferentially modify cysteine, methionine and/or histidine residues. Thus in some embodiments, the inactivated SARS-CoV-2 particle comprises one or more beta-propiolactone-modified cysteine, methionine and/or histidine residues. However, in embodiments of the present invention, the beta-propiolactone-inactivated SARS-CoV-2 particles show relatively few protein modifications. Thus, for example, an inactivated SARS-CoV-2 particle in the vaccine may comprise fewer than 200, 100, 50, 30, 20, 15, 10, 9, 8, 7 or 6 beta-propiolactone-modified amino acid residues. Preferably a spike (S) protein of the inactivated SARS-CoV-2 particle comprises fewer than 100, 50, 30, 20, 15, 10, 9, 8, 7 or 6 beta-propiolactone-modified amino acid residues. More preferably the inactivated SARS-CoV-2 particle or spike protein thereof comprises 20 or fewer, 15 or fewer, 10 or fewer, or 5 or fewer beta-propiolactone-modified amino acid residues. Most preferably the inactivated SARS-CoV-2 particle or spike protein thereof comprises 1 to 100, 2 to 70, 3 to 50, 4 to 30, 5 to 25, 5 to 20, 10 to 20 or about 15 beta-propiolactone-modified amino acid residues.

In another embodiment, fewer than 20%, 15%, 10%, 5% or 4% of SARS-CoV-2 polypeptides are beta-propiolactone-modified. For instance, 0.1 to 10%, 1 to 8%, 2 to 7% or about 3%, 4%, 5% or 6% of SARS-CoV-2 polypeptides in the particle may be beta-propiolactone-modified. Beta-propiolactone modification of residues and/or polypeptides in the vaccine may be detected by mass spectrometry, e.g. using liquid chromatography with tandem mass spectrometry (LC-MS-MS), for instance using a method as described in Example 7 or Example 10. In such a method, the SARS-CoV-2 particles may be digested in order to fragment proteins into SARS-CoV-2 polypeptides for LC-MS-MS analysis. The digestion step may be performed by any suitable enzyme or combination of enzymes, e.g. by trypsin, chymotrypsin and/or PNGase F (peptide:N-glycosidase F), or by e.g. acid hydrolysis. Preferably the percentage of BPL-modified polypeptides detected by LC-MS-MS following enzymatic digestion or acid hydrolysis is: (a) trypsin digestion, 1 to 5%, 2 to 4% or about 3%; (b) trypsin + PNGase F digestion, 1 to 5%, 2 to 4% or about 3%; (c) chymotrypsin, 1 to 10%, 3 to 8% or about 6% ; (d) acid hydrolysis, 1 to 6%, 2 to 5% or about 4%. In this context, a "beta-propiolactone-modified" polypeptide means that the polypeptide comprises at least one beta-propiolactone modification, e.g. at least one beta-propiolactone-modified residue.

In some embodiments, a spike (S) protein of the inactivated SARS-CoV-2 particle comprises a beta-propiolactone modification at one or more of the following residues: 49, 146, 166, 177, 207, 245, 379, 432, 519, 625, 1029, 1032, 1058, 1083, 1088, 1101, 1159 and/or 1271, e.g. in SEQ ID NO:3, or a corresponding position in SEQ ID NO: 19, 21, 23, 25 or 27. Preferably the inactivated SARS-CoV-2 particle comprises a beta-propiolactone modification at one or more of the following residues: H49, H146, C166, M177, H207, H245, C432, H519, H625, M1029, H1058, H1083, H1088, H1101, H1159 and/or H1271, e.g. in SEQ ID NO:3, or a corresponding position in SEQ ID NO: 19, 21, 23, 25 or 27. In another embodiment, the inactivated SARS-CoV-2 particle comprises a beta-propiolactone modification at one or more of the following residues: H207, H245, C379, M1029 and/or C1032, e.g. in SEQ ID NO:3, or a corresponding position in SEQ ID NO: 19, 21, 23, 25 or 27. By "a corresponding position" it is meant a position in SEQ ID NO: 19, 21, 23, 25 or 27 that aligns with position H207, H245, C379, M1029 and/or C1032 in SEQ ID NO:3, e.g. when SEQ ID NO: 19, 21, 23, 25 or 27 is aligned with SEQ ID NO:3 using a program such as NCBI Basic Local Alignment Search Tool (BLAST).

For instance, in some embodiments, the positions in SEQ ID NO: 19, 21, 23, 25 or 27 corresponding to H207, H245, C379, M1029 and C1032 in SEQ ID NO:3 are shown below:

| Seq ID | | | | | |
|---|---|---|---|---|---|
| 3 | H207 | H245 | C379 | M1029 | C1032 |
| 19 | H207 | H244 | C378 | M1028 | C1031 |
| 21 | H207 | H245 | C379 | M1029 | C1032 |
| 23 | H204 | H242 | C376 | M1026 | C1029 |
| 25 | H207 | H245 | C379 | M1029 | C1032 |
| 27 | H207 | H245 | C379 | M1029 | C1032 |

In some embodiments, a membrane (M) glycoprotein of the inactivated SARS-CoV-2 particle comprises a beta-propiolactone modification at one or more of the following residues: 125, 154, 155, 159 and/or 210, preferably H154, H155, C159 and/or H210, e.g. in SEQ ID NO: 29.

In some embodiments, a nucleocapsid (N) protein of the inactivated SARS-CoV-2 particle comprises a beta-propiolactone modification at M234, e.g. in SEQ ID NO: 28.

In some embodiments, fewer than 30%, 20%, 10%, 5%, 3% or 1% of one or more of the following residues in the inactivated SARS-CoV-2 particles are beta-propiolactone modified: (i) in the spike (S) protein, e.g. in SEQ ID NO:3, or a corresponding position in SEQ ID NO: 19, 21, 23, 25 or 27: residues 49, 146, 166, 177, 207, 245, 379, 432, 519, 625, 1029, 1032, 1058, 1083, 1088, 1101, 1159 and/or 1271; preferably H49, H146, C166, M177, H207, H245, C432, H519, H625, M1029, H1058, H1083, H1088, H1101, H1159 and/or H1271; alternatively H207, H245, C379, M1029 and/or C1032; (ii) in the membrane (M) glycoprotein, e.g. in SEQ ID NO: 29: residues 125, 154, 155, 159 and/or 210; preferably H154, H155, C159 and/or H210; and/or (iii) M234 of the nucleocapsid (N) protein, e.g. in SEQ ID NO: 28. In preferred embodiments, fewer than 30%, 20%, 10%, 5%, 3% or 1% of at least 2, 3, 4, 5, 6, 7, 8, 9, 10, 11, 12, 13, 14, 15, 16, 17, 18, 19, 20 or each of the above residues in the inactivated SARS-CoV-2 particles are beta-propiolactone modified. In this paragraph, the % of modified residues is intended to refer to the site occupancy, e.g. the ratio of modified to unmodified peptide for the same modification site normalized to the protein abundance as described in Example 7 or 10 below.

In another preferred embodiment, the proportion of beta-propiolactone-modified residues (i.e. site occupancy) at the following positions in the inactivated SARS-CoV-2 particles is:
(i) in the spike (S) protein (e.g. of SEQ ID NO: 3, or a corresponding position in SEQ ID NO: 19, 21, 23, 25 or 27):
   (a) H207: less than 30%, preferably 0.01 to 25%; and/or
   (b) H245: less than 10%, preferably 0.1 to 5%; and/or
   (c) C379: less than 5%, less than 1% or less than 0.1%; and/or
   (d) M1029: less than 5%, less than 1% or less than 0.1%; and/or
   (e) C1032: less than 5%, less than 1% or less than 0.1%; and/or
(ii) in the membrane (M) glycoprotein (e.g. of SEQ ID NO: 29):
   (f) H154: less than 5%, less than 1% or less than 0.1%; and/or
   (g) H155: less than 10%, preferably 0.1 to 5%; and/or
   (h) C159: less than 5%, less than 1% or less than 0.1%; and/or
   (i) H210: less than 20%, preferably 0.1 to 10%; and/or
(iii) in the nucleocapsid (N) protein (e.g. of SEQ ID NO: 28):
   (j) M234: less than 90%, less than 10% or less than 0.1%.

In another preferred embodiment, the proportion of beta-propiolactone-modified residues (i.e. site occupancy) at each of the following positions in the spike (S) protein (e.g. of SEQ ID NO: 3, or a corresponding position in SEQ ID NO: 19, 21, 23, 25 or 27) of the inactivated SARS-CoV-2 particles is:
(a) residues H49, H146, C166, H207, H519, M1029, H1083, H1088, H1101, H1159 and/or H1271: less than 20%, preferably 0.01 to 10%, more preferably 0.1 to 5%; and/or
(b) residues M177, C432, H625: less than 30%, preferably 0.1 to 20%, more preferably 1 to 10%; and/or
(c) residues H245, H1058: less than 30%, preferably 0.1 to 20%, more preferably 5 to 15%;

In some embodiments, the proportion of beta-propiolactone-modified amino acid residues in the inactivated SARS-CoV-2 particle (or spike (S) protein thereof) may be at least 5%, 10%, 20%, 30%, 50%, 70% or 90% lower than the proportion of modified residues in a beta-propiolactone-inactivated influenza particle (or hemagglutinin (HA) or neuraminidase (NA) protein thereof), e.g. in an influenza particle that has been inactivated under similar conditions to the SARS-CoV-2 particle.

In an alternative embodiment, the viral RNA may be inactivated by treatment with ultraviolet (UV) light. UV treatment can be used to preferentially target RNA (compared to polypeptides) in the viral particles, resulting in e.g. modified nucleotides and/or fragmentation. In some embodiments, UV treatment can be combined with beta-propiolactone treatment to improve inactivation of the virus, e.g. a beta-propiolactone treatment step can be followed by a UV treatment step or vice versa, or a UV treatment step can be performed at the same time as the beta-propiolactone treatment step.

In other embodiments, the native SARS-CoV-2 particles may be inactivated using formaldehyde. However, formaldehyde inactivation is typically less preferred in the present invention, as it is less suitable for preferentially targeting viral RNA while preserving immunogenic epitopes in the viral surface proteins.

Therefore, in preferred embodiments, the inactivation step(s) (especially when using formaldehyde, but also when using other inactivating agents such as e.g. beta-propiolactone) are performed under mild conditions in order to preserve surface antigen integrity, especially integrity of the S protein.

In one embodiment, such a mild inactivation method comprises contacting a liquid composition comprising native SARS-CoV-2 particles with a chemical viral inactivating agent (such as e.g. any of the chemical inactivation agents as listed above or a combination thereof, for instance formaldehyde or preferably beta-propiolactone) in a container, mixing the chemical viral inactivating agent and the liquid composition comprising SARS-CoV-2 particles under conditions of laminar flow but not turbulent flow, and incubating the chemical viral inactivating agent and the liquid composition comprising SARS-CoV-2 particles for a time sufficient to inactivate the viral particles. The mild inactivation step is optionally performed in a flexible bioreactor bag. The mild inactivation step preferably comprises five or less container inversions during the period of inactivation. Preferably, the mixing of the chemical viral inactivating agent and the composition comprising native SARS-CoV-2 particles comprises subjecting the container to rocking, rotation, orbital shaking, or oscillation for not more than 10 minutes at not more than 10 rpm during the period of incubation.

Suitable mild or gentle inactivation methods are described below in the Examples. Further details of such methods are also described in WO 2021/048221, the contents of which are incorporated herein in their entirety.

Typically, the inactivation step substantially eliminates infectivity of mammalian (e.g. human) cells by the inactivated SARS-CoV-2 particle. For instance, infectivity of mammalian cells may be reduced by at least 99%, 99.99% or 99.9999% as compared to a native SARS-CoV-2 particle, or infectivity of human cells by the inactivated A SARS-CoV-2 particle may be undetectable. Standard assays may be used for determining residual infectivity and effective viral titer, e.g. plaque assays, determination of TCID₅₀ (50% tissue culture infectious dose). For instance, the mammalian cells may be MDCK, COS or Vero cells.

In preferred embodiments of the present invention, a native surface conformation of the SARS-CoV-2 particles is preserved in the inactivated virus particles. By this it is meant that e.g. one or more, most or all immunogenic (neutralizing) epitopes are retained in the inactivated virus particles, such that the inactivated particles are capable of generating neutralizing antibodies against native SARS-CoV-2 particles when administered to a human subject. By "native surface conformation", it is meant to refer to the surface conformation found in native SARS-CoV-2 particles, i.e. SARS-CoV-2 particles (virions) that have not been inactivated. The property of the vaccine or inactivated SARS-CoV-2 particles in generating neutralizing antibodies in a subject may be determined using e.g. a plaque reduction neutralization test (PRNT assay), e.g. using a serum sample from the immunized subject as known in the art.

In preferred embodiments, the present invention comprises that a native conformation of (i) spike (S) protein; (ii) nucleocapsid (N) protein; (iii) membrane (M) glycoprotein; and/or (iv) envelope (E) protein is preserved in the inactivated viral particles. Preferably, the inactivated SARS-CoV-2 particle comprises a native conformation spike (S) protein. Thus, the S (and/or N and/or M and/or E) protein in the inactivated SARS-CoV-2 particle preferably comprises one or more or all (intact) immunogenic (neutralizing) epitopes present in native SARS-CoV-2 particles. Preferably, the S (and/or N and/or M and/or E) protein in the inactivated viral particles are not modified, or not substantially modified by the inactivation step.

Preservation of the surface conformation of the viral particles can be assessed using standard techniques. For instance, methods such as X-ray crystallography, MS analysis (shift of amino acid mass by modification) and cryo-electron microscopy may be used to visualize the virus surface. The secondary and tertiary structures of proteins present on the surface of viral particles may also be analyzed by methods such as by circular dichroism (CD) spectroscopy (e.g. in the far (190-250 nm) UV or near (250-300 nm) UV range). Moreover, preservation of a native surface conformation can be confirmed by using antibodies directed against epitopes present on the native viral surface, e.g. in the S protein. Reactivity of anti-SARS-CoV-2 antibodies with both the inactivated and native virus particles can thus be used to demonstrate retention of potentially neutralizing epitopes in the vaccine.

The surface conformation of SARS-CoV-2 virions and in particular the spike (S) protein is known, and has been published in several recent studies. See for instance Shang, J. et al. (Structural basis of receptor recognition by SARS-CoV-2. Nature https://doi.org/10.1038/s41586-020-2179-y (2020)), which describes the crystal structure of the SARS-CoV-2 receptor binding domain. In addition, Walls et al. (Structure, Function, and Antigenicity of the SARS-CoV-2 Spike Glycoprotein, Cell 180, 1-12 (2020), https://doi.org/10.1016/j.cell.2020.02.058) provides a detailed description of the S protein surface conformation using cryo-EM, and describes cross-neutralizing antibodies that target conserved S protein epitopes.

Monoclonal antibodies against SARS-CoV-2 surface epitopes (including in the S protein) are described in the literature (e.g. as mentioned above), available from commercial sources and/or can be generated using standard techniques, such as immunization of experimental animals. For example, as of September 9, 2020, at least 169 different antibodies against SARS-CoV-2 were available from MyBioSource, Inc., San Diego, CA (e.g. cat. no. MBS8574747, see www.MyBioSource.com). On the same date at least 28 different antibodies against SARS-CoV-2 were available from Sino Biological US Inc., Wayne, PA (e.g. cat. no. 40150-D006, see https://www.sinobiological.com/). Further suitable antibodies are described in Ou et al. (Characterization of spike glycoprotein of SARS-CoV-2 on virus entry and its immune cross-reactivity with SARS-CoV, Nature Communications (2020) 11:1620; https://doi.org/10.1038/s41467-020-15562-9). In embodiments of the present invention, a skilled person can detect preservation of a native surface conformation of SARS-CoV-2 (or e.g. the S protein thereof) via binding of such antibodies to the inactivated particles. In other words, the inactivated particles bind specifically to one or more anti-SARS-CoV-2 antibodies directed against surface epitopes, preferably anti-S-protein antibodies, e.g. to antibodies generated against neutralizing epitopes in native SARS-CoV-2 virions.

The SARS-CoV-2 particles in the vaccine composition may be derived from any known strain of SARS-CoV-2, or variants thereof. For instance, the virus may be a strain as defined in Figure 2, or may comprise a nucleotide or amino acid sequence as defined therein, or a variant sequence having at least e.g. 95% sequence identity thereto. For instance, in one embodiment the SARS-CoV-2 particle comprises an RNA sequence corresponding to a DNA sequence as defined by SEQ ID NOs: 1, 9, 12 or 15. In a preferred embodiment, the SARS-CoV-2 particle comprises an RNA sequence corresponding to the DNA sequence defined by SEQ ID NO: 9; i.e. the Italy-INMIl SARS CoV-2 virus. This SARS-CoV-2 isolate was the first to be identified and characterized at the National Institute for Infectious Diseases "Lazzaro Spallanzani" IRCCS, Rome, Italy (Capobianchi MR, et al. Molecular characterization of SARS-CoV-2 from the first case of COVID-19 in Italy. 2020 Clin Microbiol Infect. 2020 Jul; 26(7): 954-956; doi: 10.1016/j.cmi.2020.03.025). By "corresponding to", it will be understood that the defined DNA sequence is an equivalent of the viral RNA sequence, i.e. is a DNA or cDNA sequence that encodes the viral RNA or a sequence complementary to the viral RNA. As described herein, the inactivation process may result in modification (e.g. alkylation or acylation) and/or fragmentation of viral RNA, and thus it will be understood that the inactivated viral particles may not comprise an intact RNA sequence as defined herein, but rather are derived from native viral particles which do comprise such a sequence.

The SARS-CoV-2 particles may also comprise variants of the known SARS-CoV-2 Wuhan-Hu-1 lineage or also referred to as the reference lineage, e.g. sequences having at least 85%, at least 90%, at least 95% or at least 99% sequence identity to SEQ ID NO: 1 and/or NCBI Reference Sequence NC_045512.2. Preferably, the variant sequence encodes an infectious SARS-CoV-2 particle, e.g. a native (non-inactivated) SARS-CoV-2 particle comprising the RNA sequence that is able to pack a virulent SARS-CoV-2 virus.

Further known SARS-CoV-2 particles may also comprise variants of the known SARS-CoV-2 South African lineage B.1.351, e.g. sequences having at least 85%, at least 90%, at least 95% or at least 99% sequence identity to SEQ ID NO: 18 and/or NCBI Reference Sequence MW598408. Preferably, the variant sequence encodes an infectious SARS-CoV-2 particle, e.g. a native (non-inactivated) SARS-CoV-2 particle comprising the RNA sequence that is able to pack a virulent SARS-CoV-2 virus. Further examples of variants of the known SARS-CoV-2 South African lineage B.1.351 are given in Figure 2.

Further known SARS-CoV-2 particles may also comprise variants of the known SARS-CoV-2 Brazilian lineage P.1, e.g. sequences having at least 85%, at least 90%, at least 95% or at least 99% sequence identity to SEQ ID NO: 20 and/or NCBI Reference Sequence MW520923. Preferably, the variant sequence encodes an infectious SARS-CoV-2 particle, e.g. a native (non-inactivated) SARS-CoV-2 particle comprising the RNA sequence that is able to pack a virulent SARS-CoV-2 virus. Further examples of variants of the known SARS-CoV-2 Brazilian lineage P.1 are given in Figure 2.

Further known SARS-CoV-2 particles may also comprise variants of the known SARS-CoV-2 UK lineage B.1.1.7, e.g. sequences having at least 85%, at least 90%, at least 95% or at least 99% sequence identity to SEQ ID NO: 22 and/or NCBI Reference Sequence MW422256. Preferably, the variant sequence encodes an infectious SARS-CoV-2 particle, e.g. a native (non-inactivated) SARS-CoV-2 particle comprising the RNA sequence that is able to pack a virulent SARS-CoV-2 virus. Further examples of variants of the known SARS-CoV-2 UK lineage B.1.1.7 are given in Figure 2.

Further known SARS-CoV-2 particles may also comprise variants of the known SARS-CoV-2 Californian lineages B. 1.427 and B.1.429, e.g. sequences having at least 85%, at least 90%, at least 95% or at least 99% sequence identity to SEQ ID NO: 24 and/or SEQ ID NO: 26. Preferably, the variant sequence encodes an infectious SARS-CoV-2 particle, e.g. a native (non-inactivated) SARS-CoV-2 particle comprising the RNA sequence that is able to pack a virulent SARS-CoV-2 virus. Further examples of variants of the known SARS-CoV-2 Californian lineages can be found in Genebank.

Similarly, in preferred embodiments the SARS-CoV-2 particle comprises an S protein of the Wuhan lineage comprising or consisting of (i) an amino acid sequence as defined in SEQ ID NO: 3, or (ii) an amino acid sequence having at least 95%, at least 97% or at least 99% identity to SEQ ID NO: 3.

In further preferred embodiments the SARS-CoV-2 particle comprises an S protein of the South African B1.351 lineage comprising or consisting of (i) an amino acid sequence as defined in SEQ ID NO: 19, or (ii) an amino acid sequence having at least 95%, at least 97% or at least 99% identity to SEQ ID NO: 19.

In further preferred embodiments the SARS-CoV-2 particle comprises an S protein of the Brazilian P.1 lineage comprising or consisting of (i) an amino acid sequence as defined in SEQ ID NO: 21, or (ii) an amino acid sequence having at least 95%, at least 97% or at least 99% identity to SEQ ID NO: 21.

In further preferred embodiments the SARS-CoV-2 particle comprises an S protein of the UK B.1.1.7 lineage comprising or consisting of (i) an amino acid sequence as defined in SEQ ID NO: 23, or (ii) an amino acid sequence having at least 95%, at least 97% or at least 99% identity to SEQ ID NO: 23. In further preferred embodiments the SARS-CoV-2 particle comprises an S protein of the Californian B.1.427 lineage comprising or consisting of (i) an amino acid sequence as defined in SEQ ID NO: 25, or (ii) a an amino acid sequence having at least 95%, at least 97% or at least 99% identity to SEQ ID NO: 25.

In further preferred embodiments the SARS-CoV-2 particle comprises an S protein of the Californian B.1.429 lineage comprising or consisting of (i) an amino acid sequence as defined in SEQ ID NO: 27, or (ii) an amino acid sequence having at least 95%, at least 97% or at least 99% identity to SEQ ID NO: 27.

In some embodiments, the inactivated SARS-CoV-2 particles are combined with other inactivated SARS-CoV-2 particles in the vaccine (other = other sequence).

In some embodiments, a combination of SARS-CoV-2 particles in the vaccine comprises or consists of at least two SARS-CoV-2 particles selected from the group consisting of i) the reference Wuhan_1 lineage such as e.g. SEQ ID NOs 1, 9, 12, 15; ii) the South African B.1.531 lineage such as e.g. SEQ ID NO: 18; the Brazilian P.1 lineage such as e.g. SEQ ID NO: 20; the UK B.1.1.7 lineage such as e.g. SEQ ID NO: 22 and the Californian B.1.427 such as e.g. SEQ ID NO: 24 or B.1.429 lineages such as e.g. SEQ ID NO: 26. A preferred embodiment is a combination comprising i) a Wuhan_1 lineage such as e.g. SEQ ID NO: 9; and ii) a South African B.1.531 lineage such as e.g. SEQ ID NO: 18.

In a further embodiment, a combination of SARS-CoV-2 particles in the vaccine comprises or consists of at least three, e.g. three SARS-CoV-2 particles selected from the group consisting of i) the reference Wuhan_1 lineage such as e.g. SEQ ID NOs 1, 9, 12, 15; ii) the South African B.1.531 lineage such as e.g. SEQ ID NO: 18; the Brazilian P.1 lineage such as e.g. SEQ ID NO: 20; the UK B.1.1.7 lineage such as e.g. SEQ ID NO: 22 and the Californian B.1.427 such as e.g. SEQ ID NO: 24 or B.1.429 lineages such as e.g. SEQ ID NO: 26. A preferred embodiment of such a trivalent vaccine is a combination comprising i) a Wuhan_1 lineage such as e.g. SEQ ID NO: 9; and ii) a South African B.1.531 lineage such as e.g. SEQ ID NO: 18; and iii) an UK B.1.1.7 lineage such as e.g. SEQ ID NO: 22. Another preferred embodiment of such a trivalent vaccine is a combination comprising i) a Wuhan_1 lineage such as e.g. SEQ ID NO: 9; and ii) a South African B.1.531 lineage such as e.g. SEQ ID NO: 18; and iii) a Brazilian P.1 lineage such as e.g. SEQ ID NO: 20.

The similarity between amino acid sequences and/or nucleic acid sequences is expressed in terms of the percentage of identical matches between the sequences, otherwise referred to as sequence identity. Sequence identity is frequently measured in terms of percentage identity; the higher the percentage, the more similar the two sequences are. Homologs, orthologs, or variants of a polynucleotide or polypeptide will possess a relatively high degree of sequence identity when aligned using standard methods.

Methods of alignment of sequences for comparison are well known in the art. Various programs and alignment algorithms are described in: Smith & Waterman, Adv. Appl. Math. 2:482, 1981; Needleman & Wunsch, Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5: 151-3, 1989; Corpet et al., Nuc. Acids Res. 16: 10881-90, 1988; Huang et al. Computer Appls. in the Biosciences 8, 155-65, 1992; and Pearson et al., Meth. Mol. Bio. 24:307-31, 1994. Altschul et al, J. Mol. Biol. 215:403-10, 1990, presents a detailed consideration of sequence alignment methods and homology calculations.

Once aligned, the number of matches is determined by counting the number of positions where an identical nucleotide or amino acid residue is present in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. Preferably, the percentage sequence identity is determined over the full length of the sequence. For example, a peptide sequence that has 1166 matches when aligned with a test sequence having 1554 amino acids is 75.0 percent identical to the test sequence (1166÷1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer.

The NCBI Basic Local Alignment Search Tool (BLAST) (Altschul et al., Mol. Biol. 215:403, 1990) is available from several sources, including the National Center for Biotechnology Information (NCBI, Bethesda, MD) and on the internet, for use in connection with the sequence analysis programs BLASTP, BLASTN, BLASTX, TBLASTN and TBLASTX. A description of how to determine sequence identity using this program is available on the NCBI website on the internet. The BLAST and the BLAST 2.0 algorithms are also described in Altschul et al., Nucleic Acids Res. 25:3389-3402, 1977. Software for performing BLAST analyses is publicly available through the National Center for Biotechnology Information (ncbi.nlm.nih.gov). The BLASTN program (for nucleotide sequences) uses as defaults a word length (W) of 11, alignments (B) of 50, expectation (E) of 10, M=5, N=-4, and a comparison of both strands. The BLASTP program (for amino acid sequences) uses as defaults a word length (W) of 3, and expectation (E) of 10, and the BLOSUM62 scoring matrix (see Henikoff & Henikoff, Proc. Natl. Acad. Sci. USA 89: 10915, 1989).

Homologs and variants of a polynucleotide or polypeptide are typically characterized by possession of at least about 75%, for example at least about 80%, 85%, 90%, 91%, 92%, 93%, 94%, 95%, 96%, 97%, 98% or 99% sequence identity counted over at least 50, 100, 150, 250, 500, 1000, 2000, 5000 or 10,000 nucleotide or amino acid residues of the reference sequence, over the full length of the reference sequence or over the full length alignment with the reference amino acid sequence of interest. Polynucleotides or proteins with even greater similarity to the reference sequences will show increasing percentage identities when assessed by this method, such as at least 80%, at least 85%, at least 90%, at least 95%, at least 98%, or at least 99% sequence identity. For sequence comparison of amino acid or nucleic acid sequences, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters are used.

One example of a useful algorithm is PILEUP, which uses a simplification of the progressive alignment method of Feng & Doolittle, Mol. Evol. 35:351-360, 1987. The method used is similar to the method described by Higgins & Sharp, CABIOS 5:151-153, 1989. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., Nuc. Acids Res. 12:387-395, 1984).

As used herein, reference to "at least 80% identity" refers to at least 80%, at least 85%, at least 90%, at least 91%, at least 92%, at least 93%, at least 94%, at least 95%, at least 96%, at least 97%, at least 98%, at least 99%, or even 100% identity to a specified reference sequence, e.g. to at least 50, 100, 150, 250, 500, 1000, 5000 or 10,000 nucleotide or amino acid residues of the reference sequence or to the full length of the sequence. As used herein, reference to "at least 90% identity" refers to at least 90%, at least 91%, at least 92%, at least 93%, at least 94%, at least 95%, at least 96%, at least 97%, at least 98%, at least 99%, or even 100% identity to a specified reference sequence, e.g. to at least 50, 100, 150, 250, 500, 1000, 5000 or 10,000 nucleotide or amino acid residues of the reference sequence or to the full length of the sequence.

The SARS-CoV-2 vaccine may be produced by methods involving a step of inactivation of native SARS-CoV-2 particles, as described above. Generally, the native SARS-CoV-2 particles may be obtained by standard culture methods, e.g.by *in vitro* production in mammalian cells, preferably using Vero cells. For instance, the native SARS-CoV-2 particles may be produced using methods analogous to those described in e.g. WO 2017/109225 and/or WO 2019/057793 (the contents of which are incorporated herein in their entirety), which describe methods for the production of Zika and Chikungunya viruses in Vero cells. The steps such as passaging, harvesting, precipitation, dialysis, filtering and purification described in those documents are equally applicable to the present process for producing SARS-CoV-2 particles.

For instance, in some embodiments, the method may comprise purifying the inactivated SARS-CoV-2 particles by one or more size exclusion methods such as (i) a sucrose density gradient centrifugation, (ii) a solid-phase matrix packed in a column comprising a ligand-activated core and an inactive shell comprising pores, wherein the molecular weight cut-off of the pores excludes the virus particles from entering the ligand-activated core, and wherein a molecule smaller than the molecular weight cut-off of the pores can enter the ligand-activated core and collecting the virus particles, and/or (iii) batch or size exclusion chromatography; to obtain purified inactivated SARS-CoV-2 particles. Preferably, in the resulting purified preparation of viral particles, (i) the concentration of residual host cell DNA is less than 100 ng/mL; (ii) the concentration of residual host cell protein is less than 1 µg/mL; and (iii) the concentration of residual aggregates of infectious virus particles is less than 1 µg/mL.

In some embodiments, the method may comprise a step of precipitating a harvested culture medium comprising SARS-CoV-2 particles, thereby producing native SARS-CoV-2 particles in a supernatant. The precipitating step may comprise contacting the culture medium with protamine sulfate or benzonase. By using such a step, both contaminating DNA derived from host cells as well as immature and otherwise non-infectious virus particles can be separated from the preparation. Moreover, protamine sulfate can be very efficiently separated from the virus fraction, e.g. using sucrose density centrifugation or a solid-phase matrix packed in a column comprising a ligand-activated core and an inactive shell comprising pores, wherein the pores comprise a molecular weight cut-off that excludes the virus particles from entering the ligand-activated core, and wherein a molecule smaller than the molecular weight cut-off of the pores (e.g. the protamine sulfate) can enter the ligand-activated core,, allowing for a safer vaccine produced at high yields.

Thus the residual host cell DNA of the obtained virus preparation or vaccine may be less than 1 µg/mL. especially less than 900, 800, 700, 600, 500, 400, 300 or 200 ng/mL, preferably less than 150 or 100 ng/mL. In a preferred embodiment, the residual host cell DNA of the virus preparation or vaccine is less than 40 pg/mL. In some embodiments, the residual host cell protein of the virus preparation or vaccine is less than 10 µg/mL, especially less than 9, 8, 7, 6, 5, 4, 3 or 2 µg/mL, preferably less than 1 µg/mL. In a preferred embodiment, the residual host cell protein of the virus preparation or vaccine is less than 150 ng/mL. In some embodiments, the residual non-infectious virus particles of the virus preparation or vaccine is less than 10 µg/mL, especially less than 9, 8, 7, 6, 5, 4, 3 or 2 µg/mL. preferably less than 1 µg/mL. In a preferred embodiment, the content of residual non-infectious virus particles of the virus preparation or vaccine is less than 100 ng/mL.

In some embodiments, the vaccine and/or SARS-CoV-2 particles may comprise residual protamine (e.g. protamine sulfate), typically in trace amounts. In some embodiments, residual protamine (e.g. protamine sulfate) in the virus preparation or vaccine is less than 2 µg/mL or 1 µg/mL, especially less than 900, 800, 700, 600, 500, 400, 300 or 200 ng/mL, preferably less than 100 ng/mL, more preferably is below the detection limit of HPLC, in particular below the detection limit in the final drug substance. In some embodiments, the PS content is tested by HPLC or size exclusion chromatography (SEC). For example, HPLC is validated for PS determination in JEV sucrose gradient pool samples as a routine release assay and is very sensitive (i.e., limit of quantification (LOQ) 3 µg/mL: limit of detection (LOD) 1 µg/mL). In the current invention, PS content in SARS-CoV-2 drug substance was <LOD. In one embodiment, the HPLC assessment of PS content can be performed on a Superdex Peptide 10/300GL column (GE: 17-5176-01) using 30% Acetonitrile, 0,1% Trifluoroacetic acid as solvent with a flow rate of 0.6 ml/min at 25°C and detection at 214 nm. A more sensitive method of measurement for residual protamine in a purified virus preparation is mass spectrometry (MS). In some embodiments, the residual PS levels in a Zika virus preparation are tested by MS or other such highly sensitive method, e.g. nuclear magnetic resonance (NMR). With this method, residual PS, as well as fragments and/or break-down products of PS, can be detected at trace amounts, such as levels as low as, for example, 10⁶, 10⁷ or 10⁸ molecules per typical sample load. In some embodiments, the PS levels are tested in the drug product. In some embodiments, the PS levels are tested in the drug substance.

Preferably an amount of the inactivating agent (e.g. beta-propiolactone) in the drug product or drug substance (e.g. vaccine composition) is very low, e.g. less than 100 ppm, less than 10 ppm, or less than 1 ppm (by weight).

The SARS-CoV-2 vaccine may be administered to a subject, preferably a mammalian subject, more preferably a human subject. Typically, the SARS-CoV-2 vaccine is administered to a subject at risk of SARS-CoV-2 infection, e.g. in order to prevent SARS-CoV-2 infection and/or to prevent SARS-CoV-2 associated disease (COVID-19). The subject is preferably (i) an elderly subject (e.g. older than 65 years, 70 years or 80 years) (ii) a pregnant subject (iii) an immunocompromised subject or (iv) a child (e.g. a person younger than 18 years, 16 years, 14 years, 12 years, 10 years, 8 years, 6 years, 4 years, 2 years or younger). The SARS-CoV-2 vaccine described herein is advantageously capable of generating robust immune responses in subjects particularly susceptible or vulnerable to SARS-CoV-2 morbidity or mortality, i.e. immunocompromised, pregnant or elderly subjects. The SARS-CoV-2 vaccine may be administered to the subject in a single dose or two or more doses, e.g. separated by intervals of about 7, 14, 21 or 28 days.

In a preferred embodiment, on administration to a human subject the vaccine does not induce vaccine mediated disease enhancement (potentially through Antibody dependent enhancement). Vaccine-mediated disease enhancement is characterized by a vaccine that results in increased disease severity if the subject is later infected by the natural virus. It is an advantage of the present invention that the inactivated SARS-CoV-2 vaccine described herein does not promote vaccine mediated disease enhancement in human subjects, and can therefore be safely used for mass vaccination purposes. In particular, the vaccine described herein retains high quality immunogenic epitopes, which therefore results in high neutralizing antibody titers and diminishes the risk of vaccine mediated disease enhancement on administration to subjects. The risk of vaccine mediated disease enhancement development may be assessed in non-human primates. Guidance in this regard is given in the Consensus summary report for CEPI/BC March 12-13, 2020 meeting: Assessment of risk of disease enhancement with COVID-19 vaccines (Lambert, P-H, et al. 2020, doi: 10.1016/j.vaccine.2020.05.064).

In another preferred embodiment, on administration to a human subject the vaccine does not result in immunopathology. In mice, SARS-CoV-1 vaccine induced immunopathology was considered a consequence of a dominant Th2 type response to the vaccine antigens (Tseng et al., 2012, supra). In embodiments of the present invention, a Th1 type response is enhanced or favored, e.g. by use of the Th1-directing adjuvant CpG. The risk of immunopathology developing may be assessed in animal models, e.g. as described in Tseng C.T. et al. (2012) PLoS ONE 7(4):e35421.

Any of the SARS-CoV-2 vaccines or compositions described herein may be administered to a subject in a therapeutically effective amount or a dose of a therapeutically effective amount. As used herein, a "therapeutically effective amount" of vaccine is any amount that results in a desired response or outcome in a subject, such as those described herein, including but not limited to prevention of infection, an immune response or an enhanced immune response to SARS-CoV-2, or prevention or reduction of symptoms associated with SARS-CoV-2 disease.

In some embodiments, the therapeutically effective amount or prophylactically effective amount (dosage) of a SARS-CoV-2 vaccine or composition described herein is an amount sufficient to generate antigen-specific antibodies (*e.g.,* anti-SARS-CoV-2 antibodies). In some embodiments, the therapeutically or prophylactically effective amount is sufficient to seroconvert a subject with at least 70% probability. In some embodiments, the therapeutically or prophylactically effective amount is sufficient to seroconvert a subject with at least 75%, 80%, 85% 90%, 95%, 96%, 97%, 98%, or at least 99% probability. Whether a subject has seroconverted can be assessed by any method known in the art, such as obtaining a serum sample from the subject and performing an assay to detect anti-SARS-CoV-2 antibodies. In some embodiments, a subject is seroconverted if a serum sample from the subject contains an amount of anti-SARS-CoV-2 antibodies that surpasses a threshold or predetermined baseline. A subject is generally considered seroconverted if there is at least a 4-fold increase in anti-SARS-CoV-2 antibodies (*i.e.,* anti-SARS-CoV-2 S protein IgG antibodies) present in a serum sample from the subject as compared to a serum sample previously taken from the same subject.

In one embodiment, the dose of the inactivated SARS-CoV-2 virus in the vaccine composition of the current invention is between about 0.01 and 25 mAU (milli-absorption units x minutes as assessed by SEC-HPLC), preferably between about 0.05 and 10 mAU, more preferably between about 0.1 and 5 mAU, most preferably between about 0.25 and 2.5 mAU. In one embodiment, the dose is between about 0.05 and 50 µg total protein as measured by (µ)BCA assay, between about 0.1 and 25 µg, between about 0.25 and 12.5 µg, preferably between about 0.5 and 5 µg total protein. More preferably the dose of the inactivated SARS-CoV-2 virus in the vaccine composition is at least 2.5 µg total protein, at least 3.5 µg total protein or at least 2.5 µg total protein, e.g. the vaccine composition comprises 2.5 µg to 25 µg, 3.5 µg to 10 µg or 4 µg to 6 µg total protein/dose, preferably about 5 µg total protein/dose. In some embodiments, the dosage is determined by the total amount of S protein in the inactivated SARS-CoV-2 formulation, as assessed by e.g. ELISA. The mass of antigen may also be estimated by assessing the SE-HPLC peak area per dose equivalent (recorded as milli-absorption units x minutes; mAU), which is estimated to be approximately 2 µg/ml total surface protein and approximately 1 µg/mL S-protein. In one embodiment, the dose is between about 0.025 and 25 µg S-protein as measured by ELISA, between about 0.05 and 12.5 µg, between about 0.125 and 6.25 µg, preferably between about 0.25 and 2.5 µg S-protein.

In a preferred embodiment, the amount of antigen in the SARS-CoV-2 vaccine is determined by ELISA. In one embodiment, the ELISA measures a SARS-CoV-2 protein or portion of a protein, e.g., nucleocapsid (N), membrane (M) or spike (S) protein; i.e., the ELISA utilizes a coating antibody specific to a SARS-CoV-2 protein or portion of a protein. In a preferred embodiment, the coating antibody is specific to the SARS-CoV-2 Spike protein S1 subunit, e.g. residues 14-685 (or 14-683) of the S-protein sequence of SEQ ID NO:3, 19, 21, 23, 25 or 27, or to the Receptor Binding Domain (RBD), e.g. residues 331 to 528 (or 319 to 541) of the S-protein sequence of SEQ ID NO: 3, 19, 21, 23, 25 or 27 (see Figure 13). In one embodiment, the ELISA readout is a mass per unit measure of the detected protein, e.g. µg/mL S-protein. In a preferred embodiment, the standard used is a spike protein trimer and the results of the SARS-CoV-2 ELISA are reported as "antigen units" (AU), corresponding to the ACE-2 binding ability of the standard protein (determined by the manufacturer).

In one embodiment, the amount of SARS-CoV-2 antigen administered to a subject is between about 1 to 100 AU/dose, preferably between about 2 to 75 AU/dose, preferably between about 3 and 60 AU/dose, more preferably between about 3 and 55 AU/dose, more preferably between about 3 and 53 AU/dose. In an even more preferred embodiment, the amount of SARS-CoV-2 antigen administered to a subject is 3 AU, 10 AU or 40 AU per dose, most preferred 40 AU per dose. In further preferred embodiments, the amount of SARS-CoV-2 antigen administered to a subject is at least 10 AU/dose, at least 20 AU/dose, at least 25 AU/dose or at least 30 AU/dose, e.g. about 10 to 60 AU/dose, 20 to 50 AU/dose, 25 to 45 AU/dose or 30 to 40 AU/dose, e.g. about 35 AU/dose. The amount of SARS-CoV-2 antigen (e.g. in AU/dose) may be assessed, for example, by a SARS-CoV-2 ELISA assay as described in Example 1. It is estimated that there are about 1 to 1.5 x 10⁷ viral particles per AU, and the amounts of SARS-CoV-2 antigen described above may be construed accordingly. Thus in some embodiments, the amount of SARS-CoV-2 antigen administered to a subject is between about 1.5 x 10⁷ to 1.5 x 10⁹ viral particles/dose, or between about 4.5 x 10⁷ to 9.0 x 10⁸ viral particles/dose, e.g. at least 1.5 x 10⁸ viral particles/dose or at least 3.0 x 10⁸ viral particles/dose, about 1.5 x 10⁸ to 7.5 x 10⁸ viral particles/dose or about 4.5 x 10⁸ to 6.0 x 10⁸ viral particles/dose.

In some embodiments, seroconversion of a subject is assessed by performing a plaque reduction neutralization test (PRNT). Briefly, PRNT is used to determine the serum titer required to reduce the number of SARS-CoV-2 plaques by 50% (PRNT₅₀) as compared to a control serum/antibody. The PRNT₅₀ may be carried out using monolayers of Vero cells or any other cell type/line that can be infected with SARS-CoV-2. Sera from subjects are diluted and incubated with live, non-inactivated SARS-CoV-2. The serum/virus mixture may be applied to Vero cells and incubated for a period of time. Plaques formed on the Vero cell monolayers are counted and compared to the number of plaques formed by the SARS-CoV-2 in the absence of serum or a control antibody. As a guideline, a threshold of neutralizing antibodies of 1:10 dilution of serum in a PRNT₅₀ is generally accepted as evidence of protection in the case of JEV (Hombach et. al. Vaccine (2005) 23:5205-5211).

In some embodiments, the SARS-CoV-2 particles may be formulated for administration in a composition, such as a pharmaceutical composition. The term "pharmaceutical composition" as used herein means a product that results from the mixing or combining of at least one active ingredient, such as an inactivated SARS-CoV-2, and one or more inactive ingredients, which may include one or more pharmaceutically acceptable excipients. A preferred pharmaceutically acceptable excipient is human serum albumin (HSA), such as, especially recombinant HSA (rHSA). In one embodiment, the SARS-CoV-2 vaccine of the invention contains about 10 to 50 µg HSA/dose, preferably about 20 to 40 µg HSA/dose, more preferably about 25 to 35 µg HSA/dose.

Pharmaceutical compositions of the invention, including vaccines, can be prepared in accordance with methods well-known and routinely practiced in the art (see *e.g.,* Remington: The Science and Practice of Pharmacy, Mack Publishing Co. 20th ed. 2000; and Ingredients of Vaccines - Fact Sheet from the Centers for Disease Control and Prevention, e.g., adjuvants and enhancers as described above to help the vaccine improve its work, preservatives and stabilizers to help the vaccine remain unchanged (e.g., albumin, phenols, glycine)). As used herein, the term "vaccine" refers to an immunogenic composition, e.g. a composition capable of inducing an immune response in a (human) subject against an antigen (e.g. against a SARS-CoV-2 antigen). For instance, the vaccine or composition may be capable of generating neutralizing antibodies against SARS-CoV-2, e.g. as determined in an assay described herein (e.g. a microneutralization assay). In some embodiments, the vaccine or composition is capable of generating antibodies (e.g. IgG) against SARS-CoV-2 S (spike) protein, e.g. as detected by an S-protein IgG ELISA assay as described herein. In some embodiments, the vaccine or composition is capable of generating a T cell response against SARS-CoV-2 proteins or peptides, for instance a T cell response against a SARS-CoV-2 S-protein, membrane (M) protein and/or nucleocapsid (N) protein or peptides derived therefrom, e.g. as detected by an ELISPOT assay as described herein (e.g. based on IFN-γ production). Preferably the vaccine or immunogenic composition generates neutralizing antibodies and a T cell response against SARS-CoV-2. Typically the vaccine or immunogenic composition is capable of inducing a protective effect against a disease caused by the antigen, e.g. a protective effect against SARS-CoV-2 infection (e.g. symptomatic and/or asymptomatic infection) and/or COVID-19 disease).

Pharmaceutical compositions are preferably manufactured under GMP conditions. Typically, a therapeutically or prophylactically effective dose of the inactivated SARS-CoV-2 vaccine preparation is employed in the pharmaceutical composition of the invention. The inactivated SARS-CoV-2 particles are formulated into pharmaceutically acceptable dosage forms by conventional methods known to those of skill in the art. Dosage regimens are adjusted to provide the optimum desired response (*e.g.,* the prophylactic response).

Dosages of the active ingredients in the pharmaceutical compositions of the present invention can be varied so as to obtain an amount of the active ingredient which is effective to achieve the desired pharmaceutical response for a particular subject, composition, and mode of administration, without being toxic to the subject. The selected dosage level depends upon a variety of pharmacokinetic factors, including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the subject being treated, and like factors.

A physician, veterinarian or other trained practitioner, can start dosing of the inactivated SARS-CoV-2 vaccine employed in the pharmaceutical composition at levels lower than that required to achieve the desired therapeutic or prophylactic effect and gradually increase the dosage until the desired effect (*e.g.,* production of anti-SARS-CoV-2 virus antibodies) is achieved. In general, effective doses of the compositions of the present invention, for the prophylactic treatment of groups of people as described herein vary depending upon many different factors, including means of administration, target site, physiological state of the patient, whether the patient is human or an animal, other medications administered, and the titer of anti-SARS-CoV-2 antibodies desired. Dosages need to be titrated to optimize safety and efficacy. In some embodiments, the dosing regimen entails subcutaneous or intramuscular administration of a dose of inactivated SARS-CoV-2 vaccine twice, once at day 0 and once at about day 7. In some embodiments, the dosing regimen entails subcutaneous administration of a dose of inactivated SARS-CoV-2 vaccine twice, once at day 0 and once at about day 14. In some embodiments, the dosing regimen entails subcutaneous administration of a dose of inactivated SARS-CoV-2 vaccine twice, once at day 0 and once at about day 21. In some embodiments, the dosing regimen entails subcutaneous administration of a dose of inactivated SARS-CoV-2 vaccine twice, once at day 0 and once at about day 28. In some embodiments, the inactivated SARS-CoV-2 vaccine is administered to the subject once. In a preferred embodiment, the SARS-CoV-2 vaccine is administered to the subject more than once, preferably two times. In a preferred embodiment, the vaccine is administered on day 0 and day 21. In another preferred embodiment, the vaccine is administered on day 0 and day 28.

In further embodiments, a first (prime) dose of the inactivated SARS-CoV-2 vaccine is administered and a second (boost) dose of the inactivated SARS-CoV-2 vaccine is administered at least 28 days, at least 60 days, at least 70 days, at least 80 days or 90 days after the first dose. Thus in some embodiments, the second dose of the inactivated SARS-CoV-2 vaccine is administered 30 to 120 days or 1 to 4 months (preferably about 3 months) after the first dose.

In other embodiments, the inactivated SARS-CoV-2 vaccine is administered as a booster dose only, e.g. a first (prime) dose of a (different) SARS-CoV-2 vaccine is administered and then a second (boost) dose of the inactivated SARS-CoV-2 vaccine is administered, e.g. at least 7, 14, 28, 60 or 90 days after the first dose. The first (prime) dose of the SARS-CoV-2 vaccine may comprise any other vaccine or immunogenic composition that stimulates an immune response and/or a protective effect in subjects against SARS-CoV-2 virus. For example, the first dose of SARS-CoV-2 vaccine may comprise a recombinant viral vector or an mRNA sequence encoding one or more SARS-CoV-2 proteins and/or fragments thereof, e.g. a SARS-CoV-2 spike (S) protein. Alternatively the first dose of SARS-CoV-2 vaccine may comprise a subunit vaccine, e.g. comprising one or more SARS-CoV-2 proteins and/or fragments thereof, e.g. a SARS-CoV-2 spike (S) protein or fragment thereof.

Also within the scope of the present disclosure are kits for use in prophylactic administration to a subject, for example to prevent or reduce the severity of SARS-CoV-2 infection. Such kits can include one or more containers comprising a composition containing inactivated SARS-CoV-2, such as an inactivated SARS-CoV-2 vaccine. In some embodiments, the kit may further include one or more additional components comprising a second composition, such as a second vaccine, e.g. a second kind of SARS-CoV-2 vaccine that applies a different technology than in the first dose. In some embodiments, the second vaccine is a vaccine for an arbovirus. In some embodiments, the second vaccine is a Japanese encephalitis virus vaccine, a Zika virus vaccine, a Dengue virus vaccine and/or a Chikungunya virus vaccine.

In some embodiments, the kit comprises instructions for use in accordance with any of the methods described herein. The included instructions may comprise a description of administration of the composition containing inactivated SARS-CoV-2 vaccine to prevent, delay the onset of, or reduce the severity of SARS-CoV-2 infection. The kit may further comprise a description of selecting a subject suitable for administration based on identifying whether that subject is at risk for exposure to SARS-CoV-2 or contracting a SARS-CoV-2 infection. In still other embodiments, the instructions comprise a description of administering a composition containing inactivated SARS-CoV-2 vaccine to a subject at risk of exposure to SARS-CoV-2 or contracting SARS-CoV-2 infection.

The instructions relating to the use of the composition containing inactivated SARS-CoV-2 vaccine generally include information as to the dosage, dosing schedule, and route of administration for the intended treatment. The containers may be unit doses, bulk packages (e.g., multi-dose packages) or sub-unit doses. Instructions supplied in the kits of the invention are typically written instructions on a label or package insert (*e.g.,* a paper sheet included in the kit), but machine-readable instructions are also acceptable.

The kits of the present disclosure are in suitable packaging. Suitable packaging includes, but is not limited to, vials, bottles, jars, flexible packaging, and the like. Also contemplated are packages for use in combination with a specific device, such as a syringe or an infusion device. The container may have a sterile access port, for example the container may be a vial having a stopper pierceable by a hypodermic injection needle. At least one active agent in the composition is an inactivated SARS-CoV-2, as described herein.

This invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including", "comprising", or "having", "containing", "involving", and variations thereof herein, is meant to encompass the items listed thereafter and equivalents thereof as well as additional items.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well-known in the art. Generally, nomenclatures used in connection with, and techniques of biochemistry, enzymology, molecular and cellular biology, microbiology, virology, cell or tissue culture, genetics and protein and nucleic chemistry described herein are those well-known and commonly used in the art. The methods and techniques of the present disclosure are generally performed according to conventional methods well-known in the art and as described in various general and more specific references that are cited and discussed throughout the present specification unless otherwise indicated.

The present invention is further illustrated by the following examples, which in no way should be construed as further limiting. The entire contents of all of the references (including literature references, issued patents, published patent applications, and co-pending patent applications) cited throughout this application are hereby expressly incorporated by reference, in particular for the teaching that is referenced hereinabove. However, the citation of any reference is not intended to be an admission that the reference is prior art.

### EXAMPLES

### Example 1. Drug substance production

For the production of SARS-CoV-2, the JEV process platform (Srivastava et al., Vaccine 19 (2001) 4557-4565; US 6,309,650B1) was used as a basis, also taking into account improvements in the process as adapted to Zika virus purification as disclosed in WO2017/109223A1 (which is incorporated herein in its entirety). Briefly, non-infectious SARS-CoV-2 particle aggregates, HCP and other LMW impurities are removed by protamine sulfate precipitation or benzonase treatment and the resulting preparation is optionally further purified by sucrose gradient centrifugation. See **Fig. 1** for an outline of the production process.

The first SARS-CoV-2 isolate from Italy, identified and characterized at the National Institute for Infectious Diseases "Lazzaro Spallanzani" IRCCS, Rome, Italy (Accession No: MT066156), the RNA sequence thereof corresponding to the DNA sequence provided by SEQ ID NO: 9, was used in all Examples disclosed herein. Other novel coronavirus SARS-CoV-2 isolates may also be obtained from the following sources:
1. -EVAg (European Virus Archive), e.g. one of the following strains:
   BetaCoV/France/IDF0372/2020 (Ref-SKU:014V-03890, https://www.european-virus-archive.com/virus/human-2019-ncov-0); 2019-nCoV/Italy-INMI1, (Ref-SKU:008V-03893, SEQ ID NO: 9; https:/Avww.european-virus-archive.com/virus/human-2019-ncov-strain-2019-ncovitaly-inmi1); BetaCoV/Netherlands/01, (Ref-SKU: 010V-03903, https://www.european-virus-archive.com/virus/sars-cov-2-strain-nl2020)
2. -BEI Resources (Biodefense and Emerging Infections Research Resources): e.g. Isolate USA-WA1/2020, NIAID, NIH: SARS-Related Coronavirus 2, NR-52281 (GenBank accession MN985325).
3. -PHE (Public Health England): https://www.gov.uk/government/collections/contacts-public-health-england-regions-local-centres-and-emergency: e.g. isolate of UK B.1.1.7 (UK_MIG457: EVAg Ref-SKU: 004V-04032; SEQ ID NO: 22) or South African B.1.531 (SA_P2: EVAg Ref-SKU: 004V-04071; SEQ ID NO: 18) lineage

***Cell buildup and infection with SARS-CoV-2*** The Vero cells used in the methods described herein were the VERO (WHO) cell line, obtained from the Health Protection Agency general cell collection under catalogue number 88020401, from which a master cell bank was generated. A research viral seed bank (rVSB) of SARS-CoV-2 (strain used 2019-nCoV/Italy-INMI1) was prepared following two rounds of plaque purification on Vero cells and the genomic sequence was checked by sequencing. For production of SARS-CoV-2, Vero cells were grown in Eagle's minimal essential medium (EMEM) containing 10% fetal bovine serum (FBS) and monolayers were infected with SARS-CoV-2 at a multiplicity of infection (moi) of 0.01 plaque forming units (pfu) per cell. Alternatively, the moi may be 0.001 to 1, preferably 0.005 to 0.2, preferably 0.01 plaque forming units (pfu) per cell. After allowing virus adsorption, the cultures were washed 2-4 times with PBS, fed with serum-free Opti-Pro and incubated at 35°C with 5% CO₂ until the virus titer reached a desired level.

***SARS-CoV-2 harvest*** The culture medium was harvested at day 3 and harvests and centrifuged in a standard centrifuge. The crude harvest was treated with 0.2mg/mL Protamine sulfate and filtered (0.8/0.4µm) followed by incubation at RT°C for 15-30 minutes. Host cell DNA and protein reduction, as well as reduction of non-infectious virus aggregates in the concentrated material, was achieved by precipitation with protamine sulfate. The clarified harvest was concentrated by TFF ultrafiltration to remove cell culture medium components and reduce batch volume. Alternatively, the diafiltrated SARS-CoV-2 material may be treated with benzonase.

***Optional primary inactivation*** The SARS-CoV-2 virus was inactivated by treatment with beta-propiolactone directly after removal of virus-containing cell culture medium from Vero cells, in order to render the virus safe to handle at BSL2. Inactivation is possible at any stage in the purification process, however, such as e.g., after centrifugation, before, during or after treatment with protamine sulfate or benzonase or before or after sucrose gradient centrifugation. Inactivation may be carried out by the use of a chemical inactivation agent such as formaldehyde (formalin); enzyme; beta-propiolactone; ethanol; trifluroacetic acid; acetonitrile; bleach; urea; guanidine hydrochloride; tri-n-butyl phosphate; ethylene-imine or a derivative thereof; an organic solvent, optionally Tween, Triton, sodium deoxycholate, or sulfobetaine; or a combination thereof. It is particularly preferred that inactivation is carried out using beta-propiolactone, which preferentially targets viral RNA whilst relatively sparing viral surface proteins and their immunogenic epitopes. Inactivation may also be achieved by pH changes (very high or very low pH), by heat treatment or by irradiation such as gamma irradiation or UV irradiation, particularly UV-C irradiation. The SARS-CoV-2 virus may be optionally inactivated by two separate inactivation steps, such as, e.g. beta-propiolactone treatment and UV-C irradiation.

### EVALUATION OF BPL STARTING CONCENTRATION FOR INACTIVATION OF A HIGHLY RESISTANT MODEL VIRUS PPV

A preliminary study for evaluation of PPV virus inactivation kinetic was conducted to initially support our proposed SARS-CoV-2 BPL inactivation procedure. Porcine Parvovirus (PPV) was selected as a model virus to evaluate the inactivation capability of BPL in aqueous solution because of its high resistance to physico-chemical inactivation. Three starting concentrations of BPL were evaluated, 300 ppm (1/3333), 500 ppm (1/2000) and 700 ppm (1/1429). Virus solution was spiked with BPL at these concentrations and incubated at 5±2°C for 24 hours. Kinetic samples were taken at 0.5, 2, 6, 24h and after the BPL hydrolyzation step and analysed for remaining infectivity. The results are shown in Table A.

| **Table A: Summary of virus titers and reduction factors for PPV inactivation by BPL concentration [ppm]** | | | |
|---|---|---|---|
| | **300** | **500** | **700** |
| titer w/o BPL [TCID50/mL] | 9.97 | 10.04 | 9.98 |
| titer 24h incubation [TCID50/mL] | 6.66 | 4.98 | 4.1 |
| titer after hydrolysis [TCID50/mL] | 5.1 | 2.6 ** | <LOD.* |
| reduction factor after hydrolysis | **4.84 ± 0.39** | **7.43 ± 0.92** | **≥6.89 ± 0.23** |

| | | | |
|---|---|---|---|
| *below limit of detection **Note limit of detection for 500ppm BPL is lower than for 700ppm BPL | | | |

A clear effect of initial BPL concentration on the inactivation effectivity was observed with a reduction between 3.3 and 5.9 log10 after 24h incubation at 5±2°C (before hydrolysis). The following hydrolysis step further reduced the titers by on average addition 1.7 log 10 while the hold control titers remained constant throughout the whole procedure. This indicated that for highly resistant virus contaminations the hydrolysis step might serve as an additional inactivation step.

With overall reduction factors of 4.84 (300 ppm), 7.43 (500 ppm) and below the limit of detection (700 ppm) the applied BPL treatment was considered effective for the inactivation of Parvoviridae at concentrations > 300ppm.

Therefore, we decided to select 500ppm for SARS-CoV-2 virus inactivation in all further studies. SARS-CoV-2 virus inactivation by BPL

Based on existing data on the inactivation of model viruses by BPL (see section above on PPV inactivation) a BPL concentration of 500 ppm (1/2000) was selected for the inactivation of SARS-CoV-2 virus harvest material. As the stability of BPL in solutions is highly temperature dependent an incubation temperature of 5±3°C and an incubation time of 24 hours were selected to ensure enough BPL present throughout the whole inactivation. After addition and mixing of BPL to the concentrated harvest, the inactivation solution is transferred to a fresh container where the inactivation takes place under controlled conditions. This transfer excludes the possibility of virus particles in potential dead-spots during initial mixing not being in contact with BPL.

To stabilize the pH of the inactivated viral solution during hydrolysis of the BPL, protamine sulfate (PS) treated concentrated harvest pre-cooled to 5±3°C is supplemented with 25 mM HEPES pH 7.4.

To reduce remaining BPL after the inactivation the solution is warmed to temperatures above 32°C for a total time of 2.5 hours ± 0.5 hours in a temperature-controlled incubator set to 37±2°C. The total time of the hydrolyzation step for the current process volume of about 1L was between 5 hours 15 minutes and 6 hours 15 minutes including the warming to and the incubation above 32°C.

After completion of the hydrolysis, the inactivated viral solution (IVS) was immediately cooled down to 5±3°C in a temperature-controlled fridge and stored there until inactivation was confirmed by large volume plaque assay and serial passaging assay which currently requires 18 days in total.

Recovery of virus particles throughout the inactivation process was monitored by size-exclusion chromatography.

Initial studies at lab-scale from 15 mL up to 1000mL indicated a very fast inactivation kinetic for SARS-CoV-2 where virus titers of up to 8 log 10 pfu/mL were reduced below detectable levels within 2 hours after BPL addition. These results were confirmed for GMP production runs at a final inactivation volume of approximately 1L.

Taken together with the inactivation data for model viruses the applied BPL treatment can be considered efficient and includes a significant safety margin for inactivation of SARS-CoV-2 concentrated harvest material.

In a further preferred embodiment, the inactivation step(s) are particularly gentle, in order to preserve surface antigen integrity, especially integrity of the S protein. In one embodiment, the gentle inactivation method comprises contacting a liquid composition comprising SARS-CoV-2 particles with a chemical viral inactivating agent (such as e.g. any of the chemical inactivation agents as listed above or a combination thereof, preferably beta-propiolactone) in a container, mixing the chemical viral inactivating agent and the liquid composition comprising SARS-CoV-2 particles under conditions of laminar flow but not turbulent flow, and incubating the chemical viral inactivating agent and the liquid composition comprising SARS-CoV-2 particles for a time sufficient to inactivate the viruses. The gentle inactivation step is optionally performed in a flexible bioreactor bag. The gentle inactivation step preferably comprises five or less container inversions during the period of inactivation. Preferably, the mixing of the chemical viral inactivating agent and the composition comprising SARS-CoV-2 particles comprises subjecting the container to rocking, rotation, orbital shaking, or oscillation for not more than 10 minutes at not more than 10 rpm during the period of incubation.

***Purification of SARS-CoV-2*** Optionally, the material was immediately further processed by batch adsorption (also known herein as batch chromatography) with Capto™ Core 700 (CC700) or CC400 chromatography media at a final concentration of ∼1% CC700 or CC400. The material was incubated at 4°C for 15 minutes under constant agitation using a magnetic stirrer. After incubation, if used, the CC700 or CC400 solid matter was allowed to settle by gravity for 10 minutes and the SARS-CoV-2 material is removed from the top of the solution in order to avoid blockage of the filter by CaptoCore particles. Any remaining CaptoCore particles and DNA precipitate were then removed from the solution by filtration using a 0.2 µm Mini Kleenpak EKV filter capsule (Pall). The filtered harvest material was adjusted to a final concentration of 25 mM Tris pH 7.5 and 10% sucrose (w/w) using stock solutions of both components. This allowed for freezing the concentrated harvest at <-65°C if required.

The resulting filtrate was further processed by sucrose density gradient centrifugation (also known herein as batch centrifugation) for final concentration and polishing of the SARS-CoV-2 material. The concentrated protamine sulfate (PS) or benzonase treated (preferred is PS treated) harvest is loaded on top of a solution consisting of three layers of sucrose with different densities. The volumes of individual layers for a centrifugation in 100 mL bottle scale are shown in **Table 1.**

**Table 1: Volumes for sucrose density centrifugation.**

| **Sucrose solution (w/w)** | **Volume (mL)** |
|---|---|
| PS-treated SARS-CoV-2 harvest (10% sucrose) | 40 |
| 15% sucrose | 15 |
| 35% sucrose | 15 |
| 50% sucrose | 20 |
| **Total volume** | 90 |

The sucrose gradient bottles are prepared by stratifying the individual sucrose layers by pumping the solutions into the bottom of the bottles, starting with the SARS-CoV-2 material with the lowest sucrose density (10% sucrose (w/w)), followed by the other sucrose solutions in ascending order. The described setup is shown in **Figure 3****.** The prepared SG bottles are transferred into a rotor pre-cooled to 4°C and centrifuged at ∼11,000 RCF max at 4 °C for at least 20 hours, without brake/deceleration.

After centrifugation, harvest of serial 2 mL fractions of the sucrose gradient was performed from the bottom up with a peristaltic pump. The fractions were immediately tested by SDS-PAGE / silver staining to identify virus-containing fractions with sufficiently high purity. Thus, identified fractions were pooled and further processed. The purified SARS-CoV-2 was stored at <-65°C or immediately formulated.

***Formulation of SARS-CoV-2 with adjuvant*** CpG, and optionally alum, were added to the SARS-CoV-2 composition and/or prepared in a separate vial for bedside mixing.

*SARS-CoV-2 ELISA Assay* Inactivated SARS-CoV-2 antigen content (i.e. content of S1 as the major antigenic protein) in preparations described herein was determined (quantified) by ELISA. The SARS-CoV-2 ELISA used herein is a four-layer immuno-enzymatic assay with a SARS-CoV-2 spike antibody (AM001414; coating antibody) immobilized on a microtiter plate to which the SARS-CoV-2 sample was added. On binding of the antigen to the coating antibody, the plate was further treated with primary antibody (i.e. AbFlex®SARS-COV-2 spike antibody (rAb) (AM002414)). This was followed by addition of the secondary antibody, which is an enzyme linked conjugate antibody (i.e. Goat anti-Mouse IgG HRP Conjugate). The plates were washed between various steps using a mild detergent solution (PBS-T) to remove any unbound proteins or antibodies. The plate was developed by addition of a tetramethyl benzidine (TMB) substrate. The hydrolyzed TMB forms a stable colored conjugate that is directly proportional to the concentration of antigen content in the sample. The antigen quantification was carried out by spectrophotometric detection at λ450nm (λ630nm reference) using the standard curve generated in an automated plate reader as a reference. Standards were prepared starting with a 20 antigen units (AU)/mL spike trimer working solution neat, which was further serially diluted 1:2 for the following standard concentrations: 20 AU/mL, 10 AU/mL, 5 AU/mL, 2.5 AU/mL, 1.25 AU/mL, 0.625 AU/mL, 0.3125 AU/mL and 0.1263 AU/mL. Each dilution was tested in duplicate per plate. An "antigen unit" of the spike trimer standard, according to the supplier (R&D Systems), corresponds to its binding ability in a functional ELISA with Recombinant Human ACE-2 His-tag.

### Reference Standards and Antibodies:

Coating Antibody: SARS-CoV-2 Spike Antibody (AM001414)
Spike Trimer (S1+S2), His-tag (SARS-CoV-2) (e.g. BPS Lot# 200826; Cat#100728)
SARS-CoV-2 QC (e.g. RSQC240920AGR)
Primary Detection Antibody AbFlex® SARS-CoV-2 Spike Antibody (rAb) (AM002414)
Secondary Detection Antibody Goat anti-Mouse IgG HRP Conjugate
Coating buffer: Carbonate buffer
ELISA wash buffer: PBS + 0.05% Tween-20 (PBS-T).
Sample dilution buffer: PBS-T + 1% BSA.

Production process delivered high density and intact spike proteins (see **Figure 7****).** Estimated were about 1 to 1.5 x 10⁷ viral particles per AU. Inactivation process by beta-propiolactone provided for a fast inactivation kinetic and no detectable chemical modification of the S-protein. Key parameters and relevant process related impurities were similar to the commercial IXIARO® production process (see **Table 1b**). SARS-CoV-2 drug substance according to the invention was highly pure (>95%) according to SDS-PAGE (silver stain, reduced) and free from aggregates (monomer virus (>95%) according to SE-HPLC (see **Figure 8****).**

Further confirmatory studies aimed at characterizing modifications of S-protein following beta-propiolactone-inactivated SARS-CoV2 are carried out by mass spectrometric analysis of tryptic digests of the S-protein. The modification of amino acids in important epitopes is minimal. Initial alignment of receptor binding domains (RBD) within the S protein and hACE2 interfaces and epitopes of several known (cross)-neutralizing antibodies (SARS-CoV and SARS-CoV-2) have shown no amino acids within these epitopes with potential high conversion and only few with potential lower conversion rates.

**Table 1b. Comparison of key parameters and relevant process related impurities of the SARS-CoV-2 drug substance and IXIARO® drug substance.**

| | **SARS-CoV-2** | **IXIARO** |
|---|---|---|
| Viral yield at harvest (log₁₀ PFU/mL) | > 7.8 | > 7.3 |
| Residual host cell protein (HCP) (ng/mL) | <150 | <100 |
| Residual host cell DNA (hcDNA) (pg/mL) (LOQ 40 pg/mL) | < LOQ | < LOQ |
| Virus Monomer by SEC-MALLS (%) | >95 | >95 |
| Residual Protamine sulfate* (µg/mL) (LOQ 2 µg/mL) | < LOQ | < LOQ |
| Endotoxins (EU/mL) | < 0.05 | < 0.05 |
| Residual Inactivation reagent | < LOQ | < LOQ |
| | (LOQ 1ppm, β-Propiolactone) | (LOQ 50ppm, Formalin) |

### Example 2. In vitro and in vivo assessment of immunogenicity and protective capacity of inactivated CoV-2 virus and adjuvanting/dose-sparing effects of CpG

*Immunogenicity* Prior to immunization, experimental groups of 10 BALB/c mice were bled and pre-immune sera are prepared. The mice are administered a dose titration of inactivated SARS-CoV-2 formulated with alum or alum and CpG 1018 subcutaneously (see **Table 2).** At two different intervals after immunization (see below), blood was collected and immune sera prepared, spleens were collected at the final bleed. All animal experiments were carried out in accordance with Austrian law (BGB1 Nr. 501/1989) and approved by "Magistratsabteilung 58". Sera were assessed for total IgG and subclasses (IgG1/IgG2a) by ELISA and neutralizing antibodies by PRNT. Th1/Th2 responses were further assessed by IFN-γ ELISpot and intracellular cytokine staining (CD4⁺/CD8⁺).
- Schedule 1: Immunizations Day 0/Day 7, interim bleed Day 14, final bleed and spleen harvest Day 28
- Schedule 2: Immunizations Day 0/Day 21, interim bleeds Day 14/Day 28 and final bleed and spleen harvest Day 35

**Table 2. Design of dosing experiments, 10 mice/group: 3 dosage groups, first set of experiments (A) with higher dosages ranging from 0.1-1 mAU, second set of experiments (B) with lower dosages ranging from 0.05-0.5 mAU. (Total viral protein/mAU was estimated to be approximately 2 µg/ml).**

| | **Adjuvants (mice/group)** | |
|---|---|---|
| **Inactivated SARS-CoV-2 dosages (A/B)** | **Aluminium hydroxide (50 µg)** | **Aluminium hydroxide (50µg)/CpG 1018 (10 µg)** |
| **(1/0.5 mAU*)** | 10 | 10 |
| **(0.5/0.2 mAU)** | 10 | 10 |
| **(0.1/0.05 mAU)** | 10 | 10 |
| **Placebo** | 10 | 10 |

| | | |
|---|---|---|
| * mAU - SE-HPLC peak area per dose equivalent (recorded as milli-absorption units x minutes; mAU) | | |

***Plaque reduction neutralization test (PRNT)*** Each well of a twelve-well tissue culture plate was seeded with Vero cells and incubated 35°C with 5% CO₂ for three days. Serial dilutions from pools of heat-inactivated sera from each treatment group are tested. Each serum preparation was incubated with approximately 50-80 pfu of SARS-CoV-2 at 35°C with 5% CO₂ for 1 hour. The cell culture medium was aspirated from the Vero cells and the SARS-CoV-2 /serum mixtures are added to each well. The plates were gently rocked and then incubated for 2 hours at 35°C with 5% CO₂. To each well, 1 mL of a 2% methylcellulose solution containing EMEM and nutrients were added, and the plates were further incubated for 4 days at 35°C with 5% CO₂. The cells were then stained for 1 hour with crystal violet/5% formaldehyde and washed 3 times with deionized water. The plates were air dried and the numbers of plaques in each well manually counted. Alternatively, other methods, such as e.g. TCID₅₀ may be applied.

**Table 3. Design of schedule and longevity experiments. Immunization schedule as for Table 2, but in addition; interim bleeds 2, 6, 10, 14, 18 and 22 weeks after second immunization; end-bleed 26 weeks after second immunization.**

| | **Adjuvants (mice/group)** | |
|---|---|---|
| | **Aluminium hydroxide (50 µg/ml)** | **Aluminium hydroxide (50 µg/ml)/ CpG 1018 (10 µg/ml)** |
| **Vaccine (high dose), s.c** | 20 | 20 |
| **Placebo, s.c.** | 10 | 10 |

***Protective capacity*** The protective capacity of inactivated SARS-CoV-2 is assessed using a SARS-susceptible transgenic mouse expressing a humanized ACE2 protein (Jackson Laboratory) (Tseng, C.-T.K. et al., Severe Acute Respiratory Syndrome Coronavirus Infection of Mice Transgenic for the Human Angiotensin-Converting Enzyme 2 Virus Receptor (2007) J of Virol 81:1162-1173) or a NHP model developed for SARS-CoV-2 infection. Groups of animals are immunized subcutaneously (s.c.) with different dosages of inactivated SARS-CoV-2 with or without adjuvant or PBS as a negative control. Three weeks after the last dose, animals are challenged with SARS-CoV-2 and monitored for disease progression and survival. In addition, serum samples are taken in order to determine the neutralizing antibody titers induced by vaccination in a PRNT assay.

**Table 3A. Design of dosing experiment 4743 using SARS-CoV-2 ELISA-determined dosages.**

| | |
|---|---|
| Material | SGP rVSB |
| Buffer | PBS |
| AU/mouse | 3.0 |
| | 1.2 |
| | 0.3 |
| Al³⁺/mouse | 17 µg |
| CpG 1018/mouse | 10 µg |
| Immunization | D0/D21 |
| Bleeds | D28/D35 |

*Experiment 4743 Protocol* Female BALB/c mice (10 mice/group) were immunized two times s.c. (100 µL) on days 0 and 21 with doses and adjuvants as outlined in **Table 3A.** The readouts from the experiment were total IgG and subclasses (IgG1/IgG2a) and virus neutralization (PRNT). Vaccine formulation used in experiment 4743: purified inactivated SARS-CoV-2 produced from a research virus seed bank (rVSB) formulated in PBS with 17 µg Al³⁺ (alum)/dose and 10 µg/dose CpG 1018.

**Antibody response to SARS-CoV-2 proteins** The immune responses in mice for the different doses and adjuvant formulations were assessed with a total IgG ELISA **(****Figure 4****).** Plates were coated with either the S1 part **(****Figure 4A****)** or receptor binding domain (RBD) **(****Figure 4B****)** of the spike glycoprotein or the nucleoprotein **(****Figure 4C****).** Sera taken on days 28 and 35 were analyzed. Plates were coated with 2 µg/mL antigen (S1, RBD and N protein) and mouse sera were tested at a starting dilution of 1:50 in 4-fold dilutions. For detection a secondary monoclonal antibody (HRP-conjugated goat anti-mouse IgG) was used and developed with ABTS and read at absorbance 405 nm. Wells were washed with PBS-T between each step. Endpoint titers were determined with a cut-off set to 3-fold the blank.

**IgG subclass immune response** Plates were coated with the S1 part (Figure 4A) of spike glycoprotein and sera taken on day 35 were analyzed. Subclass specific secondary antibodies (IgG1 and IgG2a) conjugated with HRP were used for detection. As standard curves (4-paramater regression) for determination of the amount of the different IgG subclasses (IgG1 and IgG2a), monoclonal antibodies with different subclasses were used (IgG1 mAb clone 43 and IgG2a mAb clone CR3022). Bound HRP-conjugated secondary mAbs were developed with ABTS and read at absorbance 405 nm. Wells were washed with PBS-T between each step. The relative IgG subclass concentration is shown in **Figure 5A** and the ratio of IgG2a/IgG1 in **Figure 5B****.**

***Observations from Experiment 4743*** Inactivated SARS-CoV-2 formulated with alum and CpG 1018 induced antibodies in mice against SARS-CoV-2 detected by ELISA measuring antibodies to S1 protein, receptor binding domain (RBD) and nucleocapsid protein (N) **(****Fig. 4A-C****).** An increase in immunogenicity was observed between bleeds on day 28 and day 35. A significant increase in immunogenicity, i.e., a dose-sparing effect, was seen for S1 and RBD in the presence of CpG 1018. In groups receiving the lowest doses (0.3 AU), a smaller increase not significantly above the placebo was seen for nucleoprotein, S1 and RBD ELISA titers.

The addition of CpG 1018 to alum-adjuvanted inactivated SARS-CoV-2 promoted a substantial immune response shift towards Th1 (IgG2a) compared with Th2 (IgG1) as demonstrated by quantification of IgG subclasses by S1 ELISA. The amounts of IgG2a and IgG1 measured and the ratio of IgG2a:IgG1 in the treatment groups are shown in **Figs. 5A** and **5B****,** respectively. A significant shift in the immune response toward Th1 (IgG2a) in the presence of CpG 1018 was observed. In the presence of CpG 1018 a stronger induction of IgG2a than IgG1 was observed. In the alum only groups a stronger induction of IgG1 than IgG2a is observed.

Finally, the neutralizing response in the presence of alum/CpG in the group immunized with the highest SARS-CoV-2 dose was in the range of plasma from convalescent donors positive for SARS-CoV-2 (NIBSC 20/162; three pooled donors)(see **Fig. 6****).**

Further immunization experiments are carried out in mice using GMP material with low doses (3, 1.2 and 0.3 AU) as a bridge between research and GMP material, as well as analyses of GMP material in mice with human doses (540, 10 and 3 AU).

Additionally, a challenge study is carried out in immunized non-human primates (NHP) (see **Figure 9****)** and a passive transfer study is carried out in hamsters using sera from human subjects vaccinated with the SARS-CoV-2 vaccine candidate of the invention (see **Table 1c**).

**Table 1c. Passive transfer study of the SARS-CoV-2 vaccine candidate of the invention in hamsters.**

| | |
|---|---|
| Study objective: Proof of concept that the SARS-CoV-2 vaccine candidate of the invention induces neutralizing antibodies providing protection against SARS-CoV-2 challenge in an animal model | |
| Study design: Syrian hamsters receive sera from vaccinated subjects of the SARS-CoV-2 vaccine candidate of the invention Phase 1/2 study (see **Example 4** below). | |
| Hamsters are then challenged intranasally with SARS-CoV-2, Victoria/1/2020 | |
| A 10 day follow up includes: | |
| | Clinical observations and body weights recorded daily |
| | Viral shedding/viral loads determined via RT-qPCR |
| | Circulating antibodies prior to challenge (neutralization) |
| | Tissues (lung and upper respiratory tract) taken at necropsy for determination of viral load and for histology |

### Example 3. Testing of SARS-CoV-2 vaccine for antibody-dependent enhancement (ADE) of disease and immunopathology

Although the mechanism is poorly understood, antibodies produced in response to a previous coronavirus infection or vaccination can increase the risk for 1) immunopathology and/or 2) antibody-dependent enhancement of disease during subsequent coronavirus infection. As such, any stimulation of antibodies to SARS-CoV-2 presents a hypothetical risk. In this regard, several approaches are undertaken to ensure safety of the current vaccine.

*In vitro antibody-dependent enhancement assays* Immune sera from inactivated SARS-CoV-2-vaccinated mice are assessed for hallmarks of enhanced disease *in vitro.* Such assays are described by e.g. Wang, S.-F., et al. 2014 (Antibody-dependent SARS coronavirus infection is mediated by antibodies against spike proteins (2014) BBRC 451:208-214). Briefly, susceptible cell types or cell lines are incubated with immune sera and subsequently infected with SARS-CoV-2. Cells are assessed for cytopathic effect and production of inflammatory markers.

*Mouse model of immunopathology* The risk of vaccine-enhanced immunopathology on challenge is assessed in a BALB/c mouse model as described by Tseng C.T. et al. (Immunization with SARS Coronavirus Vaccines Leads to Pulmonary Immunopathology on Challenge with the SARS Virus (2012) PLoS ONE 7(4):e35421). Briefly, the mice are immunized twice at two-week intervals with inactivated SARS-CoV-2 formulated as described herein followed by challenge with SARS-CoV-2. SARS-CoV-2 titers and immune cell infiltration of the lung are tested.

*Non-human primate model of ADE* The risk of ADE development in non-human primates is assessed as described by Luo F, et al. (Evaluation of Antibody-Dependent Enhancement of SARS-CoV Infection in Rhesus Macaques Immunized with an Inactivated SARS-CoV Vaccine (2018) Virologica Sinica 33:201-204). Briefly, NHPs are immunized with inactivated SARS-CoV-2, followed by SARS-CoV-2 challenge and evaluation of symptoms and disease pathology.

### Example 4. Clinical Phase 1 study

*Formulation of inactivated SARS-CoV-2 for Phase 1 trial* The objective of the Phase 1 trial is to assess the safety of the vaccine, along with immunogenicity, and to determine an optimal dose and adjuvant in healthy human subjects. As such, three antigen doses were tested in clinical phase 1: High, Medium and Low, which are chosen to have a distance between each dose of approximately 3-fold and a span covering about a 10-fold difference between the high and low doses (e.g., 0.5, 2 and 5 µg/dose or 3, 10 and 40 AU/dose). About 150 healthy volunteers were enrolled (153), 50 subjects per dose group aged 18 to 55 (see **Figure 10****).** The dose range is selected in part to indicate any potential dose-sparing effect of CpG. As previously reported in the literature, a 2-3 fold reduction in required antigen may be expected in the presence of CpG. The three dosages are tested without antigen and with 1 mg CpG plus 0.5 mg alum (Al³⁺). Currently 1^{st} vaccination completed, 2^{nd} vaccination ongoing. Two DSMB meetings have been performed with first meeting following availability of initial safety data from sentinel subjects (N=15), there no safety concerns identified, thus blinded randomized study phase initiated. 2^{nd} DSMB for interim safety review upon completion of first vaccinations (N=153), there no safety concerns identified. First data readout (day 36 safety and immunogenicity) expected by April 2021 as basis for dose decision for phase 3 (see **Example 5).**

The SARS-CoV-2 virus as purified herein has a high purity of >90% as assessed by SDS-PAGE, SE-HPLC and/or SARS-CoV-2 ELISA **(****Figure 8****).** Furthermore, preliminary studies have indicated that the incidence of genetic heterogeneities during passage of the virus is low and no particular individual mutations stand out (data not shown).

To arrive at a dose range, the SARS-CoV-2 virus was compared with JEV, specifically assessing SE-HPLC peak area per dose equivalent (recorded as milli-absorption units x minutes; mAU), the total amount of inactivated viral particles per dose and the total viral surface equivalent per dose (see **Table 4).** This assessment was based on the assumption of a similar surface antigen density between S (spike; SARS-CoV-2) and E (envelope; JEV) proteins. Total protein was determined by µBCA assay **(Table 4).** Although the assay was variable, a correspondence of 1 mAU to ∼2 µg total protein per mL was observed. Another determination using an optimized SARS-CoV-2 S-protein ELISA, as outlined in Example 1, was also performed.

**Table 4. Comparison of JEV and SARS-CoV-2 quantification parameters and an approximation of the total protein in Low, Medium and High SARS-CoV-2 dosage groups for Phase 1 clinical trials.**

| **Dose** | **SE-HPLC peak area equivalent CoV/JEV** | **Ratio total particle equivalent CoV/JEV** | **Ratio surface antigen equivalent CoV/JEV** | **Estimated total protein/dose in µg (µBCA assay)** |
|---|---|---|---|---|
| Low | 0.25 | 0.015 | 0.070 | 0.5 |
| Medium | 1 | 0.058 | 0.288 | 2 |
| High | 2.5 | 0.145 | 0.719 | 5 |

As SARS-CoV-2 virus particles (∼92nm diameter) are much larger than Flavivirus particles (∼40nm), corresponding to an approximately 5-fold greater virus surface area per particle, an equivalently higher antigen content is expected. Furthermore, other inactivated virus vaccine preparations, including JEV (IXIARO), TBE (Encepur) and HepA (VAQTA) reported antigen dose in the low µg to ng protein range. As these viruses are all formalin inactivated, the BPL-inactivated SARS-CoV-2 virus of the current invention has better preserved surface antigen proteins, i.e., a better quality antigen, and requires a lower total protein dose.

For entry into the clinic a further antigen determination assay (SARS-CoV-2 ELISA assay as described in Example 1) was developed and the doses of the vaccine formulations for entry into Phase 1 trials were determined using this assay. The Phase 1 treatment groups are set forth in **Table 5.**

### Formulation of SARS-CoV-2 vaccine for phase 1 trial (0.5 mL/dose):

- Antigen (inactivated SARS-CoV-2) target doses (one dose = 0.5 mL):
   Low: about 3 AU/0.5 mL *
   Medium: about 10 AU/0.5 mL) *
   High: about 40 AU/0.5 mL *
   *doses determined by the SARS-CoV-2 ELISA assay as described in Example 1
- Aluminium hydroxide (Al³⁺): 0.5 mg/dose (1 mg/mL)
- CpG 1018: 1 mg/dose (2 mg/mL)
- Recombinant Human Serum Albumin (rHSA): ∼25 µg/dose (∼50 µg/mL)
- Buffer: Phosphate buffered saline (PBS)

In some cases, vaccinated subjects are challenged with an infectious dose of live SARS-CoV-2 virus (Asian and/or European lineage).

**Table 5. Treatment groups for Phase 1 testing of inactivated SARS-CoV-2 vaccine (low, medium and high doses as set forth above). Two doses are administered (day 0 and day 21).**

| **Group** | **Antigen** | **Aluminium hydroxide** | **CpG 1018** |
|---|---|---|---|
| 1 | Low | - | - |
| 2 | Med | - | - |
| 3 | High | - | - |
| 4 | Low | + | + |
| 5 | Med | + | + |
| 6 | High | + | + |

### Example 5. Clinical Phase 3 study

### Neutralizing antibodies are emerging as a robust clinical parameter:

- Emerging clinical evidence suggesting neutralizing antibodies are associated with protection against COVID-19
- Protection from moderate to severe COVID-19 disease has been shown to coincide with emergence of neutralizing antibody levels against SARS-CoV-2 (field efficacy study with the mRNA vaccine of Pfizer/Biontech)
- Monoclonal antibody (Bamlanivimab) was shown to prevent subjects from developing symptomatic COVID-19 by 80% in a phase 3 trial conducted by the National Institutes of Health's National Institute of Allergy and Infectious Diseases (NIAID),
- Monoclonal antibody administration in hamsters or non-human primates have been shown to protect from disease induced by COVID-19 in these animal models.
- Adoptive transfer of purified IgG from convalescent rhesus macaques (Macaca mulatta) has been shown to protect naive recipient macaques against challenge with SARS-CoV-2 in a dose-dependent fashion

### Plan for phase 3 Non-inferiority study (Figure 11)

- Randomized, observer-blind, controlled, non-inferiority study to compare the immunogenicity of inactivated SARS-CoV-2 vaccine of the invention*** to [Active Comparator, e.g. AZD1222 or BNT162b2, to be determined]
- Dosage: two i.m. vaccinations within 1 month; Dose level selected based on Phase 1/2
- Study Population: 4000* healthy volunteers aged 18 years and above, randomized 3:1 to receive inactivated SARS-CoV-2 vaccine of the invention or [Active Comparator] to establish safety database for initial licensure
- Immunogenicity sub-set of approximately 600 participants** who have tested sero-negative for SARS-CoV-2 at screening; 1:1 distributed to inactivated SARS-CoV-2 vaccine of the invention or [Active Comparator]
- Primary Endpoint:
   ∘ Non-inferiority of immune response 4 weeks after completion of a 2-dose Immunization Schedule, as determined by the GMT of SARS-CoV-2-specific neutralizing antibodies or seroconversion rate
   * A sample size of 3000 subjects vaccinated with the inactivated SARS-CoV-2 vaccine of the invention will allow for the detection of at least 1 rare event (incidence rate 1/1000) with a probability of 94% in this study.
   ** Sample size to be confirmed upon availability of phase 1/2 immunogenicity results
   ***See **Figure 12****.** Phase clinical trial material comparable to clinical study material of phase 1 (see

### Example 1)

### Example 6. Testing of Sera of vaccinated organism with a neutralization assay

Sera of vaccinated mice, hamsters, non-human primates or humans can be tested in neutralization assays such as e.g. described in "Szurgot, I., Hanke, L., Sheward, D.J. et al. DNA-launched RNA replicon vaccines induce potent anti-SARS-CoV-2 immune responses in mice. Sci Rep 11, 3125 (2021). https://doi.ors/10.1038/s41598-021-82498-5".

The read out of the test gives an indication how well sera of vaccinated subjects can neutralize new variants and thus guides in the design of the vaccine.

### Example 7. Liquid chromatography with tandem mass spectrometry (LC-MSMS) analysis of inactivated SARS-CoV-2

### Methodology:

Two samples of the BPL-inactivated SARS-CoV-2 particles were separated using SDS-polyacrylamide gel electrophoresis and the bands were visualized by silver staining. The bands were cut and subjected to in-gel digestion with trypsin and the resulting peptides analysed with nano-liquid chromatography coupled to a high-resolution accurate mass spectrometer. Peptides were identified from raw spectra using the MaxQuant software package and the UniProt reference databases for SARS-CoV-2 and Chlorocebus sabaeus. To account for modifications the data were re-searched specifically for β-propiolactone modifications, and the obtained results were confirmed with a second independent search algorithm (Sequest in Proteome Discoverer suite). Additionally, data were searched with the FragPipe package to account for further unknown MS-detectable modifications.

### Results:

### Protein identification:

The bands could be clearly attributed to the three main viral proteins (Spike-protein, Membrane-protein, Nucleoprotein) as well as to background proteins from the host system (see Figure 10). Traces of SARS-CoV-2 ORF9b and the replicase polyprotein could also be detected, but these proteins were probably not well resolved on the gel due to their size. The separation pattern on the gel was very similar for both samples with the exception of a host protein band (band 2.3), a slightly different S-protein pattern (bands 2.10-2.13), and an expected strong band of serum albumin in one of the samples (sample 2). Additionally, a number of typical lab contaminants of human origin (e.g. keratins) were detected in the background of both samples. The processing of the Spike-protein (from full length to S1, S2, and S2') is difficult to resolve with the applied methodology but is most likely represented by the pattern in bands 9-13 in both samples.

### Modification analysis:

Based on a publication by Uittenbogaard et al. (Reactions of β-Propiolactone with Nucleobase Analogues, Nucleosides, and Peptides, Protein Structure and Folding| Volume 286, ISSUE 42, P36198-36214, October 21, 2011), it was expected to find β-propiolactone (BPL) modifications on cysteine, methionine, and histidine. Uittenbogaard et al. studied amino acids which are subject to modification by beta-propiolactone, along with the type of modification, e.g., acylation, alkylation. They have shown that BPL can react with up to 9 different amino acids (C,H,M,D,E,Y,K,E,S) depending on actual pH. In their studies higher conversions within the relevant pH range 7 to 9 were observed for Cysteine (>95%), Histidine (15-25%) and Methionine (36%) residues. The conversion rates for Aspartic Acid, Glutamic acid and Tyrosine were much lower in the range of approximately 3-15%. It was shown that disulfide groups in Cystine residues do not react.

In BPL-inactivated SARS-CoV-2 particles, BPL modifications could be detected (mainly in the form of +72 Da) but at a low abundance. Out of 2894 (sample 1) and 3086 (sample 2) identified spectra for SARS-CoV-2 proteins only 73 and 110, respectively, carried a BPL modification, which translates to 2.5 to 3.65 % (see Table 6). This was also confirmed by the open modification search using FragPipe, which attributed a similarly low fraction of spectra to mass differences matching the BPL-modification.

**Table 6. Number of identified SARS-CoV-2 peptide spectra**

| **Sample** | **Total spectra** | **BPL spectra** | **% modified** |
|---|---|---|---|
| Sample 1 | 2894 | 73 | 2.5% |
| Sample 2 | 3086 | 110 | 3.6% |

Spectra of all BPL-modified peptides reported for SARS-CoV-2 proteins were inspected manually of which 6 to 8 sites were confirmed for sample 1 and 2, respectively (see Table 6). For all of these validated sites also the unmodified peptides were identified suggesting that the modification with BPL never reached 100%. We estimated the degree of modification on a particular site (the so-called site occupancy) as the ratio of modified to unmodified peptide for the same modification site normalized to the protein abundance for each band. We then selected the maximum occupancy for each site as a conservative measure of the degree of site modification. As shown in Table 7 the occupancy was in general rather low for the sites identified, in agreement with the total number of identified spectra. The only exception, M234 of the nucleoprotein, has to be interpreted carefully, as that particular peptide sequence has problematic features which likely make the estimation for this particular peptide less accurate and reliable as compared to the other sites.

**Table 7. BPL-modified sites identified and their occupancy**

| **Protein** | **Site Position** | **% occupancy sample 1** | **% occupancy sample 2** |
|---|---|---|---|
| Spike-protein | H207 | <0.1% | 16% |
| Spike-protein | H245 | 1% | 3% |
| Spike-protein | C379 | <0.1% | n.d. |
| Spike-protein | M1029 | <0.1% | <0.1% |
| Spike-Protein | C1032 | <0.1% | n.d. |
| Membrane protein | H154 | <0.1% | <0.1% |
| Membrane protein | H155 | 1% | 1% |
| Membrane protein | C159 | n.d. | <0.1% |
| Membrane protein | H210 | 5% | 6% |
| Nucleoprotein | M234 | <0.1% | 88%* |

| | | | |
|---|---|---|---|
| n.q. = not quantified; n.d. = not detected *quantification uncertain, due to missed cleavages and oxidation | | | |

Apart from the expected modifications the FragPipe search revealed two other modifications (most likely acetaldehyde and acetylation) to occur in around 10% of the spectra. These modifications represent most likely artifacts introduced during gel staining and sample preparation, as they also occur on contaminant proteins.

### Summary

Based on the results described above it is concluded that the main components in these samples corresponds to SARS-CoV2 proteins. The BPL modifications were detectable but appeared to be low, i.e. around 3% on whole SARS-CoV-2 proteome level (i.e. all SARS-CoV-2 proteins identified). Only 5 amino acids of the S-protein were found to be modified and this was also only detected for a minority of the analysed S-protein (e.g. around 16% for the Spike-protein at the H207 amino acids, i.e. the probability to have a modification at H207 was around 16%). The two samples differ only slightly with respect to some background proteins and in their degree of modification, with sample 1 showing slightly lower levels of BPL-modification. Please note that only about 30 to 40% of the amino acids of the Spike protein could be tested.

### Conclusion:

This data supports the view that the mild inactivation approach of the invention minimizes the modifications within the S-protein and thus the native surface of the S-protein is largely preserved. In comparison, determination of modifications by BPL inactivation of flu samples were more frequent, i.e. 83 sites on HA and 43 sites on NA for one sample flu vaccine (NIBRG-121xp) and 99 sites on HA and 39 sites on NA for another sample (NYMC-X181A) were modified, wherein HA and NA are the two major membrane glycoproteins, i.e. the primary immunogens for flu (She Yi-Min et al., Surface modifications of influenza proteins upon virus inactivation by beta-propiolactone; Proteomics 2013, 13, 3537-3547, DOI 10.1002/pmic.201300096). Thus BPL inactivation of influenza virus can lead to numerous protein modifications including some affecting membrane fusion.

### Example 8. Immunogenicity, neutralizing antibody responses and protective effect in non-human primates

In this study, non-human primates (cynomolgus macaques) were immunized with either a medium dose or high dose of an inactivated SARS-CoV-2-vaccine as described above (see e.g. Example 4). Immunogenicity, neutralizing antibody responses and protective effect were then determined (see e.g. Examples 2 and 6).

### Total IgG immune responses in cynomolgus macaques

The immune responses in cynomolgus macaques for the two doses (medium and high) were assessed with a total IgG ELISA. Plates were coated with either the soluble ectodomain of spike protein with a T4 trimerization domain (A) or receptor binding domain (RBD) (B) of the spike glycoprotein or the nucleoprotein (C). Sera taken at baseline (day -1) and on days 0, 14, 21 28, 35 and 47/49 were analyzed. Antibody units (AU) were determined by fitting the relative luminescence to the respective standard curves with a known IgG concentration in AU/mL.

The results are shown in Figure 16. The inactivated SARS-CoV-2-vaccine was highly immunogenic in cynomolgus macaques, and a plateau in the immune response was already reached one week after the second immunization. There was no significant difference in the immune responses between high and medium dose.

### Neutralizing antibody responses in cynomolgus macaques

Presence of functional antibodies in macaque immune sera after immunization with the inactivated SARS-CoV-2-vaccine was assessed in a serum neutralization test. Serial dilutions of sera (days 0, 14, 21, 28, 35 and 47/49) were incubated with SARS-CoV-2 (SARS-CoV-2 strain Slovakia/SK-BMC5/2020), where spike glycoprotein specific antibodies bind to the virus, thereby blocking virus attachment to the ACE2 receptor on the surface of the target cells, and prevent entry into the cell. The 50% endpoint titers (SNT₅₀) were determined with the Spearman-Karber method.

The results are shown in Figure 17. As for the total IgG titers, a plateau was reached for both medium and high doses one week (day 28) after the second immunization. There seems to be a difference between the high and the medium doses on days 28 and 35, however, this difference is not statistically significant.

### Viral colonization in cynomolgus macaques

Four weeks after the second immunization, macaques were challenged intranasally (0.25 mL per nostril) and intratracheally (4.5 mL) with 1×10⁵ PFU SARS-CoV-2 (SARS-CoV-2 strain BetaCoV/France/IDF/ 0372/2020 SARS-CoV-2). Nasopharyngeal and tracheal swabs were taken before challenge (day 35) and on days 1, 2, 3, 4, 7 11 and 15 days post infection. Bronchoalveolar lavage (BAL) was collected on day 3 post infection. Swabs and BAL were analyzed with RT-qPCR (genomic and subgenomic) for presence of SARS-CoV-2.

The results are shown in Figures 18 to 20. Cynomolgus macaques immunized with the inactivated SARS-CoV-2-vaccine were protected from a combined intranasal and tracheal challenge with SARS-CoV-2. SARS-CoV-2 genomic RNA could transiently be detected in immunized macaques on day 2 post infection at a very low level in nasopharyngeal and tracheal swabs, but not at all in BAL. Sub-genomic SARS-CoV-2 RNA was not detectable at all in nasopharyngeal and tracheal swabs, nor in BAL. Sub-genomic RNA is a measure of virus which has replicated in the host. No difference between the high and medium dose of the inactivated SARS-CoV-2-vaccine could be detected in regard to the level of protection observed.

### Example 9. Immunogenicity of inactivated SARS-CoV-2 vaccine in rats

This study relates to a prophylactic, purified, inactivated SARS-CoV-2 vaccine for the prevention of COVID-19 caused by SARS-CoV-2. SARS-CoV-2 is propagated on Vero cells and inactivated by β-propiolactone. In this example, a repeat dose and local tolerance toxicity study was performed in female and male rats to assess potential systemic toxicity and local tolerability of the inactivated SARS-CoV-2 vaccine.

A human dose of 53 antigen units (AU)/0.5 mL was formulated with an aluminium salt (Alhydrogel) and CpG 1018. Rats were given intramuscular injections (2 sites×0.2 mL, 42 AU) on three occasions with 2 weeks interval over a period of 29 days. Blood was collected by venipuncture from the jugular (or other suitable) vein on day 8, day 15 and day 22 as well as on day 51.

Serum samples from the animals assigned to the recovery phase of the study were analyzed to assess the immunogenicity of the vaccine and monitor the immune response over time.

### MATERIAL AND METHODS

### EQUIPMENT

Multiplate washer BioTek ELx405 Select CW with Bio-Stack.

Multiplate reader BioTek Synergy 2 using Microplate software Gen5 (version 3.10.06) for data acquisition and evaluation.

GraphPad Prism (version 8.4.3) was used for plotting and visualization of data.

### MATERIAL AND REAGENTS

Nunc MaxiSorp® flat-bottom 96 well microtiter plates (Thermo Scientific, #439454) Microplate, 96 well, PS, F-bottom, clear (Greiner Bio-One International, #655161) Dulbecco's Phosphate Buffered Saline (DPBS) 1× (Gibco, #14190-094) Dulbecco's Phosphate Buffered Saline (DPBS) 10× (Gibco, #14200-067)
Bovine Serum Albumin (BSA) Fraction V (biomol, #01400.100)
Tween 20 (Sigma-Aldrich, #P7949-500ml)
Goat Anti-Rat IgG-HRP (Southern Biotech, #3030-05)
ABTS [2,2' Azino-bis(3-ethylbenzothiazoline-6-sulfonic acid)] (Sigma-Aldrich, #A3219-100ml) Sodium dodecyl sulfate (SDS) (Sigma-Aldrich, #71729)
Spike S1 protein, 78 kDa (Aviva, #OPAN06665-100UG)

### Biological material

Rat serum samples were collected on study days 8, 15, 22 and 51 from the two groups in the recovery study (Table 8), frozen at the test facility, shipped on dry-ice and stored at -80°C until analysis.

**Table 8: Experimental design, recovery groups.**

| **Treatment** | **Dosage Level (AU/dose)** | **Dose Volume (mL)*** | **Number of Animals** | |
|---|---|---|---|---|
| | | | **Males** | **Females** |
| 0.9% sodium chloride | 0 | 0.4 | 5 | 5 |
| Vaccine | 42 | 0.4 | 5 | 5 |

| | | | | |
|---|---|---|---|---|
| * One injection to each hind limb (2×0.2 mL) on each dosing occasion | | | | |

### METHODS

### ELISA

Vaccine-specific serum antibody responses were determined using the ELISA protocol as described below.

ELISA plates (Maxisorp, Nunc) were coated with 100 ng spike S1 protein in PBS and incubated at 4°C overnight. The plates were blocked with 5% BSA, 0.05% Tween-20, PBS for 1-2 hours at RT. Plates were washed with PBS/0.IT (PBS with 0.1% Tween-20). Individual rat sera (five per plate) were diluted in blocking buffer (five-fold dilution), added to 96-well plates and tested in duplicates by incubating for 1 hour at RT. Plates were then washed with PBS/0. IT. The secondary antibody (Goat Anti-Rat IgG-HRP, Southern Biotech) was diluted 1:4,000 in blocking buffer, added to the 96-well plates and incubated for 1 hour at RT. Plates were washed with PBS/0.IT and ABTS (Sigma-Aldrich) was added as substrate. After incubation for 30 min, the reaction was stopped by the addition of 1% SDS and the absorbance was read at 405 nm. The half-max titer (the reciprocal of the dilution that corresponds to the mean absorbance between highest and lowest dilution) was determined.

### Sample fate

After completion of the analysis and acceptance of the results, the remaining samples were discarded.

### RESULTS

Serum samples obtained from two study groups at different time points were analyzed by ELISA. The geometric mean titers for male and female rats from each group, time point are shown in the Table 9 below.

**Table 9: Geometric mean half-max titers for each group split into male and female rats**

| **Group** | **Sex** | **Study Day** | | | |
|---|---|---|---|---|---|
| | | 8 | 15 | 22 | 51 |
| 0.9% sodium chloride | Male | <50 | <50 | <50 | <50 |
| | Female | <50 | <50 | <50 | <50 |
| Vaccine | Male | <50 | 253 | 6983 | 11431 |
| | Female | <50 | 127 | 19680 | 12279 |

Sera from rats in the placebo group (0.9% sodium chloride) showed no reactivity (half-max titer <50) at any of the time points when analyzed by ELISA (see Table 9 above).

Rats in the group vaccinated with inactivated SARS-CoV-2 vaccine showed after the first immunization a weak response, however, below a measurable half-max titer (data not shown). One week after the second immunization (day 22) a plateau in the immune response was reached (see Table 9 above, Figure 21 and Figure 22). A slightly higher immune response was observed in female rats on study days 15 and 22 (see Table 9 above, Figure 21). However, this difference was not statistically significant.

### DISCUSSION

In this study, the inactivated SARS-CoV-2 vaccine was shown to be highly immunogenic in all rats. However, a low immune response was observed following a single immunization. Subsequent immunization resulted in continued increases in anti-spike S1 protein antibody titers. A plateau was reached after the second immunization, suggesting that a two-dose regime could be sufficient to reach high antibody titers at least in rats. The third immunization did not further increase the antibody titers. A normal dose kinetics was seen in the animals.

### CONCLUSION

The analysis described in this report indicated that the inactivated SARS-CoV-2 vaccine was highly immunogenic in rats and induced robust antibody titers in rats.

### Example 10. Further liquid chromatography with tandem mass spectrometry (LC-MSMS) analysis of inactivated SARS-CoV-2

### Methodology:

A further LC-MSMS analysis of BPL-inactivated SARS-CoV-2 particles, as described in Example 7, was performed in order to obtain greater coverage of the proteins. Five aliquots of the BPL-inactivated SARS-CoV-2 sample were separated on SDS-PAGE and the bands visualized by either silver staining for visualization or Coomassie staining for processing. The Coomassie-stained bands corresponding to spike protein (based on previous analysis) were subjected to in-gel digestion with trypsin or chymotrypsin or to acid hydrolysis. Trypsin digests were performed twice, once with and once without previous PNGase F (peptide:N-glycosidase F) digestion, to identify peptides masked by glycosylation.

Digested peptides were analysed by LC-MSMS essentially as described in Example 7. In particular, the resulting peptides were analyzed with nano-liquid chromatography coupled to a high-resolution accurate mass spectrometer. Peptides were identified from raw spectra using the MaxQuant software package and the UniProt reference databases for SARS-CoV-2 and *Chlorocebus sabaeus* in combination with a database of common lab contaminants. To account for modifications the data were also searched specifically for β-propiolactone (BPL) modifications, and spectra of all BPL-modified peptides of the SARS-CoV-2 spike protein were manually validated. The degree of modification was globally estimated as the percentage of BPL-modified spectra identified, and on site-level by calculating site occupancies from the ratio of modified to unmodified peptides for each peptide/site separately.

### Results:

The total coverage of particular SARS-CoV-2 proteins, using the combination of four digestion methods (i.e. (i) trypsin (ii) trypsin + PNGase F (iii) chymotrypsin and (iv) acid hydrolysis) was as follows:
Spike (S) protein - 91.5%
Membrane (M) protein - 60.36%
Nucleoprotein (N) - 74.70%

The number of BPL-modified peptides in the inactivated SARS-CoV-2 particles, based on each digestion method, is shown in Table 10 below:

**Table 10: Number of identified SARS-CoV-2 peptide spectra across all bands analyzed**

| **Sample** | **Total** | **BPL modified** | **% BPL modified** |
|---|---|---|---|
| Trypsin | 3148 | 97 | 3.1% |
| Trypsin + PNGase F | 2354 | 61 | 2.6% |
| Chymotrypsin | 2753 | 174 | 6.3% |
| Acid hydrolysis | 939 | 33 | 3.5% |
| **Total** | **9194** | **365** | **4.0%** |

As shown in Example 7, this confirms that the percentage of BPL-modified peptides is low regardless of the digestion method, e.g. less than 7%, 2 to 7% or around 2-5% on average.

Using a combination of the four digestion methods described above, a greater coverage of amino acid residues in SARS-CoV-2 proteins could be achieved. Accordingly, BPL-modifications were detected at the positions in the spike (S) and membrane (M) proteins shown in Table 11 below. The mean % occupancy at each site, as described in Example 7 above, is also shown in Table 11.

**Table 11. BPL-modified sites identified in S protein and their occupancy**

| **Protein** | **Site Position** | **% occupancy** |
|---|---|---|
| Spike-protein | H49 | 1% |
| Spike-protein | H146 | 2% |
| Spike-protein | C166 | 1% |
| Spike-protein | M177 | 6% |
| Spike-Protein | H207 | 1% |
| Spike-protein | H245 | 13% |
| Spike-protein | C432 | 8% |
| Spike-protein | H519 | 2% |
| Spike-protein | H625 | 7% |
| Spike-Protein | M1029 | 2% |
| Spike-Protein | H1058 | 11% |
| Spike-protein | H1083 | 3% |
| Spike-protein | H1088 | 4% |
| Spike-protein | H1101 | 1% |
| Spike-protein | H1159 | 4% |
| Spike-Protein | H1271 | 1% |
| Membrane protein | H125 | <10% |
| Membrane protein | H154 | <10% |
| Membrane protein | H155 | <10% |
| Membrane protein | H210 | <10% |

From the data in Table 11, it can be seen that up to around 16 residues in the spike (S) protein may be modified, and up to 4 residues in the membrane (M) protein. The occupancy at each site is low, e.g. less than 20%, typically less than 10%. Therefore the inactivated SARS-CoV-2 particles show a low degree of BPL-modifications.

### Example 11. A Phase I/II Randomized, Dose- Finding Study To Evaluate The Safety, Tolerability And Immunogenicity Of an Inactivated, Adjuvanted SARS-CoV-2 Virus Vaccine Candidate Against COVID-19 In Healthy Subjects

A Phase I/II clinical study based on the methodology set out in Example 4 above was carried out. Thus the product composition is essentially as described in Examples 1 and 4 above. Three dose levels (low, medium and high) were studied, as described in Table 12 below. The dose levels (in AU/dose) may be determined by the SARS-CoV-2 ELISA assay as described in Example 1. For all dose levels, the adjuvant comprised 0.5mg/dose aluminium hydroxide and 1 mg/dose CpG 1018.

**Table 12. Product composition - 0.5 mL/dose**

| Active substance | | Measured Antigen Units per dose in final product by ELISA |
|---|---|---|
| SARS-CoV-2 inactivated virus | Low dose | 3 AU |
| | Medium dose | 7 AU |
| | High dose | 35 AU |
| Excipients and buffer components | | |
| Aluminum hydroxide | All dose levels | 0.5 mg/dose |
| CpG 1018 | All dose levels | 1 mg/dose |
| Dulbecco's Phosphate Buffered Saline (DPBS)/Tris Buffered Saline¹) | | |
| rHA | All dose levels | ≤25µg/dose |

| | | |
|---|---|---|
| ¹DPBS composition: 200 mg/mL KCl (2.68 mM), 200 mg/mL KH₂PO₄ (1.47 mM), 8000 mg/mL NaCl (136.9 mM), 2160 mg/mL Na₂HPO₄*7H₂O (8.06 mM); Tris buffered saline: 20 mM Tris, 100 mM NaCl, pH 7.5. | | |

### Study design

The study is a randomized, dose-escalation, multicenter study with three dose groups. Two doses were administered to each subject, 21 days apart (Day 1 and 22). The study population was approximately 150 healthy volunteers aged 18 to 55 years. The study is conducted in two Parts. Part A (covering the follow-up from Day 1 to Day 36) and Part B (covering the follow-up from Day 37 to Day 208). The study was carried out at 4 sites in the UK, Birmingham, Bristol, Newcastle, Southampton. The immunization route was intramuscular (i.m.).

### Objectives

### Primary Objective:

The primary objective of this study is to evaluate the safety, tolerability and immunogenicity of the inactivated, adjuvanted SARS-CoV-2 vaccine candidate up to 14 days after completion of a two-dose (Day 1 and 22) schedule in healthy adults aged 18 to 55 years.

### Secondary Objectives:

To determine the optimal dose level of inactivated, adjuvanted SARS-CoV-2 vaccine candidate in healthy adults aged 18 to 55 years.

To evaluate tolerability, safety and immunogenicity of the inactivated, adjuvanted SARSCoV-2 vaccine candidate up to 6 months after the last vaccination in healthy adults aged 18 to 55 years.

**Table 13. Study endpoints**

| **Primary Endpoints** | **Secondary Endpoints** |
|---|---|
| SAFETY | SAFETY |
| + Frequency and severity of solicited adverse events (AEs) (local and systemic reactions) within 7 days after any vaccination. | + Frequency and severity of any unsolicited AE until Day 36. |
| | + Frequency and severity of any vaccine-related AE until Day 36. |
| | + Frequency and severity of any AE until Day 208. |
| | + Frequency and severity of any vaccine-related AE until Day 208. |
| | + Frequency and severity of any SAE until Day 36. |
| | + Frequency and severity of any AESI until Day 36. |
| | + Frequency and severity of any SAE until Day 208. |
| | + Frequency and severity of an AESI until Day 208. |
| IMMUNOGENICITY | IMMUNOGENICITY |
| + Geometric mean titre (GMT) for neutralizing antibodies against SARS-CoV-2 determined by wild-type virus microneutralizing assay at Day 36. | + Immune response as measured by neutralizing antibody titers against SARS-CoV-2 on Day 8, Day 22, Day 106 and Day 208. |
| | + Proportion of subjects with seroconversion in terms of neutralizing antibodies on Day 8, Day 22, Day 36, Day 106 and Day 208. |
| | + Fold increase of SARS-CoV-2 neutralizing antibody titers on Day 8, Day 22, Day 36, Day 106 and Day 208 compared with baseline. |
| | + GMTs for IgG antibodies against SARS-CoV-2, determined by IgG S-ELISA, at Day 1, 8, 22, 36,106 and 208. |
| | + Proportion of subjects with seroconversion in terms of IgG antibodies against SARS-CoV-2 as determined by ELISA on Day 8, Day 22, Day 36, Day 106 and Day 208. |

### Results - Safety and Tolerability

### Primary Endpoint:

Frequency and severity of solicited adverse events (AEs) (local and systemic reactions) within 7 days after each vaccination. Solicited injection site reactions include injection site pain, itching, tenderness, redness and swelling/induration. Solicited systemic reactions include include fever/body temperature, fatigue, headache, nausea/vomiting, muscle pain.

Overall, 81.7% of participants reported at least one solicited reaction within 7 days after any vaccination.
> 47.7% (after first vaccination) and 51.3% (after second vaccination) of participants experienced a solicited injection site reaction.
> 52.3% (after first vaccination) and 52.0% (after second vaccination) of participants experienced a solicited systemic reaction.
+ All solicited AEs across the dose groups were assessed as mild or moderate with the exception of 3 events reported by 2 participants who experienced severe (Grade 3) solicited adverse events (one subject: severe headache and fatigue; one subject: severe fatigue). Both participants were in the high dose group.
+ Majority of solicited AEs resolved within 7 days post vaccination.

### Solicited Local AEs:

+ Across all dose levels 66.7% of vaccinees reported at least one solicited injection site reaction (no statistical significant difference; p-value overall 0.631) with 68.6% in the low dose, 60.8% in the medium dose and 70.6% in the high dose group

+ The most common injection site reaction after either vaccination was tenderness affecting 58.2% across dose groups and injection site pain 41.8% across dose groups.

### Solicited Systemic AEs:

+ Across all dose levels 69.3% of vaccinees reported at least one solicited systemic reaction (no statistically significant difference between groups; p-value overall 0.507); the rate of vaccinees with solicited symptoms was 72.5% in both low and high dose and 62.7% in the medium dose group.
+ Overall, most frequently reported solicited systemic adverse events following vaccination included headache (46.4%), fatigue (39.2%) and muscle pain (32.7%).

### Unsolicited AEs:

+ Overall, 39.2% of participants reported at least one unsolicited adverse event. No statistically significant differences between dose groups were observed.
+ All unsolicited adverse events were mild and moderate.
+ 2 Cases of COVID-19 were confirmed by PCR up to Day 36. One mild case occurred in a participant of the medium dose group 16 days after the first vaccination. A moderate COVID-19 case was confirmed by PCR in a participant of the low dose group 4 days after the second vaccination.
+ Rates of unsolicited adverse events considered treatment-related up to Day 36 were 17.6% (27/153) with 23.5% in the low dose group, 13.7% in the medium dose group and 15.7% in the high dose group.
+ One adverse event of special interest has been reported (event term: chilblains); the event was assessed as mild and not-related to the study vaccination by the investigator.
+ AESI has been reported as serious adverse event as per protocol (medically important condition); no other serious adverse event has been reported.

### Conclusions - Safety and Tolerability

+ The vaccine candidate was generally safe and well tolerated across all dose groups tested, with no safety concerns identified by an independent Data Safety Monitoring Board.
+ There were no statistically significant differences between dose groups and no differences between first and second vaccinations in terms of reactogenicity.
+ The majority of Adverse Events (AEs) were mild or moderate and only two subjects reported severe solicited AEs (headache and fatigue).
+ All solicited AEs resolved quickly.
+ Only 17.6% of unsolicited adverse events up to day 36 were considered related to the vaccine and no severe unsolicited AEs were reported.
+ There were no serious related AEs.

### Results - Immunogenicity

### Neutralizing antibodies as measured by MNA50

Neutralizing antibodies were measured by a microneutralization assay (MNA50). Vero/E6 cells are seeded at 2.5 x 10⁵ cells/mL in 100µl/well in a microtiter plate and incubated at 37°C overnight. Serum samples from subjects were heat inactivated at 56°C for 30 minutes. The neutralization plate comprising virus and serum samples is prepared at 37°C for 1-1.5 hours. The neutralized virus is then transferred to the Vero/E6 cells in the microtiter plate and incubated at 37°C for 1-1.5 hours. Cells are overlayed in 2% CMC in 2xMEM and incubated at 37°C for 22-26 hours. Cells are fixed with 10% formalin at room temperature for >8 hours. Plaques are then visualized with a SARS-CoV-2 RBD spike antibody, detected by a secondary antibody and HRP-polymer. Pfu/well are counted and used to calculate the ND₅₀.
+ A clear dose dependent response was observed with the highest Geometric Mean Titre (GMT) for neutralizing antibodies in the high dose group at both Day 22 (GMT 46.5; 95%CI: 38.79, 55.66)) and Day 36 (GMT 530.4; 95%CI: 421.49, 667.52) - see Figure 23.
+ On Day 36, the GMT of the high dose group was statistically significantly higher than each of the other dose groups. GMT in the low dose was 161.1 (95%CI: 121.35, 213.82) and in medium dose group, 222.3 (95%CI: 171.84, 287.67)
+ GMT- fold increases for neutralizing antibodies at Day 36 were 5.55 (95%CI: 4.18, 7.37) in the low dose, 7.22 (95%CI: 5.64, 9.25) in the medium dose and 17.68 (95%CI: 14.04, 22.26) in the high dose group.
+ On Day 22, prior to the second study vaccination, the number of participants with seroconversion for neutralizing antibodies (defined as ≥4-fold increase from baseline) was 10.0% (5/50 participants) + At Day 36, 90.0% (95%CI: 0.78,0.97) of participants in the high dose group, were seroconverted which was statistically significantly higher compared to the low dose (51.0%; 95%CI: 0.37, 0.65) and medium dose group (73.5%; 95%CI: 0.59, 0.85) - see Figure 24.

### S-protein binding antibodies as measured by IgG ELISA

S-protein-binding antibodies were detected by a SARS-CoV-2 spike IgG ELISA. The ELISA plate is coated with a Spike protein (Wuhan). Anti-S protein antibodies in the serum sample bind to the immobilized Spike protein antigen, and are detected by a secondary (peroxidase-conjugated) antihuman IgG antibody. Results are presented as ELISA laboratory units per mL (ELU/mL). A commercially available human serum screened for high pre-existing S antibody titers is used as a control.
+ The S-protein IgG antibody titers correlated with neutralization titers (r=0.79, p<0.001) -see Figure 25.
+ Similar to the neutralization titer, a clear dose dependent response was observed with the highest GMT for neutralizing antibodies in the high dose group at both Day 22 (GMT 30.0 (95%CI: 26.92, 33.48)) and Day 36 (GMT 2147.9 (95%CI: 1705.98, 2704.22)) - see Figure 26.
+ On Day 36, the GMT of the high dose group was statistically significantly higher than each of the other dose groups. GMT in the low dose was 325.1 (95%CI: 245.45, 430.46) and in the medium dose group 691.6 (95%CI: 494.91, 966,52)
+ GMFR for S-protein binding antibodies at Day 36 were 12.69 (95%CI: 9.54, 16.88) in the low dose, 26.16 (95%CI: 18.73, 36.53) in the medium dose and 85.91 (95%CI: 68.24, 108.17) in the high dose group.
+ At Day 36, 100% (95%CI: 0.93,1.00) of participants in the high dose group were seroconverted and 89.3% (95%CI: 0.78,0.97) in the medium dose group as well as 84.3% (95%CI: 0.71,0.93) (p=0.053 compared to high dose) in the low dose group (p=0.017 compared to high dose).

### Cellular response:

Exploratory endpoints evaluated T-cell responses by IFNgamma ELISpot analysis against S-protein, Membrane-protein and Nucleocapsid-protein. Isolated PBMCs (fresh) are stimulated with SARS-CoV-2 peptides from the S, M and N proteins. Interferon-gamma production by T cells is detected by an anti-IFNy antibody and visualized with a labelled secondary antibody. Spots are thus produced where interferon-gamma was released by activated T cells. A nil control and phytohemagglutinin (PHA) control are used. A 6 spot reactivity cut off was used, i.e. a sample is considered reactive against an individual stimulation panel (peptide pools) if normalized spot counts (Nil control counts substracted) per 2.10 x 10⁵ PBMCs ≥ 6.

At Day 36 in the high dose group, 76% of study participants (34/45) were reactive against peptide pools spanning the full-length S-protein (see Figure 27), 36% (16/45) against the M-protein (see Figure 28) and 49% (22/45) against the N-protein (see Figure 29).

### Conclusions - Immunogenicity

+ The vaccine candidate was highly immunogenic with more than 90% of all study participants developing significant levels of antibodies to the SARS-CoV-2 virus spike protein across all dose groups tested.
+ Seroconversion Rates (SCR) for S-protein binding IgG antibodies were 89.8% in the medium dose and 100% in the high dose group.
+ Two weeks after completion of the two dose schedule, Geometric Mean Fold Rises (GMFRs) from baseline were 26 in the medium dose and 86 in the high dose group.
+ Of note, the IgG antibody response was highly correlated with neutralization titres (MNA50) (r=0.79, p<0.001).
+ The vaccine candidate induced a dose dependent response with statistically significant higher Geometric Mean Titres (GMTs) for both IgG and neutralizing antibodies in the high dose group compared to the low and medium dose groups.
+ In the high dose group, the GMT of neutralizing antibodies antibody titres measured 2 weeks after completion of the 2-dose schedule was at or above levels for a panel of convalescent sera (GMT 530.4 (95% CI: 421.49, 667.52)).
+ With a GMT ratio of vaccine vs. convalescent sera ≥ 1 vaccine efficacy has been reported above 80% for other vaccines *).
*)Earle et al. MedRxiv, March 2021, https://doi.org/10.1101/2021.03.17.20200246); Khoury et al. MedRxiv, March 2021, https://doi.org/10.1101/2021.03.09.21252641
+ The vaccine candidate induced broad T-cell responses across participants with antigen-specific IFNgamma producing T-cells against the S-protein, M and N protein detected in 75.6 %, 35.6% and 48.9% of study participants, respectively.

### ADDITIONAL ASPECTS OF THE INVENTION

In further aspects, the present invention provides:
A1. A SARS-CoV-2 vaccine comprising an optimally (e.g. wherein the native surface of the S-protein is preserved) inactivated SARS-CoV-2 particle, wherein the SARS-CoV-2 particle is able to seroconvert a subject that is administered the SARS-CoV-2 vaccine with at least a 70% probability.
A2. The SARS-CoV-2 vaccine of aspect A1, wherein the SARS-CoV-2 particle is able to seroconvert the subject that is administered the SARS-CoV-2 vaccine with at least 80%, 85%, 90%, or 95% probability.
A3. The vaccine of aspect A1 or A2, wherein the SARS-CoV-2 particle has a RNA genome corresponding to the DNA sequence provided by any one of the nucleic acid sequences of
   - SEQ ID NO: 1 (see Genbank NC_045512.2), or a variant nucleic acid sequence that is at least 85% identical to SEQ ID NO: 1 and able to pack a virulent SARS-CoV-2; or
   - SEQ ID NO: 9 (see NCBI MT066156), or a variant nucleic acid sequence that is at least 85% identical to SEQ ID NO: 1 and able to pack a virulent SARS-CoV-2; or
   - SEQ ID NO: 18 (see NCBI MW598408). or a variant nucleic acid sequence that is at least 85% identical to SEQ ID NO: 18 and able to pack a virulent SARS-CoV-2; or
   - SEQ ID NO: 20 (see NCBI MW520923). or a variant nucleic acid sequence that is at least 85% identical to SEQ ID NO: 20 and able to pack a virulent SARS-CoV-2; or
   - SEQ ID NO: 22 (see NCBI MW422256). or a variant nucleic acid sequence that is at least 85% identical to SEQ ID NO: 22 and able to pack a virulent SARS-CoV-2; or
   - SEQ ID NO: 22 (see NCBI MW422256). or a variant nucleic acid sequence that is at least 85% identical to SEQ ID NO: 24 and able to pack a virulent SARS-CoV-2; or
   - SEQ ID NO: 22 (see NCBI MW422256). or a variant nucleic acid sequence that is at least 85% identical to SEQ ID NO: 26 and able to pack a virulent SARS-CoV-2. A4. The vaccine of any one of aspects A1-A3, wherein the SARS-CoV-2 particle has an S protein as defined by the amino acid sequence
   - SEQ ID NO: 3, or a variant amino acid sequence that is at least 95% identical to SEQ ID NO: 3 and able to pack a virulent SARS-CoV-2; or
   - SEQ ID NO: 11, or a variant amino acid sequence that is at least 95% identical to SEQ ID NO: 3 and able to pack a virulent SARS-CoV-2; or
   - SEQ ID NO: 19, or a variant amino acid sequence that is at least 95% identical to SEQ ID NO: 3 and able to pack a virulent SARS-CoV-2; or
   - SEQ ID NO: 21, or a variant amino acid sequence that is at least 95% identical to SEQ ID NO: 3 and able to pack a virulent SARS-CoV-2; or
   - SEQ ID NO: 23, or a variant amino acid sequence that is at least 95% identical to SEQ ID NO: 3 and able to pack a virulent SARS-CoV-2; or
   - SEQ ID NO: 25, or a variant amino acid sequence that is at least 95% identical to SEQ ID NO: 3 and able to pack a virulent SARS-CoV-2; or
   - SEQ ID NO: 27, or a variant amino acid sequence that is at least 95% identical to SEQ ID NO: 3 and able to pack a virulent SARS-CoV-2.
A5. The SARS-CoV-2 vaccine of any one of aspects A 1-A4, wherein the SARS-CoV-2 particle has a polyprotein selected from the amino acid sequences provided by any one of SEQ ID NOs: 2, 10, 13 or 16, preferably SEQ ID NO: 10, or a variant amino acid sequence that is at least 95% identical to any one of SEQ ID NOs: 2, 10, 13 or 16, preferably SEQ ID NO: 10, and able to pack a virulent SARS-CoV-2.
A6. The SARS-CoV-2 vaccine of any one of aspects A1-A5, wherein the SARS-CoV-2 is inactivated by chemical inactivation, thermal inactivation, pH inactivation, UV inactivation or radiation inactivation.
A7. The SARS-CoV-2 vaccine of aspect A6, wherein the chemical inactivation comprises contacting the SARS-CoV-2 particles with a chemical inactivation agent for longer than is required to completely inactivate the SARS-CoV-2 as measured by plaque assay or as measured by plaque assay plus one day.
A8. The SARS-CoV-2 vaccine of aspect A7, wherein the chemical inactivation comprises contacting the SARS-CoV-2 particle with formaldehyde and/or beta-propiolactone, preferably beta-propiolactone.
A9. The SARS-CoV-2 vaccine of aspect A8, wherein the formaldehyde and/or beta-propiolactone inactivation comprises contacting the SARS-CoV-2 particle with formaldehyde and/or beta-propiolactone for between 2-10 days.
A10. The SARS-CoV-2 vaccine of any one of aspects A6-A9, wherein the chemical activation is performed at about 4°C or about 22°C.
A11. The SARS-CoV-2 vaccine of any one of aspects A1-A10, further comprising an adjuvant.
A12. The SARS-CoV-2 vaccine of aspect A11, wherein the adjuvant is a CpG, preferably CpG 1018, optionally also comprising an aluminium salt adjuvant.
A13. The SARS-CoV-2 vaccine of aspect A12, wherein the aluminium salt adjuvant is aluminium hydroxide or aluminium phosphate salt.
A14. The SARS-CoV-2 vaccine of aspect A13, wherein the aluminium hydroxide comprises less than 1.25 ppb Cu.
A15. The SARS-CoV-2 vaccine of any one of aspects A12 to A14, wherein the alum:CpG (w/w) ratio is about 1:10, about 1:5, about 1:4, about 1:3, about 1:2, about 1:1, about 2:1, about 3:1, about 4:1, about 5:1, about 10:1, preferably between about 1:3 and 3:1, more preferably between about 1:2 and 1:1, most preferably about 1:2.
A16. The SARS-CoV-2 vaccine according to any one of aspects A12 to A14, wherein the amount of free (unbound) CpG in the vaccine composition is greater than 10%, greater than 20%, greater than 30%, greater than 40%, greater than 50%, greater than 60%, greater than 70%, greater than 80%, greater than 90%, greater than 95%, preferably about 70% to 95%, most preferably about 80% to 90%.
A17. The SARS-CoV-2 vaccine according to any one of aspects A1 to A16, wherein the vaccine composition comprises at least one buffer, preferably a phosphate buffer.
A18. The SARS-CoV-2 vaccine of any one of A11-A13, wherein the vaccine comprises or further comprises an adjuvant comprising a peptide and a deoxyinosine-containing immunostimulatory oligodeoxynucleic acid molecule (I-ODN).
A19. The SARS-CoV-2 vaccine of aspect A18, wherein the peptide comprises the sequence KLKL₅KLK (SEQ ID NO: 5) and the I-ODN comprises oligo-d(IC)₁₃ (SEQ ID NO: 6).
A20. The SARS-CoV-2 vaccine of any one of aspects A1-A19, further comprising one or more pharmaceutically acceptable excipients.
B1. A kit comprising a SARS-CoV-2 vaccine of any one of aspects A1-A15.
B2. The kit of aspect B1, further comprising a second vaccine.
B3. The kit of aspect B2, wherein the second vaccine is another SARS-CoV-2 virus vaccine (e.g. of another technology such as mRNA or adenovirus vectored), an influenza virus vaccine or a Chikungunya virus vaccine.
C1. A method, comprising administering a first dose of a prophylactically or therapeutically effective amount of the SARS-CoV-2 vaccine of any one of aspects A1-A15 to a subject in need thereof.
C2. The method of aspect C1, further comprising administering a second dose of a prophylactically or therapeutically effective amount of the SARS-CoV-2 vaccine.
C3. The method of aspect C1 or C2, wherein the second dose of the SARS-CoV-2 vaccine is administered about 7 days after the first dose of the SARS-CoV-2 vaccine.
C4. The method of aspect C1 or C2, wherein the second dose of the SARS-CoV-2 vaccine is administered about 14 days after the first dose of the SARS-CoV-2 vaccine.
C5. The method of aspect C1 or C2, wherein the second dose of the SARS-CoV-2 vaccine is administered about 21 days after the first dose of the SARS-CoV-2 vaccine.
C6. The method of aspect C1 or C2, wherein the second dose of the SARS-CoV-2 vaccine is administered about 28 days after the first dose of the SARS-CoV-2 vaccine.
C7. The method of any one of aspects C1-C6, wherein the administering results in production of SARS-CoV-2 neutralizing antibodies.
C8. The method of any one of aspects C1-C7, wherein the prophylactically or therapeutically effective amount of the SARS-CoV-2 vaccine is defined as about 0.01 to 25 mAU (milli-absorption units x minutes), preferably about 0.05 to 10 mAU, more preferably about 0.1 to 5 mAU, most preferably about 0.25 to 2.5 mAU, as assessed by SE-HPLC.
C9. The method of any one of aspects C1-C7, wherein the prophylactically or therapeutically effective amount of the SARS-CoV-2 vaccine is defined as about 0.05 to 50 µg total protein, about 0.1 to 25 µg, about 0.25 to 12.5 µg, preferably about 0.5 to 5 µg total protein, as measured by (µ)BCA.
C10. The method of any one of aspects C1-C7, wherein the prophylactically or therapeutically effective amount of the SARS-CoV-2 vaccine is defined as about 0.025 to 25 µg S-protein, about 0.05 to 12.5 µg, about 0.125 to 6.25 µg, preferably about 0.25 to 2.5 µg S-protein, as measured by ELISA.
D1. A method of producing a SARS-CoV-2 vaccine, comprising
   (i) passaging a SARS-CoV-2 on Vero cells, thereby producing a culture medium comprising the SARS-CoV-2;
   (ii) harvesting the culture medium of (i);
   (iii) precipitating the harvested culture medium of (ii), thereby producing a SARS-CoV-2 supernatant; and
   (iv) optimally inactivating the SARS-CoV-2 in the SARS-CoV-2 supernatant of (iii) thereby producing an inactivated SARS-CoV-2.
D2. The method of aspect D1, further comprising concentrating the culture medium of (ii) prior to step (iii).
D3. The method of aspect D1 or D2, wherein the precipitation of (iii) comprises contacting the culture medium of (ii) with protamine sulfate or benzonase.
D4. The method of any one of aspects D1-D3, further comprising (v) dialyzing the inactivated SARS-CoV-2 of (iv), thereby producing a dialyzed SARS-CoV-2.
D5. The method of aspect D4, further comprising (vi) filtering the dialyzed SARS-CoV-2 of (v).
D6. The method of any one of aspects D1-D5, wherein the inactivating is by chemical inactivation, thermal inactivation, pH inactivation, or UV inactivation.
D7. The method of aspect D6, wherein the chemical inactivation comprises contacting the SARS-CoV-2 particle with a chemical inactivation agent for at least 4 days.
D8. The method of aspect D6 or D7, wherein the chemical inactivation agent comprises formaldehyde.
D9. The method of any one of aspects D6-D8, wherein the chemical activation is performed at about 4°C or about 22°C.
D10. The method of aspect D8 or D9, further comprising neutralizing the formaldehyde.
D11. The method of aspect D10, wherein the neutralizing is performed with sodium metabisulfite.
D12. The method of any one of aspects D1-D11, wherein the chemical inactivation is performed with BPL, preferably at a concentration of 300 to 700ppm, more preferably 500ppm and inactivated for about 1 to 48h, preferably 20 to 28h, most preferred 24 hours ± 2 hours (such as also ± 1 hour or ± 0.5 hour) at 2°C to 8°C.
D13. The method of aspect D12, wherein the chemical inactivation is followed by a hydrolyzation step for 2.5 hours ± 0.5 hours at 35°C to 39°C, preferably around 37°C.
E1. The use of the optimally inactivated SARS-CoV-2 vaccine of any one of aspects A1-A15 for the treatment and prevention of a SARS-CoV-2 infection.
E2. The use of aspect E1, wherein the inactivated SARS-CoV-2 vaccine is administered in a first dose of a therapeutically effective amount to a subject in need thereof.
E3. The use of aspect E2, wherein the inactivated SARS-CoV-2 vaccine is administered in a second dose of a therapeutically effective amount to the subject.
E4. The use of aspect E3, wherein the second dose of the inactivated SARS-CoV-2 vaccine is administered about 7 days after the first dose of the SARS-CoV-2 vaccine.
E5. The use of aspect E3, wherein the second dose of the SARS-CoV-2 vaccine is administered about 14 days after the first dose of the SARS-CoV-2 vaccine.
E6. The use of aspect E3, wherein the second dose of the SARS-CoV-2 vaccine is administered about 21 days after the first dose of the SARS-CoV-2 vaccine.
E7. The use of aspect E3, wherein the second dose of the SARS-CoV-2 vaccine is administered about 28 days after the first dose of the SARS-CoV-2 vaccine.
E8. The use of any one of aspects E1-E6, wherein the administering results in production of SARS-CoV-2 neutralizing antibodies.
F1. A pharmaceutical composition for use in the treatment and prevention of a SARS-CoV-2 infection, wherein said pharmaceutical composition comprises the optimally inactivated SARS-CoV-2 vaccine of any one of aspects A1-A15.
F2. The pharmaceutical composition of aspect F1, wherein the inactivated SARS-CoV-2 vaccine is administered in a first dose of a therapeutically effective amount to a subject in need thereof.
F3. The use of aspect F2, wherein the inactivated SARS-CoV-2 vaccine is administered in a second dose of a therapeutically effective amount to the subject.
F4. The use of aspect F3, wherein the second dose of the inactivated SARS-CoV-2 vaccine is administered about 7 days after the first dose of the SARS-CoV-2 vaccine.
F5. The use of aspect F3, wherein the second dose of the SARS-CoV-2 vaccine is administered about 14 days after the first dose of the SARS-CoV-2 vaccine.
F6. The use of aspect F3, wherein the second dose of the SARS-CoV-2 vaccine is administered about 21 days after the first dose of the SARS-CoV-2 vaccine.
F7. The use of aspect F3, wherein the second dose of the SARS-CoV-2 vaccine is administered about 28 days after the first dose of the SARS-CoV-2 vaccine.
F8. The use of any one of aspects F1-F6, wherein the administering results in production of SARS-CoV-2 neutralizing antibodies.
G1. A SARS-CoV-2 vaccine comprising an effective amount of antigen, wherein said effective amount is able to seroconvert a subject that is administered the SARS-CoV-2 vaccine with at least a 70% probability.
G2. The SARS-CoV-2 vaccine according to aspect G1, wherein said effective amount is able to seroconvert a subject that is administered the SARS-CoV-2 vaccine with at least 80%, 85%, 90%, or 95% probability.
G3. The SARS-CoV-2 vaccine according to aspect G1 or G2, wherein said effective amount is between about 1 to 100 AU/dose, preferably between about 2 to 75 AU/dose, preferably between about 3 and 60 AU/dose, more preferably between about 3 and 55 AU/dose, more preferably between about 3 and 53 AU/dose.
G4. The SARS-CoV-2 vaccine according to aspect G3, where said effective amount is determined by ELISA wherein the antigen units (AU) correspond to ACE-2 binding capacity of the spike protein used as a standard.
H1. An immunogenic composition for stimulating an immune response against a severe acute respiratory syndrome coronavirus 2 (SARS-CoV-2), comprising a SARS-CoV-2 antigen and a toll-like receptor 9 (TLR9) agonist, wherein the SARS-CoV-2 antigen is an inactivated whole SARS-CoV-2, the TLR9 agonist is an oligonucleotide of from 10 to 35 nucleotides in length comprising an unmethylated cytidine-phospho-guanosine (CpG) motif, and the SARS-CoV-2 antigen and the oligonucleotide are present in the immunogenic composition in amounts effective to stimulate an immune response against the SARS-CoV-2 antigen in a mammalian subject.
H2. The composition of aspect H1, wherein the oligonucleotide comprises the sequence 5'-AACGTTCGAG-3' (SEQ ID NO:30).
H3. The composition of aspect H1, wherein the oligonucleotide comprises the sequence of 5'-TGACTGTGAA CGTTCGAGAT GA-3'(SEQ ID NO: 4).
H4. The composition of any one of aspects H1-3, wherein the oligonucleotide comprises a modified nucleoside, optionally wherein the modified nucleoside is selected from the group consisting of 2'-deoxy-7-deazaguanosine, 2'-deoxy-6-thioguanosine, arabinoguanosine, 2'-deoxy-2'substituted-arabinoguanosine, and 2'-O-substituted- arabinoguanosine.
H5. The composition of aspect H4, wherein the oligonucleotide comprises the sequence 5'-TCG₁AACG₁TTCG₁-3' (SEQ ID NO: 31) in which G1 is 2'-deoxy-7-deazaguanosine, optionally wherein the oligonucleotide comprises the sequence 5'-TCG₁AACG₁TTCG₁-X-G₁CTTG₁CAAG₁CT-5' (SEQ ID NO: 32), and in which G₁ is 2'-deoxy-7-deazaguanosine and X is glycerol (5'-SEQ ID NO: 31-3'-X-3'-SEQ ID NO: 31-5').
H6. The composition of any one of aspects H1-5, wherein the oligonucleotide comprises at least one phosphorothioate linkage, or wherein all nucleotide linkages are phosphorothioate linkages.
H7. The composition of any one of aspects HI-6, wherein the oligonucleotide is a single-stranded oligodeoxynucleotide.
H8. The composition of any one of aspects H1-7, wherein a 0.5 ml dose of the immunogenic composition comprises from about 750 to about 3000 µg of the oligonucleotide, or wherein the immunogenic composition comprises about 750 µg, about 1000 µg, about 1500 µg, or about 3000 µg of the oligonucleotide.
H9. The composition of any one of aspects H1-8, wherein the SARS-CoV-2 antigen is propagated in vitro in mammalian cells.
H10. The composition of any one of aspects H1-9, wherein the SARS-CoV-2 is inactivated by treatment with one or both of formalin and ultraviolet light.
H11. The composition of any one of aspects H1-9, wherein the SARS-CoV-2 is inactivated by treatment with beta-propiolactone.
H12. The composition of any one of aspects H1-11, wherein the SARS-CoV-2 comprises a combination of at least two different viral strains, or from two different viral clades or lineages.
H13. The composition of any one of aspects H1-12, wherein a 0.5 ml dose of the immunogenic composition comprises from about 0.025 to about 25 µg of the of the SARS-CoV- 2 spike (S) protein, or from about 0.25 to about 25 µg of the of the S protein.
H14. The composition of any one of aspects HI-13, further comprising an aluminum salt adjuvant.
H15. The composition of aspect H14, wherein the aluminum salt adjuvant comprises one or more of the group consisting of amorphous aluminum hydroxyphosphate sulfate, aluminum hydroxide, aluminum phosphate, and potassium aluminum sulfate
H16. The composition of aspect H14, wherein the aluminum salt adjuvant comprises aluminum hydroxide.
H17. The composition of any one of aspects H14-16, wherein a 0.5 ml dose of the immunogenic composition comprises from about 0.05 to about 0.50 mg A13+, or about 0.075 to about 0.175 mg A13+, or from about 0.25 to about 0.50 mg A13+, or about 0.375 mg A13+.
H18. The composition of any one of aspects HI-17, wherein the mammalian subject is a human subject.
H19. A kit comprising:
   i) the immunogenic composition of any one of aspects H1-18, and
   ii) instructions for administration of the composition to stimulate an immune response against the SARS-CoV-2 antigen in the mammalian subject.
H20. The kit of aspect H19, further comprising iii) a syringe and needle for intramuscular injection of the immunogenic composition.
H21. A method for stimulating an immune response against a severe acute respiratory syndrome coronavirus 2 (SARS-CoV-2) in a mammalian subject, comprising administering the immunogenic composition of any one of aspects H1-18 to a mammalian subject so as to stimulate an immune response against the SARS-CoV-2 antigen in the mammalian subject.
H22. The method of aspect H21, wherein the mammalian subject is a human subject and/or the immunogenic composition is administered by intramuscular injection.
H23. Use of the immunogenic composition of any one of aspects H1-18 for stimulating an immune response against a severe acute respiratory syndrome coronavirus 2 (SARS-CoV-2) in a mammalian subject, the method comprising administering to the subject an effective amount of the immunogenic composition.
H24. Use of the immunogenic composition of any one of aspects H1-18 for protecting a mammalian subject from infection with severe acute respiratory syndrome coronavirus 2 (SARS-CoV-2), the method comprising administering to the subject an effective amount of the immunogenic composition.
H25. Use of the immunogenic composition of any one of aspects H1-18 for preventing a mammalian subject from contracting COVID-19 disease, the method comprising administering to the subject an effective amount of the immunogenic composition.
H26. The use of any one of aspects H23-25, wherein the mammalian subject is a human subject and/or the immunogenic composition is administered by intramuscular injection.
J1. A SARS-CoV-2 vaccine comprising i) an inactivated SARS-CoV-2 particle; ii) a CpG-containing oligodeoxynucleotide (CpG-ODN); and iii) an alum adjuvant.
J2. The SARS-CoV-2 vaccine according to aspect J1, wherein said CpG-ODN is CpG 1018 as defined by SEQ ID NO: 4, and said alum adjuvant is aluminium hydroxide.
J3. The SARS-Cov-2 vaccine according to any one of the preceding aspects J, wherein the alum (Al³⁺):CpG (w/w) ratio in the vaccine composition is about 1:10, about 1:5, about 1:4, about 1:3, about 1:2, about 1:1, about 2:1, about 3:1, about 4:1, about 5:1, about 10:1, preferably between about 1:3 and 3:1, more preferably between about 1:2 and 1:1.
J4. The SARS-CoV-2 vaccine according to any one of the preceding aspects J, wherein the alum:CpG (w/w) ratio in the vaccine composition is about 1:2.
J5. The SARS-CoV-2 vaccine according to any one of the preceding aspects J, wherein the alum content of the vaccine is between about 0.1 and 2 mg/mL, between about 0.2 and 1.5 mg/mL, between about 0.5 and 1.3 mg/mL, especially between about 0.8 to 1.2 mg/mL, most preferably about 1 mg/mL, i.e., 0.5 mg/dose, wherein the vaccine is delivered in a volume of 0.5 mL.
J6. The SARS-CoV-2 vaccine according to any one of the preceding aspects J, wherein the CpG content of the vaccine is between about 0.25 and 6 mg/mL, between about 0.5 and 3 mg/mL, between about 1 and 3 mg/mL, especially between about 1.5 to 2.5 mg/mL, most preferably about 2 mg/mL, i.e., 1 mg/dose, wherein the vaccine is delivered in a volume of 0.5 mL.
J7. The SARS-CoV-2 vaccine according to any one of the preceding aspects J, wherein the amount of inactivated SARS-CoV-2 virus per dose in the vaccine is between about 0.01 and 25 mAU (milli-absorption units x minutes), preferably between about 0.05 and 10 mAU, more preferably between about 0.1 and 5 mAU, most preferably between about 0.25 and 2.5 mAU as assessed by SEC-HPLC, wherein the vaccine is delivered in a volume of 0.5 mL.
J8. The SARS-CoV-2 vaccine according to any one of the preceding aspects J, wherein the amount of free (unbound) CpG in the vaccine composition is greater than 10%, greater than 20%, greater than 30%, greater than 40%, greater than 50%, greater than 60%, greater than 70%, greater than 80%, greater than 90%, greater than 95%, preferably about 70% to 95%, most preferably about 80% to 90% (w/w), based on the total amount of CpG in the vaccine composition.
J9. The SARS-CoV-2 vaccine according to any one of the preceding aspects J, wherein the vaccine composition comprises at least one buffer, preferably a phosphate buffer.
J10. The SARS-CoV-2 vaccine according to any one of aspects J2 to 9, wherein said vaccine comprising aluminium hydroxide comprises less than 1.25 ppb Cu.
J11. The SARS-CoV-2 vaccine according to any one of the preceding aspects J, wherein viral RNA in the inactivated SARS-CoV-2 particle is replication-deficient.
J12. The SARS-CoV-2 vaccine according to any one of the preceding aspects J, wherein viral RNA in the inactivated SARS-CoV-2 particle (i) is alkylated and/or acylated (ii) comprises one or more modified purine (preferably guanine) residues or strand breaks and/or (iii) is cross-linked with one or more viral proteins.
J13. The SARS-CoV-2 vaccine according to any one of the preceding aspects J, wherein the virus particle is a beta-propiolactone-inactivated SARS-CoV-2 particle, preferably at a concentration of 300 to 700ppm, more preferably 500ppm and inactivated for about 1 to 48h, preferably 20 to 28h, most preferred 24h.
J14. The SARS-CoV-2 vaccine according to any one of the preceding aspects J, wherein the virus particle is an ultraviolet (UV)-inactivated SARS-CoV-2 particle.
J15. The SARS-CoV-2 vaccine according to any one of the preceding aspects J, wherein surface proteins in the inactivated SARS-CoV-2 particle comprise reduced modifications compared to viral RNA in the inactivated SARS-CoV-2 particle, preferably wherein surface proteins comprise a reduced proportion of modified residues compared to viral RNA in the inactivated SARS-CoV-2 particle; said modifications being with respect to a native SARS-CoV-2 particles, preferably wherein said modifications comprise alkylated and/or acylated nucleotide or amino acid residues.
J16. The SARS-CoV-2 vaccine according to any one of the preceding aspects J, wherein the inactivated SARS-CoV-2 particle comprises a native conformation of (i) spike (S) protein; (ii) nucleocapsid (N) protein; (iii) membrane (M) glycoprotein; and/or (iv) envelope (E) protein; preferably wherein the inactivated SARS-CoV-2 particle comprises a native conformation spike (S) protein.
J17. A SARS-CoV-2 vaccine according to any preceding aspect J, wherein the inactivated SARS-CoV-2 particle comprises one or more beta-propiolactone-modified cysteine, methionine and/or histidine residues.
J18. A SARS-CoV-2 vaccine according to any preceding aspect J, wherein an inactivated SARS-CoV-2 particle comprises fewer than 200, 100, 50, 30, 20, 15, 10, 9, 8, 7 or 6 beta-propiolactone-modified amino acid residues; preferably wherein a spike (S) protein of the inactivated SARS-CoV-2 particle comprises fewer than 100, 50, 30, 20, 15, 10, 9, 8, 7 or 6 beta-propiolactone-modified amino acid residues; more preferably wherein the inactivated SARS-CoV-2 particle or spike protein thereof comprises 20 or fewer beta-propiolactone-modified amino acid residues; most preferably wherein the inactivated SARS-CoV-2 particle or spike protein thereof comprises 1 to 100, 2 to 50, 3 to 30, 10 to 20 or about 15 beta-propiolactone-modified amino acid residues.
J19. A SARS-CoV-2 vaccine according to any preceding aspect J, wherein fewer than 20%, 15%, 10%, 5% or 4% of SARS-CoV-2 polypeptides in the particle are beta-propiolactone-modified; preferably wherein 0.1 to 10%, more preferably 1 to 5%, more preferably 2 to 8% or about 3-6% of SARS-CoV-2 polypeptides in the particle, comprise at least one beta-propiolactone modification; preferably as detected in the vaccine by mass spectroscopy, optionally following enzymatic digestion with trypsin, chymotrypsin and/or PNGase F or acid hydrolysis.
J20. A SARS-CoV-2 vaccine according to any preceding aspect J, wherein:
   (i) a spike (S) protein of the inactivated SARS-CoV-2 particle comprises a beta-propiolactone modification at one or more of the following residues: 49, 146, 166, 177, 207, 245, 379, 432, 519, 625, 1029, 1032, 1058, 1083, 1088, 1101, 1159 and/or 1271; preferably H49, H146, C166, M177, H207, H245, C432, H519, H625, M1029, H1058, H1083, H1088, H1101, H1159 and/or H1271; or H207, H245, C379, M1029 and/or C1032, e.g. in SEQ ID NO:3, or a corresponding position in SEQ ID NO: 19, 21, 23, 25 or 27; and/or
   (ii) a membrane (M) glycoprotein of the inactivated SARS-CoV-2 particle comprises a beta-propiolactone modification at one or more of the following residues: 125, 154, 155, 159 and/or 210; preferably H154, H155, C159 and/or H210, e.g. in SEQ ID NO: 29;
   (iii) a nucleocapsid (N) protein of the inactivated SARS-CoV-2 particle comprises a beta-propiolactone modification at M234, e.g. in SEQ ID NO: 28.
J21. A SARS-CoV-2 vaccine according to any preceding aspect J, wherein fewer than 30%, 20%, 10%, 5%, 3% or 1% of one or more of the following residues, preferably of at least 2, 3, 4, 5, 6, 7, 8, 9, 10, 11, 12, 13, 14, 15, 16, 17, 18, 19 or all of the following residues, in the inactivated SARS-CoV-2 particles are beta-propiolactone modified:
   (i) in the spike (S) protein, residues 49, 146, 166, 177, 207, 245, 379, 432, 519, 625, 1029, 1032, 1058, 1083, 1088, 1101, 1159 and/or 1271; preferably H49, H146, C166, M177, H207, H245, C432, H519, H625, M1029, H1058, H1083, H1088, H1101, H1159 and/or H1271; or H207, H245, C379, M1029 and/or C1032; e.g. in SEQ ID NO: 3, or a corresponding position in SEQ ID NO: 19, 21, 23, 25 or 27; and/or (ii) in the membrane (M) glycoprotein, residues 125, 154, 155, 159 and/or 210; preferably H154, H155, C159 and/or H210; e.g. in SEQ ID NO: 29; and/or
   (iii) M234 of the nucleocapsid (N) protein, e.g. in SEQ ID NO: 28.
J22. A SARS-CoV-2 vaccine according to any preceding aspect J, wherein the proportion of beta-propiolactone-modified residues at each of the following positions in the inactivated SARS-CoV-2 particles is:
   (i) in the spike (S) protein (e.g. of SEQ ID NO: 3, or a corresponding position in SEQ ID NO: 19, 21, 23, 25 or 27):
      (a) residues H49, H146, C166, H207, H519, M1029, H1083, H1088, H1101, H1159 and/or H1271: less than 20%, preferably 0.01 to 10%, more preferably 0.1 to 5%; and/or
      (b) residues M177, C432, H625: less than 30%, preferably 0.1 to 20%, more preferably 1 to 10%; and/or
      (c) residues H245, H1058: less than 30%, preferably 0.1 to 20%, more preferably 5 to 15%;
   (ii) in the membrane (M) glycoprotein (e.g. of SEQ ID NO: 29):
      (f) H154: less than 5%, less than 1% or less than 0.1%; and/or
      (g) H155: less than 10%, preferably 0.1 to 5%; and/or
      (h) C159: less than 5%, less than 1% or less than 0.1%; and/or
      (i) H210: less than 20%, preferably 0.1 to 10%; and/or
   (iii) in the nucleocapsid (N) protein (e.g. of SEQ ID NO: 28):
      (j) M234: less than 90%, less than 10% or less than 0.1%.
J23. The SARS-CoV-2 vaccine according to any one of the preceding aspects J, wherein infectivity of mammalian cells by the inactivated SARS-CoV-2 particle is reduced by at least 99%, 99.99% or 99.9999% compared with a native SARS-CoV-2 particle, or wherein infectivity of mammalian cells by the inactivated SARS-CoV-2 particle is undetectable.
J24. A SARS-CoV-2 vaccine according to any one of the preceding aspects J, further comprising one or more pharmaceutically acceptable excipients.
J25. The SARS-CoV-2 vaccine according to any one of the preceding aspects J, wherein the SARS-CoV-2 particle comprises an RNA sequence (and/or fragments thereof, optionally comprising modified (preferably alkylated or acylated) nucleotide residues) corresponding to a DNA sequence (i) as defined by SEQ ID NO: 9; or (ii) having at least 80%, at least 85%, at least 90%, at least 95% or at least 99% sequence identity to SEQ ID NO: 9; preferably wherein a native (non-inactivated) SARS-CoV-2 particle comprising the RNA sequence is able to pack a virulent SARS-CoV-2.
J26. The SARS-CoV-2 vaccine according to any one of the preceding aspects J, wherein the said vaccine comprises an additional SARS-CoV-2 particle that comprises an RNA sequence (and/or fragments thereof, optionally comprising modified (preferably alkylated or acylated) nucleotide residues) corresponding to a DNA sequence (i) as defined by SEQ ID NO: 18; or (ii) having at least 80%, at least 85%, at least 90%, at least 95% or at least 99% sequence identity to SEQ ID NO: 18; preferably wherein a native (non-inactivated) SARS-CoV-2 particle comprising the RNA sequence is able to pack a virulent SARS-CoV-2.
J27. The SARS-CoV-2 vaccine according to any one of the preceding aspects J, wherein the said vaccine comprises an additional SARS-CoV-2 particle that comprises an RNA sequence (and/or fragments thereof, optionally comprising modified (preferably alkylated or acylated) nucleotide residues) corresponding to a DNA sequence (i) as defined by SEQ ID NO: 22; or (ii) having at least 80%, at least 85%, at least 90%, at least 95% or at least 99% sequence identity to SEQ ID NO: 22; preferably wherein a native (non-inactivated) SARS-CoV-2 particle comprising the RNA sequence is able to pack a virulent SARS-CoV-2.
J28. The SARS-CoV-2 vaccine according to any one of the preceding aspects J, wherein the CpG ODN is CpG 1018, comprising the sequence 5' TGACTGTGAACGTTCGAGATGA 3' as defined by SEQ ID NO: 4.
J29. The SARS-CoV-2 vaccine according to any one of the preceding aspects J, wherein the alum adjuvant comprises aluminium hydroxide.
J30. The SARS-CoV-2 vaccine according to any one of the preceding aspects J, wherein the vaccine is able to seroconvert a subject that is administered the SARS-CoV vaccine with at least a 70% probability.
J31. The SARS-CoV-2 vaccine according to any one of the preceding aspects J, wherein the vaccine is able to seroconvert a subject that is administered the SARS-CoV vaccine with at least 90% probability.
J32. The SARS-CoV-2 vaccine according to aspect J30, wherein the SARS-CoV-2 vaccine is able to serocovert the subject that is administered the SARS-CoV-2 vaccine with at least 80%, 85%, 90%, or 95% probability.
J33. The SARS-CoV-2 vaccine according to any one of the preceding aspects J, wherein the vaccine is obtained or obtainable from Vero cells.
J34. The SARS-CoV-2 vaccine according to any one of the preceding aspects J, wherein on administration to a human subject the vaccine (i) does not induce antibody-dependent enhancement (ADE) of SARS-CoV-2-associated disease (COVID-19), and/or (ii) does not induce immunopathology in the subject.
J35. A method of preventing or treating SARS-CoV-2 infection and/or SARS-CoV-2-associated disease (COVID-19) in a human subject in need thereof, comprising administering a prophylactically or therapeutically effective amount of the SARS-CoV-2 vaccine of any preceding aspect Jto the subject.
36. The method according to aspect J35, further comprising administering a second dose of a prophylactically or therapeutically effective amount of the SARS-CoV-2 vaccine, preferably wherein the second dose of the vaccine is the same formulation as the first.
J37. The method according to aspect J35 or 36, wherein said prophylactically or therapeutically effective amount of the SARS-CoV-2 vaccine per dose is defined as about 1 to 100 AU/dose, preferably between about 2 to 75 AU/dose, preferably between about 3 and 60 AU/dose, more preferably between about 3 and 55 AU/dose, more preferably between about 3 and 53 AU/dose, as assessed by ELISA, even more preferably between about 3 and 40 AU/dose, more preferably about 10 to 60 AU/dose, 20 to 50 AU/dose, 25 to 45 AU/dose or 30 to 40 AU/dose such as e.g. 35 AU/dose or 40 AU/dose.
J38. The method according to any one of aspects J35 to 37, wherein said prophylactically or therapeutically effective amount of the SARS-CoV-2 vaccine per dose is defined as about 0.05 to 50 µg total protein, about 0.1 to 25 µg, about 0.25 to 12.5 µg, preferably about 0.5 to 5 µg total protein, more preferably at least 2.5 µg total protein, at least 3.5 µg total protein or at least 2.5 µg total protein, even more preferably 2.5 µg to 25 µg, 3.5 µg to 10 µg or 4 µg to 6 µg total protein/dose, most preferably about 5 µg total protein/dose, e.g. as measured by (µ)BCA.
J39. The method according to any one of aspects J35 to 38, wherein said prophylactically or therapeutically effective amount of the SARS-CoV-2 vaccine is defined as about 0.025 to 25 µg S-protein, about 0.05 to 12.5 µg, about 0.125 to 6.25 µg, preferably about 0.25 to 2.5 µg S-protein, as measured by ELISA.
J40. The method according to any one of aspects J35 to 39, wherein the second dose of the SARS-CoV-2 vaccine is administered about 7 days, about 14 days, about 21 days or about 28 days after a first dose of the SARS-CoV-2 vaccine, preferably about 21 days.
J41. The method according to any one of aspects J35 to 40, wherein the administering results in production of SARS-CoV-2 neutralizing antibodies.
J42. A method of producing a SARS-CoV-2 vaccine, comprising:
   (a) producing native SARS-CoV-2 particles;
   (b) inactivating the native SARS-CoV-2 particles to obtain inactivated SARS-CoV-2 particles;
   (c) incorporating the inactivated SARS-CoV-2 particles in a vaccine composition comprising a CpG-containing oligodeoxynucleotide (CpG ODN) and an alum adjuvant.
J43. The method according to aspect J42, wherein the CpG-ODN is CpG 1018 and the alum adjuvant is aluminium hydroxide.
J44. The method according to aspect J43, wherein the SARS-CoV-2 vaccine comprising aluminium hydroxide contains less than 1.25 ppb Cu.
J45. The method according to any of aspects J42 to 44, wherein a native surface conformation of the SARS-CoV-2 particle is preserved in the inactivation step, such that the vaccine is capable of generating neutralizing antibodies against native SARS-CoV-2 particles in a human subject.
J46. The method according to aspect J45, wherein the inactivation step preferentially targets viral RNA in the inactivated SARS-CoV-2 particle.
J47. The method according to any one of aspects J42 to 4046 wherein the inactivation step comprises (i) alkylating and/or acylating viral RNA (ii) modifying purine (preferably guanine) residues or introducing strand breaks into viral RNA and/or (iii) cross-linking viral RNA with one or more viral proteins.
J48. The method according to any of aspects J42 to 47, wherein the inactivation step comprises treating the native SARS-CoV-2 particles with beta-propiolactone.
J49. The method according to aspect J48, wherein the concentration of beta-propiolactone in the inactivation step is 0.01 to 1% by weight, preferably 0.05 to 0.5% by weight, more preferably about 0.1% by weight, even more preferably between 300 to 700ppm, most preferred around 500ppm at 2°C to 8°C for at least 5 hours, at least 10 hours, at least 24 hours, followed optionally by a hydrolyzation for 2.5 hours ± 0.5 hours at 35°C to 39°C, preferably around 37°C.
J50. The method according to aspect J48 or 49, wherein the native SARS-CoV-2 particles are contacted with beta-propiolactone for at least 5 hours, at least 10 hours, at least 24 hours.
J51. The method according to any one of aspects J42 to 50, wherein the inactivation step is performed at about 0°C to about 25°C, preferably about 4°C or about 8°C.
J52. The method according to any of aspects J42 to 51, wherein the inactivation step comprises treating the native SARS-CoV-2 particles with ultraviolet (UV) light.
J53. The method according to any of aspects J42 to 52, wherein step (a) comprises one or more of the following steps:
   (i) passaging a SARS-CoV-2 on Vero cells, thereby producing a culture medium comprising the SARS-CoV-2;
   (ii) harvesting the culture medium of (i);
   (iii) precipitating the harvested culture medium of (ii), thereby producing native SARS-CoV-2 particles in a supernatant.
J54. The method according to aspect J53, further comprising concentrating the culture medium of step (ii) prior to step (iii).
J55. The method according to aspect J53 or 54, wherein precipitation step (iii) comprises contacting the culture medium of step (ii) with protamine sulfate and/or benzonase.
J56. The method according to any of aspects J42 to 55, further comprising dialyzing the inactivated SARS-CoV-2 particles, thereby producing a dialyzed SARS-CoV-2.
J57. The method according to aspect J56, further comprising filtering the dialyzed SARS-CoV-2.
J58. The method according to any of aspects J42 to 57, wherein the inactivation step comprises contacting a liquid composition comprising native SARS-CoV-2 particles with a chemical viral inactivating agent in a container, mixing the chemical viral inactivating agent and the liquid composition comprising SARS-CoV-2 particles under conditions of laminar flow but not turbulent flow, and incubating the chemical viral inactivating agent and the liquid composition comprising SARS-CoV-2 particles for a time sufficient to inactivate the viral particles.
J59. The method according to aspect J58, wherein the inactivation step is performed in a flexible bioreactor bag.
J60. The method according to aspect J58 or 59, wherein the inactivation step comprises five or less container inversions during the period of inactivation.
J61. The method according to any one of aspects J58 to 60, wherein the mixing of the chemical viral inactivating agent and the composition comprising native SARS-CoV-2 particles comprises subjecting the container to rocking, rotation, orbital shaking, or oscillation for not more than 10 minutes at not more than 10 rpm during the period of incubation.
J62. The method according to any one of aspects J42 to 61, further comprising purifying the inactivated SARS-CoV-2 particles by one or more methods selected from (i) batch chromatography and/or (ii) sucrose density gradient centrifugation.
J63. The method according to any one of aspects J42 to 62, wherein said SARS-CoV-2 particle comprises an RNA sequence corresponding to a DNA sequence (i) as defined by SEQ ID NO: 9; or (ii) having at least 80%, at least 85%, at least 90%, at least 95% or at least 99% sequence identity to SEQ ID NO: 9; preferably wherein a native (non-inactivated) SARS-CoV-2 particle comprising the RNA sequence is able to pack a virulent SARS-CoV-2.
J64. The method according to any one of aspects J42 to 63, wherein said SARS-CoV-2 virus comprises an S protein comprising or consisting of (i) an amino acid sequence as defined by SEQ ID NO: 11, or (ii) an amino acid sequence having at least 95%, at least 97% or at least 99% identity to SEQ ID NO: 11.
J65. The method according to any one of aspects J42 to 64, wherein said SARS-CoV-2 virus comprises a polyprotein comprising or consisting of (i) an amino acid sequence as defined by SEQ ID NO: 10, or (ii) an amino acid sequence having at least 95%, at least 97% or at least 99% identity to SEQ ID NO: 10.
J66. A SARS-CoV-2 vaccine obtained or obtainable by the method of any one of aspects J42 to 65.
J67. Use of a SARS-CoV-2 vaccine of any one of aspects J1 to 34 or 66 for the treatment or prevention of a SARS-CoV-2 infection in a subject.
J68. The SARS-CoV-2 vaccine of any one of aspects J1 to 34 or 66 for use as a medicament.
J69. A pharmaceutical composition for use in the prevention or treatment of a SARS-CoV-2 infection in a subject, wherein said pharmaceutical composition is a vaccine of any one of aspects J1 to 34 or 66, optionally in combination with one or more pharmaceutically acceptable excipients.
J70. A vaccine, method, use or pharmaceutical composition according to any preceding aspect J, wherein the subject is (i) an elderly subject, preferably a subject over 65, over 70 or over 80 years of age; (ii) an immunocompromised subject; or (iii) a pregnant subject.
J71. A vaccine, method, use or pharmaceutical composition according to any preceding aspect J, for use in prevention or treatment of a SARS-CoV-2 infection without induction of (i) antibody-dependent enhancement (ADE) of SARS-CoV-2-associated disease (COVID-19); and/or (ii) immunopathology in the subject.

In some specific embodiments, there is provided an aspect of the invention as described herein (e.g. as in one or more of aspects A to H above, or as in the appended claims), wherein one or more of the following aspects (labelled X) is excluded:
X1. An immunogenic composition for stimulating an immune response against a severe acute respiratory syndrome coronavirus 2 (SARS-CoV-2), comprising a SARS-CoV-2 antigen and a toll-like receptor 9 (TLR9) agonist, wherein the TLR9 agonist is an oligonucleotide of from 10 to 35 nucleotides in length comprising an unmethylated cytidine- phospho-guanosine (CpG) motif, and the SARS-CoV-2 antigen and the oligonucleotide are present in the immunogenic composition in amounts effective to stimulate an immune response against the SARS-CoV-2 antigen in a mammalian subject.
X2. The composition of aspect X1, wherein the oligonucleotide comprises the sequence 5'-AACGTTCGAG-3' (SEQ ID NO: 30).
X3. The composition of aspect X1, wherein the oligonucleotide comprises the sequence of 5'-TGACTGTGAACGTTCGAGATGA-3' (SEQ ID NO: 4).
X4. The composition of aspect X1, wherein the oligonucleotide comprises a modified nucleoside, optionally wherein the modified nucleoside is selected from the group consisting of 2'-deoxy-7-deazaguanosine, 2'-deoxy-6-thioguanosine, arabinoguanosine, 2'-deoxy- 2'substituted-arabinoguanosine, and 2'-O-substituted-arabinoguanosine.
X5. The composition of aspect X4, wherein the oligonucleotide comprises the sequence 5'-TCG₁AACG₁TTCG₁-3' (SEQ ID NO: 31) in which G₁ is 2'-deoxy-7-deazaguanosine, optionally wherein the oligonucleotide comprises the sequence 5'-TCG₁AACG₁TTCG₁-X-G₁CTTG₁CAAG₁CT-5' (SEQ ID NO: 32), and in which G₁ is 2'-deoxy-7-deazaguanosine and X is glycerol (5'-SEQ ID NO: 31-3'-X-3'-SEQ ID NO: 31-5').
X6. The composition of any one of aspects X1-5, wherein the oligonucleotide comprises at least one phosphorothioate linkage, optionally wherein all nucleotide linkages are phosphorothioate linkages.
X7. The composition of any one of aspects XI-6, wherein the oligonucleotide is a single-stranded oligodeoxynucleotide.
X8. The composition of any one of aspects X1-7, wherein a 0.5 ml dose of the immunogenic composition comprises from about 750 to about 3000 µg of the oligonucleotide, optionally wherein the immunogenic composition comprises about 750 µg, about 1500 µg, or about 3000 µg of the oligonucleotide.
X9. The composition of any one of aspects X1-8, wherein the SARS-CoV-2 antigen is an inactivated whole SARS-CoV-2.
X10. The composition of aspect X9, wherein the SARS-CoV-2 is inactivated by treatment with one or both of formalin and ultraviolet light.
X11. The composition of any one of aspects X1-8, wherein the SARS-CoV-2 antigen comprises the receptor-binding domain (RBD) of the SARS-CoV-2 spike (S) protein.
X12. The composition of aspect X11, wherein the SARS-CoV-2 antigen comprises a truncated, recombinant S protein devoid of signal peptide, transmembrane and cytoplasmic domains of a full length S protein.
X13. The composition of aspect X11 or X12, wherein the SARS-CoV-2 antigen further comprises one or more of the SARS-CoV-2 membrane (M) protein, nucleocapsid (N) protein, and envelope (E) protein.
X14. The composition of any one of aspects X1-13, further comprising an aluminum salt adjuvant.
X15. The composition of aspect X14, wherein the aluminum salt adjuvant comprises one or more of the group consisting of amorphous aluminum hydroxyphosphate sulfate, aluminum hydroxide, aluminum phosphate, and potassium aluminum sulfate
X16. The composition of aspect X14, wherein the aluminum salt adjuvant comprises aluminum hydroxide.
X17. The composition of any one of aspects X14-16, wherein a 0.5 ml dose of the immunogenic composition comprises from about 0.25 to about 0.50 mg A13+.
X18. The composition of any one of aspects X1-17, wherein the mammalian subject is a human subject.
X19. A kit comprising:
   i) the immunogenic composition of any one of aspects X1-18, and
   ii) instructions for administration of the composition to stimulate an immune response against the SARS-CoV-2 antigen in the mammalian subject.
X20. The kit of aspect X19, further comprising iii) a syringe and needle for intramuscular injection of the immunogenic composition.
X21. A method for stimulating an immune response against a severe acute respiratory syndrome coronavirus 2 (SARS-CoV-2) in a mammalian subject, comprising administering the immunogenic composition of any one of aspects X1-18 to a mammalian subject so as to stimulate an immune response against the SARS-CoV-2 antigen in the mammalian subject.
X22. The method of aspect X21, wherein the immunogenic composition is administered by intramuscular injection.

The present application claims priority from US 62/983,737 (1-Mar-2020), EP20168324.0 (06 Apr 2020), EP20202124.2 (15 Oct 2020), EP20211936.8 (04 Dec 2020) EP21154645.2 (01 Feb 2021), PCT/US21/20313 (1-Mar-2021) and EP21160933.4 (05 Mar 2021), the contents of which are incorporated herein by reference. All publications mentioned in the above specification are herein incorporated by reference. Various modifications and variations of the described embodiments of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

### SEQUENCES

SEQ ID NO: 1
   Severe acute respiratory syndrome coronavirus 2 (SARS-CoV-2) isolate Wuhan-Hu-1, complete genome (GenBank: MN908947; Wu, F., et al. A new coronavirus associated with human respiratory disease in China (2020) Nature 579:265-269) 
ATTAAAGGTTTATACCTTCCCAGGTAACAAACCAACCAACTTTCGATCTCTTGTAGATCTGTTCTCTAAACGAACTTTAAAATCTGTGTGG
CTGTCACTCGGCTGCATGCTTAGTGCACTCACGCAGTATAATTAATAACTAATTACTGTCGTTGACAGGACACGAGTAACTCGTCTATCTT
CTGCAGGCTGCTTACGGTTTCGTCCGTGTTGCAGCCGATCATCAGCACATCTAGGTTTCGTCCGGGTGTGACCGAAAGGTAAGATGGAG
AGCCTTGTCCCTGGTTTCAACGAGAAAACACACGTCCAACTCAGTTTGCCTGTTTTACAGGTTCGCGACGTGCTCGTACGTGGCTTTGGA
GACTCCGTGGAGGAGGTCTTATCAGAGGCACGTCAACATCTTAAAGATGGCACTTGTGGCTTAGTAGAAGTTGAAAAAGGCGTTTTGCC
TCAACTTGAACAGCCCTATGTGTTCATCAAACGTTCGGATGCTCGAACTGCACCTCATGGTCATGTTATGGTTGAGCTGGTAGCAGAACT
CGAAGGCATTCAGTACGGTCGTAGTGGTGAGACACTTGGTGTCCTTGTCCCTCATGTGGGCGAAATACCAGTGGCTTACCGCAAGGTTC
TTCTTCGTAAGAACGGTAATAAAGGAGCTGGTGGCCATAGTTACGGCGCCGATCTAAAGTCATTTGACTTAGGCGACGAGCTTGGCACT
GATCCTTATGAAGATTTTCAAGAAAACTGGAACACTAAACATAGCAGTGGTGTTACCCGTGAACTCATGCGTGAGCTTAACGGAGGGGC
ATACACTCGCTATGTCGATAACAACTTCTGTGGCCCTGATGGCTACCCTCTTGAGTGCATTAAAGACCTTCTAGCACGTGCTGGTAAAGC
TTCATGCACTTTGTCCGAACAACTGGACTTTATTGACACTAAGAGGGGTGTATACTGCTGCCGTGAACATGAGCATGAAATTGCTTGGTA
CACGGAACGTTCTGAAAAGAGCTATGAATTGCAGACACCTTTTGAAATTAAATTGGCAAAGAAATTTGACACCTTCAATGGGGAATGTC
CAAATTTTGTATTTCCCTTAAATTCCATAATCAAGACTATTCAACCAAGGGTTGAAAAGAAAAAGCTTGATGGCTTTATGGGTAGAATTC
GATCTGTCTATCCAGTTGCGTCACCAAATGAATGCAACCAAATGTGCCTTTCAACTCTCATGAAGTGTGATCATTGTGGTGAAACTTCATG
GCAGACGGGCGATTTTGTTAAAGCCACTTGCGAATTTTGTGGCACTGAGAATTTGACTAAAGAAGGTGCCACTACTTGTGGTTACTTACC
CCAAAATGCTGTTGTTAAAATTTATTGTCCAGCATGTCACAATTCAGAAGTAGGACCTGAGCATAGTCTTGCCGAATACCATAATGAATC
TGGCTTGAAAACCATTCTTCGTAAGGGTGGTCGCACTATTGCCTTTGGAGGCTGTGTGTTCTCTTATGTTGGTTGCCATAACAAGTGTGC
CTATTGGGTTCCACGTGCTAGCGCTAACATAGGTTGTAACCATACAGGTGTTGTTGGAGAAGGTTCCGAAGGTCTTAATGACAACCTTCT
TGAAATACTCCAAAAAGAGAAAGTCAACATCAATATTGTTGGTGACTTTAAACTTAATGAAGAGATCGCCATTATTTTGGCATCTTTTTCT
GCTTCCACAAGTGCTTTTGTGGAAACTGTGAAAGGTTTGGATTATAAAGCATTCAAACAAATTGTTGAATCCTGTGGTAATTTTAAAGTT
ACAAAAGGAAAAGCTAAAAAAGGTGCCTGGAATATTGGTGAACAGAAATCAATACTGAGTCCTCTTTATGCATTTGCATCAGAGGCTGC
TCGTGTTGTACGATCAATTTTCTCCCGCACTCTTGAAACTGCTCAAAATTCTGTGCGTGTTTTACAGAAGGCCGCTATAACAATACTAGAT
GGAATTTCACAGTATTCACTGAGACTCATTGATGCTATGATGTTCACATCTGATTTGGCTACTAACAATCTAGTTGTAATGGCCTACATTA
CAGGTGGTGTTGTTCAGTTGACTTCGCAGTGGCTAACTAACATCTTTGGCACTGTTTATGAAAAACTCAAACCCGTCCTTGATTGGCTTGA
AGAGAAGTTTAAGGAAGGTGTAGAGTTTCTTAGAGACGGTTGGGAAATTGTTAAATTTATCTCAACCTGTGCTTGTGAAATTGTCGGTG
GACAAATTGTCACCTGTGCAAAGGAAATTAAGGAGAGTGTTCAGACATTCTTTAAGCTTGTAAATAAATTTTTGGCTTTGTGTGCTGACT
CTATCATTATTGGTGGAGCTAAACTTAAAGCCTTGAATTTAGGTGAAACATTTGTCACGCACTCAAAGGGATTGTACAGAAAGTGTGTTA
AATCCAGAGAAGAAACTGGCCTACTCATGCCTCTAAAAGCCCCAAAAGAAATTATCTTCTTAGAGGGAGAAACACTTCCCACAGAAGTG
TTAACAGAGGAAGTTGTCTTGAAAACTGGTGATTTACAACCATTAGAACAACCTACTAGTGAAGCTGTTGAAGCTCCATTGGTTGGTACA
CCAGTTTGTATTAACGGGCTTATGTTGCTCGAAATCAAAGACACAGAAAAGTACTGTGCCCTTGCACCTAATATGATGGTAACAAACAAT
ACCTTCACACTCAAAGGCGGTGCACCAACAAAGGTTACTTTTGGTGATGACACTGTGATAGAAGTGCAAGGTTACAAGAGTGTGAATAT
CACTTTTGAACTTGATGAAAGGATTGATAAAGTACTTAATGAGAAGTGCTCTGCCTATACAGTTGAACTCGGTACAGAAGTAAATGAGTT
CGCCTGTGTTGTGGCAGATGCTGTCATAAAAACTTTGCAACCAGTATCTGAATTACTTACACCACTGGGCATTGATTTAGATGAGTGGAG
TATGGCTACATACTACTTATTTGATGAGTCTGGTGAGTTTAAATTGGCTTCACATATGTATTGTTCTTTCTACCCTCCAGATGAGGATGAA
GAAGAAGGTGATTGTGAAGAAGAAGAGTTTGAGCCATCAACTCAATATGAGTATGGTACTGAAGATGATTACCAAGGTAAACCTTTGG
AATTTGGTGCCACTTCTGCTGCTCTTCAACCTGAAGAAGAGCAAGAAGAAGATTGGTTAGATGATGATAGTCAACAAACTGTTGGTCAA
CAAGACGGCAGTGAGGACAATCAGACAACTACTATTCAAACAATTGTTGAGGTTCAACCTCAATTAGAGATGGAACTTACACCAGTTGT
TCAGACTATTGAAGTGAATAGTTTTAGTGGTTATTTAAAACTTACTGACAATGTATACATTAAAAATGCAGACATTGTGGAAGAAGCTAA
AAAGGTAAAACCAACAGTGGTTGTTAATGCAGCCAATGTTTACCTTAAACATGGAGGAGGTGTTGCAGGAGCCTTAAATAAGGCTACTA
ACAATGCCATGCAAGTTGAATCTGATGATTACATAGCTACTAATGGACCACTTAAAGTGGGTGGTAGTTGTGTTTTAAGCGGACACAATC
TTGCTAAACACTGTCTTCATGTTGTCGGCCCAAATGTTAACAAAGGTGAAGACATTCAACTTCTTAAGAGTGCTTATGAAAATTTTAATCA
GCACGAAGTTCTACTTGCACCATTATTATCAGCTGGTATTTTTGGTGCTGACCCTATACATTCTTTAAGAGTTTGTGTAGATACTGTTCGCA
CAAATGTCTACTTAGCTGTCTTTGATAAAAATCTCTATGACAAACTTGTTTCAAGCTTTTTGGAAATGAAGAGTGAAAAGCAAGTTGAAC
AAAAGATCGCTGAGATTCCTAAAGAGGAAGTTAAGCCATTTATAACTGAAAGTAAACCTTCAGTTGAACAGAGAAAACAAGATGATAAG
AAAATCAAAGCTTGTGTTGAAGAAGTTACAACAACTCTGGAAGAAACTAAGTTCCTCACAGAAAACTTGTTACTTTATATTGACATTAAT
GGCAATCTTCATCCAGATTCTGCCACTCTTGTTAGTGACATTGACATCACTTTCTTAAAGAAAGATGCTCCATATATAGTGGGTGATGTTG
TTCAAGAGGGTGTTTTAACTGCTGTGGTTATACCTACTAAAAAGGCTGGTGGCACTACTGAAATGCTAGCGAAAGCTTTGAGAAAAGTG
CCAACAGACAATTATATAACCACTTACCCGGGTCAGGGTTTAAATGGTTACACTGTAGAGGAGGCAAAGACAGTGCTTAAAAAGTGTAA
AAGTGCCTTTTACATTCTACCATCTATTATCTCTAATGAGAAGCAAGAAATTCTTGGAACTGTTTCTTGGAATTTGCGAGAAATGCTTGCA
CATGCAGAAGAAACACGCAAATTAATGCCTGTCTGTGTGGAAACTAAAGCCATAGTTTCAACTATACAGCGTAAATATAAGGGTATTAA
AATACAAGAGGGTGTGGTTGATTATGGTGCTAGATTTTACTTTTACACCAGTAAAACAACTGTAGCGTCACTTATCAACACACTTAACGA
TCTAAATGAAACTCTTGTTACAATGCCACTTGGCTATGTAACACATGGCTTAAATTTGGAAGAAGCTGCTCGGTATATGAGATCTCTCAA
AGTGCCAGCTACAGTTTCTGTTTCTTCACCTGATGCTGTTACAGCGTATAATGGTTATCTTACTTCTTCTTCTAAAACACCTGAAGAACATT
TTATTGAAACCATCTCACTTGCTGGTTCCTATAAAGATTGGTCCTATTCTGGACAATCTACACAACTAGGTATAGAATTTCTTAAGAGAGG
TGATAAAAGTGTATATTACACTAGTAATCCTACCACATTCCACCTAGATGGTGAAGTTATCACCTTTGACAATCTTAAGACACTTCTTTCTT
TGAGAGAAGTGAGGACTATTAAGGTGTTTACAACAGTAGACAACATTAACCTCCACACGCAAGTTGTGGACATGTCAATGACATATGGA
 
CAACAGTTTGGTCCAACTTATTTGGATGGAGCTGATGTTACTAAAATAAAACCTCATAATTCACATGAAGGTAAAACATTTTATGTTTTAC
CTAATGATGACACTCTACGTGTTGAGGCTTTTGAGTACTACCACACAACTGATCCTAGTTTTCTGGGTAGGTACATGTCAGCATTAAATCA
CACTAAAAAGTGGAAATACCCACAAGTTAATGGTTTAACTTCTATTAAATGGGCAGATAACAACTGTTATCTTGCCACTGCATTGTTAACA
CTCCAACAAATAGAGTTGAAGTTTAATCCACCTGCTCTACAAGATGCTTATTACAGAGCAAGGGCTGGTGAAGCTGCTAACTTTTGTGCA
CTTATCTTAGCCTACTGTAATAAGACAGTAGGTGAGTTAGGTGATGTTAGAGAAACAATGAGTTACTTGTTTCAACATGCCAATTTAGAT
TCTTGCAAAAGAGTCTTGAACGTGGTGTGTAAAACTTGTGGACAACAGCAGACAACCCTTAAGGGTGTAGAAGCTGTTATGTACATGGG
CACACTTTCTTATGAACAATTTAAGAAAGGTGTTCAGATACCTTGTACGTGTGGTAAACAAGCTACAAAATATCTAGTACAACAGGAGTC
ACCTTTTGTTATGATGTCAGCACCACCTGCTCAGTATGAACTTAAGCATGGTACATTTACTTGTGCTAGTGAGTACACTGGTAATTACCAG
TGTGGTCACTATAAACATATAACTTCTAAAGAAACTTTGTATTGCATAGACGGTGCTTTACTTACAAAGTCCTCAGAATACAAAGGTCCTA
TTACGGATGTTTTCTACAAAGAAAACAGTTACACAACAACCATAAAACCAGTTACTTATAAATTGGATGGTGTTGTTTGTACAGAAATTG
ACCCTAAGTTGGACAATTATTATAAGAAAGACAATTCTTATTTCACAGAGCAACCAATTGATCTTGTACCAAACCAACCATATCCAAACGC
AAGCTTCGATAATTTTAAGTTTGTATGTGATAATATCAAATTTGCTGATGATTTAAACCAGTTAACTGGTTATAAGAAACCTGCTTCAAGA
GAGCTTAAAGTTACATTTTTCCCTGACTTAAATGGTGATGTGGTGGCTATTGATTATAAACACTACACACCCTCTTTTAAGAAAGGAGCTA
AATTGTTACATAAACCTATTGTTTGGCATGTTAACAATGCAACTAATAAAGCCACGTATAAACCAAATACCTGGTGTATACGTTGTCTTTG
GAGCACAAAACCAGTTGAAACATCAAATTCGTTTGATGTACTGAAGTCAGAGGACGCGCAGGGAATGGATAATCTTGCCTGCGAAGAT
CTAAAACCAGTCTCTGAAGAAGTAGTGGAAAATCCTACCATACAGAAAGACGTTCTTGAGTGTAATGTGAAAACTACCGAAGTTGTAGG
AGACATTATACTTAAACCAGCAAATAATAGTTTAAAAATTACAGAAGAGGTTGGCCACACAGATCTAATGGCTGCTTATGTAGACAATTC
TAGTCTTACTATTAAGAAACCTAATGAATTATCTAGAGTATTAGGTTTGAAAACCCTTGCTACTCATGGTTTAGCTGCTGTTAATAGTGTC
CCTTGGGATACTATAGCTAATTATGCTAAGCCTTTTCTTAACAAAGTTGTTAGTACAACTACTAACATAGTTACACGGTGTTTAAACCGTG
TTTGTACTAATTATATGCCTTATTTCTTTACTTTATTGCTACAATTGTGTACTTTTACTAGAAGTACAAATTCTAGAATTAAAGCATCTATGC
CGACTACTATAGCAAAGAATACTGTTAAGAGTGTCGGTAAATTTTGTCTAGAGGCTTCATTTAATTATTTGAAGTCACCTAATTTTTCTAA
ACTGATAAATATTATAATTTGGTTTTTACTATTAAGTGTTTGCCTAGGTTCTTTAATCTACTCAACCGCTGCTTTAGGTGTTTTAATGTCTAA
TTTAGGCATGCCTTCTTACTGTACTGGTTACAGAGAAGGCTATTTGAACTCTACTAATGTCACTATTGCAACCTACTGTACTGGTTCTATAC
CTTGTAGTGTTTGTCTTAGTGGTTTAGATTCTTTAGACACCTATCCTTCTTTAGAAACTATACAAATTACCATTTCATCTTTTAAATGGGATT
TAACTGCTTTTGGCTTAGTTGCAGAGTGGTTTTTGGCATATATTCTTTTCACTAGGTTTTTCTATGTACTTGGATTGGCTGCAATCATGCAA
TTGTTTTTCAGCTATTTTGCAGTACATTTTATTAGTAATTCTTGGCTTATGTGGTTAATAATTAATCTTGTACAAATGGCCCCGATTTCAGCT
ATGGTTAGAATGTACATCTTCTTTGCATCATTTTATTATGTATGGAAAAGTTATGTGCATGTTGTAGACGGTTGTAATTCATCAACTTGTAT
GATGTGTTACAAACGTAATAGAGCAACAAGAGTCGAATGTACAACTATTGTTAATGGTGTTAGAAGGTCCTTTTATGTCTATGCTAATGG
AGGTAAAGGCTTTTGCAAACTACACAATTGGAATTGTGTTAATTGTGATACATTCTGTGCTGGTAGTACATTTATTAGTGATGAAGTTGC
GAGAGACTTGTCACTACAGTTTAAAAGACCAATAAATCCTACTGACCAGTCTTCTTACATCGTTGATAGTGTTACAGTGAAGAATGGTTC
CATCCATCTTTACTTTGATAAAGCTGGTCAAAAGACTTATGAAAGACATTCTCTCTCTCATTTTGTTAACTTAGACAACCTGAGAGCTAAT
AACACTAAAGGTTCATTGCCTATTAATGTTATAGTTTTTGATGGTAAATCAAAATGTGAAGAATCATCTGCAAAATCAGCGTCTGTTTACT
ACAGTCAGCTTATGTGTCAACCTATACTGTTACTAGATCAGGCATTAGTGTCTGATGTTGGTGATAGTGCGGAAGTTGCAGTTAAAATGT
TTGATGCTTACGTTAATACGTTTTCATCAACTTTTAACGTACCAATGGAAAAACTCAAAACACTAGTTGCAACTGCAGAAGCTGAACTTGC
AAAGAATGTGTCCTTAGACAATGTCTTATCTACTTTTATTTCAGCAGCTCGGCAAGGGTTTGTTGATTCAGATGTAGAAACTAAAGATGTT
GTTGAATGTCTTAAATTGTCACATCAATCTGACATAGAAGTTACTGGCGATAGTTGTAATAACTATATGCTCACCTATAACAAAGTTGAAA
ACATGACACCCCGTGACCTTGGTGCTTGTATTGACTGTAGTGCGCGTCATATTAATGCGCAGGTAGCAAAAAGTCACAACATTGCTTTGA
TATGGAACGTTAAAGATTTCATGTCATTGTCTGAACAACTACGAAAACAAATACGTAGTGCTGCTAAAAAGAATAACTTACCTTTTAAGT
TGACATGTGCAACTACTAGACAAGTTGTTAATGTTGTAACAACAAAGATAGCACTTAAGGGTGGTAAAATTGTTAATAATTGGTTGAAG
CAGTTAATTAAAGTTACACTTGTGTTCCTTTTTGTTGCTGCTATTTTCTATTTAATAACACCTGTTCATGTCATGTCTAAACATACTGACTTT
TCAAGTGAAATCATAGGATACAAGGCTATTGATGGTGGTGTCACTCGTGACATAGCATCTACAGATACTTGTTTTGCTAACAAACATGCT
GATTTTGACACATGGTTTAGCCAGCGTGGTGGTAGTTATACTAATGACAAAGCTTGCCCATTGATTGCTGCAGTCATAACAAGAGAAGT
GGGTTTTGTCGTGCCTGGTTTGCCTGGCACGATATTACGCACAACTAATGGTGACTTTTTGCATTTCTTACCTAGAGTTTTTAGTGCAGTT
GGTAACATCTGTTACACACCATCAAAACTTATAGAGTACACTGACTTTGCAACATCAGCTTGTGTTTTGGCTGCTGAATGTACAATTTTTA
AAGATGCTTCTGGTAAGCCAGTACCATATTGTTATGATACCAATGTACTAGAAGGTTCTGTTGCTTATGAAAGTTTACGCCCTGACACAC
GTTATGTGCTCATGGATGGCTCTATTATTCAATTTCCTAACACCTACCTTGAAGGTTCTGTTAGAGTGGTAACAACTTTTGATTCTGAGTA
CTGTAGGCACGGCACTTGTGAAAGATCAGAAGCTGGTGTTTGTGTATCTACTAGTGGTAGATGGGTACTTAACAATGATTATTACAGAT
CTTTACCAGGAGTTTTCTGTGGTGTAGATGCTGTAAATTTACTTACTAATATGTTTACACCACTAATTCAACCTATTGGTGCTTTGGACATA
TCAGCATCTATAGTAGCTGGTGGTATTGTAGCTATCGTAGTAACATGCCTTGCCTACTATTTTATGAGGTTTAGAAGAGCTTTTGGTGAAT
ACAGTCATGTAGTTGCCTTTAATACTTTACTATTCCTTATGTCATTCACTGTACTCTGTTTAACACCAGTTTACTCATTCTTACCTGGTGTTT
ATTCTGTTATTTACTTGTACTTGACATTTTATCTTACTAATGATGTTTCTTTTTTAGCACATATTCAGTGGATGGTTATGTTCACACCTTTAG
TACCTTTCTGGATAACAATTGCTTATATCATTTGTATTTCCACAAAGCATTTCTATTGGTTCTTTAGTAATTACCTAAAGAGACGTGTAGTC
TTTAATGGTGTTTCCTTTAGTACTTTTGAAGAAGCTGCGCTGTGCACCTTTTTGTTAAATAAAGAAATGTATCTAAAGTTGCGTAGTGATG
TGCTATTACCTCTTACGCAATATAATAGATACTTAGCTCTTTATAATAAGTACAAGTATTTTAGTGGAGCAATGGATACAACTAGCTACAG
AGAAGCTGCTTGTTGTCATCTCGCAAAGGCTCTCAATGACTTCAGTAACTCAGGTTCTGATGTTCTTTACCAACCACCACAAACCTCTATC
ACCTCAGCTGTTTTGCAGAGTGGTTTTAGAAAAATGGCATTCCCATCTGGTAAAGTTGAGGGTTGTATGGTACAAGTAACTTGTGGTACA
ACTACACTTAACGGTCTTTGGCTTGATGACGTAGTTTACTGTCCAAGACATGTGATCTGCACCTCTGAAGACATGCTTAACCCTAATTATG
AAGATTTACTCATTCGTAAGTCTAATCATAATTTCTTGGTACAGGCTGGTAATGTTCAACTCAGGGTTATTGGACATTCTATGCAAAATTG
TGTACTTAAGCTTAAGGTTGATACAGCCAATCCTAAGACACCTAAGTATAAGTTTGTTCGCATTCAACCAGGACAGACTTTTTCAGTGTTA
GCTTGTTACAATGGTTCACCATCTGGTGTTTACCAATGTGCTATGAGGCCCAATTTCACTATTAAGGGTTCATTCCTTAATGGTTCATGTG
GTAGTGTTGGTTTTAACATAGATTATGACTGTGTCTCTTTTTGTTACATGCACCATATGGAATTACCAACTGGAGTTCATGCTGGCACAGA
CTTAGAAGGTAACTTTTATGGACCTTTTGTTGACAGGCAAACAGCACAAGCAGCTGGTACGGACACAACTATTACAGTTAATGTTTTAGC
 
TTGGTTGTACGCTGCTGTTATAAATGGAGACAGGTGGTTTCTCAATCGATTTACCACAACTCTTAATGACTTTAACCTTGTGGCTATGAAG
TACAATTATGAACCTCTAACACAAGACCATGTTGACATACTAGGACCTCTTTCTGCTCAAACTGGAATTGCCGTTTTAGATATGTGTGCTT
CATTAAAAGAATTACTGCAAAATGGTATGAATGGACGTACCATATTGGGTAGTGCTTTATTAGAAGATGAATTTACACCTTTTGATGTTG
TTAGACAATGCTCAGGTGTTACTTTCCAAAGTGCAGTGAAAAGAACAATCAAGGGTACACACCACTGGTTGTTACTCACAATTTTGACTT
CACTTTTAGTTTTAGTCCAGAGTACTCAATGGTCTTTGTTCTTTTTTTTGTATGAAAATGCCTTTTTACCTTTTGCTATGGGTATTATTGCTA
TGTCTGCTTTTGCAATGATGTTTGTCAAACATAAGCATGCATTTCTCTGTTTGTTTTTGTTACCTTCTCTTGCCACTGTAGCTTATTTTAATA
TGGTCTATATGCCTGCTAGTTGGGTGATGCGTATTATGACATGGTTGGATATGGTTGATACTAGTTTGTCTGGTTTTAAGCTAAAAGACT
GTGTTATGTATGCATCAGCTGTAGTGTTACTAATCCTTATGACAGCAAGAACTGTGTATGATGATGGTGCTAGGAGAGTGTGGACACTT
ATGAATGTCTTGACACTCGTTTATAAAGTTTATTATGGTAATGCTTTAGATCAAGCCATTTCCATGTGGGCTCTTATAATCTCTGTTACTTC
TAACTACTCAGGTGTAGTTACAACTGTCATGTTTTTGGCCAGAGGTATTGTTTTTATGTGTGTTGAGTATTGCCCTATTTTCTTCATAACTG
GTAATACACTTCAGTGTATAATGCTAGTTTATTGTTTCTTAGGCTATTTTTGTACTTGTTACTTTGGCCTCTTTTGTTTACTCAACCGCTACT
TTAGACTGACTCTTGGTGTTTATGATTACTTAGTTTCTACACAGGAGTTTAGATATATGAATTCACAGGGACTACTCCCACCCAAGAATAG
CATAGATGCCTTCAAACTCAACATTAAATTGTTGGGTGTTGGTGGCAAACCTTGTATCAAAGTAGCCACTGTACAGTCTAAAATGTCAGA
TGTAAAGTGCACATCAGTAGTCTTACTCTCAGTTTTGCAACAACTCAGAGTAGAATCATCATCTAAATTGTGGGCTCAATGTGTCCAGTTA
CACAATGACATTCTCTTAGCTAAAGATACTACTGAAGCCTTTGAAAAAATGGTTTCACTACTTTCTGTTTTGCTTTCCATGCAGGGTGCTG
TAGACATAAACAAGCTTTGTGAAGAAATGCTGGACAACAGGGCAACCTTACAAGCTATAGCCTCAGAGTTTAGTTCCCTTCCATCATATG
CAGCTTTTGCTACTGCTCAAGAAGCTTATGAGCAGGCTGTTGCTAATGGTGATTCTGAAGTTGTTCTTAAAAAGTTGAAGAAGTCTTTGA
ATGTGGCTAAATCTGAATTTGACCGTGATGCAGCCATGCAACGTAAGTTGGAAAAGATGGCTGATCAAGCTATGACCCAAATGTATAAA
CAGGCTAGATCTGAGGACAAGAGGGCAAAAGTTACTAGTGCTATGCAGACAATGCTTTTCACTATGCTTAGAAAGTTGGATAATGATGC
ACTCAACAACATTATCAACAATGCAAGAGATGGTTGTGTTCCCTTGAACATAATACCTCTTACAACAGCAGCCAAACTAATGGTTGTCAT
ACCAGACTATAACACATATAAAAATACGTGTGATGGTACAACATTTACTTATGCATCAGCATTGTGGGAAATCCAACAGGTTGTAGATGC
AGATAGTAAAATTGTTCAACTTAGTGAAATTAGTATGGACAATTCACCTAATTTAGCATGGCCTCTTATTGTAACAGCTTTAAGGGCCAAT
TCTGCTGTCAAATTACAGAATAATGAGCTTAGTCCTGTTGCACTACGACAGATGTCTTGTGCTGCCGGTACTACACAAACTGCTTGCACT
GATGACAATGCGTTAGCTTACTACAACACAACAAAGGGAGGTAGGTTTGTACTTGCACTGTTATCCGATTTACAGGATTTGAAATGGGC
TAGATTCCCTAAGAGTGATGGAACTGGTACTATCTATACAGAACTGGAACCACCTTGTAGGTTTGTTACAGACACACCTAAAGGTCCTAA
AGTGAAGTATTTATACTTTATTAAAGGATTAAACAACCTAAATAGAGGTATGGTACTTGGTAGTTTAGCTGCCACAGTACGTCTACAAGC
TGGTAATGCAACAGAAGTGCCTGCCAATTCAACTGTATTATCTTTCTGTGCTTTTGCTGTAGATGCTGCTAAAGCTTACAAAGATTATCTA
GCTAGTGGGGGACAACCAATCACTAATTGTGTTAAGATGTTGTGTACACACACTGGTACTGGTCAGGCAATAACAGTTACACCGGAAGC
CAATATGGATCAAGAATCCTTTGGTGGTGCATCGTGTTGTCTGTACTGCCGTTGCCACATAGATCATCCAAATCCTAAAGGATTTTGTGA
CTTAAAAGGTAAGTATGTACAAATACCTACAACTTGTGCTAATGACCCTGTGGGTTTTACACTTAAAAACACAGTCTGTACCGTCTGCGG
TATGTGGAAAGGTTATGGCTGTAGTTGTGATCAACTCCGCGAACCCATGCTTCAGTCAGCTGATGCACAATCGTTTTTAAACGGGTTTGC
GGTGTAAGTGCAGCCCGTCTTACACCGTGCGGCACAGGCACTAGTACTGATGTCGTATACAGGGCTTTTGACATCTACAATGATAAAGT
AGCTGGTTTTGCTAAATTCCTAAAAACTAATTGTTGTCGCTTCCAAGAAAAGGACGAAGATGACAATTTAATTGATTCTTACTTTGTAGTT
AAGAGACACACTTTCTCTAACTACCAACATGAAGAAACAATTTATAATTTACTTAAGGATTGTCCAGCTGTTGCTAAACATGACTTCTTTA
AGTTTAGAATAGACGGTGACATGGTACCACATATATCACGTCAACGTCTTACTAAATACACAATGGCAGACCTCGTCTATGCTTTAAGGC
ATTTTGATGAAGGTAATTGTGACACATTAAAAGAAATACTTGTCACATACAATTGTTGTGATGATGATTATTTCAATAAAAAGGACTGGT
ATGATTTTGTAGAAAACCCAGATATATTACGCGTATACGCCAACTTAGGTGAACGTGTACGCCAAGCTTTGTTAAAAACAGTACAATTCT
GTGATGCCATGCGAAATGCTGGTATTGTTGGTGTACTGACATTAGATAATCAAGATCTCAATGGTAACTGGTATGATTTCGGTGATTTCA
TACAAACCACGCCAGGTAGTGGAGTTCCTGTTGTAGATTCTTATTATTCATTGTTAATGCCTATATTAACCTTGACCAGGGCTTTAACTGC
AGAGTCACATGTTGACACTGACTTAACAAAGCCTTACATTAAGTGGGATTTGTTAAAATATGACTTCACGGAAGAGAGGTTAAAACTCTT
TGACCGTTATTTTAAATATTGGGATCAGACATACCACCCAAATTGTGTTAACTGTTTGGATGACAGATGCATTCTGCATTGTGCAAACTTT
AATGTTTTATTCTCTACAGTGTTCCCACCTACAAGTTTTGGACCACTAGTGAGAAAAATATTTGTTGATGGTGTTCCATTTGTAGTTTCAAC
TGGATACCACTTCAGAGAGCTAGGTGTTGTACATAATCAGGATGTAAACTTACATAGCTCTAGACTTAGTTTTAAGGAATTACTTGTGTA
TGCTGCTGACCCTGCTATGCACGCTGCTTCTGGTAATCTATTACTAGATAAACGCACTACGTGCTTTTCAGTAGCTGCACTTACTAACAAT
GTTGCTTTTCAAACTGTCAAACCCGGTAATTTTAACAAAGACTTCTATGACTTTGCTGTGTCTAAGGGTTTCTTTAAGGAAGGAAGTTCTG
TTGAATTAAAACACTTCTTCTTTGCTCAGGATGGTAATGCTGCTATCAGCGATTATGACTACTATCGTTATAATCTACCAACAATGTGTGA
TATCAGACAACTACTATTTGTAGTTGAAGTTGTTGATAAGTACTTTGATTGTTACGATGGTGGCTGTATTAATGCTAACCAAGTCATCGTC
AACAACCTAGACAAATCAGCTGGTTTTCCATTTAATAAATGGGGTAAGGCTAGACTTTATTATGATTCAATGAGTTATGAGGATCAAGAT
GCACTTTTCGCATATACAAAACGTAATGTCATCCCTACTATAACTCAAATGAATCTTAAGTATGCCATTAGTGCAAAGAATAGAGCTCGCA
CCGTAGCTGGTGTCTCTATCTGTAGTACTATGACCAATAGACAGTTTCATCAAAAATTATTGAAATCAATAGCCGCCACTAGAGGAGCTA
CTGTAGTAATTGGAACAAGCAAATTCTATGGTGGTTGGCACAACATGTTAAAAACTGTTTATAGTGATGTAGAAAACCCTCACCTTATGG
GTTGGGATTATCCTAAATGTGATAGAGCCATGCCTAACATGCTTAGAATTATGGCCTCACTTGTTCTTGCTCGCAAACATACAACGTGTTG
TAGCTTGTCACACCGTTTCTATAGATTAGCTAATGAGTGTGCTCAAGTATTGAGTGAAATGGTCATGTGTGGCGGTTCACTATATGTTAA
ACCAGGTGGAACCTCATCAGGAGATGCCACAACTGCTTATGCTAATAGTGTTTTTAACATTTGTCAAGCTGTCACGGCCAATGTTAATGC
ACTTTTATCTACTGATGGTAACAAAATTGCCGATAAGTATGTCCGCAATTTACAACACAGACTTTATGAGTGTCTCTATAGAAATAGAGAT
GTTGACACAGACTTTGTGAATGAGTTTTACGCATATTTGCGTAAACATTTCTCAATGATGATACTCTCTGACGATGCTGTTGTGTGTTTCA
ATAGCACTTATGCATCTCAAGGTCTAGTGGCTAGCATAAAGAACTTTAAGTCAGTTCTTTATTATCAAAACAATGTTTTTATGTCTGAAGC
AAAATGTTGGACTGAGACTGACCTTACTAAAGGACCTCATGAATTTTGCTCTCAACATACAATGCTAGTTAAACAGGGTGATGATTATGT
GTACCTTCCTTACCCAGATCCATCAAGAATCCTAGGGGCCGGCTGTTTTGTAGATGATATCGTAAAAACAGATGGTACACTTATGATTGA
ACGGTTCGTGTCTTTAGCTATAGATGCTTACCCACTTACTAAACATCCTAATCAGGAGTATGCTGATGTCTTTCATTTGTACTTACAATACA
TAAGAAAGCTACATGATGAGTTAACAGGACACATGTTAGACATGTATTCTGTTATGCTTACTAATGATAACACTTCAAGGTATTGGGAAC
CTGAGTTTTATGAGGCTATGTACACACCGCATACAGTCTTACAGGCTGTTGGGGCTTGTGTTCTTTGCAATTCACAGACTTCATTAAGATG
 
TGGTGCTTGCATACGTAGACCATTCTTATGTTGTAAATGCTGTTACGACCATGTCATATCAACATCACATAAATTAGTCTTGTCTGTTAATC
CGTATGTTTGCAATGCTCCAGGTTGTGATGTCACAGATGTGACTCAACTTTACTTAGGAGGTATGAGCTATTATTGTAAATCACATAAAC
CACCCATTAGTTTTCCATTGTGTGCTAATGGACAAGTTTTTGGTTTATATAAAAATACATGTGTTGGTAGCGATAATGTTACTGACTTTAA
TGCAATTGCAACATGTGACTGGACAAATGCTGGTGATTACATTTTAGCTAACACCTGTACTGAAAGACTCAAGCTTTTTGCAGCAGAAAC
GCTCAAAGCTACTGAGGAGACATTTAAACTGTCTTATGGTATTGCTACTGTACGTGAAGTGCTGTCTGACAGAGAATTACATCTTTCATG
GGAAGTTGGTAAACCTAGACCACCACTTAACCGAAATTATGTCTTTACTGGTTATCGTGTAACTAAAAACAGTAAAGTACAAATAGGAG
AGTACACCTTTGAAAAAGGTGACTATGGTGATGCTGTTGTTTACCGAGGTACAACAACTTACAAATTAAATGTTGGTGATTATTTTGTGC
TGACATCACATACAGTAATGCCATTAAGTGCACCTACACTAGTGCCACAAGAGCACTATGTTAGAATTACTGGCTTATACCCAACACTCA
ATATCTCAGATGAGTTTTCTAGCAATGTTGCAAATTATCAAAAGGTTGGTATGCAAAAGTATTCTACACTCCAGGGACCACCTGGTACTG
GTAAGAGTCATTTTGCTATTGGCCTAGCTCTCTACTACCCTTCTGCTCGCATAGTGTATACAGCTTGCTCTCATGCCGCTGTTGATGCACTA
TGTGAGAAGGCATTAAAATATTTGCCTATAGATAAATGTAGTAGAATTATACCTGCACGTGCTCGTGTAGAGTGTTTTGATAAATTCAAA
GTGAATTCAACATTAGAACAGTATGTCTTTTGTACTGTAAATGCATTGCCTGAGACGACAGCAGATATAGTTGTCTTTGATGAAATTTCA
ATGGCCACAAATTATGATTTGAGTGTTGTCAATGCCAGATTACGTGCTAAGCACTATGTGTACATTGGCGACCCTGCTCAATTACCTGCA
CCACGCACATTGCTAACTAAGGGCACACTAGAACCAGAATATTTCAATTCAGTGTGTAGACTTATGAAAACTATAGGTCCAGACATGTTC
CTCGGAACTTGTCGGCGTTGTCCTGCTGAAATTGTTGACACTGTGAGTGCTTTGGTTTATGATAATAAGCTTAAAGCACATAAAGACAAA
TCAGCTCAATGCTTTAAAATGTTTTATAAGGGTGTTATCACGCATGATGTTTCATCTGCAATTAACAGGCCACAAATAGGCGTGGTAAGA
GAATTCCTTACACGTAACCCTGCTTGGAGAAAAGCTGTCTTTATTTCACCTTATAATTCACAGAATGCTGTAGCCTCAAAGATTTTGGGAC
TACCAACTCAAACTGTTGATTCATCACAGGGCTCAGAATATGACTATGTCATATTCACTCAAACCACTGAAACAGCTCACTCTTGTAATGT
AAACAGATTTAATGTTGCTATTACCAGAGCAAAAGTAGGCATACTTTGCATAATGTCTGATAGAGACCTTTATGACAAGTTGCAATTTAC
AAGTCTTGAAATTCCACGTAGGAATGTGGCAACTTTACAAGCTGAAAATGTAACAGGACTCTTTAAAGATTGTAGTAAGGTAATCACTG
GGTTACATCCTACACAGGCACCTACACACCTCAGTGTTGACACTAAATTCAAAACTGAAGGTTTATGTGTTGACATACCTGGCATACCTA
AGGACATGACCTATAGAAGACTCATCTCTATGATGGGTTTTAAAATGAATTATCAAGTTAATGGTTACCCTAACATGTTTATCACCCGCG
AAGAAGCTATAAGACATGTACGTGCATGGATTGGCTTCGATGTCGAGGGGTGTCATGCTACTAGAGAAGCTGTTGGTACCAATTTACCT
TTACAGCTAGGTTTTTCTACAGGTGTTAACCTAGTTGCTGTACCTACAGGTTATGTTGATACACCTAATAATACAGATTTTTCCAGAGTTA
GTGCTAAACCACCGCCTGGAGATCAATTTAAACACCTCATACCACTTATGTACAAAGGACTTCCTTGGAATGTAGTGCGTATAAAGATTG
TACAAATGTTAAGTGACACACTTAAAAATCTCTCTGACAGAGTCGTATTTGTCTTATGGGCACATGGCTTTGAGTTGACATCTATGAAGT
ATTTTGTGAAAATAGGACCTGAGCGCACCTGTTGTCTATGTGATAGACGTGCCACATGCTTTTCCACTGCTTCAGACACTTATGCCTGTTG
GCATCATTCTATTGGATTTGATTACGTCTATAATCCGTTTATGATTGATGTTCAACAATGGGGTTTTACAGGTAACCTACAAAGCAACCAT
GATCTGTATTGTCAAGTCCATGGTAATGCACATGTAGCTAGTTGTGATGCAATCATGACTAGGTGTCTAGCTGTCCACGAGTGCTTTGTT
AAGCGTGTTGACTGGACTATTGAATATCCTATAATTGGTGATGAACTGAAGATTAATGCGGCTTGTAGAAAGGTTCAACACATGGTTGTT
AAAGCTGCATTATTAGCAGACAAATTCCCAGTTCTTCACGACATTGGTAACCCTAAAGCTATTAAGTGTGTACCTCAAGCTGATGTAGAA
TGGAAGTTCTATGATGCACAGCCTTGTAGTGACAAAGCTTATAAAATAGAAGAATTATTCTATTCTTATGCCACACATTCTGACAAATTCA
CAGATGGTGTATGCCTATTTTGGAATTGCAATGTCGATAGATATCCTGCTAATTCCATTGTTTGTAGATTTGACACTAGAGTGCTATCTAA
CCTTAACTTGCCTGGTTGTGATGGTGGCAGTTTGTATGTAAATAAACATGCATTCCACACACCAGCTTTTGATAAAAGTGCTTTTGTTAAT
TTAAAACAATTACCATTTTTCTATTACTCTGACAGTCCATGTGAGTCTCATGGAAAACAAGTAGTGTCAGATATAGATTATGTACCACTAA
AGTCTGCTACGTGTATAACACGTTGCAATTTAGGTGGTGCTGTCTGTAGACATCATGCTAATGAGTACAGATTGTATCTCGATGCTTATA
ACATGATGATCTCAGCTGGCTTTAGCTTGTGGGTTTACAAACAATTTGATACTTATAACCTCTGGAACACTTTTACAAGACTTCAGAGTTT
AGAAAATGTGGCTTTTAATGTTGTAAATAAGGGACACTTTGATGGACAACAGGGTGAAGTACCAGTTTCTATCATTAATAACACTGTTTA
CACAAAAGTTGATGGTGTTGATGTAGAATTGTTTGAAAATAAAACAACATTACCTGTTAATGTAGCATTTGAGCTTTGGGCTAAGCGCAA
CATTAAACCAGTACCAGAGGTGAAAATACTCAATAATTTGGGTGTGGACATTGCTGCTAATACTGTGATCTGGGACTACAAAAGAGATG
CTCCAGCACATATATCTACTATTGGTGTTTGTTCTATGACTGACATAGCCAAGAAACCAACTGAAACGATTTGTGCACCACTCACTGTCTT
TTTTGATGGTAGAGTTGATGGTCAAGTAGACTTATTTAGAAATGCCCGTAATGGTGTTCTTATTACAGAAGGTAGTGTTAAAGGTTTACA
ACCATCTGTAGGTCCCAAACAAGCTAGTCTTAATGGAGTCACATTAATTGGAGAAGCCGTAAAAACACAGTTCAATTATTATAAGAAAGT
TGATGGTGTTGTCCAACAATTACCTGAAACTTACTTTACTCAGAGTAGAAATTTACAAGAATTTAAACCCAGGAGTCAAATGGAAATTGA
TTTCTTAGAATTAGCTATGGATGAATTCATTGAACGGTATAAATTAGAAGGCTATGCCTTCGAACATATCGTTTATGGAGATTTTAGTCAT
AGTCAGTTAGGTGGTTTACATCTACTGATTGGACTAGCTAAACGTTTTAAGGAATCACCTTTTGAATTAGAAGATTTTATTCCTATGGACA
GTACAGTTAAAAACTATTTCATAACAGATGCGCAAACAGGTTCATCTAAGTGTGTGTGTTCTGTTATTGATTTATTACTTGATGATTTTGTT
GAAATAATAAAATCCCAAGATTTATCTGTAGTTTCTAAGGTTGTCAAAGTGACTATTGACTATACAGAAATTTCATTTATGCTTTGGTGTA
AAGATGGCCATGTAGAAACATTTTACCCAAAATTACAATCTAGTCAAGCGTGGCAACCGGGTGTTGCTATGCCTAATCTTTACAAAATGC
AAAGAATGCTATTAGAAAAGTGTGACCTTCAAAATTATGGTGATAGTGCAACATTACCTAAAGGCATAATGATGAATGTCGCAAAATAT
ACTCAACTGTGTCAATATTTAAACACATTAACATTAGCTGTACCCTATAATATGAGAGTTATACATTTTGGTGCTGGTTCTGATAAAGGAG
TTGCACCAGGTACAGCTGTTTTAAGACAGTGGTTGCCTACGGGTACGCTGCTTGTCGATTCAGATCTTAATGACTTTGTCTCTGATGCAG
ATTCAACTTTGATTGGTGATTGTGCAACTGTACATACAGCTAATAAATGGGATCTCATTATTAGTGATATGTACGACCCTAAGACTAAAA
ATGTTACAAAAGAAAATGACTCTAAAGAGGGTTTTTTCACTTACATTTGTGGGTTTATACAACAAAAGCTAGCTCTTGGAGGTTCCGTGG
CTATAAAGATAACAGAACATTCTTGGAATGCTGATCTTTATAAGCTCATGGGACACTTCGCATGGTGGACAGCCTTTGTTACTAATGTGA
ATGCGTCATCATCTGAAGCATTTTTAATTGGATGTAATTATCTTGGCAAACCACGCGAACAAATAGATGGTTATGTCATGCATGCAAATT
ACATATTTTGGAGGAATACAAATCCAATTCAGTTGTCTTCCTATTCTTTATTTGACATGAGTAAATTTCCCCTTAAATTAAGGGGTACTGCT
GTTATGTCTTTAAAAGAAGGTCAAATCAATGATATGATTTTATCTCTTCTTAGTAAAGGTAGACTTATAATTAGAGAAAACAACAGAGTT
GTTATTTCTAGTGATGTTCTTGTTAACAACTAAACGAACAATGTTTGTTTTTCTTGTTTTATTGCCACTAGTCTCTAGTCAGTGTGTTAATCT
TACAACCAGAACTCAATTACCCCCTGCATACACTAATTCTTTCACACGTGGTGTTTATTACCCTGACAAAGTTTTCAGATCCTCAGTTTTAC
ATTCAACTCAGGACTTGTTCTTACCTTTCTTTTCCAATGTTACTTGGTTCCATGCTATACATGTCTCTGGGACCAATGGTACTAAGAGGTTT
GATAACCCTGTCCTACCATTTAATGATGGTGTTTATTTTGCTTCCACTGAGAAGTCTAACATAATAAGAGGCTGGATTTTTGGTACTACTT
 
TAGATTCGAAGACCCAGTCCCTACTTATTGTTAATAACGCTACTAATGTTGTTATTAAAGTCTGTGAATTTCAATTTTGTAATGATCCATTT
TTGGGTGTTTATTACCACAAAAACAACAAAAGTTGGATGGAAAGTGAGTTCAGAGTTTATTCTAGTGCGAATAATTGCACTTTTGAATAT
GTCTCTCAGCCTTTTCTTATGGACCTTGAAGGAAAACAGGGTAATTTCAAAAATCTTAGGGAATTTGTGTTTAAGAATATTGATGGTTATT
TTAAAATATATTCTAAGCACACGCCTATTAATTTAGTGCGTGATCTCCCTCAGGGTTTTTCGGCTTTAGAACCATTGGTAGATTTGCCAAT
AGGTATTAACATCACTAGGTTTCAAACTTTACTTGCTTTACATAGAAGTTATTTGACTCCTGGTGATTCTTCTTCAGGTTGGACAGCTGGT
GCTGCAGCTTATTATGTGGGTTATCTTCAACCTAGGACTTTTCTATTAAAATATAATGAAAATGGAACCATTACAGATGCTGTAGACTGTG
CACTTGACCCTCTCTCAGAAACAAAGTGTACGTTGAAATCCTTCACTGTAGAAAAAGGAATCTATCAAACTTCTAACTTTAGAGTCCAACC
AACAGAATCTATTGTTAGATTTCCTAATATTACAAACTTGTGCCCTTTTGGTGAAGTTTTTAACGCCACCAGATTTGCATCTGTTTATGCTT
GGAACAGGAAGAGAATCAGCAACTGTGTTGCTGATTATTCTGTCCTATATAATTCCGCATCATTTTCCACTTTTAAGTGTTATGGAGTGTC
TCCTACTAAATTAAATGATCTCTGCTTTACTAATGTCTATGCAGATTCATTTGTAATTAGAGGTGATGAAGTCAGACAAATCGCTCCAGGG
CAAACTGGAAAGATTGCTGATTATAATTATAAATTACCAGATGATTTTACAGGCTGCGTTATAGCTTGGAATTCTAACAATCTTGATTCTA
AGGTTGGTGGTAATTATAATTACCTGTATAGATTGTTTAGGAAGTCTAATCTCAAACCTTTTGAGAGAGATATTTCAACTGAAATCTATCA
GGCCGGTAGCACACCTTGTAATGGTGTTGAAGGTTTTAATTGTTACTTTCCTTTACAATCATATGGTTTCCAACCCACTAATGGTGTTGGT
TACCAACCATACAGAGTAGTAGTACTTTCTTTTGAACTTCTACATGCACCAGCAACTGTTTGTGGACCTAAAAAGTCTACTAATTTGGTTA
AAAACAAATGTGTCAATTTCAACTTCAATGGTTTAACAGGCACAGGTGTTCTTACTGAGTCTAACAAAAAGTTTCTGCCTTTCCAACAATT
TGGCAGAGACATTGCTGACACTACTGATGCTGTCCGTGATCCACAGACACTTGAGATTCTTGACATTACACCATGTTCTTTTGGTGGTGTC
AGTGTTATAACACCAGGAACAAATACTTCTAACCAGGTTGCTGTTCTTTATCAGGATGTTAACTGCACAGAAGTCCCTGTTGCTATTCATG
CAGATCAACTTACTCCTACTTGGCGTGTTTATTCTACAGGTTCTAATGTTTTTCAAACACGTGCAGGCTGTTTAATAGGGGCTGAACATGT
CAACAACTCATATGAGTGTGACATACCCATTGGTGCAGGTATATGCGCTAGTTATCAGACTCAGACTAATTCTCCTCGGCGGGCACGTAG
TGTAGCTAGTCAATCCATCATTGCCTACACTATGTCACTTGGTGCAGAAAATTCAGTTGCTTACTCTAATAACTCTATTGCCATACCCACAA
ATTTTACTATTAGTGTTACCACAGAAATTCTACCAGTGTCTATGACCAAGACATCAGTAGATTGTACAATGTACATTTGTGGTGATTCAAC
TGAATGCAGCAATCTTTTGTTGCAATATGGCAGTTTTTGTACACAATTAAACCGTGCTTTAACTGGAATAGCTGTTGAACAAGACAAAAA
CACCCAAGAAGTTTTTGCACAAGTCAAACAAATTTACAAAACACCACCAATTAAAGATTTTGGTGGTTTTAATTTTTCACAAATATTACCA
GATCCATCAAAACCAAGCAAGAGGTCATTTATTGAAGATCTACTTTTCAACAAAGTGACACTTGCAGATGCTGGCTTCATCAAACAATAT
GGTGATTGCCTTGGTGATATTGCTGCTAGAGACCTCATTTGTGCACAAAAGTTTAACGGCCTTACTGTTTTGCCACCTTTGCTCACAGATG
AAATGATTGCTCAATACACTTCTGCACTGTTAGCGGGTACAATCACTTCTGGTTGGACCTTTGGTGCAGGTGCTGCATTACAAATACCATT
TGCTATGCAAATGGCTTATAGGTTTAATGGTATTGGAGTTACACAGAATGTTCTCTATGAGAACCAAAAATTGATTGCCAACCAATTTAA
TAGTGCTATTGGCAAAATTCAAGACTCACTTTCTTCCACAGCAAGTGCACTTGGAAAACTTCAAGATGTGGTCAACCAAAATGCACAAGC
TTTAAACACGCTTGTTAAACAACTTAGCTCCAATTTTGGTGCAATTTCAAGTGTTTTAAATGATATCCTTTCACGTCTTGACAAAGTTGAG
GCTGAAGTGCAAATTGATAGGTTGATCACAGGCAGACTTCAAAGTTTGCAGACATATGTGACTCAACAATTAATTAGAGCTGCAGAAAT
CAGAGCTTCTGCTAATCTTGCTGCTACTAAAATGTCAGAGTGTGTACTTGGACAATCAAAAAGAGTTGATTTTTGTGGAAAGGGCTATCA
TCTTATGTCCTTCCCTCAGTCAGCACCTCATGGTGTAGTCTTCTTGCATGTGACTTATGTCCCTGCACAAGAAAAGAACTTCACAACTGCTC
CTGCCATTTGTCATGATGGAAAAGCACACTTTCCTCGTGAAGGTGTCTTTGTTTCAAATGGCACACACTGGTTTGTAACACAAAGGAATT
TTTATGAACCACAAATCATTACTACAGACAACACATTTGTGTCTGGTAACTGTGATGTTGTAATAGGAATTGTCAACAACACAGTTTATGA
TCCTTTGCAACCTGAATTAGACTCATTCAAGGAGGAGTTAGATAAATATTTTAAGAATCATACATCACCAGATGTTGATTTAGGTGACATC
TCTGGCATTAATGCTTCAGTTGTAAACATTCAAAAAGAAATTGACCGCCTCAATGAGGTTGCCAAGAATTTAAATGAATCTCTCATCGATC
TCCAAGAACTTGGAAAGTATGAGCAGTATATAAAATGGCCATGGTACATTTGGCTAGGTTTTATAGCTGGCTTGATTGCCATAGTAATG
GTGACAATTATGCTTTGCTGTATGACCAGTTGCTGTAGTTGTCTCAAGGGCTGTTGTTCTTGTGGATCCTGCTGCAAATTTGATGAAGAC
GACTCTGAGCCAGTGCTCAAAGGAGTCAAATTACATTACACATAAACGAACTTATGGATTTGTTTATGAGAATCTTCACAATTGGAACTG
TAACTTTGAAGCAAGGTGAAATCAAGGATGCTACTCCTTCAGATTTTGTTCGCGCTACTGCAACGATACCGATACAAGCCTCACTCCCTTT
CGGATGGCTTATTGTTGGCGTTGCACTTCTTGCTGTTTTTCAGAGCGCTTCCAAAATCATAACCCTCAAAAAGAGATGGCAACTAGCACT
CTCCAAGGGTGTTCACTTTGTTTGCAACTTGCTGTTGTTGTTTGTAACAGTTTACTCACACCTTTTGCTCGTTGCTGCTGGCCTTGAAGCCC
CTTTTCTCTATCTTTATGCTTTAGTCTACTTCTTGCAGAGTATAAACTTTGTAAGAATAATAATGAGGCTTTGGCTTTGCTGGAAATGCCGT
TCCAAAAACCCATTACTTTATGATGCCAACTATTTTCTTTGCTGGCATACTAATTGTTACGACTATTGTATACCTTACAATAGTGTAACTTCT
TCAATTGTCATTACTTCAGGTGATGGCACAACAAGTCCTATTTCTGAACATGACTACCAGATTGGTGGTTATACTGAAAAATGGGAATCT
GGAGTAAAAGACTGTGTTGTATTACACAGTTACTTCACTTCAGACTATTACCAGCTGTACTCAACTCAATTGAGTACAGACACTGGTGTT
GAACATGTTACCTTCTTCATCTACAATAAAATTGTTGATGAGCCTGAAGAACATGTCCAAATTCACACAATCGACGGTTCATCCGGAGTT
GTTAATCCAGTAATGGAACCAATTTATGATGAACCGACGACGACTACTAGCGTGCCTTTGTAAGCACAAGCTGATGAGTACGAACTTAT
GTACTCATTCGTTTCGGAAGAGACAGGTACGTTAATAGTTAATAGCGTACTTCTTTTTCTTGCTTTCGTGGTATTCTTGCTAGTTACACTAG
CCATCCTTACTGCGCTTCGATTGTGTGCGTACTGCTGCAATATTGTTAACGTGAGTCTTGTAAAACCTTCTTTTTACGTTTACTCTCGTGTT
AAAAATCTGAATTCTTCTAGAGTTCCTGATCTTCTGGTCTAAACGAACTAAATATTATATTAGTTTTTCTGTTTGGAACTTTAATTTTAGCC
ATGGCAGATTCCAACGGTACTATTACCGTTGAAGAGCTTAAAAAGCTCCTTGAACAATGGAACCTAGTAATAGGTTTCCTATTCCTTACA
TGGATTTGTCTTCTACAATTTGCCTATGCCAACAGGAATAGGTTTTTGTATATAATTAAGTTAATTTTCCTCTGGCTGTTATGGCCAGTAAC
TTTAGCTTGTTTTGTGCTTGCTGCTGTTTACAGAATAAATTGGATCACCGGTGGAATTGCTATCGCAATGGCTTGTCTTGTAGGCTTGATG
TGGCTCAGCTACTTCATTGCTTCTTTCAGACTGTTTGCGCGTACGCGTTCCATGTGGTCATTCAATCCAGAAACTAACATTCTTCTCAACGT
GCCACTCCATGGCACTATTCTGACCAGACCGCTTCTAGAAAGTGAACTCGTAATCGGAGCTGTGATCCTTCGTGGACATCTTCGTATTGC
TGGACACCATCTAGGACGCTGTGACATCAAGGACCTGCCTAAAGAAATCACTGTTGCTACATCACGAACGCTTTCTTATTACAAATTGGG
AGCTTCGCAGCGTGTAGCAGGTGACTCAGGTTTTGCTGCATACAGTCGCTACAGGATTGGCAACTATAAATTAAACACAGACCATTCCA
GTAGCAGTGACAATATTGCTTTGCTTGTACAGTAAGTGACAACAGATGTTTCATCTCGTTGACTTTCAGGTTACTATAGCAGAGATATTAC
TAATTATTATGAGGACTTTTAAAGTTTCCATTTGGAATCTTGATTACATCATAAACCTCATAATTAAAAATTTATCTAAGTCACTAACTGAG
AATAAATATTCTCAATTAGATGAAGAGCAACCAATGGAGATTGATTAAACGAACATGAAAATTATTCTTTTCTTGGCACTGATAACACTC
GCTACTTGTGAGCTTTATCACTACCAAGAGTGTGTTAGAGGTACAACAGTACTTTTAAAAGAACCTTGCTCTTCTGGAACATACGAGGGC
 
AATTCACCATTTCATCCTCTAGCTGATAACAAATTTGCACTGACTTGCTTTAGCACTCAATTTGCTTTTGCTTGTCCTGACGGCGTAAAACA
CGTCTATCAGTTACGTGCCAGATCAGTTTCACCTAAACTGTTCATCAGACAAGAGGAAGTTCAAGAACTTTACTCTCCAATTTTTCTTATT
GTTGCGGCAATAGTGTTTATAACACTTTGCTTCACACTCAAAAGAAAGACAGAATGATTGAACTTTCATTAATTGACTTCTATTTGTGCTT
TTTAGCCTTTCTGCTATTCCTTGTTTTAATTATGCTTATTATCTTTTGGTTCTCACTTGAACTGCAAGATCATAATGAAACTTGTCACGCCTA
AACGAACATGAAATTTCTTGTTTTCTTAGGAATCATCACAACTGTAGCTGCATTTCACCAAGAATGTAGTTTACAGTCATGTACTCAACAT
CAACCATATGTAGTTGATGACCCGTGTCCTATTCACTTCTATTCTAAATGGTATATTAGAGTAGGAGCTAGAAAATCAGCACCTTTAATTG
AATTGTGCGTGGATGAGGCTGGTTCTAAATCACCCATTCAGTACATCGATATCGGTAATTATACAGTTTCCTGTTTACCTTTTACAATTAA
TTGCCAGGAACCTAAATTGGGTAGTCTTGTAGTGCGTTGTTCGTTCTATGAAGACTTTTTAGAGTATCATGACGTTCGTGTTGTTTTAGAT
TTCATCTAAACGAACAAACTAAAATGTCTGATAATGGACCCCAAAATCAGCGAAATGCACCCCGCATTACGTTTGGTGGACCCTCAGATT
CAACTGGCAGTAACCAGAATGGAGAACGCAGTGGGGCGCGATCAAAACAACGTCGGCCCCAAGGTTTACCCAATAATACTGCGTCTTG
GTTCACCGCTCTCACTCAACATGGCAAGGAAGACCTTAAATTCCCTCGAGGACAAGGCGTTCCAATTAACACCAATAGCAGTCCAGATGA
CCAAATTGGCTACTACCGAAGAGCTACCAGACGAATTCGTGGTGGTGACGGTAAAATGAAAGATCTCAGTCCAAGATGGTATTTCTACT
ACCTAGGAACTGGGCCAGAAGCTGGACTTCCCTATGGTGCTAACAAAGACGGCATCATATGGGTTGCAACTGAGGGAGCCTTGAATAC
ACCAAAAGATCACATTGGCACCCGCAATCCTGCTAACAATGCTGCAATCGTGCTACAACTTCCTCAAGGAACAACATTGCCAAAAGGCTT
CTACGCAGAAGGGAGCAGAGGCGGCAGTCAAGCCTCTTCTCGTTCCTCATCACGTAGTCGCAACAGTTCAAGAAATTCAACTCCAGGCA
GCAGTAGGGGAACTTCTCCTGCTAGAATGGCTGGCAATGGCGGTGATGCTGCTCTTGCTTTGCTGCTGCTTGACAGATTGAACCAGCTT
GAGAGCAAAATGTCTGGTAAAGGCCAACAACAACAAGGCCAAACTGTCACTAAGAAATCTGCTGCTGAGGCTTCTAAGAAGCCTCGGC
AAAAACGTACTGCCACTAAAGCATACAATGTAACACAAGCTTTCGGCAGACGTGGTCCAGAACAAACCCAAGGAAATTTTGGGGACCA
GGAACTAATCAGACAAGGAACTGATTACAAACATTGGCCGCAAATTGCACAATTTGCCCCCAGCGCTTCAGCGTTCTTCGGAATGTCGC
GCATTGGCATGGAAGTCACACCTTCGGGAACGTGGTTGACCTACACAGGTGCCATCAAATTGGATGACAAAGATCCAAATTTCAAAGAT
CAAGTCATTTTGCTGAATAAGCATATTGACGCATACAAAACATTCCCACCAACAGAGCCTAAAAAGGACAAAAAGAAGAAGGCTGATGA
AACTCAAGCCTTACCGCAGAGACAGAAGAAACAGCAAACTGTGACTCTTCTTCCTGCTGCAGATTTGGATGATTTCTCCAAACAATTGCA
ACAATCCATGAGCAGTGCTGACTCAACTCAGGCCTAAACTCATGCAGACCACACAAGGCAGATGGGCTATATAAACGTTTTCGCTTTTCC
GTTTACGATATATAGTCTACTCTTGTGCAGAATGAATTCTCGTAACTACATAGCACAAGTAGATGTAGTTAACTTTAATCTCACATAGCAA
TCTTTAATCAGTGTGTAACATTAGGGAGGACTTGAAAGAGCCACCACATTTTCACCGAGGCCACGCGGAGTACGATCGAGTGTACAGTG
AACAATGCTAGGGAGAGCTGCCTATATGGAAGAGCCCTAATGTGTAAAATTAATTTTAGTAGTGCTATCCCCATGTGATTTTAATAGCTT
CTTAGGAGAATGACAAAAAAAAAAAAAAAAAAAAAAAAAAAAAAAAA
SEQ ID NO: 2
   Severe acute respiratory syndrome coronavirus 2 orflab polyprotein of isolate Wuhan-Hu-1 (GenBank: QHD43415)
SEQ ID NO: 3
   Severe acute respiratory syndrome coronavirus 2 surface glycoprotein (GenBank: QHD43416)
SEQ ID NO: 4
   CpG 1018
   TGACTGTGAACGTTCGAGATGA
SEQ ID NO: 5
   KLK peptide
   KLKLLLLLKLK
SEQ ID NO: 6
   Oligo-d(IC)₁₃ (ODN1a)
   ICICICICICICICICICICICICIC
SEQ ID NO: 7
   CpG 1826
   TCCATGACGTTCCTGACGTT
SEQ ID NO: 8
   CpG 7909
   TCGTCGTTTTGTCGTTTTGTCGTT
SEQ ID NO: 9
   >hCoV-19/ltaly/INMIl-isl/20201 EPI_ISL_41054512020-01-29 (Accession No: MT066156) 
ATTAAAGGTTTATACCTTCCCAGGTAACAAACCAACCAACTTTCGATCTCTTGTAGATCTGTTCTCTAAACGAACTTTAAAATCTGTGTGG
CTGTCACTCGGCTGCATGCTTAGTGCACTCACGCAGTATAATTAATAACTAATTACTGTCGTTGACAGGACACGAGTAACTCGTCTATCTT
CTGCAGGCTGCTTACGGTTTCGTCCGTGTTGCAGCCGATCATCAGCACATCTAGGTTTCGTCCGGGTGTGACCGAAAGGTAAGATGGAG
AGCCTTGTCCCTGGTTTCAACGAGAAAACACACGTCCAACTCAGTTTGCCTGTTTTACAGGTTCGCGACGTGCTCGTACGTGGCTTTGGA
GACTCCGTGGAGGAGGTCTTATCAGAGGCACGTCAACATCTTAAAGATGGCACTTGTGGCTTAGTAGAAGTTGAAAAAGGCGTTTTGCC
TCAACTTGAACAGCCCTATGTGTTCATCAAACGTTCGGATGCTCGAACTGCACCTCATGGTCATGTTATGGTTGAGCTGGTAGCAGAACT
CGAAGGCATTCAGTACGGTCGTAGTGGTGAGACACTTGGTGTCCTTGTCCCTCATGTGGGCGAAATACCAGTGGCTTACCGCAAGGTTC
TTCTTCGTAAGAACGGTAATAAAGGAGCTGGTGGCCATAGTTACGGCGCCGATCTAAAGTCATTTGACTTAGGCGACGAGCTTGGCACT
GATCCTTATGAAGATTTTCAAGAAAACTGGAACACTAAACATAGCAGTGGTGTTACCCGTGAACTCATGCGTGAGCTTAACGGAGGGGC
ATACACTCGCTATGTCGATAACAACTTCTGTGGCCCTGATGGCTACCCTCTTGAGTGCATTAAAGACCTTCTAGCACGTGCTGGTAAAGC
TTCATGCACTTTGTCCGAACAACTGGACTTTATTGACACTAAGAGGGGTGTATACTGCTGCCGTGAACATGAGCATGAAATTGCTTGGTA
CACGGAACGTTCTGAAAAGAGCTATGAATTGCAGACACCTTTTGAAATTAAATTGGCAAAGAAATTTGACACCTTCAATGGGGAATGTC
CAAATTTTGTATTTCCCTTAAATTCCATAATCAAGACTATTCAACCAAGGGTTGAAAAGAAAAAGCTTGATGGCTTTATGGGTAGAATTC
GATCTGTCTATCCAGTTGCGTCACCAAATGAATGCAACCAAATGTGCCTTTCAACTCTCATGAAGTGTGATCATTGTGGTGAAACTTCATG
GCAGACGGGCGATTTTGTTAAAGCCACTTGCGAATTTTGTGGCACTGAGAATTTGACTAAAGAAGGTGCCACTACTTGTGGTTACTTACC
CCAAAATGCTGTTGTTAAAATTTATTGTCCAGCATGTCACAATTCAGAAGTAGGACCTGAGCATAGTCTTGCCGAATACCATAATGAATC
TGGCTTGAAAACCATTCTTCGTAAGGGTGGTCGCACTATTGCCTTTGGAGGCTGTGTGTTCTCTTATGTTGGTTGCCATAACAAGTGTGC
CTATTGGGTTCCACGTGCTAGCGCTAACATAGGTTGTAACCATACAGGTGTTGTTGGAGAAGGTTCCGAAGGTCTTAATGACAACCTTCT
TGAAATACTCCAAAAAGAGAAAGTCAACATCAATATTGTTGGTGACTTTAAACTTAATGAAGAGATCGCCATTATTTTGGCATCTTTTTCT
GCTTCCACAAGTGCTTTTGTGGAAACTGTGAAAGGTTTGGATTATAAAGCATTCAAACAAATTGTTGAATCCTGTGGTAATTTTAAAGTT
ACAAAAGGAAAAGCTAAAAAAGGTGCCTGGAATATTGGTGAACAGAAATCAATACTGAGTCCTCTTTATGCATTTGCATCAGAGGCTGC
TCGTGTTGTACGATCAATTTTCTCCCGCACTCTTGAAACTGCTCAAAATTCTGTGCGTGTTTTACAGAAGGCCGCTATAACAATACTAGAT
GGAATTTCACAGTATTCACTGAGACTCATTGATGCTATGATGTTCACATCTGATTTGGCTACTAACAATCTAGTTGTAATGGCCTACATTA
CAGGTGGTGTTGTTCAGTTGACTTCGCAGTGGCTAACTAACATCTTTGGCACTGTTTATGAAAAACTCAAACCCGTCCTTGATTGGCTTGA
AGAGAAGTTTAAGGAAGGTGTAGAGTTTCTTAGAGACGGTTGGGAAATTGTTAAATTTATCTCAACCTGTGCTTGTGAAATTGTCGGTG
GACAAATTGTCACCTGTGCTAAGGAAATTAAGGAGAGTGTTCAGACATTCTTTAAGCTTGTAAATAAATTTTTGGCTTTGTGTGCTGACT
CTATCATTATTGGTGGAGCTAAACTTAAAGCCTTGAATTTAGGTGAAACATTTGTCACGCACTCAAAGGGATTGTACAGAAAGTGTGTTA
AATCCAGAGAAGAAACTGGCCTACTCATGCCTCTAAAAGCCCCAAAAGAAATTATCTTCTTAGAGGGAGAAACACTTCCCACAGAAGTG
TTAACAGAGGAAGTTGTCTTGAAAACTGGTGATTTACAACCATTAGAACAACCTACTAGTGAAGCTGTTGAAGCTCCATTGGTTGGTACA
CCAGTTTGTATTAACGGGCTTATGTTGCTCGAAATCAAAGACACAGAAAAGTACTGTGCCCTTGCACCTAATATGATGGTAACAAACAAT
ACCTTCACACTCAAAGGCGGTGCACCAACAAAGGTTACTTTTGGTGATGACACTGTGATAGAAGTGCAAGGTTACAAGAGTGTGAATAT
CACTTTTGAACTTGATGAAAGGATTGATAAAGTACTTAATGAGAAGTGCTCTGCCTATACAGTTGAACTCGGTACAGAAGTAAATGAGTT
CGCCTGTGTTGTGGCAGATGCTGTCATAAAAACTTTGCAACCAGTATCTGAATTACTTACACCACTGGGCATTGATTTAGATGAGTGGAG
TATGGCTACATACTACTTATTTGATGAGTCTGGTGAGTTTAAATTGGCTTCACATATGTATTGTTCTTTCTACCCTCCAGATGAGGATGAA
GAAGAAGGTGATTGTGAAGAAGAAGAGTTTGAGCCATCAACTCAATATGAGTATGGTACTGAAGATGATTACCAAGGTAAACCTTTGG
 
AATTTGGTGCCACTTCTGCTGCTCTTCAACCTGAAGAAGAGCAAGAAGAAGATTGGTTAGATGATGATAGTCAACAAACTGTTGGTCAA
CAAGACGGCAGTGAGGACAATCAGACAACTACTATTCAAACAATTGTTGAGGTTCAACCTCAATTAGAGATGGAACTTACACCAGTTGT
TCAGACTATTGAAGTGAATAGTTTTAGTGGTTATTTAAAACTTACTGACAATGTATACATTAAAAATGCAGACATTGTGGAAGAAGCTAA
AAAGGTAAAACCAACAGTGGTTGTTAATGCAGCCAATGTTTACCTTAAACATGGAGGAGGTGTTGCAGGAGCCTTAAATAAGGCTACTA
ACAATGCCATGCAAGTTGAATCTGATGATTACATAGCTACTAATGGACCACTTAAAGTGGGTGGTAGTTGTGTTTTAAGCGGACACAATC
TTGCTAAACACTGTCTTCATGTTGTCGGCCCAAATGTTAACAAAGGTGAAGACATTCAACTTCTTAAGAGTGCTTATGAAAATTTTAATCA
GCACGAAGTTCTACTTGCACCATTATTATCAGCTGGTATTTTTGGTGCTGACCCTATACATTCTTTAAGAGTTTGTGTAGATACTGTTCGCA
CAAATGTCTACTTAGCTGTCTTTGATAAAAATCTCTATGACAAACTTGTTTCAAGCTTTTTGGAAATGAAGAGTGAAAAGCAAGTTGAAC
AAAAGATCGCTGAGATTCCTAAAGAGGAAGTTAAGCCATTTATAACTGAAAGTAAACCTTCAGTTGAACAGAGAAAACAAGATGATAAG
AAAATCAAAGCTTGTGTTGAAGAAGTTACAACAACTCTGGAAGAAACTAAGTTCCTCACAGAAAACTTGTTACTTTATATTGACATTAAT
GGCAATCTTCATCCAGATTCTGCCACTCTTGTTAGTGACATTGACATCACTTTCTTAAAGAAAGATGCTCCATATATAGTGGGTGATGTTG
TTCAAGAGGGTGTTTTAACTGCTGTGGTTATACCTACTAAAAAGGCTGGTGGCACTACTGAAATGCTAGCGAAAGCTTTGAGAAAAGTG
CCAACAGACAATTATATAACCACTTACCCGGGTCAGGGTTTAAATGGTTACACTGTAGAGGAGGCAAAGACAGTGCTTAAAAAGTGTAA
AAGTGCCTTTTACATTCTACCATCTATTATCTCTAATGAGAAGCAAGAAATTCTTGGAACTGTTTCTTGGAATTTGCGAGAAATGCTTGCA
CATGCAGAAGAAACACGCAAATTAATGCCTGTCTGTGTGGAAACTAAAGCCATAGTTTCAACTATACAGCGTAAATATAAGGGTATTAA
AATACAAGAGGGTGTGGTTGATTATGGTGCTAGATTTTACTTTTACACCAGTAAAACAACTGTAGCGTCACTTATCAACACACTTAACGA
TCTAAATGAAACTCTTGTTACAATGCCACTTGGCTATGTAACACATGGCTTAAATTTGGAAGAAGCTGCTCGGTATATGAGATCTCTCAA
AGTGCCAGCTACAGTTTCTGTTTCTTCACCTGATGCTGTTACAGCGTATAATGGTTATCTTACTTCTTCTTCTAAAACACCTGAAGAACATT
TTATTGAAACCATCTCACTTGCTGGTTCCTATAAAGATTGGTCCTATTCTGGACAATCTACACAACTAGGTATAGAATTTCTTAAGAGAGG
TGATAAAAGTGTATATTACACTAGTAATCCTACCACATTCCACCTAGATGGTGAAGTTATCACCTTTGACAATCTTAAGACACTTCTTTCTT
TGAGAGAAGTGAGGACTATTAAGGTGTTTACAACAGTAGACAACATTAACCTCCACACGCAAGTTGTGGACATGTCAATGACATATGGA
CAACAGTTTGGTCCAACTTATTTGGATGGAGCTGATGTTACTAAAATAAAACCTCATAATTCACATGAAGGTAAAACATTTTATGTTTTAC
CTAATGATGACACTCTACGTGTTGAGGCTTTTGAGTACTACCACACAACTGATCCTAGTTTTCTGGGTAGGTACATGTCAGCATTAAATCA
CACTAAAAAGTGGAAATACCCACAAGTTAATGGTTTAACTTCTATTAAATGGGCAGATAACAACTGTTATCTTGCCACTGCATTGTTAACA
CTCCAACAAATAGAGTTGAAGTTTAATCCACCTGCTCTACAAGATGCTTATTACAGAGCAAGGGCTGGTGAAGCTGCTAACTTTTGTGCA
CTTATCTTAGCCTACTGTAATAAGACAGTAGGTGAGTTAGGTGATGTTAGAGAAACAATGAGTTACTTGTTTCAACATGCCAATTTAGAT
TCTTGCAAAAGAGTCTTGAACGTGGTGTGTAAAACTTGTGGACAACAGCAGACAACCCTTAAGGGTGTAGAAGCTGTTATGTACATGGG
CACACTTTCTTATGAACAATTTAAGAAAGGTGTTCAGATACCTTGTACGTGTGGTAAACAAGCTACAAAATATCTAGTACAACAGGAGTC
ACCTTTTGTTATGATGTCAGCACCACCTGCTCAGTATGAACTTAAGCATGGTACATTTACTTGTGCTAGTGAGTACACTGGTAATTACCAG
TGTGGTCACTATAAACATATAACTTCTAAAGAAACTTTGTATTGCATAGACGGTGCTTTACTTACAAAGTCCTCAGAATACAAAGGTCCTA
TTACGGATGTTTTCTACAAAGAAAACAGTTACACAACAACCATAAAACCAGTTACTTATAAATTGGATGGTGTTGTTTGTACAGAAATTG
ACCCTAAGTTGGACAATTATTATAAGAAAGACAATTCTTATTTCACAGAGCAACCAATTGATCTTGTACCAAACCAACCATATCCAAACGC
AAGCTTCGATAATTTTAAGTTTGTATGTGATAATATCAAATTTGCTGATGATTTAAACCAGTTAACTGGTTATAAGAAACCTGCTTCAAGA
GAGCTTAAAGTTACATTTTTCCCTGACTTAAATGGTGATGTGGTGGCTATTGATTATAAACACTACACACCCTCTTTTAAGAAAGGAGCTA
AATTGTTACATAAACCTATTGTTTGGCATGTTAACAATGCAACTAATAAAGCCACGTATAAACCAAATACCTGGTGTATACGTTGTCTTTG
GAGCACAAAACCAGTTGAAACATCAAATTCGTTTGATGTACTGAAGTCAGAGGACGCGCAGGGAATGGATAATCTTGCCTGCGAAGAT
CTAAAACCAGTCTCTGAAGAAGTAGTGGAAAATCCTACCATACAGAAAGACGTTCTTGAGTGTAATGTGAAAACTACCGAAGTTGTAGG
AGACATTATACTTAAACCAGCAAATAATAGTTTAAAAATTACAGAAGAGGTTGGCCACACAGATCTAATGGCTGCTTATGTAGACAATTC
TAGTCTTACTATTAAGAAACCTAATGAATTATCTAGAGTATTAGGTTTGAAAACCCTTGCTACTCATGGTTTAGCTGCTGTTAATAGTGTC
CCTTGGGATACTATAGCTAATTATGCTAAGCCTTTTCTTAACAAAGTTGTTAGTACAACTACTAACATAGTTACACGGTGTTTAAACCGTG
TTTGTACTAATTATATGCCTTATTTCTTTACTTTATTGCTACAATTGTGTACTTTTACTAGAAGTACAAATTCTAGAATTAAAGCATCTATGC
CGACTACTATAGCAAAGAATACTGTTAAGAGTGTCGGTAAATTTTGTCTAGAGGCTTCATTTAATTATTTGAAGTCACCTAATTTTTCTAA
ACTGATAAATATTATAATTTGGTTTTTACTATTAAGTGTTTGCCTAGGTTCTTTAATCTACTCAACCGCTGCTTTAGGTGTTTTAATGTCTAA
TTTAGGCATGCCTTCTTACTGTACTGGTTACAGAGAAGGCTATTTGAACTCTACTAATGTCACTATTGCAACCTACTGTACTGGTTCTATAC
CTTGTAGTGTTTGTCTTAGTGGTTTAGATTCTTTAGACACCTATCCTTCTTTAGAAACTATACAAATTACCATTTCATCTTTTAAATGGGATT
TAACTGCTTTTGGCTTAGTTGCAGAGTGGTTTTTGGCATATATTCTTTTCACTAGGTTTTTCTATGTACTTGGATTGGCTGCAATCATGCAA
TTGTTTTTCAGCTATTTTGCAGTACATTTTATTAGTAATTCTTGGCTTATGTGGTTAATAATTAATCTTGTACAAATGGCCCCGATTTCAGCT
ATGGTTAGAATGTACATCTTCTTTGCATCATTTTATTATGTATGGAAAAGTTATGTGCATGTTGTAGACGGTTGTAATTCATCAACTTGTAT
GATGTGTTACAAACGTAATAGAGCAACAAGAGTCGAATGTACAACTATTGTTAATGGTGTTAGAAGGTCCTTTTATGTCTATGCTAATGG
AGGTAAAGGCTTTTGCAAACTACACAATTGGAATTGTGTTAATTGTGATACATTCTGTGCTGGTAGTACATTTATTAGTGATGAAGTTGC
GAGAGACTTGTCACTACAGTTTAAAAGACCAATAAATCCTACTGACCAGTCTTCTTACATCGTTGATAGTGTTACAGTGAAGAATGGTTC
CATCCATCTTTACTTTGATAAAGCTGGTCAAAAGACTTATGAAAGACATTCTCTCTCTCATTTTGTTAACTTAGACAACCTGAGAGCTAAT
AACACTAAAGGTTCATTGCCTATTAATGTTATAGTTTTTGATGGTAAATCAAAATGTGAAGAATCATCTGCAAAATCAGCGTCTGTTTACT
ACAGTCAGCTTATGTGTCAACCTATACTGTTACTAGATCAGGCATTAGTGTCTGATGTTGGTGATAGTGCGGAAGTTGCAGTTAAAATGT
TTGATGCTTACGTTAATACGTTTTCATCAACTTTTAACGTACCAATGGAAAAACTCAAAACACTAGTTGCAACTGCAGAAGCTGAACTTGC
AAAGAATGTGTCCTTAGACAATGTCTTATCTACTTTTATTTCAGCAGCTCGGCAAGGGTTTGTTGATTCAGATGTAGAAACTAAAGATGTT
GTTGAATGTCTTAAATTGTCACATCAATCTGACATAGAAGTTACTGGCGATAGTTGTAATAACTATATGCTCACCTATAACAAAGTTGAAA
ACATGACACCCCGTGACCTTGGTGCTTGTATTGACTGTAGTGCGCGTCATATTAATGCGCAGGTAGCAAAAAGTCACAACATTGCTTTGA
TATGGAACGTTAAAGATTTCATGTCATTGTCTGAACAACTACGAAAACAAATACGTAGTGCTGCTAAAAAGAATAACTTACCTTTTAAGT
TGACATGTGCAACTACTAGACAAGTTGTTAATGTTGTAACAACAAAGATAGCACTTAAGGGTGGTAAAATTGTTAATAATTGGTTGAAG
CAGTTAATTAAAGTTACACTTGTGTTCCTTTTTGTTGCTGCTATTTTCTATTTAATAACACCTGTTCATGTCATGTCTAAACATACTGACTTT
TCAAGTGAAATCATAGGATACAAGGCTATTGATGGTGGTGTCACTCGTGACATAGCATCTACAGATACTTGTTTTGCTAACAAACATGCT
 
GATTTTGACACATGGTTTAGCCAGCGTGGTGGTAGTTATACTAATGACAAAGCTTGCCCATTGATTGCTGCAGTCATAACAAGAGAAGT
GGGTTTTGTCGTGCCTGGTTTGCCTGGCACGATATTACGCACAACTAATGGTGACTTTTTGCATTTCTTACCTAGAGTTTTTAGTGCAGTT
GGTAACATCTGTTACACACCATCAAAACTTATAGAGTACACTGACTTTGCAACATCAGCTTGTGTTTTGGCTGCTGAATGTACAATTTTTA
AAGATGCTTCTGGTAAGCCAGTACCATATTGTTATGATACCAATGTACTAGAAGGTTCTGTTGCTTATGAAAGTTTACGCCCTGACACAC
GTTATGTGCTCATGGATGGCTCTATTATTCAATTTCCTAACACCTACCTTGAAGGTTCTGTTAGAGTGGTAACAACTTTTGATTCTGAGTA
CTGTAGGCACGGCACTTGTGAAAGATCAGAAGCTGGTGTTTGTGTATCTACTAGTGGTAGATGGGTACTTAACAATGATTATTACAGAT
CTTTACCAGGAGTTTTCTGTGGTGTAGATGCTGTAAATTTACTTACTAATATGTTTACACCACTAATTCAACCTATTGGTGCTTTGGACATA
TCAGCATCTATAGTAGCTGGTGGTATTGTAGCTATCGTAGTAACATGCCTTGCCTACTATTTTATGAGGTTTAGAAGAGCTTTTGGTGAAT
ACAGTCATGTAGTTGCCTTTAATACTTTACTATTCCTTATGTCATTCACTGTACTCTGTTTAACACCAGTTTACTCATTCTTACCTGGTGTTT
ATTCTGTTATTTACTTGTACTTGACATTTTATCTTACTAATGATGTTTCTTTTTTAGCACATATTCAGTGGATGGTTATGTTCACACCTTTAG
TACCTTTCTGGATAACAATTGCTTATATCATTTGTATTTCCACAAAGCATTTCTATTGGTTCTTTAGTAATTACCTAAAGAGACGTGTAGTC
TTTAATGGTGTTTCCTTTAGTACTTTTGAAGAAGCTGCGCTGTGCACCTTTTTGTTAAATAAAGAAATGTATCTAAAGTTGCGTAGTGATG
TGCTATTACCTCTTACGCAATATAATAGATACTTAGCTCTTTATAATAAGTACAAGTATTTTAGTGGAGCAATGGATACAACTAGCTACAG
AGAAGCTGCTTGTTGTCATCTCGCAAAGGCTCTCAATGACTTCAGTAACTCAGGTTCTGATGTTCTTTACCAACCACCACAAACCTCTATC
ACCTCAGCTGTTTTGCAGAGTGGTTTTAGAAAAATGGCATTCCCATCTGGTAAAGTTGAGGGTTGTATGGTACAAGTAACTTGTGGTACA
ACTACACTTAACGGTCTTTGGCTTGATGACGTAGTTTACTGTCCAAGACATGTGATCTGCACCTCTGAAGACATGCTTAACCCTAATTATG
AAGATTTACTCATTCGTAAGTCTAATCATAATTTCTTGGTACAGGCTGGTAATGTTCAACTCAGGGTTATTGGACATTCTATGCAAAATTG
TGTACTTAAGCTTAAGGTTGATACAGCCAATCCTAAGACACCTAAGTATAAGTTTGTTCGCATTCAACCAGGACAGACTTTTTCAGTGTTA
GCTTGTTACAATGGTTCACCATCTGGTGTTTACCAATGTGCTATGAGGCCCAATTTCACTATTAAGGGTTCATTCCTTAATGGTTCATGTG
GTAGTGTTGGTTTTAACATAGATTATGACTGTGTCTCTTTTTGTTACATGCACCATATGGAATTACCAACTGGAGTTCATGCTGGCACAGA
CTTAGAAGGTAACTTTTATGGACCTTTTGTTGACAGGCAAACAGCACAAGCAGCTGGTACGGACACAACTATTACAGTTAATGTTTTAGC
TTGGTTGTACGCTGCTGTTATAAATGGAGACAGGTGGTTTCTCAATCGATTTACCACAACTCTTAATGACTTTAACCTTGTGGCTATGAAG
TACAATTATGAACCTCTAACACAAGACCATGTTGACATACTAGGACCTCTTTCTGCTCAAACTGGAATTGCCGTTTTAGATATGTGTGCTT
CATTAAAAGAATTACTGCAAAATGGTATGAATGGACGTACCATATTGGGTAGTGCTTTATTAGAAGATGAATTTACACCTTTTGATGTTG
TTAGACAATGCTCAGGTGTTACTTTCCAAAGTGCAGTGAAAAGAACAATCAAGGGTACACACCACTGGTTGTTACTCACAATTTTGACTT
CACTTTTAGTTTTAGTCCAGAGTACTCAATGGTCTTTGTTCTTTTTTTTNTATGAAAATGCCTTTTTACCTTTTGCTATGGGTATTATTGCTA
TGTCTGCTTTTGCAATGATGTTTGTCAAACATAAGCATGCATTTCTCTGTTTGTTTTTGTTACCTTCTCTTGCCACTGTAGCTTATTTTAATA
TGGTCTATATGCCTGCTAGTTGGGTGATGCGTATTATGACATGGTTGGATATGGTTGATACTAGTTTGTCTGGTTTTAAGCTAAAAGACT
GTGTTATGTATGCATCAGCTGTAGTGTTACTAATCCTTATGACAGCAAGAACTGTGTATGATGATGGTGCTAGGAGAGTGTGGACACTT
ATGAATGTCTTGACACTCGTTTATAAAGTTTATTATGGTAATGCTTTAGATCAAGCCATTTCCATGTGGGCTCTTATAATCTCTGTTACTTC
TAACTACTCAGGTGTAGTTACAACTGTCATGTTTTTGGCCAGAGGTATTGTTTTTATGTGTGTTGAGTATTGCCCTATTTTCTTCATAACTG
GTAATACACTTCAGTGTATAATGCTAGTTTATTGTTTCTTAGGCTATTTTTGTACTTGTTACTTTGGCCTCTTTTGTTTACTCAACCGCTACT
TTAGACTGACTCTTGGTGTTTATGATTACTTAGTTTCTACACAGGAGTTTAGATATATGAATTCACAGGGACTACTCCCACCCAA
GAATAGCATAGATGCCTTCAAACTCAACATTAAATTGTTGGGTGTTGGTGGCAAACCTTGTATCAAAGTAGCCACTGTACAGTCTAAAAT
GTCAGATGTAAAGTGCACATCAGTAGTCTTACTCTCAGTTTTGCAACAACTCAGAGTAGAATCATCATCTAAATTGTGGGCTCAATGTGT
CCAGTTACACAATGACATTCTCTTAGCTAAAGATACTACTGAAGCCTTTGAAAAAATGGTTTCACTACTTTCTGTTTTGCTTTCCATGCAGG
GTGCTGTAGACATAAACAAGCTTTGTGAAGAAATGCTGGACAACAGGGCAACCTTACAAGCTATAGCCTCAGAGTTTAGTTCCCTTCCAT
CATATGCAGCTTTTGCTACTGCTCAAGAAGCTTATGAGCAGGCTGTTGCTAATGGTGATTCTGAAGTTGTTCTTAAAAAGTTGAAGAAGT
CTTTGAATGTGGCTAAATCTGAATTTGACCGTGATGCAGCCATGCAACGTAAGTTGGAAAAGATGGCTGATCAAGCTATGACCCAAATG
TATAAACAGGCTAGATCTGAGGACAAGAGGGCAAAAGTTACTAGTGCTATGCAGACAATGCTTTTCACTATGCTTAGAAAGTTGGATAA
TGATGCACTCAACAACATTATCAACAATGCAAGAGATGGTTGTGTTCCCTTGAACATAATACCTCTTACAACAGCAGCCAAACTAATGGT
TGTCATACCAGACTATAACACATATAAAAATACGTGTGATGGTACAACATTTACTTATGCATCAGCATTGTGGGAAATCCAACAGGTTGT
AGATGCAGATAGTAAAATTGTTCAACTTAGTGAAATTAGTATGGACAATTCACCTAATTTAGCATGGCCTCTTATTGTAACAGCTTTAAG
GGCCAATTCTGCTGTCAAATTACAGAATAATGAGCTTAGTCCTGTTGCACTACGACAGATGTCTTGTGCTGCCGGTACTACACAAACTGC
TTGCACTGATGACAATGCGTTAGCTTACTACAACACAACAAAGGGAGGTAGGTTTGTACTTGCACTGTTATCCGATTTACAGGATTTGAA
ATGGGCTAGATTCCCTAAGAGTGATGGAACTGGTACTATCTATACAGAACTGGAACCACCTTGTAGGTTTGTTACAGACACACCTAAAG
GTCCTAAAGTGAAGTATTTATACTTTATTAAAGGATTAAACAACCTAAATAGAGGTATGGTACTTGGTAGTTTAGCTGCCACAGTACGTC
TACAAGCTGGTAATGCAACAGAAGTGCCTGCCAATTCAACTGTATTATCTTTCTGTGCTTTTGCTGTAGATGCTGCTAAAGCTTACAAAGA
TTATCTAGCTAGTGGGGGACAACCAATCACTAATTGTGTTAAGATGTTGTGTACACACACTGGTACTGGTCAGGCAATAACAGTTACACC
GGAAGCCAATATGGATCAAGAATCCTTTGGTGGTGCATCGTGTTGTCTGTACTGCCGTTGCCACATAGATCATCCAAATCCTAAAGGATT
TTGTGACTTAAAAGGTAAGTATGTACAAATACCTACAACTTGTGCTAATGACCCTGTGGGTTTTACACTTAAAAACACAGTCTGTACCGTC
TGCGGTATGTGGAAAGGTTATGGCTGTAGTTGTGATCAACTCCGCGAACCCATGCTTCAGTCAGCTGATGCACAATCGTTTTTAAACGG
GTTTGCGGTGTAAGTGCAGCCCGTCTTACACCGTGCGGCACAGGCACTAGTACTGATGTCGTATACAGGGCTTTTGACATCTACAATGAT
AAAGTAGCTGGTTTTGCTAAATTCCTAAAAACTAATTGTTGTCGCTTCCAAGAAAAGGACGAAGATGACAATTTAATTGATTCTTACTTTG
TAGTTAAGAGACACACTTTCTCTAACTACCAACATGAAGAAACAATTTATAATTTACTTAAGGATTGTCCAGCTGTTGCTAAACATGACTT
CTTTAAGTTTAGAATAGACGGTGACATGGTACCACATATATCACGTCAACGTCTTACTAAATACACAATGGCAGACCTCGTCTATGCTTTA
AGGCATTTTGATGAAGGTAATTGTGACACATTAAAAGAAATACTTGTCACATACAATTGTTGTGATGATGATTATTTCAATAAAAAGGAC
TGGTATGATTTTGTAGAAAACCCAGATATATTACGCGTATACGCCAACTTAGGTGAACGTGTACGCCAAGCTTTGTTAAAAACAGTACAA
TTCTGTGATGCCATGCGAAATGCTGGTATTGTTGGTGTACTGACATTAGATAATCAAGATCTCAATGGTAACTGGTATGATTTCGGTGAT
TTCATACAAACCACGCCAGGTAGTGGAGTTCCTGTTGTAGATTCTTATTATTCATTGTTAATGCCTATATTAACCTTGACCAGGGCTTTAA
CTGCAGAGTCACATGTTGACACTGACTTAACAAAGCCTTACATTAAGTGGGATTTGTTAAAATATGACTTCACGGAAGAGAGGTTAAAA
CTCTTTGACCGTTATTTTAAATATTGGGATCAGACATACCACCCAAATTGTGTTAACTGTTTGGATGACAGATGCATTCTGCATTGTGCAA
 
ACTTTAATGTTTATTCTCTACAGTGTTCCCACCTACAAGTTTTGGACCACTAGTGAGAAAAATATTGTTGATGGTGTTCCATTTGTAGTT
TCAACTGGATACCACTTCAGAGAGCTAGGTGTTGTACATAATCAGGATGTAAACTTACATAGCTCTAGACTTAGTTTTAAGGAATTACTT
GTGTATGCTGCTGACCCTGCTATGCACGCTGCTTCTGGTAATCTATTACTAGATAAACGCACTACGTGCTTTTCAGTAGCTGCACTTACTA
ACAATGTTGCTTTTCAAACTGTCAAACCCGGTAATTTTAACAAAGACTTCTATGACTTTGCTGTGTCTAAGGGTTTCTTTAAGGAAGGAAG
TTCTGTTGAATTAAAACACTTCTTCTTTGCTCAGGATGGTAATGCTGCTATCAGCGATTATGACTACTATCGTTATAATCTACCAACAATGT
GTGATATCAGACAACTACTATTTGTAGTTGAAGTTGTTGATAAGTACTTTGATTGTTACGATGGTGGCTGTATTAATGCTAACCAAGTCAT
CGTCAACAACCTAGACAAATCAGCTGGTTTTCCATTTAATAAATGGGGTAAGGCTAGACTTTATTATGATTCAATGAGTTATGAGGATCA
AGATGCACTTTTCGCATATACAAAACGTAATGTCATCCCTACTATAACTCAAATGAATCTTAAGTATGCCATTAGTGCAAAGAATAGAGCT
CGCACCGTAGCTGGTGTCTCTATCTGTAGTACTATGACCAATAGACAGTTTCATCAAAAATTATTGAAATCAATAGCCGCCACTAGAGGA
GCTACTGTAGTAATTGGAACAAGCAAATTCTATGGTGGTTGGCACAACATGTTAAAAACTGTTTATAGTGATGTAGAAAACCCTCACCTT
ATGGGTTGGGATTATCCTAAATGTGATAGAGCCATGCCTAACATGCTTAGAATTATGGCCTCACTTGTTCTTGCTCGCAAACATACAACG
TGTTGTAGCTTGTCACACCGTTTCTATAGATTAGCTAATGAGTGTGCTCAAGTATTGAGTGAAATGGTCATGTGTGGCGGTTCACTATAT
GTTAAACCAGGTGGAACCTCATCAGGAGATGCCACAACTGCTTATGCTAATAGTGTTTTTAACATTTGTCAAGCTGTCACGGCCAATGTT
AATGCACTTTTATCTACTGATGGTAACAAAATTGCCGATAAGTATGTCCGCAATTTACAACACAGACTTTATGAGTGTCTCTATAGAAATA
GAGATGTTGACACAGACTTTGTGAATGAGTTTTACGCATATTTGCGTAAACATTTCTCAATGATGATACTCTCTGACGATGCTGTTGTGTG
TTTCAATAGCACTTATGCATCTCAAGGTCTAGTGGCTAGCATAAAGAACTTTAAGTCAGTTCTTTATTATCAAAACAATGTTTTTATGTCTG
AAGCAAAATGTTGGACTGAGACTGACCTTACTAAAGGACCTCATGAATTTTGCTCTCAACATACAATGCTAGTTAAACAGGGTGATGATT
ATGTGTACCTTCCTTACCCAGATCCATCAAGAATCCTAGGGGCCGGCTGTTTTGTAGATGATATCGTAAAAACAGATGGTACACTTATGA
TTGAACGGTTCGTGTCTTTAGCTATAGATGCTTACCCACTTACTAAACATCCTAATCAGGAGTATGCTGATGTCTTTCATTTGTACTTACAA
TACATAAGAAAGCTACATGATGAGTTAACAGGACACATGTTAGACATGTATTCTGTTATGCTTACTAATGATAACACTTCAAGGTATTGG
GAACCTGAGTTTTATGAGGCTATGTACACACCGCATACAGTCTTACAGGCTGTTGGGGCTTGTGTTCTTTGCAATTCACAGACTTCATTAA
GATGTGGTGCTTGCATACGTAGACCATTCTTATGTTGTAAATGCTGTTACGACCATGTCATATCAACATCACATAAATTAGTCTTGTCTGT
TAATCCGTATGTTTGCAATGCTCCAGGTTGTGATGTCACAGATGTGACTCAACTTTACTTAGGAGGTATGAGCTATTATTGTAAATCACAT
AAACCACCCATTAGTTTTCCATTGTGTGCTAATGGACAAGTTTTTGGTTTATATAAAAATACATGTGTTGGTAGCGATAATGTTACTGACT
TTAATGCAATTGCAACATGTGACTGGACAAATGCTGGTGATTACATTTTAGCTAACACCTGTACTGAAAGACTCAAGCTTTTTGCAGCAG
AAACGCTCAAAGCTACTGAGGAGACATTTAAACTGTCTTATGGTATTGCTACTGTACGTGAAGTGCTGTCTGACAGAGAATTACATCTTT
CATGGGAAGTTGGTAAACCTAGACCACCACTTAACCGAAATTATGTCTTTACTGGTTATCGTGTAACTAAAAACAGTAAAGTACAAATAG
GAGAGTACACCTTTGAAAAAGGTGACTATGGTGATGCTGTTGTTTACCGAGGTACAACAACTTACAAATTAAATGTTGGTGATTATTTTG
TGCTGACATCACATACAGTAATGCCATTAAGTGCACCTACACTAGTGCCACAAGAGCACTATGTTAGAATTACTGGCTTATACCCAACAC
TCAATATCTCAGATGAGTTTTCTAGCAATGTTGCAAATTATCAAAAGGTTGGTATGCAAAAGTATTCTACACTCCAGGGACCACCTGGTA
CTGGTAAGAGTCATTTTGCTATTGGCCTAGCTCTCTACTACCCTTCTGCTCGCATAGTGTATACAGCTTGCTCTCATGCCGCTGTTGATGC
ACTATGTGAGAAGGCATTAAAATATTTGCCTATAGATAAATGTAGTAGAATTATACCTGCACGTGCTCGTGTAGAGTGTTTTGATAAATT
CAAAGTGAATTCAACATTAGAACAGTATGTCTTTTGTACTGTAAATGCATTGCCTGAGACGACAGCAGATATAGTTGTCTTTGATGAAAT
TTCAATGGCCACAAATTATGATTTGAGTGTTGTCAATGCCAGATTACGTGCTAAGCACTATGTGTACATTGGCGACCCTGCTCAATTACCT
GCACCACGCACATTGCTAACTAAGGGCACACTAGAACCAGAATATTTCAATTCAGTGTGTAGACTTATGAAAACTATAGGTCCAGACATG
TTCCTCGGAACTTGTCGGCGTTGTCCTGCTGAAATTGTTGACACTGTGAGTGCTTTGGTTTATGATAATAAGCTTAAAGCACATAAAGAC
AAATCAGCTCAATGCTTTAAAATGTTTTATAAGGGTGTTATCACGCATGATGTTTCATCTGCAATTAACAGGCCACAAATAGGCGTGGTA
AGAGAATTCCTTACACGTAACCCTGCTTGGAGAAAAGCTGTCTTTATTTCACCTTATAATTCACAGAATGCTGTAGCCTCAAAGATTTTGG
GACTACCAACTCAAACTGTTGATTCATCACAGGGCTCAGAATATGACTATGTCATATTCACTCAAACCACTGAAACAGCTCACTCTTGTAA
TGTAAACAGATTTAATGTTGCTATTACCAGAGCAAAAGTAGGCATACTTTGCATAATGTCTGATAGAGACCTTTATGACAAGTTGCAATT
TACAAGTCTTGAAATTCCACGTAGGAATGTGGCAACTTTACAAGCTGAAAATGTAACAGGACTCTTTAAAGATTGTAGTAAGGTAATCAC
TGGGTTACATCCTACACAGGCACCTACACACCTCAGTGTTGACACTAAATTCAAAACTGAAGGTTTATGTGTTGACATACCTGGCATACC
TAAGGACATGACCTATAGAAGACTCATCTCTATGATGGGTTTTAAAATGAATTATCAAGTTAATGGTTACCCTAACATGTTTATCACCCGC
GAAGAAGCTATAAGACATGTACGTGCATGGATTGGCTTCGATGTCGAGGGGTGTCATGCTACTAGAGAAGCTGTTGGTACCAATTTACC
TTTACAGCTAGGTTTTTCTACAGGTGTTAACCTAGTTGCTGTACCTACAGGTTATGTTGATACACCTAATAATACAGATTTTTCCAGAGTTA
GTGCTAAACCACCGCCTGGAGATCAATTTAAACACCTCATACCACTTATGTACAAAGGACTTCCTTGGAATGTAGTGCGTATAAAGATTG
TACAAATGTTAAGTGACACACTTAAAAATCTCTCTGACAGAGTCGTATTTGTCTTATGGGCACATGGCTTTGAGTTGACATCTATGAAGT
ATTTTGTGAAAATAGGACCTGAGCGCACCTGTTGTCTATGTGATAGACGTGCCACATGCTTTTCCACTGCTTCAGACACTTATGCCTGTTG
GCATCATTCTATTGGATTTGATTACGTCTATAATCCGTTTATGATTGATGTTCAACAATGGGGTTTTACAGGTAACCTACAAAGCAACCAT
GATCTGTATTGTCAAGTCCATGGTAATGCACATGTAGCTAGTTGTGATGCAATCATGACTAGGTGTCTAGCTGTCCACGAGTGCTTTGTT
AAGCGTGTTGACTGGACTATTGAATATCCTATAATTGGTGATGAACTGAAGATTAATGCGGCTTGTAGAAAGGTTCAACACATGGTTGTT
AAAGCTGCATTATTAGCAGACAAATTCCCAGTTCTTCACGACATTGGTAACCCTAAAGCTATTAAGTGTGTACCTCAAGCTGATGTAGAA
TGGAAGTTCTATGATGCACAGCCTTGTAGTGACAAAGCTTATAAAATAGAAGAATTATTCTATTCTTATGCCACACATTCTGACAAATTCA
CAGATGGTGTATGCCTATTTTGGAATTGCAATGTCGATAGATATCCTGCTAATTCCATTGTTTGTAGATTTGACACTAGAGTGCTATCTAA
CCTTAACTTGCCTGGTTGTGATGGTGGCAGTTTGTATGTAAATAAACATGCATTCCACACACCAGCTTTTGATAAAAGTGCTTTTGTTAAT
TTAAAACAATTACCATTTTTCTATTACTCTGACAGTCCATGTGAGTCTCATGGAAAACAAGTAGTGTCAGATATAGATTATGTACCACTAA
AGTCTGCTACGTGTATAACACGTTGCAATTTAGGTGGTGCTGTCTGTAGACATCATGCTAATGAGTACAGATTGTATCTCGATGCTTATA
ACATGATGATCTCAGCTGGCTTTAGCTTGTGGGTTTACAAACAATTTGATACTTATAACCTCTGGAACACTTTTACAAGACTTCAGAGTTT
AGAAAATGTGGCTTTTAATGTTGTAAATAAGGGACACTTTGATGGACAACAGGGTGAAGTACCAGTTTCTATCATTAATAACACTGTTTA
CACAAAAGTTGATGGTGTTGATGTAGAATTGTTTGAAAATAAAACAACATTACCTGTTAATGTAGCATTTGAGCTTTGGGCTAAGCGCAA
CATTAAACCAGTACCAGAGGTGAAAATACTCAATAATTTGGGTGTGGACATTGCTGCTAATACTGTGATCTGGGACTACAAAAGAGATG
CTCCAGCACATATATCTACTATTGGTGTTTGTTCTATGACTGACATAGCCAAGAAACCAACTGAAACGATTTGTGCACCACTCACTGTCTT
 
TTTTGATGGTAGAGTTGATGGTCAAGTAGACTTATTTAGAAATGCCCGTAATGGTGTTCTTATTACAGAAGGTAGTGTTAAAGGTTTACA
ACCATCTGTAGGTCCCAAACAAGCTAGTCTTAATGGAGTCACATTAATTGGAGAAGCCGTAAAAACACAGTTCAATTATTATAAGAAAGT
TGATGGTGTTGTCCAACAATTACCTGAAACTTACTTTACTCAGAGTAGAAATTTACAAGAATTTAAACCCAGGAGTCAAATGGAAATTGA
TTTCTTAGAATTAGCTATGGATGAATTCATTGAACGGTATAAATTAGAAGGCTATGCCTTCGAACATATCGTTTATGGAGATTTTAGTCAT
AGTCAGTTAGGTGGTTTACATCTACTGATTGGACTAGCTAAACGTTTTAAGGAATCACCTTTTGAATTAGAAGATTTTATTCCTATGGACA
GTACAGTTAAAAACTATTTCATAACAGATGCGCAAACAGGTTCATCTAAGTGTGTGTGTTCTGTTATTGATTTATTACTTGATGATTTTGTT
GAAATAATAAAATCCCAAGATTTATCTGTAGTTTCTAAGGTTGTCAAAGTGACTATTGACTATACAGAAATTTCATTTATGCTTTGGTGTA
AAGATGGCCATGTAGAAACATTTTACCCAAAATTACAATCTAGTCAAGCGTGGCAACCGGGTGTTGCTATGCCTAATCTTTACAAAATGC
AAAGAATGCTATTAGAAAAGTGTGACCTTCAAAATTATGGTGATAGTGCAACATTACCTAAAGGCATAATGATGAATGTCGCAAAATAT
ACTCAACTGTGTCAATATTTAAACACATTAACATTAGCTGTACCCTATAATATGAGAGTTATACATTTTGGTGCTGGTTCTGATAAAGGAG
TTGCACCAGGTACAGCTGTTTTAAGACAGTGGTTGCCTACGGGTACGCTGCTTGTCGATTCAGATCTTAATGACTTTGTCTCTGATGCAG
ATTCAACTTTGATTGGTGATTGTGCAACTGTACATACAGCTAATAAATGGGATCTCATTATTAGTGATATGTACGACCCTAAGACTAAAA
ATGTTACAAAAGAAAATGACTCTAAAGAGGGTTTTTTCACTTACATTTGTGGGTTTATACAACAAAAGCTAGCTCTTGGAGGTTCCGTGG
CTATAAAGATAACAGAACATTCTTGGAATGCTGATCTTTATAAGCTCATGGGACACTTCGCATGGTGGACAGCCTTTGTTACTAATGTGA
ATGCGTCATCATCTGAAGCATTTTTAATTGGATGTAATTATCTTGGCAAACCACGCGAACAAATAGATGGTTATGTCATGCATGCAAATT
ACATATTTTGGAGGAATACAAATCCAATTCAGTTGTCTTCCTATTCTTTATTTGACATGAGTAAATTTCCCCTTAAATTAAGGGGTACTGCT
GTTATGTCTTTAAAAGAAGGTCAAATCAATGATATGATTTTATCTCTTCTTAGTAAAGGTAGACTTATAATTAGAGAAAACAACAGAGTT
GTTATTTCTAGTGATGTTCTTGTTAACAACTAAACGAACAATGTTTGTTTTTCTTGTTTTATTGCCACTAGTCTCTAGTCAGTGTGTTAATCT
TACAACCAGAACTCAATTACCCCCTGCATACACTAATTCTTTCACACGTGGTGTTTATTACCCTGACAAAGTTTTCAGATCCTCAGTTTTAC
ATTCAACTCAGGACTTGTTCTTACCTTTCTTTTCCAATGTTACTTGGTTCCATGCTATACATGTCTCTGGGACCAATGGTACTAAGAGGTTT
GATAACCCTGTCCTACCATTTAATGATGGTGTTTATTTTGCTTCCACTGAGAAGTCTAACATAATAAGAGGCTGGATTTTTGGTACTACTT
TAGATTCGAAGACCCAGTCCCTACTTATTGTTAATAACGCTACTAATGTTGTTATTAAAGTCTGTGAATTTCAATTTTGTAATGATCCATTT
TTGGGTGTTTATTACCACAAAAACAACAAAAGTTGGATGGAAAGTGAGTTCAGAGTTTATTCTAGTGCGAATAATTGCACTTTTGAATAT
GTCTCTCAGCCTTTTCTTATGGACCTTGAAGGAAAACAGGGTAATTTCAAAAATCTTAGGGAATTTGTGTTTAAGAATATTGATGGTTATT
TTAAAATATATTCTAAGCACACGCCTATTAATTTAGTGCGTGATCTCCCTCAGGGTTTTTCGGCTTTAGAACCATTGGTAGATTTGCCAAT
AGGTATTAACATCACTAGGTTTCAAACTTTACTTGCTTTACATAGAAGTTATTTGACTCCTGGTGATTCTTCTTCAGGTTGGACAGCTGGT
GCTGCAGCTTATTATGTGGGTTATCTTCAACCTAGGACTTTTCTATTAAAATATAATGAAAATGGAACCATTACAGATGCTGTAGACTGTG
CACTTGACCCTCTCTCAGAAACAAAGTGTACGTTGAAATCCTTCACTGTAGAAAAAGGAATCTATCAAACTTCTAACTTTAGAGTCCAACC
AACAGAATCTATTGTTAGATTTCCTAATATTACAAACTTGTGCCCTTTTGGTGAAGTTTTTAACGCCACCAGATTTGCATCTGTTTATGCTT
GGAACAGGAAGAGAATCAGCAACTGTGTTGCTGATTATTCTGTCCTATATAATTCCGCATCATTTTCCACTTTTAAGTGTTATGGAGTGTC
TCCTACTAAATTAAATGATCTCTGCTTTACTAATGTCTATGCAGATTCATTTGTAATTAGAGGTGATGAAGTCAGACAAATCGCTCCAGGG
CAAACTGGAAAGATTGCTGATTATAATTATAAATTACCAGATGATTTTACAGGCTGCGTTATAGCTTGGAATTCTAACAATCTTGATTCTA
AGGTTGGTGGTAATTATAATTACCTGTATAGATTGTTTAGGAAGTCTAATCTCAAACCTTTTGAGAGAGATATTTCAACTGAAATCTATCA
GGCCGGTAGCACACCTTGTAATGGTGTTGAAGGTTTTAATTGTTACTTTCCTTTACAATCATATGGTTTCCAACCCACTAATGGTGTTGGT
TACCAACCATACAGAGTAGTAGTACTTTCTTTTGAACTTCTACATGCACCAGCAACTGTTTGTGGACCTAAAAAGTCTACTAATTTGGTTA
AAAACAAATGTGTCAATTTCAACTTCAATGGTTTAACAGGCACAGGTGTTCTTACTGAGTCTAACAAAAAGTTTCTGCCTTTCCAACAATT
TGGCAGAGACATTGCTGACACTACTGATGCTGTCCGTGATCCACAGACACTTGAGATTCTTGACATTACACCATGTTCTTTTGGTGGTGTC
AGTGTTATAACACCAGGAACAAATACTTCTAACCAGGTTGCTGTTCTTTATCAGGATGTTAACTGCACAGAAGTCCCTGTTGCTATTCATG
CAGATCAACTTACTCCTACTTGGCGTGTTTATTCTACAGGTTCTAATGTTTTTCAAACACGTGCAGGCTGTTTAATAGGGGCTGAACATGT
CAACAACTCATATGAGTGTGACATACCCATTGGTGCAGGTATATGCGCTAGTTATCAGACTCAGACTAATTCTCCTCGGCGGGCACGTAG
TGTAGCTAGTCAATCCATCATTGCCTACACTATGTCACTTGGTGCAGAAAATTCAGTTGCTTACTCTAATAACTCTATTGCCATACCCACAA
ATTTTACTATTAGTGTTACCACAGAAATTCTACCAGTGTCTATGACCAAGACATCAGTAGATTGTACAATGTACATTTGTGGTGATTCAAC
TGAATGCAGCAATCTTTTGTTGCAATATGGCAGTTTTTGTACACAATTAAACCGTGCTTTAACTGGAATAGCTGTTGAACAAGACAAAAA
CACCCAAGAAGTTTTTGCACAAGTCAAACAAATTTACAAAACACCACCAATTAAAGATTTTGGTGGTTTTAATTTTTCACAAATATTACCA
GATCCATCAAAACCAAGCAAGAGGTCATTTATTGAAGATCTACTTTTCAACAAAGTGACACTTGCAGATGCTGGCTTCATCAAACAATAT
GGTGATTGCCTTGGTGATATTGCTGCTAGAGACCTCATTTGTGCACAAAAGTTTAACGGCCTTACTGTTTTGCCACCTTTGCTCACAGATG
AAATGATTGCTCAATACACTTCTGCACTGTTAGCGGGTACAATCACTTCTGGTTGGACCTTTGGTGCAGGTGCTGCATTACAAATACCATT
TGCTATGCAAATGGCTTATAGGTTTAATGGTATTGGAGTTACACAGAATGTTCTCTATGAGAACCAAAAATTGATTGCCAACCAATTTAA
TAGTGCTATTGGCAAAATTCAAGACTCACTTTCTTCCACAGCAAGTGCACTTGGAAAACTTCAAGATGTGGTCAACCAAAATGCACAAGC
TTTAAACACGCTTGTTAAACAACTTAGCTCCAATTTTGGTGCAATTTCAAGTGTTTTAAATGATATCCTTTCACGTCTTGACAAAGTTGAG
GCTGAAGTGCAAATTGATAGGTTGATCACAGGCAGACTTCAAAGTTTGCAGACATATGTGACTCAACAATTAATTAGAGCTGCAGAAAT
CAGAGCTTCTGCTAATCTTGCTGCTACTAAAATGTCAGAGTGTGTACTTGGACAATCAAAAAGAGTTGATTTTTGTGGAAAGGGCTATCA
TCTTATGTCCTTCCCTCAGTCAGCACCTCATGGTGTAGTCTTCTTGCATGTGACTTATGTCCCTGCACAAGAAAAGAACTTCACAACTGCTC
CTGCCATTTGTCATGATGGAAAAGCACACTTTCCTCGTGAAGGTGTCTTTGTTTCAAATGGCACACACTGGTTTGTAACACAAAGGAATT
TTTATGAACCACAAATCATTACTACAGACAACACATTTGTGTCTGGTAACTGTGATGTTGTAATAGGAATTGTCAACAACACAGTTTATGA
TCCTTTGCAACCTGAATTAGACTCATTCAAGGAGGAGTTAGATAAATATTTTAAGAATCATACATCACCAGATGTTGATTTAGGTGACATC
TCTGGCATTAATGCTTCAGTTGTAAACATTCAAAAAGAAATTGACCGCCTCAATGAGGTTGCCAAGAATTTAAATGAATCTCTCATCGATC
TCCAAGAACTTGGAAAGTATGAGCAGTATATAAAATGGCCATGGTACATTTGGCTAGGTTTTATAGCTGGCTTGATTGCCATAGTAATG
GTGACAATTATGCTTTGCTGTATGACCAGTTGCTGTAGTTGTCTCAAGGGCTGTTGTTCTTGTGGATCCTGCTGCAAATTTGATGAAGAC
GACTCTGAGCCAGTGCTCAAAGGAGTCAAATTACATTACACATAAACGAACTTATGGATTTGTTTATGAGAATCTTCACAATTGGAACTG
TAACTTTGAAGCAAGGTGAAATCAAGGATGCTACTCCTTCAGATTTTGTTCGCGCTACTGCAACGATACCGATACAAGCCTCACTCCCTTT
CGGATGGCTTATTGTTGGCGTTGCACTTCTTGCTGTTTTTCAGAGCGCTTCCAAAATCATAACCCTCAAAAAGAGATGGCAACTAGCACT
 
CTCCAAGGGTGTTCACTTTGTTTGCAACTTGCTGTTGTTTGTAACAGTTTACTCACACCTTTTGCTCGTTGCTGCTGGCCTTGAAGCCC
CTTTTCTCTATCTTTATGCTTTAGTCTACTTCTTGCAGAGTATAAACTTTGTAAGAATAATAATGAGGCTTTGGCTTTGCTGGAAATGCCGT
TCCAAAAACCCATTACTTTATGATGCCAACTATTTCTTTGCTGGCATACTAATTGTTACGACTATTGTATACCTTACAATAGTGTAACTTCT
TCAATTGTCATTACTTCAGGTGATGGCACAACAAGTCCTATTTCTGAACATGACTACCAGATTGGTGGTTATACTGAAAAATGGGAATCT
GGAGTAAAAGACTGTGTTGTATTACACAGTTACTTCACTTCAGACTATTACCAGCTGTACTCAACTCAATTGAGTACAGACACTGGTGTT
GAACATGTTACCTTCTTCATCTACAATAAAATTGTTGATGAGCCTGAAGAACATGTCCAAATTCACACAATCGACGTTTCATCCGGAGTTG
TTAATCCAGTAATGGAACCAATTTATGATGAACCGACGACGACTACTAGCGTGCCTTTGTAAGCACAAGCTGATGAGTACGAACTTATGT
ACTCATTCGTTTCGGAAGAGACAGGTACGTTAATAGTTAATAGCGTACTTCTTTTTCTTGCTTTCGTGGTATTCTTGCTAGTTACACTAGCC
ATCCTTACTGCGCTTCGATTGTGTGCGTACTGCTGCAATATTGTTAACGTGAGTCTTGTAAAACCTTCTTTTTACGTTTACTCTCGTGTTAA
AAATCTGAATTCTTCTAGAGTTCCTGATCTTCTGGTCTAAACGAACTAAATATTATATTAGTTTTTCTGTTTGGAACTTTAATTTTAGCCAT
GGCAGATTCCAACGGTACTATTACCGTTGAAGAGCTTAAAAAGCTCCTTGAACAATGGAACCTAGTAATAGGTTTCCTATTCCTTACATG
GATTTGTCTTCTACAATTTGCCTATGCCAACAGGAATAGGTTTTTGTATATAATTAAGTTAATTTTCCTCTGGCTGTTATGGCCAGTAACTT
TAGCTTGTTTTGTGCTTGCTGCTGTTTACAGAATAAATTGGATCACCGGTGGAATTGCTATCGCAATGGCTTGTCTTGTAGGCTTGATGTG
GCTCAGCTACTTCATTGCTTCTTTCAGACTGTTTGCGCGTACGCGTTCCATGTGGTCATTCAATCCAGAAACTAACATTCTTCTCAACGTGC
CACTCCATGGCACTATTCTGACCAGACCGCTTCTAGAAAGTGAACTCGTAATCGGAGCTGTGATCCTTCGTGGACATCTTCGTATTGCTG
GACACCATCTAGGACGCTGTGACATCAAGGACCTGCCTAAAGAAATCACTGTTGCTACATCACGAACGCTTTCTTATTACAAATTGGGAG
CTTCGCAGCGTGTAGCAGGTGACTCAGGTTTTGCTGCATACAGTCGCTACAGGATTGGCAACTATAAATTAAACACAGACCATTCCAGTA
GCAGTGACAATATTGCTTTGCTTGTACAGTAAGTGACAACAGATGTTTCATCTCGTTGACTTTCAGGTTACTATAGCAGAGATATTACTAA
TTATTATGAGGACTTTTAAAGTTTCCATTTGGAATCTTGATTACATCATAAACCTCATAATTAAAAATTTATCTAAGTCACTAACTGAGAAT
AAATATTCTCAATTAGATGAAGAGCAACCAATGGAGATTGATTAAACGAACATGAAAATTATTCTTTTCTTGGCACTGATAACACTCGCT
ACTTGTGAGCTTTATCACTACCAAGAGTGTGTTAGAGGTACAACAGTACTTTTAAAAGAACCTTGCTCTTCTGGAACATACGAGGGCAAT
TCACCATTTCATCCTCTAGCTGATAACAAATTTGCACTGACTTGCTTTAGCACTCAATTTGCTTTTGCTTGTCCTGACGGCGTAAAACACGT
CTATCAGTTACGTGCCAGATCAGTTTCACCTAAACTGTTCATCAGACAAGAGGAAGTTCAAGAACTTTACTCTCCAATTTTTCTTATTGTTG
CGGCAATAGTGTTTATAACACTTTGCTTCACACTCAAAAGAAAGACAGAATGATTGAACTTTCATTAATTGACTTCTATTTGTGCTTTTTA
GCCTTTCTGCTATTCCTTGTTTTAATTATGCTTATTATCTTTTGGTTCTCACTTGAACTGCAAGATCATAATGAAACTTGTCACGCCTAAACG
AACATGAAATTTCTTGTTTTCTTAGGAATCATCACAACTGTAGCTGCATTTCACCAAGAATGTAGTTTACAGTCATGTACTCAACATCAAC
CATATGTAGTTGATGACCCGTGTCCTATTCACTTCTATTCTAAATGGTATATTAGAGTAGGAGCTAGAAAATCAGCACCTTTAATTGAATT
GTGCGTGGATGAGGCTGGTTCTAAATCACCCATTCAGTACATCGATATCGGTAATTATACAGTTTCCTGTTTACCTTTTACAATTAATTGC
CAGGAACCTAAATTGGGTAGTCTTGTAGTGCGTTGTTCGTTCTATGAAGACTTTTTAGAGTATCATGACGTTCGTGTTGTTTTAGATTTCA
TCTAAACGAACAAACTAAAATGTCTGATAATGGACCCCAAAATCAGCGAAATGCACCCCGCATTACGTTTGGTGGACCCTCAGATTCAAC
TGGCAGTAACCAGAATGGAGAACGCAGTGGGGCGCGATCAAAACAACGTCGGCCCCAAGGTTTACCCAATAATACTGCGTCTTGGTTC
ACCGCTCTCACTCAACATGGCAAGGAAGACCTTAAATTCCCTCGAGGACAAGGCGTTCCAATTAACACCAATAGCAGTCCAGATGACCA
AATTGGCTACTACCGAAGAGCTACCAGACGAATTCGTGGTGGTGACGGTAAAATGAAAGATCTCAGTCCAAGATGGTATTTCTACTACC
TAGGAACTGGGCCAGAAGCTGGACTTCCCTATGGTGCTAACAAAGACGGCATCATATGGGTTGCAACTGAGGGAGCCTTGAATACACC
AAAAGATCACATTGGCACCCGCAATCCTGCTAACAATGCTGCAATCGTGCTACAACTTCCTCAAGGAACAACATTGCCAAAAGGCTTCTA
CGCAGAAGGGAGCAGAGGCGGCAGTCAAGCCTCTTCTCGTTCCTCATCACGTAGTCGCAACAGTTCAAGAAATTCAACTCCAGGCAGCA
GTAGGGGAACTTCTCCTGCTAGAATGGCTGGCAATGGCGGTGATGCTGCTCTTGCTTTGCTGCTGCTTGACAGATTGAACCAGCTTGAG
AGCAAAATGTCTGGTAAAGGCCAACAACAACAAGGCCAAACTGTCACTAAGAAATCTGCTGCTGAGGCTTCTAAGAAGCCTCGGCAAA
AACGTACTGCCACTAAAGCATACAATGTAACACAAGCTTTCGGCAGACGTGGTCCAGAACAAACCCAAGGAAATTTTGGGGACCAGGA
ACTAATCAGACAAGGAACTGATTACAAACATTGGCCGCAAATTGCACAATTTGCCCCCAGCGCTTCAGCGTTCTTCGGAATGTCGCGCAT
TGGCATGGAAGTCACACCTTCGGGAACGTGGTTGACCTACACAGGTGCCATCAAATTGGATGACAAAGATCCAAATTTCAAAGATCAAG
TCATTTTGCTGAATAAGCATATTGACGCATACAAAACATTCCCACCAACAGAGCCTAAAAAGGACAAAAAGAAGAAGGCTGATGAAACT
CAAGCCTTACCGCAGAGACAGAAGAAACAGCAAACTGTGACTCTTCTTCCTGCTGCAGATTTGGATGATTTCTCCAAACAATTGCAACAA
TCCATGAGCAGTGCTGACTCAACTCAGGCCTAAACTCATGCAGACCACACAAGGCAGATGGGCTATATAAACGTTTTCGCTTTTCCGTTT
ACGATATATAGTCTACTCTTGTGCAGAATGAATTCTCGTAACTACATAGCACAAGTAGATGTAGTTAACTTTAATCTCACATAGCAATCTT
TAATCAGTGTGTAACATTAGGGAGGACTTGAAAGAGCCACCACATTTTCACCGAGGCCACGCGGAGTACGATCGAGTGTACAGTGAAC
AATGCTAGGGAGAGCTGCCTATATGGAAGAGCCCTAATGTGTAAAATTAATTTTAGTAGTGCTATCCCCATGTGATTTTAATAGCTTCTT
AGGAGAAT
SEQ ID NO: 10
   >Severe acute respiratory syndrome coronavirus 2 orflab polyprotein of isolate hCoV-19/Italy/|NM|1-isl/2020 (Genbank Acc. No: QIA98553) 
MESLVPGFNEKTHVQLSLPVLQVRDVLVRGFGDSVEEVLSEARQHLKDGTCGLVEVEKGVLPQLEQPYVFIKRSDARTAPHGHVMVELVAELE
GIQYGRSGETLGVLVPHVGEIPVAYRKVLLRKNGNKGAGGHSYGADLKSFDLGDELGTDPYEDFQENWNTKHSSGVTRELMRELNGGAYTRY
VDNNFCGPDGYPLECIKDLLARAGKASCTLSEQLDFIDTKRGVYCCREHEHEIAWYTERSEKSYELQTPFEIKLAKKFDTFNGECPNFVFPLNSIIK
TIQPRVEKKKLDGFMGRIRSVYPVASPNECNQMCLSTLMKCDHCGETSWQTGDFVKATCEFCGTENLTKEGATTCGYLPQNAVVKIYCPACH
NSEVGPEHSLAEYHNESGLKTILRKGGRTIAFGGCVFSYVGCHNKCAYWVPRASANIGCNHTGVVGEGSEGLNDNLLEILQKEKVNINIVGDFK
LNEEIAIILASFSASTSAFVETVKGLDYKAFKQIVESCGNFKVTKGKAKKGAWNIGEQKSILSPLYAFASEAARVVRSIFSRTLETAQNSVRVLQKA
AITILDGISQYSLRLIDAMMFTSDLATNNLVVMAYITGGVVQLTSQWLTNIFGTVYEKLKPVLDWLEEKFKEGVEFLRDGWEIVKFISTCACEIV
GGQIVTCAKEIKESVQTFFKLVNKFLALCADSIIIGGAKLKALNLGETFVTHSKGLYRKCVKSREETGLLMPLKAPKEIIFLEGETLPTEVLTEEVVLK
TGDLQPLEQPTSEAVEAPLVGTPVCINGLMLLEIKDTEKYCALAPNMMVTNNTFTLKGGAPTKVTFGDDTVIEVQGYKSVNITFELDERIDKVL
 
NEKCSAYTVELGTEVNEFACVVADAVIKTLQPVSELLTPLGIDLDEWSMATYYLFDESGEFKLASHMYCSFYPPDEDEEEGDCEEEEFEPSTQYE
YGTEDDYQGKPLEFGATSAALQPEEEQEEDWLDDDSQQTVGQQDGSEDNQTTTIQTIVEVQPQLEMELTPVVQTIEVNSFSGYLKLTDNVYI
KNADIVEEAKKVKPTVVVNAANVYLKHGGGVAGALNKATNNAMQVESDDYIATNGPLKVGGSCVLSGHNLAKHCLHVVGPNVNKGEDIQL
LKSAYENFNQHEVLLAPLLSAGIFGADPIHSLRVCVDTVRTNVYLAVFDKNLYDKLVSSFLEMKSEKQVEQKIAEIPKEEVKPFITESKPSVEQRKQ
DDKKIKACVEEVTTTLEETKFLTENLLLYIDINGNLHPDSATLVSDIDITFLKKDAPYIVGDVVQEGVLTAVVIPTKKAGGTTEMLAKALRKVPTDN
YITTYPGQGLNGYTVEEAKTVLKKCKSAFYILPSIISNEKQEILGTVSWNLREMLAHAEETRKLMPVCVETKAIVSTIQRKYKGIKIQEGVVDYGAR
FYFYTSKTTVASLINTLNDLNETLVTMPLGYVTHGLNLEEAARYMRSLKVPATVSVSSPDAVTAYNGYLTSSSKTPEEHFIETISLAGSYKDWSYS
GQSTQLGIEFLKRGDKSVYYTSNPTTFHLDGEVITFDNLKTLLSLREVRTIKVFTTVDNINLHTQVVDMSMTYGQQFGPTYLDGADVTKIKPHNS
HEGKTFYVLPNDDTLRVEAFEYYHTTDPSFLGRYMSALNHTKKWKYPQVNGLTSIKWADNNCYLATALLTLQQIELKFNPPALQDAYYRARAG
EAANFCALILAYCNKTVGELGDVRETMSYLFQHANLDSCKRVLNVVCKTCGQQQTTLKGVEAVMYMGTLSYEQFKKGVQIPCTCGKQATKYL
VQQESPFVMMSAPPAQYELKHGTFTCASEYTGNYQCGHYKHITSKETLYCIDGALLTKSSEYKGPITDVFYKENSYTTTIKPVTYKLDGVVCTEID
PKLDNYYKKDNSYFTEQPIDLVPNQPYPNASFDNFKFVCDNIKFADDLNQLTGYKKPASRELKVTFFPDLNGDVVAIDYKHYTPSFKKGAKLLHK
PIVWHVNNATNKATYKPNTWCIRCLWSTKPVETSNSFDVLKSEDAQGMDNLACEDLKPVSEEVVENPTIQKDVLECNVKTTEVVGDIILKPAN
NSLKITEEVGHTDLMAAYVDNSSLTIKKPNELSRVLGLKTLATHGLAAVNSVPWDTIANYAKPFLNKVVSTTTNIVTRCLNRVCTNYMPYFFTLL
LQLCTFTRSTNSRIKASMPTTIAKNTVKSVGKFCLEASFNYLKSPNFSKLINIIIWFLLLSVCLGSLIYSTAALGVLMSNLGMPSYCTGYREGYLNST
NVTIATYCTGSIPCSVCLSGLDSLDTYPSLETIQITISSFKWDLTAFGLVAEWFLAYILFTRFFYVLGLAAIMQLFFSYFAVHFISNSWLMWLIINLV
QMAPISAMVRMYIFFASFYYVWKSYVHVVDGCNSSTCMMCYKRNRATRVECTTIVNGVRRSFYVYANGGKGFCKLHNWNCVNCDTFCAG
STFISDEVARDLSLQFKRPINPTDQSSYIVDSVTVKNGSIHLYFDKAGQKTYERHSLSHFVNLDNLRANNTKGSLPINVIVFDGKSKCEESSAKSAS
VYYSQLMCQPILLLDQALVSDVGDSAEVAVKMFDAYVNTFSSTFNVPMEKLKTLVATAEAELAKNVSLDNVLSTFISAARQGFVDSDVETKDV
VECLKLSHQSDIEVTGDSCNNYMLTYNKVENMTPRDLGACIDCSARHINAQVAKSHNIALIWNVKDFMSLSEQLRKQIRSAAKKNNLPFKLTC
ATTRQVVNVVTTKIALKGGKIVNNWLKQLIKVTLVFLFVAAIFYLITPVHVMSKHTDFSSEIIGYKAIDGGVTRDIASTDTCFANKHADFDTWFS
QRGGSYTNDKACPLIAAVITREVGFVVPGLPGTILRTTNGDFLHFLPRVFSAVGNICYTPSKLIEYTDFATSACVLAAECTIFKDASGKPVPYCYDT
NVLEGSVAYESLRPDTRYVLMDGSIIQFPNTYLEGSVRVVTTFDSEYCRHGTCERSEAGVCVSTSGRWVLNNDYYRSLPGVFCGVDAVNLLTN
MFTPLIQPIGALDISASIVAGGIVAIVVTCLAYYFMRFRRAFGEYSHVVAFNTLLFLMSFTVLCLTPVYSFLPGVYSVIYLYLTFYLTNDVSFLAHIQ
WMVMFTPLVPFWITIAYIICISTKHFYWFFSNYLKRRVVFNGVSFSTFEEAALCTFLLNKEMYLKLRSDVLLPLTQYNRYLALYNKYKYFSGAMD
TTSYREAACCHLAKALNDFSNSGSDVLYQPPQTSITSAVLQSGFRKMAFPSGKVEGCMVQVTCGTTTLNGLWLDDVVYCPRHVICTSEDMLN
PNYEDLLIRKSNHNFLVQAGNVQLRVIGHSMQNCVLKLKVDTANPKTPKYKFVRIQPGQTFSVLACYNGSPSGVYQCAMRPNFTIKGSFLNGS
CGSVGFNIDYDCVSFCYMHHMELPTGVHAGTDLEGNFYGPFVDRQTAQAAGTDTTITVNVLAWLYAAVINGDRWFLNRFTTTLNDFNLVA
MKYNYEPLTQDHVDILGPLSAQTGIAVLDMCASLKELLQNGMNGRTILGSALLEDEFTPFDVVRQCSGVTFQSAVKRTIKGTHHWLLLTILTSL
LVLVQSTQWSLFFFXYENAFLPFAMGIIAMSAFAMMFVKHKHAFLCLFLLPSLATVAYFNMVYMPASWVMRIMTWLDMVDTSLSGFKLKD
CVMYASAVVLLILMTARTVYDDGARRVWTLMNVLTLVYKVYYGNALDQAISMWALIISVTSNYSGVVTTVMFLARGIVFMCVEYCPIFFITGN
TLQCIMLVYCFLGYFCTCYFGLFCLLNRYFRLTLGVYDYLVSTQEFRYMNSQGLLPPKNSIDAFKLNIKLLGVGGKPCIKVATVQSKMSDVKCTSV
VLLSVLQQLRVESSSKLWAQCVQLHNDILLAKDTTEAFEKMVSLLSVLLSMQGAVDINKLCEEMLDNRATLQAIASEFSSLPSYAAFATAQEAY
EQAVANGDSEVVLKKLKKSLNVAKSEFDRDAAMQRKLEKMADQAMTQMYKQARSEDKRAKVTSAMQTMLFTMLRKLDNDALNNIINNA
RDGCVPLNIIPLTTAAKLMVVIPDYNTYKNTCDGTTFTYASALWEIQQVVDADSKIVQLSEISMDNSPNLAWPLIVTALRANSAVKLQNNELSP
VALRQMSCAAGTTQTACTDDNALAYYNTTKGGRFVLALLSDLQDLKWARFPKSDGTGTIYTELEPPCRFVTDTPKGPKVKYLYFIKGLNNLNR
GMVLGSLAATVRLQAGNATEVPANSTVLSFCAFAVDAAKAYKDYLASGGQPITNCVKMLCTHTGTGQAITVTPEANMDQESFGGASCCLYC
RCHIDHPNPKGFCDLKGKYVQIPTTCANDPVGFTLKNTVCTVCGMWKGYGCSCDQLREPMLQSADAQSFLNRVCGVSAARLTPCGTGTSTD
VVYRAFDIYNDKVAGFAKFLKTNCCRFQEKDEDDNLIDSYFVVKRHTFSNYQHEETIYNLLKDCPAVAKHDFFKFRIDGDMVPHISRQRLTKYT
MADLVYALRHFDEGNCDTLKEILVTYNCCDDDYFNKKDWYDFVENPDILRVYANLGERVRQALLKTVQFCDAMRNAGIVGVLTLDNQDLNG
NWYDFGDFIQTTPGSGVPVVDSYYSLLMPILTLTRALTAESHVDTDLTKPYIKWDLLKYDFTEERLKLFDRYFKYWDQTYHPNCVNCLDDRCIL
HCANFNVLFSTVFPPTSFGPLVRKIFVDGVPFVVSTGYHFRELGVVHNQDVNLHSSRLSFKELLVYAADPAMHAASGNLLLDKRTTCFSVAALT
NNVAFQTVKPGNFNKDFYDFAVSKGFFKEGSSVELKHFFFAQDGNAAISDYDYYRYNLPTMCDIRQLLFVVEVVDKYFDCYDGGCINANQVIV
NNLDKSAGFPFNKWGKARLYYDSMSYEDQDALFAYTKRNVIPTITQMNLKYAISAKNRARTVAGVSICSTMTNRQFHQKLLKSIAATRGATVV
IGTSKFYGGWHNMLKTVYSDVENPHLMGWDYPKCDRAMPNMLRIMASLVLARKHTTCCSLSHRFYRLANECAQVLSEMVMCGGSLYVKP
GGTSSGDATTAYANSVFNICQAVTANVNALLSTDGNKIADKYVRNLQHRLYECLYRNRDVDTDFVNEFYAYLRKHFSMMILSDDAVVCFNST
YASQGLVASIKNFKSVLYYQNNVFMSEAKCWTETDLTKGPHEFCSQHTMLVKQGDDYVYLPYPDPSRILGAGCFVDDIVKTDGTLMIERFVSL
AIDAYPLTKHPNQEYADVFHLYLQYIRKLHDELTGHMLDMYSVMLTNDNTSRYWEPEFYEAMYTPHTVLQAVGACVLCNSQTSLRCGACIRR
PFLCCKCCYDHVISTSHKLVLSVNPYVCNAPGCDVTDVTQLYLGGMSYYCKSHKPPISFPLCANGQVFGLYKNTCVGSDNVTDFNAIATCDWT
NAGDYILANTCTERLKLFAAETLKATEETFKLSYGIATVREVLSDRELHLSWEVGKPRPPLNRNYVFTGYRVTKNSKVQIGEYTFEKGDYGDAVV
YRGTTTYKLNVGDYFVLTSHTVMPLSAPTLVPQEHYVRITGLYPTLNISDEFSSNVANYQKVGMQKYSTLQGPPGTGKSHFAIGLALYYPSARIV
YTACSHAAVDALCEKALKYLPIDKCSRIIPARARVECFDKFKVNSTLEQYVFCTVNALPETTADIVVFDEISMATNYDLSVVNARLRAKHYVYIGD
PAQLPAPRTLLTKGTLEPEYFNSVCRLMKTIGPDMFLGTCRRCPAEIVDTVSALVYDNKLKAHKDKSAQCFKMFYKGVITHDVSSAINRPQIGV
VREFLTRNPAWRKAVFISPYNSQNAVASKILGLPTQTVDSSQGSEYDYVIFTQTTETAHSCNVNRFNVAITRAKVGILCIMSDRDLYDKLQFTSL
EIPRRNVATLQAENVTGLFKDCSKVITGLHPTQAPTHLSVDTKFKTEGLCVDIPGIPKDMTYRRLISMMGFKMNYQVNGYPNMFITREEAIRH
VRAWIGFDVEGCHATREAVGTNLPLQLGFSTGVNLVAVPTGYVDTPNNTDFSRVSAKPPPGDQFKHLIPLMYKGLPWNVVRIKIVQMLSDTL
KNLSDRVVFVLWAHGFELTSMKYFVKIGPERTCCLCDRRATCFSTASDTYACWHHSIGFDYVYNPFMIDVQQWGFTGNLQSNHDLYCQVHG
NAHVASCDAIMTRCLAVHECFVKRVDWTIEYPIIGDELKINAACRKVQHMVVKAALLADKFPVLHDIGNPKAIKCVPQADVEWKFYDAQPCS
DKAYKIEELFYSYATHSDKFTDGVCLFWNCNVDRYPANSIVCRFDTRVLSNLNLPGCDGGSLYVNKHAFHTPAFDKSAFVNLKQLPFFYYSDSP
CESHGKQVVSDIDYVPLKSATCITRCNLGGAVCRHHANEYRLYLDAYNMMISAGFSLWVYKQFDTYNLWNTFTRLQSLENVAFNVVNKGHF
DGQQGEVPVSIINNTVYTKVDGVDVELFENKTTLPVNVAFELWAKRNIKPVPEVKILNNLGVDIAANTVIWDYKRDAPAHISTIGVCSMTDIAK
KPTETICAPLTVFFDGRVDGQVDLFRNARNGVLITEGSVKGLQPSVGPKQASLNGVTLIGEAVKTQFNYYKKVDGVVQQLPETYFTQSRNLQE
 
FKPRSQMEIDFLELAMDEFIERYKLEGYAFEHIVYGDFSHSQLGGLHLLIGLAKRFKESPFELEDFIPMDSTVKNYFITDAQTGSSKCVCSVIDLLL
DDFVEIIKSQDLSVVSKVVKVTIDYTEISFMLWCKDGHVETFYPKLQSSQAWQPGVAMPNLYKMQRMLLEKCDLQNYGDSATLPKGIMMNV
AKYTQLCQYLNTLTLAVPYNMRVIHFGAGSDKGVAPGTAVLRQWLPTGTLLVDSDLNDFVSDADSTLIGDCATVHTANKWDLIISDMYDPKT
KNVTKENDSKEGFFTYICGFIQQKLALGGSVAIKITEHSWNADLYKLMGHFAWWTAFVTNVNASSSEAFLIGCNYLGKPREQIDGYVMHANYI
FWRNTNPIQLSSYSLFDMSKFPLKLRGTAVMSLKE
SEQ ID NO: 11
   >Protein\S_2019-nCoV/Italy-INMII (Sprotein_hCoV19ItalyINMI1isl2020)(GenbankAcc. No: QIA98554)
SEQ ID NO: 12 >hCoV-19/France/1_DF0372-isl/2020|EP1_ISL 410720|2020-01-23 
ATTAAAGGTTTATACCTTCCCAGGTAACAAACCAACCAACTTTCGATCTCTTGTAGATCTGTTCTCTAAACGAACTTTAAAATCTGTGTGG
CTGTCACTCGGCTGCATGCTTAGTGCACTCACGCAGTATAATTAATAACTAATTACTGTCGTTGACAGGACACGAGTAACTCGTCTATCTT
CTGCAGGCTGCTTACGGTTTCGTCCGTGTTGCAGCCGATCATCAGCACATCTAGGTTTCGTCCGGGTGTGACCGAAAGGTAAGATGGAG
AGCCTTGTCCCTGGTTTCAACGAGAAAACACACGTCCAACTCAGTTTGCCTGTTTTACAGGTTCGCGACGTGCTCGTACGTGGCTTTGGA
GACTCCGTGGAGGAGGTCTTATCAGAGGCACGTCAACATCTTAAAGATGGCACTTGTGGCTTAGTAGAAGTTGAAAAAGGCGTTTTGCC
TCAACTTGAACAGCCCTATGTGTTCATCAAACGTTCGGATGCTCGAACTGCACCTCATGGTCATGTTATGGTTGAGCTGGTAGCAGAACT
CGAAGGCATTCAGTACGGTCGTAGTGGTGAGACACTTGGTGTCCTTGTCCCTCATGTGGGCGAAATACCAGTGGCTTACCGCAAGGTTC
TTCTTCGTAAGAACGGTAATAAAGGAGCTGGTGGCCATAGTTACGGCGCCGATCTAAAGTCATTTGACTTAGGCGACGAGCTTGGCACT
GATCCTTATGAAGATTTTCAAGAAAACTGGAACACTAAACATAGCAGTGGTGTTACCCGTGAACTCATGCGTGAGCTTAACGGAGGGGC
ATACACTCGCTATGTCGATAACAACTTCTGTGGCCCTGATGGCTACCCTCTTGAGTGCATTAAAGACCTTCTAGCACGTGCTGGTAAAGC
TTCATGCACTTTGTCCGAACAACTGGACTTTATTGACACTAAGAGGGGTGTATACTGCTGCCGTGAACATGAGCATGAAATTGCTTGGTA
CACGGAACGTTCTGAAAAGAGCTATGAATTGCAGACACCTTTTGAAATTAAATTGGCAAAGAAATTTGACACCTTCAATGGGGAATGTC
CAAATTTTGTATTTCCCTTAAATTCCATAATCAAGACTATTCAACCAAGGGTTGAAAAGAAAAAGCTTGATGGCTTTATGGGTAGAATTC
GATCTGTCTATCCAGTTGCGTCACCAAATGAATGCAACCAAATGTGCCTTTCAACTCTCATGAAGTGTGATCATTGTGGTGAAACTTCATG
GCAGACGGGCGATTTTGTTAAAGCCACTTGCGAATTTTGTGGCACTGAGAATTTGACTAAAGAAGGTGCCACTACTTGTGGTTACTTACC
CCAAAATGCTGTTGTTAAAATTTATTGTCCAGCATGTCACAATTCAGAAGTAGGACCTGAGCATAGTCTTGCCGAATACCATAATGAATC
TGGCTTGAAAACCATTCTTCGTAAGGGTGGTCGCACTATTGCCTTTGGAGGCTGTGTGTTCTCTTATGTTGGTTGCCATAACAAGTGTGC
CTATTGGGTTCCACGTGCTAGCGCTAACATAGGTTGTAACCATACAGGTGTTGTTGGAGAAGGTTCCGAAGGTCTTAATGACAACCTTCT
TGAAATACTCCAAAAAGAGAAAGTCAACATCAATATTGTTGGTGACTTTAAACTTAATGAAGAGATCGCCATTATTTTGGCATCTTTTTCT
GCTTCCACAAGTGCTTTTGTGGAAACTGTGAAAGGTTTGGATTATAAAGCATTCAAACAAATTGTTGAATCCTGTGGTAATTTTAAAGTT
ACAAAAGGAAAAGCTAAAAAAGGTGCCTGGAATATTGGTGAACAGAAATCAATACTGAGTCCTCTTTATGCATTTGCATCAGAGGCTGC
TCGTGTTGTACGATCAATTTTCTCCCGCACTCTTGAAACTGCTCAAAATTCTGTGCGTGTTTTACAGAAGGCCGCTATAACAATACTAGAT
GGAATTTCACAGTATTCACTGAGACTCATTGATGCTATGATGTTCACATCTGATTTGGCTACTAACAATCTAGTTGTAATGGCCTACATTA
CAGGTGGTGTTGTTCAGTTGACTTCGCAGTGGCTAACTAACATCTTTGGCACTGTTTATGAAAAACTCAAACCCGTCCTTGATTGGCTTGA
AGAGAAGTTTAAGGAAGGTGTAGAGTTTCTTAGAGACGGTTGGGAAATTGTTAAATTTATCTCAACCTGTGCTTGTGAAATTGTCGGTG
GACAAATTGTCACCTGTGCAAAGGAAATTAAGGAGAGTGTTCAGACATTCTTTAAGCTTGTAAATAAATTTTTGGCTTTGTGTGCTGACT
CTATCATTATTGGTGGAGCTAAACTTAAAGCCTTGAATTTAGGTGAAACATTTGTCACGCACTCAAAGGGATTGTACAGAAAGTGTGTTA
AATCCAGAGAAGAAACTGGCCTACTCATGCCTCTAAAAGCCCCAAAAGAAATTATCTTCTTAGAGGGAGAAACACTTCCCACAGAAGTG
TTAACAGAGGAAGTTGTCTTGAAAACTGGTGATTTACAACCATTAGAACAACCTACTAGTGAAGCTGTTGAAGCTCCATTGGTTGGTACA
CCAGTTTGTATTAACGGGCTTATGTTGCTCGAAATCAAAGACACAGAAAAGTACTGTGCCCTTGCACCTAATATGATGGTAACAAACAAT
ACCTTCACACTCAAAGGCGGTGCACCAACAAAGGTTACTTTTGGTGATGACACTGTGATAGAAGTGCAAGGTTACAAGAGTGTGAATAT
CACTTTTGAACTTGATGAAAGGATTGATAAAGTACTTAATGAGAAGTGCTCTGCCTATACAGTTGAACTCGGTACAGAAGTAAATGAGTT
CGCCTGTGTTGTGGCAGATGCTGTCATAAAAACTTTGCAACCAGTATCTGAATTACTTACACCACTGGGCATTGATTTAGATGAGTGGAG
TATGGCTACATACTACTTATTTGATGAGTCTGGTGAGTTTAAATTGGCTTCACATATGTATTGTTCTTTCTACCCTCCAGATGAGGATGAA
GAAGAAGGTGATTGTGAAGAAGAAGAGTTTGAGCCATCAACTCAATATGAGTATGGTACTGAAGATGATTACCAAGGTAAACCTTTGG
AATTTGGTGCCACTTCTGCTGCTCTTCAACCTGAAGAAGAGCAAGAAGAAGATTGGTTAGATGATGATAGTCAACAAACTGTTGGTCAA
 
CAAGACGGCAGTGAGGACAATCAGACAACTACTATTCAAACAATTGTTGAGGTTCAACCTCAATTAGAGATGGAACTTACACCAGTTGT
TCAGACTATTGAAGTGAATAGTTTTAGTGGTTATTTAAAACTTACTGACAATGTATACATTAAAAATGCAGACATTGTGGAAGAAGCTAA
AAAGGTAAAACCAACAGTGGTTGTTAATGCAGCCAATGTTTACCTTAAACATGGAGGAGGTGTTGCAGGAGCCTTAAATAAGGCTACTA
ACAATGCCATGCAAGTTGAATCTGATGATTACATAGCTACTAATGGACCACTTAAAGTGGGTGGTAGTTGTGTTTTAAGCGGACACAATC
TTGCTAAACACTGTCTTCATGTTGTCGGCCCAAATGTTAACAAAGGTGAAGACATTCAACTTCTTAAGAGTGCTTATGAAAATTTTAATCA
GCACGAAGTTCTACTTGCACCATTATTATCAGCTGGTATTTTTGGTGCTGACCCTATACATTCTTTAAGAGTTTGTGTAGATACTGTTCGCA
CAAATGTCTACTTAGCTGTCTTTGATAAAAATCTCTATGACAAACTTGTTTCAAGCTTTTTGGAAATGAAGAGTGAAAAGCAAGTTGAAC
AAAAGATCGCTGAGATTCCTAAAGAGGAAGTTAAGCCATTTATAACTGAAAGTAAACCTTCAGTTGAACAGAGAAAACAAGATGATAAG
AAAATCAAAGCTTGTGTTGAAGAAGTTACAACAACTCTGGAAGAAACTAAGTTCCTCACAGAAAACTTGTTACTTTATATTGACATTAAT
GGCAATCTTCATCCAGATTCTGCCACTCTTGTTAGTGACATTGACATCACTTTCTTAAAGAAAGATGCTCCATATATAGTGGGTGATGTTG
TTCAAGAGGGTGTTTTAACTGCTGTGGTTATACCTACTAAAAAGGCTGGTGGCACTACTGAAATGCTAGCGAAAGCTTTGAGAAAAGTG
CCAACAGACAATTATATAACCACTTACCCGGGTCAGGGTTTAAATGGTTACACTGTAGAGGAGGCAAAGACAGTGCTTAAAAAGTGTAA
AAGTGCCTTTTACATTCTACCATCTATTATCTCTAATGAGAAGCAAGAAATTCTTGGAACTGTTTCTTGGAATTTGCGAGAAATGCTTGCA
CATGCAGAAGAAACACGCAAATTAATGCCTGTCTGTGTGGAAACTAAAGCCATAGTTTCAACTATACAGCGTAAATATAAGGGTATTAA
AATACAAGAGGGTGTGGTTGATTATGGTGCTAGATTTTACTTTTACACCAGTAAAACAACTGTAGCGTCACTTATCAACACACTTAACGA
TCTAAATGAAACTCTTGTTACAATGCCACTTGGCTATGTAACACATGGCTTAAATTTGGAAGAAGCTGCTCGGTATATGAGATCTCTCAA
AGTGCCAGCTACAGTTTCTGTTTCTTCACCTGATGCTGTTACAGCGTATAATGGTTATCTTACTTCTTCTTCTAAAACACCTGAAGAACATT
TTATTGAAACCATCTCACTTGCTGGTTCCTATAAAGATTGGTCCTATTCTGGACAATCTACACAACTAGGTATAGAATTTCTTAAGAGAGG
TGATAAAAGTGTATATTACACTAGTAATCCTACCACATTCCACCTAGATGGTGAAGTTATCACCTTTGACAATCTTAAGACACTTCTTTCTT
TGAGAGAAGTGAGGACTATTAAGGTGTTTACAACAGTAGACAACATTAACCTCCACACGCAAGTTGTGGACATGTCAATGACATATGGA
CAACAGTTTGGTCCAACTTATTTGGATGGAGCTGATGTTACTAAAATAAAACCTCATAATTCACATGAAGGTAAAACATTTTATGTTTTAC
CTAATGATGACACTCTACGTGTTGAGGCTTTTGAGTACTACCACACAACTGATCCTAGTTTTCTGGGTAGGTACATGTCAGCATTAAATCA
CACTAAAAAGTGGAAATACCCACAAGTTAATGGTTTAACTTCTATTAAATGGGCAGATAACAACTGTTATCTTGCCACTGCATTGTTAACA
CTCCAACAAATAGAGTTGAAGTTTAATCCACCTGCTCTACAAGATGCTTATTACAGAGCAAGGGCTGGTGAAGCTGCTAACTTTTGTGCA
CTTATCTTAGCCTACTGTAATAAGACAGTAGGTGAGTTAGGTGATGTTAGAGAAACAATGAGTTACTTGTTTCAACATGCCAATTTAGAT
TCTTGCAAAAGAGTCTTGAACGTGGTGTGTAAAACTTGTGGACAACAGCAGACAACCCTTAAGGGTGTAGAAGCTGTTATGTACATGGG
CACACTTTCTTATGAACAATTTAAGAAAGGTGTTCAGATACCTTGTACGTGTGGTAAACAAGCTACAAAATATCTAGTACAACAGGAGTC
ACCTTTTGTTATGATGTCAGCACCACCTGCTCAGTATGAACTTAAGCATGGTACATTTACTTGTGCTAGTGAGTACACTGGTAATTACCAG
TGTGGTCACTATAAACATATAACTTCTAAAGAAACTTTGTATTGCATAGACGGTGCTTTACTTACAAAGTCCTCAGAATACAAAGGTCCTA
TTACGGATGTTTTCTACAAAGAAAACAGTTACACAACAACCATAAAACCAGTTACTTATAAATTGGATGGTGTTGTTTGTACAGAAATTG
ACCCTAAGTTGGACAATTATTATAAGAAAGACAATTCTTATTTCACAGAGCAACCAATTGATCTTGTACCAAACCAACCATATCCAAACGC
AAGCTTCGATAATTTTAAGTTTGTATGTGATAATATCAAATTTGCTGATGATTTAAACCAGTTAACTGGTTATAAGAAACCTGCTTCAAGA
GAGCTTAAAGTTACATTTTTCCCTGACTTAAATGGTGATGTGGTGGCTATTGATTATAAACACTACACACCCTCTTTTAAGAAAGGAGCTA
AATTGTTACATAAACCTATTGTTTGGCATGTTAACAATGCAACTAATAAAGCCACGTATAAACCAAATACCTGGTGTATACGTTGTCTTTG
GAGCACAAAACCAGTTGAAACATCAAATTCGTTTGATGTACTGAAGTCAGAGGACGCGCAGGGAATGGATAATCTTGCCTGCGAAGAT
CTAAAACCAGTCTCTGAAGAAGTAGTGGAAAATCCTACCATACAGAAAGACGTTCTTGAGTGTAATGTGAAAACTACCGAAGTTGTAGG
AGACATTATACTTAAACCAGCAAATAATAGTTTAAAAATTACAGAAGAGGTTGGCCACACAGATCTAATGGCTGCTTATGTAGACAATTC
TAGTCTTACTATTAAGAAACCTAATGAATTATCTAGAGTATTAGGTTTGAAAACCCTTGCTACTCATGGTTTAGCTGCTGTTAATAGTGTC
CCTTGGGATACTATAGCTAATTATGCTAAGCCTTTTCTTAACAAAGTTGTTAGTACAACTACTAACATAGTTACACGGTGTTTAAACCGTG
TTTGTACTAATTATATGCCTTATTTCTTTACTTTATTGCTACAATTGTGTACTTTTACTAGAAGTACAAATTCTAGAATTAAAGCATCTATGC
CGACTACTATAGCAAAGAATACTGTTAAGAGTGTCGGTAAATTTTGTCTAGAGGCTTCATTTAATTATTTGAAGTCACCTAATTTTTCTAA
ACTGATAAATATTATAATTTGGTTTTTACTATTAAGTGTTTGCCTAGGTTCTTTAATCTACTCAACCGCTGCTTTAGGTGTTTTAATGTCTAA
TTTAGGCATGCCTTCTTACTGTACTGGTTACAGAGAAGGCTATTTGAACTCTACTAATGTCACTATTGCAACCTACTGTACTGGTTCTATAC
CTTGTAGTGTTTGTCTTAGTGGTTTAGATTCTTTAGACACCTATCCTTCTTTAGAAACTATACAAATTACCATTTCATCTTTTAAATGGGATT
TAACTGCTTTTGGCTTAGTTGCAGAGTGGTTTTTGGCATATATTCTTTTCACTAGGTTTTTCTATGTACTTGGATTGGCTGCAATCATGCAA
TTGTTTTTCAGCTATTTTGCAGTACATTTTATTAGTAATTCTTGGCTTATGTGGTTAATAATTAATCTTGTACAAATGGCCCCGATTTCAGCT
ATGGTTAGAATGTACATCTTCTTTGCATCATTTTATTATGTATGGAAAAGTTATGTGCATGTTGTAGACGGTTGTAATTCATCAACTTGTAT
GATGTGTTACAAACGTAATAGAGCAACAAGAGTCGAATGTACAACTATTGTTAATGGTGTTAGAAGGTCCTTTTATGTCTATGCTAATGG
AGGTAAAGGCTTTTGCAAACTACACAATTGGAATTGTGTTAATTGTGATACATTCTGTGCTGGTAGTACATTTATTAGTGATGAAGTTGC
GAGAGACTTGTCACTACAGTTTAAAAGACCAATAAATCCTACTGACCAGTCTTCTTACATCGTTGATAGTGTTACAGTGAAGAATG
GTTCCATCCATCTTTACTTTGATAAAGCTGGTCAAAAGACTTATGAAAGACATTCTCTCTCTCATTTTGTTAACTTAGACAACCTGAGAGCT
AATAACACTAAAGGTTCATTGCCTATTAATGTTATAGTTTTTGATGGTAAATCAAAATGTGAAGAATCATCTGCAAAATCAGCGTCTGTTT
ACTACAGTCAGCTTATGTGTCAACCTATACTGTTACTAGATCAGGCATTAGTGTCTGATGTTGGTGATAGTGCGGAAGTTGCAGTTAAAA
TGTTTGATGCTTACGTTAATACGTTTTCATCAACTTTTAACGTACCAATGGAAAAACTCAAAACACTAGTTGCAACTGCAGAAGCTGAACT
TGCAAAGAATGTGTCCTTAGACAATGTCTTATCTACTTTTATTTCAGCAGCTCGGCAAGGGTTTGTTGATTCAGATGTAGAAACTAAAGA
TGTTGTTGAATGTCTTAAATTGTCACATCAATCTGACATAGAAGTTACTGGCGATAGTTGTAATAACTATATGCTCACCTATAACAAAGTT
GAAAACATGACACCCCGTGACCTTGGTGCTTGTATTGACTGTAGTGCGCGTCATATTAATGCGCAGGTAGCAAAAAGTCACAACATTGC
TTTGATATGGAACGTTAAAGATTTCATGTCATTGTCTGAACAACTACGAAAACAAATACGTAGTGCTGCTAAAAAGAATAACTTACCTTTT
AAGTTGACATGTGCAACTACTAGACAAGTTGTTAATGTTGTAACAACAAAGATAGCACTTAAGGGTGGTAAAATTGTTAATAATTGGTTG
AAGCAGTTAATTAAAGTTACACTTGTGTTCCTTTTTGTTGCTGCTATTTTCTATTTAATAACACCTGTTCATGTCATGTCTAAACATACTGAC
TTTTCAAGTGAAATCATAGGATACAAGGCTATTGATGGTGGTGTCACTCGTGACATAGCATCTACAGATACTTGTTTTGCTAACAAACAT
GCTGATTTTGACACATGGTTTAGCCAGCGTGGTGGTAGTTATACTAATGACAAAGCTTGCCCATTGATTGCTGCAGTCATAACAAGAGAA
 
GTGGGTTTTGTCGTGCCTGGTTTGCCTGGCACGATATTACGCACAACTAATGGTGACTTTTTGCATTTCTTACCTAGAGTTTTTAGTGCAG
TTGGTAACATCTGTTACACACCATCAAAACTTATAGAGTACACTGACTTTGCAACATCAGCTTGTGTTTTGGCTGCTGAATGTACAATTTT
TAAAGATGCTTCTGGTAAGCCAGTACCATATTGTTATGATACCAATGTACTAGAAGGTTCTGTTGCTTATGAAAGTTTACGCCCTGACAC
ACGTTATGTGCTCATGGATGGCTCTATTATTCAATTTCCTAACACCTACCTTGAAGGTTCTGTTAGAGTGGTAACAACTTTTGATTCTGAG
TACTGTAGGCACGGCACTTGTGAAAGATCAGAAGCTGGTGTTTGTGTATCTACTAGTGGTAGATGGGTACTTAACAATGATTATTACAG
ATCTTTACCAGGAGTTTTCTGTGGTGTAGATGCTGTAAATTTACTTACTAATATGTTTACACCACTAATTCAACCTATTGGTGCTTTGGACA
TATCAGCATCTATAGTAGCTGGTGGTATTGTAGCTATCGTAGTAACATGCCTTGCCTACTATTTTATGAGGTTTAGAAGAGCTTTTGGTGA
ATACAGTCATGTAGTTGCCTTTAATACTTTACTATTCCTTATGTCATTCACTGTACTCTGTTTAACACCAGTTTACTCATTCTTACCTGGTGT
TTATTCTGTTATTTACTTGTACTTGACATTTTATCTTACTAATGATGTTTCTTTTTTAGCACATATTCAGTGGATGGTTATGTTCACACCTTTA
GTACCTTTCTGGATAACAATTGCTTATATCATTTGTATTTCCACAAAGCATTTCTATTGGTTCTTTAGTAATTACCTAAAGAGACGTGTA
GTCTTTAATGGTGTTTCCTTTAGTACTTTTGAAGAAGCTGCGCTGTGCACCTTTTTGTTAAATAAAGAAATGTATCTAAAGTTGCGTAGTG
ATGTGCTATTACCTCTTACGCAATATAATAGATACTTAGCTCTTTATAATAAGTACAAGTATTTTAGTGGAGCAATGGATACAACTAGCTA
CAGAGAAGCTGCTTGTTGTCATCTCGCAAAGGCTCTCAATGACTTCAGTAACTCAGGTTCTGATGTTCTTTACCAACCACCACAAACCTCT
ATCACCTCAGCTGTTTTGCAGAGTGGTTTTAGAAAAATGGCATTCCCATCTGGTAAAGTTGAGGGTTGTATGGTACAAGTAACTTGTGGT
ACAACTACACTTAACGGTCTTTGGCTTGATGACGTAGTTTACTGTCCAAGACATGTGATCTGCACCTCTGAAGACATGCTTAACCCTAATT
ATGAAGATTTACTCATTCGTAAGTCTAATCATAATTTCTTGGTACAGGCTGGTAATGTTCAACTCAGGGTTATTGGACATTCTATGCAAAA
TTGTGTACTTAAGCTTAAGGTTGATACAGCCAATCCTAAGACACCTAAGTATAAGTTTGTTCGCATTCAACCAGGACAGACTTTTTCAGTG
TTAGCTTGTTACAATGGTTCACCATCTGGTGTTTACCAATGTGCTATGAGGCCCAATTTCACTATTAAGGGTTCATTCCTTAATGGTTCATG
TGGTAGTGTTGGTTTTAACATAGATTATGACTGTGTCTCTTTTTGTTACATGCACCATATGGAATTACCAACTGGAGTTCATGCTGGCACA
GACTTAGAAGGTAACTTTTATGGACCTTTTGTTGACAGGCAAACAGCACAAGCAGCTGGTACGGACACAACTATTACAGTTAATGTTTTA
GCTTGGTTGTACGCTGCTGTTATAAATGGAGACAGGTGGTTTCTCAATCGATTTACCACAACTCTTAATGACTTTAACCTTGTGGCTATGA
AGTACAATTATGAACCTCTAACACAAGACCATGTTGACATACTAGGACCTCTTTCTGCTCAAACTGGAATTGCCGTTTTAGATATGTGTGC
TTCATTAAAAGAATTACTGCAAAATGGTATGAATGGACGTACCATATTGGGTAGTGCTTTATTAGAAGATGAATTTACACCTTTTGATGTT
GTTAGACAATGCTCAGGTGTTACTTTCCAAAGTGCAGTGAAAAGAACAATCAAGGGTACACACCACTGGTTGTTACTCACAATTTTGACT
TCACTTTTAGTTTTAGTCCAGAGTACTCAATGGTCTTTGTTCTTTTTTTTGTATGAAAATGCCTTTTTACCTTTTGCTATGGGTATTATTGCT
ATGTCTGCTTTTGCAATGATGTTTGTCAAACATAAGCATGCATTTCTCTGTTTGTTTTTGTTACCTTCTCTTGCCACTGTAGCTTATTTTAAT
ATGGTCTATATGCCTGCTAGTTGGGTGATGCGTATTATGACATGGTTGGATATGGTTGATACTAGTTTGTCTGGTTTTAAGCTAAAAGAC
TGTGTTATGTATGCATCAGCTGTAGTGTTACTAATCCTTATGACAGCAAGAACTGTGTATGATGATGGTGCTAGGAGAGTGTGGACACTT
ATGAATGTCTTGACACTCGTTTATAAAGTTTATTATGGTAATGCTTTAGATCAAGCCATTTCCATGTGGGCTCTTATAATCTCTGTTACTTC
TAACTACTCAGGTGTAGTTACAACTGTCATGTTTTTGGCCAGAGGTATTGTTTTTATGTGTGTTGAGTATTGCCCTATTTTCTTCATAACTG
GTAATACACTTCAGTGTATAATGCTAGTTTATTGTTTCTTAGGCTATTTTTGTACTTGTTACTTTGGCCTCTTTTGTTTACTCAACCGCTACT
TTAGACTGACTCTTGGTGTTTATGATTACTTAGTTTCTACACAGGAGTTTAGATATATGAATTCACAGGGACTACTCCCACCCAAGAATAG
CATAGATGCCTTCAAACTCAACATTAAATTGTTGGGTGTTGGTGGCAAACCTTGTATCAAAGTAGCCACTGTACAGTCTAAAATGTCAGA
TGTAAAGTGCACATCAGTAGTCTTACTCTCAGTTTTGCAACAACTCAGAGTAGAATCATCATCTAAATTGTGGGCTCAATGTGTCCAGTTA
CACAATGACATTCTCTTAGCTAAAGATACTACTGAAGCCTTTGAAAAAATGGTTTCACTACTTTCTGTTTTGCTTTCCATGCAGGGTGCTG
TAGACATAAACAAGCTTTGTGAAGAAATGCTGGACAACAGGGCAACCTTACAAGCTATAGCCTCAGAGTTTAGTTCCCTTCCATCATATG
CAGCTTTTGCTACTGCTCAAGAAGCTTATGAGCAGGCTGTTGCTAATGGTGATTCTGAAGTTGTTCTTAAAAAGTTGAAGAAGTCTTTGA
ATGTGGCTAAATCTGAATTTGACCGTGATGCAGCCATGCAACGTAAGTTGGAAAAGATGGCTGATCAAGCTATGACCCAAATGTATAAA
CAGGCTAGATCTGAGGACAAGAGGGCAAAAGTTACTAGTGCTATGCAGACAATGCTTTTCACTATGCTTAGAAAGTTGGATAATGATGC
ACTCAACAACATTATCAACAATGCAAGAGATGGTTGTGTTCCCTTGAACATAATACCTCTTACAACAGCAGCCAAACTAATGGTTGTCAT
ACCAGACTATAACACATATAAAAATACGTGTGATGGTACAACATTTACTTATGCATCAGCATTGTGGGAAATCCAACAGGTTGTAGATGC
AGATAGTAAAATTGTTCAACTTAGTGAAATTAGTATGGACAATTCACCTAATTTAGCATGGCCTCTTATTGTAACAGCTTTAAGGGCCAAT
TCTGCTGTCAAATTACAGAATAATGAGCTTAGTCCTGTTGCACTACGACAGATGTCTTGTGCTGCCGGTACTACACAAACTGCTTGCACT
GATGACAATGCGTTAGCTTACTACAACACAACAAAGGGAGGTAGGTTTGTACTTGCACTGTTATCCGATTTACAGGATTTGAAATGGGC
TAGATTCCCTAAGAGTGATGGAACTGGTACTATCTATACAGAACTGGAACCACCTTGTAGGTTTGTTACAGACACACCTAAAGGTCCTAA
AGTGAAGTATTTATACTTTATTAAAGGATTAAACAACCTAAATAGAGGTATGGTACTTGGTAGTTTAGCTGCCACAGTACGTCTACAAGC
TGGTAATGCAACAGAAGTGCCTGCCAATTCAACTGTATTATCTTTCTGTGCTTTTGCTGTAGATGCTGCTAAAGCTTACAAAGATTATCTA
GCTAGTGGGGGACAACCAATCACTAATTGTGTTAAGATGTTGTGTACACACACTGGTACTGGTCAGGCAATAACAGTTACACCGGAAGC
CAATATGGATCAAGAATCCTTTGGTGGTGCATCGTGTTGTCTGTACTGCCGTTGCCACATAGATCATCCAAATCCTAAAGGATTTTGTGA
CTTAAAAGGTAAGTATGTACAAATACCTACAACTTGTGCTAATGACCCTGTGGGTTTTACACTTAAAAACACAGTCTGTACCGTCTGCGG
TATGTGGAAAGGTTATGGCTGTAGTTGTGATCAACTCCGCGAACCCATGCTTCAGTCAGCTGATGCACAATCGTTTTTAAACGGGTTTGC
GGTGTAAGTGCAGCCCGTCTTACACCGTGCGGCACAGGCACTAGTACTGATGTCGTATACAGGGCTTTTGACATCTACAATGATAAAGT
AGCTGGTTTTGCTAAATTCCTAAAAACTAATTGTTGTCGCTTCCAAGAAAAGGACGAAGATGACAATTTAATTGATTCTTACTTTGTAGTT
AAGAGACACACTTTCTCTAACTACCAACATGAAGAAACAATTTATAATTTACTTAAGGATTGTCCAGCTGTTGCTAAACATGACTTCTTTA
AGTTTAGAATAGACGGTGACATGGTACCACATATATCACGTCAACGTCTTACTAAATACACAATGGCAGACCTCGTCTATGCTTTAAGGC
ATTTTGATGAAGGTAATTGTGACACATTAAAAGAAATACTTGTCACATACAATTGTTGTGATGATGATTATTTCAATAAAAAGGACTGGT
ATGATTTTGTAGAAAACCCAGATATATTACGCGTATACGCCAACTTAGGTGAACGTGTACGCCAAGCTTTGTTAAAAACAGTACAATTCT
GTGATGCCATGCGAAATGCTGGTATTGTTGGTGTACTGACATTAGATAATCAAGATCTCAATGGTAACTGGTATGATTTCGGTGATTTCA
TACAAACCACGCCAGGTAGTGGAGTTCCTGTTGTAGATTCTTATTATTCATTGTTAATGCCTATATTAACCTTGACCAGGGCTTTAACTGC
AGAGTCACATGTTGACACTGACTTAACAAAGCCTTACATTAAGTGGGATTTGTTAAAATATGACTTCACGGAAGAGAGGTTAAAACTCTT
TGACCGTTATTTTAAATATTGGGATCAGACATACCACCCAAATTGTGTTAACTGTTTGGATGACAGATGCATTCTGCATTGTGCAAACTTT
AATGTTTTATTCTCTACAGTGTTCCCACCTACAAGTTTTGGACCACTAGTGAGAAAAATATTTGTTGATGGTGTTCCATTTGTAGTTTCAAC
 
TGGATACCACTTCAGAGAGCTAGGTGTTGTACATAATCAGGATGTAAACTTACATAGCTCTAGACTTAGTTTTAAGGAATTACTTGTGTA
TGCTGCTGACCCTGCTATGCACGCTGCTTCTGGTAATCTATTACTAGATAAACGCACTACGTGCTTTTCAGTAGCTGCACTTACTAACAAT
GTTGCTTTTCAAACTGTCAAACCCGGTAATTTTAACAAAGACTTCTATGACTTTGCTGTGTCTAAGGGTTTCTTTAAGGAAGGAAGTTCTG
TTGAATTAAAACACTTCTTCTTTGCTCAGGATGGTAATGCTGCTATCAGCGATTATGACTACTATCGTTATAATCTACCAACAATGTGTGA
TATCAGACAACTACTATTTGTAGTTGAAGTTGTTGATAAGTACTTTGATTGTTACGATGGTGGCTGTATTAATGCTAACCAAGTCATCGTC
AACAACCTAGACAAATCAGCTGGTTTTCCATTTAATAAATGGGGTAAGGCTAGACTTTATTATGATTCAATGAGTTATGAGGATCAAGAT
GCACTTTTCGCATATACAAAACGTAATGTCATCCCTACTATAACTCAAATGAATCTTAAGTATGCCATTAGTGCAAAGAATAGAGCTCGCA
CCGTAGCTGGTGTCTCTATCTGTAGTACTATGACCAATAGACAGTTTCATCAAAAATTATTGAAATCAATAGCCGCCACTAGAGGAGCTA
CTGTAGTAATTGGAACAAGCAAATTCTATGGTGGTTGGCACAACATGTTAAAAACTGTTTATAGTGATGTAGAAAACCCTCACCTTATGG
GTTGGGATTATCCTAAATGTGATAGAGCCATGCCTAACATGCTTAGAATTATGGCCTCACTTGTTCTTGCTCGCAAACATACAACGTGTTG
TAGCTTGTCACACCGTTTCTATAGATTAGCTAATGAGTGTGCTCAAGTATTGAGTGAAATGGTCATGTGTGGCGGTTCACTATATGTTAA
ACCAGGTGGAACCTCATCAGGAGATGCCACAACTGCTTATGCTAATAGTGTTTTTAACATTTGTCAAGCTGTCACGGCCAATGTTAATGC
ACTTTTATCTACTGATGGTAACAAAATTGCCGATAAGTATGTCCGCAATTTACAACACAGACTTTATGAGTGTCTCTATAGAAATAGAGAT
GTTGACACAGACTTTGTGAATGAGTTTTACGCATATTTGCGTAAACATTTCTCAATGATGATACTCTCTGACGATGCTGTTGTGTGTTTCA
ATAGCACTTATGCATCTCAAGGTCTAGTGGCTAGCATAAAGAACTTTAAGTCAGTTCTTTATTATCAAAACAATGTTTTTATGTCTGAAGC
AAAATGTTGGACTGAGACTGACCTTACTAAAGGACCTCATGAATTTTGCTCTCAACATACAATGCTAGTTAAACAGGGTGATGATTATGT
GTACCTTCCTTACCCAGATCCATCAAGAATCCTAGGGGCCGGCTGTTTTGTAGATGATATCGTAAAAACAGATGGTACACTTATGATTGA
ACGGTTCGTGTCTTTAGCTATAGATGCTTACCCACTTACTAAACATCCTAATCAGGAGTATGCTGATGTCTTTCATTTGTACTTACAATACA
TAAGAAAGCTACATGATGAGTTAACAGGACACATGTTAGACATGTATTCTGTTATGCTTACTAATGATAACACTTCAAGGTATTGGGAAC
CTGAGTTTTATGAGGCTATGTACACACCGCATACAGTCTTACAGGCTGTTGGGGCTTGTGTTCTTTGCAATTCACAGACTTCATTAAGATG
TGGTGCTTGCATACGTAGACCATTCTTATGTTGTAAATGCTGTTACGACCATGTCATATCAACATCACATAAATTAGTCTTGTCTGTTAATC
CGTATGTTTGCAATGCTCCAGGTTGTGATGTCACAGATGTGACTCAACTTTACTTAGGAGGTATGAGCTATTATTGTAAATCACATAAAC
CACCCATTAGTTTTCCATTGTGTGCTAATGGACAAGTTTTTGGTTTATATAAAAATACATGTGTTGGTAGCGATAATGTTACTGACTTTAA
TGCAATTGCAACATGTGACTGGACAAATGCTGGTGATTACATTTTAGCTAACACCTGTACTGAAAGACTCAAGCTTTTTGCAGCAGAAAC
GCTCAAAGCTACTGAGGAGACATTTAAACTGTCTTATGGTATTGCTACTGTACGTGAAGTGCTGTCTGACAGAGAATTACATCTTTCATG
GGAAGTTGGTAAACCTAGACCACCACTTAACCGAAATTATGTCTTTACTGGTTATCGTGTAACTAAAAACAGTAAAGTACAAATAGGAG
AGTACACCTTTGAAAAAGGTGACTATGGTGATGCTGTTGTTTACCGAGGTACAACAACTTACAAATTAAATGTTGGTGATTATTTTGTGC
TGACATCACATACAGTAATGCCATTAAGTGCACCTACACTAGTGCCACAAGAGCACTATGTTAGAATTACTGGCTTATACCCAACACTCA
ATATCTCAGATGAGTTTTCTAGCAATGTTGCAAATTATCAAAAGGTTGGTATGCAAAAGTATTCTACACTCCAGGGACCACCTGGTACTG
GTAAGAGTCATTTTGCTATTGGCCTAGCTCTCTACTACCCTTCTGCTCGCATAGTGTATACAGCTTGCTCTCATGCCGCTGTTGATGCACTA
TGTGAGAAGGCATTAAAATATTTGCCTATAGATAAATGTAGTAGAATTATACCTGCACGTGCTCGTGTAGAGTGTTTTGATAAATTCAAA
GTGAATTCAACATTAGAACAGTATGTCTTTTGTACTGTAAATGCATTGCCTGAGACGACAGCAGATATAGTTGTCTTTGATGAAATTTCA
ATGGCCACAAATTATGATTTGAGTGTTGTCAATGCCAGATTACGTGCTAAGCACTATGTGTACATTGGCGACCCTGCTCAATTACCTGCA
CCACGCACATTGCTAACTAAGGGCACACTAGAACCAGAATATTTCAATTCAGTGTGTAGACTTATGAAAACTATAGGTCCAGACATGTTC
CTCGGAACTTGTCGGCGTTGTCCTGCTGAAATTGTTGACACTGTGAGTGCTTTGGTTTATGATAATAAGCTTAAAGCACATAAAGACAAA
TCAGCTCAATGCTTTAAAATGTTTTATAAGGGTGTTATCACGCATGATGTTTCATCTGCAATTAACAGGCCACAAATAGGCGTGGTAAGA
GAATTCCTTACACGTAACCCTGCTTGGAGAAAAGCTGTCTTTATTTCACCTTATAATTCACAGAATGCTGTAGCCTCAAAGATTTTGGGAC
TACCAACTCAAACTGTTGATTCATCACAGGGCTCAGAATATGACTATGTCATATTCACTCAAACCACTGAAACAGCTCACTCTTGTAATGT
AAACAGATTTAATGTTGCTATTACCAGAGCAAAAGTAGGCATACTTTGCATAATGTCTGATAGAGACCTTTATGACAAGTTGCAATTTAC
AAGTCTTGAAATTCCACGTAGGAATGTGGCAACTTTACAAGCTGAAAATGTAACAGGACTCTTTAAAGATTGTAGTAAGGTAATCACTG
GGTTACATCCTACACAGGCACCTACACACCTCAGTGTTGACACTAAATTCAAAACTGAAGGTTTATGTGTTGACATACCTGGCATACCTA
AGGACATGACCTATAGAAGACTCATCTCTATGATGGGTTTTAAAATGAATTATCAAGTTAATGGTTACCCTAACATGTTTATCACCCGCG
AAGAAGCTATAAGACATGTACGTGCATGGATTGGCTTCGATGTCGAGGGGTGTCATGCTACTAGAGAAGCTGTTGGTACCAATTTACCT
TTACAGCTAGGTTTTTCTACAGGTGTTAACCTAGTTGCTGTACCTACAGGTTATGTTGATACACCTAATAATACAGATTTTTCCAGAGTTA
GTGCTAAACCACCGCCTGGAGATCAATTTAAACACCTCATACCACTTATGTACAAAGGACTTCCTTGGAATGTAGTGCGTATAAAGATTG
TACAAATGTTAAGTGACACACTTAAAAATCTCTCTGACAGAGTCGTATTTGTCTTATGGGCACATGGCTTTGAGTTGACATCTATGAAGT
ATTTTGTGAAAATAGGACCTGAGCGCACCTGTTGTCTATGTGATAGACGTGCCACATGCTTTTCCACTGCTTCAGACACTTATGCCTGTTG
GCATCATTCTATTGGATTTGATTACGTCTATAATCCGTTTATGATTGATGTTCAACAATGGGGTTTTACAGGTAACCTACAAAGCAACCAT
GATCTGTATTGTCAAGTCCATGGTAATGCACATGTAGCTAGTTGTGATGCAATCATGACTAGGTGTCTAGCTGTCCACGAGTGCTTTGTT
AAGCGTGTTGACTGGACTATTGAATATCCTATAATTGGTGATGAACTGAAGATTAATGCGGCTTGTAGAAAGGTTCAACACATGGTTGTT
AAAGCTGCATTATTAGCAGACAAATTCCCAGTTCTTCACGACATTGGTAACCCTAAAGCTATTAAGTGTGTACCTCAAGCTGATGTAGAA
TGGAAGTTCTATGATGCACAGCCTTGTAGTGACAAAGCTTATAAAATAGAAGAATTATTCTATTCTTATGCCACACATTCTGACAAATTCA
CAGATGGTGTATGCCTATTTTGGAATTGCAATGTCGATAGATATCCTGCTAATTCCATTGTTTGTAGATTTGACACTAGAGTGCTATCTAA
CCTTAACTTGCCTGGTTGTGATGGTGGCAGTTTGTATGTAAATAAACATGCATTCCACACACCAGCTTTTGATAAAAGTGCTTTTGTTAAT
TTAAAACAATTACCATTTTTCTATTACTCTGACAGTCCATGTGAGTCTCATGGAAAACAAGTAGTGTCAGATATAGATTATGTACCACTAA
AGTCTGCTACGTGTATAACACGTTGCAATTTAGGTGGTGCTGTCTGTAGACATCATGCTAATGAGTACAGATTGTATCTCGATGCTTATA
ACATGATGATCTCAGCTGGCTTTAGCTTGTGGGTTTACAAACAATTTGATACTTATAACCTCTGGAACACTTTTACAAGACTTCAGAGTTT
AGAAAATGTGGCTTTTAATGTTGTAAATAAGGGACACTTTGATGGACAACAGGGTGAAGTACCAGTTTCTATCATTAATAACACTGTTTA
CACAAAAGTTGATGGTGTTGATGTAGAATTGTTTGAAAATAAAACAACATTACCTGTTAATGTAGCATTTGAGCTTTGGGCTAAGCGCAA
CATTAAACCAGTACCAGAGGTGAAAATACTCAATAATTTGGGTGTGGACATTGCTGCTAATACTGTGATCTGGGACTACAAAAGAGATG
CTCCAGCACATATATCTACTATTGGTGTTTGTTCTATGACTGACATAGCCAAGAAACCAACTGAAACGATTTGTGCACCACTCACTGTCTT
TTTTGATGGTAGAGTTGATGGTCAAGTAGACTTATTTAGAAATGCCCGTAATGGTGTTCTTATTACAGAAGGTAGTGTTAAAGGTTTACA
 
ACCATCTGTAGGTCCCAAACAAGCTAGTCTTAATGGAGTCACATTAATTGGAGAAGCCGTAAAAACACAGTTCAATTATTATAAGAAAGT
TGATGGTGTTGTCCAACAATTACCTGAAACTTACTTTACTCAGAGTAGAAATTTACAAGAATTTAAACCCAGGAGTCAAATGGAAATTGA
TTTCTTAGAATTAGCTATGGATGAATTCATTGAACGGTATAAATTAGAAGGCTATGCCTTCGAACATATCGTTTATGGAGATTTTAGTCAT
AGTCAGTTAGGTGGTTTACATCTACTGATTGGACTAGCTAAACGTTTTAAGGAATCACCTTTTGAATTAGAAGATTTTATTCCTATGGACA
GTACAGTTAAAAACTATTTCATAACAGATGCGCAAACAGGTTCATCTAAGTGTGTGTGTTCTGTTATTGATTTATTACTTGATGATTTTGTT
GAAATAATAAAATCCCAAGATTTATCTGTAGTTTCTAAGGTTGTCAAAGTGACTATTGACTATACAGAAATTTCATTTATGCTTTGGTGTA
AAGATGGCCATGTAGAAACATTTTACCCAAAATTACAATCTAGTCAAGCGTGGCAACCGGGTGTTGCTATGCCTAATCTTTACAAAATGC
AAAGAATGCTATTAGAAAAGTGTGACCTTCAAAATTATGGTGATAGTGCAACATTACCTAAAGGCATAATGATGAATGTCGCAAAATAT
ACTCAACTGTGTCAATATTTAAACACATTAACATTAGCTGTACCCTATAATATGAGAGTTATACATTTTGGTGCTGGTTCTGATAAAGGAG
TTGCACCAGGTACAGCTGTTTTAAGACAGTGGTTGCCTACGGGTACGCTGCTTGTCGATTCAGATCTTAATGACTTTGTCTCTGATGCAG
ATTCAACTTTGATTGGTGATTGTGCAACTGTACATACAGCTAATAAATGGGATCTCATTATTAGTGATATGTACGACCCTAAGACTAAAA
ATGTTACAAAAGAAAATGACTCTAAAGAGGGTTTTTCACTTACATTTGTGGGTTTATACAACAAAAGCTAGCTCTTGGAGGTTCCGTGG
CTATAAAGATAACAGAACATTCTTGGAATGCTGATCTTTATAAGCTCATGGGACACTTCGCATGGTGGACAGCCTTTGTTACTAATGTGA
ATGCGTCATCATCTGAAGCATTTTTAATTGGATGTAATTATCTTGGCAAACCACGCGAACAAATAGATGGTTATGTCATGCATGCAAATT
ACATATTTTGGAGGAATACAAATCCAATTCAGTTGTCTTCCTATTCTTTATTTGACATGAGTAAATTTCCCCTTAAATTAAGGGGTACTGCT
GTTATGTCTTTAAAAGAAGGTCAAATCAATGATATGATTTTATCTCTTCTTAGTAAAGGTAGACTTATAATTAGAGAAAACAACAGAGTT
GTTATTTCTAGTGATGTTCTTGTTAACAACTAAACGAACAATGTTTGTTTTTCTTGTTTTATTGCCACTAGTCTCTAGTCAGTGTGTTAATCT
TACAACCAGAACTCAATTACCCCCTGCATACACTAATTCTTTCACACGTGGTGTTTATTACCCTGACAAAGTTTTCAGATCCTCAGTTTTAC
ATTCAACTCAGGACTTGTTCTTACCTTTCTTTTCCAATGTTACTTGGTTCCATGCTATACATGTCTCTGGGACCAATGGTACTAAGAGGTTT
GATAACCCTGTCCTACCATTTAATGATGGTGTTTATTTTGCTTCCACTGAGAAGTCTAACATAATAAGAGGCTGGATTTTTGGTACTACTT
TAGATTCGAAGACCCAGTCCCTACTTATTGTTAATAACGCTACTAATGTTGTTATTAAAGTCTGTGAATTTCAATTTTGTAATGATCCATTT
TTGGGTGTTTATTACCACAAAAACAACAAAAGTTGGATGGAAAGTGAGTTCAGAGTTTATTCTAGTGCGAATAATTGCACTTTTGAATAT
GTCTCTCAGCCTTTTCTTATGGACCTTGAAGGAAAACAGGGTAATTTCAAAAATCTTAGGGAATTTGTGTTTAAGAATATTGATGGTTATT
TTAAAATATATTCTAAGCACACGCCTATTAATTTAGTGCGTGATCTCCCTCAGGGTTTTTCGGCTTTAGAACCATTGGTAGATTTGCCAAT
AGGTATTAACATCACTAGGTTTCAAACTTTACTTGCTTTACATAGAAGTTATTTGACTCCTGGTGATTCTTCTTCAGGTTGGACAGCTGGT
GCTGCAGCTTATTATGTGGGTTATCTTCAACCTAGGACTTTTCTATTAAAATATAATGAAAATGGAACCATTACAGATGCTGTAGACTGTG
CACTTGACCCTCTCTCAGAAACAAAGTGTACGTTGAAATCCTTCACTGTAGAAAAAGGAATCTATCAAACTTCTAACTTTAGAGTCCAACC
AACAGAATCTATTGTTAGATTTCCTAATATTACAAACTTGTGCCCTTTTGGTGAAGTTTTTAACGCCACCAGATTTGCATCTGTTTATGCTT
GGAACAGGAAGAGAATCAGCAACTGTGTTGCTGATTATTCTTTCCTATATAATTCCGCATCATTTTCCACTTTTAAGTGTTATGGAGTGTC
TCCTACTAAATTAAATGATCTCTGCTTTACTAATGTCTATGCAGATTCATTTGTAATTAGAGGTGATGAAGTCAGACAAATCGCTCCAGGG
CAAACTGGAAAGATTGCTGATTATAATTATAAATTACCAGATGATTTTACAGGCTGCGTTATAGCTTGGAATTCTAACAATCTTGATTCTA
AGGTTGGTGGTAATTATAATTACCTGTATAGATTGTTTAGGAAGTCTAATCTCAAACCTTTTGAGAGAGATATTTCAACTGAAATCTATCA
GGCCGGTAGCACACCTTGTAATGGTGTTGAAGGTTTTAATTGTTACTTTCCTTTACAATCATATGGTTTCCAACCCACTAATGGTGTTGGT
TACCAACCATACAGAGTAGTAGTACTTTCTTTTGAACTTCTACATGCACCAGCAACTGTTTGTGGACCTAAAAAGTCTACTAATTTGGTTA
AAAACAAATGTGTCAATTTCAACTTCAATGGTTTAACAGGCACAGGTGTTCTTACTGAGTCTAACAAAAAGTTTCTGCCTTTCCAACAATT
TGGCAGAGACATTGCTGACACTACTGATGCTGTCCGTGATCCACAGACACTTGAGATTCTTGACATTACACCATGTTCTTTTGGTGGTGTC
AGTGTTATAACACCAGGAACAAATACTTCTAACCAGGTTGCTGTTCTTTATCAGGATGTTAACTGCACAGAAGTCCCTGTTGCTATTCATG
CAGATCAACTTACTCCTACTTGGCGTGTTTATTCTACAGGTTCTAATGTTTTTCAAACACGTGCAGGCTGTTTAATAGGGGCTGAACATGT
CAACAACTCATATGAGTGTGACATACCCATTGGTGCAGGTATATGCGCTAGTTATCAGACTCAGACTAATTCTCCTCGGCGGGCACGTAG
TGTAGCTAGTCAATCCATCATTGCCTACACTATGTCACTTGGTGCAGAAAATTCAGTTGCTTACTCTAATAACTCTATTGCCATACCCACAA
ATTTTACTATTAGTGTTACCACAGAAATTCTACCAGTGTCTATGACCAAGACATCAGTAGATTGTACAATGTACATTTGTGGTGATTCAAC
TGAATGCAGCAATCTTTTGTTGCAATATGGCAGTTTTTGTACACAATTAAACCGTGCTTTAACTGGAATAGCTGTTGAACAAGACAAAAA
CACCCAAGAAGTTTTTGCACAAGTCAAACAAATTTACAAAACACCACCAATTAAAGATTTTGGTGGTTTTAATTTTTCACAAATATTACCA
GATCCATCAAAACCAAGCAAGAGGTCATTTATTGAAGATCTACTTTTCAACAAAGTGACACTTGCAGATGCTGGCTTCATCAAACAATAT
GGTGATTGCCTTGGTGATATTGCTGCTAGAGACCTCATTTGTGCACAAAAGTTTAACGGCCTTACTGTTTTGCCACCTTTGCTCACAGATG
AAATGATTGCTCAATACACTTCTGCACTGTTAGCGGGTACAATCACTTCTGGTTGGACCTTTGGTGCAGGTGCTGCATTACAAATACCATT
TGCTATGCAAATGGCTTATAGGTTTAATGGTATTGGAGTTACACAGAATGTTCTCTATGAGAACCAAAAATTGATTGCCAACCAATTTAA
TAGTGCTATTGGCAAAATTCAAGACTCACTTTCTTCCACAGCAAGTGCACTTGGAAAACTTCAAGATGTGGTCAACCAAAATGCACAAGC
TTTAAACACGCTTGTTAAACAACTTAGCTCCAATTTTGGTGCAATTTCAAGTGTTTTAAATGATATCCTTTCACGTCTTGACAAAGTTGAG
GCTGAAGTGCAAATTGATAGGTTGATCACAGGCAGACTTCAAAGTTTGCAGACATATGTGACTCAACAATTAATTAGAGCTGCAGAAAT
CAGAGCTTCTGCTAATCTTGCTGCTACTAAAATGTCAGAGTGTGTACTTGGACAATCAAAAAGAGTTGATTTTTGTGGAAAGGGCTATCA
TCTTATGTCCTTCCCTCAGTCAGCACCTCATGGTGTAGTCTTCTTGCATGTGACTTATGTCCCTGCACAAGAAAAGAACTTCACAACTGCTC
CTGCCATTTGTCATGATGGAAAAGCACACTTTCCTCGTGAAGGTGTCTTTGTTTCAAATGGCACACACTGGTTTGTAACACAAAGGAATT
TTTATGAACCACAAATCATTACTACAGACAACACATTTGTGTCTGGTAACTGTGATGTTGTAATAGGAATTGTCAACAACACAGTTTATGA
TCCTTTGCAACCTGAATTAGACTCATTCAAGGAGGAGTTAGATAAATATTTTAAGAATCATACATCACCAGATGTTGATTTAGGTGACATC
TCTGGCATTAATGCTTCAGTTGTAAACATTCAAAAAGAAATTGACCGCCTCAATGAGGTTGCCAAGAATTTAAATGAATCTCTCATCGATC
TCCAAGAACTTGGAAAGTATGAGCAGTATATAAAATGGCCATGGTACATTTGGCTAGGTTTTATAGCTGGCTTGATTGCCATAGTAATG
GTGACAATTATGCTTTGCTGTATGACCAGTTGCTGTAGTTGTCTCAAGGGCTGTTGTTCTTGTGGATCCTGCTGCAAATTTGATGAAGAC
GACTCTGAGCCAGTGCTCAAAGGAGTCAAATTACATTACACATAAACGAACTTATGGATTTGTTTATGAGAATCTTCACAATTGGAACTG
TAACTTTGAAGCAAGGTGAAATCAAGGATGCTACTCCTTCAGATTTTGTTCGCGCTACTGCAACGATACCGATACAAGCCTCACTCCCTTT
CGGATGGCTTATTGTTGGCGTTGCACTTCTTGCTGTTTTTCAGAGCGCTTCCAAAATCATAACCCTCAAAAAGAGATGGCAACTAGCACT
CTCCAAGGGTGTTCACTTTGTTTGCAACTTGCTGTTGTTGTTTGTAACAGTTTACTCACACCTTTTGCTCGTTGCTGCTGGCCTTGAAGCCC
 
CTTTTCTCTATCTTTATGCTTTAGTCTACTTCTTGCAGAGTATAAACTTTGTAAGAATAATAATGAGGCTTTGGCTTTGCTGGAAATGCCGT
TCCAAAAACCCATTACTTTATGATGCCAACTATTTTCTTTGCTGGCATACTAATTGTTACGACTATTGTATACCTTACAATAGTGTAACTTCT
TCAATTGTCATTACTTCAGGTGATGGCACAACAAGTCCTATTTCTGAACATGACTACCAGATTGGTGGTTATACTGAAAAATGGGAATCT
GGAGTAAAAGACTGTGTTGTATTACACAGTTACTTCACTTCAGACTATTACCAGCTGTACTCAACTCAATTGAGTACAGACACTGGTGTT
GAACATGTTACCTTCTTCATCTACAATAAAATTGTTGATGAGCCTGAAGAACATGTCCAAATTCACACAATCGACGTTTCATCCGGAGTTG
TTAATCCAGTAATGGAACCAATTTATGATGAACCGACGACGACTACTAGCGTGCCTTTGTAAGCACAAGCTGATGAGTACGAACTTATGT
ACTCATTCGTTTCGGAAGAGACAGGTACGTTAATAGTTAATAGCGTACTTCTTTTTCTTGCTTTCGTGGTATTCTTGCTAGTTACACTAGCC
ATCCTTACTGCGCTTCGATTGTGTGCGTACTGCTGCAATATTGTTAACGTGAGTCTTGTAAAACCTTCTTTTTACGTTTACTCTCGTGTTAA
AAATCTGAATTCTTCTAGAGTTCCTGATCTTCTGGTCTAAACGAACTAAATATTATATTAGTTTTTCTGTTTGGAACTTTAATTTTAGCCAT
GGCAGATTCCAACGGTACTATTACCGTTGAAGAGCTTAAAAAGCTCCTTGAACAATGGAACCTAGTAATAGGTTTCCTATTCCTTACATG
GATTTGTCTTCTACAATTTGCCTATGCCAACAGGAATAGGTTTTTGTATATAATTAAGTTAATTTTCCTCTGGCTGTTATGGCCAGTAACTT
TAGCTTGTTTTGTGCTTGCTGCTGTTTACAGAATAAATTGGATCACCGGTGGAATTGCTATCGCAATGGCTTGTCTTGTAGGCTTGATGTG
GCTCAGCTACTTCATTGCTTCTTTCAGACTGTTTGCGCGTACGCGTTCCATGTGGTCATTCAATCCAGAAACTAACATTCTTCTCAACGTGC
CACTCCATGGCACTATTCTGACCAGACCGCTTCTAGAAAGTGAACTCGTAATCGGAGCTGTGATCCTTCGTGGACATCTTCGTATTGCTG
GACACCATCTAGGACGCTGTGACATCAAGGACCTGCCTAAAGAAATCACTGTTGCTACATCACGAACGCTTTCTTATTACAAATTGGGAG
CTTCGCAGCGTGTAGCAGGTGACTCAGGTTTTGCTGCATACAGTCGCTACAGGATTGGCAACTATAAATTAAACACAGACCATTCCAGTA
GCAGTGACAATATTGCTTTGCTTGTACAGTAAGTGACAACAGATGTTTCATCTCGTTGACTTTCAGGTTACTATAGCAGAGATATTACTAA
TTATTATGAGGACTTTTAAAGTTTCCATTTGGAATCTTGATTACATCATAAACCTCATAATTAAAAATTTATCTAAGTCACTAACTGAGAAT
AAATATTCTCAATTAGATGAAGAGCAACCAATGGAGATTGATTAAACGAACATGAAAATTATTCTTTTCTTGGCACTGATAACACTCGCT
ACTTGTGAGCTTTATCACTACCAAGAGTGTGTTAGAGGTACAACAGTACTTTTAAAAGAACCTTGCTCTTCTGGAACATACGAGGGCAAT
TCACCATTTCATCCTCTAGCTGATAACAAATTTGCACTGACTTGCTTTAGCACTCAATTTGCTTTTGCTTGTCCTGACGGCGTAAAACACGT
CTATCAGTTACGTGCCAGATCAGTTTCACCTAAACTGTTCATCAGACAAGAGGAAGTTCAAGAACTTTACTCTCCAATTTTTCTTATTGTTG
CGGCAATAGTGTTTATAACACTTTGCTTCACACTCAAAAGAAAGACAGAATGATTGAACTTTCATTAATTGACTTCTATTTGTGCTTTTTA
GCCTTTCTGCTATTCCTTGTTTTAATTATGCTTATTATCTTTTGGTTCTCACTTGAACTGCAAGATCATAATGAAACTTGTCACGCCTAAACG
AACATGAAATTTCTTGTTTTCTTAGGAATCATCACAACTGTAGCTGCATTTCACCAAGAATGTAGTTTACAGTCATGTACTCAACATCAAC
CATATGTAGTTGATGACCCGTGTCCTATTCACTTCTATTCTAAATGGTATATTAGAGTAGGAGCTAGAAAATCAGCACCTTTAATTGAATT
GTGCGTGGATGAGGCTGGTTCTAAATCACCCATTCAGTACATCGATATCGGTAATTATACAGTTTCCTGTTTACCTTTTACAATTAATTGC
CAGGAACCTAAATTGGGTAGTCTTGTAGTGCGTTGTTCGTTCTATGAAGACTTTTTAGAGTATCATGACGTTCGTGTTGTTTTAGATTTCA
TCTAAACGAACAAACTAAAATGTCTGATAATGGACCCCAAAATCAGCGAAATGCACCCCGCATTACGTTTGGTGGACCCTCAGATTCAAC
TGGCAGTAACCAGAATGGAGAACGCAGTGGGGCGCGATCAAAACAACGTCGGCCCCAAGGTTTACCCAATAATACTGCGTCTTGGTTC
ACCGCTCTCACTCAACATGGCAAGGAAGACCTTAAATTCCCTCGAGGACAAGGCGTTCCAATTAACACCAATAGCAGTCCAGATGACCA
AATTGGCTACTACCGAAGAGCTACCAGACGAATTCGTGGTGGTGACGGTAAAATGAAAGATCTCAGTCCAAGATGGTATTTCTACTACC
TAGGAACTGGGCCAGAAGCTGGACTTCCCTATGGTGCTAACAAAGACGGCATCATATGGGTTGCAACTGAGGGAGCCTTGAATACACC
AAAAGATCACATTGGCACCCGCAATCCTGCTAACAATGCTGCAATCGTGCTACAACTTCCTCAAGGAACAACATTGCCAAAAGGCTTCTA
CGCAGAAGGGAGCAGAGGCGGCAGTCAAGCCTCTTCTCGTTCCTCATCACGTAGTCGCAACAGTTCAAGAAATTCAACTCCAGGCAGCA
GTAGGGGAACTTCTCCTGCTAGAATGGCTGGCAATGGCGGTGATGCTGCTCTTGCTTTGCTGCTGCTTGACAGATTGAACCAGCTTGAG
AGCAAAATGTCTGGTAAAGGCCAACAACAACAAGGCCAAACTGTCACTAAGAAATCTGCTGCTGAGGCTTCTAAGAAGCCTCGGCAAA
AACGTACTGCCACTAAAGCATACAATGTAACACAAGCTTTCGGCAGACGTGGTCCAGAACAAACCCAAGGAAATTTTGGGGACCAGGA
ACTAATCAGACAAGGAACTGATTACAAACATTGGCCGCAAATTGCACAATTTGCCCCCAGCGCTTCAGCGTTCTTCGGAATGTCGCGCAT
TGGCATGGAAGTCACACCTTCGGGAACGTGGTTGACCTACACAGGTGCCATCAAATTGGATGACAAAGATCCAAATTTCAAAGATCAAG
TCATTTTGCTGAATAAGCATATTGACGCATACAAAACATTCCCACCAACAGAGCCTAAAAAGGACAAAAAGAAGAAGGCTGATGAAACT
CAAGCCTTACCGCAGAGACAGAAGAAACAGCAAACTGTGACTCTTCTTCCTGCTGCAGATTTGGATGATTTCTCCAAACAATTGCAACAA
TCCATGAGCAGTGCTGACTCAACTCAGGCCTAAACTCATGCAGACCACACAAGGCAGATGGGCTATATAAACGTTTTCGCTTTTCCGTTT
ACGATATATAGTCTACTCTTGTGCAGAATGAATTCTCGTAACTACATAGCACAAGTAGATGTAGTTAACTTTAATCTCACATAGCAATCTT
TAATCAGTGTGTAACATTAGGGAGGACTTGAAAGAGCCACCACATTTTCACCGAGGCCACGCGGAGTACGATCGAGTGTACAGTGAAC
AATGCTAGGGAGAGCTGCCTATATGGAAGAGCCCTAATGTGTAAAATTAATTTTAGTAGTGCTATCCCCATGTGATTTTAATAGCTTCTT
AGGAGAATGACAAAA
SEQ ID NO: 13
   >Severe acute respiratory syndrome coronavirus 2 orflab polyprotein of isolate hCoV-19/France/IDF0372-isl/2020
SEQ ID NO: 14
   >Protein\S_Human\2019-nCoV(Sprotein_hCoV19FranceIDF0372isl2020) 
MFVFLVLLPLVSSQCVNLTTRTQLPPAYTNSFTRGVYYPDKVFRSSVLHSTQDLFLPFFSNVTWFHAIHVSGTNGTKRFDNPVLPFNDGVYFAS
TEKSNIIRGWIFGTTLDSKTQSLLIVNNATNVVIKVCEFQFCNDPFLGVYYHKNNKSWMESEFRVYSSANNCTFEYVSQPFLMDLEGKQGNFK
NLREFVFKNIDGYFKIYSKHTPINLVRDLPQGFSALEPLVDLPIGINITRFQTLLALHRSYLTPGDSSSGWTAGAAAYYVGYLQPRTFLLKYNENGT
ITDAVDCALDPLSETKCTLKSFTVEKGIYQTSNFRVQPTESIVRFPNITNLCPFGEVFNATRFASVYAWNRKRISNCVADYSFLYNSASFSTFKCYG
VSPTKLNDLCFTNVYADSFVIRGDEVRQIAPGQTGKIADYNYKLPDDFTGCVIAWNSNNLDSKVGGNYNYLYRLFRKSNLKPFERDISTEIYQAG
STPCNGVEGFNCYFPLQSYGFQPTNGVGYQPYRVVVLSFELLHAPATVCGPKKSTNLVKNKCVNFNFNGLTGTGVLTESNKKFLPFQQFGRDI
ADTTDAVRDPQTLEILDITPCSFGGVSVITPGTNTSNQVAVLYQDVNCTEVPVAIHADQLTPTWRVYSTGSNVFQTRAGCLIGAEHVNNSYEC
DIPIGAGICASYQTQTNSPRRARSVASQSIIAYTMSLGAENSVAYSNNSIAIPTNFTISVTTEILPVSMTKTSVDCTMYICGDSTECSNLLLQYGSF
CTQLNRALTGIAVEQDKNTQEVFAQVKQIYKTPPIKDFGGFNFSQILPDPSKPSKRSFIEDLLFNKVTLADAGFIKQYGDCLGDIAARDLICAQKF
NGLTVLPPLLTDEMIAQYTSALLAGTITSGWTFGAGAALQIPFAMQMAYRFNGIGVTQNVLYENQKLIANQFNSAIGKIQDSLSSTASALGKLQ
DVVNQNAQALNTLVKQLSSNFGAISSVLNDILSRLDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTHWFVTQRNFYEPQIITTDNTFVSGNCDVVIGIVNN
TVYDPLQPELDSFKEELDKYFKNHTSPDVDLGDISGINASVVNIQKEIDRLNEVAKNLNESLIDLQELGKYEQYIKWPWYIWLGFIAGLIAIVMVT
IMLCCMTSCCSCLKGCCSCGSCCKFDEDDSEPVLKGVKLHYT
SEQ ID NO: 15
   >hCoV-19/Austria/CeMM0360/20201|EPI_ISL_438123|2020-04-05 
NNNNNNNNNNNNNNNNNNNNNNNNNNNNNNACCAACCAACTTTCGATCTCTTGTAGATCTGTTCTCTAAACGAACTTTAAAATCTG
TGTGGCTGTCACTCGGCTGCATGCTTAGTGCACTCACGCAGTATAATTAATAACTAATTACTGTCGTTGACAGGACACGAGTAACTCGTC
TATCTTCTGCAGGCTGCTTACGGTTTCGTCCGTGTTGCAGCCGATCATCAGCACATCTAGGTTTTGTCCGGGTGTGACCGAAAGGTAAGA
TGGAGAGCCTTGTCCCTGGTTTCAACGAGAAAACACACGTCCAACTCAGTTTGCCTGTTTTACAGGTTCGCGACGTGCTCGTACGTGGCT
 
TTGGAGACTCCGTGGAGGAGGTCTTATCAGAGGCACGTCAACATCTTAAAGATGGCACTTGTGGCTTAGTAGAAGTTGAAAAAGGCGT
TTTGCCTCAACTTGAACAGCCCTATGTGTTCATCAAACGTTCGGATGCTCGAACTGCACCTCATGGTCATGTTATGGTTGAGCTGGTAGC
AGAACTCGAAGGCATTCAGTACGGTCGTAGTGGTGAGACACTTGGTGTCCTTGTCCCTCATGTGGGCGAAATACCAGTGGCTTACCGCA
AGGTTCTTCTTCGTAAGAACGGTAATAAAGGAGCTGGTGGCCATAGTTACGGCGCCGATCTAAAGTCATTTGACTTAGGCGACGAGCTT
GGCACTGATCCTTATGAAGATTTTCAAGAAAACTGGAACACTAAACATAGCAGTGGTGTTACCCGTGAACTCATGCGTGAGCTTAACGG
AGGGGCATACACTCGCTATGTCGATAACAACTTCTGTGGCCCTGATGGCTACCCTCTTGAGTGCATTAAAGACCTTCTAGCACGTGCTGG
TAAAGCTTCATGCACTTTGTCCGAACAACTGGACTTTATTGACACTAAGAGGGGTGTATACTGCTGCCGTGAACATGAGCATGAAATTGC
TTGGTACACGGAACGTTCTGAAAAGAGCTATGAATTGCAGACACCTTTTGAAATTAAATTGGCAAAGAAATTTGACACCTTCAATGGGG
AATGTCCAAATTTTGTATTTCCCTTAAATTCCATAATCAAGACTATTCAACCAAGGGTTGAAAAGAAAAAGCTTGATGGCTTTATGGGTA
GAATTCGATCTGTCTATCCAGTTGCGTCACCAAATGAATGCAACCAAATGTGCCTTTCAACTCTCATGAAGTGTGATCATTGTGGTGAAA
CTTCATGGCAGACGGGCGATTTTGTTAAAGCCACTTGCGAATTTTGTGGCACTGAGAATTTGACTAAAGAAGGTGCCACTACTTGTGGTT
ACTTACCCCAAAATGCTGTTGTTAAAATTTATTGTCCAGCATGTCACAATTCAGAAGTAGGACCTGAGCATAGTCTTGCCGAATACCATAA
TGAATCTGGCTTGAAAACCATTCTTCGTAAGGGTGGTCGCACTATTGCCTTTGGAGGCTGTGTGTTCTCTTATGTTGGTTGCCATAACAA
GTGTGCCTATTGGGTTCCACGTGCTAGCGCTAACATAGGTTGTAACCATACAGGTGTTGTTGGAGAAGGTTCCGAAGGTCTTAATGACA
ACCTTCTTGAAATACTCCAAAAAGAGAAAGTCAACATCAATATTGTTGGTGACTTTAAACTTAATGAAGAGATCGCCATTATTTTGGCATC
TTTTTCTGCTTCCACAAGTGCTTTTGTGGAAACTGTGAAAGGTTTGGATTATAAAGCATTCAAACAAATTGTTGAATCCTGTGGTAATTTT
AAAGTTACAAAAGGAAAAGCTAAAAAAGGTGCCTGGAATATTGGTGAACAGAAATCAATACTGAGTCCTCTTTATGCATTTGCATCAGA
GGCTGCTCGTGTTGTACGATCAATTTTCTCCCGCACTCTTGAAACTGCTCAAAATTCTGTGCGTGTTTTACAGAAGGCCGCTATAACAATA
CTAGATGGAATTTCACAGTATTCACTGAGACTCATTGATGCTATGATGTTCACATCTGATTTGGCTACTAACAATCTAGTTGTAATGGCCT
ACATTACAGGTGGTGTTGTTCAGTTGACTTCGCAGTGGCTAACTAACATCTTTGGCACTGTTTATGAAAAACTCAAACCCGTCCTTGATTG
GCTTGAAGAGAAGTTTAAGGAAGGTGTAGAGTTTCTTAGAGACGGTTGGGAAATTGTTAAATTTATCTCAACCTGTGCTTGTGAAA
TTGTCGGTGGACAAATTGTCACCTGTGCAAAGGAAATTAAGGAGAGTGTTCAGACATTCTTTAAGCTTGTAAATAAATTTTTGGCTTTGT
GTGCTGACTCTATCATTATTGGTGGAGCTAAACTTAAAGCCTTGAATTTAGGTGAAACATTTGTCACGCACTCAAAGGGATTGTACAGAA
AGTGTGTTAAATCCAGAGAAGAAACTGGCCTACTCATGCCTCTAAAAGCCCCAAAAGAAATTATCTTCTTAGAGGGAGAAACACTTCCCA
CAGAAGTGTTAACAGAGGAAGTTGTCTTGAAAACTGGTGATTTACAACCATTAGAACAACCTACTAGTGAAGCTGTTGAAGCTCCATTG
GTTGGTACACCAGTTTGTATTAACGGGCTTATGTTGCTCGAAATCAAAGACACAGAAAAGTACTGTGCCCTTGCACCTAATATGATGGTA
ACAAACAATACCTTCACACTCAAAGGCGGTGCACCAACAAAGGTTACTTTTGGTGATGACACTGTGATAGAAGTGCAAGGTTACAAGAG
TGTGAATATCACTTTTGAACTTGATGAAAGGATTGATAAAGTACTTAATGAGAAGTGCTCTGCCTATACAGTTGAACTCGGTACAGAAGT
AAATGAGTTCGCCTGTGTTGTGGCAGATGCTGTCATAAAAACTTTGCAACCAGTATCTGAATTACTTACACCACTGGGCATTGATTTAGA
TGAGTGGAGTATGGCTACATACTACTTATTTGATGAGTCTGGTGAGTTTAAATTGGCTTCACATATGTATTGTTCTTTTTACCCTCCAGAT
GAGGATGAAGAAGAAGGTGATTGTGAAGAAGAAGAGTTTGAGCCATCAACTCAATATGAGTATGGTACTGAAGATGATTACCAAGGTA
AACCTTTGGAATTTGGTGCCACTTCTGCTGCTCTTCAACCTGAAGAAGAGCAAGAAGAAGATTGGTTAGATGATGATAGTCAACAAACT
GTTGGTCAACAAGACGGCAGTGAGGACAATCAGACAACTACTATTCAAACAATTGTTGAGGTTCAACCTCAATTAGAGATGGAACTTAC
ACCAGTTGTTCAGACTATTGAAGTGAATAGTTTTAGTGGTTATTTAAAACTTACTGACAATGTATACATTAAAAATGCAGACATTGTGGA
AGAAGCTAAAAAGGTAAAACCAACAGTGGTTGTTAATGCAGCCAATGTTTACCTTAAACATGGAGGAGGTGTTGCAGGAGCCTTAAATA
AGGCTACTAACAATGCCATGCAAGTTGAATCTGATGATTACATAGCTACTAATGGACCACTTAAAGTGGGTGGTAGTTGTGTTTTAAGCG
GACACAATCTTGCTAAACACTGTCTTCATGTTGTCGGCCCAAATGTTAACAAAGGTGAAGACATTCAACTTCTTAAGAGTGCTTATGAAA
ATTTTAATCAGCACGAAGTTCTACTTGCACCATTATTATCAGCTGGTATTTTTGGTGCTGACCCTATACATTCTTTAAGAGTTTGTGTAGAT
ACTGTTCGCACAAATGTCTACTTAGCTGTCTTTGATAAAAATCTCTATGACAAACTTGTTTCAAGCTTTTTGGAAATGAAGAGTGAAAAGC
AAGTTGAACAAAAGATCGCTGAGATTCCTAAAGAGGAAGTTAAGCCATTTATAACTGAAAGTAAACCTTCAGTTGAACAGAGAAAACAA
GATGATAAGAAAATCAAAGCTTGTGTTGAAGAAGTTACAACAACTCTGGAAGAAACTAAGTTCCTCACAGAAAACTTGTTACTTTATATT
GACATTAATGGCAATCTTCATCCAGATTCTGCCACTCTTGTTAGTGACATTGACATCACTTTCTTAAAGAAAGATGCTCCATATATAGTGG
GTGATGTTGTTCAAGAGGGTGTTTTAACTGCTGTGGTTATACCTACTAAAAAGGCTGGTGGCACTACTGAAATGCTAGCGAAAGCTTTG
AGAAAAGTGCCAACAGACAATTATATAACCACTTACCCGGGTCAGGGTTTAAATGGTTACACTGTAGAGGAGGCAAAGACAGTGCTTAA
AAAGTGTAAAAGTGCCTTTTACATTCTACCATCTATTATCTCTAATGAGAAGCAAGAAATTCTTGGAACTGTTTCTTGGAATTTGCGAGAA
ATGCTTGCACATGCAGAAGAAACACGCAAATTAATGCCTGTCTGTGTGGAAACTAAAGCCATAGTTTCAACTATACAGCGTAAATATAA
GGGTATTAAAATACAAGAGGGTGTGGTTGATTATGGTGCTAGATTTTACTTTTACACCAGTAAAACAACTGTAGCGTCACTTATCAACAC
ACTTAACGATCTAAATGAAACTCTTGTTACAATGCCACTTGGCTATGTAACACATGGCTTAAATTTGGAAGAAGCTGCTCGGTATATGAG
ATCTCTCAAAGTGCCAGCTACAGTTTCTGTTTCTTCACCTGATGCTGTTACAGCGTATAATGGTTATCTTACTTCTTCTTCTAAAACACCTG
AAGAACATTTTATTGAAACCATCTCACTTGCTGGTTCCTATAAAGATTGGTCCTATTCTGGACAATCTACACAACTAGGTATAGAATTTCT
TAAGAGAGGTGATAAAAGTGTATATTACACTAGTAATCCTACCACATTCCACCTAGATGGTGAAGTTATCACCTTTGACAATCTTAAGAC
ACTTCTTTCTTTGAGAGAAGTGAGGACTATTAAGGTGTTTACAACAGTAGACAACATTAACCTCCACACGCAAGTTGTGGACATGTCAAT
GACATATGGACAACAGTTTGGTCCAACTTATTTGGATGGAGCTGATGTTACTAAAATAAAACCTCATAATTCACATGAAGGTAAAACATT
TTATGTTTTACCTAATGATGACACTCTACGTGTTGAGGCTTTTGAGTACTACCACACAACTGATCCTAGTTTTCTGGGTAGGTACATGTCA
GCATTAAATCACACTAAAAAGTGGAAATACCCACAAGTTAATGGTTTAACTTCTATTAAATGGGCAGATAACAACTGTTATCTTGCCACT
GCATTGTTAACACTCCAACAAATAGAGTTGAAGTTTAATCCACCTGCTCTACAAGATGCTTATTACAGAGCAAGGGCTGGTGAAGCTGCT
AACTTTTGTGCACTTATCTTAGCCTACTGTAATAAGACAGTAGGTGAGTTAGGTGATGTTAGAGAAACAATGAGTTACTTGTTTCAACAT
GCCAATTTAGATTCTTGCAAAAGAGTCTTGAACGTGGTGTGTAAAACTTGTGGACAACAGCAGACAACCCTTAAGGGTGTAGAAGCTGT
TATGTACATGGGCACACTTTCTTATGAACAATTTAAGAAAGGTGTTCAGATACCTTGTACGTGTGGTAAACAAGCTACAAAATATCTAGT
ACAACAGGAGTCACCTTTTGTTATGATGTCAGCACCACCTGCTCAGTATGAACTTAAGCATGGTACATTTACTTGTGCTAGTGAGTACACT
GGTAATTACCAGTGTGGTCACTATAAACATATAACTTCTAAAGAAACTTTGTATTGCATAGACGGTGCTTTACTTACAAAGTCCTCAGAAT
ACAAAGGTCCTATTACGGATGTTTTCTACAAAGAAAACAGTTACACAACAACCATAAAACCAGTTACTTATAAATTGGATGGTGTTGTTT
 
GTACAGAAATTGACCCTAAGTTGGACAATTATTATAAGAAAGACAATTCTTATTTCACAGAGCAACCAATTGATCTTGTACCAAACCAAC
CATATCCAAACGCAAGCTTCGATAATTTTAAGTTTGTATGTGATAATATCAAATTTGCTGATGATTTAAACCAGTTAACTGGTTATAAGAA
ACCTGCTTCAAGAGAGCTTAAAGTTACATTTTTCCCTGACTTAAATGGTGATGTGGTGGCTATTGATTATAAACACTACACACCCTCTTTT
AAGAAAGGAGCTAAATTGTTACATAAACCTATTGTTTGGCATGTTAACAATGCAACTAATAAAGCCACGTATAAACCAAATACCTGGTGT
ATACGTTGTCTTTGGAGCACAAAACCAGTTGAAACATCAAATTCGTTTGATGTACTGAAGTCAGAGGACGCGCAGGGAATGGATAATCT
TGCCTGCGAAGATCTAAAACCAGTCTCTGAAGAAGTAGTGGAAAATCCTACCATACAGAAAGACGTTCTTGAGTGTAATGTGAAAACTA
CCGAAGTTGTAGGAGACATTATACTTAAACCAGCAAATAATAGTTTAAAAATTACAGAAGAGGTTGGCCACACAGATCTAATGGCTGCT
TATGTAGACAATTCTAGTCTTACTATTAAGAAACCTAATGAATTATCTAGAGTATTAGGTTTGAAAACCCTTGCTACTCATGGTTTAGCTG
CTGTTAATAGTGTCCCTTGGGATACTATAGCTAATTATGCTAAGCCTTTTCTTAACAAAGTTGTTAGTACAACTACTAACATAGTTACACG
GTGTTTAAACCGTGTTTGTACTAATTATATGCCTTATTTCTTTACTTTATTGCTACAATTGTGTACTTTTACTAGAAGTACAAATTCTAGAAT
TAAAGCATCTATGCCGACTACTATAGCAAAGAATACTGTTAAGAGTGTCGGTAAATTTTGTCTAGAGGCTTCATTTAATTATTTGAAGTCA
CCTAATTTTTCTAAACTGATAAATATTATAATTTGGTTTTTACTATTAAGTGTTTGCCTAGGTTCTTTAATCTACTCAACCGCTGCTTTAGGT
GTTTTAATGTCTAATTTAGGCATGCCTTCTTACTGTACTGGTTACAGAGAAGGCTATTTGAACTCTACTAATGTCACTATTGCAACCTACTG
TACTGGTTCTATACCTTGTAGTGTTTGTCTTAGTGGTTTAGATTCTTTAGACACCTATCCTTCTTTAGAAACTATACAAATTACCATTTCATC
TTTTAAATGGGATTTAACTGCTTTTGGCTTAGTTGCAGAGTGGTTTTTGGCATATATTCTTTTCACTAGGTTTTTCTATGTACTTGGATTGG
CTGCAATCATGCAATTGTTTTTCAGCTATTTTGCAGTACATTTTATTAGTAATTCTTGGCTTATGTGGTTAATAATTAATCTTGTACAAATG
GCCCCGATTTCAGCTATGGTTAGAATGTACATCTTCTTTGCATCATTTTATTATGTATGGAAAAGTTATGTGCATGTTGTAGACGGTTGTA
ATTCATCAACTTGTATGATGTGTTACAAACGTAATAGAGCAACAAGAGTCGAATGTACAACTATTGTTAATGGTGTTAGAAGGTCCTTTT
ATGTCTATGCTAATGGAGGTAAAGGCTTTTGCAAACTACACAATTGGAATTGTGTTAATTGTGATACATTCTGTGCTGGTAGTACATTTAT
TAGTGATGAAGTTGCGAGAGACTTGTCACTACAGTTTAAAAGACCAATAAATCCTACTGACCAGTCTTCTTACATCGTTGATAGTGTTAC
AGTGAAGAATGGTTCCATCCATCTTTACTTTGATAAAGCTGGTCAAAAGACTTATGAAAGACATTCTCTCTCTCATTTTGTTAACTTAGAC
AACCTGAGAGCTAATAACACTAAAGGTTCATTGCCTATTAATGTTATAGTTTTTGATGGTAAATCAAAATGTGAAGAATCATCTGCAAAA
TCAGCGTCTGTTTACTACAGTCAGCTTATGTGTCAACCTATACTGTTACTAGATCAGGCATTAGTGTCTGATGTTGGTGATAGTGCGGAA
GTTGCAGTTAAAATGTTTGATGCTTACGTTAATACGTTTTCATCAACTTTTAACGTACCAATGGAAAAACTCAAAACACTAGTTGCAACTG
CAGAAGCTGAACTTGCAAAGAATGTGTCCTTAGACAATGTCTTATCTACTTTTATTTCAGCAGCTCGGCAAGGGTTTGTTGATTCAGATGT
AGAAACTAAAGATGTTGTTGAATGTCTTAAATTGTCACATCAATCTGACATAGAAGTTACTGGCGATAGTTGTAATAACTATATGCTCAC
CTATAACAAAGTTGAAAACATGACACCCCGTGACCTTGGTGCTTGTATTGACTGTAGTGCGCGTCATATTAATGCGCAGGTAGCAAAAA
GTCACAACATTGCTTTGATATGGAACGTTAAAGATTTCATGTCATTGTCTGAACAACTACGAAAACAAATACGTAGTGCTGCTAAAAAGA
ATAACTTACCTTTTAAGTTGACATGTGCAACTACTAGACAAGTTGTTAATGTTGTAACAACAAAGATAGCACTTAAGGGTGGTAAAATTG
TTAATAATTGGTTGAAGCAGTTAATTAAAGTTACACTTGTGTTCCTTTTTGTTGCTGCTATTTTCTATTTAATAACACCTGTTCATGTCATGT
CTAAACATACTGACTTTTCAAGTGAAATCATAGGATACAAGGCTATTGATGGTGGTGTCACTCGTGACATAGCATCTACAGATACTTGTT
TTGCTAACAAACATGCTGATTTTGACACATGGTTTAGCCAGCGTGGTGGTAGTTATACTAATGACAAAGCTTGCCCATTGATTGCTGCAG
TCATAACAAGAGAAGTGGGTTTTGTCGTGCCTGGTTTGCCTGGCACGATATTACGCACAACTAATGGTGACTTTTTGCATTTCTTACCTAG
AGTTTTTAGTGCAGTTGGTAACATCTGTTACACACCATCAAAACTTATAGAGTACACTGACTTTGCAACATCAGCTTGTGTTTTGGCTGCT
GAATGTACAATTTTTAAAGATGCTTCTGGTAAGCCAGTACCATATTGTTATGATACCAATGTACTAGAAGGTTCTGTTGCTTATGAAAGTT
TACGCCCTGACACACGTTATGTGCTCATGGATGGCTCTATTATTCAATTTCCTAACACCTACCTTGAAGGTTCTGTTAGAGTGGTAACAAC
TTTTGATTCTGAGTACTGTAGGCACGGCACTTGTGAAAGATCAGAAGCTGGTGTTTGTGTATCTACTAGTGGTAGATGGGTACTTAACAA
TGATTATTACAGATCTTTACCAGGAGTTTTCTGTGGTGTAGATGCTGTAAATTTACTTACTAATATGTTTACACCACTAATTCAACCTATTG
GTGCTTTGGACATATCAGCATCTATAGTAGCTGGTGGTATTGTAGCTATCGTAGTAACATGCCTTGCCTACTATTTTATGAGGTTTAGAAG
AGCTTTTGGTGAATACAGTCATGTAGTTGCCTTTAATACTTTACTATTCCTTATGTCATTCACTGTACTCTGTTTAACACCAGTTTACTCATT
CTTACCTGGTGTTTATTCTGTTATTTACTTGTACTTGACATTTTATCTTACTAATGATGTTTCTTTTTTAGCACATATTCAGTGGATGGTTAT
GTTCACACCTTTAGTACCTTTCTGGATAACAATTGCTTATATCATTTGTATTTCCACAAAGCATTTCTATTGGTTCTTTAGTAATTACCTAAA
GAGACGTGTAGTCTTTAATGGTGTTTCCTTTAGTACTTTTGAAGAAGCTGCGCTGTGCACCTTTTTGTTAAATAAAGAAATGTATCTAAAG
TTGCGTAGTGATGTGCTATTACCTCTTACGCAATATAATAGATACTTAGCTCTTTATAATAAGTACAAGTATTTTAGTGGAGCAATGGATA
CAACTAGCTACAGAGAAGCTGCTTGTTGTCATCTCGCAAAGGCTCTCAATGACTTCAGTAACTCAGGTTCTGATGTTCTTTACCAACCACC
ACAAACCTCTATCACCTCAGCTGTTTTGCAGAGTGGTTTTAGAAAAATGGCATTCCCATCTGGTAAAGTTGAGGGTTGTATGGTACAAGT
AACTTGTGGTACAACTACACTTAACGGTCTTTGGCTTGATGACGTAGTTTACTGTCCAAGACATGTGATCTGCACCTCTGAAGACATGCTT
AACCCTAATTATGAAGATTTACTCATTCGTAAGTCTAATCATAATTTCTTGGTACAGGCTGGTAATGTTCAACTCAGGGTTATTGGACATT
CTATGCAAAATTGTGTACTTAAGCTTAAGGTTGATACAGCCAATCCTAAGACACCTAAGTATAAGTTTGTTCGCATTCAACCAGGACAGA
CTTTTTCAGTGTTAGCTTGTTACAATGGTTCACCATCTGGTGTTTACCAATGTGCTATGAGGCCCAATTTCACTATTAAGGGTTCATTCCTT
AATGGTTCATGTGGTAGTGTTGGTTTTAACATAGATTATGACTGTGTCTCTTTTTGTTACATGCACCATATGGAATTACCAACTGGAGTTC
ATGCTGGCACAGACTTAGAAGGTAACTTTTATGGACCTTTTGTTGACAGGCAAACAGCACAAGCAGCTGGTACGGACACAACTATTACA
GTTAATGTTTTAGCTTGGTTGTACGCTGCTGTTATAAATGGAGACAGGTGGTTTCTCAATCGATTTACCACAACTCTTAATGACTTTAACC
TTGTGGCTATGAAGTACAATTATGAACCTCTAACACAAGACCATGTTGACATACTAGGACCTCTTTCTGCTCAAACTGGAATTGCCGTTTT
AGATATGTGTGCTTCATTAAAAGAATTACTGCAAAATGGTATGAATGGACGTACCATATTGGGTAGTGCTTTATTAGAAGATGAATTTAC
ACCTTTTGATGTTGTTAGACAATGCTCAGGTGTTACTTTCCAAAGTGCAGTGAAAAGAACAATCAAGGGTACACACCACTGGTTGTTACT
CACAATTTTGACTTCACTTTTAGTTTTAGTCCAGAGTACTCAATGGTCTTTGTTCTTTTTTTTGTATGAAAATGCCTTTTTACCTTTTGCTATG
GGTATTATTGCTATGTCTGCTTTTGCAATGATGTTTGTCAAACATAAGCATGCATTTCTCTGTTTGTTTTTGTTACCTTCTCTTGCCACTGTA
GCTTATTTTAATATGGTCTATATGCCTGCTAGTTGGGTGATGCGTATTATGACATGGTTGGATATGGTTGATACTAGTTTGTCTGGTTTTA
AGCTAAAAGACTGTGTTATGTATGCATCAGCTGTAGTGTTACTAATCCTTATGACAGCAAGAACTGTGTATGATGATGGTGCTAGGAGA
GTGTGGACACTTATGAATGTCTTGACACTCGTTTATAAAGTTTATTATGGTAATGCTTTAGATCAAGCCATTTCCATGTGGGCTCTTATAA
TCTCTGTTACTTCTAACTACTCAGGTGTAGTTACAACTGTCATGTTTTTGGCCAGAGGTATTGTTTTTATGTGTGTTGAGTATTGCCCTATT
 
TTCTTCATAACTGGTAATACACTTCAGTGTATAATGCTAGTTTATTGTTTCTTAGGCTATTTTTGTACTTGTTACTTTGGCCTCTTTTGTTTAC
TCAACCGCTACTTTAGACTGACTCTTGGTGTTTATGATTACTTAGTTTCTACACAGGAGTTTAGATATATGAATTCACAGGGACTACTCCC
ACCCAAGAATAGCATAGATGCCTTCAAACTCAACATTAAATTGTTGGGTGTTGGTGGCAAACCTTGTATCAAAGTAGCCACTGTACAGTC
TAAAATGTCAGATGTAAAGTGCACATCAGTAGTCTTACTCTCAGTTTTGCAACAACTCAGAGTAGAATCATCATCTAAATTGTGGGCTCA
ATGTGTCCAGTTACACAATGACATTCTCTTAGCTAAAGATACTACTGAAGCCTTTGAAAAAATGGTTTCACTACTTTCTGTTTTGCTTTCCA
TGCAGGGTGCTGTAGACATAAACAAGCTTTGTGAAGAAATGCTGGACAACAGGGCAACCTTACAAGCTATAGCCTCAGAGTTTAGTTCC
CTTCCATCATATGCAGCTTTTGCTACTGCTCAAGAAGCTTATGAGCAGGCTGTTGCTAATGGTGATTCTGAAGTTGTTCTTAAAAAGTTGA
AGAAGTCTTTGAATGTGGCTAAATCTGAATTTGACCGTGATGCAGCCATGCAACGTAAGTTGGAAAAGATGGCTGATCAAGCTATGACC
CAAATGTATAAACAGGCTAGATCTGAGGACAAGAGGGCAAAAGTTACTAGTGCTATGCAGACAATGCTTTTCACTATGCTTAGAAAGTT
GGATAATGATGCACTCAACAACATTATCAACAATGCAAGAGATGGTTGTGTTCCCTTGAACATAATACCTCTTACAACAGCAGCCAAACT
AATGGTTGTCATACCAGACTATAACACATATAAAAATACGTGTGATGGTACAACATTTACTTATGCATCAGCATTGTGGGAAATCCAACA
GGTTGTAGATGCAGATAGTAAAATTGTTCAACTTAGTGAAATTAGTATGGACAATTCACCTAATTTAGCATGGCCTCTTATTGTAACAGC
TTTAAGGGCCAATTCTGCTGTCAAATTACAGAATAATGAGCTTAGTCCTGTTGCACTACGACAGATGTCTTGTGCTGCCGGTACTACACA
AACTGCTTGCACTGATGACAATGCGTTAGCTTACTACAACACAACAAAGGGAGGTAGGTTTGTACTTGCACTGTTATCCGATTTACAGGA
TTTGAAATGGGCTAGATTCCCTAAGAGTGATGGAACTGGTACTATCTATACAGAACTGGAACCACCTTGTAGGTTTGTTACAGACACACC
TAAAGGTCCTAAAGTGAAGTATTTATACTTTATTAAAGGATTAAACAACCTAAATAGAGGTATGGTACTTGGTAGTTTAGCTGCCACAGT
ACGTCTACAAGCTGGTAATGCAACAGAAGTGCCTGCCAATTCAACTGTATTATCTTTCTGTGCTTTTGCTGTAGATGCTGCTAAAGCTTAC
AAAGATTATCTAGCTAGTGGGGGACAACCAATCACTAATTGTGTTAAGATGTTGTGTACACACACTGGTACTGGTCAGGCAATAACAGT
TACACCGGAAGCCAATATGGATCAAGAATCCTTTGGTGGTGCATCGTGTTGTCTGTACTGCCGTTGCCACATAGATCATCCAAATCCTAA
AGGATTTTGTGACTTAAAAGGTAAGTATGTACAAATACCTACAACTTGTGCTAATGACCCTGTGGGTTTTACACTTAAAAACACAGTCTG
TACCGTCTGCGGTATGTGGAAAGGTTATGGCTGTAGTTGTGATCAACTCCGCGAACCCATGCTTCAGTCAGCTGATGCACAATCGTTTTT
AAACGGGTTTGCGGTGTAAGTGCAGCCCGTCTTACACCGTGCGGCACAGGCACTAGTACTGATGTCGTATACAGGGCTTTTGACATCTA
CAATGATAAAGTAGCTGGTTTTGCTAAATTCCTAAAAACTAATTGTTGTCGCTTCCAAGAAAAGGACGAAGATGACAATTTAATTGATTC
TTACTTTGTAGTTAAGAGACACACTTTCTCTAACTACCAACATGAAGAAACAATTTATAATTTACTTAAGGATTGTCCAGCTGTTGCTAAA
CATGACTTCTTTAAGTTTAGAATAGACGGTGACATGGTACCACATATATCACGTCAACGTCTTACTAAATACACAATGGCAGACCTCGTCT
ATGCTTTAAGGCATTTTGATGAAGGTAATTGTGACACATTAAAAGAAATACTTGTCACATACAATTGTTGTGATGATGATTATTTCAATAA
AAAGGACTGGTATGATTTTGTAGAAAACCCAGATATATTACGCGTATACGCCAACTTAGGTGAACGTGTACGCCAAGCTTTGTTAAAAAC
AGTACAATTCTGTGATGCCATGCGAAATGCTGGTATTGTTGGTGTACTGACATTAGATAATCAAGATCTCAATGGTAACTGGTATGATTT
CGGTGATTTCATACAAACCACGCCAGGTAGTGGAGTTCCTGTTGTAGATTCTTATTATTCATTGTTAATGCCTATATTAACCTTGACCAGG
GCTTTAACTGCAGAGTCACATGTTGACACTGACTTAACAAAGCCTTACATTAAGTGGGATTTGTTAAAATATGACTTCACGGAAGAGAGG
TTAAAACTCTTTGACCGTTATTTTAAATATTGGGATCAGACATACCACCCAAATTGTGTTAACTGTTTGGATGACAGATGCATTCTGCATT
GTGCAAACTTTAATGTTTTATTCTCTACAGTGTTCCCACTTACAAGTTTTGGACCACTAGTGAGAAAAATATTTGTTGATGGTGTTCCATTT
GTAGTTTCAACTGGATACCACTTCAGAGAGCTAGGTGTTGTACATAATCAGGATGTAAACTTACATAGCTCTAGACTTAGTTTTAAGGAA
TTACTTGTGTATGCTGCTGACCCTGCTATGCACGCTGCTTCTGGTAATCTATTACTAGATAAACGCACTACGTGCTTTTCAGTAGCTGCAC
TTACTAACAATGTTGCTTTTCAAACTGTCAAACCCGGTAATTTTAACAAAGACTTCTATGACTTTGCTGTGTCTAAGGGTTTCTTTAAGGA
AGGAAGTTCTGTTGAATTAAAACACTTCTTCTTTGCTCAGGATGGTAATGCTGCTATCAGCGATTATGACTACTATCGTTATAATCTACCA
ACAATGTGTGATATCAGACAACTACTATTTGTAGTTGAAGTTGTTGATAAGTACTTTGATTGTTACGATGGTGGCTGTATTAATGCTAACC
AAGTCATCGTCAACAACCTAGACAAATCAGCTGGTTTTCCATTTAATAAATGGGGTAAGGCTAGACTTTATTATGATTCAATGAGTTATG
AGGATCAAGATGCACTTTTCGCATATACAAAACGTAATGTCATCCCTACTATAACTCAAATGAATCTTAAGTATGCCATTAGTGCAAAGA
ATAGAGCTCGCACCGTAGCTGGTGTCTCTATCTGTAGTACTATGACCAATAGACAGTTTCATCAAAAATTATTGAAATCAATAGCCGCCA
CTAGAGGAGCTACTGTAGTAATTGGAACAAGCAAATTCTATGGTGGTTGGCACAACATGTTAAAAACTGTTTATAGTGATGTAGAAAAC
CCTCACCTTATGGGTTGGGATTATCCTAAATGTGATAGAGCCATGCCTAACATGCTTAGAATTATGGCCTCACTTGTTCTTGCTCGCAAAC
ATACAACGTGTTGTAGCTTGTCACACCGTTTCTATAGATTAGCTAATGAGTGTGCTCAAGTATTGAGTGAAATGGTCATGTGTGGCGGTT
CACTATATGTTAAACCAGGTGGAACCTCATCAGGAGATGCCACAACTGCTTATGCTAATAGTGTTTTTAACATTTGTCAAGCTGTCACGG
CCAATGTTAATGCACTTTTATCTACTGATGGTAACAAAATTGCCGATAAGTATGTCCGCAATTTACAACACAGACTTTATGAGTGTCTCTA
TAGAAATAGAGATGTTGACACAGACTTTGTGAATGAGTTTTACGCATATTTGCGTAAACATTTCTCAATGATGATACTCTCTGACGATGCT
GTTGTGTGTTTCAATAGCACTTATGCATCTCAAGGTCTAGTGGCTAGCATAAAGAACTTTAAGTCAGTTCTTTATTATCAAAACAATGTTT
TTATGTCTGAAGCAAAATGTTGGACTGAGACTGACCTTACTAAAGGACCTCATGAATTTTGCTCTCAACATACAATGCTAGTTAAACAGG
GTGATGATTATGTGTACCTTCCTTACCCAGATCCATCAAGAATCCTAGGGGCCGGCTGTTTTGTAGATGATATCGTAAAAACAGATGGTA
CACTTATGATTGAACGGTTCGTGTCTTTAGCTATAGATGCTTACCCACTTACTAAACATCCTAATCAGGAGTATGCTGATGTCTTTCATTTG
TACTTACAATACATAAGAAAGCTACATGATGAGTTAACAGGACACATGTTAGACATGTATTCTGTTATGCTTACTAATGATAACACTTCAA
GGTATTGGGAACCTGAGTTTTATGAGGCTATGTACACACCGCATACAGTCTTACAGGCTGTTGGGGCTTGTGTTCTTTGCAATTCACAGA
CTTCATTAAGATGTGGTGCTTGCATACGTAGACCATTCTTATGTTGTAAATGCTGTTACGACCATGTCATATCAACATCACATAAATTAGT
CTTGTCTGTTAATCCGTATGTTTGCAATGCTCCAGGTTGTGATGTCACAGATGTGACTCAACTTTACTTAGGAGGTATGAGCTATTATTGT
AAATCACATAAACCACCCATTAGTTTTCCATTGTGTGCTAATGGACAAGTTTTTGGTTTATATAAAAATACATGTGTTGGTAGCGATAATG
TTACTGACTTTAATGCAATTGCAACATGTGACTGGACAAATGCTGGTGATTACATTTTAGCTAACACCTGTACTGAAAGACTCAAGCTTTT
TGCAGCAGAAACGCTCAAAGCTACTGAGGAGACATTTAAACTGTCTTATGGTATTGCTACTGTACGTGAAGTGCTGTCTGACAGAGAAT
TACATCTTTCATGGGAAGTTGGTAAACCTAGACCACCACTTAACCGAAATTATGTCTTTACTGGTTATCGTGTAACTAAAAACAGTAAAGT
ACAAATAGGAGAGTACACCTTTGAAAAAGGTGACTATGGTGATGCTGTTGTTTACCGAGGTACAACAACTTACAAATTAAATGTTGGTG
ATTATTTTGTGCTGACATCACATACAGTAATGCCATTAAGTGCACCTACACTAGTGCCACAAGAGCACTATGTTAGAATTACTGGCTTATA
CCCAACACTCAATATCTCAGATGAGTTTTCTAGCAATGTTGCAAATTATCAAAAGGTTGGTATGCAAAAGTATTCTACACTCCAGGGACC
ACCTGGTACTGGTAAGAGTCATTTTGCTATTGGCCTAGCTCTCTACTACCCTTCTGCTCGCATAGTGTATACAGCTTGCTCTCATGCCGCT
 
GTTGATGCACTATGTGAGAAGGCATTAAAATATTTGCCTATAGATAAATGTAGTAGAATTATACCTGCACGTGCTCGTGTAGAGTGTTTT
GATAAATTCAAAGTGAATTCAACATTAGAACAGTATGTCTTTTGTACTGTAAATGCATTGCCTGAGACGACAGCAGATATAGTTGTCTTT
GATGAAATTTCAATGGCCACAAATTATGATTTGAGTGTTGTCAATGCCAGATTACGTGCTAAGCACTATGTGTACATTGGCGACCCTGCT
CAATTACCTGCACCACGCACATTGCTAACTAAGGGCACACTAGAACCAGAATATTTCAATTCAGTGTGTAGACTTATGAAAACTATAGGT
CCAGACATGTTCCTCGGAACTTGTCGGCGTTGTCCTGCTGAAATTGTTGACACTGTGAGTGCTTTGGTTTATGATAATAAGCTTAAAGCA
CATAAAGACAAATCAGCTCAATGCTTTAAAATGTTTTATAAGGGTGTTATCACGCATGATGTTTCATCTGCAATTAACAGGCCACAAATA
GGCGTGGTAAGAGAATTCCTTACACGTAACCCTGCTTGGAGAAAAGCTGTCTTTATTTCACCTTATAATTCACAGAATGCTGTAGCCTCA
AAGATTTTGGGACTACCAACTCAAACTGTTGATTCATCACAGGGCTCAGAATATGACTATGTCATATTCACTCAAACCACTGAAACAGCT
CACTCTTGTAATGTAAACAGATTTAATGTTGCTATTACCAGAGCAAAAGTAGGCATACTTTGCATAATGTCTGATAGAGACCTTTATGACA
AGTTGCAATTTACAAGTCTTGAAATTCCACGTAGGAATGTGGCAACTTTACAAGCTGAAAATGTAACAGGACTCTTTAAAGATTGTAGTA
AGGTAATCACTGGGTTACATCCTACACAGGCACCTACACACCTCAGTGTTGACACTAAATTCAAAACTGAAGGTTTATGTGTTGACATAC
CTGGCATACCTAAGGACATGACCTATAGAAGACTCATCTCTATGATGGGTTTTAAAATGAATTATCAAGTTAATGGTTACCCTAACATGTT
TATCACCCGCGAAGAAGCTATAAGACATGTACGTGCATGGATTGGCTTCGATGTCGAGGGGTGTCATGCTACTAGAGAAGCTGTTGGTA
CCAATTTACCTTTACAGCTAGGTTTTTCTACAGGTGTTAACCTAGTTGCTGTACCTACAGGTTATGTTGATACACCTAATAATACAGATTTT
TCCAGAGTTAGTGCTAAACCACCGCCTGGAGATCAATTTAAACACCTCATACCACTTATGTACAAAGGACTTCCTTGGAATGTAGTGCGT
ATAAAGATTGTACAAATGTTAAGTGACACACTTAAAAATCTCTCTGACAGAGTCGTATTTGTCTTATGGGCACATGGCTTTGAGTTGACA
TCTATGAAGTATTTTGTGAAAATAGGACCTGAGCGCACCTGTTGTCTATGTGATAGACGTGCCACATGCTTTTCCACTGCTTCAGACACTT
ATGCCTGTTGGCATCATTCTATTGGATTTGATTACGTCTATAATCCGTTTATGATTGATGTTCAACAATGGGGTTTTACAGGTAACCTACA
AAGCAACCATGATCTGTATTGTCAAGTCCATGGTAATGCACATGTAGCTAGTTGTGATGCAATCATGACTAGGTGTCTAGCTGTCCACGA
GTGCTTTGTTAAGCGTGTTGACTGGACTATTGAATATCCTATAATTGGTGATGAACTGAAGATTAATGCGGCTTGTAGAAAGGTTCAACA
CATGGTTGTTAAAGCTGCATTATTAGCAGACAAATTCCCAGTTCTTCACGACATTGGTAACCCTAAAGCTATTAAGTGTGTACCTCAAGCT
GATGTAGAATGGAAGTTCTATGATGCACAGCCTTGTAGTGACAAAGCTTATAAAATAGAAGAATTATTCTATTCTTATGCCACACATTCT
GACAAATTCACAGATGGTGTATGCCTATTTTGGAATTGCAATGTCGATAGATATCCTGCTAATTCCATTGTTTGTAGATTTGACACTAGAG
TGCTATCTAACCTTAACTTGCCTGGTTGTGATGGTGGCAGTTTGTATGTAAATAAACATGCATTCCACACACCAGCTTTTGATAAAAGTGC
TTTTGTTAATTTAAAACAATTACCATTTTTCTATTACTCTGACAGTCCATGTGAGTCTCATGGAAAACAAGTAGTGTCAGATATAGATTAT
GTACCACTAAAGTCTGCTACGTGTATAACACGTTGCAATTTAGGTGGTGCTGTCTGTAGACATCATGCTAATGAGTACAGATTGTATCTC
GATGCTTATAACATGATGATCTCAGCTGGCTTTAGCTTGTGGGTTTACAAACAATTTGATACTTATAACCTCTGGAACACTTTTACAAGAC
TTCAGAGTTTAGAAAATGTGGCTTTTAATGTTGTAAATAAGGGACACTTTGATGGACAACAGGGTGAAGTACCAGTTTCTATCATTAATA
ACACTGTTTACACAAAAGTTGATGGTGTTGATGTAGAATTGTTTGAAAATAAAACAACATTACCTGTTAATGTAGCATTTGAGCTTTGGG
CTAAGCGCAACATTAAACCAGTACCAGAGGTGAAAATACTCAATAATTTGGGTGTGGACATTGCTGCTAATACTGTGATCTGGGACTAC
AAAAGAGATGCTCCAGCACATATATCTACTATTGGTGTTTGTTCTATGACTGACATAGCCAAGAAACCAACTGAAACGATTTGTGCACCA
CTCACTGTCTTTTTTGATGGTAGAGTTGATGGTCAAGTAGACTTATTTAGAAATGCCCGTAATGGTGTTCTTATTACAGAAGGTAGTGTTA
AAGGTTTACAACCATCTGTAGGTCCCAAACAAGCTAGTCTTAATGGAGTCACATTAATTGGAGAAGCCGTAAAAACACAGTTCAATTATT
ATAAGAAAGTTGATGGTGTTGTCCAACAATTACCTGAAACTTACTTTACTCAGAGTAGAAATTTACAAGAATTTAAACCCAGGAGTCAAA
TGGAAATTGATTTCTTAGAATTAGCTATGGATGAATTCATTGAACGGTATAAATTAGAAGGCTATGCCTTCGAACATATCGTTTATGGAG
ATTTTAGTCATAGTCAGTTAGGTGGTTTACATCTACTGATTGGACTAGCTAAACGTTTTAAGGAATCACCTTTTGAATTAGAAGATTTTAT
TCCTATGGACAGTACAGTTAAAAACTATTTCATAACAGATGCGCAAACAGGTTCATCTAAGTGTGTGTGTTCTGTTATTGATTTATTACTT
GATGATTTTGTTGAAATAATAAAATCCCAAGATTTATCTGTAGTTTCTAAGGTTGTCAAAGTGACTATTGACTATACAGAAATTTCATTTA
TGCTTTGGTGTAAAGATGGCCATGTAGAAACATTTTACCCAAAATTACAATCTAGTCAAGCGTGGCAACCGGGTGTTGCTATGCCTAATC
TTTACAAAATGCAAAGAATGCTATTAGAAAAGTGTGACCTTCAAAATTATGGTGATAGTGCAACATTACCTAAAGGCATAATGATGAAT
GTCGCAAAATATACTCAACTGTGTCAATATTTAAACACATTAACATTAGCTGTACCCTATAATATGAGAGTTATACATTTTGGTGCTGGTT
CTGATAAAGGAGTTGCACCAGGTACAGCTGTTTTAAGACAGTGGTTGCCTACGGGTACGCTGCTTGTCGATTCAGATCTTAATGACTTTG
TCTCTGATGCAGATTCAACTTTGATTGGTGATTGTGCAACTGTACATACAGCTAATAAATGGGATCTCATTATTAGTGATATGTACGACCC
TAAGACTAAAAATGTTACAAAAGAAAATGACTCTAAAGAGGGTTTTTTCACTTACATTTGTGGGTTTATACAACAAAAGCTAGCTCTTGG
AGGTTCCGTGGCTATAAAGATAACAGAACATTCTTGGAATGCTGATCTTTATAAGCTCATGGGACACTTCGCATGGTGGACAGCCTTTGT
TACTAATGTGAATGCGTCATCATCTGAAGCATTTTTAATTGGATGTAATTATCTTGGCAAACCACGCGAACAAATAGATGGTTATGTCAT
GCATGCAAATTACATATTTTGGAGGAATACAAATCCAATTCAGTTGTCTTCCTATTCTTTATTTGACATGAGTAAATTTCCCCTTAAATTAA
GGGGTACTGCTGTTATGTCTTTAAAAGAAGGTCAAATCAATGATATGATTTTATCTCTTCTTAGTAAAGGTAGACTTATAATTAGAGAAA
ACAACAGAGTTGTTATTTCTAGTGATGTTCTTGTTAACAACTAAACGAACAATGTTTGTTTTTCTTGTTTTATTGCCACTAGTCTCTAGTCA
GTGTGTTAATCTTACAACCAGAACTCAATTACCCCCTGCATACACTAATTCTTTCACACGTGGTGTTTATTACCCTGACAAAGTTTTCAGAT
CCTCAGTTTTACATTCAACTCAGGACTTGTTCTTACCTTTCTTTTCCAATGTTACTTGGTTCCATGCTATACATGTCTCTGGGACCAATGGTA
CTAAGAGGTTTGATAACCCTGTCCTACCATTTAATGATGGTGTTTATTTTGCTTCCACTGAGAAGTCTAACATAATAAGAGGCTGGATTTT
TGGTACTACTTTAGATTCGAAGACCCAGTCCCTACTTATTGTTAATAACGCTACTAATGTTGTTATTAAAGTCTGTGAATTTCAATTTTGTA
ATGATCCATTTTTGGGTGTTTATTACCACAAAAACAACAAAAGTTGGATGGAAAGTGAGTTCAGAGTTTATTCTAGTGCGAATAATTGCA
CTTTTGAATATGTCTCTCAGCCTTTTCTTATGGACCTTGAAGGAAAACAGGGTAATTTCAAAAATCTTAGGGAATTTGTGTTTAAGAATAT
TGATGGTTATTTTAAAATATATTCTAAGCACACGCCTATTAATTTAGTGCGTGATCTCCCTCAGGGTTTTTCGGCTTTAGAACCATTGGTA
GATTTGCCAATAGGTATTAACATCACTAGGTTTCAAACTTTACTTGCTTTACATAGAAGTTATTTGACTCCTGGTGATTCTTCTTCAGGTTG
GACAGCTGGTGCTGCAGCTTATTATGTGGGTTATCTTCAACCTAGGACTTTTCTATTAAAATATAATGAAAATGGAACCATTACAGATGC
TGTAGACTGTGCACTTGACCCTCTCTCAGAAACAAAGTGTACGTTGAAATCCTTCACTGTAGAAAAAGGAATCTATCAAACTTCTAACTTT
AGAGKCCAACCAACAGAATCTATTGTTAGATTTCCTAATATTACAAACTTGTGCCCTTTTGGTGAAGTTTTTAACGCCACCAGATTTGCAT
CTGTTTATGCTTGGAACAGGAAGAGAATCAGCAACTGTGTTGCTGATTATTCTGTCCTATATAATTCCGCATCATTTTCCACTTTTAAGTGT
TATGGAGTGTCTCCTACTAAATTAAATGATCTCTGCTTTACTAATGTCTATGCAGATTCATTTGTAATTAGAGGTGATGAAGTCAGACAAA
 
TCGCTCCAGGGCAAACTGGAAAGATTGCTGATTATAATTATAAATTACCAGATGATTTTACAGGCTGCGTTATAGCTTGGAATTCTAACA
ATCTTGATTCTAAGGTTGGTGGTAATTATAATTACCTGTATAGATTGTTTAGGAAGTCTAATCTCAAACCTTTTGAGAGAGATATTTCAAC
TGAAATCTATCAGGCCGGTAGCACACCTTGTAATGGTGTTGAAGGTTTTAATTGTTACTTTCCTTTACAATCATATGGTTTCCAACCCACT
AATGGTGTTGGTTACCAACCATACAGAGTAGTAGTACTTTCTTTTGAACTTCTACATGCACCAGCAACTGTTTGTGGACCTAAAAAGTCTA
CTAATTTGGTTAAAAACAAATGTGTCAATTTCAACTTCAATGGTTTAACAGGCACAGGTGTTCTTACTGAGTCTAACAAAAAGTTTCTGCC
TTTCCAACAATTTGGCAGAGACATTGCTGACACTACTGATGCTGTCCGTGATCCACAGACACTTGAGATTCTTGACATTACACCATGTTCT
TTTGGTGGTGTCAGTGTTATAACACCAGGAACAAATACTTCTAACCAGGTTGCTGTTCTTTATCAGGGTGTTAACTGCACAGAAGTCCCT
GTTGCTATTCATGCAGATCAACTTACTCCTACTTGGCGTGTTTATTCTACAGGTTCTAATGTTTTTCAAACACGTGCAGGCTGTTTAATAGG
GGCTGAACATGTCAACAACTCATATGAGTGTGACATACCCATTGGTGCAGGTATATGCGCTAGTTATCAGACTCAGACTAATTCTCCTCG
GCGGGCACGTAGTGTAGCTAGTCAATCCATCATTGCCTACACTATGTCACTTGGTGCAGAAAATTCAGTTGCTTACTCTRATAACTCTATT
GCCATACCCACAAATTTTACTATTAGTGTTACCACAGAAATTCTACCAGTGTCTATGACCAAGACATCAGTAGATTGTACAATGTACATTT
GTGGTGATTCAACTGAATGCAGCAATCTTTTGTTGCAATATGGCAGTTTTTGTACACAATTAAACCGTGCTTTAACTGGAATAGCTGTTGA
ACAAGACAAAAACACCCAAGAAGTTTTTGCACAAGTCAAACAAATTTACAAAACACCACCAATTAAAGATTTTGGTGGTTTTAATTTTTCA
CAAATATTACCAGATCCATCAAAACCAAGCAAGAGGTCATTTATTGAAGATCTACTTTTCAACAAAGTGACACTTGCAGATGCTGGCTTC
ATCAAACAATATGGTGATTGCCTTGGTGATATTGCTGCTAGAGACCTCATTTGTGCACAAAAGTTTAACGGCCTTACTGTTTTGCCACCTT
TGCTCACAGATGAAATGATTGCTCAATACACTTCTGCACTGTTAGCGGGTACAATCACTTCTGGTTGGACCTTTGGTGCAGGTGCTGCAT
TACAAATACCATTTGCTATGCAAATGGCTTATAGGTTTAATGGTATTGGAGTTACACAGAATGTTCTCTATGAGAACCAAAAATTGATTG
CCAACCAATTTAATAGTGCTATTGGCAAAATTCAAGACTCACTTTCTTCCACAGCAAGTGCACTTGGAAAACTTCAAGATGTGGTCAACC
AAAATGCACAAGCTTTAAACACGCTTGTTAAACAACTTAGCTCCAATTTTGGTGCAATTTCAAGTGTTTTAAATGATATCCTTTCACGTCTT
GACAAAGTTGAGGCTGAAGTGCAAATTGATAGGTTGATCACAGGCAGACTTCAAAGTTTGCAGACATATGTGACTCAACAATTAATTAG
AGCTGCAGAAATCAGAGTTTCTGCTAATCTTGCTGCTACTAAAATGTCAGAGTGTGTACTTGGACAATCAAAAAGAGTTGATTTTTGTGG
AAAGGGCTATCATCTTATGTCCTTCCCTCAGTCAGCACCTCATGGTGTAGTCTTCTTGCATGTGACTTATGTCCCTGCACAAGAAAAGAAC
TTCACAACTGCTCCTGCCATTTGTCATGATGGAAAAGCACACTTTCCTCGTGAAGGTGTCTTTGTTTCAAATGGCACACACTGGTTTGTAA
CACAAAGGAATTTTTATGAACCACAAATCATTACTACAGACAACACATTTGTGTCTGGTAACTGTGATGTTGTAATAGGAATTGTCAACA
ACACAGTTTATGATCCTTTGCAACCTGAATTAGACTCATTCAAGGAGGAGTTAGATAAATATTTTAAGAATCATACATCACCAGATGTTG
ATTTAGGTGACATCTCTGGCATTAATGCTTCAGTTGTAAACATTCAAAAAGAAATTGACCGCCTCAATGAGGTTGCCAAGAATTTAAATG
AATCTCTCATCGATCTCCAAGAACTTGGAAAGTATGAGCAGTATATAAAATGGCCATGGTACATTTGGCTAGGTTTTATAGCTGGCTTGA
TTGCCATAGTAATGGTGACAATTATGCTTTGCTGTATGACCAGTTGCTGTAGTTGTCTCAAGGGCTGTTGTTCTTGTGGATCCTGCTGCAA
ATTTGATGAAGACGACTCTGAGCCAGTGCTCAAAGGAGTCAAATTACATTACACATAAACGAACTTATGGATTTGTTTATGAGAATCTTC
ACAATTGGAACTGTAACTTTGAAGCAAGGTGAAATCAAGGATGCTACTCCTTCAGATTTTGTTCGCGCTACTGCAACGATACCGATACAA
GCCTCACTCCCTTTCGGATGGCTTATTGTTGGCGTTGCACTTCTTGCTGTTTTTCAGAGCGCTTCCAAAATCATAACCCTCAAAAAGAGAT
GGCAACTAGCACTCTCCAAGGGTGTTCACTTTGTTTGCAACTTGCTGTTGTTGTTTGTAACAGTTTACTCACACCTTTTGCTCGTTGCTGCT
GGCCTTGAAGCCCCTTTTCTCTATCTTTATGCTTTAGTCTACTTCTTGCAGAGTATAAACTTTGTAAGAATAATAATGAGGCTTTGGCTTTG
CTGGAAATGCCGTTCCAAAAACCCATTACTTTATGATGCCAACTATTTTCTTTGCTGGCATACTAATTGTTACGACTATTGTATACCTTACA
ATAGTGTAACTTCTTCAATTGTCATTACTTCAGGTGATGGCACAACAAGTCCTATTTCTGAACATGACTACCAGATTGGTGGTTATACTGA
AAAATGGGAATCTGGAGTAAAAGACTGTGTTGTATTACACAGTTACTTCACTTCAGACTATTACCAGCTGTACTCAACTCAATTGAGTAC
AGACACTGGTGTTGAACATGTTACCTTCTTCATCTACAATAAAATTGTTGATGAGCCTGAAGAACATGTCCAAATTCACACAATCGACGG
TTCATCCGGAGTTGTTAATCCAGTAATGGAACCAATTTATGATGAACCGACGACGACTACTAGCGTGCCTTTGTAAGCACAAGCTGATGA
GTACGAACTTATGTACTCATTCGTTTCGGAAGAGMCAGGTACGTTAATAGTTAATAGCGTACTTCTTTTTCTTGCTTTCGTGGTATTCTTG
CTAGTTACACTAGCCATCCTTACTGCGCTTCGATTGTGTGCGTACTGCTGCAATATTGTTAACGTGAGTCTTGTAAAACCTTCTTTTTACGT
TTACTCTCGTGTTAAAAATCTGAATTCTTCTAGAGTTCCTGATCTTCTGGTCTAAACGAACTAAATATTATATTAGTTTTTCTGTTTGGAACT
TTAATTTTAGCCATGGCAGATTCCAACGGTACTATTACCGTTGAAGAGCTTAAAAAGCTCCTTGAACAATGGAACCTAGTAATAGGTTTC
CTATTCCTTACATGGATTTGTCTTCTACAATTTGCCTATGCCAACAGGAATAGGTTTTTGTATATAATTAAGTTAATTTTCCTCTGGCTGTT
ATGGCCAGTAACTTTAGCTTGTTTTGTGCTTGCTGCTGTTTACAGAATAAATTGGATCACCGGTGGAATTGCTATCGCAATGGCTTGTCTT
GTAGGCTTGATGTGGCTCAGCTACTTCATTGCTTCTTTCAGACTGTTTGCGCGTACGCGTTCCATGTGGTCATTCAATCCAGAAACTAACA
TTCTTCTCAACGTGCCACTCCATGGCACTATTCTGACCAGACCGCTTCTAGAAAGTGAACTCGTAATCGGAGCTGTGATCCTTCGTGGAC
ATCTTCGTATTGCTGGACACCATCTAGGACGCTGTGACATCAAGGACCTGCCTAAAGAAATCACTGTTGCTACATCACGAATGCTTTCTTA
TTACAAATTGGGAGCTTCGCAGCGTGTAGCAGGTGACTCAGGTTTTGCTGCATACAGTCGCTACAGGATTGGCAACTATAAATTAAACA
CAGACCATTCCAGTAGCAGTGACAATATTGCTTTGCTTGTACAGTAAGTGACAACAGATGTTTCATCTCGTTGACTTTCAGGTTACTATAG
CAGAGATATTACTAATTATTATGAGGACTTTTAAAGTTTCCATTTGGAATCTTGATTACATCATAAACCTCATAATTAAAAATTTATCTAAG
TCACTAACTGAGAATAAATATTCTCAATTAGATGAAGAGCAACCAATGGAGATTGATTAAACGAACATGAAAATTATTCTTTTCTTGGCA
CTGATAACACTCGCTACTTGTGAGCTTTATCACTACCAAGAGTGTGTTAGAGGTACAACAGTACTTTTAAAAGAACCTTGCTCTTCTGGAA
CATACGAGGGCAATTCACCATTTCATCCTCTAGCTGATAACAAATTTGCACTGACTTGCTTTAGCACTCAATTTGCTTTTGCTTGTCCTGAC
GGCGTAAAACACGTCTATCAGTTACGTGCCAGATCAGTTTCACCTAAACTGTTCATCAGACAAGAGGAAGTTCAAGAACTTTACTCTCCA
ATTTTTCTTATTGTTGCGGCAATAGTGTTTATAACACTTTGCTTCACACTCAAAAGAAAGACAGAATGATTGAACTTTCATTAATTGACTTC
TATTTGTGCTTTTTAGCCTTTCTGCTATTCCTTGTTTTAATTATGCTTATTATCTTTTGGTTCTCACTTGAACTGCAAGATCATAATGAAACTT
GTCACGCCTAAACGAACATGAAATTTCTTGTTTTCTTAGGAATCATCACAACTGTAGCTGCATTTCACCAAGAATGTAGTTTACAGTCATG
TACTCAACATCAACCATATGTAGTTGATGACCCGTGTCCTATTCACTTCTATTCTAAATGGTATATTAGAGTAGGAGCTAGAAAATCAGCA
CCTTTAATTGAATTGTGCGTGGATGAGGCTGGTTCTAAATCACCCATTCAGTACATCGATATCGGTAATTATACAGTTTCCTGTTTACCTTT
TACAATTAATTGCCAGGAACCTAAATTGGGTAGTCTTGTAGTGCGTTGTTCGTTCTATGAAGACTTTTTAGAGTATCATGACGTTCGTGTT
GTTTTAGATTTCATCTAAACGAACAAACTAAAATGTCTGATAATGGACCCCAAAATCAGCGAAATGCACCCCGCATTACGTTTGGTGGAC
CCTCAGATTCAACTGGCAGTAACCAGAATGGAGAACGCAGTGGGGCGCGATCAAAACAACGTCGGCCCCAAGGTTTACCCAATAATACT
 
GCGTCTTGGTTCACCGCTCTCACTCAACATGGCAAGGAAGACCTTAAATTCCCTCGAGGACAAGGCGTTCCAATTAACACCAATAGCAGT
CCAGATGACCAAATTGGCTACTACCGAAGAGCTACCAGACGAATTCGTGGTGGTGACGGTAAAATGAAAGATCTCAGTCCAAGATGGT
ATTTCTACTACCTAGGAACTGGGCCAGAAGCTGGACTTCCCTATGGTGCTAACAAAGACGGCATCATATGGGTTGCAACTGAGGGAGCC
TTGAATACACCAAAAGATCACATTGGCACCCGCAATCCTGCTAACAATGCTGCAATCGTGCTACAACTTCCTCAAGGAACAACATTGCCA
AAAGGCTTCTACGCAGAAGGGAGCAGAGGCGGCAGTCAAGCCTCTTCTCGTTCCTCATCACGTAGTCGCAACAGTTCAAGAAATTCAAC
TCCAGGCAGCAGTAAACGAACTTCTCCTGCTAGAATGGCTGGCAATGGCGGTGATGCTGCTCTTGCTTTGCTGCTGCTTGACAGATTGAA
CCAGCTTGAGAGCAAAATGTCTGGTAAAGGCCAACAACAACAAGGCCAAACTGTCACTAAGAAATCTGCTGCTGAGGCTTCTAAGAAGC
CTCGGCAAAAACGTACTGCCACTAAAGCATACAATGTAACACAAGCTTTCGGCAGACGTGGTCCAGAACAAACCCAAGGAAATTTTGGG
GACCAGGAACTAATCAGACAAGGAACTGATTACAAACATTGGCCGCAAATTGCACAATTTGCCCCCAGCGCTTCAGCGTTCTTCGGAAT
GTCGCGCATTGGCATGGAAGTCACACCTTCGGGAACGTGGTTGACCTACACAGGTGCCATCAAATTGGATGACAAAGATCCAAATTTCA
AAGATCAAGTCATTTTGCTGAATAAGCATATTGACGCATACAAAACATTCCCACCAACAGAGCCTAAAAAGGACAAAAAGAAGAAGGCT
GATGAAACTCAAGCCTTACCGCAGAGACAGAAGAAACAGCAAACTGTGACTCTTCTTCCTGCTGCAGATTTGGATGATTTCTCCAAACAA
TTGCAACAATCCATGAGCAGTGCTGACTCAACTCAGGCCTAAACTCATGCAGACCACACAAGGCAGATGGGCTATATAAACGTTTTCGCT
TTTCCGTTTACGATATATAGTCTACTCTTGTGCAGAATGAATTCTCGTAACTACATAGCACAAGTAGATGTAGTTAACTTTAATCTCACATA
GCAATCTTTAATCAGTGTGTAACATTAGGGAGGACTTGAAAGAGCCACCACATTTTCACCGAGGCCACGCGGAGTACGATCGAGTGTAC
AGTGAACAATGCTAGGGAGAGCTGCCTATATGGAAGAGCCCTAATGTGTAAAATTAATTTTAGTAGTGCTATCCCCATGTGATTTTAATA
GCTTCTTAGGAGNATGACANNNNNNNNNNNNNN
SEQ ID NO: 16
   >Severe acute respiratory syndrome coronavirus 2 orflab polyprotein of isolate hCoV-19/Austria/CeMM0360/2020
SEQ ID NO: 17
   >SARS-CoV-2_S_MedUniWien(Sprotein_hCoV19AustriaCeMM03602020) 
MFVFLVLLPLVSSQCVNLTTRTQLPPAYTNSFTRGVYYPDKVFRSSVLHSTQDLFLPFFSNVTWFHAIHVSGTNGTKRFDNPVLPFNDGVYFAS
TEKSNIIRGWIFGTTLDSKTQSLLIVNNATNVVIKVCEFQFCNDPFLGVYYHKNNKSWMESEFRVYSSANNCTFEYVSQPFLMDLEGKQGNFK
NLREFVFKNIDGYFKIYSKHTPINLVRDLPQGFSALEPLVDLPIGINITRFQTLLALHRSYLTPGDSSSGWTAGAAAYYVGYLQPRTFLLKYNENGT
ITDAVDCALDPLSETKCTLKSFTVEKGIYQTSNFRXQPTESIVRFPNITNLCPFGEVFNATRFASVYAWNRKRISNCVADYSVLYNSASFSTFKCYG
VSPTKLNDLCFTNVYADSFVIRGDEVRQIAPGQTGKIADYNYKLPDDFTGCVIAWNSNNLDSKVGGNYNYLYRLFRKSNLKPFERDISTEIYQAG
STPCNGVEGFNCYFPLQSYGFQPTNGVGYQPYRVVVLSFELLHAPATVCGPKKSTNLVKNKCVNFNFNGLTGTGVLTESNKKFLPFQQFGRDI
ADTTDAVRDPQTLEILDITPCSFGGVSVITPGTNTSNQVAVLYQGVNCTEVPVAIHADQLTPTWRVYSTGSNVFQTRAGCLIGAEHVNNSYEC
DIPIGAGICASYQTQTNSPRRARSVASQSIIAYTMSLGAENSVAYSXNSIAIPTNFTISVTTEILPVSMTKTSVDCTMYICGDSTECSNLLLQYGSFC
TQLNRALTGIAVEQDKNTQEVFAQVKQIYKTPPIKDFGGFNFSQILPDPSKPSKRSFIEDLLFNKVTLADAGFIKQYGDCLGDIAARDLICAQKFN
GLTVLPPLLTDEMIAQYTSALLAGTITSGWTFGAGAALQIPFAMQMAYRFNGIGVTQNVLYENQKLIANQFNSAIGKIQDSLSSTASALGKLQD
VVNQNAQALNTLVKQLSSNFGAISSVLNDILSRLDKVEAEVQIDRLITGRLQSLQTYVTQQLIRAAEIRVSANLAATKMSECVLGQSKRVDFCGK
GYHLMSFPQSAPHGVVFLHVTYVPAQEKNFTTAPAICHDGKAHFPREGVFVSNGTHWFVTQRNFYEPQIITTDNTFVSGNCDVVIGIVNNTV
YDPLQPELDSFKEELDKYFKNHTSPDVDLGDISGINASVVNIQKEIDRLNEVAKNLNESLIDLQELGKYEQYIKWPWYIWLGFIAGLIAIVMVTIM
LCCMTSCCSCLKGCCSCGSCCKFDEDDSEPVLKGVKLHYT
SEQ ID NO: 18
   >Severe acute respiratory syndrome coronavirus 2 isolate SARS-CoV-2/human/England/ex-SA/2021, EVAg Ref-SKU:004V-04071 (SA_P2) complete genome. South-African B.1.351 lineage 
ATTAAAGGTTTATACCTTCCCAGGTAACAAACCAACCAACTTTCGATCTCTTGTAGATCTGTTCTCTAAACGAACTTTAAAAT
CTGTGTGGCTGTCACTCGGCTGCATGCTTAGTGCACTCACGCAGTATAATTAATAACTAATTACTGTCGTTGACAGGACAC
GAGTAACTCTTCTATCTTCTGCAGGCTGCTTACGGTTTCGTCCGTGTTGCAGCCGATCATCAGCACATCTAGGTTTTGTCCG
GGTGTGACCGAAAGGTAAGATGGAGAGCCTTGTCCCTGGTTTCAACGAGAAAACACACGTCCAACTCAGTTTGCCTGTTTT
ACAGGTTCGCGACGTGCTCGTACGTGGCTTTGGAGACTCCGTGGAGGAGGTCTTATCAGAGGCACGTCAACATCTTAAAG
ATGGCACTTGTGGCTTAGTAGAAGTTGAAAAAGGCGTTTTGCCTCAACTTGAACAGCCCTATGTGTTCATCAAACGTTCGG
ATGCTCGAACTGCACCTCATGGTCATGTTATGGTTGAGCTGGTAGCAGAACTCGAAGGCATTCAGTACGGTCGTAGTGGT
GAGACACTTGGTGTCCTTGTCCCTCATGTGGGCGAAATACCAGTGGCTTACCGCAAGGTTCTTCTTCGTAAGAACGGTAAT
AAAGGAGCTGGTGGCCATAGTTACGGCGCCGATCTAAAGTCATTTGACTTAGGCGACGAGCTTGGCACTGATCCTTATGA
AGATTTTCAAGAAAACTGGAACACTAAACATAGCAGTGGTGTTACCCGTGAACTCATGCGTGAGCTTAACGGAGGGGCAT
ACACTCGCTATGTCGATAACAACTTCTGTGGCCCTGATGGCTACCCTCTTGAGTGCATTAAAGACCTTCTAGCACGTGCTGG
TAAAGCTTCATGCACTTTGTCCGAACAACTGGACTTTATTGACACTAAGAGGGGTGTATACTGCTGCCGTGAACATGAGCA
TGAAATTGCTTGGTACACGGAACGTTCTGAAAAGAGCTATGAATTGCAGACACCTTTTGAAATTAAATTGGCAAAGAAATT
TGACATCTTCAATGGGGAATGTCCAAATTTTGTATTTCCCTTAAATTCCATAATCAAGACTATTCAACCAAGGGTTGAAAAG
AAAAAGCTTGATGGCTTTATGGGTAGAATTCGATCTGTCTATCCAGTTGCGTCACCAAATGAATGCAACCAAATGTGCCTTT
CAACTCTCATGAAGTGTGATCATTGTGGTGAAACTTCATGGCAGACGGGCGATTTTGTTAAAGCCACTTGCGAATTTTGTG
GCACTGAGAATTTGACTAAAGAAGGTGCCACTACTTGTGGTTACTTACCCCAAAATGCTGTTGTTAAAATTTATTGTCCAGC
ATGTCACAATTCAGAAGTAGGACCTGAGCATAGTCTTGCCGAATACCATAATGAATCTGGCTTGAAAACCATTCTTCGTAA
GGGTGGTCGCACTATTGCCTTTGGAGGCTGTGTGTTCTCTTATGTTGGTTGCCATAACAAGTGTGCCTATTGGGTTCCACGT
GCTAGCGCTAACATAGGTTGTAACCATACAGGTGTTGTTGGAGAAGGTTCCGAAGGTCTTAATGACAACCTTCTTGAAATA
CTCCAAAAAGAGAAAGTCAACATCAATATTGTTGGTGACTTTAAACTTAATGAAGAGATCGCCATTATTTTGGCATCTTTTT
CTGCTTCCACAAGTGCTTTTGTGGAAACTGTGAAAGGTTTGGATTATAAAGCATTCAAACAAATTGTTGAATCCTGTGGTA
ATTTTAAAGTTACAAAAGGAAAAGCTAAAAAAGGTGCCTGGAATATTGGTGAACAGAAATCAATACTGAGTCCTCTTTATG
CATTTGCATCAGAGGCTGCTCGTGTTGTACGATCAATTTTCTCCCGCACTCTTGAAACTGCTCAAAATTCTGTGCGTGTTTTA
CAGAAGGCCGCTATAACAATACTAGATGGAATTTCACAGTATTCACTGAGACTCATTGATGCTATGATGTTCACATCTGATT
TGGCTACTAACAATCTAGTTGTAATGGCCTACATTACAGGTGGTGTTGTTCAGTTGACTTCGCAGTGGCTAACTAACATCTT
TGGCACTGTTTATGAAAAACTCAAACCCGTCCTTGATTGGCTTGAAGAGAAGTTTAAGGAAGGTGTAGAGTTTCTTAGAGA
CGGTTGGGAAATTGTTAAATTTATCTCAACCTGTGCTTGTGAAATTGTCGGTGGACAAATTGTCACCTGTGCAAAGGAAAT
TAAGGAGAGTGTTCAGACATTCTTTAAGCTTGTAAATAAATTTTTGGCTTTGTGTGCTGACTCTATCATTATTGGTGGAGCT
 
AAACTTAAAGCCTTGAATTTAGGTGAAACATTTGTCACGCACTCAAAGGGATTGTACAGAAAGTGTGTTAAATCCAGAGAA
GAAACTGGCCTACTCATGCCTCTAAAAGCCCCAAAAGAAATTATCTTCTTAGAGGGAGAAACACTTCCCACAGAAGTGTTA
ACAGAGGAAGTTGTCTTGAAAACTGGTGATTTACAACCATTAGAACAACCTACTAGTGAAGCTGTTGAAGCTCCATTGGTT
GGTACACCAGTTTGTATTAACGGGCTTATGTTGCTCGAAATCAAAGACACAGAAAAGTACTGTGCCCTTGCACCTAATATG
ATGGTAACTAACAATACCTTCACACTCAAAGGCGGTGCACCAACAAAGGTTACTTTTGGTGATGACACTGTGATAGAAGTG
CAAGGTTACAAGAGTGTGAATATCACTTTTGAACTTGATGAAAGGATTGATAAAGTACTTAATGAGAAGTGCTCTGCCTAT
ACAGTTGAACTCGGTACAGAAGTAAATGAGTTCGCCTGTGTTGTGGCAGATGCTGTCATAAAAACTTTGCAACCAGTATCT
GAATTACTTACACCACTGGGCATTGATTTAGATGAGTGGAGTATGGCTACATACTACTTATTTGATGAGTCTGGTGAGTTTA
AATTGGCTTCACATATGTATTGTTCTTTTTACCCTCCAGATGAGGATGAAGAAGAAGGTGATTGTGAAGAAGAAGAGTTTG
AGCCATCAACTCAATATGAGTATGGTACTGAAGATGATTACCAAGGTAAACCTTTGGAATTTGGTGCCACTTCTGCTGCTCT
TCAACCTGAAGAAGAGCAAGAAGAAGATTGGTTAGATGATGATAGTCAACAAACTGTTGGTCAACAAGACGGCAGTGAG
GACAATCAGACAACTACTATTCAAACAATTGTTGAGGTTCAACCTCAATTAGAGATGGAACTTACACCAGTTGTTCAGACTA
TTGAAGTGAATAGTTTTAGTGGTTATTTAAAACTTACTGACAATGTATACATTAAAAATGCAGACATTGTGGAAGAAGCTA
AAAAGGTAAAACCAACAGTGGTTGTTAATGCAGCCAATGTTTACCTTAAACATGGAGGAGGTGTTGCAGGAGCCTTAAAT
AAGGCTACTAACAATGCCATGCAAGTTGAATCTGATGATTACATAGCTACTAATGGACCACTTAAAGTGGGTGGTAGTTGT
GTTTTAAGCGGACACAATCTTGCTAAACACTGTCTTCATGTTGTCGGCCCAAATGTTAACAAAGGTGAAGACATTCAACTTC
TTAAGAGTGCTTATGAAAATTTTAATCAGCACGAAGTTCTACTTGCACCATTATTATCAGCTGGTATTTTTGGTGCTGACCCT
ATACATTCTTTAAGAGTTTGTGTAGATACTGTTCGCACAAATGTCTACTTAGCTGTCTTTGATAAAAATCTCTATGACAAACT
TGTTTCAAGCTTTTTGGAAATGAAGAGTGAAAAGCAAGTTGAACAAAAGATCGCTGAGATTCCTAAAGAGGAAGTTAAGC
CATTTATAACTGAAAGTAAACCTTCAGTTGAACAGAGAAAACAAGATGATAAGAAAATCAAAGCTTGTGTTGAAGAAGTT
ACAACAACTCTGGAAGAAACTAAGTTCCTCACAGAAAACTTGTTACTTTATATTGACATTAATGGCAATCTTCATCCAGATT
CTGCCACTCTTGTTAGTGACATTGACATCACTTTCTTAAAGAAAGATGCTCCATATATAGTGGGTGATGTTGTTCAAGAGGG
TGTTTTAACTGCTGTGGTTATACCTACTAAAAAGGCTGGTGGCACTACTGAAATGTTAGCGAAAGCTTTGAGAAAAGTGCC
AACAGACAATTATATAACCACTTACCCGGGTCAGGGTTTAAATGGTTACACTGTAGAGGAGGCAAAGACAGTGCTTAAAA
AGTGTAAAAGTGCCTTTTACATTCTACCATCTATTATCTCTAATGAGAAGCAAGAAATTCTTGGAACTGTTTCTTGGAATTTG
CGAGAAATGCTTGCACATGCAGAAGAAACACGCAAATTAATGCCTGTCTGTGTGGAAACTAAAGCCATAGTTTCAACTATA
CAGCGTAAATATAAGGGTATTAAAATACAAGAGGGTGTGGTTGATTATGGTGCTAGATTTTACTTTTACACCAGTAAAACA
ACTGTAGCGTCACTTATCAACACACTTAACGATCTAAATGAAACTCTTGTTACAATGCCACTTGGCTATGTAACACATGGCT
TAAATTTGGAAGAAGCTGCTCGGTATATGAGATCTCTCAAAGTGCCAGCTACAGTTTCTGTTTCTTCACCTGATGCTGTTAC
AGCGTATAATGGTTATCTTACTTCTTCTTCTAAAACACCTGAAGAACATTTTATTGAAACCATCTCACTTGCTGGTTCCTATA
AAGATTGGTCCTATTCTGGACAATCTACACAACTAGGTATAGAATTTCTTAAGAGAGGTGATAAAAGTGTATATTACACTA
GTAATCCTACCACATTCCACCTAGATGGTGAAGTTATCACCTTTGACAATCTTAAGACACTTCTTTCTTTGAGAGAAGTGAG
GACTATTAAGGTGTTTACAACAGTAGACAACATTAACCTCCACACGCAAGTTGTGGACATGTCAATGACATATGGACAACA
GTTTGGTCCAACTTATTTGGATGGAGCTGATGTTACTAAAATAAAACCTCATAATTCACATGAAGGTAAAACATTTTATGTT
TTACCTAATGATGACACTCTACGTGTTGAGGCTTTTGAGTACTACCACACAACTGATCCTAGTTTTCTGGGTAGGTACATGT
CAGCATTAAATCACACTAAAAATTGGAAATACCCACAAGTTAATGGTTTAACTTCTATTAAATGGGCAGATAACAACTGTTA
TCTTGCCACTGCATTGTTAACACTCCAACAAATAGAGTTGAAGTTTAATCCACCTGCTCTACAAGATGCTTATTACAGAGCA
AGGGCTGGTGAAGCTGCTAACTTTTGTGCACTTATCTTAGCCTACTGTAATAAGACAGTAGGTGAGTTAGGTGATGTTAGA
GAAACAATGAGTTACTTGTTTCAACATGCCAATTTAGATTCTTGCAAAAGAGTCTTGAACGTGGTGTGTAAAACTTGTGGA
CAACAGCAGACAACCCTTAAGGGTGTAGAAGCTGTTATGTACATGGGCACACTTTCTTATGAACAATTTAAGAAAGGTGTT
CAGATACCTTGTACGTGTGGTAAACAAGCTACAAAATATCTAGTACAACAGGAGTCACCTTTTGTTATGATGTCAGCACCA
CCTGCTCAGTATGAACTTAAGCATGGTACATTTACTTGTGCTAGTGAGTACACTGGTAATTACCAGTGTGGTCACTATAAAC
ATATAACTTCTAAAGAAACTTTGTATTGCATAGACGGTGCTTTACTTACAAAGTCCTCAGAATACAAAGGTCCTATTACGGA
TGTTTTCTACAAAGAAAACAGTTACACAACAACCATAAAACCAGTTACTTATAAATTGGATGGTGTTGTTTGTACAGAAATT
GACCCTAAGTTGGACAATTATTATAAGAAAGACAATTCTTATTTCACAGAGCAACCAATTGATCTTGTACCAAACCAACCAT
ATCCAAACGCAAGCTTCGATAATTTTAAGTTTGTATGTGATAATATCAAATTTGCTGATGATTTAAACCAGTTAACTGGTTA
CAAGAAACCTGCTTCAAGAGAGCTTAAAGTTACATTTTTCCCTGACTTAAATGGTGATGTGGTGGCTATTGATTATAAACAC
TACACACCCTCTTTTAAGAAAGGAGCTAAATTGTTACATAAACCTATTGTTTGGCATGTTAACAATGCAACTAATAAAGCCA
CGTATAAACCAAATACCTGGTGTATACGTTGTCTTTGGAGCACAAAACCAGTTGAAACATCAAATTCGTTTGATGTACTGAA
GTCAGAGGACGCGCAGGGAATGGATAATCTTGCCTGCGAAGATCTAAAACCAGTCTCTGAAGAAGTAGTGGAAAATCCTA
CCATACAGAAAGACGTTCTTGAGTGTAATGTGAAAACTACCGAAGTTGTAGGAGACATTATACTTAAACCAGCAAATAATA
GTTTAAAAATTACAGAAGAGGTTGGCCACACAGATCTAATGGCTGCTTATGTAGACAATTCTAGTCTTACTATTAAGAAAC
CTAATGAATTATCTAGAGTATTAGGTTTGAAAACCCTTGCTACTCATGGTTTAGCTGCTGTTAATAGTGTCCCTTGGGATAC
TATAGCTAATTATGCTAAGCCTTTTCTTAACAAAGTTGTTAGTACAACTACTAACATAGTTACACGGTGTTTAAACCGTGTTT
GTACTAATTATATGCCTTATTTCTTTACTTTATTGCTACAATTGTGTACTTTTACTAGAAGTACAAATTCTAGAATTAAAGCAT
 
CTATGCCGACTACTATAGCAAAGAATACTGTTAAGAGTGTCGGTAAATTTTGTCTAGAGGCTTCATTTAATTATTTGAAGTC
ACCTAATTTTTCTAAACTGATAAATATTATAATTTGGTTTTTACTATTAAGTGTTTGCCTAGGTTCTTTAATMTACTCAACCG
CTGCTTTAGGTGTTTTAATGTCTAATTTAGGCATGCCTTCTTACTGTACTGGTTACAGAGAAGGCTATTTGAACTCTACTAAT
GTCACTATTGCAACCTACTGTACTGGTTCTATACCTTGTAGTGTTTGTCTTAGTGGTTTAGATTCTTTAGACACCTATCCTTCT
TTAGAAACTATACAAATTACCATTTCATCTTTTAAATGGGATTTAACTGCTTTTGGCTTAGTTGCAGAGTGGTTTTTGGCATA
TATTCTTTTCACTAGGTTTTTCTATGTACTTGGATTGGCTGCAATCATGCAATTGTTTTTCAGCTATTTTGCAGTACATTTTAT
TAGTAATTCTTGGCTTATGTGGTTAATAATTAATCTTGTACAAATGGCCCCGATTTCAGCTATGGTTAGAATGTACATCTTCT
TTGCATCATTTTATTATGTATGGAAAAGTTATGTGCATGTTGTAGACGGTTGTAATTCATCAACTTGTATGATGTGTTACAA
ACGTAATAGAGCAACAAGAGTCGAATGTACAACTATTGTTAATGGTGTTAGAAGGTCCTTTTATGTCTATGCTAATGGAGG
TAAAGGCTTTTGCAAACTACACAATTGGAATTGTGTTAATTGTGATACATTCTGTGCTGGTAGTACATTTATTAGTGATGAA
GTTGCGAGAGACTTGTCACTACAGTTTAAAAGACCAATAAATCCTACTGACCAGTCTTCTTACATCGTTGATAGTGTTACAG
TGAAGAATGGTTCCATCCATCTTTACTTTGATAAAGCTGGTCAAAAGACTTATGAAAGACATTCTCTCTCTCATTTTGTTAAC
TTAGACAACCTGAGAGCTAATAACACTAAAGGTTCATTGCCTATTAATGTTATAGTTTTTGATGGTAAATCAAAATGTGAAG
AATCATCTGCAAAATCAGCGTCTGTTTACTACAGTCAGCTTATGTGTCAACCTATACTGTTACTAGATCAGGCATTAGTGTC
TGATGTTGGTGATAGTGCGGAAGTTGCAGTTAAAATGTTTGATGCTTACGTTAATACGTTTTCATCAACTTTTAACGTACCA
ATGGAAAAACTCAAAACACTAGTTGCAACTGCAGAAGCTGAACTTGCAAAGAATGTGTCCTTAGACAATGTCTTATCTACT
TTTATTTCAGCAGCTCGGCAAGGGTTTGTTGATTCAGATGTAGAAACTAAAGATGTTGTTGAATGTCTTAAATTGTCACATC
AATCTGACATAGAAGTTACTGGCGATAGTTGTAATAACTATATGCTCACCTATAACAAAGTTGAAAACATGACACCCCGTG
ACCTTGGTGCTTGTATTGACTGTAGTGCGCGTCATATTAATGCGCAGGTAGCAAAAAGTCACAACATTGCTTTGATATGGA
ACGTTAAAGATTTCATGTCATTGTCTGAACAACTACGAAAACAAATACGTAGTGCTGCTAAAAAGAATAACTTACCTTTTAA
GTTGACATGTGCAACTACTAGACAAGTTGTTAATGTTGTAACAACAAAGATAGCACTTAAGGGTGGTAAAATTGTTAATAA
TTGGTTGAAGCAGTTAATTAAAGTTACACTTGTGTTCCTTTTTGTTGCTGCTATTTTCTATTTAATAACACCTGTTCATGTCAT
GTCTAAACATACTGACTTTTCAAGTGAAATCATAGGATACAAGGCTATTGATGGTGGTGTCACTCGTGACATAGCATCTAC
AGATACTTGTTTTGCTAACAAACATGCTGATTTTGACACATGGTTTAGCCAGCGTGGTGGTAGTTATACTAATGACAAAGCT
TGCCCATTGATTGCTGCAGTCATAACAAGAGAAGTGGGTTTTGTCGTGCCTGGTTTGCCTGGCACGATATTACGCACAACT
AATGGTGACTTTTTGCATTTCTTACCTAGAGTTTTTAGTGCAGTTGGTAACATCTGTTACACACCATCAAAACTTATAGAGTA
CACTGACTTTGCAACATCAGCTTGTGTTTTGGCTGCTGAATGTACAATTTTTAAAGATGCTTCTGGTAAGCCAGTACCATAT
TGTTATGATACCAATGTACTAGAAGGTTCTGTTGCTTATGAAAGTTTACGCCCTGACACACGTTATGTGCTCATGGATGGCT
CTATTATTCAATTTCCTAACACCTACCTTGAAGGTTCTGTTAGAGTGGTAACAACTTTTGATTCTGAGTACTGTAGGCACGG
CACTTGTGAAAGATCAGAAGCTGGTGTTTGTGTATCTACTAGTGGTAGATGGGTACTTAACAATGATTATTACAGATCTTTA
CCAGGAGTTTTCTGTGGTGTAGATGCTGTAAATTTATTTACTAATATGTTTACACCACTAATTCAACCTATTGGTGCTTTGGA
CATATCAGCATCTATAGTAGCTGGTGGTATTGTAGCTATCGTAGTAACATGCCTTGCCTACTATTTTATGAGGTTTAGAAGA
GCTTTTGGTGAATACAGTCATGTAGTTGCCTTTAATACTTTACTATTCCTTATGTCATTCACTGTACTCTGTTTAACACCAGTT
TACTCATTCTTACCTGGTGTTTATTCTGTTATTTACTTGTACTTGACATTTTATCTTACTAATGATGTTTCTTTTTTAGCACATA
TTCAGTGGATGGTTATGTTCACACCTTTAGTACCTTTCTGGATAACAATTGCTTATATCATTTGTATTTCCACAAAGCATTTCT
ATTGGTTCTTTAGTAATTACCTAAAGAGACGTGTAGTCTTTAATGGTGTTTCCTTTAGTACTTTTGAAGAAGCTGCGCTGTG
CACCTTTTTGTTAAATAAAGAAATGTATCTAAAGTTGCGTAGTGATGTGCTATTACCTCTTACGCAATATAATAGATACTTA
GCTCTTTATAATAAGTACAAGTATTTTAGTGGAGCAATGGATACAACTAGCTACAGAGAAGCTGCTTGTTGTCATCTCGCA
AAGGCTCTCAATGACTTCAGTAACTCAGGTTCTGATGTTCTTTACCAACCACCACAAACCTCTATCACCTCAGCTGTTTTGCA
GAGTGGTTTTAGAAAAATGGCATTCCCATCTGGTAAAGTTGAGGGTTGTATGGTACAAGTAACTTGTGGTACAACTACACT
TAACGGTCTTTGGCTTGATGACGTAGTTTACTGTCCAAGACATGTGATCTGCACCTCTGAAGACATGCTTAACCCTAATTAT
GAAGATTTACTCATTCGTAAGTCTAATCATAATTTCTTGGTACAGGCTGGTAATGTTCAACTCAGGGTTATTGGACATTCTA
TGCAAAATTGTGTACTTAAGCTTAGGGTTGATACAGCCAATCCTAAGACACCTAAGTATAAGTTTGTTCGCATTCAACCAG
GACAGACTTTTTCAGTGTTAGCTTGTTACAATGGTTCACCATCTGGTGTTTACCAATGTGCTATGAGGCCCAATTTCACTATT
AAGGGTTCATTCCTTAATGGTTCATGTGGTAGTGTTGGTTTTAACATAGATTATGACTGTGTCTCTTTTTKTTACATGCACCA
TATGGAATTACCAACTGGAGTTCATGCTGGCACAGACTTAGAAGGTAACTTTTATGGACCTTTTGTTGACAGGCAAACAGC
ACAAGCAGCTGGTACGGACACAACTATTACAGTTAATGTTTTAGCTTGGTTGTACGCTGCTGTTATAAATGGAGACAGGTG
GTTTCTCAATCGATTTACCACAACTCTTAATGACTTTAACCTTGTGGCTATGAAGTACAATTATGAACYTCTAACACAAGACC
ATGTTGACATACTAGGACCTCTTTCTGCTCAAACTGGAATTGCCGTTTTAGATATGTGTGCTTCATTAAAAGAATTACTGCA
AAATGGTATGAATGGACGTACCATATTGGGTAGTGCTTTATTAGAAGATGAATTTACACCTTTTGATGTTGTTAGACAATG
CTCAGGTGTTACTTTCCAAAGTGCAGTGAAAAGAACAATCAAGGGTACACACCACTGGTTGTTACTCACAATTTTGACTTCA
CTTTTAGTTTTAGTCCAGAGTACTCAATGGTCTTTGTTCTTTTTTTTGTATGAAAATGCCTTTTTACCTTTTGCTATGGGTATT
ATTGCTATGTCTGCTTTTGCAATGATGTTTGTCAAACATAAGCATGCATTTCTCTGTTTGTTTTTGTTACCTTCTCTTGCCACT
GTAGCTTATTTTAATATGGTCTATATGCCTGCTAGTTGGGTGATGCGTATTATGACATGGTTGGATATGGTTGATACTAGTT
TGNNNNNNAAGCTAAAAGACTGTGTTATGTATGCATCAGCTGTAGTGTTACTAATCCTTATGACAGCAAGAACTGTGTAT
 
GATGATGGTGCTAGGAGAGTGTGGACACTTATGAATGTCTTGACACTCGTTTATAAAGTTTATTATGGTAATGCTTTAGAT
CAAGCCATTTCCATGTGGGCTCTTATAATCTCTGTTACTTCTAACTACTCAGGTGTAGTTACAACTGTCATGTTTTTGGCCAG
AGGTATTGTTTTTATGTGTGTTGAGTATTGCCCTATTTTCTTCATAACTGGTAATACACTTCAGTGTATAATGCTAGTTTATT
GTTTCTTAGGCTATTTTTGTACTTGTTACTTTGGCCTCTTTTGTTTACTCAACCGCTACTTTAGACTGACTCTTGGTGTTTATG
ATTACTTAGTTTCTACACAGGAGTTTAGATATATGAATTCACAGGGACTAYTCCCACCCAAGAATAGCATAGATGCCTTCAA
ACTCAACATTAAATTGTTGGGTGTTGGTGGCAAACCTTGTATCAAAGTAGCCACTGTACAGTCTAAAATGTCAGATGTAAA
GTGCACATCAGTAGTCTTACTCTCAGTTTTGCAACAACTCAGAGTAGAATCATCATCTAAATTGTGGGCTCAATGTGTCCAG
TTACACAATGACATTCTCTTAGCTAAAGATACTACTGAAGCCTTTGAAAAAATGGTTTCACTACTTTCTGTTTTGCTTTCCAT
GCAGGGTGCTGTAGACATAAACAAGCTTTGTGAAGAAATGCTGGACAACAGGGCAACCTTACAAGCTATAGCCTCAGAGT
TTAGTTCCCTTCCATCATATGCAGCTTTTGCTACTGCTCAAGAAGCTTATGAGCAGGCTGTTGCTAATGGTGATTCTGAAGT
TGTTCTTAAAAAGTTGAAGAAGTCTTTGAATGTGGCTAAATCTGAATTTGACCGTGATGCAGCCATGCAACGTAAGTTGGA
AAAGATGGCTGATCAAGCTATGACCCAAATGTATAAACAGGCTAGATCTGAGGACAAGAGGGCAAAAGTTACTAGTGCTA
TGCAGACAATGCTTTTCACTATGCTTAGAAAGTTGGATAATGATGCACTCAACAACATTATCAACAATGCAAGAGATGGTT
GTGTTCCCTTGAACATAATACCTCTTACAACAGCAGCCAAACTAATGGTTGTCATACCAGACTATAACACATATAAAAATAC
GTGTGATGGTACAACATTTACTTATGCATCAGCATTGTGGGAAATCCAACAGGTTGTAGATGCAGATAGTAAAATTGTTCA
ACTTAGTGAAATTAGTATGGACAATTCACCTAATTTAGCATGGCCTCTTATTGTAACAGCTTTAAGGGCCAATTCTGCTGTC
AAATTACAGAATAATGAGCTTAGTCCTGTTGCACTACGACAGATGTCTTGTGCTGCCGGTACTACACAAACTGCTTGCACT
GATGACAATGCGTTAGCTTACTACAACACAACAAAGGGAGGTAGGTTTGTACTTGCACTGTTATCCGATTTACAGGATTTG
AAATGGGCTAGATTCCCTAAGAGTGATGGAACTGGTACTATCTATACAGAACTGGAACCACCTTGTAGGTTTGTTACAGAC
ACACCTAAAGGTCCTAAAGTGAAGTATTTATACTTTATTAAAGGATTAAACAACCTAAATAGAGGTATGGTACTTGGTAGT
TTAGCTGCCACAGTACGTCTACAAGCTGGTAATGCAACAGAAGTGCCTGCCAATTCAACTGTATTATCTTTCTGTGCTTTTG
CTGTAGATGCTGCTAAAGCTTACAAAGATTATCTAGCTAGTGGGGGACAACCAATCACTAATTGTGTTAAGATGTTGTGTA
CACACACTGGTACTGGTCAGGCAATAACAGTTACACCGGAAGCCAATATGGATCAAGAATCCTTTGGTGGTGCATCGTGTT
GTCTGTACTGCCGTTGCCACATAGATCATCCAAATCCTAAAGGATTTTGTGACTTAAAAGGTAAGTATGTACAAATACCTAC
AACTTGTGCTAATGACCCTGTGGGTTTTACACTTAAAAACACAGTCTGTACCGTCTGCGGTATGTGGAAAGGTTATGGCTG
TAGTTGTGATCAACTCCGCGAACCCATGCTTCAGTCAGCTGATGCACAATCGTTTTTAAACGGGTTTGCGGTGTAAGTGCA
GCCCGTCTTACACCGTGCGGCACAGGCACTAGTACTGATGTCGTATACAGGGCTTTTGACATCTACAATGATAAAGTAGCT
GGTTTTGCTAAATTCCTAAAAACTAATTGTTGTCGCTTCCAAGAAAAGGACGAAGATGACAATTTAATTGATTCTTACTTTG
TAGTTAAGAGACACACTTTCTCTAACTACCAACATGAAGAAACAATTTATAATTTACTTAAGGATTGTCCAGCTGTTGCTAA
ACATGACTTCTTTAAGTTTAGAATAGACGGTGACATGGTACCACATATATCACGTCAACGTCTTACTAAATACACAATGGCA
GACCTCGTCTATGCTTTAAGGCATTTTGATGAAGGTAATTGTGACACATTAAAAGAAATACTTGTCACATACAATTGTTGTG
ATGATGATTATTTCAATAAAAAGGACTGGTATGATTTTGTAGAAAACCCAGATATATTACGCGTATACGCCAACTTAGGTG
AACGTGTACGCCAAGCTTTGTTAAAAACAGTACAATTCTGTGATGCCATGCGAAATGCTGGTATTGTTGGTGTACTGACAT
TAGATAATCAAGATCTCAATGGTAACTGGTATGATTTCGGTGATTTCATACAAACCACGCCAGGTAGTGGAGTTCCTGTTG
TAGATTCTTATTATTCATTGTTAATGCCTATATTAACCTTGACCAGGGCTTTAACTGCAGAGTCACATGTTGACACTGACTTA
ACAAAGCCTTACATTAAGTGGGATTTGTTAAAATATGACTTCACGGAAGAGAGGTTAAAACTCTTTGACCGTTATTTTAAAT
ATTGGGATCAGACATACCACCCAAATTGTGTTAACTGTTTGGATGACAGATGCATTCTGCATTGTGCAAACTTTAATGTTTT
ATTCTCTACAGTGTTCCCACTTACAAGTTTTGGACCACTAGTGAGAAAAATATTTGTTGATGGTGTTCCATTTGTAGTTTCAA
CTGGATACCACTTCAGAGAGCTAGGTGTTGTACATAATCAGGATGTAAACTTACATAGCTCTAGACTTAGTTTTAAGGAAT
TACTTGTGTATGCTGCTGACCCTGCTATGCACGCTGCTTCTGGTAATCTATTACTAGATAAACGCACTACGTGCTTTTCAGT
AGCTGCACTTACTAACAATGTTGCTTTTCAAACTGTCAAACCCGGTAATTTTAACAAAGACTTCTATGACTTTGCTGTGTCTA
AGGGTTTCTTTAAGGAAGGAAGTTCTGTTGAATTAAAACACTTCTTCTTTGCTCAGGATGGTAATGCTGCTATCAGCGATTA
TGACTACTATCGTTATAATCTACCAACAATGTGTGATATCAGACAACTACTATTTGTAGTTGAAGTTGTTGATAAGTACTTT
GATTGTTACGATGGTGGCTGTATTAATGCTAACCAAGTCATCGTCAACAACCTAGACAAATCAGCTGGTTTTCCATTTAATA
AATGGGGTAAGGCTAGACTTTATTATGATTCAATGAGTTATGAGGATCAAGATGCACTTTTCGCATATACAAAACGTAATG
TCATCCCTACTATAACTCAAATGAATCTTAAGTATGCCATTAGTGCAAAGAATAGAGCTCGCACCGTAGCTGGTGTCTCTAT
CTGTAGTACTATGACCAATAGACAGTTTCATCAAAAATTATTGAAATCAATAGCCGCCACTAGAGGAGCTACTGTAGTAAT
TGGAACAAGCAAATTCTATGGTGGTTGGCACAACATGTTAAAAACTGTTTATAGTGATGTAGAAAACCCTCACCTTATGGG
TTGGGATTATCCTAAATGTGATAGAGCCATGCCTAACATGCTTAGAATTATGGCCTCACTTGTTCTTGCTCGCAAACATACA
ACGTGTTGTAGCTTGTCACACCGTTTCTATAGATTAGCTAATGAGTGTGCTCAAGTATTGAGTGAAATGGTCATGTGTGGC
GGTTCACTATATGTTAAACCAGGTGGAACCTCATCAGGAGATGCCACAACTGCTTATGCTAATAGTGTTTTTAACATTTGTC
AAGCTGTCACGGCCAATGTTAATGCACTTTTATCTACTGATGGTAACAAAATTGCCGATAAGTATGTCCGCAATTTACAAC
ACAGACTTTATGAGTGTCTCTATAGAAATAGAGATGTTGACACAGACTTTGTGAATGAGTTTTACGCATATTTGCGTAAAC
ATTTCTCAATGATGATACTCTCTGACGATGCTGTTGTGTGTTTCAATAGCACTTATGCATCTCAAGGTCTAGTGGCTAGCAT
AAAGAACTTTAAGTCAGTTCTTTATTATCAAAACAATGTTTTTATGTCTGAAGCAAAATGTTGGACTGAGACTGACCTTACT
 
AAAGGACCTCATGAATTTTGCTCTCAACATACAATGCTAGTTAAACAGGGTGATGATTATGTGTACCTTCCTTACCCAGATC
CATCAAGAATCCTAGGGGCCGGCTGTTTTGTAGATGATATCGTAAAAACAGATGGTACACTTATGATTGAACGGTTCGTGT
CTTTAGCTATAGATGCTTACCCACTTACTAAACATCCTAATCAGGAGTATGCTGATGTCTTTCATTTGTACTTACAATACATA
AGAAAGCTACATGATGAGTTAACAGGACACATGTTAGACATGTATTCTGTTATGCTTACTAATGATAACACTTCAAGGTATT
GGGAACCTGAGTTTTATGAGGCTATGTACACACCGCATACAGTCTTACAGGCTGTTGGGGCTTGTGTTCTTGCAATTCAC
AGACTTCATTAAGATGTGGTGCTTGCATACGTAGACCATTCTTATGTTGTAAATGCTGTTACGACCATGTCATATCAACATC
ACATAAATTAGTCTTGTCTGTTAATCCGTATGTTTGCAATGCTTCAGGTTGTGATGTCACAGATGTGACTCAACTTTACTTAG
GAGGTATGAGCTATTATTGTAAATCACATAAACCACCCATTAGTTTTCCATTGTGTGCTAATGGACAAGTTTTTGGTTTATAT
AAAAATACATGTGTTGGTAGCGATAATGTTACTGACTTTAATGCAATTGCAACATGTGACTGGACAAATGCTGGTGATTAC
ATTTTAGCTAACACCTGTACTGAAAGACTCAAGCTTTTTGCAGCAGAAACGCTCAAAGCTACTGAGGAGACATTTAAACTG
TCTTATGGTATTGCTACTGTACGTGAAGTGCTGTCTGACAGAGAATTACATCTTTCATGGGAAGTTGGTAAACCTAGACCA
CCACTTAACCGAAATTATGTCTTTACTGGTTATCGTGTAACTAAAAACAGTAAAGTACAAATAGGAGAGTACACCTTTGAA
AAAGGTGACTATGGTGATGCTGTTGTTTACCGAGGTACAACAACTTACAAATTAAATGTTGGTGATTATTTTGTGCTGACAT
CACATACAGTAATGCCATTAAGTGCACCTACACTAGTGCCACAAGAGCACTATGTTAGAATTACTGGCTTATACCCAACACT
CAATATCTCAGATGAGTTTTCTAGCAATGTTGCAAATTATCAAAAGGTTGGTATGCAAAAGTATTCTACACTCCAGGGACCA
CCTGGTACTGGTAAGAGTCATTTTGCTATTGGCCTAGCTCTCTACTACCCTTCTGCTCGCATAGTGTATACAGCTTGCTCTCA
TGCCGCTGTTGATGCACTATGTGAGAAGGCATTAAAATATTTGCCTATAGATAAATGTAGTAGAATTATACCTGCACGTGC
TCGTGTAGAGTGTTTTGATAAATTCAAAGTGAATTCAACATTAGAACAGTATGTCTTTTGTACTGTAAATGCATTGCCTGAG
ACGACAGCAGATATAGTTGTCTTTGATGAAATTTCAATGGCCACAAATTATGATTTGAGTGTTGTCAATGCCAGATTACGT
GCTAAGCACTATGTGTACATTGGCGACCCTGCTCAATTACCTGCACCACGCACATTGCTAACTAAGGGCACACTAGAACCA
GAATATTTCAATTCAGTGTGTAGACTTATGAAAACTATAGGTCCAGACATGTTCCTCGGAACTTGTCGGCGTTGTCCTGCTG
AAATTGTTGACACTGTGAGTGCTTTGGTTTATGATAATAAGCTTAAAGCACATAAAGACAAATCAGCTCAATGCTTTAAAAT
GTTTTATAAGGGTGTTATCACGCATGATGTTTCATCTGCAATTAACAGGCCACAAATAGGCGTGGTAAGAGAATTCCTTAC
ACGTAACCCTGCTTGGAGAAAAGCTGTCTTTATTTCACCTTATAATTCACAGAATGCTGTAGCCTCAAAGATTTTGGGACTA
CCAACTCAAACTGTTGATTCATCACAGGGCTCAGAATATGACTATGTCATATTCACTCAAACCACTGAAACAGCTCACTCTT
GTAATGTAAACAGATTTAATGTTGCTATTACCAGAGCAAAAGTAGGCATACTTTGCATAATGTCTGATAGAGACCTTTATG
ACAAGTTGCAATTTACAAGTCTTGAAATTCCACGTAGGAATGTGGCAACTTTACAAGCTGAAAATGTAACAGGACTCTTTA
AAGATTGTAGTAAGGTAATCACTGGGTTACATCCTACACAGGCACCTACACACCTCAGTGTTGACACTAAATTCAAAACTG
AAGGTTTATGTGTTGACATACCTGGCATACCTAAGGACATGACCTATAGAAGACTCATCTCTATGATGGGTTTTAAAATGA
ATTATCAAGTTAATGGTTACCCTAACATGTTTATCACCCGCGAAGAAGCTATAAGACATGTACGTGCATGGATTGGCTTCG
ATGTCGAGGGGTGTCATGCTACTAGAGAAGCTGTTGGTACCAATTTACCTTTACAGCTAGGTTTTTCTACAGGTGTTAACCT
AGTTGCTGTACCTACAGGTTATGTTGATACACCTAATAATACAGATTTTTCCAGAGTTAGTGCTAAACCACCGCCTGGAGAT
CAATTTAAACACCTCATACCACTTATGTACAAAGGACTTCCTTGGAATGTAGTGCGTATAAAGATTGTACAAATGTTAAGTG
ACACACTTAAAAATCTCTCTGACAGAGTCGTATTTGTCTTATGGGCACATGGCTTTGAGTTGACATCTATGAAGTATTTTGT
GAAAATAGGACCTGAGCGCACCTGTTGTCTATGTGATAGACGTGCCACATGCTTTTCCACTGCTTCAGACACTTATGCCTGT
TGGCATCATTCTATTGGATTTGATTACGTCTATAATCCGTTTATGATTGATGTTCAACAATGGGGTTTTACAGGTAACCTACA
AAGCAACCATGATCTGTATTGTCAAGTCCATGGTAATGCACATGTAGCTAGTTGTGATGCAATCATGACTAGGTGTCTAGC
TGTCCACGAGTGCTTTGTTAAGCGTGTTGACTGGACTATTGAATATCCTATAATTGGTGATGAACTGAAGATTAATGCGGC
TTGTAGAAAGGTTCAACACATGGTTGTTAAAGCTGCATTATTAGCAGACAAATTCCCAGTTCTTCACGACATTGGTAACCC
TAAAGCTATTAAGTGTGTACCTCAAGCTGATGTAGAATGGAAGTTCTATGATGCACAGCCTTGTAGTGACAAAGCTTATAA
AATAGAAGAATTATTCTATTCTTATGCCACACATTCTGACAAATTCACAGATGGTGTATGCCTATTTTGGAATTGCAATGTC
GATAGATATCCTGCTAATTCCATTGTTTGTAGATTTGACACTAGAGTGCTATCTAACCTTAACTTGCCTGGTTGTGATGGTG
GCAGTTTGTATGTAAATAAACATGCATTCCACACACCAGCTTTTGATAAAAGTGCTTTTGTTAATTTAAAACAATTACCATTT
TTCTATTACTCTGACAGTCCATGTGAGTCTCATGGAAAACAAGTAGTGTCAGATATAGATTATGTACCACTAAAGTCTGCTA
CGTGTATAACACGTTGCAATTTAGGTGGTGCTGTCTGTAGACATCATGCTAATGAGTACAGATTGTATCTCGATGCTTATAA
CATGATGATCTCAGCTGGCTTTAGCTTGTGGGTTTACAAACAATTTGATACTTATAACCTCTGGAACACTTTTACAAGACTTC
AGAGTTTAGAAAATGTGGCTTTTAATGTTGTAAATAAGGGACACTTTGATGGACAACAGGGTGAAGTACCAGTTTCTATCA
TTAATAACACTGTTTACACAAAAGTTGATGGTGTTGATGTAGAATTGTTTGAAAATAAAACAACATTACCTGTTAATGTAGC
ATTTGAGCTTTGGGCTAAGCGCAACATTAAACCAGTACCAGAGGTGAAAATACTCAATAATTTGGGTGTGGACATTGCTGC
TAATACTGTGATCTGGGACTACAAAAGAGATGCTCCAGCACATATATCTACTATTGGTGTTTGTTCTATGACTGACATAGCC
AAGAAACCAACTGAAACGATTTGTGCACCACTCACTGTCTTTTTTGATGGTAGAGTTGATGGTCAAGTAGACTTATTTAGA
AATGCCCGTAATGGTGTTCTTATTACAGAAGGTAGTGTTAAAGGTTTACAACCATCTGTAGGTCCCAAACAAGCTAGTCTT
AATGGAGTCACATTAATTGGAGAAGCCGTAAAAACACAGTTCAATTATTATAAGAAAGTTGATGGTGTTGTCCAACAATTA
CCTGAAACTTACTTTACTCAGAGTAGAAATTTACAAGAATTTAAACCCAGGAGTCAAATGGAAATTGATTTCTTAGAATTAG
CTATGGATGAATTCATTGAACGGTATAAATTAGAAGGCTATGCCTTCGAACATATCGTTTATGGAGATTTTAGTCATAGTCA
 
GTTAGGTGGTTTACATCTACTGATTGGACTAGCTAAACGTTTTAAGGAATCACCTTTTGAATTAGAAGATTTTATTCCTATG
GACAGTACAGTTAAAAACTATTTCATAACAGATGCGCAAACAGGTTCATCTAAGTGTGTGTGTTCTGTTATTGATTTATTAC
TTGATGATTTTGTTGAAATAATAAAATCCCAAGATTTATCTGTAGTTTCTAAGGTTGTCAAAGTGACTATTGACTATACAGA
AATTTCATTTATGCTTTGGTGTAAAGATGGCCATGTAGAAACATTTTACCCAAAATTACAATCTAGTCAAGCGTGGCAACCG
GGTGTTGCTATGCCTAATCTTTACAAAATGCAAAGAATGCTATTAGAAAAGTGTGACCTTCAAAATTATGGTGATAGTGCA
ACATTACCTAAAGGCATAATGATGAATGTCGCAAAATATACTCAACTGTGTCAATATTTAAACACATTAACATTAGCTGTAC
CCTATAATATGAGAGTTATACATTTTGGTGCTGGTTCTGATAAAGGAGTTGCACCAGGTACAGCTGTTTTAAGACAGTGGT
TGCCTACGGGTACGCTGCTTGTCGATTCAGATCTTAATGACTTTGTCTCTGATGCAGATTCAACTTTGATTGGTGATTGTGC
AACTGTACATACAGCTAATAAATGGGATCTCATTATTAGTGATATGTACGACCCTAAGACTAAAAATGTTACAAAAGAAAA
TGACTCTAAAGAGGGTTTTTTCACTTACATTTGTGGGTTTATACAACAAAAGCTAGCTCTTGGAGGTTCCGTGGCTATAAAG
ATAACAGAACATTCTTGGAATGCTGATCTTTATAAGCTCATGGGACACTTCGCATGGTGGACAGCCTTTGTTACTAATGTGA
ATGCGTCATCATCTGAAGCATTTTTAATTGGATGTAATTATCTTGGCAAACCACGCGAACAAATAGATGGTTATGTCATGCA
TGCAAATTACATATTTTGGAGGAATACAAATCCAATTCAGTTGTCTTCCTATTCTTTATTTGACATGAGTAAATTTCCCCTTA
AATTAAGGGGTACTGCTGTTATGTCTTTAAAAGAAGGTCAAATCAATGATATGATTTTATCTCTTCTTAGTAAAGGTAGACT
TATAATTAGAGAAAACAACAGAGTTGTTATTTCTAGTGATGTTCTTGTTAACAACTAAACGAACAATGTTTGTTTTTCTTGT
TTATTGCCACTAGTCTCTAGTCAGTGTGTTAATCTTACAACCAGAACTCAATTACCCCCTGCATACACTAATTCTTTCACACG
TGGTGTTTATTACCCTGACAAAGTTTTCAGATCCTCAGTTTTACATTCAACTCAGGACTTGTTCTTACCTTTCTTTTCCAATGT
TACTTGGTTCCATGCTATACATGTCTCTGGGACCAATGGTACTAAGAGGTTTGCTAACCCTGTCCTACCATTTAATGATGGT
GTTTATTTTGCTTCCACTGAGAAGTCTAACATAATAAGAGGCTGGATTTTTGGTACTACTTTAGATTCGAAGACCCAGTCCC
TACTTATTGTTAATAACGCTACTAATGTTGTTATTAAAGTCTGTGAATTTCAATTTTGTAATGATCCATTTTTGGGTGTTTATT
ACCACAAAAACAACAAAAGTTGGATGGAAAGTGAGTTCAGAGTTTATTCTAGTGCGAATAATTGCACTTTTGAATATGTCT
CTCAGCCTTTTCTTATGGACCTTGAAGGAAAACAGGGTAATTTCAAAAATCTTAGGGAATTTGTGTTTAAGAATATTGATG
GTTATTTTAAAATATATTCTAAGCACACGCCTATTAATTTAGTGCGTGGTCTCCCTCAGGGTTTTTCGGCTTTAGAACCATTG
GTAGATTTGCCAATAGGTATTAACATCACTAGGTTTCAAANNNNNNCTTTACATAGAAGTTATTTGACTCCTGGTGATTCTT
CTTCAGGTTGGACAGCTGGTGCTGCAGCTTATTATGTGGGTTATCTTCAACCTAGGACTTTTCTATTAAAATATAATGAAA
ATGGAACCATTACAGATGCTGTAGACTGTGCACTTGACCCTCTCTCAGAAACAAAGTGTACGTTGAAATCCTTCACTGTAG
AAAAAGGAATCTATCAAACTTCTAACTTTAGAGTCCAACCAACAGAATCTATTGTTAGATTTCCTAATATTACAAACTTGTG
CCCTTTTGGTGAAGTTTTTAACGCCACCAGATTTGCATCTGTTTATGCTTGGAACAGGAAGAGAATCAGCAACTGTGTTGCT
GATTATTCTGTCCTATATAATTCCGCATCATTTTCCACTTTTAAGTGTTATGGAGTGTCTCCTACTAAATTAAATGATCTCTGC
TTTACTAATGTCTATGCAGATTCATTTGTAATTAGAGGTGATGAAGTCAGACAAATCGCTCCAGGGCAAACTGGAAATATT
GCTGATTATAATTATAAATTACCAGATGATTTTACAGGCTGCGTTATAGCTTGGAATTCTAACAATCTTGATTCTAAGGTTG
GTGGTAATTATAATTACCTGTATAGATTGTTTAGGAAGTCTAATCTCAAACCTTTTGAGAGAGATATTTCAACTGAAATCTA
TCAGGCCGGTAGCACACCTTGTAATGGTGTTAAAGGTTTTAATTGTTACTTTCCTTTACAATCATATGGTTTCCAACCCACTT
ATGGTGTTGGTTACCAACCATACAGAGTAGTAGTACTTTCTTTTGAACTTCTACATGCACCAGCAACTGTTTGTGGACCTAA
AAAGTCTACTAATTTGGTTAAAAACAAATGTGTCAATTTCAACTTCAATGGTTTAACAGGCACAGGTGTTCTTACTGAGTCT
AACAAAAAGTTTCTGCCTTTCCAACAATTTGGCAGAGACATTGCTGACACTACTGATGCTGTCCGTGATCCACAGACACTTG
AGATTCTTGACATTACACCATGTTCTTTTGGTGGTGTCAGTGTTATAACACCAGGAACAAATACTTCTAACCAGGTTGCTGT
TCTTTATCAGGGTGTTAACTGCACAGAAGTCCCTGTTGCTATTCATGCAGATCAACTTACTCCTACTTGGCGTGTTTATTCTA
CAGGTTCTAATGTTTTCAAACACGTGCAGGCTGTTTAATAGGGGCTGAACATGTCAACAACTCATATGAGTGTGACATAC
CCATTGGTGCAGGTATATGCGCTAGTTATCAGACTCAGACTAATTCTCCTCGGCGGGCACGTAGTGTAGCTAGTCAATCCA
TCATTGCCTACACTATGTCACTTGGTGTAGAAAATTCAGTTGCTTACTCTAATAACTCTATTGCCATACCCACAAATTTTACT
ATTAGTGTTACCACAGAAATTCTACCAGTGTCTATGACCAAGACATCAGTAGATTGTACAATGTACATTTGTGGTGATTCAA
CTGAATGCAGCAATCTTTTGTTGCAATATGGCAGTTTTTGTACACAATTAAACCGTGCTTTAACTGGAATAGCTGTTGAACA
AGACAAAAACACCCAAGAAGTTTTTGCACAAGTCAAACAAATTTACAAAACACCACCAATTAAAGATTTTGGTGGTTTTAA
TTTTTCACAAATATTACCAGATCCATCAAAACCAAGCAAGAGGTCATTTATTGAAGATCTACTTTTCAACAAAGTGACACTT
GCAGATGCTGGCTTCATCAAACAATATGGTGATTGCCTTGGTGATATTGCTGCTAGAGACCTCATTTGTGCACAAAAGTTT
AACGGCCTTACTGTTTTGCCACCTTTGCTCACAGATGAAATGATTGCTCAATACACTTCTGCACTGTTAGCGGGTACAATCA
CTTCTGGTTGGACCTTTGGTGCAGGTGCTGCATTACAAATACCATTTGCTATGCAAATGGCTTATAGGTTTAATGGTATTGG
AGTTACACAGAATGTTCTCTATGAGAACCAAAAATTGATTGCCAACCAATTTAATAGTGCTATTGGCAAAATTCAAGACTCA
CTTTCTTCCACAGCAAGTGCACTTGGAAAACTTCAAGATGTGGTCAACCAAAATGCACAAGCTTTAAACACGCTTGTTAAAC
AACTTAGCTCCAATTTTGGTGCAATTTCAAGTGTTTTAAATGATATCCTTTCACGTCTTGACAAAGTTGAGGCTGAAGTGCA
AATTGATAGGTTGATCACAGGCAGACTTCAAAGTTTGCAGACATATGTGACTCAACAATTAATTAGAGCTGCAGAAATCAG
AGCTTCTGCTAATCTTGCTGCTACTAAAATGTCAGAGTGTGTACTTGGACAATCAAAAAGAGTTGATTTTTGTGGAAAGGG
CTATCATCTTATGTCCTTCCCTCAGTCAGCACCTCATGGTGTAGTCTTCTTGCATGTGACTTATGTCCCTGCACAAGAAAAGA
ACTTCACAACTGCTCCTGCCATTTGTCATGATGGAAAAGCACACTTTCCTCGTGAAGGTGTCTTTGTTTCAAATGGCACACA
 
CTGGTTTGTAACACAAAGGAATTTTTATGAACCACAAATCATTACTACAGACAACACATTTGTGTCTGGTAACTGTGATGTT
GTAATAGGAATTGTCAACAACACAGTTTATGATCCTTTGCAACCTGAATTAGACTCATTCAAGGAGGAGTTAGATAAATAT
TTTAAGAATCATACATCACCAGATGTTGATTTAGGTGACATCTCTGGCATTAATGCTTCAGTTGTAAACATTCAAAAAGAAA
TTGACCGCCTCAATGAGGTTGCCAAGAATTTAAATGAATCTCTCATCGATCTCCAAGAACTTGGAAAGTATGAGCAGTATA
TAAAATGGCCATGGTACATTTGGCTAGGTTTTATAGCTGGCTTGATTGCCATAGTAATGGTGACAATTATGCTTTGCTGTAT
GACCAGTTGCTGTAGTTGTCTCAAGGGCTGTTGTTCTTGTGGATCCTGCTGCAAATTTGATGAAGACGACTCTGAGCCAGT
GCTCAAAGGAGTCAAATTACATTACACATAAACGAACTTATGGATTTGTTTATGAGAATCTTCACAATTGGAACTGTAACTT
TGAAGCAAGGTGAAATCAAGGATGCTACTCCTTCAGATTTTGTTCGCGCTACTGCAACGATACCGATACAAGCCTCACTCC
CTTTCGGATGGCTTATTGTTGGCGTTGCACTTCTTGCTGTTTTTCATAGCGCTTCCAAAATCATAACCCTCAAAAAGAGATG
GCAACTAGCACTCTCCAAGGGTGTTCACTTTGTTTGCAACTTGCTGTTGTTGTTTGTAACAGTTTACTCACACCTTTTGCTCG
TTGCTGCTGGCCTTGAAGCCCCTTTTCTCTATCTTTATGCTTTAGTCTACTTCTTGCAGAGTATAAACTTTGTAAGAATAATA
ATGAGGCTTTGGCTTTGCTGGAAATGCCGTTCCAAAAACCCATTACTTTATGATGCCAACTATTTTCTTTGCTGGCATACTAA
TTGTTACGACTATTGTATACCTTACAATAGTGTAACTTCTTCAATTGTCATTACTTTAGGTGATGGCACAACAAGTCCTATTT
CTGAACATGACTACCAGATTGGTGGTTATACTGAAAAATGGGAATCTGGAGTAAAAGACTGTGTTGTATTACACAGTTACT
TCACTTCAGACTATTACCAGCTGTACTCAACTCAATTGAGTACAGACACTGGTGTTGAACATGTTACCTTCTTCATCTACAAT
AAAATTGTTGATGAGCCTGAAGAACATGTCCAAATTCACACAATCGACGGTTCATCCGGAGTTGTTAATCCAGTAATGGAA
CCAATTTATGATGAACCGACGACGACTACTAGCGTGCCTTTGTAAGCACAAGCTGATGAGTACGAACTTATGTACTCATTC
GTTTCGGAAGAGACAGGTACGTTAATAGTTAATAGCGTACTTCTTTTTCTTGCTTTCGTGGTATTCTTGCTAGTTACACTAGC
CATCCTTACTGCGCTTCGATTGTGTGCGTACTGCTGCAATATTGTTAACGTGAGTCTTGTAAAACCTTCTTTTTACGTTTACT
CTCGTGTTAAAAATCTGAATTCTTCTAGAGTTCTTGATCTTCTGGTCTAAACGAACTAAATATTATATTAGTTTTTCTGTTTGG
AACTTTAATTTTAGCCATGGCAGATTCCAACGGTACTATTACCGTTGAAGAGCTTAAAAAGCTCCTTGAACAATGGAACCTA
GTAATAGGTTTCCTATTCCTTACATGGATTTGTCTTCTACAATTTGCCTATGCCAACAGGAATAGGTTTTTGTATATAATTAA
GTTAATTTTCCTCTGGCTGTTATGGCCAGTAACTTTAGCTTGTTTTGTGCTTGCTGCTGTTTACAGAATAAATTGGATCACCG
GTGGAATTGCTATCGCAATGGCTTGTCTTGTAGGCTTGATGTGGCTCAGCTACTTCATTGCTTCTTTCAGACTGTTTGCGCG
TACGCGTTCCATGTGGTCATTCAATCCAGAAACTAACATTCTTCTCAACGTGCCACTCCATGGCACTATTCTGACCAGACCG
CTTCTAGAAAGTGAACTCGTAATCGGAGCTGTGATCCTTCGTGGACATCTTCGTATTGCTGGACACCATCTAGGACGCTGT
GACATCAAGGACCTGCCTAAAGAAATCACTGTTGCTACATCACGAACGCTTTCTTATTACAAATTGGGAGCTTCGCAGCGT
GTAGCAGGTGACTCAGGTTTTGCTGCATACAGTCGCTACAGGATTGGCAACTATAAATTAAACACAGACCATTCCAGTAGC
AGTGACAATATTGCTTTGCTTGTACAGTAAGCGACAACAGATGTTTCATCTCGTTGACTTTCAGGTTACTATAGCAGAGATA
TTACTAATTATTATGAGGACTTTTAAAGTTTCCATTTGGAATCTTGATTACATCATAAACCTCATAATTAAAAATTTATCTAA
GTCACTAACTGAGAATAAATATTCTCAATTAGATGAAGAGCAACCAATGGAGATTGATTAAACGAACATGAAAATTATTCT
TTTCTTGGCACTGATAACACTCGCTACTTGTGAGCTTTATCACTACCAAGAGTGTGTTAGAGGTACAACAGTACTTTTAAAA
GAACCTTGCTCTTCTGGAACATACGAGGGCAATTCACCATTTCATCCTCTAGCTGATAACAAATTTGCACTGACTTGCTTTA
GCACTCAATTTGCTTTTGCTTGTCCTGACGGCGTAAAACACGTCTATCAGTTACGTGCCAGATCAGTTTCACCTAAACTGTTC
ATCAGACAAGAGGAAGTTCAAGAACTTTACTCTCCAATTTTTCTTATTGTTGCGGCAATAGTGTTTATAACACTTTGCTTCAC
ACTCAAAAGAAAGACAGAATGATTGAACTTTCATTAATTGACTTCTATTTGTGCTTTTTAGCCTTTCTGCTATTCCTTGTTTTA
ATTATGCTTATTATCTTTTGGTTCTCACTTGAACTGCAAGATCATAATGAAACTTGTCACGCCTAAACGAACATGAAATTTCT
TGTTTTCTTAGGAATCATCACAACTGTAGCTGCATTTCACCAAGAATGTAGTTTACAGTCATGTACTCAACATCAACCATAT
GTAGTTGATGACCCGTGTCCTATTCACTTCTATTCTAAATGGTATATTAGAGTAGGAGCTAGAAAATCAGCACCTTTAATTG
AATTGTGCGTGGATGAGGCTGGTTCTAAATCACCCATTCAGTACATCGATATCGGTAATTATACAGTTTCCTGTTTACCTTTT
ACAATTAATTGCCAGGAACCTAAATTGGGTAGTCTTGTAGTGCGTTGTTCGTTCTATGAAGACTTTTTAGAGTATCATGACG
TTCGTGTTGTTTTAGATTTTATCTAAACGAACAAACTAAAATGTCTGATAATGGACCCCAAAATCAGCGAAATGCACCCCGC
ATTACGTTTGGTGGACCCTCAGATTCAACTGGCAGTAACCAGAATGGAGAACGCAGTGGGGCGCGATCAAAACAACGTCG
GCCCCAAGGTTTACCCAATAATACTGCGTCTTGGTTCACCGCTCTCACTCAACATGGCAAGGAAGACCTTAAATTCCCTCGA
GGACAAGGCGTTCCAATTAACACCAATAGCAGTCCAGATGACCAAATTGGCTACTACCGAAGAGCTACCAGACGAATTCG
TGGTGGTGACGGTAAAATGAAAGATCTCAGTCCAAGATGGTATTTCTACTACCTAGGAACTGGGCCAGAAGCTGGACTT
CCCTATGGTGCTAACAAAGACGGCATCATATGGGTTGCAACTGAGGGAGCCTTGAATACACCAAAAGATCACATTGGCAC
CCGCAATCCTGCTAACAATGCTGCAATCGTGCTACAACTTCCTCAAGGAACAACATTGCCAAAAGGCTTCTACGCAGAAGG
GAGCAGAGGCGGCAGTCAAGCCTCTTCTCGTTCCTCATCACGTAGTCGCAACAGTTCAAGAAATTCAACTCCAGGCAGCAG
TAGGGGAATTTCTCCTGCTAGAATGGCTGGCAATGGCGGTGATGCTGCTCTTGCTTTGCTGCTGCTTGACAGATTGAACCA
GCTTGAGAGCAAAATGTCTGGTAAAGGCCAACAACAACAAGGCCAAACTGTCACTAAGAAATCTGCTGCTGAGGCTTCTA
AGAAGCCTCGGCAAAAACGTACTGCCACTAAAGCATACAATGTAACACAAGCTTTCGGCAGACGTGGTCCAGAACAAACC
CAAGGAAATTTTGGGGACCAGGAACTAATCAGACAAGGAACTGATTACAAACATTGGCCGCAAATTGCACAATTTGCCCC
CAGCGCTTCAGCGTTCTTCGGAATGTCGCGCATTGGCATGGAAGTCACACCTTCGGGAACGTGGTTGACCTACACAGGTG
CCATCAAATTGGATGACAAAGATCCAAATTTCAAAGATCAAGTCATTTTGCTGAATAAGCATATTGACGCATACAAAACATT
 
CCCACCAACAGAGCCTAAAAGGACAAAAGAAGAAGGCTGATGAAACTCAAGCCTTACCGCAGAGACAGAAGAAACAG
CAAACTGTGACTCTTCTTCCTGCTGCAGATTTGGATGATTTCTCCAAACAATTGCAACAATCCATGAGCAGTGCTGACTCAA
CTCAGGCCTAAACTCATGCAGACCACACAAGGCAGATGGGCTATATAAACGTTTTCGCTTTTCCGTTTACGATATATAGTCT
ACTCTTGTGCAGAATGAATTCTCGTAACTACATAGCACAAGTAGATGTAGTTAACTTTAATCTCACATAGCAATCTTTAATC
AGTGTGTAACATTAGGGAGGACTTGAAAGAGCCACCACATTTTCACCGAGGCCACGCGGAGTACGATCGAGTGTACAGTG
AACAATGCTAGGGAGAGCTGCCTATATGGAAGAGCCCTAATGTGTAAAATTAATTTTAGTAGTGCTATCCCCATGNNNNN
NNNNNNNNNNNNNNNNNNNNNNNAAAAAAAAAAAAAAAAAAAAAAAAAAAAAAAAAAA
SEQ ID NO: 19
   >SA_P2_gp02 surface glycoprotein, from genome accession SA_P2_t0.9_q20 
MFVFLVLLPLVSSQCVNLTTRTQLPPAYTNSFTRGVYYPDKVFRSSVLHSTQDLFLPFFSNVTWFHAIHVSGTNGTKRFANPVLPF
NDGVYFASTEKSNIIRGWIFGTTLDSKTQSLLIVNNATNVVIKVCEFQFCNDPFLGVYYHKNNKSWMESEFRVYSSANNCTFEYV
SQPFLMDLEGKQGNFKNLREFVFKNIDGYFKIYSKHTPINLVRGLPQGFSALEPLVDLPIGINITRFQXXXLHRSYLTPGDSSSGWT
AGAAAYYVGYLQPRTFLLKYNENGTITDAVDCALDPLSETKCTLKSFTVEKGIYQTSNFRVQPTESIVRFPNITNLCPFGEVFNATR
FASVYAWNRKRISNCVADYSVLYNSASFSTFKCYGVSPTKLNDLCFTNVYADSFVIRGDEVRQIAPGQTGNIADYNYKLPDDFTG
CVIAWNSNNLDSKVGGNYNYLYRLFRKSNLKPFERDISTEIYQAGSTPCNGVKGFNCYFPLQSYGFQPTYGVGYQPYRVVVLSFE
LLHAPATVCGPKKSTNLVKNKCVNFNFNGLTGTGVLTESNKKFLPFQQFGRDIADTTDAVRDPQTLEILDITPCSFGGVSVITPGT
NTSNQVAVLYQGVNCTEVPVAIHADQLTPTWRVYSTGSNVFQTRAGCLIGAEHVNNSYECDIPIGAGICASYQTQTNSPRRARS
VASQSIIAYTMSLGVENSVAYSNNSIAIPTNFTISVTTEILPVSMTKTSVDCTMYICGDSTECSNLLLQYGSFCTQLNRALTGIAVEQ
DKNTQEVFAQVKQIYKTPPIKDFGGFNFSQILPDPSKPSKRSFIEDLLFNKVTLADAGFIKQYGDCLGDIAARDLICAQKFNGLTVL
PPLLTDEMIAQYTSALLAGTITSGWTFGAGAALQIPFAMQMAYRFNGIGVTQNVLYENQKLIANQFNSAIGKIQDSLSSTASALG
KLQDVVNQNAQALNTLVKQLSSNFGAISSVLNDILSRLDKVEAEVQIDRLITGRLQSLQTYVTQQLIRAAEIRASANLAATKMSEC
VLGQSKRVDFCGKGYHLMSFPQSAPHGVVFLHVTYVPAQEKNFTTAPAICHDGKAHFPREGVFVSNGTHWFVTQRNFYEPQII
TTDNTFVSGNCDVVIGIVNNTVYDPLQPELDSFKEELDKYFKNHTSPDVDLGDISGINASVVNIQKEIDRLNEVAKNLNESLIDLQE
LGKYEQYIKWPWYIWLGFIAGLIAIVMVTIMLCCMTSCCSCLKGCCSCGSCCKFDEDDSEPVLKGVKLHYT
SEQ ID NO: 20
   >MW52O923.1 Severe acute respiratory syndrome coronavirus 2 isolate SARS-CoV-2/human/USA/MN-MDH-2399/2021, complete genome, example fo Brazilian PI lineage. 
CAACTTTCGATCTCTTGTAGATCTGTTCTCTAAACGAACTTTAAAATCTGTGTGGCTGTCACTCGGCTGCATGCTTAGTG
CACTCACGCAGTATAATTAATAACTAATTACTGTCGTTGACAGGACACGAGTAACTCGTCTATCTTCTGCAGGCTGCTTA
CGGTTTCGTCCGTGTTGCAGCCGATCATCAGCACATCTAGGTTTTGTCCGGGTGTGACCGAAAGGTAAGATGGAGAGCCT
TGTCCCTGGTTTCAACGAGAAAACACACGTCCAACTCAGTTTGCCTGTTTTACAGGTTCGCGACGTGCTCGTACGTGGCT
TTGGAGACTCCGTGGAGGAGGTCTTATCAGAGGCACGTCAACATCTTAAAGATGGCACTTGTGGCTTAGTAGAAGTTGAA
AAAGGCGTTTTGCCTCAACTTGAACAGCCCTATGTGTTCATCAAACGTTCGGATGCTCGAACTGCACCTCATGGTCATGT
TATGGTTGAGCTGGTAGCAGAACTCGAAGGCATTCAGTACGGTCGTAGTGGTGAGACACTTGGTGTCCTTGTCCCTCATG
TGGGCGAAATACCAGTGGCTTACCGCAAGGTTCTTCTTCGTAAGAACGGTAATAAAGGAGCTGGTGGCCATAGTTACGGC
GCCGATCTAAAGTCATTTGACTTAGGCGACGAGCTTGGCACTGATCCTTATGAAGACTTTCAAGAAAACTGGAACACTAA
ACATAGCAGTGGTGTTACCCGTGAACTCATGCGTGAGCTTAACGGAGGGGCATACACTCGCTATGTCGATAACAACTTCT
GTGGCCCTGATGGCTACCCTCTTGAGTGCATTAAAGACCTTCTAGCACGTGCTGGTAAAGCTTCATGCACTTTGTCCGAA
CAACTGGACTTTATTGACACTAAGAGGGGTGTATACTGCTGCCGTGAACATGAGCATGAAATTGCTTGGTACACGGAACG
TTCTGAAAAGAGCTATGAATTGCAGACACCTTTTGAAATTAAATTGGCAAAGAAATTTGACACCTTCAATGGGGAATGTC
CAAATTTTGTATTTCCCTTAAATTCCATAATCAAGACTATTCAACCAAGGGTTGAAAAGAAAAAGCTTGATGGCTTTATG
GGTAGAATTCGATCTGTCTATCCAGTTGCGTCACCAAATGAATGCAACCAAATGTGCCTTTCAACTCTCATGAAGTGTGA
TCATTGTGGTGAAACTTCATGGCAGACGGGCGATTTTGTTAAAGCCACTTGCGAATTTTGTGGCACTGAGAATTTGACTA
AAGAAGGTGCCACTACTTGTGGTTACTTACCCCAAAATGCTGTTGTTAAAATTTATTGTCCAGCATGTCACAATTCAGAA
GTAGGACCTGAGCATAGTCTTGCCGAATACCATAATGAATCTGGCTTGAAAACCATTCTTCGTAAGGGTGGTCGCACTAT
TGCCTTTGGAGGCTGTGTGTTCTCTTATGTTGGTTGCCATAACAAGTGTGCCTATTGGGTTCCACGTGCTAGCGCTAACA
TAGGTTGTAACCATACAGGTGTTGTTGGAGAAGGTTCCGAAGGTCTTAATGACAACCTTCTTGAAATACTCCAAAAAGAG
AAAGTCAACATCAATATTGTTGGTGACTTTAAACTTAATGAAGAGATCGCCATTATTTTGGCATCTTTTTCTGCTTCCAC
AAGTGCTTTTGTGGAAACTGTGAAAGGTTTGGATTATAAAGCATTCAAACAAATTGTTGAATCCTGTGGTAATTTTAAAG
TTACAAAAGGAAAAGCTAAAAAAGGTGCCTGGAATATTGGTGAACAGAAATCAATACTGAGTCCTCTTTATGCATTTGCA
TCAGAGGCTGCTCGTGTTGTACGATCAATTTTCTCCCGCACTCTTGAAACTGCTCAAAATTCTGTGCGTGTTTTACAGAA
GGCCGCTATAACAATACTAGATGGAATTTCACAGTATTCACTGAGACTCATTGATGCTATGATGTTCACATCTGATTTGG
CTACTAACAATCTAGTTGTAATGGCCTACATTACAGGTGGTGTTGTTCAGTTGACTTCGCAGTGGCTAACTAACATCTTT
 
GGCACTGTTTATGAAAAACTCAAACCCGTCCTTGATTGGCTTGAAGAGAAGTTTAAGGAAGGTGTAGAGTTTCTTAGAGA
CGGTTGGGAAATTGTTAAATTTATCTCAACCTGTGCTTGTGAAATTGTCGGTGGACAAATTGTCACCTGTGCAAAGGAAA
TTAAGGAGAGTGTTCAGACATTCTTTAAGCTTGTAAATAAATTTTTGGCTTTGTGTGCTGACTCTATCATTATTGGTGGA
GCTAAACTTAAAGCCTTGAATTTAGGTGAAACATTTGTCACGCACTCAAAGGGATTGTACAGAAAGTGTGTTAAATCCAG
AGAAGAAACTGGCCTACTCATGCCTCTAAAAGCCCCAAAAGAAATTATCTTCTTAGAGGGAGAAACACTTCCCACAGAAG
TGTTAACAGAGGAAGTTGTCTTGAAAACTGGTGATTTACAACCATTAGAACAACCTACTAGTGAAGCTGTTGAAGCTCCA
TTGGTTGGTACACCAGTTTGTATTAACGGGCTTATGTTGCTCGAAATCAAAGACACAGAAAAGTACTGTGCCCTTGCACC
TAATATGATGGTAACAAACAATACCTTCACACTCAAAGGCGGTGCACCAACAAAGGTTACTTTTGGTGATGATACTGTGA
TAGAAGTGCAAGGTTACAAGAGTGTGAATATCACTTTTGAACTTGATGAAAGGATTGATAAAGTACTTAATGAGAAGTGC
TCTGCCTATACAGTTGAACTCGGTACAGAAGTAAATGAGTTCGCCTGTGTTGTGGCAGATGCTGTCATAAAAACTTTGCA
ACCAGTATCTGAATTACTTACACCACTGGGCATTGATTTAGATGAGTGGAGTATGGCTACATACTACTTATTTGATGAGT
CTGGTGAGTTTAAATTGGCTTCACATATGTATTGTTCTTTTTACCCTCCAGATGAGGATGAAGAAGAAGGTGATTGTGAA
GAAGAAGAGTTTGAGCCATCAACTCAATATGAGTATGGTACTGAAGATGATTACCAAGGTAAACCTTTGGAATTTGGTGC
CACTTCTGCTGCTCTTCAACCTGAAGAAGAGCAAGAAGAAGATTGGTTAGATGATGATAGTCAACAAACTGTTGGTCAAC
AAGACGGCAGTGAGGACAATCAGACAACTACTATTCAAACAATTGTTGAGGTTCAACCTCAATTAGAGATGGAACTTACA
CCAGTTGTTCAGACTATTGAAGTGAATAGTTTTAGTGGTTATTTAAAACTTACTGACAATGTATACATTAAAAATGCAGA
CATTGTGGAAGAAGCTAAAAAGGTAAAACCAACAGTGGTTGTTAATGCAGCCAATGTTTACCTTAAACATGGAGGAGGTG
TTGCAGGAGCCTTAAATAAGGCTACTAACAATGCCATGCAAGTTGAATCTGATGATTACATAGCTACTAATGGACCACTT
AAAGTGGGTGGTAGTTGTGTTTTAAGCGGACACAATCTTGCTAAACACTGTCTTCATGTTGTCGGCCCAAATGTTAACAA
AGGTGAAGACATTCAACTTCTTAAGAGTGCTTATGAAAATTTTAATCAGCACGAAGTTCTACTTGCACCATTATTATCAG
CTGGTATTTTTGGTGCTGACCCTATACATTCTTTAAGAGTTTGTGTAGATACTGTTCGCACAAATGTCTACTTAGCTGTC
TTTGATAAAAATCTCTATGACAAACTTGTTTTAAGCTTTTTGGAAATGAAGAGTGAAAAGCAAGTTGAACAAAAGATCGC
TGAGATTCCTAAAGAGGAAGTTAAGCCATTTATAACTGAAAGTAAACCTTCAGTTGAACAGAGAAAACAAGATGATAAGA
AAATCAAAGCTTGTGTTGAAGAAGTTACAACAACTCTGGAAGAAACTAAGTTCCTCACAGAAAACTTGTTACTTTATATT
GACATTAATGGCAATCTTCATCCAGATTCTGCCACTCTTGTTAGTGACATTGACATCACTTTCTTAAAGAAAGATGCTCC
ATATATAGTGGGTGATGTTGTTCAAGAGGGTGTTTTAACTGCTGTGGTTATACCTACTAAAAAGGCTGGTGGCACTACTG
AAATGCTAGCGAAAGCTTTGAGAAAAGTGCCAACAGACAATTATATAACCACTTACCCGGGTCAGGGTTTAAATGGTTAC
ACTGTAGAGGAGGCAAAGACAGTGCTTAAAAAGTGTAAAAGTGCCTTTTACATTCTACCATCTATTATCTCTAATGAGAA
GCAAGAAATTCTTGGAACTGTTTCTTGGAATTTGCGAGAAATGCTTGCACATGCAGAAGAAACACGCAAATTAATGCCTG
TCTGTGTGGAAACTAAAGCCATAGTTTCAACTATACAGCGTAAATATAAGGGTATTAAAATACAAGAGGGTGTGGTTGAT
TATGGTGCTAGATTTTACTTTTACACCAGTAAAACAACTGTAGCGTCACTTATCAACACACTTAACGATCTAAATGAAAC
TCTTGTTACAATGCCACTTGGCTATGTAACACATGGCTTAAATTTGGAAGAAGCTGCTCGGTATATGAGATCTCTCAAAG
TGCCAGCTACAGTTTCTGTTTCTTCACCTGATGCTGTTACAGCGTATAATGGTTATCTTACTTCTTCTTCTAAAACACCT
GAAGAACATTTTATTGAAACCATCTCACTTGCTGGTTCCTATAAAGATTGGTCCTATTCTGGACAATCTACACAACTAGG
TATAGAATTTCTTAAGAGAGGTGATAAAAGTGTATATTACACTAGTAATCCTACCACATTCCACCTAGATGGTGAAGTTA
TCACCTTTGACAATCTTAAGACACTTCTTTCTTTGAGAGAAGTGAGGACTATTAAGGTGTTTACAACAGTAGACAACATT
AACCTCCACACGCAAGTTGTGGACATGTCAATGACATATGGACAACAGTTTGGTCCAACTTATTTGGATGGAGCTGATGT
TACTAAAATAAAACCTCATAATTCACATGAAGGTAAAACATTTTATGTTTTACCTAATGATGACACTCTACGTGTTGAGG
CTTTTGAGTACTACCACACAACTGATCCTAGTTTTCTGGGTAGGTACATGTCAGCATTAAATCACACTAAAAAGTGGAAA
TACCCACAAGTTAATGGTTTAACTTCTATTAAATGGGCAGATAACAACTGTTATCTTGCCACTGCATTGTTAACACTCCA
ACAAATAGAGTTGAAGTTTAATCCACCTGCTCTACAAGATGCTTATTACAGAGCAAGGGCTGGTGAAGCTGCTAACTTTT
GTGCACTTATCTTAGCCTACTGTAATAAGACAGTAGGTGAGTTAGGTGATGTTAGAGAAACAATGAGTTACTTGTTTCAA
CATGCCAATTTAGATTCTTGCAAAAGAGTCTTGAACGTGGTGTGTAAAACTTGTGGACAACAGCAGACAACCCTTAAGGG
TGTAGAAGCTGTTATGTACATGGGCACACTTTCTTATGAACAATTTAAGAAAGGTGTTCAGATACCTTGTACGTGTGGTA
AACAAGCTACACAATATCTAGTACAACAGGAGTCACCTTTTGTTATGATGTCAGCACCACCTGCTCAGTATGAACTTAAG
CATGGTACATTTACTTGTGCTAGTGAGTACACTGGTAATTACCAGTGTGGTCACTATAAACATATAACTTCTAAAGAAAC
TTTGTATTGCATAGACGGTGCTTTACTTACAAAGTCCTCAGAATACAAAGGTCCTATTACGGATGTTTTCTACAAAGAAA
ACAGTTACACAACAACCATAAAACCAGTTACTTATAAATTGGATGGTGTTGTTTGTACAGAAATTGACCCTAAGTTGGAC
AATTATTATAAGAAAGACAATTCTTATTTCACAGAGCAACCAATTGATCTTGTACCAAACCAACCATATCCAAACGCAAG
CTTCGATAATTTTAAGTTTGTATGTGATAATATCAAATTTGCTGATGATTTAAACCAGTTAACTGGTTATAAGAAACCTG
CTTCAAGAGAGCTTAAAGTTACATTTTTCCCTGACTTAAATGGTGATGTGGTGGCTATTGATTATAAACACTACACACCC
TCTTTTAAGAAAGGAGCTAAATTGTTACATAAACCTATTGTTTGGCATGTTAACAATGCAACTAATAAAGCCACGTATAA
ACCAAATACCTGGTGTATACGTTGTCTTTGGAGCACAAAACCGGTTGAAACATCAAATTCGTTTGATGTACTGAAGTCAG
AGGACGCGCAGGGAATGGATAATCTTGCCTGCGAAGATCTAAAACCAGTCTCTGAAGAAGTAGTGGAAAATCCTACCATA
CAGAAAGACGTTCTTGAGTGTAATGTGAAAACTACCGAAGTTGTAGGAGACATTATACTTAAACCAGCAAATAATAGTTT
 
AAAAATTACAGAAGAGGTTGGCCACACAGATCTAATGGCTGCTTATGTAGACAATTCTAGTCTTACTATTAAGAAACCTA
ATGAATTATCTAGAGTGTTAGGTTTGAAAACCCTTGCTACTCATGGTTTAGCTGCTGTTAATAGTGTCCCTTGGGATACT
ATAGCTAATTATGCTAAGCCTTTTCTTAACAAAGTTGTTAGTACAACTACTAACATAGTTACACGGTGTTTAAACCGTGT
TTGTACTAATTATATGCCTTATTTCTTTACTTTATTGCTACAATTGTGTACTTTTACTAGAAGTACAAATTCTAGAATTA
AAGCATCTATGCCGACTACTATAGCAAAGAATACTGTTAAGAGTGTCGGTAAATTTTGTCTAGAGGCTTCATTTAATTAT
TTGAAGTCACCTAATTTTTCTAAACTGATAAATATTATAATTTGGTTTTTACTATTAAGTGTTTGCCTAGGTTCTTTAAT
CTACTCAACCGCTGCTTTAGGTGTTTTAATGTCTAATTTAGGCATGCCTTCTTACTGTACTGGTTACAGAGAAGGCTATT
TGAACTCTACTAATGTCACTATTGCAACCTACTGTACTGGTTCTATACCTTGTAGTGTTTGTCTTAGTGGTTTAGATTCT
TTAGACACCTATCCTTCTTTAGAAACTATACAAATTACCATTTCATCTTTTAAATGGGATTTAACTGCTTTTGGCTTAGT
TGCAGAGTGGTTTTTGGCATATATTCTTTTCACTAGGTTTTTCTATGTACTTGGATTGGCTGCAATCATGCAATTGTTTT
TCAGCTATTTTGCAGTACATTTTATTAGTAATTCTTGGCTTATGTGGTTAATAATTAATCTTGTACAAATGGCCCCGATT
TCAGCTATGGTTAGAATGTACATCTTCTTTGCATCATTTTATTATGTATGGAAAAGTTATGTGCATGTTGTAGACGGTTG
TAATTCATCAACTTGTATGATGTGTTACAAACGTAATAGAGCAACAAGAGTCGAATGTACAACTATTGTTAATGGTGTTA
GAAGGTCCTTTTATGTCTATGCTAATGGAGGTAAAGGCTTTTGCAAACTACACAATTGGAATTGTGTTAATTGTGATACA
TTCTGTGCTGGTAGTACATTTATTAGTGATGAAGTTGCGAGAGACTTGTCACTACAGTTTAAAAGACCAATAAATCCTAC
TGACCAGTCTTCTTACATCGTTGATAGTGTTACAGTGAAGAATGGTTCCATCCATCTTTACTTTGATAAAGCTGGTCAAA
AGACTTATGAAAGACATTCTCTCTCTCATTTTGTTAACTTAGACAACCTGAGAGCTAATAACACTAAAGGTTCATTGCCT
ATTAATGTTATAGTTTTTGATGGTAAATCAAAATGTGAAGAATCATCTGCAAAATCAGCGTCTGTTTACTACAGTCAGCT
TATGTGTCAACCTATACTGTTACTAGATCAGGCATTAGTGTCTGATGTTGGTGATAGTGCGGAAGTTGCAGTTAAAATGT
TTGATGCTTACGTTAATACGTTTTCATCAACTTTTAACGTACCAATGGAAAAACTCAAAACACTAGTTGCAACTGCAGAA
GCTGAACTTGCAAAGAATGTGTCCTTAGACAATGTCTTATCTACTTTTATTTCAGCAGCTCGGCAAGGGTTTGTTGATTC
AGATGTAGAAACTAAAGATGTTGTTGAATGTCTTAAATTGTCACATCAATCTGACATAGAAGTTACTGGCGATAGTTGTA
ATAACTATATGCTCACCTATAACAAAGTTGAAAACATGACACCCCGTGACCTTGGTGCTTGTATTGACTGTAGTGCGCGT
CATATTAATGCGCAGGTAGCAAAAAGTCACAACATTGCTTTGATATGGAACGTTAAAGATTTCATGTCATTGTCTGAACA
ACTACGAAAACAAATACGTAGTGCTGCTAAAAAGAATAACTTACCTTTTAAGTTGACATGTGCAACTACTAGACAAGTTG
TTAATGTTGTAACAACAAAGATAGCACTTAAGGGTGGTAAAATTGTTAATAATTGGTTGAAGCAGTTAATTAAAGTTACA
CTTGTGTTCCTTTTTGTTGCTGCTATTTTCTATTTAATAACACCTGTTCATGTCATGTCTAAACATACTGACTTTTCAAG
TGAAATCATAGGATACAAGGCTATTGATGGTGGTGTCACTCGTGACATAGCATCTACAGATACTTGTTTTGCTAACAAAC
ATGCTGATTTTGACACATGGTTTAGCCAGCGTGGTGGTAGTTATACTAATGACAAAGCTTGCCCATTGATTGCTGCAGTC
ATAACAAGAGAAGTGGGTTTTGTCGTGCCTGGTTTGCCTGGCACGATATTACGCACAACTAATGGTGACTTTTTGCATTT
CTTACCTAGAGTTTTTAGTGCAGTTGGTAACATCTGTTACACACCATCAAAACTTATAGAGTACACTGACTTTGCAACAT
CAGCTTGTGTTTTGGCTGCTGAATGTACAATTTTTAAAGATGCTTCTGGTAAGCCAGTACCATATTGTTATGATACCAAT
GTACTAGAAGGTTCTGTTGCTTATGAAAATTTACGCCCTGACACACGTTATGTGCTCATGGATGGCTCTATTATTCAATT
TCCTAACACCTACCTTGAAGGTTCTGTTAGAGTGGTAACAACTTTTGATTCTGAGTACTGTAGGCACGGCACTTGTGAAA
GATCAGAAGCTGGTGTTTGTGTATCTACTAGTGGTAGATGGGTACTTAACAATGATTATTACAGATCTTTACCAGGAGTT
TTCTGTGGTGTAGATGCTGTAAATTTACTTACTAATATGTTTACACCACTAATTCAACCTATTGGTGCTTTGGACATATC
AGCATCTATAGTAGCTGGTGGTATTGTAGCTATCGTAGTAACATGCCTTGCCTACTATTTTATGAGGTTTAGAAGAGCTT
TTGGTGAATACAGTCATGTAGTTGCCTTTAATACTTTACTATTCCTTATGTCATTCACTGTACTCTGTTTAACACCAGTT
TACTCATTCTTACCTGGTGTTTATTCTGTTATTTACTTGTACTTGACATTTTATCTTACTAATGATGTTTCTTTTTTAGC
ACATATTCAGTGGATGGTTATGTTCACACCTTTAGTACCTTTCTGGATAACAATTGCTTATATCATTTGTATTTCCACAA
AGCATTTCTATTGGTTCTTTAGTAATTACCTAAAGAGACGTGTAGTCTTTAATGGTGTTTCCTTTAGTACTTTTGAAGAA
GCTGCGCTGTGCACCTTTTTGTTAAATAAAGAAATGTATCTAAAGTTGCGTAGTGATGTGCTATTACCTCTTACGCAATA
TAATAGATACTTAGCTCTTTATAATAAGTACAAGTATTTTAGTGGAGCAATGGATACAACTAGCTACAGAGAAGCTGCTT
GTTGTCATCTCGCAAAGGCTCTCAATGACTTCAGTAACTCAGGTTCTGATGTTCTTTACCAACCACCACAAACCTCTATC
ACCTCAGCTGTTTTGCAGAGTGGTTTTAGAAAAATGGCATTCCCATCTGGTAAAGTTGAGGGTTGTATGGTACAAGTAAC
TTGTGGTACAACTACACTTAACGGTCTTTGGCTTGATGACGTAGTTTACTGTCCAAGACATGTGATCTGCACCTCTGAAG
ACATGCTTAACCCTAATTATGAAGATTTACTCATTCGTAAGTCTAATCATAATTTCTTGGTACAGGCTGGTAATGTTCAA
CTCAGGGTTATTGGACATTCTATGCAAAATTGTGTACTTAAGCTTAAGGTTGATACAGCCAATCCTAAGACACCTAAGTA
TAAGTTTGTTCGCATTCAACCAGGACAGACTTTTTCAGTGTTAGCTTGTTACAATGGTTCACCATCTGGTGTTTACCAAT
GTGCTATGAGGCCCAATTTCACTATTAAGGGTTCATTCCTTAATGGTTCATGTGGTAGTGTTGGTTTTAACATAGATTAT
GACTGTGTCTCTTTTTGTTACATGCACCATATGGAATTACCAACTGGAGTTCATGCTGGCACAGACTTAGAAGGTAACTT
TTATGGACCTTTTGTTGACAGGCAAACAGCACAAGCAGCTGGTACGGACACAACTATTACAGTTAATGTTTTAGCTTGGT
TGTACGCTGCTGTTATAAATGGAGACAGGTGGTTTCTCAATCGATTTACCACAACTCTTAATGACTTTAACCTTGTGGCT
ATGAAGTACAATTATGAACCTCTAACACAAGACCATGTTGACATACTAGGACCTCTTTCTGCTCAAACTGGAATTGTCGT
TTTAGATATGTGTGCTTCATTAAAAGAATTACTGCAAAATGGTATGAATGGACGTACCATATTGGGTAGTGCTTTATTAG
 
AAGATGAATTTACACCTTTTGATGTTGTTAGACAATGCTCAGGTGTTACTTTCCAAAGTGCAGTGAAAAGAACAATCAAG
GGTACACACCACTGGTTGTTACTCACAATTTTGACTTCACTTTTAGTTTTAGTCCAGAGTACTCAATGGTCTTTGTTCTT
TTTTTTGTATGAAAATGCCTTTTTACCTTTTGCTATGGGTATTATTGCTATGTCTGCTTTTGCAATGATGTTTGTCAAAC
ATAAGCATGCATTTCTCTGTTTGTTTTTGTTACCTTCTCTTGCCACTGTAGCTTATTTTAATATGGTCTATATGCCTGCT
AGTTGGGTGATGCGTATTATGACATGGTTGGATATGGTTGATACTAGTTTGAAGCTAAAAGACTGTGTTATGTATGCATC
AGCTGTAGTGTTACTAATCCTTATGACAGCAAGAACTGTGTATGATGATGGTGCTAGGAGAGTGTGGACACTTATGAATG
TCTTGACACTCGTTTATAAAGTTTATTATGGTAATGCTTTAGATCAAGCCATTTCCATGTGGGCTCTTATAATCTCTGTT
ACTTCTAACTACTCAGGTGTAGTTACAACTGTCATGTTTTTGGCCAGAGGTATTGTTTTTATGTGTGTTGAGTATTGCCC
TATTTTCTTCATAACTGGTAATACACTTCAGTGTATAATGCTAGTTTATTGTTTCTTAGGCTATTTTTGTACTTGTTACT
TTGGCCTCTTTTGTTTACTCAACCGCTACTTTAGACTGACTCTTGGTGTTTATGATTACTTAGTTTCTACACAGGAGTTT
AGATATATGAATTCACAGGGACTACTCCCACCCAAGAATAGCATAGATGCCTTCAAACTCAACATTAAATTGTTGGGTGT
TGGTGGCAAACCTTGTATCAAAGTAGCCACTGTACAGTCTAAAATGTCAGATGTAAAGTGCACATCAGTAGTCTTACTCT
CAGTTTTGCAACAACTCAGAGTAGAATCATCATCTAAATTGTGGGCTCAATGTGTCCAGTTACACAATGACATTCTCTTA
GCTAAAGATACTACTGAAGCCTTTGAAAAAATGGTTTCACTACTTTCTGTTTTGCTTTCCATGCAGGGTGCTGTAGACAT
AAACAAGCTTTGTGAAGAAATGCTGGACAACAGGGCAACCTTACAAGCTATAGCCTCAGAGTTTAGTTCCCTTCCATCAT
ATGCAGCTTTTGCTACTGCTCAAGAAGCTTATGAGCAGGCTGTTGCTAATGGTGATTCTGAAGTTGTTCTTAAAAAGTTG
AAGAAGTCTTTGAATGTGGCTAAATCTGAATTTGACCGTGATGCAGCCATGCAACGTAAGTTGGAAAAGATGGCTGATCA
AGCTATGACCCAAATGTATAAACAGGCTAGATCTGAGGACAAGAGGGCAAAAGTTACTAGTGCTATGCAGACAATGCTTT
TCACTATGCTTAGAAAGTTGGATAATGATGCACTCAACAACATTATCAACAATGCAAGAGATGGTTGTGTTCCCTTGAAC
ATAATACCTCTTACAACAGCAGCCAAACTAATGGTTGTCATACCAGACTATAACACATATAAAAATACGTGTGATGGTAC
AACATTTACTTATGCATCAGCATTGTGGGAAATCCAACAGGTTGTAGATGCAGATAGTAAAATTGTTCAACTTAGTGAAA
TTAGTATGGACAATTCACCTAATTTAGCATGGCCTCTTATTGTAACAGCTTTAAGGGCCAATTCTGCTGTCAAATTACAG
AATAATGAGCTTAGTCCTGTTGCACTACGACAGATGTCTTGTGCTGCCGGTACTACACAAACTGCTTGCACTGATGACAA
TGCGTTAGCTTATTACAACACAACAAAGGGAGGTAGGTTTGTACTTGCACTGTTATCCGATTTACAGGATTTGAAATGGG
CTAGATTCCCTAAGAGTGATGGAACTGGTACTATCTATACAGAACTGGAACCACCTTGTAGGTTTGTTACAGACACACCT
AAAGGTCCTAAAGTGAAGTATTTATACTTTATTAAAGGATTAAACAACCTAAATAGAGGTATGGTACTTGGTAGTTTAGC
TGCCACAGTACGTCTACAAGCTGGTAATGCAACAGAAGTGCCTGCCAATTCAACTGTATTATCTTTCTGTGCTTTTGCTG
TAGATGCTGCTAAAGCTTACAAAGATTATCTAGCTAGTGGGGGACAACCAATCACTAATTGTGTTAAGATGTTGTGTACA
CACACTGGTACTGGTCAGGCAATAACAGTTACACCGGAAGCCAATATGGATCAAGAATCCTTTGGTGGTGCATCGTGTTG
TCTGTACTGCCGTTGCCACATAGATCATCCAAATCCTAAAGGATTTTGTGACTTAAAAGGTAAGTATGTACAAATACCTA
CAACTTGTGCTAATGACCCTGTGGGTTTTACACTTAAAAACACAGTCTGTACCGTCTGCGGTATGTGGAAAGGTTATGGC
TGTAGTTGTGATCAACTCCGCGAACCCATGCTTCAGTCAGCTGATGCACAATCGTTTTTAAACGGGTTTGCGGTGTAAGT
GCAGCCCGTCTTACACCGTGCGGCACAGGCACTAGTACTGATGTCGTATACAGGGCTTTTGACATCTACAATGATAAAGT
AGCTGGTTTTGCTAAATTCCTAAAAACTAATTGTTGTCGCTTCCAAGAAAAGGACGAAGATGACAATTTAATTGATTCTT
ACTTTGTAGTTAAGAGACACACTTTCTCTAACTACCAACATGAAGAAACAATTTATAATTTACTTAAGGATTGTCCAGCT
GTTGCTAAACATGACTTCTTTAAGTTTAGAATAGACGGTGACATGGTACCACATATATCACGTCAACGTCTTACTAAATA
CACAATGGCAGACCTCGTCTATGCTTTAAGGCATTTTGATGAAGGTAATTGTGATACATTAAAAGAAATACTTGTCACAT
ACAATTGTTGTGATGATGATTATTTCAATAAAAAGGACTGGTATGATTTTGTAGAAAACCCAGATATATTACGCGTATAC
GCCAACTTAGGTGAACGTGTACGCCAAGCTTTGTTAAAAACAGTACAATTCTGTGATGCCATGCGAAATGCTGGTATTGT
TGGTGTACTGACATTAGATAATCAAGATCTCAATGGTAACTGGTATGATTTCGGTGATTTCATACAAACCACGCCAGGTA
GTGGAGTTCCTGTTGTAGATTCTTATTATTCATTGTTAATGCCTATATTAACCTTGACCAGGGCTTTAACTGCAGAGTCA
CATGTTGACACTGACTTAACAAAGCCTTACATTAAGTGGGATTTGTTAAAATATGACTTCACGGAAGAGAGGTTAAAACT
CTTTGACCGTTATTTTAAATATTGGGATCAGACATACCACCCAAATTGTGTTAACTGTTTGGATGACAGATGCATTCTGC
ATTGTGCAAACTTTAATGTTTTATTCTCTACAGTGTTCCCACTTACAAGTTTTGGACCACTAGTGAGAAAAATATTTGTT
GATGGTGTTCCATTTGTAGTTTCAACTGGATACCACTTCAGAGAGCTAGGTGTTGTACATAATCAGGATGTAAACTTACA
TAGCTCTAGACTTAGTTTTAAGGAATTACTTGTGTATGCTGCTGACCCTGCTATGCACGCTGCTTCTGGTAATCTATTAC
TAGATAAACGCACTACGTGCTTTTCAGTAGCTGCACTTACTAACAATGTTGCTTTTCAAACTGTCAAACCCGGTAATTTT
AACAAAGACTTCTATGACTTTGCTGTGTCTAAGGGTTTCTTTAAGGAAGGAAGTTCTGTTGAATTAAAACACTTCTTCTT
TGCTCAGGATGGTAATGCTGCTATCAGCGATTATGACTACTATCGTTATAATCTACCAACAATGTGTGATATCAGACAAC
TACTATTTGTAGTTGAAGTTGTTGATAAGTACTTTGATTGTTACGATGGTGGCTGTATTAATGCTAACCAAGTCATCGTC
AACAACCTAGACAAATCAGCTGGTTTTCCATTTAATAAATGGGGTAAGGCTAGACTTTATTATGATTCAATGAGTTATGA
GGATCAAGATGCACTTTTCGCATATACAAAACGTAATGTCATCCCTACTATAACTCAAATGAATCTTAAGTATGCCATTA
GTGCAAAGAATAGAGCTCGCACCGTAGCTGGTGTCTCTATCTGTAGTACTATGACCAATAGACAGTTTCATCAAAAATTA
TTGAAATCAATAGCCGCCACTAGAGGAGCTACTGTAGTAATTGGAACAAGCAAATTCTATGGTGGTTGGCACAACATGTT
AAAAACTGTTTATAGTGATGTAGAAAACCCTCACCTTATGGGTTGGGATTATCCTAAATGTGATAGAGCCATGCCTAACA
 
TGCTTAGAATTATGGCCTCACTTGTTCTTGCTCGCAAACATACAACGTGTTGTAGCTTGTCACACCGTTTCTATAGATTA
GCTAATGAGTGTGCTCAAGTATTGAGTGAAATGGTCATGTGTGGCGGTTCACTATATGTTAAACCAGGTGGAACCTCATC
AGGAGATGCCACAACTGCTTATGCTAATAGTGTTTTTAACATTTGTCAAGCTGTCACGGCCAATGTTAATGCACTTTTAT
CTACTGATGGTAACAAAATTGCCGATAAGTATGTCCGCAATTTACAACACAGACTTTATGAGTGTCTCTATAGAAATAGA
GATGTTGACACAGACTTTGTGAATGAGTTTTACGCATATTTGCGTAAACATTTCTCAATGATGATACTCTCTGACGATGC
TGTTGTGTGTTTCAATAGCACTTATGCATCTCAAGGTCTAGTGGCTAGCATAAAGAACTTTAAGTCAGTTCTTTATTATC
AAAACAATGTTTTTATGTCTGAAGCAAAATGTTGGACTGAGACTGACCTTACTAAAGGACCTCATGAATTTTGCTCTCAA
CATACAATGCTAGTTAAACAGGGTGATGATTATGTGTACCTTCCTTACCCAGATCCATCAAGAATCCTAGGGGCCGGCTG
TTTTGTAGATGATATCGTAAAAACAGATGGTACACTTATGATTGAACGGTTCGTGTCTTTAGCTATAGATGCTTACCCAC
TTACTAAACATCCTAATCAGGAGTATGCTGATGTCTTTCATTTGTACTTACAATACATAAGAAAGCTACATGATGAGTTA
ACAGGACACATGTTAGACATGTATTCTGTTATGCTTACTAATGATAACACTTCAAGGTATTGGGAACCTGAGTTTTATGA
GGCTATGTACACACCGCATACAGTCTTACAGGCTGTTGGGGCTTGTGTTCTTTGCAATTCACAGACTTCATTAAGATGTG
GTGCTTGCATACGTAGACCATTCTTATGTTGTAAATGCTGTTACGACCATGTCATATCAACATCACATAAATTAGTCTTG
TCTGTTAATCCGTATGTTTGCAATGCTCCAGGTTGTGATGTCACAGATGTGACTCAACTTTACTTAGGAGGTATGAGCTA
TTATTGTAAATCACATAAACCACCCATTAGTTTTCCATTGTGTGCTAATGGACAAGTTTTTGGTTTATATAAAAATACAT
GTGTTGGTAGCGATAATGTTACTGACTTTAATGCAATTGCAACATGTGACTGGACAAATGCTGGTGATTACATTTTAGCT
AACACCTGTACTGAAAGACTCAAGCTTTTTGCAGCAGAAACGCTCAAAGCTACTGAGGAGACATTTAAACTGTCTTATGG
TATTGCTACTGTACGTGAAGTGCTGTCTGACAGAGAATTACATCTTTCATGGGAAGTTGGTAAACCTAGACCACCACTTA
ACCGAAATTATGTCTTTACTGGTTATCGTGTAACTAAAAACAGTAAAGTACAAATAGGAGAGTACACCTTTGAAAAAGGT
GACTATGGTGATGCTGTTGTTTACCGAGGTACAACAACTTACAAATTAAATGTTGGTGATTATTTTGTGCTGACATCACA
TACAGTAATGCCATTAAGTGCACCTACACTAGTGCCACAAGAGCACTATGTTAGAATTACTGGCTTATACCCAACACTCA
ATATCTCAGATGAGTTTTCTAGCAATGTTGCAAATTATCAAAAGGTTGGTATGCAAAAGTATTCTACACTCCAGGGACCA
CCTGGTACTGGTAAGAGTCATTTTGCTATTGGCCTAGCTCTCTACTACCCTTCTGCTCGCATAGTGTATACAGCTTGCTC
TCATGCCGCTGTTGATGCACTATGTGAGAAGGCATTAAAATATTTGCCTATAGATAAATGTAGTAGAATTATACCTGCAC
GTGCTCGTGTAGATTGTTTTGATAAATTCAAAGTGAATTCAACATTAGAACAGTATGTCTTTTGTACTGTAAATGCATTG
CCTGAGACGACAGCAGATATAGTTGTCTTTGATGAAATTTCAATGGCCACAAATTATGATTTGAGTGTTGTCAATGCCAG
ATTACGTGCTAAGCACTATGTGTACATTGGCGACCCTGCTCAATTACCTGCACCACGCACATTGCTAACTAAGGGCACAC
TAGAACCAGAATATTTCAATTCAGTGTGTAGACTTATGAAAACTATAGGTCCAGACATGTTCCTCGGAACTTGTCGGCGT
TGTCCTGCTGAAATTGTTGACACTGTGAGTGCTTTGGTTTATGATAATAAGCTTAAAGCACATAAAGACAAATCAGCTCA
ATGCTTTAAAATGTTTTATAAGGGTGTTATCACGCATGATGTTTCATCTGCAATTAACAGGCCACAAATAGGCGTGGTAA
GAGAATTCCTTACACGTAACCCTGCTTGGAGAAAAGCTGTCTTTATTTCACCTTATAATTCACAGAATGCTGTAGCCTCA
AAGATTTTGGGACTACCAACTCAAACTGTTGATTCATCACAGGGCTCAGAATATGACTATGTCATATTCACTCAAACCAC
TGAAACAGCTCACTCTTGTAATGTAAACAGATTTAATGTTGCTATTACCAGAGCAAAAGTAGGCATACTTTGCATAATGT
CTGATAGAGACCTTTATGACAAGTTGCAATTTACAAGTCTTGAAATTCCACGTAGGAATGTGGCAACTTTACAAGCTGAA
AATGTAACAGGACTCTTTAAAGATTGTAGTAAGGTAATCACTGGGTTACATCCTACACAGGCACCTACACACCTCAGTGT
TGACACTAAATTCAAAACTGAAGGTTTATGTGTTGACATACCTGGCATACCTAAGGACATGACCTATAGAAGACTCATCT
CTATGATGGGTTTTAAAATGAATTATCAAGTTAATGGTTACCCTAACATGTTTATCACCCGCGAAGAAGCTATAAGACAT
GTACGTGCATGGATTGGCTTCGATGTCGAGGGGTGTCATGCTACTAGAGAAGCTGTTGGTACCAATTTACCTTTACAGCT
AGGTTTTTCTACAGGTGTTAACCTAGTTGCTGTACCTACAGGTTATGTTGATACACCTAATAATACAGATTTTTCCAGAG
TTAGTGCTAAACCACCGCCTGGAGATCAATTTAAACACCTCATACCACTTATGTACAAAGGACTTCCTTGGAATGTAGTG
CGTATAAAGATTGTACAAATGTTAAGTGACACACTTAAAAATCTCTCTGACAGAGTCGTATTTGTCTTATGGGCACATGG
CTTTGAGTTGACATCTATGAAGTATTTTGTGAAAATAGGACCTGAGCGCACCTGTTGTCTATGTGATAGACGTGCCACAT
GCTTTTCCACTGCTTCAGACACTTATGCCTGTTGGCATCATTCTATTGGATTTGATTACGTCTATAATCCGTTTATGATT
GATGTTCAACAATGGGGTTTTACAGGTAACCTACAAAGCAACCATGATCTGTATTGTCAAGTCCATGGTAATGCACATGT
AGCTAGTTGTGATGCAATCATGACTAGGTGTCTAGCTGTCCACGAGTGCTTTGTTAAGCGTGTTGACTGGACTATTGAAT
ATCCTATAATTGGTGATGAACTGAAGATTAATGCGGCTTGTAGAAAGGTTCAACACATGGTTGTTAAAGCTGCATTATTA
GCAGACAAATTCCCAGTTCTTCACGACATTGGTAACCCTAAAGCTATTAAGTGTGTACCTCAAGCTGATGTAGAATGGAA
GTTCTATGATGCACAGCCTTGTAGTGACAAAGCTTATAAAATAGAAGAATTATTCTATTCTTATGCCACACATTCTGACA
AATTCACAGATGGTGTATGCCTATTTTGGAATTGCAATGTCGATAGATATCCTGCTAATTCCATTGTTTGTAGATTTGAC
ACTAGAGTGCTATCTAACCTTAACTTGCCTGGTTGTGATGGTGGCAGTTTGTATGTAAATAAACATGCATTCCACACACC
AGCTTTTGATAAAAGTGCTTTTGTTAATTTAAAACAATTACCATTTTTCTATTACTCTGACAGTCCATGTGAGTCTCATG
GAAAACAAGTAGTGTCAGATATAGATTATGTACCACTAAAGTCTGCTACGTGTATAACACGTTGCAATTTAGGTGGTGCT
GTCTGTAGACATCATGCTAATGAGTACAGATTGTATCTCGATGCTTATAACATGATGATCTCAGCTGGCTTTAGCTTGTG
GGTTTACAAACAATTTGATACTTATAACCTCTGGAACACTTTTACAAGACTTCAGAGTTTAGAAAATGTGGCTTTTAATG
TTGTAAATAAGGGACACTTTGATGGACAACAGGGTGAAGTACCAGTTTCTATCATTAATAACACTGTTTACACAAAAGTT
 
GATGGTGTTGATGTAGAATTGTTTGAAAATAAAACAACATTACCTGTTAATGTAGCATTTGAGCTTTGGGCTAAGCGCAA
CATTAAACCAGTACCAGAGGTGAAAATACTCAATAATTTGGGTGTGGACATTGCTGCTAATACTGTGATCTGGGACTACA
AAAGAGATGCTCCAGCACATATATCTACTATTGGTGTTTGTTCTATGACTGACATAGCCAAGAAACCAACTGAAACGATT
TGTGCACCACTCACTGTCTTTTTTGATGGTAGAGTTGATGGTCAAGTAGACTTATTTAGAAATGCCCGTAATGGTGTTCT
TATTACAGAAGGTAGTGTTAAAGGTTTACAACCATCTGTAGGTCCCAAACAAGCTAGTCTTAATGGAGTCACATTAATTG
GAGAAGCCGTAAAAACACAGTTCAATTATTATAAGAAAGTTGATGGTGTTGTCCAACAATTACCTGAAACTTACTTTACT
CAGAGTAGAAATTTACAAGAATTTAAACCCAGGAGTCAAATGGAAATTGATTTCTTAGAATTAGCTATGGATGAATTCAT
TGAACGGTATAAATTAGAAGGCTATGCCTTCGAACATATCGTTTATGGAGATTTTAGTCATAGTCAGTTAGGTGGTTTAC
ATCTACTGATTGGACTAGCTAAACGTTTTAAGGAATCACCTTTTGAATTAGAAGATTTTATTCCTATGGACAGTACAGTT
AAAAACTATTTCATAACAGATGCGCAAACAGGTTCATCTAAGTGTGTGTGTTCTGTTATTGATTTATTACTTGATGATTT
TGTTGAAATAATAAAATCCCAAGATTTATCTGTAGTTTCTAAGGTTGTCAAAGTGACTATTGACTATACAGAAATTTCAT
TTATGCTTTGGTGTAAAGATGGCCATGTAGAAACATTTTACCCAAAATTACAATCTAGTCAAGCGTGGCAACCGGGTGTT
GCTATGCCTAATCTTTACAAAATGCAAAGAATGCTATTAGAAAAGTGTGACCTTCAAAATTATGGTGATAGTGCAACATT
ACCTAAAGGCATAATGATGAATGTCGCAAAATATACTCAACTGTGTCAATATTTAAACACATTAACATTAGCTGTACCCT
ATAATATGAGAGTTATACATTTTGGTGCTGGTTCTGATAAAGGAGTTGCACCAGGTACAGCTGTTTTAAGACAGTGGTTG
CCTACGGGTACGCTGCTTGTCGATTCAGATCTTAATGACTTTGTCTCTGATGCAGATTCAACTTTGATTGGTGATTGTGC
AACTGTACATACAGCTAATAAATGGGATCTCATTATTAGTGATATGTACGACCCTAAGACTAAAAATGTTACAAAAGAAA
ATGACTCTAAAGAGGGTTTTTTCACTTACATTTGTGGGTTTATACAACAAAAGCTAGCTCTTGGAGGTTCCGTGGCTATA
AAGATAACAGAACATTCTTGGAATGCTGATCTTTATAAGCTCATGGGACACTTCGCATGGTGGACAGCCTTTGTTACTAA
TGTGAATGCGTCATCATCTGAAGCATTTTTAATTGGATGTAATTATCTTGGCAAACCACGCGAACAAATAGATGGTTATG
TCATGCATGCAAATTACATATTTTGGAGGAATACAAATCCAATTCAGTTGTCTTCCTATTCTTTATTTGACATGAGTAAA
TTTCCCCTTAAATTAAGGGGTACTGCTGTTATGTCTTTAAAAGAAGGTCAAATCAATGATATGATTTTATCTCTTCTTAG
TAAAGGTAGACTTATAATTAGAGAAAACAACAGAGTTGTTATTTCTAGTGATGTTCTTGTTAACAACTAAACGAACAATG
TTTGTTTTTCTTGTTTTATTGCCACTAGTCTCTAGTCAGTGTGTTAATTTTACAAACAGAACTCAATTACCCTCTGCATA
CACTAATTCTTTCACACGTGGTGTTTATTACCCTGACAAAGTTTTCAGATCCTCAGTTTTACATTCAACTCAGGACTTGT
TCTTACCTTTCTTTTCCAATGTTACTTGGTTCCATGCTATACATGTCTCTGGGACCAATGGTACTAAGAGGTTTGATAAC
CCTGTCCTACCATTTAATGATGGTGTTTATTTTGCTTCCACTGAGAAGTCTAACATAATAAGAGGCTGGATTTTTGGTAC
TACTTTAGATTCGAAGACCCAGTCCCTACTTATTGTTAATAACGCTACTAATGTTGTTATTAAAGTCTGTGAATTTCAAT
TTTGTAATTATCCATTTTTGGGTGTTTATTACCACAAAAACAACAAAAGTTGGATGGAAAGTGAGTTCAGAGTTTATTCT
AGTGCGAATAATTGCACTTTTGAATATGTCTCTCAGCCTTTTCTTATGGACCTTGAAGGAAAACAGGGTAATTTCAAAAA
TCTTAGTGAATTTGTGTTTAAGAATATTGATGGTTATTTTAAAATATATTCTAAGCACACGCCTATTAATTTAGTGCGTG
ATCTCCCTCAGGGTTTTTCGGCTTTAGAACCATTGGTAGATTTGCCAATAGGTATTAACATCACTAGGTTTCAAACTTTA
CTTGCTTTACATAGAAGTTATTTGACTCCTGGTGATTCTTCTTCAGGTTGGACAGCTGGTGCTGCAGCTTATTATGTGGG
TTATCTTCAACCTAGGACTTTTCTATTAAAATATAATGAAAATGGAACCATTACAGATGCTGTAGACTGTGCACTTGACC
CTCTCTCAGAAACAAAGTGTACGTTGAAATCCTTCACTGTAGAAAAAGGAATCTATCAAACTTCTAACTTTAGAGTCCAA
CCAACAGAATCTATTGTTAGATTTCCTAATATTACAAACTTGTGCCCTTTTGGTGAAGTTTTTAACGCCACCAGATTTGC
ATCTGTTTATGCTTGGAACAGGAAGAGAATCAGCAACTGTGTTGCTGATTATTCTGTCCTATATAATTCCGCATCATTTT
CCACTTTTAAGTGTTATGGAGTGTCTCCTACTAAATTAAATGATCTCTGCTTTACTAATGTCTATGCAGATTCATTTGTA
ATTAGAGGTGATGAAGTCAGACAAATCGCTCCAGGGCAAACTGGAACGATTGCTGATTATAATTATAAATTACCAGATGA
TTTTACAGGCTGCGTTATAGCTTGGAATTCTAACAATCTTGATTCTAAGGTTGGTGGTAATTATAATTACCTGTATAGAT
TGTTTAGGAAGTCTAATCTCAAACCTTTTGAGAGAGATATTTCAACTGAAATCTATCAGGCCGGTAGCACACCTTGTAAT
GGTGTTAAAGGTTTTAATTGTTACTTTCCTTTACAATCATATGGTTTCCAACCCACTTATGGTGTTGGTTACCAACCATA
CAGAGTAGTAGTACTTTCTTTTGAACTTCTACATGCACCAGCAACTGTTTGTGGACCTAAAAAGTCTACTAATTTGGTTA
AAAACAAATGTGTCAATTTCAACTTCAATGGTTTAACAGGCACAGGTGTTCTTACTGAGTCTAACAAAAAGTTTCTGCCT
TTCCAACAATTTGGCAGAGACATTGCTGACACTACTGATGCTGTCCGTGATCCACAGACACTTGAGATTCTTGACATTAC
ACCATGTTCTTTTGGTGGTGTCAGTGTTATAACACCAGGAACAAATACTTCTAATCAGGTTGCTGTTCTTTATCAGGGTG
TTAACTGCACAGAAGTCCCTGTTGCTATTCATGCAGATCAACTTACTCCTACTTGGCGTGTTTATTCTACAGGTTCTAAT
GTTTTTCAAACACGTGCAGGCTGTTTAATAGGGGCTGAATATGTCAACAACTCATATGAGTGTGACATACCCATTGGTGC
AGGTATATGCGCTAGTTATCAGACTCAGACTAATTCTCCTCGGCGGGCACGTAGTGTAGCTAGTCAATCCATCATTGCCT
ACACTATGTCACTTGGTGCAGAAAATTCAGTTGCTTACTCTAATAACTCTATTGCCATACCCACAAATTTTACTATTAGT
GTTACCACAGAAATTCTACCAGTGTCTATGACCAAGACATCAGTAGATTGTACAATGTACATTTGTGGTGATTCAACTGA
ATGCAGCAATCTTTTGTTGCAATATGGCAGTTTTTGTACACAATTAAACCGTGCTTTAACTGGAATAGCTGTTGAACAAG
ACAAAAACACCCAAGAAGTTTTTGCACAAGTCAAACAAATTTACAAAACACCACCAATTAAAGATTTTGGTGGTTTTAAT
TTTTCACAAATATTACCAGATCCATCAAAACCAAGCAAGAGGTCATTTATTGAAGATCTACTTTTCAACAAAGTGACACT
TGCAGATGCTGGCTTCATCAAACAATATGGTGATTGCCTTGGTGATATTGCTGCTAGAGACCTCATTTGTGCACAAAAGT
 
TTAACGGCCTTACTGTTTTGCCACCTTTGCTCACAGATGAAATGATTGCTCAATACACTTCTGCACTGTTAGCGGGTACA
ATCACTTCTGGTTGGACCTTTGGTGCAGGTGCTGCATTACAAATACCATTTGCTATGCAAATGGCTTATAGGTTTAATGG
TATTGGAGTTACACAGAATGTTCTCTATGAGAACCAAAAATTGATTGCCAACCAATTTAATAGTGCTATTGGCAAAATTC
AAGACTCACTTTCTTCCACAGCAAGTGCACTTGGAAAACTTCAAGATGTGGTCAACCAAAATGCACAAGCTTTAAACACG
CTTGTTAAACAACTTAGCTCCAATTTTGGTGCAATTTCAAGTGTTTTAAATGATATCCTTTCACGTCTTGACAAAGTTGA
GGCTGAAGTGCAAATTGATAGGTTGATCACAGGCAGACTTCAAAGTTTGCAGACATATGTGACTCAACAATTAATTAGAG
CTGCAGAAATCAGAGCTTCTGCTAATCTTGCTGCTATTAAAATGTCAGAGTGTGTACTTGGACAATCAAAAAGAGTTGAT
TTTTGTGGAAAGGGCTATCATCTTATGTCCTTCCCTCAGTCAGCACCTCATGGTGTAGTCTTCTTGCATGTGACTTATGT
CCCTGCACAAGAAAAGAACTTCACAACTGCTCCTGCCATTTGTCATGATGGAAAAGCACACTTTCCTCGTGAAGGTGTCT
TTGTTTCAAATGGCACACACTGGTTTGTAACACAAAGGAATTTTTATGAACCACAAATCATTACTACAGACAACACATTT
GTGTCTGGTAACTGTGATGTTGTAATAGGAATTGTCAACAACACAGTTTATGATCCTTTGCAACCTGAATTAGACTCATT
CAAGGAGGAGTTAGATAAATATTTTAAGAATCATACATCACCAGATGTTGATTTAGGTGACATCTCTGGCATTAATGCTT
CATTTGTAAACATTCAAAAAGAAATTGACCGCCTCAATGAGGTTGCCAAGAATTTAAATGAATCTCTCATCGATCTCCAA
GAACTTGGAAAGTATGAGCAGTATATAAAATGGCCATGGTACATTTGGCTAGGTTTTATAGCTGGCTTGATTGCCATAGT
AATGGTGACAATTATGCTTTGCTGTATGACCAGTTGCTGTAGTTGTCTCAAGGGCTGTTGTTCTTGTGGATCCTGCTGCA
AATTTGATGAAGACGACTCTGAGCCAGTGCTCAAAGGAGTCAAATTACATTACACATAAACGAACTTATGGATTTGTTTA
TGAGAATCTTCACAATTGGAACTGTAACTTTGAAGCAAGGTGAAATCAAGGATGCTACTCCTTCAGATTTTGTTCGCGCT
ACTGCAACGATACCGATACAAGCCTCACTCCCTTTCGGATGGCTTATTGTTGGCGTTGCACTTCTTGCTGTTTTTCAGAG
CGCTTCCAAAATCATAACCCTCAAAAAGAGATGGCAACTAGCACTCTCCAAGGGTGTTCACTTTGTTTGCAACTTGCTGT
TGTTGTTTGTAACAGTTTACTCACACCTTTTGCTCGTTGCTGCTGGCCTTGAAGCCCCTTTTCTCTATCTTTATGCTTTA
GTCTACTTCTTGCAGAGTATAAACTTTGTAAGAATAATAATGAGGCTTTGGCTTTGCTGGAAATGCCGTTCCAAAAACCC
ATTACTTTATGATGCCAACTATTTTCTTTGCTGGCATACTAATTGTTACGACTATTGTATACCTTACAATAGTGTAACTT
CTTCAATTGTCATTACTTCAGGTGATGGCACAACAAGTCCTATTTCTGAACATGACTACCAGATTGGTGGTTATACTGAA
AAATGGGAATCTGGAGTAAAAGACTGTGTTGTATTACACAGTTACTTCACTTCAGACTATTACCAGCTGTACTCAACTCA
ATTGAGTACAGACACTGGTGTTGAACATGTTACCTTCTTCATCTACAATAAAATTGTTGATGAGCCTGAAGAACATGTCC
AAATTCACACAATCGACGGTTCACCCGGAGTTGTTAATCCAGTAATGGAACCAATTTATGATGAACCGACGACGACTACT
AGCGTGCCTTTGTAAGCACAAGCTGATGAGTACGAACTTATGTACTCATTCGTTTCGGAAGAGACAGGTACGTTAATAGT
TAATAGCGTACTTCTTTTTCTTGCTTTCGTGGTATTCTTGCTAGTTACACTAGCCATCCTTACTGCGCTTCGATTGTGTG
CGTACTGCTGCAATATTGTTAACGTGAGTCTTGTAAAACCTTCTTTTTACGTTTACTCTCGTGTTAAAAATCTGAATTCT
TCTAGAGTTCCTGATCTTCTGGTCTAAACGAACTAAATATTATATTAGTTTTTCTGTTTGGAACTTTAATTTTAGCCATG
GCAGATTCCAACGGTACTATTACCGTTGAAGAGCTTAAAAAGCTCCTTGAACAATGGAACCTAGTAATAGGTTTCCTATT
CCTTACATGGATTTGTCTTCTACAATTTGCCTATGCCAACAGGAATAGGTTTTTGTATATAATTAAGTTAATTTTCCTCT
GGCTGTTATGGCCAGTAACTTTAGCTTGTTTTGTGCTTGCTGCTGTTTACAGAATAAATTGGATCACCGGTGGAATTGCT
ATCGCAATGGCTTGTCTTGTAGGCTTGATGTGGCTCAGCTACTTCATTGCTTCTTTCAGACTGTTTGCGCGTACGCGTTC
CATGTGGTCATTCAATCCAGAAACTAACATTCTTCTCAACGTGCCACTCCATGGCACTATTCTGACCAGACCGCTTCTAG
AAAGTGAACTCGTAATCGGAGCTGTGATCCTTCGTGGACATCTTCGTATTGCTGGACACCATCTAGGACGCTGTGACATC
AAGGACCTGCCTAAAGAAATCACTGTTGCTACATCACGAACGCTTTCTTATTACAAATTGGGAGCTTCGCAGCGTGTAGC
AGGTGACTCAGGTTTTGCTGCATACAGTCGCTACAGGATTGGCAACTATAAATTAAACACAGACCATTCCAGTAGCAGTG
ACAATATTGCTTTGCTTGTACAGTAAGTGACAACAGATGTTTCATCTCGTTGACTTTCAGGTTACTATAGCAGAGATATT
ACTAATTATTATGAGGACTTTTAAAGTTTCCATTTGGAATCTTGATTACATCATAAACCTCATAATTAAAAATTTATCTA
AGTCACTAACTGAGAATAAATATTCTCAATTAGATGAAGAGCAACCAATGGAGATTGATTAAACGAACATGAAAATTATT
CTTTTCTTGGCACTGATAACACTCGCTACTTGTGAGCTTTATCACTACCAAGAGTGTGTTAGAGGTACAACAGTACTTTT
AAAAGAACCTTGCTCTTCTGGAACATACGAGGGCAATTCACCATTTCATCCTCTAGCTGATAACAAATTTGCACTGACTT
GCTTTAGCACTCAATTTGCTTTTGCTTGTCCTGACGGCGTAAAACACGTCTATCAGTTACGTGCCAGATCAGTTTCACCT
AAACTGTTCATCAGACAAGAGGAAGTTCAAGAACTTTACTCTCCAATTTTTCTTATTGTTGCGGCAATAGTGTTTATAAC
ACTTTGCTTCACACTCAAAAGAAAGACAGAATGATTGAACTTTCATTAATTGACTTCTATTTGTGCTTTTTAGCCTTTCT
GCTATTCCTTGTTTTAATTATGCTTATTATCTTTTGGTTCTCACTTGAACTGCAAGATCATAATGAAACTTGTCACGCCT
AAACGAACATGAAATTTCTTGTTTTCTTAGGAATCATCACAACTGTAGCTGCATTTCACCAAGAATGTAGTTTACAGTCA
TGTACTCAACATCAACCATATGTAGTTGATGACCCGTGTCCTATTCACTTCTATTCTAAATGGTATATTAGAGTAGGAGC
TAGAAAATCAGCACCTTTAATTGAATTGTGCGTGGATGAGGCTGGTTCTAAATCACCCATTCAGTACATCGATATCGGTA
ATTATACAGTTTCCTGTTTACCTTTTACAATTAATTGCCAGAAACCTAAATTGGGTAGTCTTGTAGTGCGTTGTTCGTTC
TATGAAGACTTTTTAGAGTATCATGACGTTCGTGTTGTTTTAGATTTCATCTAAACGAACAAACAAACTAAAATGTCTGA
TAATGGACCCCAAAATCAGCGAAATGCACCCCGCATTACGTTTGGTGGACCCTCAGATTCAACTGGCAGTAACCAGAATG
GAGAACGCAGTGGGGCGCGATCAAAACAACGTCGGCCCCAAGGTTTACCCAATAATACTGCGTCTTGGTTCACCGCTCTC
ACTCAACATGGCAAGGAAGACCTTAAATTCCCTCGAGGACAAGGCGTTCCAATTAACACCAATAGCAGTCGAGATGACCA
 
AATTGGCTACTACCGAAGAGCTACCAGACGAATTCGTGGTGGTGACGGTAAAATGAAAGATCTCAGTCCAAGATGGTATT
TCTACTACCTAGGAACTGGGCCAGAAGCTGGACTTCCCTATGGTGCTAACAAAGACGGCATCATATGGGTTGCAACTGAG
GGAGCCTTGAATACACCAAAAGATCACATTGGCACCCGCAATCCTGCTAACAATGCTGCAATCGTGCTACAACTTCCTCA
AGGAACAACATTGCCAAAAGGCTTCTACGCAGAAGGGAGCAGAGGCGGCAGTCAAGCCTCTTCTCGTTCCTCATCACGTA
GTCGCAACAGTTCAAGAAATTCAACTCCAGGCAGCTCTAAACGAACTTCTCCTGCTAGAATGGCTGGCAATGGCGGTGAT
GCTGCTCTTGCTTTGCTGCTGCTTGACAGATTGAACCAGCTTGAGAGCAAAATGTCTGGTAAAGGCCAACAACAACAAGG
CCAAACTGTCACTAAGAAATCTGCTGCTGAGGCTTCTAAGAAGCCTCGGCAAAAACGTACTGCCACTAAAGCATACAATG
TAACACAAGCTTTCGGCAGACGTGGTCCAGAACAAACCCAAGGAAATTTTGGGGACCAGGAACTAATCAGACAAGGAACT
GATTACAAACATTGGCCGCAAATTGCACAATTTGCCCCCAGCGCTTCAGCGTTCTTCGGAATGTCGCGCATTGGCATGGA
AGTCACACCTTCGGGAACGTGGTTGACCTACACAGGTGCCATCAAATTGGATGACAAAGATCCAAATTTCAAAGATCAAG
TCATTTTGCTGAATAAGCATATTGACGCATACAAAACATTCCCACCAACAGAGCCTAAAAAGGACAAAAAGAAGAAGGCT
GATGAAACTCAAGCCTTACCGCAGAGACAGAAGAAACAGCAAACTGTGACTCTTCTTCCTGCTGCAGATTTGGATGATTT
CTCCAAACAATTGCAACAATCCATGAGCAGTGCTGACTCAACTCAGGCCTAAACTCATGCAGACCACACAAGGCAGATGG
GCTATATAAACGTTTTCGCTTTTCCGTTTACGATATATAGTCTACTCTTGTGCAGAATGAATTCTCGTAACTACATAGCA
CAAGTAGATGTAGTTAACTTTAATCTCACATAGCAATCTTTAATCAGTGTGTAACATTAGGGAGGACTTGAAAGAGCCAC
CACATTTTCACCGAGGCCACGCGGAGTACGATCGAGTGTACAGTGAACAATGCTAGGGAGAGCTGCCTATATGGAAGAGC
CCTAATGTGTAAAATTAATTTTAGTAGTGCTAACCCCATGTGATTTTAATAGCTTCTTA
SEQ ID NO: 21
   >QQX12069.1 surface glycoprotein, from genome accession MW520923 
MFVFLVLLPLVSSQCVNFTNRTQLPSAYTNSFTRGVYYPDKVFRSSVLHSTQDLFLPFFSNVTWFHAIHVSGTNGTKRFDNPVLP
FNDGVYFASTEKSNIIRGWIFGTTLDSKTQSLLIVNNATNVVIKVCEFQFCNYPFLGVYYHKNNKSWMESEFRVYSSANNCTFEY
VSQPFLMDLEGKQGNFKNLSEFVFKNIDGYFKIYSKHTPINLVRDLPQGFSALEPLVDLPIGINITRFQTLLALHRSYLTPGDSSSG
WTAGAAAYYVGYLQPRTFLLKYNENGTITDAVDCALDPLSETKCTLKSFTVEKGIYQTSNFRVQPTESIVRFPNITNLCPFGEVFN
ATRFASVYAWNRKRISNCVADYSVLYNSASFSTFKCYGVSPTKLNDLCFTNVYADSFVIRGDEVRQIAPGQTGTIADYNYKLPDD
FTGCVIAWNSNNLDSKVGGNYNYLYRLFRKSNLKPFERDISTEIYQAGSTPCNGVKGFNCYFPLQSYGFQPTYGVGYQPYRVVVL
SFELLHAPATVCGPKKSTNLVKNKCVNFNFNGLTGTGVLTESNKKFLPFQQFGRDIADTTDAVRDPQTLEILDITPCSFGGVSVIT
PGTNTSNQVAVLYQGVNCTEVPVAIHADQLTPTWRVYSTGSNVFQTRAGCLIGAEYVNNSYECDIPIGAGICASYQTQTNSPRR
ARSVASQSIIAYTMSLGAENSVAYSNNSIAIPTNFTISVTTEILPVSMTKTSVDCTMYICGDSTECSNLLLQYGSFCTQLNRALTGIA
VEQDKNTQEVFAQVKQIYKTPPIKDFGGFNFSQILPDPSKPSKRSFIEDLLFNKVTLADAGFIKQYGDCLGDIAARDLICAQKFNGL
TVLPPLLTDEMIAQYTSALLAGTITSGWTFGAGAALQIPFAMQMAYRFNGIGVTQNVLYENQKLIANQFNSAIGKIQDSLSSTAS
ALGKLQDVVNQNAQALNTLVKQLSSNFGAISSVLNDILSRLDKVEAEVQIDRLITGRLQSLQTYVTQQLIRAAEIRASANLAAIKM
SECVLGQSKRVDFCGKGYHLMSFPQSAPHGVVFLHVTYVPAQEKNFTTAPAICHDGKAHFPREGVFVSNGTHWFVTQRNFYE
PQIITTDNTFVSGNCDVVIGIVNNTVYDPLQPELDSFKEELDKYFKNHTSPDVDLGDISGINASFVNIQKEIDRLNEVAKNLNESLID
LQELGKYEQYIKWPWYIWLGFIAGLIAIVMVTIMLCCMTSCCSCLKGCCSCGSCCKFDEDDSEPVLKGVKLHYT
SEQ ID NO: 22
   > Severe acute respiratory syndrome coronavirus 2 isolate SARS-CoV-2/human/England/MIG457/2020, EVAg Ref-SKU:004V-04032, complete genome. UK B 1.1.7 lineage 
ATTAAAGGTTTATACCTTCCCAGGTAACAAACCAACCAACTTTCGATCTCTTGTAGATCTGTTCTCTAAACGAACTTTAAAAT
CTGTGTGGCTGTCACTCGGCTGCATGCTTAGTGCACTCACGCAGTATAATTAATAACTAATTACTGTCGTTGACAGGACAC
GAGTAACTCGTCTATCTTCTGCAGGCTGCTTACGGTTTCGTCCGTGTTGCAGCCGATCATCAGCACATCTAGGTTTTGTCCG
GGTGTGACCGAAAGGTAAGATGGAGAGCCTTGTCCCTGGTTTCAACGAGAAAACACACGTCCAACTCAGTTTGCCTGTTTT
ACAGGTTCGCGACGTGCTCGTACGTGGCTTTGGAGACTCCGTGGAGGAGGTCTTATCAGAGGCACGTCAACATCTTAAAG
ATGGCACTTGTGGCTTAGTAGAAGTTGAAAAAGGCGTTTTGCCTCAACTTGAACAGCCCTATGTGTTCATCAAACGTTCGG
ATGCTCGAACTGCACCTCATGGTCATGTTATGGTTGAGCTGGTAGCAGAACTCGAAGGCATTCAGTACGGTCGTAGTGGT
GAGACACTTGGTGTCCTTGTCCCTCATGTGGGCGAAATACCAGTGGCTTACCGCAAGGTTCTTCTTCGTAAGAACGGTAAT
AAAGGAGCTGGTGGCCATAGTTACGGCGCCGATCTAAAGTCATTTGACTTAGGCGACGAGCTTGGCACTGATCCTTATGA
AGATTTTCAAGAAAACTGGAACACTAAACATAGCAGTGGTGTTACCCGTGAACTCATGCGTGAGCTTAACGGAGGGGCAT
ACACTCGCTATGTCGATAACAACTTCTGTGGCCCTGATGGCTACCCTCTTGAGTGCATTAAAGACCTTCTAGCACGTGCTGG
TAAAGCTTCATGCACTTTGTCTGAACAACTGGACTTTATTGACACTAAGAGGGGTGTATACTGCTGCCGTGAACATGAGCA
TGAAATTGCTTGGTACACGGAACGTTCTGAAAAGAGCTATGAATTGCAGACACCTTTTGAAATTAAATTGGCAAAGAAATT
TGACACCTTCAATGGGGAATGTCCAAATTTTGTATTTCCCTTAAATTCCATAATCAAGACTATTCAACCAAGGGTTGAAAAG
AAAAAGCTTGATGGCTTTATGGGTAGAATTCGATCTGTCTATCCAGTTGCGTCACCAAATGAATGCAACCAAATGTGCCTTT
CAACTCTCATGAAGTGTGATCATTGTGGTGAAACTTCATGGCAGACGGGCGATTTTGTTAAAGCCACTTGCGAATTTTGTG
 
GCACTGAGAATTTGACTAAAGAAGGTGCCACTACTTGTGGTTACTTACCCCAAAATGCTGTTGTTAAAATTTATTGTCCAGC
ATGTCACAATTCAGAAGTAGGACCTGAGCATAGTCTTGCCGAATACCATAATGAATCTGGCTTGAAAACCATTCTTCGTAA
GGGTGGTCGCACTATTGCCTTTGGAGGCTGTGTGTTCTCTTATGTTGGTTGCCATAACAAGTGTGCCTATTGGGTTCCACGT
GCTAGCGCTAACATAGGTTGTAACCATACAGGTGTTGTTGGAGAAGGTTCCGAAGGTCTTAATGACAACCTTCTTGAAATA
CTCCAAAAAGAGAAAGTCAACATCAATATTGTTGGTGACTTTAAACTTAATGAAGAGATCGCCATTATTTTGGCATCTTTTT
CTGCTTCCACAAGTGCTTTTGTGGAAACTGTGAAAGGTTTGGATTATAAAGCATTCAAACAAATTGTTGAATCCTGTGGTA
ATTTTAAAGTTACAAAAGGAAAAGCTAAAAAAGGTGCCTGGAATATTGGTGAACAGAAATCAATACTGAGTCCTCTTTATG
CATTTGCATCAGAGGCTGCTCGTGTTGTACGATCAATTTTCTCCCGCACTCTTGAAACTGCTCAAAATTCTGTGCGTGTTTTA
CAGAAGGCCGCTATAACAATACTAGATGGAATTTCACAGTATTCACTGAGACTCATTGATGCTATGATGTTCACATCTGATT
TGGCTACTAACAATCTAGTTGTAATGGCCTACATTACAGGTGGTGTTGTTCAGTTGACTTCGCAGTGGCTAACTAACATCTT
TGGCACTGTTTATGAAAAACTCAAACCCGTCCTTGATTGGCTTGAAGAGAAGTTTAAGGAAGGTGTAGAGTTTCTTAGAGA
CGGTTGGGAAATTGTTAAATTTATCTCAACCTGTGCTTGTGAAATTGTCGGTGGACAAATTGTCACCTGTGCAAAGGAAAT
TAAGGAGAGTGTTCAGACATTCTTTAAGCTTGTAAATAAATTTTTGGCTTTGTGTGCTGACTCTATCATTATTGGTGGAGCT
AAACTTAAAGCCTTGAATTTAGGTGAAACATTTGTCACGCACTCAAAGGGATTGTACAGAAAGTGTGTTAAATCCAGAGAA
GAAACTGGCCTACTCATGCCTCTAAAAGCCCCAAAAGAAATTATCTTCTTAGAGGGAGAAACACTTCCCACAGAAGTGTTA
ACAGAGGAAGTTGTCTTGAAAACTGGTGATTTACAACCATTAGAACAACCTACTAGTGAAGCTGTTGAAGCTCCATTGGTT
GGTACACCAGTTTGTATTAACGGGCTTATGTTGCTCGAAATCAAAGACACAGAAAAGTACTGTGCCCTTGCACCTAATATG
ATGGTAACAAACAATACCTTCACACTCAAAGGCGGTGCACCAACAAAGGTTACTTTTGGTGATGACACTGTGATAGAAGTG
CAAGGTTACAAGAGTGTGAATATCACTTTTGAACTTGATGAAAGGATTGATAAAGTACTTAATGAGAAGTGCTCTGCCTAT
ACAGTTGAACTCGGTACAGAAGTAAATGAGTTCGCCTGTGTTGTGGCAGATGCTGTCATAAAAACTTTGCAACCAGTATCT
GAATTACTTACACCACTGGGCATTGATTTAGATGAGTGGAGTATGGCTACATACTACTTATTTGATGAGTCTGGTGAGTTTA
AATTGGCTTCACATATGTATTGTTCTTTTTACCCTCCAGATGAGGATGAAGAAGAAGGTGATTGTGAAGAAGAAGAGTTTG
AGCCATCAACTCAATATGAGTATGGTACTGAAGATGATTACCAAGGTAAACCTTTGGAATTTGGTGCCACTTCTGCTGCTCT
TCAACCTGAAGAAGAGCAAGAAGAAGATTGGTTAGATGATGATAGTCAACAAACTGTTGGTCAACAAGACGGCAGTGAG
GACAATCAGACAACTATTATTCAAACAATTGTTGAGGTTCAACCTCAATTAGAGATGGAACTTACACCAGTTGTTCAGACTA
TTGAAGTGAATAGTTTTAGTGGTTATTTAAAACTTACTGACAATGTATACATTAAAAATGCAGACATTGTGGAAGAAGCTA
AAAAGGTAAAACCAACAGTGGTTGTTAATGCAGCCAATGTTTACCTTAAACATGGAGGAGGTGTTGCAGGAGCCTTAAAT
AAGGCTACTAACAATGCCATGCAAGTTGAATCTGATGATTACATAGCTACTAATGGACCACTTAAAGTGGGTGGTAGTTGT
GTTTTAAGCGGACACAATCTTGCTAAACACTGTCTTCATGTTGTCGGCCCAAATGTTAACAAAGGTGAAGACATTCAACTTC
TTAAGAGTGCTTATGAAAATTTTAATCAGCACGAAGTTCTACTTGCACCATTATTATCAGCTGGTATTTTTGGTGCTGACCCT
ATACATTCTTTAAGAGTTTGTGTAGATACTGTTCGCACAAATGTCTACTTAGCTGTCTTTGATAAAAATCTCTATGACAAACT
TGTTTCAAGCTTTTTGGAAATGAAGAGTGAAAAGCAAGTTGAACAAAAGATCGCTGAGATTCCTAAAGAGGAAGTTAAGC
CATTTATAACTGAAAGTAAACCTTCAGTTGAACAGAGAAAACAAGATGATAAGAAAATCAAAGCTTGTGTTGAAGAAGTT
ACAACAACTCTGGAAGAAACTAAGTTCCTCACAGAAAACTTGTTACTTTATATTGACATTAATGGCAATCTTCATCCAGATT
CTGCCACTCTTGTTAGTGACATTGACATCACTTTCTTAAAGAAAGATGCTCCATATATAGTGGGTGATGTTGTTCAAGAGGG
TGTTTTAACTGCTGTGGTTATACCTACTAAAAAGGCTGGTGGCACTACTGAAATGCTAGCGAAAGCTTTGAGAAAAGTGCC
AACAGACAATTATATAACCACTTACCCGGGTCAGGGTTTAAATGGTTACACTGTAGAGGAGGCAAAGACAGTGCTTAAAA
AGTGTAAAAGTGCCTTTTACATTCTACCATCTATTATCTCTAATGAGAAGCAAGAAATTCTTGGAACTGTTTCTTGGAATTTG
CGAGAAATGCTTGCACATGCAGAAGAAACACGCAAATTAATGCCTGTCTGTGTGGAAACTAAAGCCATAGTTTCAACTATA
CAGCGTAAATATAAGGGTATTAAAATACAAGAGGGTGTGGTTGATTATGGTGCTAGATTTTACTTTTACACCAGTAAAACA
ACTGTAGCGTCACTTATCAACACACTTAACGATCTAAATGAAACTCTTGTTACAATGCCACTTGGCTATGTAACACATGGCT
TAAATTTGGAAGAAGCTGCTCGGTATATGAGATCTCTCAAAGTGCCAGCTACAGTTTCTGTTTCTTCACCTGATGCTGTTAC
AGCGTATAATGGTTATCTTACTTCTTCTTCTAAAACACCTGAAGAACATTTTATTGAAACCATCTCACTTGCTGGTTCCTATA
AAGATTGGTCCTATTCTGGACAATCTACACAACTAGGTATAGAATTTCTTAAGAGAGGTGATAAAAGTGTATATTACACTA
GTAATCCTACCACATTCCACCTAGATGGTGAAGTTATCACCTTTGACAATCTTAAGACACTTCTTTCTTTGAGAGAAGTGAG
GACTATTAAGGTGTTTACAACAGTAGACAACATTAATCTCCACACGCAAGTTGTGGACATGTCAATGACATATGGACAACA
GTTTGGTCCAACTTATTTGGATGGAGCTGATGTTACTAAAATAAAACCTCATAATTCACATGAAGGTAAAACATTTTATGTT
TTACCTAATGATGACACTCTACGTGTTGAGGCTTTTGAGTACTACCACACAACTGATCCTAGTTTTCTGGGTAGGTACATGT
CAGCATTAAATCACACTAAAAAGTGGAAATACCCACAAGTTAATGGTTTAACTTCTATTAAATGGGCAGATAACAACTGTT
ATCTTGCCACTGCATTGTTAACACTCCAACAAATAGAGTTGAAGTTTAATCCACCTGCTCTACAAGATGCTTATTACAGAGC
AAGGGCTGGTGAAGCTGATAACTTTTGTGCACTTATCTTAGCCTACTGTAATAAGACAGTAGGTGAGTTAGGTGATGTTAG
AGAAACAATGAGTTACTTGTTTCAACATGCCAATTTAGATTCTTGCAAAAGAGTCTTGAACGTGGTGTGTAAAACTTGTGG
ACAACAGCAGACAACCCTTAAGGGTGTAGAAGCTGTTATGTACATGGGCACACTTTCTTATGAACAATTTAAGAAAGGTGT
TCAGATACCTTGTACGTGTGGTAAACAAGCTACAAAATATCTAGTACAACAGGAGTCACCTTTTGTTATGATGTCAGCACCA
CCTGCTCAGTATGAACTTAAGCATGGTACATTTACTTGTGCTAGTGAGTACACTGGTAATTACCAGTGTGGTCACTATAAAC
 
ATATAACTTCTAAAGAAACTTTGTATTGCATAGACGGTGCTTTACTTACAAAGTCCTCAGAATACAAAGGTCCTATTACGGA
TGTTTTCTACAAAGAAAACAGTTACACAACAACCATAAAACCAGTTACTTATAAATTGGATGGTGTTGTTTGTACAGAAATT
GACCCTAAGTTGGACAATTATTATAAGAAAGACAATTCTTATTTTACAGAGCAACCAATTGATCTTGTACCAAACCAACCAT
ATCCAAACGCAAGCTTCGATAATTTTAAGTTTGTATGTGATAATATCAAATTTGCTGATGATTTAAACCAGTTAACTGGTTA
TAAGAAACCTGCTTCAAGAGAGCTTAAAGTTACATTTTTCCCTGACTTAAATGGTGATGTGGTGGCTATTGATTATAAACAC
TACACACCCTCTTTTAAGAAAGGAGCTAAATTGTTACATAAACCTATTGTTTGGCATGTTAACAATGCAACTAATAAAGCCA
CGTATAAACCAAATACCTGGTGTATACGTTGTCTTTGGAGCACAAAACCAGTTGAAACATCAAATTCGTTTGATGTACTGAA
GTCAGAGGACGCGCAGGGAATGGATAATCTTGCCTGCGAAGATCTAAAACCAGTCTCTGAAGAAGTAGTGGAAAATCCTA
CCATACAGAAAGACGTTCTTGAGTGTAATGTGAAAACTACCGAAGTTGTAGGAGACATTATACTTAAACCAGCAAATAATA
GTTTAAAAATTACAGAAGAGGTTGGCCACACAGATCTAATGGCTGCTTATGTAGACAATTCTAGTCTTACTATTAAGAAAC
CTAATGAATTATCTAGAGTATTAGGTTTGAAAACCCTTGTTACTCATGGTTTAGCTGCTGTTAATAGTGTCCCTTGGGATAC
TATAGCTAATTATGCTAAGCCTTTTCTTAACAAAGTTGTTAGTACAACTACTAACATAGTTACACGGTGTTTAAACCGTGTTT
GTACTAATTATATGCCTTATTTCTTTACTTTATTGCTACAATTGTGTACTTTTACTAGAAGTACAAATTCTAGAATTAAAGCAT
CTATGCCGACTACTATAGCAAAGAATACTGTTAAGAGTGTCGGTAAATTTTGTCTAGAGGCTTCATTTAATTATTTGAAGTC
ACCTAATTTTTCTAAACTGATAAATATTACAATTTGGTTTTTACTATTAAGTGTTTGCCTAGGTTCTTTAATCTACTCAACCGC
TGCTTTAGGTGTTTTAATGTCTAATTTAGGCATGCCTTCTTACTGTACTGGTTACAGAGAAGGCTATTTGAACTCTACTAATG
TCACTATTGCAACCTACTGTACTGGTTCTATACCTTGTAGTGTTTGTCTTAGTGGTTTAGATTCTTTAGACACCTATCCTTCTT
TAGAAACTATACAAATTACCATTTCATCTTTTAAATGGGATTTAACTGCTTTTGGCTTAGTTGCAGAGTGGTTTTTGGCATAT
ATTCTTTTCACTAGGTTTTTCTATGTACTTGGATTGGCTGCAATCATGCAATTGTTTTTCAGCTATTTTGCAGTACATTTTATT
AGTAATTCTTGGCTTATGTGGTTAATAATTAATCTTGTACAAATGGCCCCGATTTCAGCTATGGTTAGAATGTACATCTTCTT
TGCATCATTTTATTATGTATGGAAAAGTTATGTGCATGTTGTAGACGGTTGTAATTCATCAACTTGTATGATGTGTTACAAA
CGTAATAGAGCAACAAGAGTCGAATGTACAACTATTGTTAATGGTGTTAGAAGGTCCTTTTATGTCTATGCTAATGGAGGT
AAAGGCTTTTGCAAACTACACAATTGGAATTGTGTTAATTGTGATACATTCTGTGCTGGTAGTACATTTATTAGTGATGAAG
TTGCGAGAGACTTGTCACTACAGTTTAAAAGACCAATAAATCCTACTGACCAGTCTTCTTACATCGTTGATAGTGTTACAGT
GAAGAATGGTTCCATCCATCTTTACTTTGATAAAGCTGGTCAAAAGACTTATGAAAGACATTCTCTCTCTCATTTTGTTAACT
TAGACAACCTGAGAGCTAATAACACTAAAGGTTCATTGCCTATTAATGTTATAGTTTTTGATGGTAAATCAAAATGTGAAG
AATCATCTGCAAAATCAGCGTCTGTTTACTACAGTCAGCTTATGTGTCAACCTATACTGTTACTAGATCAGGCATTAGTGTC
TGATGTTGGTGATAGTGCGGAAGTTGCAGTTAAAATGTTTGATGCTTACGTTAATACGTTTTCATCAACTTTTAACGTACCA
ATGGAAAAACTCAAAACACTAGTTGCAACTGCAGAAGCTGAACTTGCAAAGAATGTGTCCTTAGACAATGTCTTATCTACT
TTTATTTCAGCAGCTCGGCAAGGGTTTGTTGATTCAGATGTAGAAACTAAAGATGTTGTTGAATGTCTTAAATTGTCACATC
AATCTGACATAGAAGTTACTGGCGATAGTTGTAATAACTATATGCTCACCTATAACAAAGTTGAAAACATGACACCCCGTG
ACCTTGGTGCTTGTATTGACTGTAGTGCGCGTCATATTAATGCGCAGGTAGCAAAAAGTCACAACATTGCTTTGATATGGA
ACGTTAAAGATTTCATGTCATTGTCTGAACAACTACGAAAACAAATACGTAGTGCTGCTAAAAAGAATAACTTACCTTTTAA
GTTGACATGTGCAACTACTAGACAAGTTGTTAATGTTGTAACAACAAAGATAGCACTTAAGGGTGGTAAAATTGTTAATAA
TTGGTTGAAGCAGTTAATTAAAGTTACACTTGTGTTCCTTTTTGTTGCTGCTATTTTCTATTTAATAACACCTGTTCATGTCAT
GTCTAAACATACTGACTTTTCAAGTGAAATCATAGGATACAAGGCTATTGATGGTGGTGTCACTCGTGACATAGCATCTAC
AGATACTTGTTTTGCTAACAAACATGCTGATTTTGACACATGGTTTAGCCAGCGTGGTGGTAGTTATACTAATGACAAAGCT
TGCCCATTGATTGCTGCAGTCATAACAAGAGAAGTGGGTTTTGTCGTGCCTGGTTTGCCTGGCACGATATTACGCACAACT
AATGGTGACTTTTTGCATTTCTTACCTAGAGTTTTTAGTGCAGTTGGTAACATCTGTTACACACCATCAAAACTTATAGAGTA
CACTGACTTTGCAACATCAGCTTGTGTTTTGGCTGCTGAATGTACAATTTTTAAAGATGCTTCTGGTAAGCCAGTACCATAT
TGTTATGATACCAATGTACTAGAAGGTTCTGTTGCTTATGAAAGTTTACGCCCTGACACACGTTATGTGCTCATGGATGGCT
CTATTATTCAATTTCCTAACACCTACCTTGAAGGTTCTGTTAGAGTGGTAACAACTTTTGATTCTGAGTACTGTAGGCACGG
CACTTGTGAAAGATCAGAAGCTGGTGTTTGTGTATCTACTAGTGGTAGATGGGTACTTAACAATGATTATTACAGATCTTTA
CCAGGAGTTTTCTGTGGTGTAGATGCTGTAAATTTACTTACTAATATGTTTACACCACTAATTCAACCTATTGGTGCTTTGGA
CATATCAGCATCTATAGTAGCTGGTGGTATTGTAGCTATCGTAGTAACATGCCTTGCCTACTATTTTATGAGGTTTAGAAGA
GCTTTTGGTGAATACAGTCATGTAGTTGCCTTTAATACTTTACTATTCCTTATGTCATTCACTGTACTCTGTTTAACACCAGTT
TACTCATTCTTACCTGGTGTTTATTCTGTTATTTACTTGTACTTGACATTTTATCTTACTAATGATGTTTCTTTTTTAGCACATA
TTCAGTGGATGGTTATGTTCACACCTTTAGTACCTTTCTGGATAACAATTGCTTATATCATTTGTATTTCCACAAAGCATTTCT
ATTGGTTCTTTAGTAATTACCTAAAGAGACGTGTAGTCTTTAATGGTGTTTCCTTTAGTACTTTTGAAGAAGCTGCGCTGTG
CACCTTTTTGTTAAATAAAGAAATGTATCTAAAGTTGCGTAGTGATGTGCTATTACCTCTTACGCAATATAATAGATACTTA
GCTCTTTATAATAAGTACAAGTATTTTAGTGGAGCAATGGATACAACTAGCTACAGAGAAGCTGCTTGTTGTCATCTCGCA
AAGGCTCTCAATGACTTCAGTAACTCAGGTTCTGATGTTCTTTACCAACCACCACAAACCTCTATCACCTCAGCTGTTTTGCA
GAGTGGTTTTAGAAAAATGGCATTCCCATCTGGTAAAGTTGAGGGTTGTATGGTACAAGTAACTTGTGGTACAACTACACT
TAACGGTCTTTGGCTTGATGACGTAGTTTACTGTCCAAGACATGTGATCTGCACCTCTGAAGACATGCTTAACCCTAATTAT
GAAGATTTACTCATTCGTAAGTCTAATCATAATTTCTTGGTACAGGCTGGTAATGTTCAACTCAGGGTTATTGGACATTCTA
 
TGCAAAATTGTGTACTTAAGCTTAAGGTTGATACAGCCAATCCTAAGACACCTAAGTATAAGTTTGTTCGCATTCAACCAGG
ACAGACTTTTTCAGTGTTAGCTTGTTACAATGGTTCACCATCTGGTGTTTACCAATGTGCTATGAGGCCCAATTTCACTATTA
AGGGTTCATTCCTTAATGGTTCATGTGGTAGTGTTGGTTTTAACATAGATTATGACTGTGTCTCTTTTTGTTACATGCACCAT
ATGGAATTACCAACTGGAGTTCATGCTGGCACAGACTTAGAAGGTAACTTTTATGGACCTTTTGTTGACAGGCAAACAGCA
CAAGCAGCTGGTACGGACACAACTATTACAGTTAATGTTTTAGCTTGGTTGTACGCTGCTGTTATAAATGGAGACAGGTGG
TTTCTCAATCGATTTACCACAACTCTTAATGACTTTAACCTTGTGGCTATGAAGTACAATTATGAACCTCTAACACAAGACCA
TGTTGACATACTAGGACCTCTTTCTGCTCAAACTGGAATTGCCGTTTTAGATATGTGTGCTTCATTAAAAGAATTACTGCAA
AATGGTATGAATGGACGTACCATATTGGGTAGTGCTTTATTAGAAGATGAATTTACACCTTTTGATGTTGTTAGACAATGCT
CAGGTGTTACTTTCCAAAGTGCAGTGAAAAGAACAATCAAGGGTACACACCACTGGTTGTTACTCACAATTTTGACTTCACT
TTTAGTTTTAGTCCAGAGTACTCAATGGTCTTTGTTCTTTTTTTGTATGAAAATGCCTTTTTACCTTTTGCTATGGGTATTAT
TGCTATGTCTGCTTTTGCAATGATGTTTGTCAAACATAAGCATGCATTTCTCTGTTTGTTTTTGTTACCTTCTCTTGCCACTGT
AGCTTATTTTAATATGGTCTATATGCCTGCTAGTTGGGTGATGCGTATTATGACATGGTTGGATATGGTTGATACTAGTTTG
AAGCTAAAAGACTGTGTTATGTATGCATCAGCTGTAGTGTTACTAATCCTTATGACAGCAAGAACTGTGTATGATGATGGT
GCTAGGAGAGTGTGGACACTTATGAATGTCTTGACACTCGTTTATAAAGTTTATTATGGTAATGCTTTAGATCAAGCCATTT
CCATGTGGGCTCTTATAATCTCTGTTACTTCTAACTACTCAGGTGTAGTTACAACTGTCATGTTTTTGGCCAGAGGTATTGT
TTTATGTGTGTTGAGTATTGCCCTATTTTCTTCATAACTGGTAATACACTTCAGTGTATAATGCTAGTTTATTGTTTCTTAGGC
TATTTTTGTACTTGTTACTTTGGCCTCTTTTGTTTACTCAACCGCTACTTTAGACTGACTCTTGGTGTTTATGATTACTTAGTTT
CTACACAGGAGTTTAGATATATGAATTCACAGGGACTACTCCCACCCAAGAATAGCATAGATGCCTTCAAACTCAACATTA
AATTGTTGGGTGTTGGTGGCAAACCTTGTATCAAAGTAGCCACTGTACAGTCTAAAATGTCAGATGTAAAGTGCACATCAR
TAGTCTTACTCTCAGTTTTGCAACAACTCAGAGTAGAATCATCATCTAAATTGTGGGCTCAATGTGTCCAGTTACACAATGA
CATTCTCTTAGCTAAAGATACTACTGAAGCCTTTGAAAAAATGGTTTCACTACTTTCTGTTTTGCTTTCCATGCAGGGTGCTG
TAGACATAAACAAGCTTTGTGAAGAAATGCTGGACAACAGGGCAACCTTACAAGCTATAGCCTCAGAGTTTAGTTCCCTTC
CATCATATGCAGCTTTTGCTACTGCTCAAGAAGCTTATGAGCAGGCTGTTGCTAATGGTGATTCTGAAGTTGTTCTTAAAAA
GTTGAAGAAGTCTTTGAATGTGGCTAAATCTGAATTTGACCGTGATGCAGCCATGCAACGTAAGTTGGAAAAGATGGCTG
ATCAAGCTATGACCCAAATGTATAAACAGGCTAGATCTGAGGACAAGAGGGCAAAAGTTACTAGTGCTATGCAGACAATG
CTTTTCACTATGCTTAGAAAGTTGGATAATGATGCACTCAACAACATTATCAACAATGCAAGAGATGGTTGTGTTCCCTTGA
ACATAATACCTCTTACAACAGCAGCCAAACTAATGGTTGTCATACCAGACTATAACACATATAAAAATACGTGTGATGGTA
CAACATTTACTTATGCATCAGCATTGTGGGAAATCCAACAGGTTGTAGATGCAGATAGTAAAATTGTTCAACTTAGTGAAA
TTAGTATGGACAATTCACCTAATTTAGCATGGCCTCTTATTGTAACAGCTTTAAGGGCCAATTCTGCTGTCAAATTACAGAA
TAATGAGCTTAGTCCTGTTGCACTACGACAGATGTCTTGTGCTGCCGGTACTACACAAACTGCTTGCACTGATGACAATGC
GTTAGCTTACTACAACACAACAAAGGGAGGTAGGTTTGTACTTGCACTGTTATCCGATTTACAGGATTTGAAATGGGCTAG
ATTCCCTAAGAGTGATGGAACTGGTACTATCTATACAGAACTGGAACCACCTTGTAGGTTTGTTACAGACACACCTAAAGG
TCCTAAAGTGAAGTATTTATACTTTATTAAAGGATTAAACAACCTAAATAGAGGTATGGTACTTGGTAGTTTAGCTGCCACA
GTACGTCTACAAGCTGGTAATGCAACAGAAGTGCCTGCCAATTCAACTGTATTATCTTTCTGTGCTTTTGCTGTAGATGCTG
CTAAAGCTTACAAAGATTATCTAGCTAGTGGGGGACAACCAATCACTAATTGTGTTAAGATGTTGTGTACACACACTGGTA
CTGGTCAGGCAATAACAGTTACACCGGAAGCCAATATGGATCAAGAATCCTTTGGTGGTGCATCGTGTTGTCTGTACTGCC
GTTGCCACATAGATCATCCAAATCCTAAAGGATTTTGTGACTTAAAAGGTAAGTATGTACAAATACCTACAACTTGTGCTAA
TGACCCTGTGGGTTTTACACTTAAAAACACAGTCTGTACCGTCTGCGGTATGTGGAAAGGTTATGGCTGTAGTTGTGATCA
ACTCCGCGAACCCATGCTTCAGTCAGCTGATGCACAATCGTTTTTAAACGGGTTTGCGGTGTAAGTGCAGCCCGTCTTACA
CCGTGCGGCACAGGCACTAGTACTGATGTCGTATACAGGGCTTTTGACATCTACAATGATAAAGTAGCTGGTTTTGCTAAA
TTCCTAAAAACTAATTGTTGTCGCTTCCAAGAAAAGGACGAAGATGACAATTTAATTGATTCTTACTTTGTAGTTAAGAGAC
ACACTTTCTCTAACTACCAACATGAAGAAACAATTTATAATTTACTTAAGGATTGTCCAGCTGTTGCTAAACATGACTTCTTT
AAGTTTAGAATAGACGGTGACATGGTACCACATATATCACGTCAACGTCTTACTAAATACACAATGGCAGACCTCGTCTAT
GCTTTAAGGCATTTTGATGAAGGTAATTGTGACACATTAAAAGAAATACTTGTCACATACAATTGTTGTGATGATGATTATT
TCAATAAAAAGGACTGGTATGATTTTGTAGAAAACCCAGATATATTACGCGTATACGCCAACTTAGGTGAACGTGTACGCC
AAGCTTTGTTAAAAACAGTACAATTCTGTGATGCCATGCGAAATGCTGGTATTGTTGGTGTACTGACATTAGATAATCAAG
ATCTCAATGGTAACTGGTATGATTTCGGTGATTTCATACAAACCACGCCAGGTAGTGGAGTTCCTGTTGTAGATTCTTATTA
TTCATTGTTAATGCCTATATTAACCTTGACCAGGGCTTTAACTGCAGAGTCACATGTTGACACTGACTTAACAAAGCCTTAC
ATTAAGTGGGATTTGTTAAAATATGACTTCACGGAAGAGAGGTTAAAACTCTTTGACCGTTATTTTAAATATTGGGATCAG
ACATACCACCCAAATTGTGTTAACTGTTTGGATGACAGATGCATTCTGCATTGTGCAAACTTTAATGTTTTATTCTCTACAGT
GTTCCCACTTACAAGTTTTGGACCACTAGTGAGAAAAATATTTGTTGATGGTGTTCCATTTGTAGTTTCAACTGGATACCAC
TTCAGAGAGCTAGGTGTTGTACATAATCAGGATGTAAACTTACATAGCTCTAGACTTAGTTTTAAGGAATTACTTGTGTATG
CTGCTGACCCTGCTATGCACGCTGCTTCTGGTAATCTATTACTAGATAAACGCACTACGTGCTTTTCAGTAGCTGCACTTACT
AACAATGTTGCTTTTCAAACTGTCAAACCTGGTAATTTTAACAAAGACTTCTATGACTTTGCTGTGTCTAAGGGTTTCTTTAA
GGAAGGAAGTTCTGTTGAATTAAAACACTTCTTCTTTGCTCAGGATGGTAATGCTGCTATCAGCGATTATGACTACTATCGT
 
TATAATCTACCAACAATGTGTGATATCAGACAACTACTATTTGTAGTTGAAGTTGTTGATAAGTACTTTGATTGTTACGATG
GTGGCTGTATTAATGCTAACCAAGTCATCGTCAACAACCTAGACAAATCAGCTGGTTTTCCATTTAATAAATGGGGTAAGG
CTAGACTTTATTATGATTCAATGAGTTATGAGGATCAAGATGCACTTTTCGCATATACAAAACGTAATGTCATCCCTACTAT
AACTCAAATGAATCTTAAGTATGCCATTAGTGCAAAGAATAGAGCTCGCACCGTAGCTGGTGTCTCTATCTGTAGTACTAT
GACCAATAGACAGTTTCATCAAAAATTATTGAAATCAATAGCCGCCACTAGAGGAGCTACTGTAGTAATTGGAACAAGCAA
ATTCTATGGTGGTTGGCACAACATGTTAAAAACTGTTTATAGTGATGTAGAAAACCCTCATCTTATGGGTTGGGATTATCCT
AAATGTGATAGAGCCATGCCTAACATGCTTAGAATTATGGCCTCACTTGTTCTTGCTCGCAAACATACAACGTGTTGTAGCT
TGTCACACCGTTTCTATAGATTAGCTAATGAGTGTGCTCAAGTATTGAGTGAAATGGTCATGTGTGGCGGTTCACTATATGT
TAAACCAGGTGGAACCTCATCAGGAGATGCCACAACTGCTTATGCTAATAGTGTTTTTAACATTTGTCAAGCTGTCACGGC
CAATGTTAATGCACTTTTATCTACTGATGGTAACAAAATTGCCGATAAGTATGTCCGCAATTTACAACACAGACTTTATGAG
TGTCTCTATAGAAATAGAGATGTTGACACAGACTTTGTGAATGAGTTTTACGCATATTTGCGTAAACATTTCTCAATGATGA
TACTCTCTGACGATGCTGTTGTGTGTTTCAATAGCACTTATGCATCTCAAGGTCTAGTGGCTAGCATAAAGAACTTTAAGTC
AGTTCTTTATTATCAAAACAATGTTTTTATGTCTGAAGCAAAATGTTGGACTGAGACTGACCTTACTAAAGGACCTCATGAA
TTTTGCTCTCAACATACAATGCTAGTTAAACAGGGTGATGATTATGTGTACCTTCCTTACCCAGATCCATCAAGAATCCTAG
GGGCCGGCTGTTTTGTAGATGATATCGTAAAAACAGATGGTACACTTATGATTGAACGGTTCGTGTCTTTAGCTATAGATG
CTTACCCACTTACTAAACATCCTAATCAGGAGTATGCTGATGTCTTTCATTTGTACTTACAATACATAAGAAAGCTACATGAT
GAGTTAACAGGACACATGTTAGACATGTATTCTGTTATGCTTACTAATGATAACACCTCAAGGTATTGGGAACCTGAGTTTT
ATGAGGCTATGTACACACCGCATACAGTCTTACAGGCTGTTGGGGCTTGTGTTCTTTGCAATTCACAGACTTCATTAAGATG
TGGTGCTTGCATACGTAGACCATTCTTATGTTGTAAATGCTGTTACGACCATGTCATATCAACATCACATAAATTAGTCTTGT
CTGTTAATCCGTATGTTTGCAATGCTCCAGGTTGTGATGTCACAGATGTGACTCAACTTTACTTAGGAGGTATGAGCTATTA
TTGTAAATCACATAAACCACCCATTAGTTTTCCATTGTGTGCTAATGGACAAGTTTTTGGTTTATATAAAAATACATGTGTTG
GTAGCGATAATGTTACTGACTTTAATGCAATTGCAACATGTGACTGGACAAATGCTGGTGATTACATTTTAGCTAACACCTG
TACTGAAAGACTCAAGCTTTTTGCAGCAGAAACGCTCAAAGCTACTGAGGAGACATTTAAACTGTCTTATGGTATTGCTAC
TGTACGTGAAGTGCTGTCTGACAGAGAATTACATCTTTCATGGGAAGTTGGTAAACCTAGACCACCACTTAACCGAAATTA
TGTCTTTACTGGTTATCGTGTAACTAAAAACAGTAAAGTACAAATAGGAGAGTACACCTTTGAAAAAGGTGACTATGGTGA
TGCTGTTGTTTACCGAGGTACAACAACTTACAAATTAAATGTTGGTGATTATTTTGTGCTGACATCACATACAGTAATGCCA
TTAAGTGCACCTACACTAGTGCCACAAGAGCACTATGTTAGAATTACTGGCTTATACCCAACACTCAATATCTCAGATGAG
TTTTCTAGCAATGTTGCAAATTATCAAAAGGTTGGTATGCAAAAGTATTCTACACTCCAGGGACCACCTGGTACTGGTAAG
AGTCATTTTGCTATTGGCCTAGCTCTCTACTACCCTTCTGCTCGCATAGTGTATACAGCTTGCTCTCATGCCGCTGTTGATGC
ACTATGTGAGAAGGCATTAAAATATTTGCCTATAGATAAATGTAGTAGAATTATACCTGCACGTGCTCGTGTAGAGTGTTT
TGATAAATTCAAAGTGAATTCAACATTAGAACAGTATGTCTTTTGTACTGTAAATGCATTGCCTGAGACGACAGCAGATAT
AGTTGTCTTTGATGAAATTTCAATGGCCACAAATTATGATTTGAGTGTTGTCAATGCCAGATTACGTGCTAAGCACTATGTG
TACATTGGCGACCCTGCTCAATTACCTGCACCACGCACATTGCTAACTAAGGGCACACTAGAACCAGAATATTTCAATTCAG
TGTGTAGACTTATGAAAACTATAGGTCCAGACATGTTCCTCGGAACTTGTCGGCGTTGTCCTGCTGAAATTGTTGACACTGT
GAGTGCTTTGGTTTATGATAATAGGCTTAAAGCACATAAAGACAAATCAGCTCAATGCTTTAAAATGTTTTATAAGGGTGT
TATCACGCATGATGTTTCATCTGCAATTAACAGGCCACAAATAGGCGTGGTAAGAGAATTCCTTACACGTAACCCTGCTTG
GAGAAAAGCTGTCTTTATTTCACCTTATAATTCACAGAATGCTGTAGCCTCAAAGATTTTGGGACTACCAACTCAAACTGTT
GATTCATCACAGGGCTCAGAATATGACTATGTCATATTCACTCAAACCACTGAAACAGCTCACTCTTGTAATGTAAACAGAT
TTAATGTTGCTATTACCAGAGCAAAAGTAGGCATACTTTGCATAATGTCTGATAGAGACCTTTATGACAAGTTGCAATTTAC
AAGTCTTGAAATTCCACGTAGGAATGTGGCAACTTTACAAGCTGAAAATGTAACAGGACTCTTTAAAGATTGTAGTAAGGT
AATCACTGGGTTACATCCTACACAGGCACCTACACACCTCAGTGTTGACACTAAATTCAAAACTGAAGGTTTATGTGTTGAC
ATACCTGGCATACCTAAGGACATGACCTATAGAAGACTCATCTCTATGATGGGTTTTAAAATGAATTATCAAGTTAATGGTT
ACCCTAACATGTTTATCACCCGCGAAGAAGCTATAAGACATGTACGTGCATGGATTGGCTTCGATGTCGAGGGGTGTCATG
CTACTAGAGAAGCTGTTGGTACCAATTTACCTTTACAGCTAGGTTTTTCTACAGGTGTTAACCTAGTTGCTGTACCTACAGG
TTATGTTGATACACCTAATAATACAGATTTTTCCAGAGTTAGTGCTAAACCACCGCCTGGAGATCAATTTAAACACCTCATA
CCACTTATGTACAAAGGACTTCCTTGGAATGTAGTGCGTATAAAGATTGTACAAATGTTAAGTGACACACTTAAAAATCTCT
CTGACAGAGTCGTATTTGTCTTATGGGCACATGGCTTTGAGTTGACATCTATGAAGTATTTTGTGAAAATAGGACCTGAGC
GCACCTGTTGTCTATGTGATAGACGTGCCACATGCTTTTCCACTGCTTCAGACACTTATGCCTGTTGGCATCATTCTATTGGA
TTTGATTACGTCTATAATCCGTTTATGATTGATGTTCAACAATGGGGTTTTACAGGTAACCTACAAAGCAACCATGATCTGT
ATTGTCAAGTCCATGGTAATGCACATGTAGCTAGTTGTGATGCAATCATGACTAGGTGTCTAGCTGTCCACGAGTGCTTTG
TTAAGCGTGTTGACTGGACTATTGAATATCCTATAATTGGTGATGAACTGAAGATTAATGCGGCTTGTAGAAAGGTTCAAC
ACATGGTTGTTAAAGCTGCATTATTAGCAGACAAATTCCCAGTTCTTCACGACATTGGTAACCCTAAAGCTATTAAGTGTGT
ACCTCAAGCTGATGTAGGATGGAAGTTCTATGATGCACAGCCTTGTAGTGACAAAGCTTATAAAATAGAAGAATTATTCTA
TTCTTATGCCACACATTCTGACAAATTCACAGATGGTGTATGCCTATTTTGGAATTGCAATGTCGATAGATATCCTGCTAATT
CCATTGTTTGTAGATTTGACACTAGAGTGCTATCTAACCTTAACTTGCCTGGTTGTGATGGTGGCAGTTTGTATGTAAATAA
 
ACATGCATTCCACACACCAGCTTTTGATAAAAGTGCTTTTGTTAATTTAAAACAATTACCATTTTTCTATTACTCTGACAGTCC
ATGTGAGTCTCATGGAAAACAAGTAGTGTCAGATATAGATTATGTACCACTAAAGTCTGCTACGTGTATAACACGTTGCAA
TTTAGGTGGTGCTGTCTGTAGACATCATGCTAATGAGTACAGATTGTATCTCGATGCTTATAACATGATGATCTCAGCTGGC
TTTAGCTTGTGGGTTTACAAACAATTTGATACTTATAACCTCTGGAACACTTTTACAAGACTTCAGAGTTTAGAAAATGTGG
CTTTTAATGTTGTAAATAAGGGACACTTTGATGGACAACAGGGTGAAGTACCAGTTTCTATCATTAATAACACTGTTTACAC
AAAAGTTGATGGTGTTGATGTAGAATTGTTTGAAAATAAAACAACATTACCTGTTAATGTAGCATTTGAGCTTTGGGCTAA
GCGCAACATTAAACCAGTACCAGAGGTGAAAATACTCAATAATTTGGGTGTGGATATTGCTGCTAATACTGTGATCTGGGA
CTACAAAAGAGATGCTCCAGCACATATATCTACTATTGGTGTTTGTTCTATGACTGACATAGCCAAGAAACCAACTGAAAC
GATTTGTGCACCACTCACTGTCTTTTTTGATGGTAGAGTTGATGGTCAAGTAGACTTATTTAGAAATGCCCGTAATGGTGTT
CTTATTACAGAAGGTAGTGTTAAAGGTTTACAACCATCTGTAGGTCCCAAACAAGCTAGTCTTAATGGAGTCACATTAATT
GGAGAAGCCGTAAAAACACAGTTCAATTATTATAAGAAAGTTGATGGTGTTGTCCAACAATTACCTGAAACTTACTTTACTC
AGAGTAGAAATTTACAAGAATTTAAACCCAGGAGTCAAATGGAAATTGATTTCTTAGAATTAGCTATGGATGAATTCATTG
AACGGTATAAATTAGAAGGCTATGCCTTCGAACATATCGTTTATGGAGATTTTAGTCATAGTCAGTTAGGTGGTTTACATCT
ACTGATTGGACTAGCTAAACGTTTTAAGGAATCACCTTTTGAATTAGAAGATTTTATTCCTATGGACAGTACAGTTAAAAAC
TATTTCATAACAGATGCGCAAACAGGTTCATCTAAGTGTGTGTGTTCTGTTATTGATTTATTACTTGATGATTTTGTTGAAAT
AATAAAATCCCAAGATTTATCTGTAGTTTCTAAGGTTGTCAAAGTGACTATTGACTATACAGAAATTTCATTTATGCTTTGTG
TAAAGATGGCCATGTAGAAACATTTTACCCAAAATTACAATCTAGTCAAGCGTGGCAACCGGGTGTTGCTATGCCTAATCT
TTACAAAATGCAAAGAATGCTATTAGAAAAGTGTGACCTTCAAAATTATGGTGATAGTGCAACATTACCTAAAGGCATAAT
GATGAATGTCGCAAAATATACTCAACTGTGTCAATATTTAAACACATTAACATTAGCTGTACCCTATAATATGAGAGTTATA
CATTTTGGTGCTGGTTCTGATAAAGGAGTTGCACCAGGTACAGCTGTTTTAAGACAGTGGTTGCCTACGGGTACGCTGCTT
GTCGATTCAGATCTTAATGACTTTGTCTCTGATGCAGATTCAACTTTGATTGGTGATTGTGCAACTGTACATACAGCTAATA
AATGGGATCTCATTATTAGTGATATGTACGACCCTAAGACTAAAAATGTTACAAAAGAAAATGACTCTAAAGAGGGTTTTT
TCACTTACATTTGTGGGTTTATACAACAAAAGCTAGCTCTTGGAGGTTCCGTGGCTATAAAGATAACAGAACATTCTTGGA
ATGCTGATCTTTATAAGCTCATGGGACACTTCGCATGGTGGACAGCCTTTGTTACTAATGTGAATGCGTCATCATCTGAAGC
ATTTTTAATTGGATGTAATTATCTTGGCAAACCACGCGAACAAATAGATGGTTATGTCATGCATGCAAATTACATATTTTGG
AGGAATACAAATCCAATTCAGTTGTCTTCCTATTCTTTATTTGACATGAGTAAATTTCCCCTTAAATTAAGGGGTACTGCTGT
TATGTCTTTAAAAGAAGGTCAAATCAATGATATGATTTTATCTCTTCTTAGTAAAGGTAGACTTATAATTAGAGAAAACAAC
AGAGTTGTTATTTCTAGTGATGTTCTTGTTAACAACTAAACGAACAATGTTTGTTTTTCTTGTTTTATTGCCACTAGTCTCTAG
TCAGTGTGTTAATCTTACAACCAGAACTCAATTACCCCCTGCATACACTAATTCTTTCACACGTGGTGTTTATTACCCTGACA
AAGTTTTCAGATCCTCAGTTTTACATTCAACTCAGGACTTGTTCTTACCTTTCTTTTCCAATGTTACTTGGTTCCATGCTATCT
CTGGGACCAATGGTACTAAGAGGTTTGATAACCCTGTCCTACCATTTAATGATGGTGTTTATTTTGCTTCCACTGAGAAGTC
TAACATAATAAGAGGCTGGATTTTTGGTACTACTTTAGATTCGAAGACCCAGTCCCTACTTATTGTTAATAACGCTACTAAT
GTTGTTATTAAAGTCTGTGAATTTCAATTTTGTAATGATCCATTTTTGGGTGTTTACCACAAAAACAACAAAAGTTGGATGG
AAAGTGAGTTCAGAGTTTATTCTAGTGCGAATAATTGCACTTTTGAATATGTCTCTCAGCCTTTTCTTATGGACCTTGAAGG
AAAACAGGGTAATTTCAAAAATCTTAGGGAATTTGTGTTTAAGAATATTGATGGTTATTTTAAAATATATTCTAAGCACACG
CCTATTAATTTAGTGCGTGATCTCCCTCAGGGTTTTTCGGCTTTAGAACCATTGGTAGATTTGCCAATAGGTATTAACATCAC
TAGGTTTCAAACTTTACTTGCTTTACATAGAAGTTATTTGACTCCTGGTGATTCTTCTTCAGGTTGGACAGCTGGTGCTGCA
GCTTATTATGTGGGTTATCTTCAACCTAGGACTTTTCTATTAAAATATAATGAAAATGGAACCATTACAGATGCTGTAGACT
GTGCACTTGACCCTCTCTCAGAAACAAAGTGTACGTTGAAATCCTTCACTGTAGAAAAAGGAATCTATCAAACTTCTAACTT
TAGAGTCCAACCAACAGAATCTATTGTTAGATTTCCTAATATTACAAACTTGTGCCCTTTTGGTGAAGTTTTTAACGCCACCA
GATTTGCATCTGTTTATGCTTGGAACAGGAAGAGAATCAGCAACTGTGTTGCTGATTATTCTGTCCTATATAATTCCGCATC
ATTTTCCACTTTTAAGTGTTATGGAGTGTCTCCTACTAAATTAAATGATCTCTGCTTTACTAATGTCTATGCAGATTCATTTGT
AATTAGAGGTGATGAAGTCAGACAAATCGCTCCAGGGCAAACTGGAAAGATTGCTGATTATAATTATAAATTACCAGATG
ATTTTACAGGCTGCGTTATAGCTTGGAATTCTAACAATCTTGATTCTAAGGTTGGTGGTAATTATAATTACCTGTATAGATT
GTTTAGGAAGTCTAATCTCAAACCTTTTGAGAGAGATATTTCAACTGAAATCTATCAGGCCGGTAGCACACCTTGTAATGG
TGTTGAAGGTTTTAATTGTTACTTTCCTTTACAATCATATGGTTTCCAACCCACTTATGGTGTTGGTTACCAACCATACAGAG
TAGTAGTACTTTCTTTTGAACTTCTACATGCACCAGCAACTGTTTGTGGACCTAAAAAGTCTACTAATTTGGTTAAAAACAA
ATGTGTCAATTTCAACTTCAATGGTTTAACAGGCACAGGTGTTCTTACTGAGTCTAACAAAAAGTTTCTGCCTTTCCAACAA
TTTGGCAGAGACATTGATGACACTACTGATGCTGTCCGTGATCCACAGACACTTGAGATTCTTGACATTACACCATGTTCTT
TTGGTGGTGTCAGTGTTATAACACCAGGAACAAATACTTCTAACCAGGTTGCTGTTCTTTATCAGGGTGTTAACTGCACAG
AAGTCCCTGTTGCTATTCATGCAGATCAACTTACTCCTACTTGGCGTGTTTATTCTACAGGTTCTAATGTTTTCAAACACGT
GCAGGCTGTTTAATAGGGGCTGAACATGTCAACAACTCATATGAGTGTGACATACCCATTGGTGCAGGTATATGCGCTAGT
TATCAGACTCAGACTAATTCTCATCGGCGGGCACGTAGTGTAGCTAGTCAATCCATCATTGCCTACACTATGTCACTTGGTG
CAGAAAATTCAGTTGCTTACTCTAATAACTCTATTGCCATACCCATAAATTTTACTATTAGTGTTACCACAGAAATTCTACCA
GTGTCTATGACCAAGACATCAGTAGATTGTACAATGTACATTTGTGGTGATTCAACTGAATGCAGCAATCTTTTGTTGCAAT
 
ATGGCAGTTTTTGTACACAATTAAACCGTGCTTTAACTGGAATAGCTGTTGAACAAGACAAAAACACCCAAGAAGTTTTTG
CACAAGTCAAACAAATTTACAAAACACCACCAATTAAAGATTTTGGTGGTTTTAATTTTTCACAAATATTACCAGATCCATCA
AAACCAAGCAAGAGGTCATTTATTGAAGATCTACTTTTCAACAAAGTGACACTTGCAGATGCTGGCTTCATCAAACAATAT
GGTGATTGCCTTGGTGATATTGCTGCTAGAGACCTCATTTGTGCACAAAAGTTTAACGGCCTTACTGTTTTGCCACCTTTGC
TCACAGATGAAATGATTGCTCAATACACTTCTGCACTGTTAGCGGGTACAATCACTTCTGGTTGGACCTTTGGTGCAGGTG
CTGCATTACAAATACCATTTGCTATGCAAATGGCTTATAGGTTTAATGGTATTGGAGTTACACAGAATGTTCTCTATGAGAA
CCAAAAATTGATTGCCAACCAATTTAATAGTGCTATTGGCAAAATTCAAGACTCACTTTCTTCCACAGCAAGTGCACTTGGA
AAACTTCAAGATGTGGTCAACCAAAATGCACAAGCTTTAAACACGCTTGTTAAACAACTTAGCTCCAATTTTGGTGCAATTT
CAAGTGTTTTAAATGATATCCTTGCACGTCTTGACAAAGTTGAGGCTGAAGTGCAAATTGATAGGTTGATCACAGGCAGAC
TTCAAAGTTTGCAGACATATGTGACTCAACAATTAATTAGAGCTGCAGAAATCAGAGCTTCTGCTAATCTTGCTGCTACTAA
AATGTCAGAGTGTGTACTTGGACAATCAAAAAGAGTTGATTTTTGTGGAAAGGGCTATCATCTTATGTCCTTCCCTCAGTCA
GCACCTCATGGTGTAGTCTTCTTGCATGTGACTTATGTCCCTGCACAAGAAAAGAACTTCACAACTGCTCCTGCCATTTGTC
ATGATGGAAAAGCACACTTTCCTCGTGAAGGTGTCTTTGTTTCAAATGGCACACACTGGTTTGTAACACAAAGGAATTTTTA
TGAACCACAAATCATTACTACACACAACACATTTGTGTCTGGTAACTGTGATGTTGTAATAGGAATTGTCAACAACACAGTT
TATGATCCTTTGCAACCTGAATTAGACTCATTCAAGGAGGAGTTAGATAAATATTTTAAGAATCATACATCACCAGATGTTG
ATTTAGGTGACATCTCTGGCATTAATGCTTCAGTTGTAAACATTCAAAAAGAAATTGACCGCCTCAATGAGGTTGCCAAGA
ATTTAAATGAATCTCTCATCGATCTCCAAGAACTTGGAAAGTATGAGCAGTATATAAAATGGCCATGGTACATTTGGCTAG
GTTTTATAGCTGGCTTGATTGCCATAGTAATGGTGACAATTATGCTTTGCTGTATGACCAGTTGCTGTAGTTGTCTCAAGGG
CTGTTGTTCTTGTGGATCCTGCTGCAAATTTGATGAAGACGACTCTGAGCCAGTGCTCAAAGGAGTCAAATTACATTACAC
ATAAACGAACTTATGGATTTGTTTATGAGAATCTTCACAATTGGAACTGTAACTTTGAAGCAAGGTGAAATCAAGGATGCT
ACTCCTTCAGATTTTGTTCGCGCTACTGCAACGATACCGATACAAGCCTCACTCCCTTTCGGATGGCTTATTGTTGGCGTTGC
ACTTCTTGCTGTTTTTCAGAGCGCTTCCAAAATCATAACCCTCAAAAAGAGATGGCAACTAGCACTCTCCAAGGGTGTTCAC
TTTGTTTGCAACTTGCTGTTGTTGTTTGTAACAGTTTACTCACACCTTTTGCTCGTTGCTGCTGGCCTTGAAGCCCCTTTTCTC
TATCTTTATGCTTTAGTCTACTTCTTGCAGAGTATAAACTTTGTAAGAATAATAATGAGGCTTTGGCTTTGCTGGAAATGCC
GTTCCAAAAACCCATTACTTTATGATGCCAACTATTTTCTTTGCTGGCATACTAATTGTTACGACTATTGTATACCTTACAATA
GTGTAACTTCTTCAATTGTCATTACTTCAGGTGATGGCACAACAAGTCCTATTTCTGAACATGACTACCAGATTGGTGGTTA
TACTGAAAAATGGGAATCTGGAGTAAAAGACTGTGTTGTATTACACAGTTACTTCACTTCAGACTATTACCAGCTGTACTCA
ACTCAATTGAGTACAGACACTGGTGTTGAACATGTTACCTTCTTCATCTACAATAAAATTGTTGATGAGCCTGAAGAACATG
TCCAAATTCACACAATCGACGGTTCATCCGGAGTTGTTAATCCAGTAATGGAACCAATTTATGATGAACCGACGACGACTA
CTAGCGTGCCTTTGTAAGCACAAGCTGATGAGTACGAACTTATGTACTCATTCGTTTCGGAAGAGACAGGTACGTTAATAG
TTAATAGCGTACTTCTTTTTCTTGCTTTCGTGGTATTCTTGCTAGTTACACTAGCCATCCTTACTGCGCTTCGATTGTGTGCGT
ACTGCTGCAATATTGTTAACGTGAGTCTTGTAAAACCTTCTTTTTACGTTTACTCTCGTGTTAAAAATCTGAATTCTTCTAGA
GTTCCTGATCTTCTGGTCTAAACGAACTAAATATTATATTAGTTTTTCTGTTTGGAACTTTAATTTTAGCCATGGCAGATTCC
AACGGTACTATTACCGTTGAAGAGCTTAAAAAGCTCCTTGAACAATGGAACCTAGTAATAGGTTTCCTATTCCTTACATGGA
TTTGTCTTCTACAATTTGCCTATGCCAACAGGAATAGGTTTTTGTATATAATTAAGTTAATTTTCCTCTGGCTGTTATGGCCA
GTAACTTTAGCTTGTTTTGTGCTTGCTGCTGTTTACAGAATAAATTGGATCACCGGTGGAATTGCTATCGCAATGGCTTGTC
TTGTAGGCTTGATGTGGCTCAGCTACTTCATTGCTTCTTTCAGACTGTTTGCGCGTACGCGTTCCATGTGGTCATTCAATCCA
GAAACTAACATTCTTCTCAACGTGCCACTCCATGGCACTATTCTGACCAGACCGCTTCTAGAAAGTGAACTCGTAATCGGAG
CTGTGATCCTTCGTGGACATCTTCGTATTGCTGGACACCATCTAGGACGCTGTGACATCAAGGACCTGCCTAAAGAAATCA
CTGTTGCTACATCACGAACGCTTTCTTATTACAAATTGGGAGCTTCGCAGCGTGTAGCAGGTGACTCAGGTTTTGCTGCAT
ACAGTCGCTACAGGATTGGCAACTATAAATTAAACACAGACCATTCCAGTAGCAGTGACAATATTGCTTTGCTTGTACAGT
AAGTGACAACAGATGTTTCATCTCGTTGACTTTCAGGTTACTATAGCAGAGATATTACTAATTATTATGAGGACTTTTAAAG
TTTCCATTTGGAATCTTGATTACATCATAAACCTCATAATTAAAAATTTATCTAAGTCACTAACTGAGAATAAATATTCTCAA
TTAGATGAAGAGCAACCAATGGAGATTGATTAAACGAACATGAAAATTATTCTTTTCTTGGCACTGATAACACTCGCTACTT
GTGAGCTTTATCACTACCAAGAGTGTGTTAGAGGTACAACAGTACTTTTAAAAGAACCTTGCTCTTCTGGAACATACGAGG
GCAATTCACCATTTCATCCTCTAGCTGATAACAAATTTGCACTGACTTGCTTTAGCACTCAATTTGCTTTTGCTTGTCCTGAC
GGCGTAAAACACGTCTATCAGTTACGTGCCAGATCAGTTTCACCTAAACTGTTCATCAGACAAGAGGAAGTTCAAGAACTT
TACTCTCCAATTTTTCTTATTGTTGCGGCAATAGTGTTTATAACACTTTGCTTCACACTCAAAAGAAAGACAGAATGATTGAA
CTTTCATTAATTGACTTCTATTTGTGCTTTTTAGCCTTTCTGCTATTCCTTGTTTTAATTATGCTTATTATCTTTTGGTTCTCACT
TGAACTGCAAGATCATAATGAAACTTGTCACGCCTAAACGAACATGAAATTTCTTGTTTTCTTAGGAATCATCACAACTGTA
GCTGCATTTCACCAAGAATGTAGTTTACAGTCATGTACTTAACATCAACCATATGTAGTTGATGACCCGTGTCCTATTCACTT
CTATTCTAAATGGTATATTAGAGTAGGAGCTATAAAATCAGCACCTTTAATTGAATTGTGCGTGGATGAGGCTGGTTCTAA
ATCACCCATTCAGTGCATCGATATCGGTAATTATACAGTTTCCTGTTTACCTTTTACAATTAATTGCCAGGAACCTAAATTGG
GTAGTCTTGTAGTGCGTTGTTCGTTCTATGAAGACTTTTTAGAGTATCATGACGTTCGTGTTGTTTTAGATTTCATCTAAACG
AACAAACTAAATGTCTCTAAATGGACCCCAAAATCAGCGAAATGCACCCCGCATTACGTTTGGTGGACCCTCAGATTCAAC
 
TGGCAGTAACCAGAATGGAGAACGCAGTGGGGCGATCAAAACAACGTCGGCCCCAAGGTTTACCCAATAATACTGCGT
CTTGGTTCACCGCTCTCACTCAACATGGCAAGGAAGACCTTAAATTCCCTCGAGGACAAGGCGTTCCAATTAACACCAATA
GCAGTCCAGATGACCAAATTGGCTACTACCGAAGAGCTACCAGACGAATTCGTGGTGGTGACGGTAAAATGAAAGATCTC
AGTCCAAGATGGTATTTCTACTACCTAGGAACTGGGCCAGAAGCTGGACTTCCCTATGGTGCTAACAAAGACGGCATCATA
TGGGTTGCAACTGAGGGAGCCTTGAATACACCAAAAGATCACATTGGCACCCGCAATCCTGCTAACAATGCTGCAATCGTG
CTACAACTTCCTCAAGGAACAACATTGCCAAAAGGCTTCTACGCAGAAGGGAGCAGAGGCGGCAGTCAAGCCTCTTCTCG
TTCCTCATCACGTAGTCGCAACAGTTCAAGAAATTCAACTCCAGGCAGCAGTAAACGAACTTCTCCTGCTAGAATGGCTGG
CAATGGCGGTGATGCTGCTCTTGCTTTGCTGCTGCTTGACAGATTGAACCAGCTTGAGAGCAAAATGTTTGGTAAAGGCCA
ACAACAACAAGGCCAAACTGTCACTAAGAAATCTGCTGCTGAGGCTTCTAAGAAGCCTCGGCAAAAACGTACTGCCACTA
AAGCATACAATGTAACACAAGCTTTCGGCAGACGTGGTCCAGAACAAACCCAAGGAAATTTTGGGGACCAGGAACTAATC
AGACAAGGAACTGATTACAAACATTGGCCGCAAATTGCACAATTTGCCCCCAGCGCTTCAGCGTTCTTCGGAATGTCGCGC
ATTGGCATGGAAGTCACACCTTCGGGAACGTGGTTGACCTACACAGGTGCCATCAAATTGGATGACAAAGATCCAAATTTC
AAAGATCAAGTCATTTTGCTGAATAAGCATATTGACGCATACAAAACATTCCCACCAACAGAGCCTAAAAAGGACAAAAAG
AAGAAGGCTGATGAAACTCAAGCCTTACCGCAGAGACAGAAGAAACAGCAAACTGTGACTCTTCTTCCTGCTGCAGATTT
GGATGATTTCTCCAAACAATTGCAACAATCCATGAGCAGTGCTGACTCAACTCAGGCCTAAACTCATGCAGACCACACAAG
GCAGATGGGCTATATAAACGTTTTCGCTTTTCCGTTTACGATATATAGTCTACTCTTGTGCAGAATGAATTCTCGTAACTACA
TAGCACAAGTAGATGTAGTTAACTTTAATCTCACATAGCAATCTTTAATCAGTGTGTAACATTAGGGAGGACTTGAAAGAG
CCACCACATTTTCACCGAGGCCACGCGGAGTACGATCGAGTGTACAGTGAACAATGCTAGGGAGAGCTGCCTATATGGAA
GAGCCCTAATGTGTAAAATTAATTTTAGTAGTGCTATCCCCATGTGATTTTAATAGCTTCTTAGGAGAATGNNNNNNNNN
NNNNNNNNNNNNNNNNNNNNNNNNN
SEQ ID NO: 23
   > MIG457_gp02 QQH18545.1 surface glycoprotein, from UK_MIG457genome accession MW422256 
MFVFLVLLPLVSSQCVNLTTRTQLPPAYTNSFTRGVYYPDKVFRSSVLHSTQDLFLPFFSNVTWFHAISGTNGTKRFDNPVLPFN
DGVYFASTEKSNIIRGWIFGTTLDSKTQSLLIVNNATNVVIKVCEFQFCNDPFLGVYHKNNKSWMESEFRVYSSANNCTFEYVSQ
PFLMDLEGKQGNFKNLREFVFKNIDGYFKIYSKHTPINLVRDLPQGFSALEPLVDLPIGINITRFQTLLALHRSYLTPGDSSSGWTA
GAAAYYVGYLQPRTFLLKYNENGTITDAVDCALDPLSETKCTLKSFTVEKGIYQTSNFRVQPTESIVRFPNITNLCPFGEVFNATRF
ASVYAWNRKRISNCVADYSVLYNSASFSTFKCYGVSPTKLNDLCFTNVYADSFVIRGDEVRQIAPGQTGKIADYNYKLPDDFTGC
VIAWNSNNLDSKVGGNYNYLYRLFRKSNLKPFERDISTEIYQAGSTPCNGVEGFNCYFPLQSYGFQPTYGVGYQPYRVVVLSFEL
LHAPATVCGPKKSTNLVKNKCVNFNFNGLTGTGVLTESNKKFLPFQQFGRDIDDTTDAVRDPQTLEILDITPCSFGGVSVITPGT
NTSNQVAVLYQGVNCTEVPVAIHADQLTPTWRVYSTGSNVFQTRAGCLIGAEHVNNSYECDIPIGAGICASYQTQTNSHRRAR
SVASQSIIAYTMSLGAENSVAYSNNSIAIPINFTISVTTEILPVSMTKTSVDCTMYICGDSTECSNLLLQYGSFCTQLNRALTGIAVE
QDKNTQEVFAQVKQIYKTPPIKDFGGFNFSQILPDPSKPSKRSFIEDLLFNKVTLADAGFIKQYGDCLGDIAARDLICAQKFNGLTV
LPPLLTDEMIAQYTSALLAGTITSGWTFGAGAALQIPFAMQMAYRFNGIGVTQNVLYENQKLIANQFNSAIGKIQDSLSSTASAL
GKLQDVVNQNAQALNTLVKQLSSNFGAISSVLNDILARLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHFPREGVFVSNGTHWFVTQRNFYEP
QIITTHNTFVSGNCDVVIGIVNNTVYDPLQPELDSFKEELDKYFKNHTSPDVDLGDISGINASVVNIQKEIDRLNEVAKNLNESLID
LQELGKYEQYIKWPWYIWLGFIAGLIAIVMVTIMLCCMTSCCSCLKGCCSCGSCCKFDEDDSEPVLKGVKLHYT
SEQ ID NO: 24
   >MW493681.1 Severe acute respiratory syndrome coronavirus 2 isolate SARS-CoV-2/human/USA/NMDOH-2021013232/2021, complete genome. [Severe acute respiratory syndrome coronavirus 2 (SARS-CoV-2)], Californian B.1.427 lineage 
AAGGTTTATACCTTCCCAGGTAACAAACCAACCAACTTTCGATCTCTTGTAGATCTGTTCTCTAAACGAACTTTAAAATCTGT
GTGGCTGTCACTCGGCTGCATGCTTAGTGCACTCACGCAGTATAATTAATAACTAATTACTGTCGTTGACAGGACACGAGT
AACTCGTCTATCTTCTGCAGGCTGCTTACGGTTTCGTCCGTGTTGCAGCCGATCATCAGCACATCTAGGTTTTGTCCGGGTG
TGACCGAAAGGTAAGATGGAGAGCCTTGTCCCTGGTTTCAACGAGAAAACACACGTCCAACTCAGTTTGCCTGTTTTACAG
GTTCGCGACGTGCTCGTACGTGGCTTTGGAGACTCCGTGGAGGAGGTCTTATCAGAGGCACGTCAACATCTTAAAGATGG
CACTTGTGGCTTAGTAGAAGTTGAAAAAGGCGTTTTGCCTCAACTTGAACAGCCCTATGTGTTCATCAAACGTTCGGATGC
TCGAACTGCACCTCATGGTCATGTTATGGTTGAGCTGGTAGCAGAACTCGAAGGCATTCAGTACGGTCGTAGTGGTGAGA
CACTTGGTGTCCTTGTCCCTCATGTGGGCGAAATACCAGTGGCTTACCGCAAGGTTCTTCTTCGTAAGAACGGTAATAAAG
GAGCTGGTGGCCATAGTTACGGCGCCGATCTAAAGTCATTTGACTTAGGCGACGAGCTTGGCACTGATCCTTATGAAGATT
TTCAAGAAAACTGGAACACTAAACATAGCAGTGGTGTTACCCGTGAACTCATGCGTGAGCTTAACGGAGGGGCATACACT
CGCTATGTCGATAACAACTTCTGTGGCCCTGATGGCTACCCTCTTGAGTGCATTAAAGACCTTCTAGCACGTGCTGGTAAA
 
GCTTCATGCACTTTGTCCGAACAACTGGACTTTATTGACACTAAGAGGGGTGTATACTGCTGCCGTGAACATGAGCATGAA
ATTGCTTGGTACACGGAACGTTCTGAAAAGAGCTATGAATTGCAGACACCTTTTGAAATTAAATTGGCAAAGAAATTT
GACATCTTCAATGGGGAATGTCCAAATTTTGTATTTCCCTTAAATTCCATAATCAAGACTATTCAACCAAGGGTTGAAAAGA
AAAAGCTTGATGGCTTTATGGGTAGAATTCGATCTGTCTATCCAGTTGCGTCACCAAATGAATGCAACCAAATGTGCCTTTC
AACTCTCATGAAGTGTGATCATTGTGGTGAAACTTCATGGCAGACGGCGATTTTGTTAAAGCCACTTGCGAATTTTGTGG
CACTGAGAATTTGACTAAAGAAGGTGCCACTACTTGTGGTTACTTACCCCAAAATGCTGTTGTTAAAATTTATTGTCCAGCA
TGTCACAATTCAGAAGTAGGACCTGAGCATAGTCTTGCCGAATACCATAATGAATCTGGCTTGAAAACCATTCTTCGTAAG
GGTGGTCGCACTATTGCCTTTGGAGGCTGTGTGTTCTCTTATGTTGGTTGCCATAACAAGTGTGCCTATTGGGTTCCACGTG
CTAGCGCTAACATAGGTTGTAACCATACAGGTGTTGTTGGAGAAGGTTCCGAAGGTCTTAATGACAACCTTCTTGAAATAC
TCCAAAAAGAGAAAGTCAACATCAATATTGTTGGTGACTTTAAACTTAATGAAGAGATCGCCATTATTTTGGCATCTTTTTC
TGCTTCCACAAGTGCTTTTGTGGAAACTGTGAAAGGTTTGGATTATAAAGCATTCAAACAAATTGTTGAATCCTGTGGTAAT
TTTAAAGTTACAAAAGGAAAAGCTAAAAAAGGTGCCTGGAATATTGGTGAACAGAAATCAATACTGAGTCCTCTTTATGCA
TTTGCATCAGAGGCTGCTCGTGTTGTACGATCAATTTTCTCCCGCACTCTTGAAACTGCTCAAAATTCTGTGCGTGTTTACA
GAAGGCCGCTATAACAATACTAGATGGAATTTCACAGTATTCACTGAGACTCATTGATGCTATGATGTTCACATCTGATTT
GGCTACTAACAATCTAGTTGTAATGGCCTACATTACAGGTGGTGTTGTTCAGTTGACTTCGCAGTGGCTAACTAACATCTTT
GGCACTGTTTATGAAAAACTCAAACCCGTCCTTGATTGGCTTGAAGAGAAGTTTAAGGAAGGTGTAGAGTTTCTTAGAGAC
GGTTGGGAAATTGTTAAATTTATCTCAACCTGTGCTTGTGAAATTGTCGGTGGACAAATTGTCACCTGTGCAAAGGAAATT
AAGGAGAGTGTTCAGACATTCTTTAAGCTTGTAAATAAATTTTTGGCTTTGTGTGCTGACTCTATCATTATTGGTGGAGCTA
AACTTAAAGCCTTGAATTTAGGTGAAACATTTGTCACGCACTCAAAGGGATTGTACAGAAAGTGTGTTAAATCCAGAGAAG
AAACTGGCCTACTCATGCCTCTAAAAGCCCCAAAAGAAATTATCTTCTTAGAGGGAGAAACACTTCCCACAGAAGTGTTAA
CAGAGGAAGTTGTCTTGAAAACTGGTGATTTACAACCATTAGAACAACCTACTAGTGAAGCTGTTGAAGCTCCATTGGTTG
GTACACCAGTTTGTATTAACGGGCTTATGTTGCTCGAAATCAAAGACACAGAAAAGTACTGTGCCCTTGCACCTAATATGA
TGGTAACAAACAATACCTTCACACTCAAAGGCGGTGCACCAACAAAGGTTACTTTTGGTGATGACACTGTGATAGAAGTGC
AAGGTTACAAGAGTGTGAATATCACTTTTGAACTTGATGAAAGGATTGATAAAGTACTTAATGAGAAGTGCTCTGCCTATA
CAGTTGAACTCGGTACAGAAGTAAATGAGTTCGCCTGTGTTGTGGCAGATGCTGTCATAAAAACTTTGCAACCAGTATCTG
AATTACTTACACCACTGGGCATTGATTTAGATGAGTGGAGTATGGCTACATACTACTTATTTGATGAGTCTGGTGAGTTTAA
ATTGGCTTCACATATGTATTGTTCTTTTTACCCTCCAGATGAGGATGAAGAAGAAGGTGATTGTGAAGAAGAAGAGTTTGA
GCCATCAACTCAATATGAGTATGGTACTGAAGATGATTACCAAGGTAAACCTTTGGAATTTGGTGCCACTTCTGCTGCTCTT
CAACCTGAAGAAGAGCAAGAAGAAGATTGGTTAGATGATGATAGTCAACAAACTGTTGGTCAACAAGACGGCAGTGAGG
ACAATCAGACAACTACTATTCAAACAATTGTTGAGGTTCAACCTCAATTAGAGATGGAACTTACACCAGTTGTTCAGACTAT
TGAAGTGAATAGTTTTAGTGGTTATTTAAAACTTACTGACAATGTATACATTAAAAATGCAGACATTGTGGAAGAAGCTAA
AAAGGTAAAACCAACAGTGGTTGTTAATGCAGCCAATGTTTACCTTAAACATGGAGGAGGTGTTGCAGGAGCCTTAAATA
AGGCTACTAACAATGCCATGCAAGTTGAATCTGATGATTACATAGCTACTAATGGACCACTTAAAGTGGGTGGTAGTTGT
GTTTTAAGCGGACACAATCTTGCTAAACACTGTCTTCATGTTGTCGGCCCAAATGTTAACAAAGGTGAAGACATTCAACTTC
TTAAGAGTGCTTATGAAAATTTTAATCAGCACGAAGTTCTACTTGCACCATTATTATCAGCTGGTATTTTTGGTGCTGACCCT
ATACATTCTTTAAGAGTTTGTGTAGATACTGTTCGCACAAATGTCTACTTAGCTGTCTTTGATAAAAATCTCTATGACAAACT
TGTTTCAAGCTTTTTGGAAATGAAGAGTGAAAAGCAAGTTGAACAAAAGATCGCTGAGATTCCTAAAGAGGAAGTTAAGC
CATTTATAACTGAAAGTAAACCTTCAGTTGAACAGAGAAAACAAGATGATAAGAAAATCAAAGCTTGTGTTGAAGAAGTT
ACAACAACTCTGGAAGAAACTAAGTTCCTCACAGAAAACTTGTTACTTTATATTGACATTAATGGCAATCTTCATCCAGATT
CTGCCACTCTTGTTAGTGACATTGACATCACTTTCTTAAAGAAAGATGCTCCATATATAGTGGGTGATGTTGTTCAAGAGGG
TGTTTTAACTGCTGTGGTTATACCTACTAAAAAGGCTGGTGGCACTACTGAAATGCTAGCGAAAGCTTTGAGAAAAGTGCC
AACAGACAATTATATAACCACTTACCCGGGTCAGGGTTTAAATGGTTACACTGTAGAGGAGGCAAAGACAGTGCTTAAAA
AGTGTAAAAGTGCCTTTTACATTCTACCATCTATTATCTCTAATGAGAAGCAAGAAATTCTTGGAACTGTTTCTTGGAATTTG
CGAGAAATGCTTGCACATGCAGAAGAAACACGCAAATTAATGCCTGTCTGTGTGGAAACTAAAGCCATAGTTTCAACTATA
CAGCGTAAATATAAGGGTATTAAAATACAAGAGGGTGTGGTTGATTATGGTGCTAGATTTTACTTTTACACCAGTAAAACA
ACTGTAGCGTCACTTATCAACACACTTAACGATCTAAATGAAACTCTTGTTACAATGCCACTTGGCTATGTAACACATGGCT
TAAATTTGGAAGAAGCTGCTCGGTATATGAGATCTCTCAAAGTGCCAGCTACAGTTTCTGTTTCTTCACCTGATGCTGTTAC
AGCGTATAATGGTTATCTTACTTCTTCTTCTAAAACACCTGAAGAACATTTTATTGAAACCATCTCACTTGCTGGTTCCTATA
AAGATTGGTCCTATTCTGGACAATCTACACAACTAGGTATAGAATTTCTTAAGAGAGGTGATAAAAGTGTATATTACACTA
GTAATCCTACCACATTCCACCTAGATGGTGAAGTTATCACCTTTGACAATCTTAAGACACTTCTTTCTTTGAGAGAAGTGAG
GACTATTAAGGTGTTTACAACAGTAGACAACATTAACCTCCACACGCAAGTTGTGGACATGTCAATGACATATGGACAACA
GTTTGGTCCAACTTATTTGGATGGAGCTGATGTTACTAAAATAAAACCTCATAATTCACATGAAGGTAAAACATTTTATGTT
TTACCTAATGATGACACTCTACGTGTTGAGGCTTTTGAGTACTACCACACAACTGATCCTAGTTTTCTGGGTAGGTACATGT
CAGCATTAAATCACACTAAAAAGTGGAAATACCCACAAGTTAATGGTTTAACTTCTATTAAATGGGCAGATAACAACTGTT
ATCTTGCCACTGCATTGTTAACACTCCAACAAATAGAGTTGAAGTTTAATCCACCTGCTCTACAAGATGCTTATTACAGAGC
 
AAGGGCTGGTGAAGCTGCTAACTTTTGTGCACTTATCTTAGCCTACTGTAATAAGACAGTAGGTGAGTTAGGTGATGTTAG
AGAAACAATGAGTTACTTGTTTCAACATGCCAATTTAGATTCTTGCAAAAGAGTCTTGAACGTGGTGTGTAAAACTTGTGG
ACAACAGCAGACAACCCTTAAGGGTGTAGAAGCTGTTATGTACATGGGCACACTTTCTTATGAACAATTTAAGAAAGGTGT
TCAGATACCTTGTACGTGTGGTAAACAAGCTACAAAATATCTAGTACAACAGGAGTCACCTTTTGTTATGATGTCAGCACCA
CCTGCTCAGTATGAACTTAAGCATGGTACATTTACTTGTGCTAGTGAGTACACTGGTAATTACCAGTGTGGTCACTATAAAC
ATATAACTTCTAAAGAAACTTTGTATTGCATAGACGGTGCTTTACTTACAAAGTCCTCAGAATACAAAGGTCCTATTACGGA
TGTTTTCTACAAAGAAAACAGTTACACAACAACCATAAAACCAGTTACTTATAAATTGGATGGTGTTGTTTGTACAGAAATT
GACCCTAAGTTGGACAATTATTATAAGAAAGACAATTCTTATTTCACAGAGCAACCAATTGATCTTGTACCAAACCAACCAT
ATCCAAACGCAAGCTTCGATAATTTTAAGTTTGTATGTGATAATATCAAATTTGCTGATGATTTAAACCAGTTAACTGGTTA
TAAGAAACCTGCTTCAAGAGAGCTTAAAGTTACATTTTTCCCTGACTTAAATGGTGATGTGGTGGCTATTGATTATAAACAC
TACACACCCTCTTTTAAGAAAGGAGCTAAATTGTTACATAAACCTATTGTTTGGCATGTTAACAATGCAACTAATAAAGCCA
CGTATAAACCAAATACCTGGTGTATACGTTGTCTTTGGAGCACAAAACCAGTTGAAACATCAAATTCGTTTGATGTACTGAA
GTCAGAGGACGCGCAGGGAATGGATAATCTTGTCTGCGAAGATCTAAAACCAGTCTCTGAAGAAGTAGTGGAAAATCCTA
CCATACAGAAAGACGTTCTTGAGTGTAATGTGAAAACTACCGAAGTTGTAGGAGACATTATACTTAAACCAGCAAATAATA
GTTTAAAAATTACAGAAGAGGTTGGCCACACAGATCTAATGGCTGCTTATGTAGACAATTCTAGTCTTACTATTAAGAAAC
CTAATGAATTATCTAGAGTATTAGGTTTGAAAACCCTTGCTACTCATGGTTTAGCTGCTGTTAATAGTGTCCCTTGGGATAC
TATAGCTAATTATGCTAAGCCTTTTCTTAACAAAGTTGTTAGTACAACTACTAACATAGTTACACGGTGTTTAAACCGTGTTT
GTACTAATTATATGCCTTATTTCTTTACTTTATTGCTACAATTGTGTACTTTTACTAGAAGTACAAATTCTAGAATTAAAGCAT
CTATGCCGACTACTATAGCAAAGAATACTGTTAAGAGTGTCGGTAAATTTTGTCTAGAGGCTTCATTTAATTATTTGAAGTC
ACCTAATTTTTCTAAACTGATAAATATTATAATTTGGTTTTTACTATTAAGTGTTTGCCTAGGTTCTTTAATCTACTCAACCGC
TGCTTTAGGTGTTTTAATGTCTAATTTAGGCATGCCTTCTTACTGTACTGGTTACAGAGAAGGCTATTTGAACTCTACTAATG
TCACTATTGCAACCTACTGTACTGGTTCTATACCTTGTAGTGTTTGTCTTAGTGGTTTAGATTCTTTAGACACCTATCCTTCTT
TAGAAACTATACAAATTACCATTTCATCTTTTAAATGGGATTTAACTGCTTTTGGCTTAGTTGCAGAGTGGTTTTTGGCATAT
ATTCTTTTCACTAGGTTTTTCTATGTACTTGGATTGGCTGCAATCATGCAATTGTTTTTCAGCTATTTTGCAGTACATTTTATT
AGTAATTCTTGGCTTATGTGGTTAATAATTAATCTTGTACAAATGGCCCCGATTTCAGCTATGGTTAGAATGTACATCTTCT
TTGCATCATTTTATTATGTATGGAAAAGTTATGTGCATGTTGTAGACGGTTGTAATTCATCAACTTGTATGATGTGTTACAA
ACGTAATAGAGCAACAAGAGTCGAATGTACAACTATTGTTAATGGTGTTAGAAGGTCCTTTTATGTCTATGCTAATGGAGG
TAAAGGCTTTTGCAAACTACACAATTGGAATTGTGTTAATTGTGATACATTCTGTGCTGGTAGTACATTTATTAGTGATGAA
GTTGCGAGAGACTTGTCACTACAGTTTAAAAGACCAATAAATCCTACTGACCAGTCTTCTTACATCGTTGATAGTGTTACAG
TGAAGAATGGTTCCATCCATCTTTACTTTGATAAAGCTGGTCAAAAGACTTATGAAAGACATTCTCTCTCTCATTTTGTTAAC
TTAGACAACCTGAGAGCTAATAACACTAAAGGTTCATTGCCTATTAATGTTATAGTTTTTGATGGTAAATCAAAATGTGAAG
AATCATCTGCAAAATCAGCGTCTGTTTACTACAGTCAGCTTATGTGTCAACCTATACTGTTACTAGATCAGGCATTAGTGTC
TGATGTTGGTGATAGTGCGGAAGTTGCAGTTAAAATGTTTGATGCTTACGTTAATACGTTTTCATCAACTTTTAACGTACCA
ATGGAAAAACTCAAAACACTAGTTGCAACTGCAGAAGCTGAACTTGCAAAGAATGTGTCCTTAGACAATGTCTTATCTACT
TTTATTTCAGCAGCTCGGCAAGGGTTTGTTGATTCAGATGTAGAAACTAAAGATGTTGTTGAATGTCTTAAATTGTCACATC
AATCTGACATAGAAGTTACTGGCGATAGTTGTAATAACTATATGCTCACCTATAACAAAGTTGAAAACATGACACCCCGTG
ACCTTGGTGCTTGTATTGACTGTAGTGCGCGTCATATTAATGCGCAGGTAGCAAAAAGTCACAACATTGCTTTGATATGGA
ACGTTAAAGATTTCATGTCATTGTCTGAACAACTACGAAAACAAATACGTAGTGCTGCTAAAAAGAATAACTTACCTTTTAA
GTTGACATGTGCAACTACTAGACAAGTTGTTAATGTTGTAACAACAAAGATAGCACTTAAGGGTGGTAAAATTGTTAATAA
TTGGTTGAAGCAGTTAATTAAAGTTACACTTGTGTTCCTTTTTGTTGCTGCTATTTTCTATTTAATAACACCTGTTCATGTCAT
GTCTAAACATACTGACTTTTCAAGTGAAATCATAGGATACAAGGCTATTGATGGTGGTGTCACTCGTGACATAGCATCTAC
AGATACTTGTTTTGCTAACAAACATGCTGATTTTGACACATGGTTTAGCCAGCGTGGTGGTAGTTATACTAATGACAAAGCT
TGCCCATTGATTGCTGCAGTCATAACAAGAGAAGTGGGTTTTGTCGTGCCTGGTTTGCCTGGCACGATATTACGCACAACT
AATGGTGACTTTTTGCATTTCTTACCTAGAGTTTTTAGTGCAGTTGGTAACATCTGTTACACACCATCAAAACTTATAGAGTA
CACTGACTTTGCAACATCAGCTTGTGTTTTGGCTGCTGAATGTACAATTTTTAAAGATGCTTCTGGTAAGCCAGTACCATAT
TGTTATGATACCAATGTACTAGAAGGTTCTGTTGCTTATGAAAGTTTACGCCCTGACACACGTTATGTGCTCATGGATGGCT
CTATTATTCAATTTCCTAACACCTACCTTGAAGGTTCTGTTAGAGTGGTAACAACTTTTGATTCTGAGTACTGTAGGCACGG
CACTTGTGAAAGATCAGAAGCTGGTGTTTGTGTATCTACTAGTGGTAGATGGGTACTTAACAATGATTATTACAGATCTTTA
CCAGGAGTTTTCTGTGGTGTAGATGCTGTAAATTTACTTACTAATATGTTTACACCACTAATTCAACCTATTGGTGCTTTGGA
CATATCAGCATCTATAGTAGCTGGTGGTATTGTAGCTATCGTAGTAACATGCCTTGCCTACTATTTTATGAGGTTTAGAAGA
GCTTTTGGTGAATACAGTCATGTAGTTGCCTTTAATACTTTACTATTCCTTATGTCATTCACTGTACTCTGTTTAACACCAGTT
TACTCATTCTTACCTGGTGTTTATTCTGTTATTTACTTGTACTTGACATTTTATCTTACTAATGATGTTTCTTTTTTAGCACATA
TTCAGTGGATGGTTATGTTCACACCTTTAGTACCTTTCTGGATAACAATTGCTTATATCATTTGTATTTCCACAAAGCATTTCT
ATTGGTTCTTTACTAATTACCTAAAGAGACGTGTAGTCTTTAATGGTGTTTCCTTTAGTACTTTTGAAGAAGCTGCGCTGTG
CACCTTTTTGTTAAATAAAGAAATGTATCTAAAGTTGCGTAGTGATGTGCTATTACCTCTTACGCAATATAATAGATACTTA
 
GCTCTTTATAATAAGTACAAGTATTTTAGTGGAGCAATGGATACAACTAGCTACAGAGAAGCTGCTTGTTGTCATCTCGCA
AAGGCTCTCAATGACTTCAGTAACTCAGGTTCTGATGTTCTTTACCAACCACCACAAACCTCTATCACCTCAGCTGTTTTGCA
GAGTGGTTTTAGAAAAATGGCATTCCCATCTGGTAAAGTTGAGGGTTGTATGGTACAAGTAACTTGTGGTACAACTACACT
TAACGGTCTTTGGCTTGATGACGTAGTTTACTGTCCAAGACATGTGATCTGCACCTCTGAAGACATGCTTAACCCTAATTAT
GAAGATTTACTCATTCGTAAGTCTAATCATAATTTCTTGGTACAGGCTGGTAATGTTCAACTCAGGGTTATTGGACATTCTA
TGCAAAATTGTGTACTTAAGCTTAAGGTTGATACAGCCAATCCTAAGACACCTAAGTATAAGTTTGTTCGCATTCAACCAGG
ACAGACTTTTTCAGTGTTAGCTTGTTACAATGGTTCACCATCTGGTGTTTACCAATGTGCTATGAGGCCCAATTTCACTATTA
AGGGTTCATTCCTTAATGGTTCATGTGGTAGTGTTGGTTTTAACATAGATTATGACTGTGTCTCTTTTTGTTACATGCACCAT
ATGGAATTACCAACTGGAGTTCATGCTGGCACAGACTTAGAAGGTAACTTTTATGGACCTTTTGTTGACAGGCAAACAGCA
CAAGCAGCTGGTACGGACACAACTATTACAGTTAATGTTTTAGCTTGGTTGTACGCTGCTGTTATAAATGGAGACAGGTGG
TTTCTCAATCGATTTACCACAACTCTTAATGACTTTAACCTTGTGGCTATGAAGTACAATTATGAACCTCTAACACAAGACCA
TGTTGACATACTAGGACCTCTTTCTGCTCAAACTGGAATTGCCGTTTTAGATATGTGTGCTTCATTAAAAGAATTACTGCAA
AATGGTATGAATGGACGTACCATATTGGGTAGTGCTTTATTAGAAGATGAATTTACACCTTTTGATGTTGTTAGACAATGCT
CAGGTGTTACTTTCCAAAGTGCAGTGAAAAGAACAATCAAGGGTACACACCACTGGTTGTTACTCACAATTTTGACTTCACT
TTTAGTTTTAGTCCAGAGTACTCAATGGTCTTTGTTCTTTTTTTGTATGAAAATGCCTTTTTACCTTTTGCTATGGGTATTAT
TGCTATGTCTGCTTTTGCAATGATGTTTGTCAAACATAAGCATGCATTTCTCTGTTTGTTTTTGTTACCTTCTCTTGCCACTG
TAGCTTATTTTAATATGGTCTATATGCCTGCTAGTTGGGTGATGCGTATTATGACATGGTTGGATATGGTTGATACTAGTTT
GTCTGGTTTTAAGCTAAAAGACTGTGTTATGTATGCATCAGCTGTAGTGTTACTAATCCTTATGACAGCAAGAACTGTGTAT
GATGATGGTGCTAGGAGAGTGTGGACACTTATGAATGTCTTGACACTCGTTTATAAAGTTTATTATGGTAATGCTTTAGAT
CAAGCCATTTCCATGTGGGCTCTTATAATCTCTGTTACTTCTAACTACTCAGGTGTAGTTACAACTGTCATGTTTTTGGCCAA
AGGTATTGTTTTTATGTGTGTTGAGTATTGCCCTATTTTCTTCATAACTGGTAATACACTTCAGTGTATAATGCTAGTTTATT
GTTTCTTAGGCTATTTTTGTACTTGTTACTTTGGCCTCTTTTGTTTACTCAACCGCTACTTTAGACTGACTCTTGGTGTTTATG
ATTACTTAGTTTCTACACAGGAGTTTAGATATATGAATTCACAGGGACTACTCCCACCCAAGAATAGCATAGATGCCTTCAA
ACTCAACATTAAATTGTTGGGTGTTGGTGGCAAACCTTGTATCAAAGTAGCCACTGTACAGTCTAAAATGTCAGATGTAAA
GTGCACATCAGTAGTCTTACTCTCAGTTTTGCAACAACTCAGAGTAGAATCATCATCTAAATTGTGGGCTCAATGTGTCCAG
TTACACAATGACATTCTCTTAGCTAAAGATACTACTGAAGCCTTTGAAAAAATGGTTTCACTACTTTCTGTTTTGCTTTCCAT
GCAGGGTGCTGTAGACATAAACAAGCTTTGTGAAGAAATGCTGGACAACAGGGCAACCTTACAAGCTATAGCCTCAGAGT
TTAGTTCCCTTCCATCATATGCAGCTTTTGCTACTGCTCAAGAAGCTTATGAGCAGGCTGTTGCTAATGGTGATTCTGAAGT
TGTTCTTAAAAAGTTGAAGAAGTCTTTGAATGTGGCTAAATCTGAATTTGACCGTGATGCAGCCATGCAACGTAAGTTGGA
AAAGATGGCTGATCAAGCTATGACCCAAATGTATAAACAGGCTAGATCTGAGGACAAGAGGGCAAAAGTTACTAGTGCTA
TGCAGACAATGCTTTTCACTATGCTTAGAAAGTTGGATAATGATGCACTCAACAACATTATCAACAATGCAAGAGATGGTT
GTGTTCCCTTGAACATAATACCTCTTACAACAGCAGCCAAATTAATGGTTGTCATACCAGACTATAACACATATAAAAATAC
GTGTGATGGTACAACATTTACTTATGCATCAGCATTGTGGGAAATCCAACAGGTTGTAGATGCAGATAGTAAAATTGTTCA
ACTTAGTGAAATTAGTATGGACAATTCACCTAATTTAGCATGGCCTCTTATTGTAACAGCTTTAAGGGCCAATTCTGCTGTC
AAATTACAGAATAATGAGCTTAGTCCTGTTGCACTACGACAGATGTCTTGTGCTGCCGGTACTACACAAACTGCTTGCACT
GATGACAATGCGTTAGCTTACTACAACACAACAAAGGGAGGTAGGTTTGTACTTGCACTGTTATCCGATTTACAGGATTTG
AAATGGGCTAGATTCCCTAAGAGTGATGGAACTGGTACTATCTATACAGAACTGGAACCACCTTGTAGGTTTGTTACAGAC
ACACCTAAAGGTCCTAAAGTGAAGTATTTATACTTTATTAAAGGATTAAACAACCTAAATAGAGGTATGGTACTTGGTAGT
TTAGCTGCCACAGTACGTCTACAAGCTGGTAATGCAACAGAAGTGCCTGCCAATTCAACTGTATTATCTTTCTGTGCTTTTG
CTGTAGATGCTGCTAAAGCTTACAAAGATTATCTAGCTAGTGGGGGACAACCAATCACTAATTGTGTTAAGATGTTGTGTA
CACACACTGGTACTGGTCAGGCAATAACAGTTACACCGGAAGCCAATATGGATCAAGAATCCTTTGGTGGTGCATCGTGTT
GTCTGTACTGCCGTTGCCACATAGATCATCCAAATCCTAAAGGATTTTGTGACTTAAAAGGTAAGTATGTACAAATACCTAC
AACTTGTGCTAATGACCCTGTGGGTTTTACACTTAAAAACACAGTCTGTACCGTCTGCGGTATGTGGAAAGGTTATGGCTG
TAGTTGTGATCAACTCCGCGAACCCATGCTTCAGTCAGCTGATGCACAATCGTTTTTAAACGGGTTTGCGGTGTAAGTGCA
GCCCGTCTTACACCGTGCGGCACAGGCACTAGTACTGATGTCGTATACAGGGCTTTTGACATCTACAATGATAAAGTAGCT
GGTTTTGCTAAATTCCTAAAAACTAATTGTTGTCGCTTCCAAGAAAAGGACGAAGATGACAATTTAATTGATTCTTACTTTG
TAGTTAAGAGACACACTTTCTCTAACTACCAACATGAAGAAACAATTTATAATTTACTTAAAGATTGTCCAGCTGTTGCTAA
ACATGACTTCTTTAAGTTTAGAATAGACGGTGACATGGTACCACATATATCACGTCAACGTCTTACTAAATACACAATGGCA
GACCTCGTCTATGCTTTAAGGCATTTTGATGAAGGTAATTGTGACACATTAAAAGAAATACTTGTCACATACAATTGTTGTG
ATGATGATTATTTCAATAAAAAGGACTGGTATGATTTTGTAGAAAACCCAGATATATTACGCGTATACGCCAACTTAGGTG
AACGTGTACGCCAAGCTTTGTTAAAAACAGTACAATTCTGTGATGCCATGCGAAATGCTGGTATTGTTGGTGTACTGACAT
TAGATAATCAAGATCTCAATGGTAACTGGTATGATTTCGGTGATTTCATACAAACCACGCCAGGTAGTGGAGTTCCTGTTG
TAGATTCTTATTATTCATTGTTAATGCCTATATTAACCTTGACCAGGGCTTTAACTGCAGAGTCACATGTTGACACTGACTTA
ACAAAGCCTTACATTAAGTGGGATTTGTTAAAATATGACTTCACGGAAGAGAGGTTAAAACTCTTTGACCGTTATTTTAAAT
ATTGGGATCAGACATACCACCCAAATTGTGTTAACTGTTTGGATGACAGATGCATTCTGCATTGTGCAAACTTTAATGTTTT
 
ATTCTCTACAGTGTTCCCACTTACAAGTTTGGACCACTAGTGAGAAAAATATTTGTTGATGGTGTTCCATTTGTAGTTTCAA
CTGGATACCACTTCAGAGAGCTAGGTGTTGTACATAATCAGGATGTAAACTTACATAGCTCTAGACTTAGTTTTAAGGAAT
TACTTGTGTATGCTGCTGACCCTGCTATGCACGCTGCTTCTGGTAATCTATTACTAGATAAACGCACTACGTGCTTTTCAGTA
GCTGCACTTACTAACAATGTTGCTTTTCAAACTGTCAAACCCGGTAATTTTAACAAAGACTTCTATGACTTTGCTGTGTCTAA
GGGTTTCTTTAAGGAAGGAAGTTCTGTTGAATTAAAACACTTCTTCTTTGCTCAGGATGGTAATGCTGCTATCAGCGATTAT
GACTACTATCGTTATAATCTACCAACAATGTGTGATATCAGACAACTACTATTTGTAGTTGAAGTTGTTGATAAGTACTTTG
ATTGTTACGATGGTGGCTGTATTAATGCTAACCAAGTCATCGTCAACAACCTAGACAAATCAGCTGGTTTTCCATTTAATAA
ATGGGGTAAGGCTAGACTTTATTATGATTCAATGAGTTATGAGGATCAAGATGCACTTTTCGCATATACAAAACGTAATGT
CATCCCTACTATAACTCAAATGAATCTTAAGTATGCCATTAGTGCAAAGAATAGAGCTCGCACCGTAGCTGGTGTCTCTATC
TGTAGTACTATGACCAATAGACAGTTTCATCAAAAATTATTGAAATCAATAGCCGCCACTAGAGGAGCTACTGTAGTAATT
GGAACAAGCAAATTCTATGGTGGTTGGCACAACATGTTAAAAACTGTTTATAGTGATGTAGAAAACCCTCACCTTATGGGT
TGGGATTATCCTAAATGTGATAGAGCCATGCCTAACATGCTTAGAATTATGGCCTCACTTGTTCTTGCTCGCAAACATACAA
CGTGTTGTAGCTTGTCACACCGTTTCTATAGATTAGCTAATGAGTGTGCTCAAGTATTGAGTGAAATGGTCATGTGTGGCG
GTTCACTATATGTTAAACCAGGTGGAACCTCATCAGGAGATGCCACAACTGCTTATGCTAATAGTGTTTTTAACATTTGTCA
AGCTGTCACGGCCAATGTTAATGCACTTTTATCTACTGATGGTAACAAAATTGCCGATAAGTATGTCCGCAATTTACAACAC
AGACTTTATGAGTGTCTCTATAGAAATAGAGATGTTGACACAGACTTTGTGAATGAGTTTTACGCATATTTGCGTAAACATT
TCTCAATGATGATACTCTCTGACGATGCTGTTGTGTGTTTCAATAGCACTTATGCATCTCAAGGTCTAGTGGCTAGCATAAA
GAACTTTAAGTCAGTTCTTTATTATCAAAACAATGTTTTTATGTCTGAAGCAAAATGTTGGACTGAGACTGACCTTACTAAA
GGACCTCATGAATTTTGCTCTCAACATACAATGCTAGTTAAACAGGGTGATGATTATGTGTACCTTCCTTACCCAGATCCAT
CAAGAATCCTAGGGGCCGGCTGTTTTGTAGATGATATCGTAAAAACAGATGGTACACTTATGATTGAACGGTTCGTGTCTT
TAGCTATAGATGCTTACCCACTTACTAAACATCCTAATCAGGAGTATGCTGATGTCTTTCATTTGTACTTACAATACATAAGA
AAGCTACATGATGAGTTAACAGGACACATGTTAGACATGTATTCTGTTATGCTTACTAATGATAACACTTCAAGGTATTGG
GAACCTGAGTTTTATGAGGCTATGTACACACCGCATACAGTCTTACAGGCTGTTGGGGCTTGTGTTCTTTGCAATTCACAGA
CTTCATTAAGATGTGGTGCTTGCATACGTAGACCATTCTTATGTTGTAAATGCTGTTACGACCATGTCATATCAACATCACAT
AAATTAGTCTTGTCTGTTAATCCGTATGTTTGCAATGCTCTAGGTTGTGATGTCACAGATGTGACTCAACTTTACTTAGGAG
GTATGAGCTATTATTGTAAATCACATAAACCACCCATTAGTTTTCCATTGTGTGCTAATGGACAAGTTTTTGGTTTATATAAA
AATACATGTGTTGGTAGCGATAATGTTACTGACTTTAATGCAATTGCAACATGTGACTGGACAAATGCTGGTGATTACATTT
TAGCTAACACCTGTACTGAAAGACTCAAGCTTTTTGCAGCAGAAACGCTCAAAGCTACTGAGGAGACATTTAAACTGTCTT
ATGGTATTGCTACTGTACGTGAAGTGCTGTCTGACAGAGAATTACATCTTTCATGGGAAGTTGGTAAACCTAGACCACCAC
TTAACCGAAATTATGTCTTTACTGGTTATCGTGTAACTAAAAACAGTAAAGTACAAATAGGAGAGTACACCTTTGAAAAAG
GTGACTATGGTGATGCTGTTGTTTACCGAGGTACAACAACTTACAAATTAAATGTTGGTGATTATTTTGTGCTGACATCACA
TACAGTAATGCCATTAAGTGCACCTACACTAGTGCCACAAGAGCACTATGTTAGAATTACTGGCTTATACCCAACACTCAAT
ATCTCAGATGAGTTTTCTAGCAATGTTGCAAATTATCAAAAGGTTGGTATGCAAAAGTATTCTACACTCCAGGGACCACCTG
GTACTGGTAAGAGTCATTTTGCTATTGGCCTAGCTCTCTACTACCCTTCTGCTCGCATAGTGTATACAGCTTGCTCTCATGCC
GCTGTTGATGCACTATGTGAGAAGGCATTAAAATATTTGCCTATAGATAAATGTAGTAGAATTATACCTGCACGTGCTCGT
GTAGAGTGTTTTGATAAATTCAAAGTGAATTCAACATTAGAACAGTATGTCTTTTGTACTGTAAATGCATTGCCTGAGACGA
CAGCAGATATAGTTGTCTTTGATGAAATTTCAATGGCCACAAATTATGATTTGAGTGTTGTCAATGCCAGATTACGTGCTAA
GCACTATGTGTACATTGGCGACCCTGCTCAATTACCTGCACCACGCACATTGCTAACTAAGGGCACACTAGAACCAGAATA
TTTCAATTCAGTGTGTAGACTTATGAAAACTATAGGTCCAGACATGTTCCTCGGAACTTGTCGGCGTTGTCCTGCTGAAATT
GTTGACACTGTGAGTGCTTTGGTTTATGATAATAAGCTTAAAGCACATAAAGACAAATCAGCTCAATGCTTTAAAATGTTTT
ATAAGGGTGTTATCACGCATGATGTTTCATCTGCAATTAACAGGCCACAAATAGGCGTGGTAAGAGAATTCCTTACACGTA
ACCCTGCTTGGAGAAAAGCTGTCTTTATTTCACCTTATAATTCACAGAATGCTGTAGCCTCAAAGATTTTGGGACTACCAAC
TCAAACTGTTGATTCATCACAGGGCTCAGAATATGACTATGTCATATTCACTCAAACCACTGAAACAGCTCACTCTTGTAAT
GTAAACAGATTTAATGTTGCTATTACCAGAGCAAAAGTAGGCATACTTTGCATAATGTCTGATAGAGACCTTTATGACAAG
TTGCAATTTACAAGTCTTGAAATTCCACGTAGGAATGTGGCAACTTTACAAGCTGAAAATGTAACAGGACTCTTTAAAGATT
GTAGTAAGGTAATCACTGGGTTACATCCTACACAGGCACCTACACACCTCAGTGTTGACACTAAATTCAAAACTGAAGGTT
TATGTGTTGACATACCTGGCATACCTAAGGACATGACCTATAGAAGACTCATCTCTATGATGGGTTTTAAAATGAATTATCA
AGTTAATGGTTACCCTAACATGTTTATCACCCGCGAAGAAGCTATAAGACATGTACGTGCATGGATTGGCTTCGATGTCGA
GGGGTGTCATGCTACTAGAGAAGCTGTTGGTACCAATTTACCTTTACAGCTAGGTTTTTCTACAGGTGTTAACCTAGTTGCT
GTACCTACAGGTTATGTTGATACACCTAATAATACAGATTTTTCCAGAGTTAGTGCTAAACCACCGCCTGGAGATCAATTTA
AACACCTCATACCACTTATGTACAAAGGACTTCCTTGGAATGTAGTGCGTATAAAGATTGTACAAATGTTAAGTGACACACT
TAAAAATCTCTCTGACAGAGTCGTATTTGTCTTATGGGCACATGGCTTTGAGTTGACATCTATGAAGTATTTTGTGAAAATA
GGACCTGAGCGCACCTGTTGTCTATGTGATAGACGTGCCACATGCTTTTCCACTGCTTCAGACACTTATGCCTGTTGGCATC
ATTCTATTGGATTTGATTACGTCTATAATCCGTTTATGATTGATGTTCAACAATGGGGTTTTACAGGTAACCTACAAAGCAA
CCATGATCTGTATTGTCAAGTCCATGGTAATGCACATGTAGCTAGTTGTGATGCAATCATGACTAGGTGTCTAGCTGTCCAC
 
GAGTGCTTTGTTAAGCGTGTTGACTGGACTATTGAATATCCTATAATTGGTGATGAACTGAAGATTAATGCGGCTTGTAGA
AAGGTTCAACACATGGTTGTTAAAGCTGCATTATTAGCAGACAAATTCCCAGTTCTTCACGACATTGGTAACCCTAAAGCTA
TTAAGTGTGTACCTCAAGCTGATGTAGAATGGAAGTTCTATGATGCACAGCCTTGTAGTGACAAAGCTTATAAAATAGAAG
AATTATTCTATTCTTATGCCACACATTCTGACAAATTCACAGATGGTGTATGCCTATTTTGGAATTGCAATGTCGATAGATAT
CCTGCTAATTCCATTGTTTGTAGATTTGACACTAGAGTGCTATCTAACCTTAACTTGCCTGGTTGTGATGGTGGCAGTTTGT
ATGTAAATAAACATGCATTCCACACACCAGCTTTTGATAAAAGTGCTTTTGTTAATTTAAAACAATTACCATTTTTCTATTAC
TCTGACAGTCCATGTGAGTCTCATGGAAAACAAGTAGTGTCAGATATAGATTATGTACCACTAAAGTCTGCTACGTGTATA
ACACGTTGCAATTTAGGTGGTGCTGTCTGTAGACATCATGCTAATGAGTACAGATTGTATCTCGATGCTTATAACATGATG
ATCTCAGCTGGCTTTAGCTTGTGGGTTTACAAACAATTTGATACTTATAACCTCTGGAACACTTTTACAAGACTTCAGAGTTT
AGAAAATGTGGCTTTTAATGTTGTAAATAAGGGACACTTTGATGGACAACAGGGTGAAGTACCAGTTTCTATCATTAATAA
CACTGTTTACACAAAAGTTGATGGTGTTGATGTAGAATTGTTTGAAAATAAAACAACATTACCTGTTAATGTAGCATTTGAG
CTTTGGGCTAAGCGCAACATTAAACCAGTACCAGAGGTGAAAATACTCAATAATTTGGGTGTGGACATTGCTGCTAATACT
GTGATCTGGGACTACAAAAGAGATGCTCCAGCACATATATCTACTATTGGTGTTTGTTCTATGACTGACATAGCCAAGAAA
CCAACTGAAACGATTTGTGCACCACTCACTGTCTTTTTTGATGGTAGAGTTGATGGTCAAGTAGACTTATTTAGAAATGCCC
GTAATGGTGTTCTTATTACAGAAGGTAGTGTTAAAGGTTTACAACCATCTGTAGGTCCCAAACAAGCTAGTCTTAATGGAG
TCACATTAATTGGAGAAGCCGTAAAAACACAGTTCAATTATTATAAGAAAGTTGATGGTGTTGTCCAACAATTACCTGAAA
CTTACTTTACTCAGAGTAGAAATTTACAAGAATTTAAACCCAGGAGTCAAATGGAAATTGATTTCTTAGAATTAGCTATGGA
TGAATTCATTGAACGGTATAAATTAGAAGGCTATGCCTTCGAACATATCGTTTATGGAGATTTTAGTCATAGTCAGTTAGGT
GGTTTACATCTACTGATTGGACTAGCTAAACGTTTTAAGGAATCACCTTTTGAATTAGAAGATTTTATTCCTATGGACAGTA
CAGTTAAAAACTATTTCATAACAGATGCGCAAACAGGTTCATCTAAGTGTGTGTGTTCTGTTATTGATTTATTACTTGATGA
TTTTGTTGAAATAATAAAATCCCAAGATTTATCTGTAGTTTCTAAGGTTGTCAAAGTGACTATTGACTATACAGAAATTTCAT
TTATGCTTTGGTGTAAAGATGGCCATGTAGAAACATTTTACCCAAAATTACAATCTAGTCAAGCGTGGCAACCGGGTGTTG
CTATGCCTAATCTTTACAAAATGCAAAGAATGCTATTAGAAAAGTGTGACCTTCAAAATTATGGTGATAGTGCAACATTACC
TAAAGGCATAATGATGAATGTCGCAAAATATACTCAACTGTGTCAATATTTAAACACATTAACATTAGCTGTACCCTATAAT
ATGAGAGTTATACATTTTGGTGCTGGTTCTGATAAAGGAGTTGCACCAGGTACAGCTGTTTTAAGACAGTGGTTGCCTACG
GGTACGCTGCTTGTCGATTCAGATCTTAATGACTTTGTCTCTGATGCAGATTCAACTTTGATTGGTGATTGTGCAACTGTAC
ATACAGCTAATAAATGGGATCTCATTATTAGTGATATGTACGACCCTAAGACTAAAAATGTTACAAAAGAAAATGACTCTA
AAGAGGGTTTTTTCACTTACATTTGTGGGTTTATACAACAAAAGCTAGCTCTTGGAGGTTCCGTGGCTATAAAGATAACAG
AACATTCTTGGAATGCTGATCTTTATAAGCTCATGGGACACTTCGCATGGTGGACAGCCTTTGTTACTAATGTGAATGCGTC
ATCATCTGAAGCATTTTTAATTGGATGTAATTATCTTGGCAAACCACGCGAACAAATAGATGGTTATGTCATGCATGCAAAT
TACATATTTTGGAGGAATACAAATCCAATTCAGTTGTCTTCCTATTCTTTATTTGACATGAGTAAATTTCCCCTTAAATTAAG
GGGTACTGCTGTTATGTCTTTAAAAGAAGGTCAAATCAATGATATGATTTTATCTCTTCTTAGTAAAGGTAGACTTATAATT
AGAGAAAACAACAGAGTTGTTATTTCTAGTGATGTTCTTGTTAACAACTAAACGAACAATGTTTGTTTTTCTTGTTTTATTGC
CACTAGTCTCTATTCAGTGTGTTAATCTTACAACCAGAACTCAATTACCCCCTGCATACACTAATTCTTTCACACGTGGTGTT
TATTACCCTGACAAAGTTTTCAGATCCTCAGTTTTACATTCAACTCAGGACTTGTTCTTACCTTTCTTTTCCAATGTTACTTGG
TTCCATGCTATACATGTCTCTGGGACCAATGGTACTAAGAGGTTTGATAACCCTGTCCTACCATTTAATGATGGTGTTTATTT
TGCTTCCACTGAGAAGTCTAACATAATAAGAGGCTGGATTTTTGGTACTACTTTAGATTCGAAGACCCAGTCCCTACTTATT
GTTAATAACGCTACTAATGTTGTTATTAAAGTCTGTGAATTTCAATTTTGTAATGATCCATTTTTGGGTGTTTATTACCACAA
AAACAACAAAAGTTGTATGGAAAGTGAGTTCAGAGTTTATTCTAGTGCGAATAATTGCACTTTTGAATATGTCTCTCAGCCT
TTTCTTATGGACCTTGAAGGAAAACAGGGTAATTTCAAAAATCTTAGGGAATTTGTGTTTAAGAATATTGATGGTTATTTTA
AAATATATTCTAAGCACACGCCTATTAATTTAGTGCGTGATCTCCCTCAGGGTTTTTCGGCTTTAGAACCATTGGTAGATTT
GCCAATAGGTATTAACATCACTAGGTTTCAAACTTTACTTGCTTTACATAGAAGTTATTTGACTCCTGGTGATTCTTCTTCAG
GTTGGACAGCTGGTGCTGCAGCTTATTATGTGGGTTATCTTCAACCTAGGACTTTTCTATTAAAATATAATGAAAATGGAAC
CATTACAGATGCTGTAGACTGTGCACTTGACCCTCTCTCAGAAACAAAGTGTACGTTGAAATCCTTCACTGTAGAAAAAGG
AATCTATCAAACTTCTAACTTTAGAGTCCAACCAACAGAATCTATTGTTAGATTTCCTAATATTACAAACTTGTGCCCTTTTG
GTGAAGTTTTTAACGCCACCAGATTTGCATCTGTTTATGCTTGGAACAGGAAGAGAATCAGCAACTGTGTTGCTGATTATTC
TGTCCTATATAATTCCGCATCATTTTCCACTTTTAAGTGTTATGGAGTGTCTCCTACTAAATTAAATGATCTCTGCTTTACTAA
TGTCTATGCAGATTCATTTGTAATTAGAGGTGATGAAGTCAGACAAATCGCTCCAGGGCAAACTGGAAAGATTGCTGATTA
TAATTATAAATTACCAGATGATTTTACAGGCTGCGTTATAGCTTGGAATTCTAACAATCTTGATTCTAAGGTTGGTGGTAAT
TATAATTACCGGTATAGATTGTTTAGGAAGTCTAATCTCAAACCTTTTGAGAGAGATATTTCAACTGAAATCTATCAGGCCG
GTAGCACACCTTGTAATGGTGTTGAAGGTTTTAATTGTTACTTTCCTTTACAATCATATGGTTTCCAACCCACTAATGGTGTT
GGTTACCAACCATACAGAGTAGTAGTACTTTCTTTTGAACTTCTACATGCACCAGCAACTGTTTGTGGACCTAAAAAGTCTA
CTAATTTGGTTAAAAACAAATGTGTCAATTTCAACTTCAATGGTTTAACAGGCACAGGTGTTCTTACTGAGTCTAACAAAAA
GTTTCTGCCTTTCCAACAATTTGGCAGAGACATTGCTGACACTACTGATGCTGTCCGTGATCCACAGACACTTGAGATTCTT
GACATTACACCATGTTCTTTTGGTGGTGTCAGTGTTATAACACCAGGAACAAATACTTCTAACCAGGTTGCTGTTCTTTATC
 
AGGGTGTTAACTGCACAGAAGTCCCTGTTGCTATTCATGCAGATCAACTTACTCCTACTTGGCGTGTTTATTCTACAGGTTC
TAATGTTTTTCAAACACGTGCAGGCTGTTTAATAGGGGCTGAACATGTCAACAACTCATATGAGTGTGACATACCCATTGG
TGCAGGTATATGCGCTAGTTATCAGACTCAGACTAATTCTCCTCGGCGGGCACGTAGTGTAGCTAGTCAATCCATCATTGC
CTACACTATGTCACTTGGTGCAGAAAATTCAGTTGCTTACTCTAATAACTCTATTGCCATACCCACAAATTTTACTATTAGTG
TTACCACAGAAATTCTACCAGTGTCTATGACCAAGACATCAGTAGATTGTACAATGTACATTTGTGGTGATTCAACTGAATG
CAGCAATCTTTTGTTGCAATATGGCAGTTTTTGTACACAATTAAACCGTGCTTTAACTGGAATAGCTGTTGAACAAGACAAA
AACACCCAAGAAGTTTTTGCACAAGTCAAACAAATTTACAAAACACCACCAATTAAAGATTTTGGTGGTTTTAATTTTTCAC
AAATATTACCAGATCCATCAAAACCAAGCAAGAGGTCATTTATTGAAGATCTACTTTTCAACAAAGTGACACTTGCAGATG
CTGGCTTCATCAAACAATATGGTGATTGCCTTGGTGATATTGCTGCTAGAGACCTCATTTGTGCACAAAAGTTTAACGGCCT
TACTGTTTTGCCACCTTTGCTCACAGATGAAATGATTGCTCAATACACTTCTGCACTGTTAGCGGGTACAATCACTTCTGGTT
GGACCTTTGGTGCAGGTGCTGCATTACAAATACCATTTGCTATGCAAATGGCTTATAGGTTTAATGGTATTGGAGTTACAC
AGAATGTTCTCTATGAGAACCAAAAATTGATTGCCAACCAATTTAATAGTGCTATTGGCAAAATTCAAGACTCACTTTCTTC
CACAGCAAGTGCACTTGGAAAACTTCAAGATGTGGTCAACCAAAATGCACAAGCTTTAAACACGCTTGTTAAACAACTTAG
CTCCAATTTTGGTGCAATTTCAAGTGTTTTAAATGATATCCTTTCACGTCTTGACAAAGTTGAGGCTGAAGTGCAAATTGAT
AGGTTGATCACAGGCAGACTTCAAAGTTTGCAGACATATGTGACTCAACAATTAATTAGAGCTGCAGAAATCAGAGCTTCT
GCTAATCTTGCTGCTACTAAAATGTCAGAGTGTGTACTTGGACAATCAAAAAGAGTTGATTTTTGTGGAAAGGGCTATCAT
CTTATGTCCTTCCCTCAGTCAGCACCTCATGGTGTAGTCTTCTTGCATGTGACTTATGTCCCTGCACAAGAAAAGAACTTCAC
AACTGCTCCTGCCATTTGTCATGATGGAAAAGCACACTTTCCTCGTGAAGGTGTCTTTGTTTCAAATGGCACACACTGGTTT
GTAACACAAAGGAATTTTTATGAACCACAAATCATTACTACAGACAACACATTTGTGTCTGGTAACTGTGATGTTGTAATAG
GAATTGTCAACAACACAGTTTATGATCCTTTGCAACCTGAATTAGACTCATTCAAGGAGGAGTTAGATAAATATTTTAAGAA
TCATACATCACCAGATGTTGATTTAGGTGACATCTCTGGCATTAATGCTTCAGTTGTAAACATTCAAAAAGAAATTGACCGC
CTCAATGAGGTTGCCAAGAATTTAAATGAATCTCTCATCGATCTCCAAGAACTTGGAAAGTATGAGCAGTATATAAAATGG
CCATGGTACATTTGGCTAGGTTTTATAGCTGGCTTGATTGCCATAGTAATGGTGACAATTATGCTTTGCTGTATGACCAGTT
GCTGTAGTTGTCTCAAGGGCTGTTGTTCTTGTGGATCCTGCTGCAAATTTGATGAAGACGACTCTGAGCCAGTGCTCAAAG
GAGTCAAATTACATTACACATAAACGAACTTATGGATTTGTTTATGAGAATCTTCACAATTGGAACTGTAACTTTGAAGCAA
GGTGAAATCAAGGATGCTACTCCTTCAGATTTTGTTCGCGCTACTGCAACGATACCGATACAAGCCTCACTCCCTTTCGGAT
GGCTTATTGTTGGCGTTGCACTTCTTGCTGTTTTTCATAGCGCTTCCAAAATCATAACCCTCAAAAAGAGATGGCAACTAGC
ACTCTCCAAGGGTGTTCACTTTGTTTGCAACTTGCTGTTGTTTGTAACAGTTTACTCACACCTTTTGCTCGTTGCTGCTG
GCCTTGAAGCCCCTTTTCTCTATCTTTATGCTTTAGTCTACTTCTTGCAGAGTATAAACTTTGTAAGAATAATAATGAGGCTT
TGGCTTTGCTGGAAATGCCGTTCCAAAAACCCATTACTTTATGATGCCAACTATTTTCTTTGCTGGCATACTAATTGTTACGA
CTATTGTATACCTTACAATAGTGTAACTTCTTCAATTGTCATTACTTCAGGTGATGGCACAACAAGTCCTATTTCTGAACATG
ACTACCAGATTGGTGGTTATACTGAAAAATGGGAATCTGGAGTAAAAGACTGTGTTGTATTACACAGTTACTTCACTTCAG
ACTATTACCAGCTGTACTCAACTCAATTGAGTACAGACACTGGTGTTGAACATGTTACCTTCTTCATCTACAATAAAATTGTT
GATGAGCCTGAAGAACATGTCCAAATTCACACAATCGACGGTTCATCCGGAGTTGTTAATCCAGTAATGGAACCAATTTAT
GATGAACCGACGACGACTACTAGCGTGCCTTTGTAAGCACAAGCTGATGAGTACGAACTTATGTACTCATTCGTTTCGGAA
GAGACAGGTACGTTAATAGTTAATAGCGTACTTCTTTTTCTTGCTTTCGTGGTATTCTTGCTAGTTACACTAGCCATCCTTAC
TGCGCTTCGATTGTGTGCGTACTGCTGCAATATTGTTAACGTGAGTCTTGTAAAACCTTCTTTTTACGTTTACTCTCGTGTTA
AAAATCTGAATTCTTCTAGAGTTCCTGATCTTCTGGTCTAAACGAACTAAATATTATATTAGTTTTTCTGTTTGGAACTTTAAT
TTTAGCCATGGTAGATTCCAACGGTACTATTACCGTTGAAGAGCTTAAAAAGCTCCTTGAACAATGGAACCTAGTAATAGG
TTTCCTATTCCTTACATGGATTTGTCTTCTACAATTTGCCTATGCCAACAGGAATAGGTTTTTGTATATAATTAAGTTAATTTT
TCTCTGGCTGTTATGGCCAGTAACTTTAGCTTGTTTTGTGCTTGCTGCTGTTTACAGAATAAATTGGATCACCGGTGGAATT
GCTATCGCAATGGCTTGTCTTGTAGGCTTGATGTGGCTCAGCTACTTCATTGCTTCTTTCAGACTGTTTGCGCGTACGCGTT
CCATGTGGTCATTCAATCCAGAAACTAACATTCTTCTCAACGTGCCACTCCATGGCACTATTCTGACCAGACCGCTTCTAGA
AAGTGAACTCGTAATCGGAGCTGTGATCCTTCGTGGACATCTTCGTATTGCTGGACACCATCTAGGACGCTGTGACATCAA
GGACCTGCCTAAAGAAATCACTGTTGCTACATCACGAACGCTTTCTTATTACAAATTGGGAGCTTCGCAGCGTGTAGCAGG
TGACTCAGGTTTTGCTGCATACAGTCGCTACAGGATTGGCAACTATAAATTAAACACAGACCATTCCAGTAGCAGTGACAA
TATTGCTTTGCTTGTACAGTAAGTGACAACAGATGTTTCATCTCGTTGACTTTCAGGTTACTATAGCAGAGATATTACTAATT
ATTATGAGGACTTTTAAAGTTTCCATTTGGAATCTTGATTACATCATAAACCTCATAATTAAAAATTTATCTAAGTCACTAAC
TGAGAATAAATATTCTCAATTAGATGAAGAGCAACCAATGGAGATTGATTAAACGAACATGAAAATTATTCTTTTCTTGGC
ACTGATAACACTCGCTACTTGTGAGCTTTATCACTACCAAGAGTGTGTTAGAGGTACAACAGTACTTTTAAAAGAACCTTGC
TCTTCTGGAACATACGAGGGCAATTCACCATTTCATCCTCTAGCTGATAACAAATTTGCACTGACTTGCTTTAGCACTCAATT
TGCTTTTGCTTGTCCTGACGGCGTAAAACACGTCTATCAGTTACGTGCCAGATCAGTTTCACCTAAACTGTTCATCAGACAA
GAGGAAGTTCAAGAACTTTACTCTCCAATTTTTCTTATTGTTGCGGCAATAGTGTTTATAACACTTTGCTTCACACTCAAAAG
AAAGACAGAATGATTGAACTTTCATTAATTGACTTCTATTTGTGCTTTTTAGCCTTTCTGCTATTCCTTGTTTTAATTATGCTT
ATTATCTTTTGGTTCTCACTTGAACTGCAAGATCATAATGAAACTTGTCACGCCTAAACGAACATGAAATTTCTTGTTTTCTT
 
AGGAATCATCACAACTGTAGCTGCATTTCACCAAGAATGTAGTTTACAGTCATGTACTCAACATCAACCATATGTAGTTGAT
GACCCGTGTCCTATTCACTTCTATTCTAAATGGTATATTAGAGTAGGAGCTAGAAAATCAGCACCTTTAATTGAATTGTGCG
TGGATGAGGCTGGTTCTAAATCACCCATTCAGTACATCGATATCGGTAATTATACAGTTTCCTGTTTACCTTTTACAATTAAT
TGCCAGGAACCTAAATTGGGTAGTCTTGTAGTGCGTTGTTCGTTCTATGAAGACTTTTTAGAGTATCATGACGTTCGTGTTG
TTTTAGATTTCATCTAAACGAACAAACTATAATGTCTGATAATGGACCCCAAAATCAGCGAAATGCACCCCGCATTACGTTT
GGTGGACCCTCAGATTCAACTGGCAGTAACCAGAATGGAGAACGCAGTGGGGCGCGATCAAAACAACGTCGGCCCCAAG
GTTTACCCAATAATACTGCGTCTTGGTTCACCGCTCTCACTCAACATGGCAAGGAAGACCTTAAATTCCCTCGAGGACAAG
GCGTTCCAATTAACACCAATAGCAGTCCAGATGACCAAATTGGCTACTACCGAAGAGCTACCAGACGAATTCGTGGTGGT
GACGGTAAAATGAAAGATCTCAGTCCAAGATGGTATTTCTACTACCTAGGAACTGGGCCAGAAGCTGGACTTCCCTATGGT
GCTAACAAAGACGGCATCATATGGGTTGCAACTGAGGGAGCCTTGAATACACCAAAAGATCACATTGGCACCCGCAATCC
TGCTAACAATGCTGCAATCGTGCTACAACTTCCTCAAGGAACAACATTGCCAAAAGGCTTCTACGCAGAAGGGAGCAGAG
GCGGCAGTCAAGCCTCTTCTCGTTCCTCATCACGTAGTCGCAACAGTTCAAGAAATTCAACTCCAGGCAGCAGTAGGGGAA
TTTCTCCTGCTAGAATGGCTGGCAATGGCGGTGATGCTGCTCTTGCTTTGCTGCTGCTTGACAGATTGAACCAGCTTGAGA
GCAAAATGTCTGGTAAAGGCCAACAACAACAAGGCCAAACTGTCACTAAGAAATCTGCTGCTGAGGCTTCTAAGAAGCCT
CGGCAAAAACGTACTGCCACTAAAGCATACAATGTAACACAAGCTTTCGGCAGACGTGGTCCAGAACAAACCCAAGG
AAATTTTGGGGACCAGGAACTAATCAGACAAGGAACTGATTACAAACATTGGCCGCAAATTGCACAATTTGCCCCCAGCG
CTTCAGCGTTCTTCGGAATGTCGCGCATTGGCATGGAAGTCACACCTTCGGGAACGTGGTTGACCTACACAGGTGCCATCA
AATTGGATGACAAAGATCCAAATTTCAAAGATCAAGTCATTTTGCTGAATAAGCATATTGACGCATACAAAACATTTCCACC
AACAGAGCCTAAAAAGGACAAAAAGAAGAAGGCTGATGAAACTCAAGCCTTACCGCAGAGACAGAAGAAACAGCAAACT
GTGACTCTTCTTCCTGCTGCAGATTTGGATGATTTCTCCAAACAATTGCAACAATCCATGAGCAGTGCTGACTCAACTCAGG
CCTAAACTCATGCAGACCACACAAGGCAGATGGGCTATATAAACGTTTTCGCTTTTCCGTTTACGATATATAGTCTACTCTT
GTGCAGAATGAATTCTCGTAACTACATAGCACAAGTAGATGTAGTTAACTTTAATCTCACATAGCAATCTTTAATCAGTGTG
TAACATTAGGGAGGACTTGAAAGAGCCACCACATTTTCACCGAGGCCACGCGGAGTACGATCGAGTGTACAGTGAACAAT
GCTAGGGAGAGCTGCCTATATGGAAGAGCCCTAATGTGTAAAATTAATTTTAGTAGTGCTATCC
SEQ ID NO: 25
   >QQV21856.1: S surface protein 
MFVFLVLLPLVSIQCVNLTTRTQLPPAYTNSFTRGVYYPDKVFRSSVLHSTQDLFLPFFSNVTWFHAIHVSGTNGTKRFDNPVLPF
NDGVYFASTEKSNIIRGWIFGTTLDSKTQSLLIVNNATNVVIKVCEFQFCNDPFLGVYYHKNNKSCMESEFRVYSSANNCTFEYVS
QPFLMDLEGKQGNFKNLREFVFKNIDGYFKIYSKHTPINLVRDLPQGFSALEPLVDLPIGINITRFQTLLALHRSYLTPGDSSSGWT
AGAAAYYVGYLQPRTFLLKYNENGTITDAVDCALDPLSETKCTLKSFTVEKGIYQTSNFRVQPTESIVRFPNITNLCPFGEVFNATR
FASVYAWNRKRISNCVADYSVLYNSASFSTFKCYGVSPTKLNDLCFTNVYADSFVIRGDEVRQIAPGQTGKIADYNYKLPDDFTG
CVIAWNSNNLDSKVGGNYNYRYRLFRKSNLKPFERDISTEIYQAGSTPCNGVEGFNCYFPLQSYGFQPTNGVGYQPYRVVVLSF
ELLHAPATVCGPKKSTNLVKNKCVNFNFNGLTGTGVLTESNKKFLPFQQFGRDIADTTDAVRDPQTLEILDITPCSFGGVSVITPG
TNTSNQVAVLYQGVNCTEVPVAIHADQLTPTWRVYSTGSNVFQTRAGCLIGAEHVNNSYECDIPIGAGICASYQTQTNSPRRAR
SVASQSIIAYTMSLGAENSVAYSNNSIAIPTNFTISVTTEILPVSMTKTSVDCTMYICGDSTECSNLLLQYGSFCTQLNRALTGIAVE
QDKNTQEVFAQVKQIYKTPPIKDFGGFNFSQILPDPSKPSKRSFIEDLLFNKVTLADAGFIKQYGDCLGDIAARDLICAQKFNGLTV
LPPLLTDEMIAQYTSALLAGTITSGWTFGAGAALQIPFAMQMAYRFNGIGVTQNVLYENQKLIANQFNSAIGKIQDSLSSTASAL
GKLQDVVNQNAQALNTLVKQLSSNFGAISSVLNDILSRLDKVEAEVQIDRLITGRLQSLQTYVTQQLIRAAEIRASANLAATKMSE
CVLGQSKRVDFCGKGYHLMSFPQSAPHGVVFLHVTYVPAQEKNFTTAPAICHDGKAHFPREGVFVSNGTHWFVTQRNFYEPQ
IITTDNTFVSGNCDVVIGIVNNTVYDPLQPELDSFKEELDKYFKNHTSPDVDLGDISGINASVVNIQKEIDRLNEVAKNLNESLIDL
QELGKYEQYIKWPWYIWLGFIAGLIAIVMVTIMLCCMTSCCSCLKGCCSCGSCCKFDEDDSEPVLKGVKLHYT
SEQ ID NO: 26
   >MW306426.1 Severe acute respiratory syndrome coronavirus 2 isolate SARS-CoV-2/human/USA/CA-CZB-12872/2020, complete genome. [Severe acute respiratory syndrome coronavirus 2 (SARS-CoV-2)]. Californian B.1.429 lineage 
ACTTTCGATCTCTTGTAGATCTGTTCTCTAAACGAACTTTAAAATCTGTGTGGCTGTCACTCGGCTGCATGCTTAGTGCACTC
ACGCAGTATAATTAATAACTAATTACTGTCGTTGACAGGACACGAGTAACTCGTCTATCTTCTGCAGGCTGCTTACGGTTTC
GTCCGTGTTGCAGCCGATCATCAGCACATCTAGGTTTTGTCCGGGTGTGACCGAAAGGTAAGATGGAGAGCCTTGTCCCT
GGTTTCAACGAGAAAACACACGTCCAACTCAGTTTGCCTGTTTTACAGGTTCGCGACGTGCTCGTACGTGGCTTTGGAGAC
TCCGTGGAGGAGGTCTTATCAGAGGCACGTCAACATCTTAAAGATGGCACTTGTGGCTTAGTAGAAGTTGAAAAAGGCGT
TTTGCCTCAACTTGAACAGCCCTATGTGTTCATCAAACGTTCGGATGCTCGAACTGCACCTCATGGTCATGTTATGGTTGAG
CTGGTAGCAGAACTCGAAGGCATTCAGTACGGTCGTAGTGGTGAGACACTTGGTGTCCTTGTCCCTCATGTGGGCGAAAT
ACCAGTGGCTTACCGCAAGGTTCTTCTTCGTAAGAACGGTAATAAAGGAGCTGGTGGCCATAGTTACGGCGCCGATCTAA
 
AGTCATTTGACTTAGGCGACGAGCTTGGCACTGATCCTTATGAAGATTTTCAAGAAAACTGGAACACTAAACATAGCAGTG
GTGTTACCCGTGAACTCATGCGTGAGCTTAACGGAGGGGCATACACTCGCTATGTCGATAACAACTTCTGTGGCCCTGATG
GCTACCCTCTTGAGTGCATTAAAGACCTTCTAGCACGTGCTGGTAAAGCTTCATGCACTTTGTCCGAACAACTGGACTTTAT
TGACACTAAGAGGGGTGTATACTGCTGCCGTGAACATGAGCATGAAATTGCTTGGTACACGGAACGTTCTGAAAAGAGCT
ATGAATTGCAGACACCTTTTGAAATTAAATTGGCAAAGAAATTTGACATCTTCAATGGGGAATGTCCAAATTTTGTATTTCC
CTTAAATTCCATAATCAAGACTATTCAACCAAGGGTTGAAAAGAAAAAGCTTGATGGCTTTATGGGTAGAATTCGATCTGT
CTATCCAGTTGCGTCACCAAATGAATGCAACCAAATGTGCCTTTCAACTCTCATGAAGTGTGATCATTGTGGTGAAACTTCA
TGGCAGACGGGCGATTTTGTTAAAGCCACTTGCGAATTTTGTGGCACTGAGAATTTGACTAAAGAAGGTGCCACTACTTGT
GGTTACTTACCCCAAAATGCTGTTGTTAAAATTTATTGTCCAGCATGTCACAATTCAGAAGTAGGACCTGAGCATAGTCTTG
CCGAATACCATAATGAATCTGGCTTGAAAACCATTCTTCGTAAGGGTGGTCGCACTATTGCCTTTGGAGGCTGTGTGTTCTC
TTATGTTGGTTGCCATAACAAGTGTGCCTATTGGGTTCCACGTGCTAGCGCTAACATAGGTTGTAACCATACAGGTGTTGTT
GGAGAAGGTTCCGAAGGTCTTAATGACAACCTTCTTGAAATACTCCAAAAAGAGAAAGTCAACATCAATATTGTTGGTGAC
TTTAAACTTAATGAAGAGATCGCCATTATTTGGCATCTTTTCTGCTTCCACAAGTGCTTTTGTGGAAACTGTGAAAGGTTT
GGATTATAAAGCATTCAAACAAATTGTTGAATCCTGTGGTAATTTTAAAGTTACAAAAGGAAAAGCTAAAAAAGGTGCCTG
GAATATTGGTGAACAGAAATCAATACTGAGTCCTCTTTATGCATTTGCATCAGAGGCTGCTCGTGTTGTACGATCAATTTTC
TCCCGCACTCTTGAAACTGCTCAAAATTCTGTGCGTGTTTTACAGAAGGCCGCTATAACAATACTAGATGGAATTTCACAGT
ATTCACTGAGACTCATTGATGCTATGATGTTCACATCTGATTTGGCTACTAACAATCTAGTTGTAATGGCCTACATTACAGG
TGGTGTTGTTCAGTTGACTTCGCAGTGGCTAACTAACATCTTTGGCACTGTTTATGAAAAACTCAAACCCGTCCTTGATTGG
CTTGAAGAGAAGTTTAAGGAAGGTGTAGAGTTTCTTAGAGACGGTTGGGAAATTGTTAAATTTATCTCAACCTGTGCTTGT
GAAATTGTCGGTGGACAAATTGTCACCTGTGCAAAGGAAATTAAGGAGAGTGTTCAGACATTCTTTAAGCTTGTAAATAAA
TTTTTGGCTTTGTGTGCTGACTCTATCATTATTGGTGGAGCTAAACTTAAAGCCTTGAATTTAGGTGAAACATTTGTTACGC
ACTCAAAGGGATTGTACAGAAAGTGTGTTAAATCCAGAGAAGAAACTGGCCTACTCATGCCTCTAAAAGCCCCAAAAGAA
ATTATCTTCTTAGAGGGAGAAACACTTCCCACAGAAGTGTTAACAGAGGAAGTTGTCTTGAAAACTGGTGATTTACAACCA
TTAGAACAACCTACTAGTGAAGCTGTTGAAGCTCCACTGGTTGGTACACCAGTTTGTATTAACGGGCTTATGTTGCTCGAA
ATCAAAGACACAGAAAAGTACTGTGCCCTTGCACCTAATATGATGGTAACAAACAATACCTTCACACTCAAAGGCGGTGCA
CCAACAAAGGTTACTTTTGGTGATGACACTGTGATAGAAGTGCAAGGTTACAAGAGTGTGAATATCACTTTTGAACTTGAT
GAAAGGATTGATAAAGTACTTAATGAGAAGTGCTCTGCCTATACAGTTGAACTCGGTACAGAAGTAAATGAGTTCGCCTGT
GTTGTGGCAGATGCTGTCATAAAAACTTTGCAACCAGTATCTGAATTACTTACACCACTGGGCATTGATTTAGATGAGTGG
AGTATGGCTACATACTACTTATTTGATGAGTCTGGTGAGTTTAAATTGGCTTCACATATGTATTGTTCTTTTTACCCTCCAGA
TGAGGATGAAGAAGAAGGTGATTGTGAAGAAGAAGAGTTTGAGCCATCAACTCAATATGAGTATGGTACTGAAGATGAT
TACCAAGGTAAACCTTTGGAATTTGGTGCCACTTCTGCTGCTCTTCAACCTGAAGAAGAGCAAGAAGAAGATTGGTTAGAT
GATGATAGTCAACAAACTGTTGGTCAACAAGACGGCAGTGAGGACAATCAGACAACTACTATTCAAACAATTGTTGAGGT
TCAACCTCAATTAGAGATGGAACTTACACCAGTTGTTCAGACTATTGAAGTGAATAGTTTTAGTGGTTATTTAAAACTTACT
GACAATGTATACATTAAAAATGCAGACATTGTGGAAGAAGCTAAAAAGGTAAAACCAACAGTGGTTGTTAATGCAGCCAA
TGTTTACCTTAAACATGGAGGAGGTGTTGCAGGAGCCTTAAATAAGGCTACTAACAATGCCATGCAAGTTGAATCTGATGA
TTACATAGCTACTAATGGACCACTTAAAGTGGGTGGTAGTTGTGTTTTAAGCGGACACAATCTTGCTAAACACTGTCTTCAT
GTTGTCGGCCCAAATGTTAACAAAGGTGAAGACATTCAACTTCTTAAGAGTGCTTATGAAAATTTTAATCAGCACGAAGTT
CTACTTGCACCATTATTATCAGCTGGTATTTTTGGTGCTGACCCTATACATTCTTTAAGAGTTTGTGTAGATACTGTTCGCAC
AAATGTCTACTTAGCTGTCTTTGATAAAAATCTCTATGACAAACTTGTTTCAAGCTTTTTGGAAATGAAGAGTGAAAAGCAA
GTTGAACAAAAGATCGCTGAGATTCCTAAAGAGGAAGTTAAGCCATTTATAACTGAAAGTAAACCTTCAGTTGAACAGAG
AAAACAAGATGATAAGAAAATCAAAGCTTGTGTTGAAGAAGTTACAACAACTCTGGAAGAAACTAAGTTCCTCACAGAAA
ACTTGTTACTTTATATTGACATTAATGGCAATCTTCATCCAGATTCTGCCACTCTTGTTAGTGACATTGACATCACTTTCTTAA
AGAAAGATGCTCCATATATAGTGGGTGATGTTGTTCAAGAGGGTGTTTTAACTGCTGTGGTTATACCTACTAAAAAGGCTG
GTGGCACTACTGAAATGCTAGCGAAAGCTTTGAGAAAAGTGCCAACAGACAATTATATAACCACTTACCCGGGTCAGGGT
TTAAATGGTTACACTGTAGAGGAGGCAAAGACAGTGCTTAAAAAGTGTAAAAGTGCCTTTTACATTCTACCATCTATTATCT
CTAATGAGAAGCAAGAAATTCTTGGAACTGTTTCTTGGAATTTGCGAGAAATGCTTGCACATGCAGAAGAAACACGCAAA
TTAATGCCTGTCTGTGTGGAAACTAAAGCCATAGTTTCAACTATACAGCGTAAATATAAGGGTATTAAAATACAAGAGGGT
GTGGTTGATTATGGTGCTAGATTTTACTTTTACACCAGTAAAACAACTGTAGCGTCACTTATCAACACACTTAACGATCTAA
ATGAAACTCTTGTTACAATGCCACTTGGCTATGTAACACATGGCTTAAATTTGGAAGAAGCTGCTCGGTATATGAGATCTCT
CAAAGTGCCAGCTACAGTTTCTGTTTCTTCACCTGATGCTGTTACAGCGTATAATGGTTATCTTACTTCTTCTTCTAAAACAC
CTGAAGAACATTTTATTGAAACCATCTCACTTGCTGGTTCCTATAAAGATTGGTCCTATTCTGGACAATCTACACAACTAGG
TATAGAATTTCTTAAGAGAGGTGATAAAAGTGTATATTACACTAGTAATCCTACCACATTCCACCTAGATGGTGAAGTTATC
ACCTTTGACAATCTTAAGACACTTCTTTCTTTGAGAGAAGTGAGGACTATTAAGGTGTTTACAACAGTAGACAACATTAACC
TCCACACGCAAGTTGTGGACATGTCAATGACATATGGACAACAGTTTGGTCCAACTTATTTGGATGGAGCTGATGTTACTA
AAATAAAACCTCATAATTCACATGAAGGTAAAACATTTTATGTTTTACCTAATGATGACACTCTACGTGTTGAGGCTTTTGA
 
GTACTACCACACAACTGATCCTAGTTTTCTGGGTAGGTACATGTCAGCATTAAATCACACTAAAAAGTGGAAATACCCACA
AGTTAATGGTTTAACTTCTATTAAATGGGCAGATAACAACTGTTATCTTGCCACTGCATTGTTAACACTCCAACAAATAGAG
TTGAAGTTTAATCCACCTGCTCTACAAGATGCTTATTACAGAGCAAGGGCTGGTGAAGCTGCTAACTTTTGTGCACTTATCT
TAGCCTACTGTAATAAGACAGTAGGTGAGTTAGGTGATGTTAGAGAAACAATGAGTTACTTGTTTCAACATGCCAATTTAG
ATTCTTGCAAAAGAGTCTTGAACGTGGTGTGTAAAACTTGTGGACAACAGCAGACAACCCTTAAGGGTGTAGAAGCTGTT
ATGTACATGGGCACACTTTCTTATGAACAATTTAAGAAAGGTGTTCAGATACCTTGTACGTGTGGTAAACAAGCTACAAAA
TATCTAGTACAACAGGAGTCACCTTTTGTTATGATGTCAGCACCACCTGCTCAGTATGAACTTAAGCATGGTACATTTACTT
GTGCTAGTGAGTACACTGGTAATTACCAGTGTGGTCACTATAAACATATAACTTCTAAAGAAACTTTGTATTGCATAGACG
GTGCTTTACTTACAAAGTCCTCAGAATACAAAGGTCCTATTACGGATGTTTTCTACAAAGAAAACAGTTACACAACAACCAT
AAAACCAGTTACTTATAAATTGGATGGTGTTGTTTGTACAGAAATTGACCCTAAGTTGGACAATTATTATAAGAAAGACAA
TTCTTATTTCACAGAGCAACCAATTGATCTTGTACCAAACCAACCATATCCAAACGCAAGCTTCGATAATTTTAAGTTTGTA
TGTGATAATATCAAATTTGCTGATGATTTAAACCAGTTAACTGGTTATAAGAAACCTGCTTCAAGAGAGCTTAAAGTTACAT
TTTTCCCTGACTTAAATGGTGATGTGGTGGCTATTGATTATAAACACTACACACCCTCTTTTAAGAAAGGAGCTAAATTGTT
ACATAAACCTATTGTTTGGCATGTTAACAATGCAACTAATAAAGCCACGTATAAACCAAATACCTGGTGTATACGTTGTCTT
TGGAGCACAAAACCAGTTGAAACATCAAATTCGTTTGATGTACTGAAGTCAGAGGACGCGCAGGGAATGGATAATCTTGC
CTGCGAAGATCTAAAACCAGTCTCTGAAGAAGTAGTGGAAAATCCTACCATACAGAAAGACGTTCTTGAGTGTAATGTGA
AAACTACCGAAGTTGTAGGAGACATTATACTTAAACCAGCAAATAATAGTTTAAAAATTACAGAAGAGGTTGGCCACACA
GATCTAATGGCTGCTTATGTAGACAATTCTAGTCTTACTATTAAGAAACCTAATGAATTATCTAGAGTATTAGGTTTGAAAA
CCCTTGCTACTCATGGTTTAGCTGCTGTTAATAGTGTCCCTTGGGATACTATAGCTAATTATGCTAAGCCTTTTCTTAACAAA
GTTGTTAGTACAACTACTAACATAGTTACACGGTGTTTAAACCGTGTTTGTACTAATTATATGCCTTATTTCTTTACTTTATTG
CTACAATTGTGTACTTTTACTAGAAGTACAAATTCTAGAATTAAAGCATCTATGCCGACTACTATAGCAAAGAATACTGTTA
AGAGTGTCGGTAAATTTTGTCTAGAGGCTTCATTTAATTATTTGAAGTCACCTAATTTTTCTAAACTGATAAATATTATAATT
TGGTTTTTACTATTAAGTGTTTGCCTAGGTTCTTTAATCTACTCAACCGCTGCTTTAGGTGTTTTAATGTCTAATTTAGGCAT
GCCTTCTTACTGTACTGGTTACAGAGAAGGCTATTTGAACTCTACTAATGTCACTATTGCAACCTACTGTACTGGTTCTATAC
CTTGTAGTGTTTGTCTTAGTGGTTTAGATTCTTTAGACACCTATCCTTCTTTAGAAACTATACAAATTACCATTTCATCTTTTA
AATGGGATTTAACTGCTTTTGGCTTAGTTGCAGAGTGGTTTTTGGCATATATTCTTTTCACTAGGTTTTTCTATGTACTTGGA
TTGGCTGCAATCATGCAATTGTTTTTCAGCTATTTTGCAGTACATTTTATTAGTAATTCTTGGCTTATGTGGTTAATAATTAAT
CTTGTACAAATGGCCCCGATTTCAGCTATGGTTAGAATGTACATCTTCTTTGCATCATTTTATTATGTATGGAAAAGTTATGT
GCATGTTGTAGACGGTTGTAATTCATCAACTTGTATGATGTGTTACAAACGTAATAGAGCAACAAGAGTCGAATGTACAAC
TATTGTTAATGGTGTTAGAAGGTCCTTTTATGTCTATGCTAATGGAGGTAAAGGCTTTTGCAAACTACACAATTGGAATTGT
GTTAATTGTGATACATTCTGTGCTGGTAGTACATTTATTAGTGATGAAGTTGCGAGAGACTTGTCACTACAGTTTAAAAGAC
CAATAAATCCTACTGACCAGTCTTCTTACATCGTTGATAGTGTTACAGTGAAGAATGGTTCCATCCATCTTTACTTTGATAAA
GCTGGTCAAAAGACTTATGAAAGACATTCTCTCTCTCATTTTGTTAACTTAGACAACCTGAGAGCTAATAACACTAAAGGTT
CATTGCCTATTAATGTTATAGTTTTTGATGGTAAATCAAAATGTGAAGAATCATCTGCAAAATCAGCGTCTGTTTACTACAG
TCAGCTTATGTGTCAACCTATACTGTTACTAGATCAGGCATTAGTGTCTGATGTTGGTGATAGTGCGGAAGTTGCAGTTAA
AATGTTTGATGCTTACGTTAATACGTTTTCATCAACTTTTAACGTACCAATGGAAAAACTCAAAACACTAGTTGCAACTGCA
GAAGCTGAACTTGCAAAGAATGTGTCCTTAGACAATGTCTTATCTACTTTTATTTCAGCAGCTCGGCAAGGGTTTGTTGATT
CAGATGTAGAAACTAAAGATGTTGTTGAATGTCTTAAATTGTCACATCAATCTGACATAGAAGTTACTGGCGATAGTTGTA
ATAACTATATGCTCACCTATAACAAAGTTGAAAACATGACACCCCGTGACCTTGGTGCTTGTATTGACTGTAGTGCGCGTCA
TATTAATGCGCAGGTAGCAAAAAGTCACAACATTGCTTTGATATGGAACGTTAAAGATTTCATGTCATTGTCTGAACAACTA
CGAAAACAAATACGTAGTGCTGCTAAAAAGAATAACTTACCTTTTAAGTTGACATGTGCAACTACTAGACAAGTTGTTAAT
GTTGTAACAACAAAGATAGCACTTAAGGGTGGTAAAATTGTTAATAATTGGTTGAAGCAGTTAATTAAAGTTACACTTGTG
TTCCTTTTTGTTGCTGCTATTTTCTATTTAATAACACCTGTTCATGTCATGTCTAAACATACTGACTTTTCAAGTGAAATCATA
GGATACAAGGCTATTGATGGTGGTGTCACTCGTGACATAGCATCTACAGATACTTGTTTTGCTAACAAACATGCTGATTTTG
ACACATGGTTTAGCCAGCGTGGTGGTAGTTATACTAATGACAAAGCTTGCCCATTGATTGCTGCAGTCATAACAAGAGAAG
TGGGTTTTGTCGTGCCTGGTTTGCCTGGCACGATATTACGCACAACTAATGGTGACTTTTTGCATTTCTTACCTAGAGTTTTT
AGTGCAGTTGGTAATATCTGTTACACACCATCAAAACTTATAGAGTACACTGACTTTGCAACATCAGCTTGTGTTTTGGCTG
CTGAATGTACAATTTTTAAAGATGCTTCTGGTAAGCCAGTACCATATTGTTATGATACCAATGTACTAGAAGGTTCTGTTGC
TTATGAAAGTTTACGCCCTGACACACGTTATGTGCTCATGGATGGCTCTATTATTCAATTTCCTAACACCTACCTTGAAGGTT
CTGTTAGAGTGGTAACAACTTTTGATTCTGAGTACTGTAGGCACGGCACTTGTGAAAGATCAGAAGCTGGTGTTTGTGTAT
CTACTAGTGGTAGATGGGTACTTAACAATGATTATTACAGATCTTTACCAGGAGTTTTCTGTGGTGTAGATGCTGTAAATTT
ACTTACTAATATGTTTACACCACTAATTCAACCTATTGGTGCTTTGGACATATCAGCATCTATAGTAGCTGGTGGTATTGTA
GCTATCGTAGTAACATGCCTTGCCTACTATTTTATGAGGTTTAGAAGAGCTTTTGGTGAATACAGTCATGTAGTTGCCTTTA
ATACTTTACTATTCCTTATGTCATTCACTGTACTCTGTTTAACACCAGTTTACTCATTCTTACCTGGTGTTTATTCTGTTATTTA
CTTGTACTTGACATTTTATCTTACTAATGATGTTTCTTTTTTAGCACATATTCAGTGGATGGTTATGTTCACACCTTTAGTACC
 
TTTCTGGATAACAATTGCTTATATCATTTGTATTTCCACAAAGCATTTCTATTGGTTCTTTAGTAATTACCTAAAGAGACGTG
TAGTCTTTAATGGTGTTTCCTTTAGTACTTTTGAAGAAGCTGCGCTGTGCACCTTTTTGTTAAATAAAGAAATGTATCTAAAG
TTGCGTAGTGATGTGCTATTACCTCTTACGCAATATAATAGATACTTAGCTCTTTATAATAAGTACAAGTATTTTAGTGGAG
CAATGGATACAACTAGCTACAGAGAAGCTGCTTGTTGTCATCTCGCAAAGGCTCTCAATGACTTCAGTAACTCAGGTTCTG
ATGTTCTTTACCAACCACCACAAACCTCTATCACCTCAGCTGTTTTGCAGAGTGGTTTTAGAAAAATGGCATTCCCATCTGGT
AAAGTTGAGGGTTGTATGGTACAAGTAACTTGTGGTACAACTACACTTAACGGTCTTTGGCTTGATGACGTAGTTTACTGT
CCAAGACATGTGATCTGCACCTCTGAAGACATGCTTAACCCTAATTATGAAGATTTACTCATTCGTAAGTCTAATCATAATTT
CTTGGTACAGGCTGGTAATGTTCAACTCAGGGTTATTGGACATTCTATGCAAAATTGTGTACTTAAGCTTAAGGTTGATACA
GCCAATCCTAAGACACCTAAGTATAAGTTTGTTCGCATTCAACCAGGACAGACTTTTTCAGTGTTAGCTTGTTACAATGGTT
CACCATCTGGTGTTTACCAATGTGCTATGAGGCCCAATTTCACTATTAAGGGTTCATTCCTTAATGGTTCATGTGGTAGTGT
TGGTTTTAACATAGATTATGACTGTGTCTCTTTTTGTTACATGCACCATATGGAATTACCAACTGGAGTTCATGCTGGCACA
GACTTAGAAGGTAACTTTTATGGACCTTTTGTTGACAGGCAAACAGCACAAGCAGCTGGTACGGACACAACTATTACAGTT
AATGTTTTAGCTTGGTTGTACGCTGCTGTTATAAATGGAGACAGGTGGTTTCTCAATCGATTTACCACAACTCTTAATGACT
TTAACCTTGTGGCTATGAAGTACAATTATGAACCTCTAACACAAGACCATGTTGACATACTAGGACCTCTTTCTGCTCAAAC
TGGAATTGCCGTTTTAGATATGTGTGCTTCATTAAAAGAATTACTGCAAAATGGTATGAATGGACGTACCATATTGGGTAG
TGCTTTATTAGAAGATGAATTTACACCTTTTGATGTTGTTAGACAATGCTCAGGTGTTACTTTCCAAAGTGCAGTGAAAAGA
ACAATCAAGGGTACACACCACTGGTTGTTACTCACAATTTTGACTTCACTTTTAGTTTTAGTCCAGAGTACTCAATGGTCTTT
GTTCTTTTTTTTGTATGAAAATGCCTTTTTACCTTTTGCTATGGGTATTATTGCTATGTCTGCTTTTGCAATGATGTTTGTCAA
ACATAAGCATGCATTTCTCTGTTTGTTTTTGTTACCTTCTCTTGCCACTGTAGCTTATTTTAATATGGTCTATATGCCTGCTAG
TTGGGTGATGCGTATTATGACATGGTTGGATATGGTTGATACTAGTTTGTCTGGTTTTAAGCTAAAAGACTGTGTTATGTAT
GCATCAGCTGTAGTGTTACTAATCCTTATGACAGCAAGAACTGTGTATGATGATGGTGCTAGGAGAGTGTGGACACTTATG
AATGTCTTGACACTCGTTTATAAAGTTTATTATGGTAATGCTTTAGATCAAGCCATTTCCATGTGGGCTCTTATAATCTCTGT
TACTTCTAACTACTCAGGTGTAGTTACAACTGTCATGTTTTTGGCCAGAGGTATTGTTTTTATGTGTGTTGAGTATTGCCCTA
TTTTCTTCATAACTGGTAATACACTTCAGTGTATAATGCTAGTTTATTGTTTCTTAGGCTATTTTTGTACTTGTTACTTTGGCC
TCTTTTGTTTACTCAACCGCTACTTTAGACTGACTCTTGGTGTTTATGATTACTTAGTTTCTACACAGGAGTTTAGATATATG
AATTCACAGGGACTACTCCCACCCAAGAATAGCATAGATGCCTTCAAACTCAACATTAAATTGTTGGGTGTTGGTGGCAAA
CCTTGTATCAAAGTAGCCACTGTACAGTCTAAAATGTCAGATGTAAAGTGCACATCAGTAGTCTTACTCTCAGTTTTGCAAC
AACTCAGAGTAGAATCATCATCTAAATTGTGGGCTCAATGTGTCCAGTTACACAATGACATTCTCTTAGCTAAAGATACTAC
TGAAGCCTTTGAAAAAATGGTTTCACTACTTTCTGTTTTGCTTTCCATGCAGGGTGCTGTAGACATAAACAAGCTTTGTGAA
GAAATGCTGGACAACAGGGCAACCTTACAAGCTATAGCTTCAGAGTTTAGTTCCCTTCCATCATATGCAGCTTTTGCTACTG
CTCAAGAAGCTTATGAGCAGGCTGTTGCTAATGGTGATTCTGAAGTTGTTCTTAAAAAGTTGAAGAAGTCTTTGAATGTGG
CTAAATCTGAATTTGACCGTGATGCAGCCATGCAACGTAAGTTGGAAAAGATGGCTGATCAAGCTATGACCCAAATGTATA
AACAGGCTAGATCTGAGGACAAGAGGGCAAAAGTTACTAGTGCTATGCAGACAATGCTTTTCACTATGCTTAGAAAGTTG
GATAATGATGCACTCAACAACATTATCAACAATGCAAGAGATGGTTGTGTTCCCTTGAACATAATACCTCTTACAACAGCA
GCCAAACTAATGGTTGTCATACCAGACTATAACACATATAAAAATACGTGTGATGGTACAACATTTACTTATGCATCAGCAT
TGTGGGAAATCCAACAGGTTGTAGATGCAGATAGTAAAATTGTTCAACTTAGTGAAATTAGTATGGACAATTCACCTAATT
TAGCATGGCCTCTTATTGTAACAGCTTTAAGGGCCAATTCTGCTGTCAAATTACAGAATAATGAGCTTAGTCCTGTTGCACT
ACGACAGATGTCTTGTGCTGCCGGTACTACACAAACTGCTTGCACTGATGACAATGCGTTAGCTTACTACAACACAACAAA
GGGAGGTAGGTTTGTACTTGCACTGTTATCCGATTTACAGGATTTGAAATGGGCTAGATTCCCTAAGAGTGATGGAACTG
GTACTGTCTATACAGAACTGGAACCACCTTGTAGGTTTGTTACAGACACACCTAAAGGTCCTAAAGTGAAGTATTTATACTT
TATTAAAGGATTAAACAACCTAAATAGAGGTATGGTACTTGGTAGTTTAGCTGCCACAGTACGTCTACAAGCTGGTAATGC
AACAGAAGTGCCTGCCAATTCAACTGTATTATCTTTCTGTGCTTTTGCTGTAGATGCTGCTAAAGCTTACAAAGATTATCTA
GCTAGTGGGGGACAACCAATCACTAATTGTGTTAAGATGTTGTGTACACACACTGGTACTGGTCAGGCAATAACAGTTACA
CCGGAAGCCAATATGGATCAAGAATCCTTTGGTGGTGCATCGTGTTGTCTGTACTGCCGTTGCCACATAGATCATCCAAAT
CCTAAAGGATTTTGTGACTTAAAAGGTAAGTATGTACAAATACCTACAACTTGTGCTAATGACCCTGTGGGTTTTACACTTA
AAAACACAGTCTGTACCGTCTGCGGTATGTGGAAAGGTTATGGCTGTAGTTGTGATCAACTCCGCGAACCCATGCTTCAGT
CAGCTGATGCACAATCGTTTTTAAACGGGTTTGCGGTGTAAGTGCAGCCCGTCTTACACCGTGCGGCACAGGCACTAGTAC
TGATGTCGTATACAGGGCTTTTGACATCTACAATGATAAAGTAGCTGGTTTTGCTAAATTCCTAAAAACTAATTGTTGTCGC
TTCCAAGAAAAGGACGAAGATGACAATTTAATTGATTCTTACTTTGTAGTTAAGAGACACACTTTCTCTAACTACCAACATG
AAGAAACAATTTATAATTTACTTAAGGATTGTCCAGCTGTTGCTAAACATGACTTCTTTAAGTTTAGAATAGACGGTGACAT
GGTACCACATATATCACGTCAACGTCTTACTAAATACACAATGGCAGACCTCGTCTATGCTTTAAGGCATTTTGATGAAGGT
AATTGTGACACATTAAAAGAAATACTTGTCACATACAATTGTTGTGATGATGATTATTTCAATAAAAAGGACTGGTATGATT
TTGTAGAAAACCCAGATATATTACGCGTATACGCCAACTTAGGTGAACGTGTACGCCAAGCTTTGTTAAAAACAGTACAAT
TCTGTGATGCCATGCGAAATGCTGGTATTGTTGGTGTACTGACATTAGATAATCAAGATCTCAATGGTAACTGGTATGATTT
CGGTGATTTCATACAAACCACGCCAGGTAGTGGAGTTCCTGTTGTAGATTCTTATTATTCATTGTTAATGCCTATATTAACC
 
TTGACCAGGGCTTTAACTGCAGAGTCACATGTTGACACTGACTTAACAAAGCCTTACATTAAGTGGGATTTGTTAAAATAT
GACTTCACGGAAGAGAGGTTAAAACTCTTTGACCGTTATTTTAAATATTGGGATCAGACATACCACCCAAATTGTGTTAACT
GTTTGGATGACAGATGCATTCTGCATTGTGCAAACTTTAATGTTTTATTCTCTACAGTGTTCCCACTTACAAGTTTTGGACCA
CTAGTGAGAAAAATATTTGTTGATGGTGTTCCATTTGTAGTTTCAACTGGATACCACTTCAGAGAGCTAGGTGTTGTACATA
ATCAGGATGTAAACTTACATAGCTCTAGACTTAGTTTTAAGGAATTACTTGTGTATGCTGCTGACCCTGCTATGCACGCTGC
TTCTGGTAATCTATTACTAGATAAACGCACTACGTGCTTTTCAGTAGCTGCACTTACTAACAATGTTGCTTTTCAAACTGTCA
AACCCGGTAATTTTAACAAAGACTTCTATGACTTTGCTGTGTCTAAGGGTTTCTTTAAGGAAGGAAGTTCTGTTGAATTAAA
ACACTTCTTCTTTGCTCAGGATGGTAATGCTGCTATCAGCGATTATGACTACTATCGTTATAATCTACCAACAATGTGTGATA
TCAGACAACTACTATTTGTAGTTGAAGTTGTTGATAAGTACTTTGATTGTTACGATGGTGGCTGTATTAATGCTAACCAAGT
CATCGTCAACAACCTAGACAAATCAGCTGGTTTTCCATTTAATAAATGGGGTAAGGCTAGACTTTATTATGATTCAATGAGT
TATGAGGATCAAGATGCACTTTTCGCATATACAAAACGTAATGTCATCCCTACTATAACTCAAATGAATCTTAAGTATGCCA
TTAGTGCAAAGAATAGAGCTCGCACCGTAGCTGGTGTCTCTATCTGTAGTACTATGACCAATAGACAGTTTCATCAAAAAT
TATTGAAATCAATAGCCGCCACTAGAGGAGCTACTGTAGTAATTGGAACAAGCAAATTCTATGGTGGTTGGCACAACATGT
TAAAAACTGTTTATAGTGATGTAGAAAACCCTCACCTTATGGGTTGGGATTATCCTAAATGTGATAGAGCCATGCCTAACA
TGCTTAGAATTATGGCCTCACTTGTTCTTGCTCGCAAACATACAACGTGTTGTAGCTTGTCACACCGTTTCTATAGATTAGCT
AATGAGTGTGCTCAAGTATTGAGTGAAATGGTCATGTGTGGCGGTTCACTATATGTTAAACCAGGTGGAACCTCATCAGG
AGATGCCACAACTGCTTATGCTAATAGTGTTTTTAACATTTGTCAAGCTGTCACGGCCAATGTTAATGCACTTTTATCTACTG
ATGGTAACAAAATTGCCGATAAGTATGTCCGCAATTTACAACACAGACTTTATGAGTGTCTCTATAGAAATAGAGATGTTG
ACACAGACTTTGTGAATGAGTTTTACGCATATTTGCGTAAACATTTCTCAATGATGATACTCTCTGACGATGCTGTTGTGTG
TTTCAATAGCACTTATGCATCTCAAGGTCTAGTGGCTAGCATAAAGAACTTTAAGTCAGTTCTTTATTATCAAAACAATGTTT
TTATGTCTGAAGCAAAATGTTGGACTGAGACTGACCTTACTAAAGGACCTCATGAATTTTGCTCTCAACATACAATGCTAGT
TAAACAGGGTGATGATTATGTGTACCTTCCTTACCCAGATCCATCAAGAATCCTAGGGGCCGGCTGTTTTGTAGATGATAT
CGTAAAAACAGATGGTACACTTATGATTGAACGGTTCGTGTCTTTAGCTATAGATGCTTACCCACTTACTAAACATCCTAAT
CAGGAGTATGCTGATGTCTTTCATTTGTACTTACAATACATAAGAAAGCTACATGATGAGTTAACAGGACACATGTTAGAC
ATGTATTCTGTTATGCTTACTAATGATAACACTTCAAGGTATTGGGAACCTGAGTTTTATGAGGCTATGTACACACCGCATA
CAGTCTTACAGGCTGTTGGGGCTTGTGTTCTTTGCAATTCACAGACTTCATTAAGATGTGGTGCTTGCATACGTAGACCATT
CTTATGTTGTAAATGCTGTTACGACCATGTCATATCAACATCACATAAATTAGTCTTGTCTGTTAATCCGTATGTTTGCAATG
CTCCAGGTTGTGATGTCACAGATGTGACTCAACTTTACTTAGGAGGTATGAGCTATTATTGTAAATCACATAAACCACCCAT
TAGTTTTCCATTGTGTGCTAATGGACAAGTTTTTGGTTTATATAAAAATACATGTGTTGGTAGCGATAATGTTACTGACTTTA
ATGCAATTGCAACATGTGACTGGACAAATGCTGGTGATTACATTTTAGCTAACACCTGTACTGAAAGACTCAAGCTTTTTGC
AGCAGAAACGCTCAAAGCTACTGAGGAGACATTTAAACTGTCTTATGGTATTGCTACTGTACGTGAAGTGCTGTCTGACAG
AGAATTACATCTTTCATGGGAAGTTGGTAAACCTAGACCACCACTTAACCGAAATTATGTCTTTACTGGTTATCGTGTAACT
AAAAACAGTAAAGTACAAATAGGAGAGTACACCTTTGAAAAAGGTGACTATGGTGATGCTGTTGTTTACCGAGGTACAAC
AACTTACAAATTAAATGTTGGTGATTATTTTGTGCTGACATCACATACAGTAATGCCATTAAGTGCACCTACACTAGTGCCA
CAAGAGCACTATGTTAGAATTACTGGCTTATACCCAACACTCAATATCTCATATGAGTTTTCTAGCAATGTTGCAAATTATC
AAAAGGTTGGTATGCAAAAGTATTCTACACTCCAGGGACCACCTGGTACTGGTAAGAGTCATTTTGCTATTGGCCTAGCTC
TCTACTACCCTTCTGCTCGCATAGTGTATACAGCTTGCTCTCATGCCGCTGTTGATGCACTATGTGAGAAGGCATTAAAATA
TTTGCCTATAGATAAATGTAGTAGAATTATACCTGCACGTGCTCGTGTAGAGTGTTTTGATAAATTCAAAGTGAATTCAACA
TTAGAACAGTATGTCTTTTGTACTGTAAATGCATTGCCTGAGACGACAGCAGATATAGTTGTCTTTGATGAAATTTCAATGG
CCACAAATTATGATTTGAGTGTTGTCAATGCCAGATTACGTGCTAAGCACTATGTGTACATTGGCGACCCTGCTCAATTACC
TGCACCACGCACATTGCTAACTAAGGGCACACTAGAACCAGAATATTTCAATTCAGTGTGTAGACTTATGAAAACTATAGG
TCCAGACATGTTCCTCGGAACTTGTCGGCGTTGTCCTGCTGAAATTGTTGACACTGTGAGTGCTTTGGTTTATGATAATAAG
CTTAAAGCACATAAAGACAAATCAGCTCAATGCTTTAAAATGTTTTATAAGGGTGTTATCACGCATGATGTTTCATCTGCAA
TTAACAGGCCACAAATAGGCGTGGTAAGAGAATTCCTTACACGTAACCCTGCTTGGAGAAAAGCTGTCTTTATTTCACCTT
ATAATTCACAGAATGCTGTAGCCTCAAAGATTTTGGGACTACCAACTCAAACTGTTGATTCATCACAGGGCTCAGAATATG
ACTATGTCATATTCACTCAAACCACTGAAACAGCTCACTCTTGTAATGTAAACAGATTTAATGTTGCTATTACCAGAGCAAA
AGTAGGCATACTTTGCATAATGTCTGATAGAGACCTTTATGACAAGTTGCAATTTACAAGTCTTGAAATTCCACGTAGGAAT
GTGGCAACTTTACAAGCTGAAAATGTAACAGGACTCTTTAAAGATTGTAGTAAGGTAATCACTGGGTTACATCCTACACAG
GCACCTACACACCTCAGTGTTGACACTAAATTCAAAACTGAAGGTTTATGTGTTGACATACCTGGCATACCTAAGGACATG
ACCTATAGAAGACTCATCTCTATGATGGGTTTTAAAATGAATTATCAAGTTAATGGTTACCCTAACATGTTTATCACCCGCG
AAGAAGCTATAAGACATGTACGTGCATGGATTGGCTTCGATGTCGAGGGGTGTCATGCTACTAGAGAAGCTGTTGGTACC
AATTTACCTTTACAGCTAGGTTTTTCTACAGGTGTTAACCTAGTTGCTGTACCTACAGGTTATGTTGATACACCTAATAATAC
AGATTTTTCCAGAGTTAGTGCTAAACCACCGCCTGGAGATCAATTTAAACACCTCATACCACTTATGTACAAAGGACTTCCT
TGGAATGTAGTGCGTATAAAGATTGTACAAATGTTAAGTGACACACTTAAAAATCTCTCTGACAGAGTCGTATTTGTCTTAT
GGGCACATGGCTTTGAGTTGACATCTATGAAGTATTTTGTGAAAATAGGACCTGAGCGCACCTGTTGTCTATGTGATAGAC
 
GTGCCACATGCTTTTCCACTGCTTCAGACACTTATGCCTGTTGGCATCATTCTATTGGATTTGATTACGTCTATAATCCGTTT
ATGATTGATGTTCAACAATGGGGTTTTACAGGTAACCTACAAAGCAACCATGATCTGTATTGTCAAGTCCATGGTAATGCA
CATGTAGCTAGTTGTGATGCAATCATGACTAGGTGTCTAGCTGTCCACGAGTGCTTTGTTAAGCGTGTTGACTGGACTATT
GAATATCCTATAATTGGTGATGAACTGAAGATTAATGCGGCTTGTAGAAAGGTTCAACACATGGTTGTTAAAGCTGCATTA
TTAGCAGACAAATTCCCAGTTCTTCACGACATTGGTAACCCTAAAGCTATTAAGTGTGTACCTCAAGCTGATGTAGAATGG
AAGTTCTATGATGCACAGCCTTGTAGTGACAAAGCTTATAAAATAGAAGAATTATTCTATTCTTATGCCACACATTCTGACA
AATTCACAGATGGTGTATGCCTATTTTGGAATTGCAATGTCGATAGATATCCTGCTAATTCCATTGTTTGTAGATTTGACACT
AGAGTGCTATCTAACCTTAACTTGCCTGGTTGTGATGGTGGCAGTTTGTATGTAAATAAACATGCATTCCACACACCAGCTT
TTGATAAAAGTGCTTTTGTTAATTTAAAACAATTACCATTTTTCTATTACTCTGACAGTCCATGTGAGTCTCATGGAAAACAA
GTAGTGTCAGATATAGATTATGTACCACTAAAGTCTGCTACGTGTATAACACGTTGCAATTTAGGTGGTGCTGTCTGTAGA
CATCATGCTAATGAGTACAGATTGTATCTCGATGCTTATAACATGATGATCTCAGCTGGCTTTAGCTTGTGGGTTTACAAAC
AATTTGATACTTATAACCTCTGGAACACTTTTACAAGACTTCAGAGTTTAGAAAATGTGGCTTTTAATGTTGTAAATAAGGG
ACACTTTGATGGACAACAGGGTGAAGTACCAGTTTCTATCATTAATAACACTGTTTACACAAAAGTTGATGGTGTTGATGT
AGAATTGTTTGAAAATAAAACAACATTACCTGTTAATGTAGCATTTGAGCTTTGGGCTAAGCGCAACATTAAACCAGTACC
AGAGGTGAAAATACTCAATAATTTGGGTGTGGACATTGCTGCTAATACTGTGATCTGGGACTACAAAAGAGATGCTCCAG
CACATATATCTACTATTGGTGTTTGTTCTATGACTGACATAGCCAAGAAACCAACTGAAACGATTTGTGCACCACTCACTGT
CTTTTTTGATGGTAGAGTTGATGGTCAAGTAGACTTATTTAGAAATGCCCGTAATGGTGTTCTTATTACAGAAGGTAGTGTT
AAAGGTTTACAACCATCTGTAGGTCCCAAACAAGCTAGTCTTAATGGAGTCACATTAATTGGAGAAGCCGTAAAAACACAG
TTCAATTATTATAAGAAAGTTGATGGTGTTGTCCAACAATTACCTGAAACTTACTTTACTCAGAGTAGAAATTTACAAGAAT
TTAAACCCAGGAGTCAAATGGAAATTGATTTCTTAGAATTAGCTATGGATGAATTCATTGAACGGTATAAATTAGAAGGCT
ATGCCTTCGAACATATCGTTTATGGAGATTTTAGTCATAGTCAGTTAGGTGGTTTACATCTACTGATTGGACTAGCTAAACG
TTTTAAGGAATCACCTTTTGAATTAGAAGATTTTATTCCTATGGACAGTACAGTTAAAAACTATTTCATAACAGATGCGCAA
ACAGGTTCATCTAAGTGTGTGTGTTCTGTTATTGATTTATTACTTGATGATTTTGTTGAAATAATAAAATCCCAAGATTTATC
TGTAGTTTCTAAGGTTGTCAAAGTGACTATTGACTATACAGAAATTTCATTTATGCTTTGGTGTAAAGATGGCCATGTAGAA
ACATTTTACCCAAAATTACAATCTAGTCAAGCGTGGCAACCGGGTGTTGCTATGCCTAATCTTTACAAAATGCAAAGAATGC
TATTAGAAAAGTGTGACCTTCAAAATTATGGTGATAGTGCAACATTACCTAAAGGCATAATGATGAATGTCGCAAAATATA
CTCAACTGTGTCAATATTTAAACACATTAACATTAGCTGTACCCTATAATATGAGAGTTATACATTTTGGTGCTGGTTCTGAT
AAAGGAGTTGCACCAGGTACAGCTGTTTTAAGACAGTGGTTGCCTACGGGTACGCTGCTTGTCGATTCAGATCTTAATGAC
TTTGTCTCTGATGCAGATTCAACTTTGATTGGTGATTGTGCAACTGTACATACAGCTAATAAATGGGATCTCATTATTAGTG
ATATGTACGACCCTAAGACTAAAAATGTTACAAAAGAAAATGACTCTAAAGAGGGTTTTTTCACTTACATTTGTGGGTTTAT
ACAACAAAAGCTAGCTCTTGGAGGTTCCGTGGCTATAAAGATAACAGAACATTCTTGGAATGCTGATCTTTATAAGCTCAT
GGGACACTTCGCATGGTGGACAGCCTTTGTTACTAATGTGAATGCGTCATCATCTGAAGCATTTTTAATTGGATGTAATTAT
CTTGGCAAACCACGCGAACAAATAGATGGTTATGTCATGCATGCAAATTACATATTTTGGAGGAATACAAATCCAATTCAG
TTGTCTTCCTATTCTTTATTTGACATGAGTAAATTTCCCCTTAAATTAAGGGGTACTGCTGTTATGTCTTTAAAAGAAGGTCA
AATCAATGATATGATTTTATCTCTTCTTAGTAAAGGTAGACTTATAATTAGAGAAAACAACAGAGTTGTTATTTCTAGTGAT
GTTCTTGTTAACAACTAAACGAACAATGTTTGTTTTTCTTGTTTTATTGCCACTAGTCTCTATTCAGTGTGTTAATCTTACAAC
CAGAACTCAATTACCCCCTGCATACACTAATTCTTTCACACGTGGTGTTTATTACCCTGACAAAGTTTTCAGATCCTCAGTTT
TACATTCAACTCAGGACTTGTTCTTACCTTTCTTTTCCAATGTTACTTGGTTCCATGCTATACATGTCTCTGGGACCAATGGT
ACTAAGAGGTTTGATAACCCTGTCCTACCATTTAATGATGGTGTTTATTTTGCTTCCACTGAGAAGTCTAACATAATAAGAG
GCTGGATTTTTGGTACTACTTTAGATTCGAAGACCCAGTCCCTACTTATTGTTAATAACGCTACTAATGTTGTTATTAAAGTC
TGTGAATTTCAATTTTGTAATGATCCATTTTTGGGTGTTTATTACCACAAAAACAACAAAAGTTGTATGGAAAGTGAGTTCA
GAGTTTATTCTAGTGCGAATAATTGCACTTTTGAATATGTCTCTCAGCCTTTTCTTATGGACCTTGAAGGAAAACAGGGTAA
TTTCAAAAATCTTAGGGAATTTGTGTTTAAGAATATTGATGGTTATTTTAAAATATATTCTAAGCACACGCCTATTAATTTAG
TGCGTGATCTCCCTCAGGGTTTTTCGGCTTTAGAACCATTGGTAGATTTGCCAATAGGTATTAACATCACTAGGTTTCAAAC
TTTACTTGCTTTACATAGAAGTTATTTGACTCCTGGTGATTCTTCTTCAGGTTGGACAGCTGGTGCTGCAGCTTATTATGTGG
GTTATCTTCAACCTAGGACTTTTCTATTAAAATATAATGAAAATGGAACCATTACAGATGCTGTAGACTGTGCACTTGACCC
TCTCTCAGAAACAAAGTGTACGTTGAAATCCTTCACTGTAGAAAAAGGAATCTATCAAACTTCTAACTTTAGAGTCCAACCA
ACAGAATCTATTGTTAGATTTCCTAATATTACAAACTTGTGCCCTTTTGGTGAAGTTTTTAACGCCACCAGATTTGCATCTGT
TTATGCTTGGAACAGGAAGAGAATCAGCAACTGTGTTGCTGATTATTCTGTCCTATATAATTCCGCATCATTTTCCACTTTTA
AGTGTTATGGAGTGTCTCCTACTAAATTAAATGATCTCTGCTTTACTAATGTCTATGCAGATTCATTTGTAATTAGAGGTGAT
GAAGTCAGACAAATCGCTCCAGGGCAAACTGGAAAGATTGCTGATTATAATTATAAATTACCAGATGATTTTACAGGCTGC
GTTATAGCTTGGAATTCTAACAATCTTGATTCTAAGGTTGGTGGTAATTATAATTACCGGTATAGATTGTTTAGGAAGTCTA
ATCTCAAACCTTTTGAGAGAGATATTTCAACTGAAATCTATCAGGCCGGTAGCACACCTTGTAATGGTGTTGAAGGTTTTAA
TTGTTACTTTCCTTTACAATCATATGGTTTCCAACCCACTAATGGTGTTGGTTACCAACCATACAGAGTAGTAGTACTTTCTT
TTGAACTTCTACATGCACCAGCAACTGTTTGTGGACCTAAAAAGTCTACTAATTTGGTTAAAAACAAATGTGTCAATTTCAA
 
CTTCAATGGTTTAACAGGCACAGGTGTTCTTACTGAGTCTAACAAAAAGTTTCTGCCTTTCCAACAATTTGGCAGAGACATT
GCTGACACTACTGATGCTGTCCGTGATCCACAGACACTTGAGATTCTTGACATTACACCATGTTCTTTTGGTGGTGTCAGTG
TTATAACACCAGGAACAAATACTTCTAACCAGGTTGCTGTTCTTTATCAGGGTGTTAACTGCACAGAAGTCCCTGTTGCTAT
TCATGCAGATCAACTTACTCCTACTTGGCGTGTTTATTCTACAGGTTCTAATGTTTTTCAAACACGTGCAGGCTGTTTAATAG
GGGCTGAACATGTCAACAACTCATATGAGTGTGACATACCCATTGGTGCAGGTATATGCGCTAGTTATCAGACTCAGACTA
ATTCTCCTCGGCGGGCACGTAGTGTAGCTAGTCAATCCATCATTGCCTACACTATGTCACTTGGTGCAGAAAATTCAGTTGC
TTACTCTAATAACTCTATTGCCATACCCACAAATTTTACTATTAGTGTTACCACAGAAATTCTACCAGTGTCTATGACCAAGA
CATCAGTAGATTGTACAATGTACATTTGTGGTGATTCAACTGAATGCAGCAATCTTTTGTTGCAATATGGCAGTTTTTGTAC
ACAATTAAACCGTGCTTTAACTGGAATAGCTGTTGAACAAGACAAAAACACCCAAGAAGTTTTTGCACAAGTCAAACAAAT
TTACAAAACACCACCAATTAAAGATTTTGGTGGTTTTAATTTTTCACAAATATTACCAGATCCATCAAAACCAAGCAAGAGG
TCATTTATTGAAGATCTACTTTTCAACAAAGTGACACTTGCAGATGCTGGCTTCATCAAACAATATGGTGATTGCCTTGGTG
ATATTGCTGCTAGAGACCTCATTTGTGCACAAAAGTTTAACGGCCTTACTGTTTTGCCACCTTTGCTCACAGATGAAATGAT
TGCTCAATACACTTCTGCACTGTTAGCGGGTACAATCACTTCTGGTTGGACCTTTGGTGCAGGTGCTGCATTACAAATACCA
TTTGCTATGCAAATGGCTTATAGGTTTAATGGTATTGGAGTTACACAGAATGTTCTCTATGAGAACCAAAAATTGATTGCCA
ACCAATTTAATAGCGCTATTGGCAAAATTCAAGACTCACTTTCTTCCACAGCAAGTGCACTTGGAAAACTTCAAGATGTGGT
CAACCAAAATGCACAAGCTTTAAACACGCTTGTTAAACAACTTAGCTCCAATTTTGGTGCAATTTCAAGTGTTTTAAATGAT
ATCCTTTCACGTCTTGACAAAGTTGAGGCTGAAGTGCAAATTGATAGGTTGATCACAGGCAGACTTCAAAGTTTGCAGACA
TATGTGACTCAACAATTAATTAGAGCTGCAGAAATCAGAGCTTCTGCTAATCTTGCTGCTACTAAAATGTCAGAGTGTGTAC
TTGGACAATCAAAAAGAGTTGATTTTTGTGGAAAGGGCTATCATCTTATGTCCTTCCCTCAGTCAGCACCTCATGGTGTAGT
CTTCTTGCATGTGACTTATGTCCCTGCACAAGAAAAGAACTTCACAACTGCTCCTGCCATTTGTCATGATGGAAAAGCACAC
TTTCCTCGTGAAGGTGTCTTTGTTTCAAATGGCACACACTGGTTTGTAACACAAAGGAATTTTTATGAACCACAAATCATTA
CTACAGACAACACATTTGTGTCTGGTAACTGTGATGTTGTAATAGGAATTGTCAACAACACAGTTTATGATCCTTTGCAACC
TGAATTAGACTCATTCAAGGAGGAGTTAGATAAATATTTTAAGAATCATACATCACCAGATGTTGATTTAGGTGACATCTCT
GGCATTAATGCTTCAGTTGTAAACATTCAAAAAGAAATTGACCGCCTCAATGAGGTTGCCAAGAATTTAAATGAATCTCTC
ATCGATCTCCAAGAACTTGGAAAGTATGAGCAGTATATAAAATGGCCATGGTACATTTGGCTAGGTTTTATAGCTGGCTTG
ATTGCCATAGTAATGGTGACAATTATGCTTTGCTGTATGACCAGTTGCTGTAGTTGTCTCAAGGGCTGTTGTTCTTGTGGAT
CCTGCTGCAAATTTGATGAAGACGACTCTGAGCCAGTGCTCAAAGGAGTCAAATTACATTACACATAAACGAACTTATGGA
TTTGTTTATGAGAATCTTCACAATTGGAACTGTAACTTTGAAGCAAGGTGAAATCAAGGATGCTACTCCTTCAGATTTTGTT
CGCGCTACTGCAACGATACCGATACAAGCCTCACTCCCTTTCGGATGGCTTATTGTTGGCGTTGCACTTCTTGCTGTTTTTCA
TAGCGCTTCCAAAATCATAACCCTCAAAAAGAGATGGCAACTAGCACTCTCCAAGGGTGTTCACTTTGTTTGCAACTTGCTG
TTGTTGTTTGTAACAGTTTACTCACACCTTTTGCTCGTTGCTGTTGGCCTTGAAGCCCCTTTTCTCTATCTTTATGCTTTAGTC
TACTTCTTGCAGAGTATAAACTTTGTAAGAATAATAATGAGGCTTTGGCTTTGCTGGAAATGCCGTTCCAAAAACCCATTAC
TTTATGATGCCAACTATTTTCTTTGCTGGCATACTAATTGTTACGACTATTGTATACCTTACAATAGTGTAACTTCTTCAATTG
TCATTACTTCAGGTGATGGCACAACAAGTCCTATTTCTGAACATGACTACCAGATTGGTGGTTATACTGAAAAATGGGAAT
CTGGAGTAAAAGACTGTGTTGTATTACACAGTTACTTCACTTCAGACTATTACCAGCTGTACTCAACTCAATTGAGTACAGA
CACTGGTGTTGAACATGTTACCTTCTTCATCTACAATAAAATTGTTGATGAGCCTGAAGAACATGTCCAAATTCACACAATC
GACGGTTCATCCGGAGTTGTTAATCCAGTAATGGAACCAATTTATGATGAACCGACGACGACTACTAGCGTGCCTTTGTAA
GCACAAGCTGATGAGTACGAACTTATGTACTCATTCGTTTCGGAAGAGACAGGTACGTTAATAGTTAATAGCGTACTTCTT
TTTCTTGCTTTCGTGGTATTCTTGCTAGTTACACTAGCCATCCTTACTGCGCTTCGATTGTGTGCGTACTGCTGCAATATTGTT
AACGTGAGTCTTGTAAAACCTTCTTTTTACGTTTACTCTCGTGTTAAAAATCTGAATTCTTCTAGAGTTCCTGATCTTCTGGTC
TAAACGAACTAAATATTATATTAGTTTTTCTGTTTGGAACTTTAATTTTAGCCATGGCAGATTCCAACGGTACTATTACCGTT
GAAGAGCTTAAAAAGCTCCTTGAACAATGGAACCTAGTAATAGGTTTCCTATTCCTTACATGGATTTGTCTTCTACAATTTG
CCTATGCCAACAGGAATAGGTTTTTGTATATAATTAAGTTAATTTTTCTCTGGCTGTTATGGCCAGTAACTTTAGCTTGTTTT
GTGCTTGCTGCTGTTTACAGAATAAATTGGATCACCGGTGGAATTGCTATCGCAATGGCTTGTCTTGTAGGCTTGATGTGG
CTCAGCTACTTCATTGCTTCTTTCAGACTGTTTGCGCGTACGCGTTCCATGTGGTCATTCAATCCAGAAACTAACATTCTTCT
CAACGTGCCACTCCATGGCACTATTCTGACCAGACCGCTTCTAGAAAGTGAACTCGTAATCGGAGCTGTGATCCTTCGTGG
ACATCTTCGTATTGCTGGACACCATCTAGGACGCTGTGACATCAAGGACCTGCCTAAAGAAATCACTGTTGCTACATCACG
AACGCTTTCTTATTACAAATTGGGAGCTTCGCAGCGTGTAGCAGGTGACTCAGGTTTTGCTGCATACAGTCGCTACAGGAT
TGGCAACTATAAATTAAACACAGACCATTCCAGTAGCAGTGACAATATTGCTTTGCTTGTACAGTAAGTGACAACAGATGT
TTCATCTCGTTGACTTTCAGGTTACTATAGCAGAGATATTACTAATTATTATGAGGACTTTTAAAGTTTCCATTTGGAATCTT
GATTACATCATAAACCTCATAATTAAAAATTTATCTAAGTCACTAACTGAGAATAAATATTCTCAATTAGATGAAGAGCAAC
CAATGGAGATTGATTAAACGAACATGAAAATTATTCTTTTCTTGGCACTGATAACACTCGCTACTTGTGAGCTTTATCACTA
CCAAGAGTGTGTTAGAGGTACAACAGTACTTTTAAAAGAACCTTGCTCTTCTGGAACATACGAGGGCAATTCACCATTTCA
TCCTCTAGCTGATAACAAATTTGCACTGACTTGCTTTAGCACTCAATTTGCTTTTGCTTGTCCTGACGGCGTAAAACACGTCT
ATCAGTTACGTGCCAGATCAGTTTCACCTAAACTGTTCATCAGACAAGAGGAAGTTCAAGAACTTTACTCTCCAATTTTTCTT
 
ATTGTTGCGGCAATAGTGTTTATAACACTTTGCTTCACACTCAAAAGAAAGACAGAATGATTGAACTTTCATTAATTGACTT
CTATTTGTGCTTTTTAGCCTTTCTGCTATTCCTTGTTTAATTATGCTTATTATCTTTTGGTTCTCACTTGAACTGCAAGATCAT
AATGAAACTTGTCACGCCTAAACTAACATGAAATTTCTTGTTTCTTAGGAATCATCACAACTGTAGCTGCATTTCACCAAG
AATGTAGTTTACAGTCATGTACTCAACATCAACCATATGTAGTTGATGACCCGTGTCCTATTCACTTCTATTCTAAATGGTAT
ATTAGAGTAGGAGCTAGAAAATCAGCACCTTTAATTGAATTGTGCGTGGATGAGGCTGGTTCTAAATCACCCATTCAGTAC
ATCGATATCGGTAATTATACAGTTTCCTGTTTACCTTTTACAATTAATTGCCAGGAACCTAAATTGGGTAGTCTTGTAGTGC
GTTGTTCGTTCTATGAAGACTTTTTAGAGTATCATGACGTTCGTGTTGTTTTAGATTTCATCTAAACGAACAAACTATAATGT
CTGATAATGGACCCCAAAATCAGCGAAATGCACCCCGCATTACGTTTGGTGGACCCTCAGATTCAACTGGCAGTAACCAGA
ATGGAGAACGCAGTGGGGCGCGATCAAAACAACGTCGGCCCCAAGGTTTACCCAATAATACTGCGTCTTGGTTCACCGCT
CTCACTCAACATGGCAAGGAAGACCTTAAATTCCCTCGAGGACAAGGCGTTCCAATTAACACCAATAGCAGTCCAGATGAC
CAAATTGGCTACTACCGAAGAGCTACCAGACGAATTCGTGGTGGTGACGGTAAAATGAAAGATCTCAGTCCAAGATGGT
ATTTCTACTACCTAGGAACTGGGCCAGAAGCTGGACTTCCCTATGGTGCTAACAAAGACGGCATCATATGGGTTGCAACTG
AGGGAGCCTTGAATACACCAAAAGATCACATTGGCACCCGCAATCCTGCTAACAATGCTGCAATCGTGCTACAACTTCCTC
AAGGAACAACATTGCCAAAAGGCTTCTACGCAGAAGGGAGCAGAGGCGGCAGTCAAGCCTCTTCTCGTTCCTCATCACGT
AGTCGCAACAGTTCAAGAAATTCAACTCCAGGCAGCAGTAGGGGAATTTCTCCTGCTAGAATGGCTGGCAATGGCGGTGA
TGCTGCTCTTGCTTTGCTGCTGCTTGACAGATTGAACCAGCTTGAGAGCAAAATGTCTGGTAAAGGCCAACAACAACAAGG
CCAAACTGTCACTAAGAAATCTGCTGCTGAGGCTTCTAAGAAGCCTCGGCAAAAACGTACTGCCACTAAAGCATACAATGT
AACACAAGCTTTCGGCAGACGTGGTCCAGAACAAACCCAAGGAAATTTTGGGGACCAGGAACTAATCAGACAAGGAACT
GATTACAAACATTGGCCGCAAATTGCACAATTTGCCCCCAGCGCTTCAGCGTTCTTCGGAATGTCGCGCATTGGCATGGAA
GTCACACCTTCGGGAACGTGGTTGACCTACACAGGTGCCATCAAATTGGATGACAAAGATCCAAATTTCAAAGATCAAGTC
ATTTTGCTGAATAAGCATATTGACGCATACAAAACATTTCCACCAACAGAGCCTAAAAAGGACAAAAAGAAGAAGGCTGA
TGAAACTCAAGCCTTACCGCAGAGACAGAAGAAACAGCAAACTGTGACTCTTCTTCCTGCTGCAGATTTGGATGATTTCTC
CAAACAATTGCAACAATCCATGAGCAGTGCTGACTCAACTCAGGCCTAAACTCATGCAGACCACACAAGGCAGATGGGCT
ATATAAACGTTTTCGCTTTTCCGTTTACGATATATAGTCTACTCTTGTGCAGAATGAATTCTCGTAACTACATAGCACAAGTA
GATGTAGTTAACTTTAATCTCACATAGCAATCTTTAATCAGTGTGTAACATTAGGGAGGACTTGAAAGAGCCACCACATTTT
CACCGAGGCCACGCGGAGTACGATCGAGTGTACAGTGAACAATGCTAGGGAGAGCTGCCTATATGGAAGAGCCCTAATG
TGTAAAATTAATTTTAGTAGTGCTATCCCCATGTGATTTTAATAGC
SEQ ID NO: 27
   > QPJ72086.1. S-protein surface glycoprotein 
MFVFLVLLPLVSIQCVNLTTRTQLPPAYTNSFTRGVYYPDKVFRSSVLHSTQDLFLPFFSNVTWFHAIHVSGTNGTKRFDNPVLPF
NDGVYFASTEKSNIIRGWIFGTTLDSKTQSLLIVNNATNVVIKVCEFQFCNDPFLGVYYHKNNKSCMESEFRVYSSANNCTFEYVS
QPFLMDLEGKQGNFKNLREFVFKNIDGYFKIYSKHTPINLVRDLPQGFSALEPLVDLPIGINITRFQTLLALHRSYLTPGDSSSGWT
AGAAAYYVGYLQPRTFLLKYNENGTITDAVDCALDPLSETKCTLKSFTVEKGIYQTSNFRVQPTESIVRFPNITNLCPFGEVFNATR
FASVYAWNRKRISNCVADYSVLYNSASFSTFKCYGVSPTKLNDLCFTNVYADSFVIRGDEVRQIAPGQTGKIADYNYKLPDDFTG
CVIAWNSNNLDSKVGGNYNYRYRLFRKSNLKPFERDISTEIYQAGSTPCNGVEGFNCYFPLQSYGFQPTNGVGYQPYRVVVLSF
ELLHAPATVCGPKKSTNLVKNKCVNFNFNGLTGTGVLTESNKKFLPFQQFGRDIADTTDAVRDPQTLEILDITPCSFGGVSVITPG
TNTSNQVAVLYQGVNCTEVPVAIHADQLTPTWRVYSTGSNVFQTRAGCLIGAEHVNNSYECDIPIGAGICASYQTQTNSPRRAR
SVASQSIIAYTMSLGAENSVAYSNNSIAIPTNFTISVTTEILPVSMTKTSVDCTMYICGDSTECSNLLLQYGSFCTQLNRALTGIAVE
QDKNTQEVFAQVKQIYKTPPIKDFGGFNFSQILPDPSKPSKRSFIEDLLFNKVTLADAGFIKQYGDCLGDIAARDLICAQKFNGLTV
LPPLLTDEMIAQYTSALLAGTITSGWTFGAGAALQIPFAMQMAYRFNGIGVTQNVLYENQKLIANQFNSAIGKIQDSLSSTASAL
GKLQDVVNQNAQALNTLVKQLSSNFGAISSVLNDILSRLDKVEAEVQIDRLITGRLQSLQTYVTQQLIRAAEIRASANLAATKMSE
CVLGQSKRVDFCGKGYHLMSFPQSAPHGVVFLHVTYVPAQEKNFTTAPAICHDGKAHFPREGVFVSNGTHWFVTQRNFYEPQ
IITTDNTFVSGNCDVVIGIVNNTVYDPLQPELDSFKEELDKYFKNHTSPDVDLGDISGINASVVNIQKEIDRLNEVAKNLNESLIDL
QELGKYEQYIKWPWYIWLGFIAGLIAIVMVTIMLCCMTSCCSCLKGCCSCGSCCKFDEDDSEPVLKGVKLHYT
SEQ ID NO: 28
   >nucleocapsid phosphoprotein [Severe acute respiratory syndrome coronavirus 2] (Accession No: QIA98561) 
MSDNGPQNQRNAPRITFGGPSDSTGSNQNGERSGARSKQRRPQGLPNNTASWFTALTQHGKEDLKFPRGQGVPINTNSSP
DDQIGYYRRATRRIRGGDGKMKDLSPRWYFYYLGTGPEAGLPYGANKDGIIWVATEGALNTPKDHIGTRNPANNAAIVLQLP
QGTTLPKGFYAEGSRGGSQASSRSSSRSRNSSRNSTPGSSRGTSPARMAGNGGDAALALLLLDRLNQLESKMSGKGQQQQG
QTVTKKSAAEASKKPRQKRTATKAYNVTQAFGRRGPEQTQGNFGDQELIRQGTDYKHWPQIAQFAPSASAFFGMSRIGMEV
TPSGTWLTYTGAIKLDDKDPNFKDQVILLNKHIDAYKTFPPTEPKKDKKKKADETQALPQRQKKQQTVTLLPAADLDDFSKQLQ
QSMSSADSTQA
SEQ ID NO: 29
   >membrane glycoprotein [Severe acute respiratory syndrome coronavirus 2] (Accession No: QIA98557) 
MADSNGTITVEELKKLLEQWNLVIGFLFLTWICLLQFAYANRNRFLYIIKLIFLWLLWPVTLACFVLAAVYRINWITGGIAIAMACL
VGLMWLSYFIASFRLFARTRSMWSFNPETNILLNVPLHGTILTRPLLESELVIGAVILRGHLRIAGHHLGRCDIKDLPKEITVATSRT
LSYYKLGASQRVAGD SGFAAYSRYRIGNYKLNTDHSSSSDNIALLVQ
SEQ ID NO: 30
   >TLR9 agonist oligo
   AACGTTCGAG
SEQ ID NO: 31
   >modified oligo with 2'-deoxy-7-deazaguanosine TCG₁AACG₁TTCG₁
   wherein G₁ is 2'-deoxy-7-deazaguanosine
SEQ ID NO: 32
   >modified oligo with 2'-deoxy-7-deazaguanosine and glycerol TCG₁AACG₁TTCG₁XG₁CTTG₁CAAG₁CT
   wherein G₁ is 2'-deoxy-7-deazaguanosine and X is glycerol

## Claims

1. A SARS-CoV-2 vaccine comprising i) an inactivated SARS-CoV-2 particle; ii) a CpG-containing oligodeoxynucleotide (CpG-ODN); and iii) an alum adjuvant.

2. The SARS-CoV-2 vaccine according to claim 1, wherein said CpG-ODN is CpG 1018 as defined by SEQ ID NO: 4, and said alum adjuvant is aluminium hydroxide.

3. The SARS-Cov-2 vaccine according to any one of the preceding claims, wherein the alum (Al³⁺):CpG (w/w) ratio in the vaccine composition is about 1:10, about 1:5, about 1:4, about 1:3, about 1:2, about 1:1, about 2:1, about 3:1, about 4:1, about 5:1, about 10:1, preferably between about 1:3 and 3:1, more preferably between about 1:2 and 1:1.

4. The SARS-CoV-2 vaccine according to any one of the preceding claims, wherein the alum:CpG (w/w) ratio in the vaccine composition is about 1:2.

5. The SARS-CoV-2 vaccine according to any one of the preceding claims, wherein the alum content of the vaccine is between about 0.1 and 2 mg/mL, between about 0.2 and 1.5 mg/mL, between about 0.5 and 1.3 mg/mL, especially between about 0.8 to 1.2 mg/mL, most preferably about 1 mg/mL, i.e., 0.5 mg/dose, wherein the vaccine is delivered in a volume of 0.5 mL.

6. The SARS-CoV-2 vaccine according to any one of the preceding claims, wherein the CpG content of the vaccine is between about 0.25 and 6 mg/mL, between about 0.5 and 3 mg/mL, between about 1 and 3 mg/mL, especially between about 1.5 to 2.5 mg/mL, most preferably about 2 mg/mL, i.e., 1 mg/dose, wherein the vaccine is delivered in a volume of 0.5 mL.

7. The SARS-CoV-2 vaccine according to any one of the preceding claims, wherein the amount of inactivated SARS-CoV-2 virus per dose in the vaccine is between about 0.01 and 25 mAU (milli-absorption units x minutes), preferably between about 0.05 and 10 mAU, more preferably between about 0.1 and 5 mAU, most preferably between about 0.25 and 2.5 mAU as assessed by SEC-HPLC, wherein the vaccine is delivered in a volume of 0.5 mL.

8. The SARS-CoV-2 vaccine according to any one of the preceding claims, wherein the amount of free (unbound) CpG in the vaccine composition is greater than 10%, greater than 20%, greater than 30%, greater than 40%, greater than 50%, greater than 60%, greater than 70%, greater than 80%, greater than 90%, greater than 95%, preferably about 70% to 95%, most preferably about 80% to 90% (w/w), based on the total amount of CpG in the vaccine composition.

9. The SARS-CoV-2 vaccine according to any one of the preceding claims, wherein the virus particle is a beta-propiolactone-inactivated SARS-CoV-2 particle, preferably at a concentration of 300 to 700ppm, more preferably 500ppm and inactivated for about 1 to 48h, preferably 20 to 28h, most preferred 24h; preferably wherein the inactivated SARS-CoV-2 particle comprises one or more beta-propiolactone-modified cysteine, methionine and/or histidine residues.

10. A SARS-CoV-2 vaccine according to any preceding claim, wherein an inactivated SARS-CoV-2 particle comprises fewer than 200, 100, 50, 30, 20, 15, 10, 9, 8, 7 or 6 beta-propiolactone-modified amino acid residues; preferably wherein a spike (S) protein of the inactivated SARS-CoV-2 particle comprises fewer than 100, 50, 30, 20, 15, 10, 9, 8, 7 or 6 beta-propiolactone-modified amino acid residues; more preferably wherein the inactivated SARS-CoV-2 particle or spike protein thereof comprises 20 or fewer beta-propiolactone-modified amino acid residues; most preferably wherein the inactivated SARS-CoV-2 particle or spike protein thereof comprises 1 to 100, 2 to 50, 3 to 30, 10 to 20 or about 15 beta-propiolactone-modified amino acid residues; and/or
wherein fewer than 20%, 15%, 10%, 5% or 4% of SARS-CoV-2 polypeptides in the particle are beta-propiolactone-modified; preferably wherein 0.1 to 10%, more preferably 1 to 5%, more preferably 2 to 8% or about 3-6% of SARS-CoV-2 polypeptides in the particle, comprise at least one beta-propiolactone modification; preferably as detected in the vaccine by mass spectroscopy, optionally following enzymatic digestion with trypsin, chymotrypsin and/or PNGase F or acid hydrolysis; and/or
(i) a spike (S) protein of the inactivated SARS-CoV-2 particle comprises a beta-propiolactone modification at one or more of the following residues: 49, 146, 166, 177, 207, 245, 379, 432, 519, 625, 1029, 1032, 1058, 1083, 1088, 1101, 1159 and/or 1271; preferably H49, H146, C166, M177, H207, H245, C432, H519, H625, M1029, H1058, H1083, H1088, H1101, H1159 and/or H1271; or H207, H245, C379, M1029 and/or C1032, e.g. in SEQ ID NO:3, or a corresponding position in SEQ ID NO: 19, 21, 23, 25 or 27; and/or
(ii) a membrane (M) glycoprotein of the inactivated SARS-CoV-2 particle comprises a beta-propiolactone modification at one or more of the following residues: 125, 154, 155, 159 and/or 210; preferably H154, H155, C159 and/or H210, e.g. in SEQ ID NO: 29; and/or
(iii) a nucleocapsid (N) protein of the inactivated SARS-CoV-2 particle comprises a beta-propiolactone modification at M234, e.g. in SEQ ID NO: 28; and/or
wherein fewer than 30%, 20%, 10%, 5%, 3% or 1% of one or more of the following residues, preferably of at least 2, 3, 4, 5, 6, 7, 8, 9, 10, 11, 12, 13, 14, 15, 16, 17, 18, 19 or all of the following residues, in the inactivated SARS-CoV-2 particles are beta-propiolactone modified:
(i) in the spike (S) protein, residues 49, 146, 166, 177, 207, 245, 379, 432, 519, 625, 1029, 1032, 1058, 1083, 1088, 1101, 1159 and/or 1271; preferably H49, H146, C166, M177, H207, H245, C432, H519, H625, M1029, H1058, H1083, H1088, H1101, H1159 and/or H1271; or H207, H245, C379, M1029 and/or C1032; e.g. in SEQ ID NO: 3, or a corresponding position in SEQ ID NO: 19, 21, 23, 25 or 27; and/or (ii) in the membrane (M) glycoprotein, residues 125, 154, 155, 159 and/or 210; preferably H154, H155, C159 and/or H210; e.g. in SEQ ID NO: 29; and/or (iii) M234 of the nucleocapsid (N) protein, e.g. in SEQ ID NO: 28; and/or
wherein the proportion of beta-propiolactone-modified residues at each of the following positions in the inactivated SARS-CoV-2 particles is:
(i) in the spike (S) protein (e.g. of SEQ ID NO: 3, or a corresponding position in SEQ ID NO: 19, 21, 23, 25 or 27):
(a) residues H49, H146, C166, H207, H519, M1029, H1083, H1088, H1101, H1159 and/or H1271: less than 20%, preferably 0.01 to 10%, more preferably 0.1 to 5%; and/or
(b) residues M177, C432, H625: less than 30%, preferably 0.1 to 20%, more preferably 1 to 10%; and/or
(c) residues H245, H1058: less than 30%, preferably 0.1 to 20%, more preferably 5 to 15%;
(ii) in the membrane (M) glycoprotein (e.g. of SEQ ID NO: 29):
(f) H154: less than 5%, less than 1% or less than 0.1%; and/or
(g) H155: less than 10%, preferably 0.1 to 5%; and/or
(h) C159: less than 5%, less than 1% or less than 0.1%; and/or
(i) H210: less than 20%, preferably 0.1 to 10%; and/or
(iii) in the nucleocapsid (N) protein (e.g. of SEQ ID NO: 28):
(j) M234: less than 90%, less than 10% or less than 0.1%.

11. The SARS-CoV-2 vaccine according to any one of the preceding claims, wherein the SARS-CoV-2 particle comprises an RNA sequence (and/or fragments thereof, optionally comprising modified (preferably alkylated or acylated) nucleotide residues) corresponding to a DNA sequence (i) as defined by SEQ ID NO: 9; or (ii) having at least 80%, at least 85%, at least 90%, at least 95% or at least 99% sequence identity to SEQ ID NO: 9; preferably wherein a native (non-inactivated) SARS-CoV-2 particle comprising the RNA sequence is able to pack a virulent SARS-CoV-2; and/or
wherein the said vaccine comprises an additional SARS-CoV-2 particle that comprises an RNA sequence (and/or fragments thereof, optionally comprising modified (preferably alkylated or acylated) nucleotide residues) corresponding to a DNA sequence (i) as defined by SEQ ID NO: 18; or (ii) having at least 80%, at least 85%, at least 90%, at least 95% or at least 99% sequence identity to SEQ ID NO: 18; preferably wherein a native (non-inactivated) SARS-CoV-2 particle comprising the RNA sequence is able to pack a virulent SARS-CoV-2; and/or
wherein the said vaccine comprises an additional SARS-CoV-2 particle that comprises an RNA sequence (and/or fragments thereof, optionally comprising modified (preferably alkylated or acylated) nucleotide residues) corresponding to a DNA sequence (i) as defined by SEQ ID NO: 22; or (ii) having at least 80%, at least 85%, at least 90%, at least 95% or at least 99% sequence identity to SEQ ID NO: 22; preferably wherein a native (non-inactivated) SARS-CoV-2 particle comprising the RNA sequence is able to pack a virulent SARS-CoV-2.

12. The SARS-CoV-2 vaccine according to any one of the preceding claims, wherein the CpG ODN is CpG 1018, comprising the sequence 5' TGACTGTGAACGTTCGAGATGA 3' as defined by SEQ ID NO: 4; and/or wherein the alum adjuvant comprises aluminium hydroxide.

13. The SARS-CoV-2 vaccine according to any one of the preceding claims, wherein the vaccine is able to seroconvert a subject that is administered the SARS-CoV vaccine with at least a 70%, 80%, 85%, 90%, or 95% probability.

14. A method of producing a SARS-CoV-2 vaccine, comprising:
(a) producing native SARS-CoV-2 particles;
(b) inactivating the native SARS-CoV-2 particles to obtain inactivated SARS-CoV-2 particles;
(c) incorporating the inactivated SARS-CoV-2 particles in a vaccine composition comprising a CpG-containing oligodeoxynucleotide (CpG ODN) and an alum adjuvant.

15. A pharmaceutical composition for use in the prevention or treatment of a SARS-CoV-2 infection in a subject, wherein said pharmaceutical composition is a vaccine of any one of claims 1 to 13, optionally in combination with one or more pharmaceutically acceptable excipients.
